# EUROPEAN PATENT APPLICATION

(11) **EP 4 397 664 A1**
(43) Date of publication of application: **10.07.2024**
(21) Application number: 22863520.7
(22) Date of filing: 31.08.2022
(51) Int. Cl.: C07D 498/14, C07D 498/22, A61K 31/553, A61P 35/00

(54) **PYRIMIDINE-FUSED CYCLIC COMPOUND AND PREPARATION METHOD AND USE THEREOF**

(30) Priority: 31.08.2021 CN 202111011437; 30.09.2021 CN 202111164532; 07.01.2022 CN 202210017432; 28.01.2022 CN 202210106509; 29.03.2022 CN 202210320649; 17.08.2022 CN 202210986983
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: JIANG, Tao, Shanghai 201203 (CN); ZHOU, Fusheng, Shanghai 201203 (CN); LIANG, Tao, Shanghai 201203 (CN); ZHANG, Leitao, Shanghai 201203 (CN); CAI, Lijian, Shanghai 201203 (CN); LIU, Zhubo, Shanghai 201203 (CN); HUANG, Qi, Shanghai 201203 (CN); XU, Xiaoming, Shanghai 201203 (CN); ZHAO, Jichen, Shanghai 201203 (CN); LU, Yandong, Shanghai 201203 (CN); MA, Kai, Shanghai 201203 (CN); LIN, Chonglan, Shanghai 201203 (CN); LAN, Jiong, Shanghai 201203 (CN)
(74) Representative: Elzaburu S.L.P.
(86) International application number: PCT/CN2022/116210
(87) International publication number: WO 2023/030385

(57) **Abstract**

Provided are a pyrimidine-fused cyclic compound represented by formula (A) having an inhibitory effect on KRAS gene mutation, or a pharmaceutically acceptable salt, a stereoisomer or a solvate thereof, a pharmaceutical composition containing the compound, and an application thereof in the preparation of anticancer drugs

## Description

### TECHNICAL FIELD

The present disclosure belongs to the field of medical technology, and specifically relates to a class of pyrimidine-fused cyclic compounds and preparation method and use thereof.

### BACKGROUND

KRAS is a member of 21kD protein of the Ras family of GTP enzyme proteins, and is an essential component for cell signaling. The role of KRAS in malignant tumors and mutations in various tumor types (e.g., G12C mutation, G12D mutation, G12V mutation, etc.) are known to the public, and thus KRAS has become a very attractive target for cancer therapy in the pharmaceutical industry. Compounds that inhibit KRAS activity are highly desirable to researchers in the art and are under sizzling research. At present, breakthroughs have been made in the art for KRAS G12C, e.g., KRAS G12C inhibitors of AMG, MIRATI have shown adequate safety and efficacy. However, there is a continuing interest and effort to develop inhibitors of KRAS, in particular inhibitors for activating KRAS mutants, especially KRAS G12D.

### SUMMARY OF THE INVENTION

The present disclosure provides a novel pyrimidine-fused cyclic compound, which is used as a KRAS G12D inhibitor with high activity, good selectivity, low toxic and low side effects, etc.

In a first aspect, the present disclosure provides a compound, or a pharmaceutically acceptable salt, a stereoisomer or a solvate thereof, wherein the compound is a compound shown in formula (A): 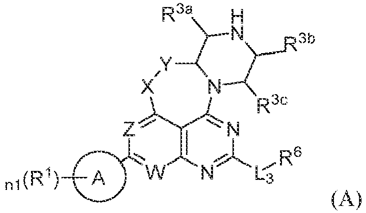 in the formula (A),
X is O, S or NR¹¹; wherein R¹¹ is selected from H, C1-C6 alkyl, C1-C6 deuterated alkyl and cycloalkyl;
Y is CR¹²R¹³, CR¹⁴R¹⁵CR¹⁶R¹⁷, C(O) or C(O)CR¹⁸R¹⁹; wherein
R¹², R¹³ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; or R¹², R¹³ with the carbon atoms to which R¹², R¹³ are attached to form a cycloalkyl;
R¹⁴ and R¹⁵ are each independently selected from H, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; or R¹⁴, R¹⁵ with the carbon atoms to which R¹⁴, R¹⁵ are attached to form a cycloalkyl;
R¹⁶, R¹⁷ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²;
R¹⁸, R¹⁹ are each independently selected from H, C1-C3 alkyl and halogen;
R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl;
wherein the cycloalkyl, the heterocyclyl, the aryl, the heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy;
W is N or CR²⁰; wherein R²⁰ is H, cyano, C1-C6 alkyl, halogen, C1-C6 halogenated alkyl, C1-C6 alkoxy, cycloalkyl, cycloalkyl-O-, heterocyclyl or heterocyclyl-O-; wherein the cycloalkyl, the heterocyclyl are independently and optionally substituted by halogen;
ring A (the ring A used herein refers to 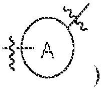 is selected from the group: aryl and heteroaryl;
R¹ is a substituent at any position on ring A; n1 is 0, 1, 2, 3, 4 or 5;
each R¹ is respectively and independently selected from: C1-C6 alkyl, halogen, hydroxyl, cyano, C1-C6 alkoxy, C1-C6 halogenated alkyl, C1-C6 halogenated alkoxy, cyano, NR²¹R²², C(O)NR²³R²⁴, CH₂R²⁵, N=S(O) (C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxy) C1-C6 halogenated alkyl- or cycloalkyl; wherein the cycloalkyl is optionally substituted by halogen or C1-C6 alkyl; wherein,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C6 alkyl or C1-C6 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SOzCI-C6 alkyl; wherein R²⁵¹, R²⁵², R²⁵³, R²⁵⁴ are each independently H or C1-C6 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C6 alkyl, C1-C6 halogenated alkyl or NR²⁶¹R²⁶², wherein R²⁶¹, R²⁶² are each independently H or C1-C6 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
Z is N or CR²; wherein R² is H, cyano, halogen, C1-C6 alkyl, C1-C6 alkoxy, C1-C6 halogenated alkyl, C1-C6 halogenated alkoxy, C1-C6 deuterated alkoxy, NR³¹R³², C2-C4 alkynyl or CH₂OR³³; wherein R³¹, R³² are each independently hydrogen or C1-C6 alkyl; or R³¹, R³² are taken together with the nitrogen atom to which R³¹, R³² are attached to form a 3- to 6-membered nitrogen-containing heterocyclyl; R³³ is hydrogen or C1-C6 alkyl;
R^{3a}, R^{3b}, R^{3c} are each independently hydrogen, halogen, hydroxyl, cyano, C1-C3 alkyl, C1-C3 deuterated alkyl or C1-C3 halogenated alkyl; or
R^{3a} and R^{3b} are connected to form -CHR^{3a1}- or -CHR^{3a2}CHR^{3a3}-; R^{3c} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl; or
R^{3a} and R^{3c} are connected to form -CHR^{3a1}- or -CHR^{3a2}CHR^{3a3}-; R^{3b} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl;
L₁ is a bond, O or NR³⁴;
R⁶ is -L-6- to 10-membered fused heterocyclyl, -L-7- to 11-membered spiro heterocyclyl, -L-spirocyclic ring substituted 6- to 10-membered fused heterocyclyl, -L-spirocyclic ring substituted 7-to 11-membered spiro heterocyclyl, -L-fused ring substituted 6- to 10-membered fused heterocyclyl, -L-fused ring substituted 7- to 11-membered spiro heterocyclyl or -L-spirocyclic and fused ring substituted 6- to 10-membered fused heterocyclyl;
the spirocyclic ring substituted 6- to 10-membered fused heterocyclyl refers to that two hydrogen atoms on the same carbon atom of any one or two carbon atoms on the 6- to 10-membered fused heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 6- to 10-membered fused heterocyclyl ;
the spirocyclic ring substituted 7- to 11-membered spiro heterocyclyl refers to that two hydrogen atoms on the same carbon atom of any one or two carbon atoms on the 7- to 11-membered spiro heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 7- to 11-membered spiro heterocyclyl;
the fused ring substituted 6- to 10-membered fused heterocyclyl refers to that one hydrogen atom on each carbon atom on the same carbon atom pair of any one or two pairs of adjacent carbon atoms on the 6- to 10-membered fused heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 6- to 10-membered fused heterocyclyl; or the fused ring substituted 6- to 10-membered fused heterocyclyl refers to that hydrogen atoms on any two non-adjacent carbon atoms on the 6- to 10-membered fused heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 6- to 10-membered fused heterocyclyl;
the fused ring substituted 7- to 11-membered spiro heterocyclyl refers to that one hydrogen atom on each carbon atom on the same carbon atom pair of any one or two pairs of adjacent carbon atoms on the 7- to 11-membered spiro heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 7- to 11-membered spiro heterocyclyl; or the fused ring substituted 7- to 11-membered spiro heterocyclyl refers to that hydrogen atoms on any two non-adjacent carbon atoms on the 7- to 11-membered spiro heterocyclyl are substituted by -(CH₂)ₘ₄-, -(CH₂)ₘ₅(CH₂)ₘ₆- or -(CH₂)ₘ₇NRⁿ(CH₂)ₘ₈ to form fused ring substituted 7- to 11-membered spiro heterocyclyl;
the spirocyclic and fused ring substituted 6- to 10-membered fused heterocyclyl refers to that one hydrogen atom on each carbon atom on any pair of adjacent carbon atoms on the 6- to 10-membered fused heterocyclyl is simultaneously substituted by -(CH₂)ₘ₄-, -(CH₂)ₘ₅O(CH₂)m₆- or - (CH₂)ₘ₇NRⁿ(CH₂)ₘ₈ to form paracyclic substituent, and two hydrogen atoms on the other carbon atom on the 6- to 10-membered fused heterocyclyl are simultaneously substituted by -(CH₂)ₘ₄-, - (CH₂)ₘ₅O(CH₂)ₘ₆- or -(CH₂)ₘ₇NRⁿ(CH₂)ₘ₈ to form spirocyclic ring substituent, thereby to form spirocyclic and fused ring substituted 6- to 10-membered fused heterocyclyl;
the 6- to 10-membered fused heterocycly or the 7- to 11-membered spiro heterocyclyl containing 1, 2, 3 or 4 heteroatoms selected from N(R^{m}), S, S(=O), S(=O)₂, O as ring atoms;
the spirocyclic ring substituted 6- to 10-membered fused heterocyclyl, the spirocyclic ring substituted 7- to 11-membered spiro heterocyclyl, the fused ring substituted 6- to 10-membered fused heterocyclyl, the fused ring substituted 7- to 11-membered spiro heterocyclyl or the spirocyclic and fused ring substituted 6- to 10-membered fused heterocyclyl each independently containing 1, 2, 3, 4 or 5 heteroatoms selected from N(R^{m}), S, S(=O), S(=O)₂, O as ring atoms;
Rⁿ is hydrogen, C1-C4 alkyl, deuterated C1-C4 alkyl, C1-C4 halogenated alkyl, C3-C20 cycloalkyl or 3- to 20-membered heterocyclyl;
R^{m} is absent, hydrogen, C1-C4 alkyl, deuterated C1-C4 alkyl, C1-C4 halogenated alkyl, C3-C20 cycloalkyl or 3- to 20-membered heterocyclyl;
wherein the 6- to 10-membered fused heterocyclyl, the 7- to 11-membered spiro heterocyclyl are each independently saturated or partially unsaturated; when the 6- to 10-membered fused heterocyclyl, the 7- to 11-membered spiro heterocyclyl is partially unsaturated, the ring containing 1 or 2 double bonds;
two hydrogen atoms on the same carbon atom of any one or two carbon atoms on the 6- to 10-membered fused heterocyclyl (the 6- to 10-membered fused heterocyclyl refers to the 6- to 10-membered fused heterocyclyl in -L-6- to 10-membered fused heterocyclyl, -L- spirocyclic ring substituted 6- to 10-membered fused heterocyclyl, -L-fused ring substituted 6- to 10-membered fused heterocyclyl or -L-spirocyclic and fused ring substituted 6- to 10membered fused heterocyclyl) are optionally and simultaneously substituted by =CR^{2a}R^{2b}; the 6- to 10-membered fused heterocyclyl is also optionally substituted by one or more R^{6a};
two hydrogen atoms on the same carbon atom of any one or two carbon atoms on the 7- to 11-membered spiro heterocyclyl (the 7- to 11-membered spiro heterocyclyl refers to the 7- to 11-membered spiro heterocyclyl in -L-7- to 11-membered spiro heterocyclyl, -L-spirocyclic ring substituted 7- to 11-membered spiro heterocyclyl or -L-fused ring substituted 7- to 11-membered spiro heterocyclyl) are optionally and simultaneously substituted by =CR^{2a}R^{2b}; the 7- to 11-membered spiro heterocyclyl is also optionally substituted by one or more R^{6a};
wherein R^{2a}, R^{2b} are each independently hydrogen, halogen, cyano, C1-C4 alkyl, C1-C4 halogenated alkyl, C6-C10 aryl or 5- or 6-membered heteroaryl; the C6-C10 aryl or 5- or 6-membered heteroaryl is optionally substituted by 1, 2, 3 groups selected from halogen, cyano, C1-C4 alkyl, C1-C4 alkoxy, C1-C4 halogenated alkyl, C1-C4 halogenated alkoxy; or each pair of R^{2a}, R^{2b} independently with the carbon atom to which R^{2a}, R^{2b} are attached together form a C3-C6 monocyclic cycloalkyl or a 3-to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl;
each R^{6a} is respectively and independently halogen, hydroxyl, C1-C6 hydroxyalkyl, C1-C6 alkyl, C2-C6 alkenyl, C2-C6 halogenated alkenyl, C2-C6 alkynyl, C2-C6 halogenated alkynyl, C1-C6 halogenated alkyl, C1-C6 alkoxy, C1-C6 halogenated alkoxy, C3-C6 cycloalkyl, cyano, -Q-phenyl, -Q-phenyl-SOzF, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})₂, (C1-C3 alkoxy)C1-C3 alkyl, (C1-C3 alkyl)C(O)-, oxo, (C1-C3 halogenated alkyl)C(O)-, -SO₂F, (C1-C3 alkoxy)C1-C3 alkoxy, - CH₂OC(O)N(R^{6b})₂, -CH₂NHC(O)OC1-C6 alkyl, -CH₂NHC(O)N(R^{6b})₂, -CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, - UC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O)C1-C6 alkyl, -OC(O)C1-C6 halogenated alkyl, -C1-C3 alkyl-OC(O)C1-C6 alkyl, -C1-C3 alkyl-OC(O)C1-C6 halogenated alkyl, -OC(O)phenyl, -C1-C3 alkyl-OC(O)phenyl or -CH₂ heterocyclyl; wherein the phenyl in the group above is optionally substituted by -C(O)H or OH; the heterocyclyl in the -C1-C3 alkyl-heterocyclyl is optionally substituted by oxo; Q is a bond or O; each R^{6b} above is independently hydrogen, C1-C6 alkyl, C1-C6 deuterated alkyl or C1-C6 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy; the C3-C6 cycloalkyl is optionally substituted by 1 or 2 C1-C6 alkyl;
each m4 is 2, 3 or 4;
each m5, m6, m7, m8 are each independently 0, 1, 2 or 3; m5 and m6 are not simultaneously 0; m7 and m8 are not simultaneously 0;
   or
R⁶ is hydrogen, -N(R³⁴)₂, heterocyclyl, cycloalkyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH or -L-C(O)OC1-C6 alkyl; wherein the heterocyclyl, the cycloalkyl, the aryl in the -L-NR³⁴C(O)-aryl, the heterocyclyl in the - L-heterocyclyl, the cycloalkyl in the -L-cycloalkyl each optionally substituted by one or more R³⁵ or two hydrogen atoms on the same carbon atom are optionally and simultaneously substituted by - CH₂CH₂- or -CH₂CH₂CH₂- to form cyclopropyl or cyclobutyl; the aryl in the -L-aryl, the heteroaryl in the -L-heteroaryl each optionally substituted by one or more R³⁶;
wherein each L is independently a bond, C1-C4 alkylene or heteroaryl; wherein one or two hydrogen atoms on any carbon atom or on any same carbon atom of the C1-C4 alkylene are independently and optionally substituted by deuterium, C1-C6 alkyl, C1-C6 halogenated alkyl, C2-C4 alkenylene or C2-C4 halogenated alkenylene; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, - (CH₂)ₘ₁-NR⁹-(CH₂)ₘ₂- to form a cyclic substituent; m3 is 1 or 2; each m1 is respectively and independently 0, 1, 2, or 3; each m2 is respectively and independently 0, 1, 2, or 3; and m1, m2 are not simultaneously 0;
R⁹ is hydrogen, C1-C6 alkyl, C1-C6 deuterated alkyl, C1-C6 halogenated alkyl, C1-C6 alkyl-hydroxyl, C1-C6 alkyl-cyano, C1-C6 alkoxy, C1-C6 alkyl-Cl-C6 alkoxy, C3-C6 monocyclic cycloalkyl, C3-C6 deuterated cycloalkyl or C3-C6 halogenated cycloalkyl;
each R³⁵ is respectively and independently halogen, hydroxyl, C1-C3 alkyl, C2-C4 alkenylene, C2-C4 halogenated alkenylene, C1-C3 halogenated alkyl, C1-C3 alkoxy, C3-C6 cycloalkyl, -C1-C3 alkyl-C1-C3 alkoxy, -C1-C3 alkyl-hydroxyl, heterocyclyl(e.g., 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy)C1-C3 alkyl, (C1-C3 alkyl)C(O), oxo, (C1-C3 halogenated alkyl)C(O)-, -SO₂F, (C1-C3 alkoxy)Cl-C3 alkoxy, -CH₂OC(O)N(R³⁴)₂, -CH₂NHC(O)OC1-C6 alkyl, -CH₂NHC(O)N(R³⁴)₂, - CH₂NHC(O)C1-C6 alkyl, -CH₂(pyrazolyl), -CH₂NHSO₂C1-C6 alkyl, -CH₂OC(O)heterocyclyl, - OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, - OC(O)heterocyclyl(e.g., -OC(O)-3- to 6-membered heterocyclyl) or -CH₂ heterocyclyl (e.g., -CH₂-3- to 6-membered heterocyclyl); wherein, the phenyl in -NHC(O)phenyl or -OC(O)NH(C1-C3 alkyl)O(C1-C3 alkyl)phenyl is optionally substituted by -C(O)H or OH; the heterocyclyl in the - CH₂ heterocyclyl is optionally substituted by oxo; the hydrogen atom on -C1-C3 alkyl- is substituted by 1 or 2 groups selected from halogen, deuterium, C1-C3 alkyl, C1-C3 halogenated alkyl;
each R³⁶ is respectively and independently halogen, hydroxyl, HC(O)-, C1-C4 alkyl, C1-C4 alkoxy, C1-C4 halogenated alkyl, C1-C4 hydroxyalkyl or -N(R³⁴)₂;
each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, each R^{6a} is respectively and independently halogen, hydroxyl, C1-C6 hydroxyalkyl, C1-C6 alkyl, C2-C6 alkenyl, C2-C6 halogenated alkenyl, C2-C6 alkynyl, C2-C6 halogenated alkynyl, C1-C6 halogenated alkyl, C1-C6 alkoxy, C1-C6 halogenated alkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})₂, (C1-C3 alkoxy)C1-C3 alkyl, (C1-C3 alkyl)C(O)-, oxo, (C1-C3 halogenated alkyl)C(O)-, -SO₂F, (C1-C3 alkoxy)C1-C3 alkoxy, -CH₂OC(O)N(R^{6b})₂, -CH₂NHC(O)OC1-C6 alkyl, -CH₂NHC(O)N(R^{6b})₂, - CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O)C1-C6 alkyl, -OC(O)C1-C6 halogenated alkyl, -C1-C3 alkyl-OC(U)C1-C6 alkyl, -C1-C3 alkyl-OC(O)C1-C6 halogenated alkyl, -OC(O)phenyl, -C1-C3 alkyl-OC(O)phenyl or -CH₂ heterocyclyl; wherein the phenyl in the group above is optionally substituted by -C(O)H or OH; the heterocyclyl in the -C1-C3 alkyl-heterocyclyl is optionally substituted by oxo; Q is a bond or O; each R^{6b} above is independently hydrogen, C1-C6 alkyl, C1-C6 deuterated alkyl or C1-C6 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy; the C3-C6 cycloalkyl is optionally substituted by 1 or 2 C1-C6 alkyl;

In an embodiment, each R^{6a} is respectively and independently halogen, hydroxyl, C1-C3 hydroxyalkyl, C1-C3 alkyl, C2-C4 alkenyl, halogenated C2-C4 alkenyl, C2-C4 alkynyl, halogenated C2-C4 alkynyl, C1-C3 halogenated alkyl, C1-C3 alkoxy, C1-C3 halogenated alkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})₂, (C1-C3 alkoxy)Cl-C3 alkyl, (C1-C3 alkyl)C(O)-, oxo, (C1-C3 halogenated alkyl)C(O)-, -SO₂F, (C1-C3 alkoxy)C1-C3 alkoxy, -CH₂OC(O)N(R^{6b})₂, -CH₂NHC(O)OC1-C3 alkyl, -CH₂NHC(O)N(R^{6b})₂, - CH₂NHC(O)C1-C3 alkyl, -CH₂(pyrazolyl), -CH₂NHSO₂C1-C3 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 allcyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O) C1-C3 alkyl, -OC(O)C1-C3 halogenated alkyl, -C1-C3 alkyl-OC(O)C1-C3 alkyl, -C1-C3 alkyl-OC(O)C1-C3 halogenated alkyl, -OC(O)phenyl, -C1-C3 alkyl-OC(O)phenyl or -CH₂ heterocyclyl; wherein the phenyl in the above group is optionally substituted by -C(O)H or OH; the heterocyclyl in the -C1-C3 alkyl-heterocyclyl is optionally substituted by oxo; Q is a bond or U; each R^{6b} above is independently hydrogen, C1-C3 alkyl, C1-C3 deuterated alkyl or C1-C3 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy; the C3-C6 cycloalkyl is optionally substituted by 1 or 2 C1-C6 alkyl;

In an embodiment, each R^{6a} is respectively and independently halogen, hydroxyl, C1-C3 alkyl, C2-C4 alkenyl, C2-C4 halogenated alkenyl, C2-C4 alkynyl, C1-C3 halogenated alkyl, C3-C6 cycloalkyl, cyano, -N(R^{6b})₂; each R^{6b} above is independently hydrogen, C1-C3 alkyl, C1-C3 deuterated alkyl or C1-C3 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy; the C3-C6 cycloalkyl is optionally substituted by 1 or 2 methyl;

In an embodiment, each R^{6a} is respectively and independently halogen, hydroxyl, methyl, ethyl, propyl, vinyl, halogenated vinyl (e.g., monofluorovinyl, difluorovinyl, etc.), ethynyl, propynyl, halogenated methyl, halogenated ethyl, cyclopropyl, cyano, -N(R^{6b})₂; wherein each R^{6b} above is respectively and independently hydrogen or methyl; the cyclopropyl is optionally substituted by one or two methyl.

In an embodiment, the compound is shown in formula (I): 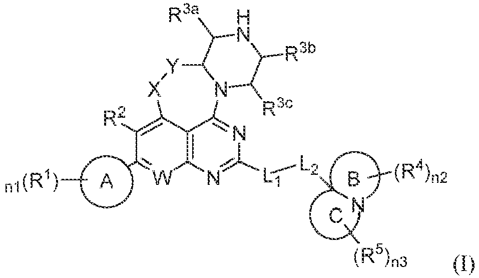 in the formula,
X, Y, W, ring A, R¹, n1, R² are each defined as above;
R^{3a}, R^{3b}, R^{3c} are each independently hydrogen, halogen, hydroxyl, cyano, C1-C3 alkyl, C1-C3 deuterated alkyl or C1-C3 halogenated alkyl;
L₁ is a bond, O or NR³⁴;
L₂ is C1-C4 alkylene or heteroaryl, wherein one or two hydrogen atoms on any carbon atom or on any same carbon atom of the C1-C4 alkylene are independently and optionally substituted by deuterium, C1-C6 alkyl, or C1-C6 halogenated alkyl; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1, m2 are not simultaneously 0;
ring B is a 3- to 6-membered nitrogen-containing heterocyclyl;
ring C is a 3- to 6-membered nitrogen-containing heterocyclyl;
R⁴ is a substituent at any position on ring B; n2 is 0, 1, 2 or 3;
R⁵ is a substituent at any position on ring C; n3 is 0, 1, 2 or 3;
R⁴, R⁵ are defined as follow:
   (a) each R⁴, each R⁵ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SOzF, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), - CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -UC(O)NH(C1-C3 alkyl)O(C1-C3 alkyl)phenyl(Cl-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
   (b) one R⁴ is -CH₂CH₂- or -CH₂CH₂CH₂-, and the R⁴ are taken together with the carbon atom to which R⁴ is attached to form a cyclopropyl or cyclobutyl; the remaining R⁴, each R⁵ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), - UC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in - NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
   (c) one R⁵ is -CH2CH₂- or -CH₂CH₂CH₂-, and the R⁵ is taken together with the carbon atom to which R⁵ is attached to form a cyclopropyl or cyclobutyl; the remaining R⁵, each R⁴ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), - OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂ heterocyclyl; wherein, the phenyl in - NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
   (d) one R⁴ is -CH₂CH₂- or -CH₂CH₂CH₂-, and the R⁴ are taken together with the carbon atom to which R⁴ is attached to form a cyclopropyl or cyclobutyl; one R⁵ is -CH₂CH₂- or -CH₂CH₂CH₂-, and the R⁵ is taken together with the carbon atom to which R⁵ is attachedto form a cyclopropyl or cyclobutyl; the remaining R⁴, the remaining R⁵ are each independently halogen, hydroxyl, C1-C3 hydroxyalkyl, C1-C3 alkyl, C1-C3 halogenated alkyl, C2-C4 alkenylene, C2-C4 halogenated alkenylene, C1-C3 alkoxy, C2-C6 alkenyl, C2-C6 halogenated alkenyl, cyano, phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, - OC(O)NH(C1-C3 alkyl)O(C1-C3 alkyl), -OC(O)NH(C1-C3 allcyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(C1-C3 alkyl)phenyl is optionally substituted by -C(O)H or OH; the heterocyclyl in -CH₂ heterocyclyl is optionally substituted by oxo;
each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, the compound is shown in formula (II): 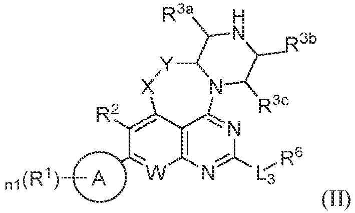
in the formula, X, Y, W, ring A, R¹, n1, R², L₃, R⁶ are each defined as above;
R^{3a} and R^{3b} are connected to form -CHR^{3a1}- or -CHR^{3a2}CHR^{3a3}-; R^{3c} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl; or
R^{3a} and R^{3c} are connected to form -CHR^{3a1}- or -CHR^{3a2}CHR^{3a3}-; R^{3b} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl;

In an embodiment, the compound is shown in formula (II-1) or formula (II-2): 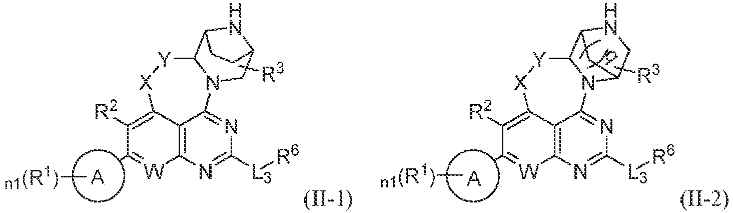 in each formula, q is 1 or 2; R³ is a substituent at any position on a bridge ring (as used herein, the bridge ring where R³ located is generally bridge ring as 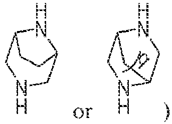 , and is respectively and independently hydrogen, halogen, hydroxyl, cyano, C1-C3 alkyl, C1-C3 deuterated alkyl or C1-C3 halogenated alkyl; R¹, n1, ring A, R², X, Y, W, L₃, R⁶ are each defined as above.

In an embodiment, the compound is shown in formula (III): 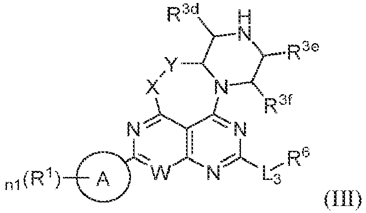 in the formula, X, Y, W, ring A, R¹, n1, L₃, R⁶ are each defined as above;
R ^{3d}, R^{3e}, R^{3f} are each independently hydrogen, halogen, hydroxyl, cyano, C1-C3 alkyl, C1-C3 deuterated alkyl or C1-C3 halogenated alkyl; or
R^{3d} and R^{3e} are connected to form -CHR^{3d1}- or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3d1}, R^{3d2}, R^{3d3} are each independently hydrogen, halogen, hydroxyl, cyano, C1-C3 alkyl, C1-C3 deuterated alkyl or C1-C3 halogenated alkyl; or
R^{3d} and R^{3f} are connected to form -CHR^{3f1}- or -CHR^{3f2}CHR^{3f3}-; R^{3e} is hydrogen, halogen, hydroxyl, cyano, C1-C3 alkyl, C1-C3 deuterated alkyl or C1-C3 halogenated alkyl; R^{3f1}, R^{3f2}, R^{3f3} are each independently hydrogen, halogen, hydroxyl, cyano, C1-C3 alkyl, C1-C3 deuterated alkyl or C1-C3 halogenated alkyl;

In an embodiment, the present disclosure provides a compound, or a pharmaceutically acceptable salt, a stereoisomer or a solvate thereof shown in formula (I): 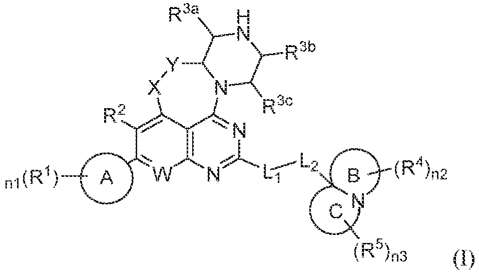
in the formula, X is O or NR¹¹; wherein R¹¹ is selected from H and C1-C6 alkyl;
Y is CR¹²R¹³, CR¹⁴R¹⁵CR¹⁶R¹⁷, C(O) or C(O)CR¹⁸R¹⁹; wherein R¹², R¹³, R¹⁴ and R¹⁵ are each independently selected from H, cyano, C1-C3 alkyl, C1-C3 deuterated alkyl and C1-C3 halogenated alkyl; R¹⁶, R¹⁷, R¹⁸, R¹⁹ are each independently selected from H, C1-C3 alkyl and halogen;
W is N or CR²⁰; wherein R²⁰ is H, cyano, C1-C6 alkyl, halogen, C1-C6 halogenated alkyl, C1-C6 alkoxy, cycloalkyl, cycloalkyl-O-, heterocyclyl or heterocyclyl-O-; wherein the cycloalkyl, the heterocyclyl are each independently substituted by halogen;
ring A is selected from the group: aryl, heteroaryl;
R¹ is a substituent at any position on ring A; each R¹ is respectively and independently selected from: C1-C6 alkyl, halogen, hydroxyl, C1-C6 alkoxy, C1-C6 halogenated alkyl, C1-C6 halogenated alkoxy, cyano, NR²¹R²², C(O)NR²³R²⁴, CH₂R²⁵, N=S(O) (C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxy) C1-C6 halogenated alkyl- or cycloalkyl; wherein the cycloalkyl is optionally substituted by halogen or C1-C6 alkyl; wherein,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C6 alkyl or C1-C6 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; wherein R²⁵¹, R²⁵², R²⁵³, R²⁵⁴ are each independently H or C1-C6 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C6 alkyl, C1-C6 halogenated alkyl or NR²⁶¹R²⁶², wherein R²⁶¹, R²⁶² are each independently H or C1-C6 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxy, C1-C6 halogenated alkyl, C1-C6 halogenated alkoxy, C1-C6 deuterated alkoxy, NR³¹R³², C2-C4 alkynyl or CH₂OR³³; wherein R³¹, R³², R³³ are each independently hydrogen or C1-C6 alkyl;
R^{3a}, R^{3b}, R^{3c} are each independently hydrogen, halogen, hydroxyl, cyano, C1-C3 alkyl, C1-C3 deuterated alkyl or C1-C3 halogenated alkyl;
L₁ is a bond, O or NR³⁴;
L₂ is C1-C4 alkylene or heteroaryl, wherein one or two hydrogen atoms on any carbon atom or on any same carbon atom of the C1-C4 alkylene are independently and optionally substituted by deuterium, C1-C6 alkyl, or C1-C6 halogenated alkyl; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m 1, m2 are not simultaneously 0;
ring B is a 3- to 6-membered nitrogen-containing heterocyclyl;
ring C is a 3- to 6-membered nitrogen-containing heterocyclyl;
R⁴ is a substituent at any position on ring B; n2 is 0, 1, 2 or 3;
R⁵ is a substituent at any position on ring C; n3 is 0, 1, 2 or 3;
R⁴, R⁵ are defined as follow:
   (a) each R⁴, each R⁵ are respectively and independently halogen, hydroxyl, C1-C3 hydroxyalkyl, C1-C3 alkyl, C1-C3 halogenated alkyl, C1-C3 alkoxy, cyano, -phenyl, -phenyl-SO₂F, -O-phenyl, - U-phenyl-SUzF, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, - CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, - OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(C1-C3 alkyl)phenyl is optionally substituted by -C(O)H or OH; the heterocyclyl in -CH₂ heterocyclyl is optionally substituted by oxo; or
   (b) one R⁴ is -CH₂CH₂- or -CH₂CH₂CH₂-, and the R⁴ are taken together with the carbon atom to which R⁴ is attached to form a cyclopropyl or cyclobutyl; the remaining R⁴, each R⁵ are respectively and independently halogen, hydroxyl, C1-C3 hydroxyalkyl, C1-C3 alkyl, C1-C3 halogenated alkyl, C1-C3 alkoxy, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), - OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in - NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
   (c) one R⁵ is -CH₂CH₂- or -CH₂CH₂CH₂-, and the R⁵ is taken together with the carbon atom which R⁵ is attached to form a cyclopropyl or cyclobutyl; the remaining R⁵, each R⁴ are respectively and independently halogen, hydroxyl, C1-C3 hydroxyalkyl, C1-C3 alkyl, C1-C3 halogenated alkyl, C1-C3 alkoxy, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -UC(O)NH(C1-C3 alkyl)O(C1-C3 alkyl), - OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂ heterocyclyl; wherein, the phenyl in - NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
   (d) one R⁴ is -CH₂CH₂- or -CH₂CH₂CH₂-, and the R⁴ are taken together with the carbon atom to which R⁴ is attached to form a cyclopropyl or cyclobutyl; one R⁵ is -CH₂CH₂- or -CH₂CH₂CH₂-, and the R⁵ is taken together with the carbon atom to which R⁵ is attached to form a cyclopropyl or cyclobutyl; the remaining R⁴, the remaining R⁵ are each independently halogen, hydroxyl, C1-C3 hydroxyalkyl, C1-C3 alkyl, C1-C3 halogenated alkyl, C1-C3 alkoxy, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, - CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CHzOC(O) heterocyclyl, -OC(O)N(R³⁴)₂, - OC(O)NH(C1-C3 alkyl)O(C1-C3 alkyl), -OC(O)NH(C1-C3 allcyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(C1-C3 alkyl)phenyl is optionally substituted by -C(O)H or OH; the heterocyclyl in -CH₂ heterocyclyl is optionally substituted by oxo; each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, the compound is shown in formula (1-1) or formula (1-2): 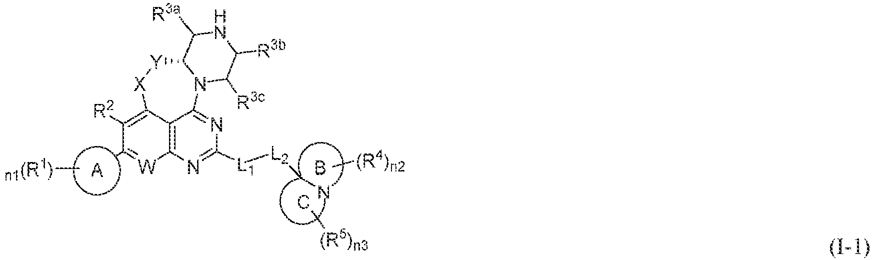 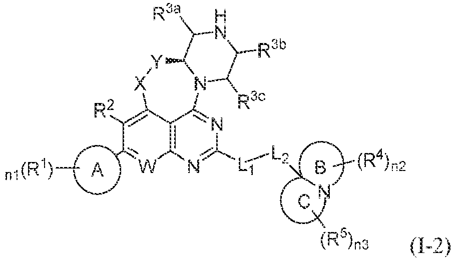 in each formula, X, Y, W, R¹, R², R^{3a}, R^{3b}, R^{3c}, R⁴, R⁵, n1, n2, n3, L₁, L₂, ring A, ring B, ring C are each defined as formula (I).

In an embodiment, the present disclosure provides a compound, or a pharmaceutically acceptable salt, a stereoisomer or a solvate thereof shown in formula (II-1) or formula (II-2): 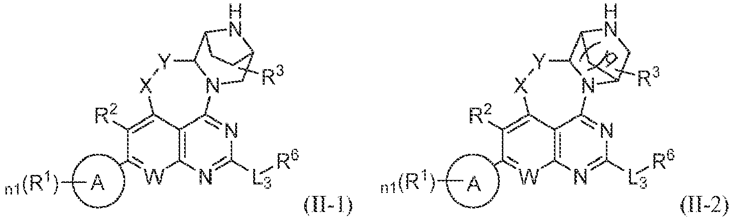 in each formula,
q is 1 or 2;
X is O or NR¹¹; wherein R¹¹ is selected from H and C1-C6 alkyl;
Y is CR¹²R¹³, CR¹⁴R¹⁵CR¹⁶R¹⁷, C(O) or C(O)CR¹⁸R¹⁹; wherein R¹², R¹³, R¹⁴ and R¹⁵ are each independently selected from H, cyano, C1-C3 alkyl, C1-C3 deuterated alkyl and C1-C3 halogenated alkyl; R¹⁶, R¹⁷, R¹⁸, R¹⁹ are each independently selected from H, C1-C3 alkyl and halogen;
W is N or CR²⁰; wherein R²⁰ is H, cyano, C1-C6 alkyl, halogen, C1-C6 halogenated alkyl, C1-C6 alkoxy, cycloalkyl, cycloalkyl-O-, heterocyclyl or heterocyclyl-O-; wherein the cycloalkyl, the heterocyclyl are each independently substituted by halogen;
ring A is selected from the group: aryl, heteroaryl;
R¹ is a substituent at any position on ring A; each R¹ is respectively and independently selected from: C1-C6 alkyl, halogen, hydroxyl, C1-C6 alkoxy, C1-C6 halogenated alkyl, C1-C6 halogenated alkoxy, cyano, NR²¹R²², C(O)NR²³R²⁴, CH₂R²⁵, N=S(O) (C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxy) C1-C6 halogenated alkyl- or cycloalkyl; wherein the cycloalkyl is optionally substituted by halogen or C1-C6 alkyl; wherein,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C6 alkyl or C1-C6 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; wherein R²⁵¹, R²⁵², R²⁵³, R²⁵⁴ are each independently H or C1-C6 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C6 alkyl, C1-C6 halogenated alkyl or NR²⁶¹R²⁶², wherein R²⁶¹, R²⁶² are each independently H or C1-C6 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxy, C1-C6 halogenated alkyl, C1-C6 halogenated alkoxy, C1-C6 deuterated alkoxy, NR³¹R³², C2-C4 alkynyl or CH₂OR³³; wherein R³¹, R³², R³³ are each independently hydrogen or C1-C6 alkyl;
R³ is a substituent at any position on a bridge ring, and is hydrogen, halogen, hydroxyl, cyano, C1-C3 alkyl, C1-C3 deuterated alkyl or C1-C3 halogenated alkyl;
L₃ is a bond, O or NR³⁴;
R⁶ is -L-6- to 10-membered fused heterocyclyl, -L-7- to 11-membered spiro heterocyclyl, -L-spirocyclic ring substituted 6- to 10-membered fused heterocyclyl, -L-spirocyclic ring substituted 7-to 11-membered spiro heterocyclyl, -L-fused ring substituted 6- to 10-membered fused heterocyclyl, -L-fused ring substituted 7- to 11-membered spiro heterocyclyl or -L-spirocyclic and fused ring substituted 6- to 10-membered fused heterocyclyl;
the spirocyclic ring substituted 6- to 10-membered fused heterocyclyl refers to that two hydrogen atoms on the same carbon atom of any one or two carbon atoms on the 6- to 10-membered fused heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 6- to 10-membered fused heterocyclyl;
the spirocyclic ring substituted 7- to 11-membered spiro heterocyclyl refers to that two hydrogen atoms on the same carbon atom of any one or two carbon atoms on the 7- to 11-membered spiro heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 7- to 11-membered spiro heterocyclyl;
the fused ring substituted 6- to 10-membered fused heterocyclyl refers to that one hydrogen atom on each carbon atom on the same carbon atom pair of any one or two pairs of adjacent carbon atoms on the 6- to 10-membered fused heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 6- to 10-membered fused heterocyclyl; or the fused ring substituted 6- to 10-membered fused heterocyclyl refers to that hydrogen atoms on any two non-adjacent carbon atoms on the 6- to 10-membered fused heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 6- to 10-membered fused heterocyclyl;
the fused ring substituted 7- to 11-membered spiro heterocyclyl refers to that one hydrogen atom on each carbon atom on the same carbon atom pair of any one or two pairs of adjacent carbon atoms on the 7- to 11-membered spiro heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 7- to 11-membered spiro heterocyclyl; or the fused ring substituted 7- to 11-membered spiro heterocyclyl refers to that hydrogen atoms on any two non-adjacent carbon atoms on the 7- to 11-membered spiro heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 7- to 11-membered spiro heterocyclyl;
the spirocyclic and fused ring substituted 6- to 10-membered fused heterocyclyl refers to that one hydrogen atom on each carbon atom on any pair of adjacent carbon atoms on the 6- to 10-membered fused heterocyclyl is simultaneously substituted by -(CH₂)ₘ₄-, -(CH₂)ₘ₅O(CH₂)ₘ₆- or - (CH₂)ₘ₇NRⁿ(CH₂)ₘ₈ to form paracyclic substituent, and two hydrogen atoms on the other carbon atom on the 6- to 10-membered fused heterocyclyl are simultaneously substituted by -(CH₂)ₘ₄-, - (CH₂)ₘ₅O(CH₂)ₘ₆- or -(CH₂)ₘ₇NRⁿ(CH₂)ₘ₈ to form spirocyclic ring substituent, thereby to form spirocyclic and fused ring substituted 6- to 10-membered fused heterocyclyl;
the 6- to 10-membered fused heterocycly or the 7- to 11-membered spiro heterocyclyl containing 1, 2, 3 or 4 heteroatoms selected from N(R^{m}), S, S(=O), S(=O)₂, O as ring atoms;
the spirocyclic ring substituted 6- to 10-membered fused heterocyclyl, the spirocyclic ring substituted 7- to 11-membered spiro heterocyclyl, the fused ring substituted 6- to 10-membered fused heterocyclyl, the fused ring substituted 7- to 11-membered spiro heterocyclyl or the spirocyclic and fused ring substituted 6- to 10-membered fused heterocyclyl each independently containing 1, 2, 3, 4 or 5 heteroatoms selected from N(R^{m}), S, S(=O), S(=O)₂, O as ring atoms;
Rⁿ is hydrogen, C1-C4 alkyl, deuterated C1-C4 alkyl, C1-C4 halogenated alkyl, C3-C20 cycloalkyl or 3- to 20-membered heterocyclyl;
R^{m} is absent, hydrogen, C1-C4 alkyl, deuterated C1-C4 alkyl, C1-C4 halogenated alkyl, C3-C20 cycloalkyl or 3- to 20-membered heterocyclyl;
wherein the 6- to 10-membered fused heterocyclyl or the 7- to 11-membered spiro heterocyclyl is saturated or partially unsaturated; when the 6- to 10-membered fused heterocyclyl or the 7- to 11-membered spiro heterocyclyl is partially unsaturated, the ring containing 1 or 2 double bonds; the 6- to 10-membered fused heterocyclyl or two hydrogen atoms on the same carbon atom of any one or two carbon atoms on the 7- to 11-membered spiro heterocyclyl are optionally and simultaneously substituted by =CR^{2a}R^{2b}; the 6- to 10-membered fused heterocyclyl or the 7- to 11-membered spiro heterocyclyl is also optionally substituted by one or more (e.g., 1, 2, 3 4 or 5) R^{6a};
wherein R^{2a}, R^{2b} are each independently hydrogen, halogen, cyano, C1-C4 alkyl, C1-C4 halogenated alkyl, C6-C10 aryl or 5- or 6-membered heteroaryl; the C6-C10 aryl or 5- or 6-membered heteroaryl is optionally substituted by 1, 2, 3 groups selected from halogen, cyano, C1-C4 alkyl, C1-C4 alkoxy, C1-C4 halogenated alkyl, C1-C4 halogenated alkoxy; or each pair of R^{2a}, R^{2b} independently with the carbon atom to which R^{2a}, R^{2b} are attached together form a C3-C6 monocyclic cycloalkyl or a 3-to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl;
each R^{6a} is respectively and independently halogen, hydroxyl, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, cyano, -Q-phenyl, -Q-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})₂, (C1-C3 alkoxy)C1-C3 alkyl, (C1-C3 alkyl)C(O)-, oxo, (C1-C3 halogenated alkyl)C(O)-, -SO₂F, (C1-C3 alkoxy)Cl-C3 alkoxy, -CH₂OC(O)N(R^{6b})₂, -CH₂NHC(O)OC1-C6 alkyl, -CH₂NHC(O)N(R^{6b})₂, - CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 alkyl)O(C1-C3 alkyl)phenyl(Cl-C3 alkyl)N(CH₃)₂, -UC(U)NH(C1-C3 alkyl)O(Cl-C3 alkyl)phenyl, -OC(O)heterocyclyl, -OC(O)C1-C6 alkyl, -OC(O)C1-C6 halogenated alkyl, -C1-C3 alkyl-OC(O)C1-C6 alkyl, -C1-C3 alkyl-OC(O)Cl-C6 halogenated alkyl, -OC(O)phenyl, -C1-C3 alkyl-OC(O)phenyl or -CH₂ heterocyclyl; wherein the phenyl in the group above is optionally substituted by -C(O)H or OH; the heterocyclyl in the -C1-C3 alkyl-heterocyclyl is optionally substituted by oxo; Q is a bond or O; each R^{6b} above is independently hydrogen, C1-C6 alkyl, C1-C6 deuterated alkyl or C1-C6 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy;
each m4 is 2, 3 or 4;
each m5, m6, m7, m8 are each independently 0, 1, 2 or 3; m5 and m6 are not simultaneously 0; m7 and m8 are not simultaneously 0;
   or
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH or -L-C(O)OC1-C6 alkyl; wherein the heterocyclyl, the aryl in the -L-NR³⁴C(O)-aryl, the heterocyclyl in the -L-heterocyclyl, the cycloalkyl in the -L-cycloalkyl each optionally substituted by one or more (e.g., 1, 2, 3, 4 or 5) R³⁵ or two hydrogen atoms on the same carbon atom are optionally and simultaneously substituted by - CH₂CH₂- or -CH₂CH₂CH₂- to form cyclopropyl or cyclobutyl; the aryl in the -L-aryl, the heteroaryl in the -L-heteroaryl each optionally substituted by one or more (e.g., 1, 2, 3, 4 or 5) R³⁶;
wherein each L is independently a bond, C1-C4 alkylene or heteroaryl; wherein one or two hydrogen atoms on any carbon atom or on any same carbon atom of the C1-C4 alkylene are independently and optionally substituted by deuterium, C1-C6 alkyl or C1-C6 halogenated alkyl; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, -(CH₂)ₘ₁-NR⁹-(CH₂)ₘ₂- to form a cyclic substituent; m3 is 1 or 2; each m1 is respectively and independently 0, 1, 2, or 3; each m2 is respectively and independently 0, 1, 2, or 3; and m1, m2 are not 0 simultaneously;
R⁹ is hydrogen, C1-C6 alkyl, C1-C6 deuterated alkyl, C1-C6 halogenated alkyl, C1-C6 alkyl-hydroxyl, C1-C6 alkyl-cyano, C1-C6 alkoxy, C1-C6 alkyl-C1-C6 alkoxy, C3-C6 monocyclic cycloalkyl, C3-C6 deuterated cycloalkyl or C3-C6 halogenated cycloalkyl;
each R³⁵ is respectively and independently halogen, hydroxyl, C1-C3 alkyl, C1-C3 halogenated alkyl, C1-C3 alkoxy, C3-C6 cycloalkyl, -C1-C3 alkyl-C1-C3 alkoxy, -C1-C3 alkyl-hydroxyl, heterocyclyl(e.g., 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy)C1-C3 alkyl, (C1-C3 alkyl)C(O), oxo, (C1-C3 halogenated alkyl)C(O)-, -SO₂F, (C1-C3 alkoxy)C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), - CH₂NHSO₂C1-C6 alkyl, -CH₂OC(O)heterocyclyl, -OC(O)N(R³⁴)₂, -OC(O)NH(C1-C3 alkyl)U(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl(e.g., -OC(O)-3- to 6-membered heterocyclyl) or -CH₂ heterocyclyl(e.g., -CH₂-3- to 6-membered heterocyclyl); wherein, the phenyl in - NHC(O)phenyl or -OC(O)NH(C1-C3 alkyl)O(C1-C3 alkyl)phenyl is optionally substituted by - C(O)H or OH; the heterocyclyl in the -CH₂ heterocyclyl is optionally substituted by oxo; the hydrogen atom on -C1-C3 alkyl- is substituted by 1 or 2 groups selected from halogen, deuterium, C1-C3 alkyl, C1-C3 halogenated alkyl;
each R³⁶ is respectively and independently halogen, hydroxyl, HC(O)-, C1-C4 alkyl, C1-C4 alkoxy, C1-C4 halogenated alkyl, C1-C4 hydroxyalkyl or -N(R³⁴)₂;
each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, the compound is shown in formula (II-1A), formula (II-1B), formula (II-2A), formula (II-2B): 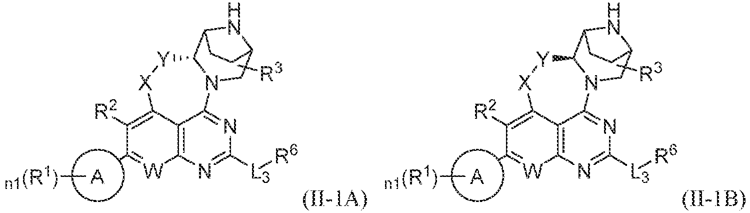 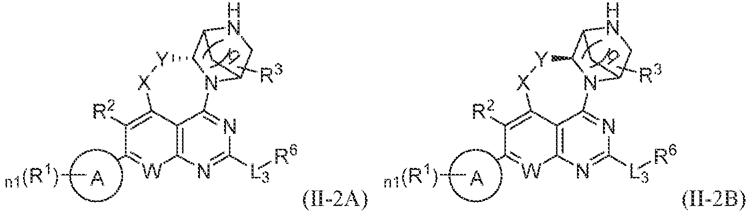 in each formula, X, Y, W, R¹, R², R³, R⁶, n1, L₃, q, ring A are each defined as formula (II-1) or formula (II-2).

In an embodiment, the compound is shown in formula (II-1A1), formula (II-1A2), formula (II-1B1), formula (II-1B2), formula (II-1C1) or formula (II-1C2): 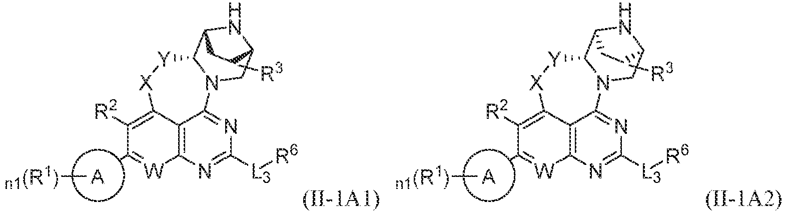 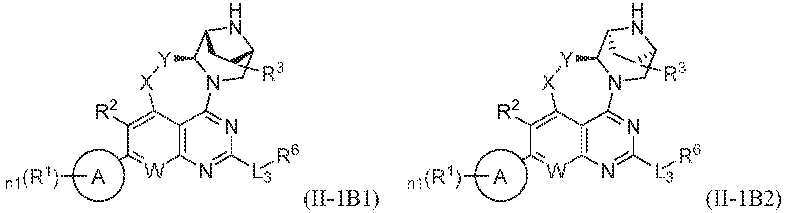 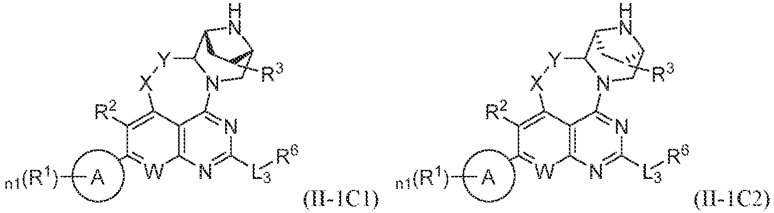 in each formula, X, Y, W, R¹, R², R³, R⁶, n1, L₃, ring A are each defined as formula (II-1) or formula (II-2).

In an embodiment, the present disclosure provides a compound, or a pharmaceutically acceptable salt, a stereoisomer or a solvate thereof shown in formula (III): 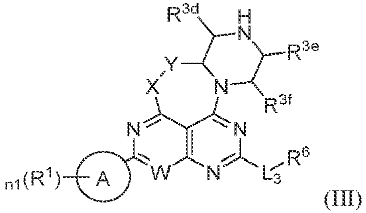 in the formula,
X is O or NR¹¹; wherein R¹¹ is selected from H and C1-C6 alkyl;
Y is CR¹²R¹³, CR¹⁴R¹⁵CR¹⁶R¹⁷, C(O) or C(O)CR¹⁸R¹⁹; wherein R¹², R¹³, R¹⁴ and R¹⁵ are each independently selected from H, cyano, C1-C3 alkyl, C1-C3 deuterated alkyl and C1-C3 halogenated alkyl; R¹⁶, R¹⁷, R¹⁸, R¹⁹ are each independently selected from H, C1-C3 alkyl and halogen;
W is N or CR²⁰; wherein R²⁰ is H, cyano, C1-C6 alkyl, halogen, C1-C6 halogenated alkyl, C1-C6 alkoxy, cycloalkyl, cycloalkyl-O-, heterocyclyl or heterocyclyl-O-; wherein the cycloalkyl, the heterocyclyl are each independently substituted by halogen;
ring A is selected from the group: aryl, heteroaryl;
R¹ is a substituent at any position on ring A; C1-C6 alkyl, halogen, hydroxyl, C1-C6 alkoxy, C1-C6 halogenated alkyl, C1-C6 halogenated alkoxy, cyano, NR²¹R²², C(O)NR²³R²⁴, CH₂R²⁵, N=S(O) (C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, - CH₂C(O)NR²⁷R²⁸, -C2-C6 alkynyl NR²⁹R³⁰, C2-C6 deuterated alkynyl, (C1-C6 alkoxy) C1-C6 halogenated alkyl- or cycloalkyl; wherein the cycloalkyl is optionally substituted by halogen or C1-C6 alkyl; wherein,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C6 alkyl or C1-C6 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; wherein R²⁵¹, R²⁵², R²⁵³, R²⁵⁴ are each independently H or C1-C6 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C6 alkyl, C1-C6 halogenated alkyl or NR²⁶¹R²⁶², wherein R²⁶¹, R²⁶² are each independently H or C1-C6 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
n1 is 0, 1, 2 or 3;
R ^{3d}, R^{3e}, R^{3f} are each independently hydrogen, halogen, hydroxyl, cyano, C1-C3 alkyl, C1-C3 deuterated alkyl or C1-C3 halogenated alkyl; or
R^{3d} and R^{3e} are connected to form -CHR^{3d1} - or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3d1}, R^{3d2}, R^{3a3} are each independently hydrogen, halogen, hydroxyl, cyano, C1-C3 alkyl, C1-C3 deuterated alkyl or C1-C3 halogenated alkyl; or
R^{3d} and R^{3f} are connected to form -CHR^{3f1}- or -CHR^{3f2}CHR^{3f3}-; R^{3e} is hydrogen, halogen, hydroxyl, cyano, C1-C3 alkyl, C1-C3 deuterated alkyl or C1-C3 halogenated alkyl; R^{3f1}, R^{3f2}, R^{3f3} are each independently hydrogen, halogen, hydroxyl, cyano, C1-C3 alkyl, C1-C3 deuterated alkyl or C1-C3 halogenated alkyl;
L₃ is a bond, O or NR³⁴;
R₆ is -L-6- to 10-membered fused heterocyclyl, -L-7- to 11-membered spiro heterocyclyl, -L-spirocyclic ring substituted 6- to 10-membered fused heterocyclyl, -L-spirocyclic ring substituted 7-to 11-membered spiro heterocyclyl, -L-fused ring substituted 6- to 10-membered fused heterocyclyl, -L-fused ring substituted 7- to 11-membered spiro heterocyclyl or -L-spirocyclic and fused ring substituted 6- to 10-membered fused heterocyclyl;
the spirocyclic ring substituted 6- to 10-membered fused heterocyclyl refers to that two hydrogen atoms on the same carbon atom of any one or two carbon atoms on the 6- to 10-membered fused heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 6- to 10-membered fused heterocyclyl;
the spirocyclic ring substituted 7- to 11-membered spiro heterocyclyl refers to that two hydrogen atoms on the same carbon atom of any one or two carbon atoms on the 7- to 11-membered spiro heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 7- to 11-membered spiro heterocyclyl;
the fused ring substituted 6- to 10-membered fused heterocyclyl refers to that one hydrogen atom on each carbon atom on the same carbon atom pair of any one or two pairs of adjacent carbon atoms on the 6- to 10-membered fused heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 6- to 10-membered fused heterocyclyl; or the fused ring substituted 6- to 10-membered fused heterocyclyl refers to that hydrogen atoms on any two non-adjacent carbon atoms on the 6- to 10-membered fused heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 6- to 10-membered fused heterocyclyl;
the fused ring substituted 7- to 11-membered spiro heterocyclyl refers to that one hydrogen atom on each carbon atom on the same carbon atom pair of any one or two pairs of adjacent carbon atoms on the 7- to 11-membered spiro heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 7- to 11-membered spiro heterocyclyl; or the fused ring substituted 7- to 11-membered spiro heterocyclyl refers to that hydrogen atoms on any two non-adjacent carbon atoms on the 7- to 11 -membered spiro heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 7- to 11-membered spiro heterocyclyl;
the spirocyclic and fused ring substituted 6- to 10-membered fused heterocyclyl refers to that one hydrogen atom on each carbon atom on any pair of adjacent carbon atoms on the 6- to 10-membered fused heterocyclyl is simultaneously substituted by -(CH₂)ₘ₄-, -(CH₂)ₘ₅O(CH2)ₘ₆- or - (CH₂)ₘ₇NRⁿ(CH₂)ₘ₈ to form paracyclic substituent, and two hydrogen atoms on the other carbon atom on the 6- to 10-membered fused heterocyclyl are simultaneously substituted by -(CH₂)ₘ₄-, - (CH₂)ₘ₅O(CH₂)ₘ₆- or -(CH₂)ₘ₇NRⁿ(CH₂)ₘ₈ to form spirocyclic ring substituent, thereby to form spirocyclic and fused ring substituted 6- to 10-membered fused heterocyclyl;
the 6- to 10-membered fused heterocycly or the 7- to 11-membered spiro heterocyclyl containing 1, 2, 3 or 4 heteroatoms selected from N(R^{m}), S, S(=O), S(=O)₂, O as ring atoms;
the spirocyclic ring substituted 6- to 10-membered fused heterocyclyl, the spirocyclic ring substituted 7- to 11-membered spiro heterocyclyl, the fused ring substituted 6- to 10-membered fused heterocyclyl, the fused ring substituted 7-to 11-membered spiro heterocyclyl or the spirocyclic and fused ring substituted 6- to 10-membered fused heterocyclyl each independently containing 1, 2, 3, 4 or 5 heteroatoms selected from N(R^{m}), S, S(=O), S(=O)₂, O as ring atoms;
Rⁿ is hydrogen, C1-C4 alkyl, deuterated C1-C4 alkyl, C1-C4 halogenated alkyl, C3-C20 cycloalkyl or 3- to 20-membered heterocyclyl;
R^{m} is absent, hydrogen, C1-C4 alkyl, deuterated C1-C4 alkyl, C1-C4 halogenated alkyl, C3-C20 cycloalkyl or 3- to 20-membered heterocyclyl;
wherein the 6- to 10-membered fused heterocyclyl or the 7- to 11-membered spiro heterocyclyl is saturated or partially unsaturated; when the 6- to 10-membered fused heterocyclyl or the 7- to 11-membered spiro heterocyclyl is partially unsaturated, the ring containing 1 or 2 double bonds; the 6- to 10-membered fused heterocyclyl or two hydrogen atoms on the same carbon atom of any one or two carbon atoms on the 7- to 11-membered spiro heterocyclyl are optionally and simultaneously substituted by =CR^{2a}R^{2b}; the 6- to 10-membered fused heterocyclyl or the 7- to 11-membered spiro heterocyclyl is also optionally substituted by one or more (e.g., 1, 2, 3 4 or 5) R^{6a};
wherein R^{2a}, R^{2b} are each independently hydrogen, halogen, cyano, C1-C4 alkyl, C1-C4 halogenated alkyl, C6-C10 aryl or 5- or 6-membered heteroaryl; the C6-C10 aryl or 5- or 6-membered heteroaryl is optionally substituted by 1, 2, 3 groups selected from halogen, cyano, C1-C4 alkyl, C1-C4 alkoxy, C1-C4 halogenated alkyl, C1-C4 halogenated alkoxy; or each pair of R^{2a}, R^{2b} independently with the carbon atom to which R²³, R^{2b} are attached together form a C3-C6 monocyclic cycloalkyl or a 3-to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl;
each R^{6a} is respectively and independently halogen, hydroxyl, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, cyano, -Q-phenyl, -Q-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})₂, (C1-C3 alkoxy)C1-C3 alkyl, (C1-C3 alkyl)C(O)-, oxo, (C1-C3 halogenated alkyl)C(O)-, -SO₂F, (C1-C3 allcoxy)C1-C3 alkoxy, -CH₂OC(O)N(R^{6b})₂, -CH₂NHC(O)OC1-C6 alkyl, -CH₂NHC(O)N(R^{6b})₂, - CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O)C1-C6 alkyl, -OC(O)C1-C6 halogenated alkyl, -C1-C3 alkyl-OC(U)C1-C6 alkyl, -C1-C3 alkyl-OC(O)C1-C6 halogenated alkyl, -OC(O)phenyl, -C1-C3 alkyl-OC(O)phenyl or -CH₂ heterocyclyl; wherein the phenyl in the group above is optionally substituted by -C(O)H or OH; the heterocyclyl in the -C1-C3 alkyl-heterocyclyl is optionally substituted by oxo; Q is a bond or O; each R^{6b} above is independently hydrogen, C1-C6 alkyl, C1-C6 deuterated alkyl or C1-C6 halogenated alkyl; or two R^{6b} each independently are taken together with the nitrogen atom to which R^{6b} are attached, form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy;
each m4 is 2, 3 or 4;
each m5, m6, m7, m8 are each independently 0, 1, 2 or 3; m5 and m6 are not simultaneously 0; m7 and m8 are not simultaneously 0;
   or
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH or -L-C(O)OC1-C6 alkyl; wherein the heterocyclyl, the aryl in the -L-NR³⁴C(O)-aryl, the heterocyclyl in the -L-heterocyclyl, the cycloalkyl in the -L-cycloalkyl each optionally substituted by one or more (e.g., 1, 2, 3 4 or 5) R³⁵ or two hydrogen atoms on the same carbon atom are optionally and simultaneously substituted by - CH₂CH₂- or -CH₂CH₂CH₂- to form cyclopropyl or cyclobutyl; the aryl in the -L-aryl, the heteroaryl in the -L-heteroaryl each optionally substituted by one or more (e.g., 1, 2, 3 4 or 5) R³⁶;
wherein each L is independently a bond, C1-C4 alkylene or heteroaryl; wherein one or two hydrogen atoms on any carbon atom or on any same carbon atom of the C1-C4 alkylene are independently and optionally substituted by deuterium, C1-C6 alkyl or C1-C6 halogenated alkyl; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, -(CH₂)ₘ₁-NR⁹-(CH₂)ₘ₂- to form a cyclic substituent; m3 is 1 or 2; each m1 is respectively and independently 0, 1, 2, or 3; each m2 is respectively and independently 0, 1, 2, or 3; and m1, m2 are not 0 simultaneously;
R⁹ is hydrogen, C1-C6 alkyl, C1-C6 deuterated alkyl, C1-C6 halogenated alkyl, C1-C6 alkyl-hydroxyl, C1-C6 alkyl-cyano, C1-C6 alkoxy, C1-C6 alkyl-C1-C6 alkoxy, C3-C6 monocyclic cycloalkyl, C3-C6 deuterated cycloalkyl or C3-C6 halogenated cycloalkyl;
each R³⁵ is respectively and independently halogen, hydroxyl, C1-C3 alkyl, C1-C3 halogenated alkyl, C1-C3 alkoxy, C3-C6 cycloalkyl, -C1-C3 alkyl-C1-C3 alkoxy, -C1-C3 alkyl-hydroxyl, heterocyclyl(e.g., 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy)C1-C3 alkyl, (C1-C3 alkyl)C(O), oxo, (C1-C3 halogenated alkyl)C(O)-, -SO₂F, (C1-C3 alkoxy)C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), - CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(U)heterocyclyl(e.g., -OC(O)-3- to 6-membered heterocyclyl) or -CH₂ heterocyclyl (e.g., -CH₂-3- to 6-membered heterocyclyl); wherein, the phenyl in - NHC(O)phenyl or -OC(O)NH(C1-C3 alkyl)U(C1-C3 alkyl)phenyl is optionally substituted by - C(O)H or OH; the heterocyclyl in the -CH₂ heterocyclyl is optionally substituted by oxo; the hydrogen atom on -C1-C3 alkyl- is substituted by 1 or 2 groups selected from halogen, deuterium, C1-C3 alkyl, C1-C3 halogenated alkyl;
each R³⁶ is respectively and independently halogen, hydroxyl, HC(O)-, C1-C4 alkyl, C1-C4 alkoxy, C1-C4 halogenated alkyl, C1-C4 hydroxyalkyl or -N(R³⁴)₂;
each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, the compound is shown in formula (III-1) or formula (III-2): 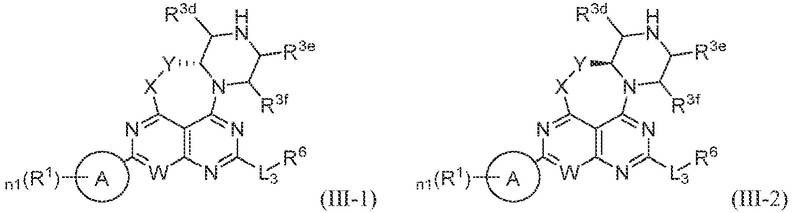 in each formula, X, Y, W, R¹, R^{3d}, R^{3e}, R^{3f}, R⁶, n1, L₃, ring A are each defined as formula (III).

In an embodiment, the compound is shown in formula (III-1a), formula (III-1b), formula (III-2a) or formula (III-2b): 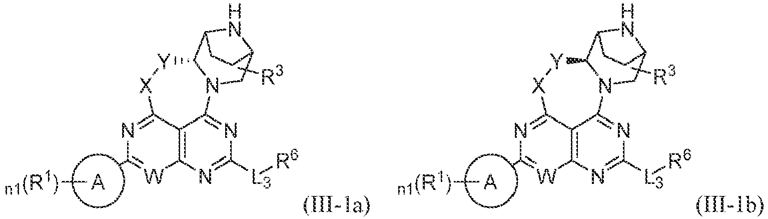 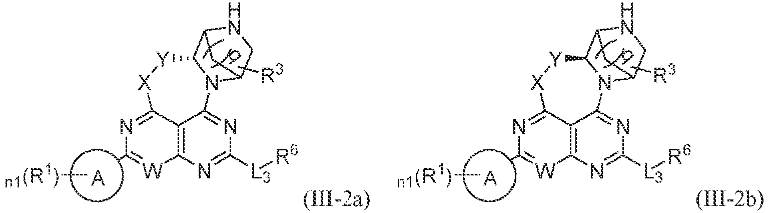 in each formula, q is 1 or 2; R³ is a substituent at any position on a bridge ring, and is hydrogen, halogen, hydroxyl, cyano, C1-C3 alkyl, C1-C3 deuterated alkyl or C1-C3 halogenated alkyl; X, Y, W, ring A, R¹, n1, L₃, R⁶ are defined as formula (III-1) or formula (III-2).

In an embodiment, the compound is shown in formula (III-1a1), formula (III-1a2), formula (III-1b1), formula (III-1b2), formula (III-1c1) or formula (III-1c2): 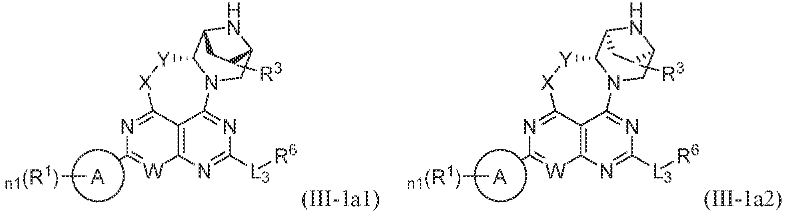 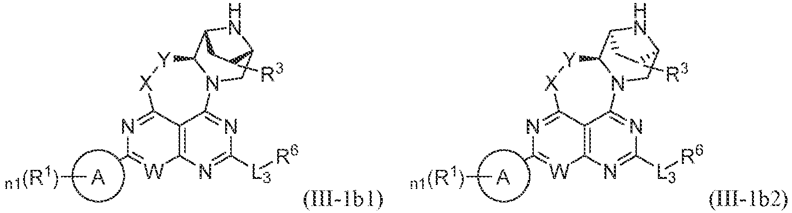 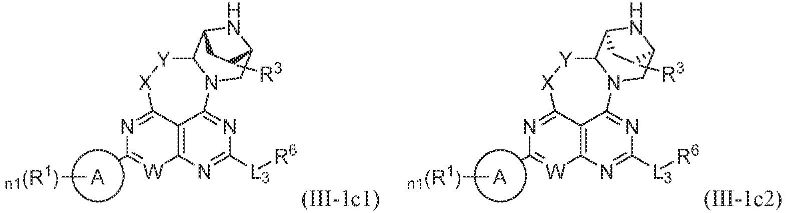 in each formula, R³ is a substituent at any position on a bridge ring, and is hydrogen, halogen, hydroxyl, cyano, C1-C3 alkyl, C1-C3 deuterated alkyl or C1-C3 halogenated alkyl; X, Y, W, ring A, R¹, n1, L₃, R⁶ are as defined in formula (III-1) or formula (III-2).

In an embodiment, R³ is hydrogen.

In an embodiment, Y is -CH₂-.

In an embodiment, the compound is shown in formula (III-1a3), formula (III-1a4), formula (III-1b3) or formula (III-1b4): 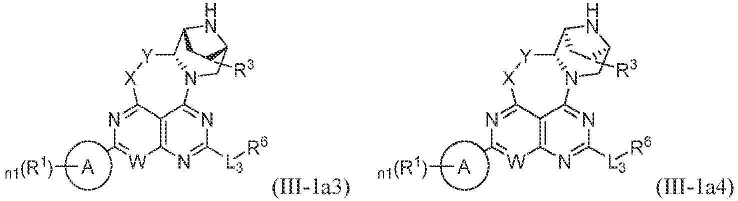 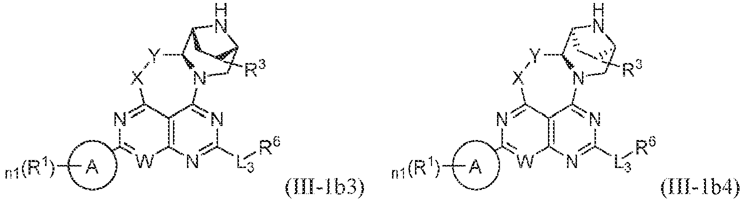 in each formula,
R³ is a substituent at any position on a bridge ring, and is hydrogen, halogen, hydroxyl, cyano, C1-C3 alkyl, C1-C3 deuterated alkyl or C1-C3 halogenated alkyl;
Y is CR¹²R¹³, CR¹⁴R¹⁵CR¹⁶R¹⁷, C(O) or C(O)CR⁸R¹⁹; wherein
R¹², R¹³ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 allcyl-C1-C6 halogenated alkoxy, -C1-C4 alkyl-hydroxyl, -C1-C4 alkyl-cyano, -C1-C4 alkyl-NR¹²¹R¹²²; 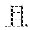 R¹², R¹³ cannot be H simultaneously; or R¹², R¹³ with the carbon atoms to which R¹², R¹³ are attached to form a cycloalkyl;
R¹⁴ and R¹⁵ are each independently selected from H, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; 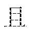 R¹⁴ , R¹⁵ cannot be H simultaneously; or R¹⁴, R¹⁵ with the carbon atoms to which R¹², R¹³ are attached to form a cycloalkyl;
R¹⁶, R¹⁷ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; 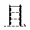 R¹⁶, R¹⁷ cannot be H simultaneously;
R¹⁸, R¹⁹ are each independently selected from H, C1-C3 alkyl and halogen;
R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl;
wherein the cycloalkyl, the heterocyclyl, the aryl, the heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy;
X, W, ring A, R¹, n1, L₃, R⁶ are as defined in formula (III-1) or formula (III-2).

In an embodiment, R³ is hydrogen.

In an embodiment, Y is CR¹²R¹³; wherein R¹² is selected from halogen, hydroxyl, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, -C1-C4 alkyl-C1-C6 alkoxy, -C1-C4 alkyl-Cl-C6 halogenated alkoxy, -C1-C4 alkyl-hydroxyl, -C1-C4 alkyl-cyano, -C1-C4 alkyl-NR¹²¹R¹²²; R¹³ is H; R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl; or R¹², R¹³ with the carbon atoms to which R¹², R¹³ are attached to form a cycloalkyl; wherein the cycloalkyl, the heterocyclyl, the aryl, the heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy.

In an embodiment, in formula (III-1a3), formula (III-1a4), formula (III-1b3) or formula (III-1b4), Y is CR¹²R¹³; wherein R¹² is selected from halogen, hydroxyl, cyano, methyl, ethyl, n-propyl, isopropyl, trideuterated methyl, trifluoromethyl, cyclopropyl, -C1-C4 alkyl-methoxy, -C1-C4 alkyl-hydroxyl, -C1-C4 alkyl-cyano; R¹³ is H; or R¹², R¹³ are taken together with the nitrogen atom to which R¹², R¹³ are attached to form a cyclopropyl.

In an embodiment, in formula (III-1a3), formula (III-1a4), formula (III-1b3) or formula (III-1b4), Y is -CH(CH₃)-, -CH(CH₂CH₃)-, -CH(CH₂CH₂CH₃)-, -CH(CH(CH₃)₂)-, -CH(cyclopropyl)-, - CH(CH₂OH)-, -CH(CH₂CH₂OH)-, -CH(CH₂CN)-, -CH(CH₂CH₂CN)-, -CH(CH₂CF₃)-, - CH(CH₂CF₂H)-, -CH(CH₂CH₂F)-, -CH(CH₂F)-, -CH(CF₂H)-, -CH(CF₃)-, -CH(CN)-, - CH(CH₂OCH₃)-, , -C(CH₃)₂-, -CH(CD₃)-.

In an embodiment, the cycloalkyl is C3-C20 cycloalkyl. Preferably, the cycloalkyl is C3-C12 cycloalkyl (more preferably, C3-C8 monocyclic cycloalkyl), C5-C20 spiro cycloalkyl, C5-C20 fused cycloalkyl or C5-C20 bridged cycloalkyl.

In an embodiment, the heterocyclyl is 3- to 20-membered heterocyclyl. Preferably, the heterocyclyl is monocyclic heterocyclyl (e.g. 3- to 8-membered monocyclic heterocyclyl), 5- to 20-membered spiro heterocyclyl, 5- to 20-membered fused heterocyclyl, and 5- to 20-membered bridged heterocyclyl.

In an embodiment, the aryl is C6-C14 aryl. Preferably, the aryl is monocyclic aryl, non-fused polycyclic aryl, and aromatic fused polycyclic aryl.

In an embodiment, the heteroaryl is 5- to 14-membered heteroaryl. Preferably, the heteroaryl is monocyclic heteroaryl (e.g. 5- or 6-membered monocyclic heteroaryl), fused bicyclic heteroaryl (e.g. 8- to 10-membered bicyclic heteroaryl), or fused tricyclic heteroaryl.

In an embodiment, W is N or CR²⁰; wherein R²⁰ is H, cyano, C1-C3 alkyl, halogen, C1-C3 halogenated alkyl, C1-C3 alkoxy, C3-C6 monocyclic cycloalkyl, C3-C6 monocyclic cycloalkyl-O-, 3- to 6-membered heterocyclyl or 3- to 6-membered heterocyclyl-O-; wherein the C3-C6 monocyclic cycloalkyl, the 3- to 6-membered heterocyclyl are independently and optionally substituted by halogen.

In an embodiment, W is N or CR²⁰; wherein R²⁰ is hydrogen, fluorine, methyl, methoxy or cyclopropoxy.

In an embodiment, W is N or CR²⁰; wherein R²⁰ is H, fluorine, chlorine, methyl, cyclopropyl, methoxy or cyclopropoxy.

In an embodiment, X is -N(CH₃)- or -N(cyclopropyl)-.

In an embodiment, X is -N(CD₃)-.

In an embodiment, X is O.

In an embodiment, Y is CR¹²R¹³ or CR¹⁴R¹⁵CR¹⁶R¹⁷; wherein R¹², R¹³, R¹⁴ and R¹⁵ are each independently selected from H, cyano, C1-C6 alkyl, C1-C6 halogenated alkyl, C1-C6 alkoxy, C1-C6 halogenated alkoxy, C3-C6 monocyclic cycloalkyl, C3-C6 monocyclic cycloalkyl-O-, 3- to 6-membered heterocyclyl, 3- to 6-membered heterocyclyl-O-, phenyl, 5- or 6-membered heteroaryl, phenyl-O-, 5- or 6-membered heteroaryl-O-, -C1-C2 alkyl-C1-C3 alkoxy, -C1-C2 alkyl-Cl-C3 halogenated alkoxy, -C1-C2 alkyl-hydroxyl, -C1-C2 alkyl-cyano, -C1-C2 alkyl-NR¹²¹R¹²²; R¹⁶, R¹⁷ are each independently selected from H; R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl; wherein the C3-C6 monocyclic cycloalkyl, the 3- to 6-membered heterocyclyl, the phenyl, the 5- or 6-membered heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy.

In an embodiment, Y is -CH₂-, -CH₂CH₂-, -CH(CH₂OCH₃)-, -CH(CN)-, -CH(OH)-, -CH(CH₃)-, - CH(CD₃)-, -C(CH₃)₂-, -CH(CF₃)-, -CH(CHF₂)-, -CH(CH₂F)-, -CH(CH₂CH₃)-, -CH(CH₂CH2F)-, - CH(CH₂CF₂H)-, -CH(CH₂CF₃)-, -CH(CH₂CH₂CN)-, -CH(CH₂OH)-, -CH(CH₂CN)-, - CH(CH₂CH₂OH)-, -CH(cyclopropyl)-, -CH(isopropyl)-, -CH(CH₂CH₂CH₃)-, - CH(CH₂CH₂CH₂CH₃)- or

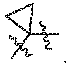

In an embodiment, Y is -CH₂-.

In an embodiment, Y is selected from the group:

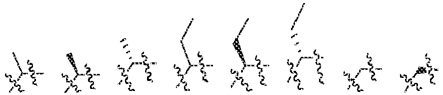

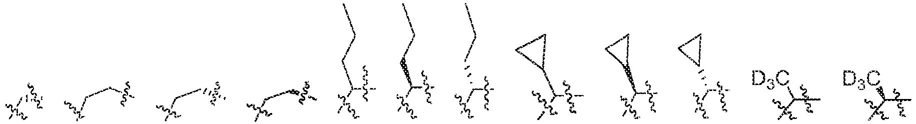

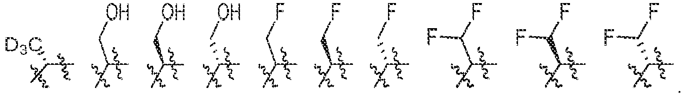

In an embodiment, R² is H, cyano, halogen, C1-C3 alkyl, C1-C3 alkoxy, C1-C3 halogenated alkyl, C1-C3 halogenated alkoxy, C1-C3 deuterated alkoxy, NR³¹R³², C2-C4 alkynyl or CH₂OR³³; wherein R³¹, R³² are each independently hydrogen or C1-C6 alkyl; or R³¹, R³² are taken together with the nitrogen atom to which R³¹, R³² are attached to form a 3- to 6-membered nitrogen-containing heterocyclyl; R¹³ is hydrogen or C1-C6 alkyl.

In an embodiment, R² is fluorine, chlorine, hydroxyl, methoxy or cyano.

In an embodiment, R² is hydrogen, fluorine, chlorine, hydroxyl, methoxy or cyano.

In an embodiment, ring A is phenyl, naphthyl, pyridyl, pyrimidinyl, pyrazinyl, pyridazinyl, pyrrolyl, pyrazolyl, imidazolyl, 1,2,3,4-tetrahydronaphthyl, 2,3-dihydro-1H-indenyl, quinolyl, isoquinolyl, quinazolinyl, indolyl, indazolyl or benzo[d][1,3]dioxolane.

In an embodiment, ring A is selected from the group: 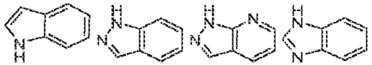 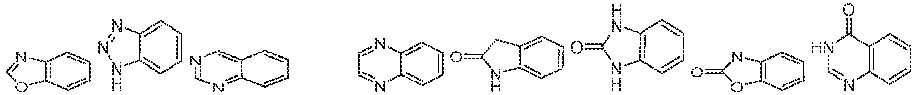 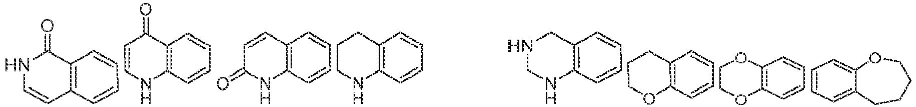 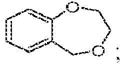 and these groups attached to the rest of the molecule through any suitable ring atom.

In an embodiment, R¹ is a substituent at any position on ring A; n1 is 0, 1, 2 or 3; each R¹ is respectively and independently selected from: C1-C3 alkyl, halogen, hydroxyl, C1-C3 alkoxy, C1-C3 halogenated alkyl, C1-C3 halogenated alkoxy, cyano, NR²¹R²², C(O)NR²³R²⁴, CH₂R²⁵, N=S(O) (C1-C3 alkyl)₂, S(O)C1-C3 alkyl, S(O)₂R²⁶, -S-C1-C3 alkyl, C2-C4 alkenyl, C2-C4 alkynyl, C2-C4 hydroxyalkynyl, C1-C3 cyanoalkyl, triazolyl, -S-C1-C3 halogenated alkyl, C1-C3 hydroxyalkyl, - CH₂C(O)NR²⁷R²⁸, -C2-C4 alkynyl NR²⁹R³⁰, C2-C4 deuterated alkynyl, (C1-C3 alkoxy) C1-C3 halogenated alkyl- or cycloalkyl; wherein the cycloalkyl is optionally substituted by halogen or C1-C3 alkyl; wherein,
R²¹ is H, C1-C3 alkyl, C1-C3 halogenated alkyl, C(O)C1-C3 alkyl or C(O)₂C1-C3 alkyl; R²² is H, C1-C3 alkyl or C1-C3 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C3 alkyl or C1-C3 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C3 alkyl; wherein R²⁵¹, R²⁵², R²⁵³, R²⁵⁴ are each independently H or C1-C3 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C3 alkyl, C1-C3 halogenated alkyl or NR²⁶¹R²⁶², wherein R²⁶¹, R²⁶² are each independently H or C1-C3 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C3 alkyl or C1-C3 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C3 alkyl or C1-C3 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;

In an embodiment, L₂ is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl.

In an embodiment, R^{3a}, R^{3b}, R^{3c} are each independently hydrogen, fluorine, methyl or methoxyl.

In an embodiment, 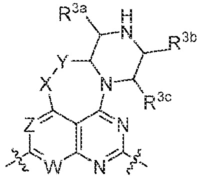 is selected from the group:

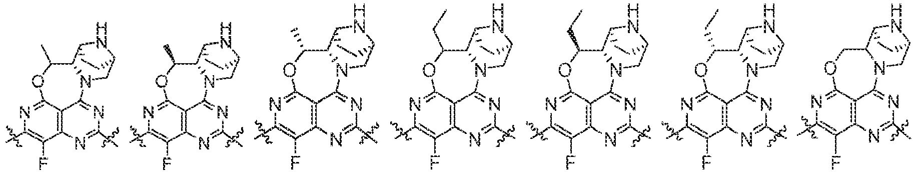

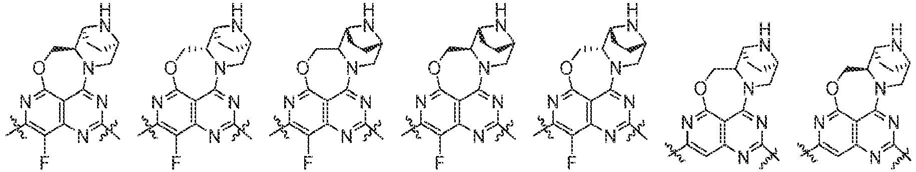

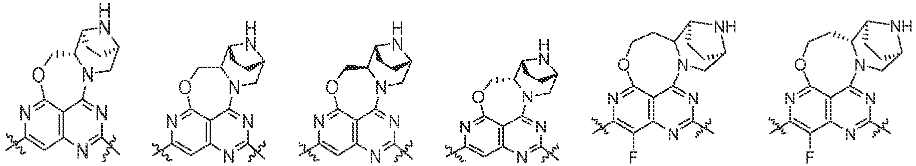

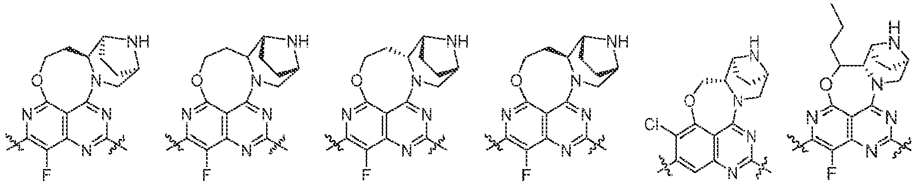

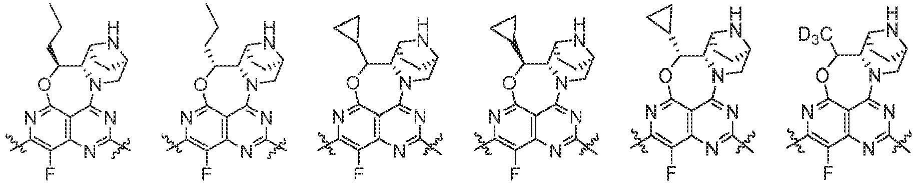

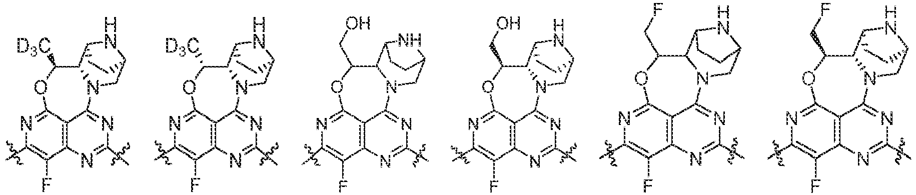

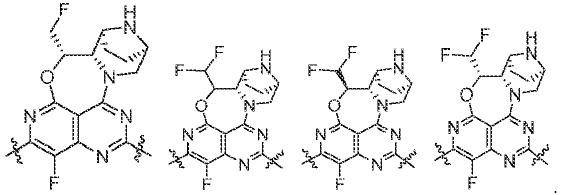

In an embodiment, 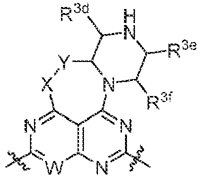 is selected from the group:

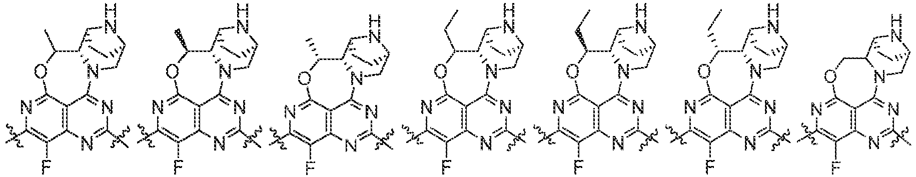

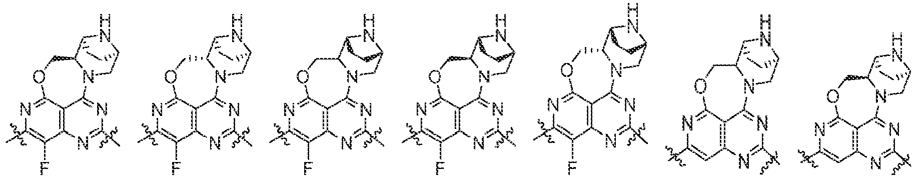

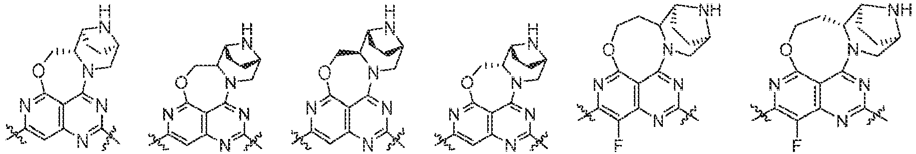

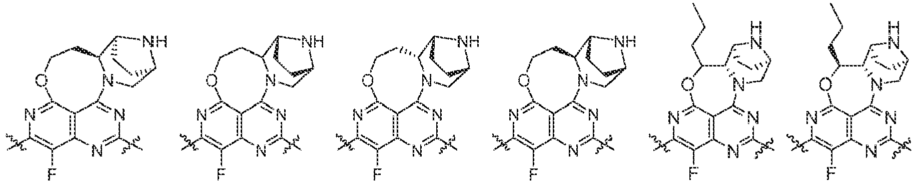

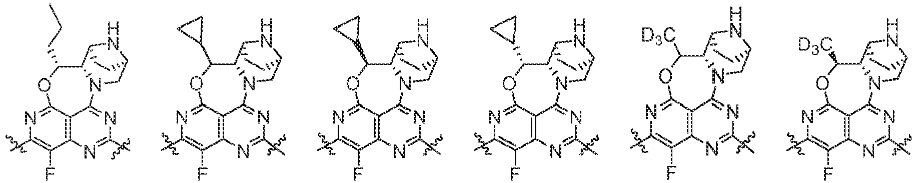

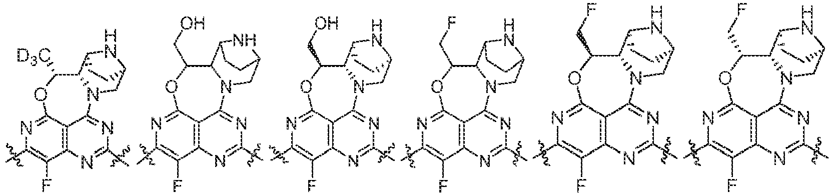

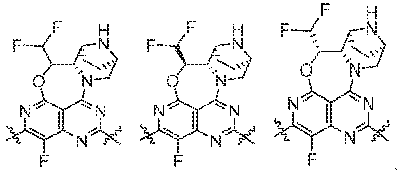

In an embodiment, 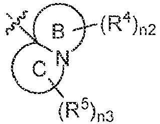 is selected from the group:

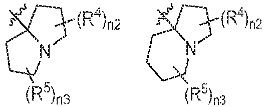

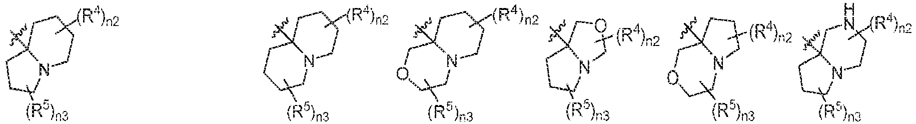

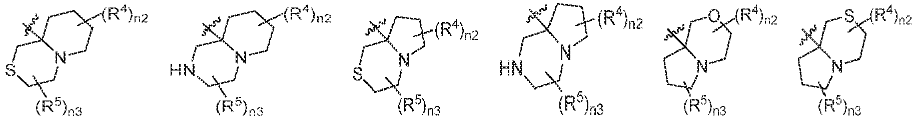

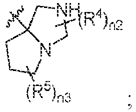

In each formula, each R⁴, R⁵, n2, n3 are respectively and independently as defined in formula (I).

In an embodiment, 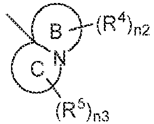 is selected from the group:

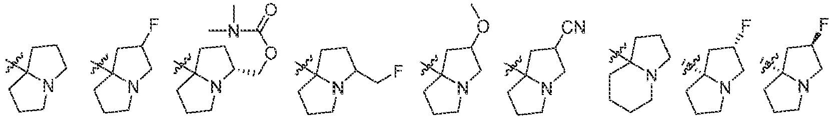

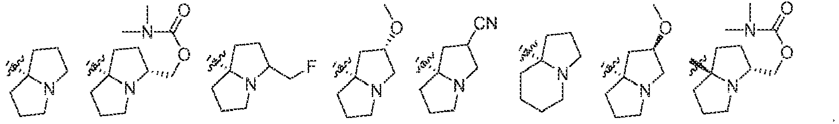

In an embodiment, R³ is hydrogen, fluorine, methyl or methoxy.

In an embodiment, R³ is hydrogen.

In an embodiment, R^{3a}, R^{3e}, R^{3f} are each independently hydrogen, fluorine, methyl or methoxy; or R^{3d} and R^{3e} are connected to form -CHR^{3d1}- or -CHR^{3d2}CHR^{3d3}-; R^{3f} is hydrogen, fluorine, methyl or methoxy; R^{3d1}, R^{3d2}, R^{3d3} are each independently hydrogen, fluorine, methyl or methoxy; or R^{3d} and R^{3f} are connected to form -CHR^{3f1}- or -CHR^{3f2}CHR^{3f3}-; R^{3e} is hydrogen, fluorine, methyl or methoxy; R^{3f1}, R^{3f2}, R^{3f3} are each independently hydrogen, fluorine, methyl or methoxy.

In an embodiment, L is methylene or ethylene or propylene, wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl.

In an embodiment, R⁶ is hydrogen, -N(R³⁴)₂, 3- to 20-membered heterocyclyl, C1-C6 alkyl, -L-3- to 20-membered heterocyclyl, -L-C6-C14aryl, -L-5- to 14-membered heteroaryl, -L-C3-C20 cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-C6-C14aryl, -L-COOH or -L-C(O)OC1-C6 alkyl; wherein the 3- to 20-membered heterocyclyl, the C6-C14 aryl in the -L-NR³⁴C(O)-C6-C14 aryl, the C3-C20 cycloalkyl optionally substituted by one or more (e.g., 1, 2, 3 4 or 5) R³⁵ or two hydrogen atoms on the same carbon atom are optionally and simultaneously substituted by -CH₂CH₂- or -CH₂CH₂CH₂-to form cyclopropyl or cyclobutyl; the C6-C14 aryl in the -L-C6-C14 aryl or 5- to 14-membered heteroaryl in -L-5- to 14-membered heteroaryl optionally substituted by one or more (e.g., 1, 2, 3 4 or 5) R³⁶; each L, each R³⁴, each R³⁵, each R³⁶ are each as defined.

In an embodiment, R⁶ is hydrogen, -N(R³⁴)₂, 3- to 8-membered monocyclic heterocyclyl, 5- to 20-membered spiro heterocyclyl, 5- to 20-membered fused heterocyclyl, 5- to 20-membered bridged heterocyclyl, C1-C6 alkyl, -L-3- to 8-membered monocyclic heterocyclyl, -L-5- to 20-membered spiro heterocyclyl, -L-5- to 20-membered fused heterocyclyl, -L-5- to 20-membered bridged heterocyclyl, -L-phenyl, -L-naphthyl, -L-5- to 14-membered heteroaryl, -L-C3-C12 cycloalkyl, -L-C5-C20 spiro cycloalkyl, C5-C20 fused cycloalkyl, -L-C5-C20 bridged cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-phenyl, -L-NR³⁴C(O)-naphthyl, -L-COOH or -L-C(O)OC1-C6 alkyl; wherein the 3-to 8-membered monocyclic heterocyclyl, 5- to 20-membered spiro heterocyclyl, 5- to 20-membered fused heterocyclyl, 5- to 20-membered bridged heterocyclyl, C3-C12 cycloalkyl, C5-C20 spiro cycloalkyl, C5-C20 fused cycloalkyl, C5-C20 bridged cycloalkyl, the phenyl in the -L-NR³⁴C(O)-phenyl, the naphthyl in the -L-NR³⁴C(O)-naphthyl optionally substituted by one or more (e.g., 1, 2, 3 4 or 5) R³⁵ or two hydrogen atoms on the same carbon atom are optionally and simultaneously substituted by -CH₂CH₂- or -CH₂CH2CH₂- to form cyclopropyl or cyclobutyl; phenyl in -L-phenyl, naphthyl in -L-naphthyl, 5- to 14-membered heteroaryl in -L-5- to 14-membered heteroaryl optionally substituted by one or more (e.g., 1, 2, 3 4 or 5) R³⁶; each L, each R³⁴, each R³⁵, each R³⁶ are each defined as above.

In an embodiment, R⁶ is hydrogen or -N(R³⁴)₂. Preferably, each R³⁴ is respectively and independently hydrogen or C1-C3 alkyl; or one R³⁴ is hydrogen and the other R³⁴ is C1-C3 alkyl.

In an embodiment, R³⁴ is hydrogen, C1-C3 alkyl or C1-C3 cyanoalkyl.

In an embodiment, the C1-C6 alkyl is methyl, ethyl, isopropyl, or isobutyl.

In an embodiment, L is methylene or ethylene or propylene, wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl.

In an embodiment, each R⁶ is respectively and independently 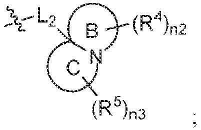 R⁴, R³, L₂, ring B, ring C, n2, n3 are as defined in the respective groups in formula (I).

In an embodiment, the heterocyclyl in R⁶ is respectively and independently hexahydro-1H-pyrrolizinyl, hexahydro-3H-pyrrolizin-3-one, hexahydro-1H-pyrrolo[2,1-c][1,4]oxazinyl, octahydroindolizinyl, hexahydropyrrolizinyl 4(1H)-oxide, azetidinyl, pyrrolidinyl, pyrrolidin-2-one, oxetanyl, piperidinyl, 1-azabicyclo[2.2.1]heptyl, morpholinyl, oxa-5-azabicyclo[2.2.1]hept-5-yl, thiopyranyl, 6-oxa-2-azaspiro[3.4]octyl, 7-oxa-2-azaspiro[3.5]nonyl, 2',3'-dihydrospiro[cyclopropane-1,1'-indenyl], (2S)-1-azabicyclo[2.2.1]heptan-2-yl or tetrahydrofuranyl; each group above is independently and optionally substituted by one or more (e.g., 1, 2, 3 4 or 5) R³³, or two hydrogen atoms on the same carbon atom are optionally and simultaneously substituted by -CH₂CH₂- or -CH₂CH₂CH₂- to form cyclopropyl or cyclobutyl; each R³⁵ is respectively defined as above.

In an embodiment, R⁶ is -L-heterocyclyl; the heterocyclyl is hexahydro-1H-pyrnolizinyl.

In an embodiment, R⁶ is -L-heterocyclyl; the heterocyclyl is hexahydro-1H-pyrrolizinyl substituted by one R³⁵, or is hexahydro-1H-pyrrolizinyl, wherein two hydrogen atoms on the same carbon atom are simultaneously substituted by -CH₂CH₂- or -CH₂CH₂CH₂- to form cyclopropyl or cyclobutyl; wherein R³⁵ is halogen, hydroxyl, C1-C3 hydroxyalkyl, C1-C3 halogenated alkyl, C1-C3 alkyl, C1-C3 alkoxy, phenyl, pyrazolyl, or -CH₂OC(O)N(R³⁴)₂.

In an embodiment, the halogen is fluorine.

In an embodiment, the heterocyclyl is hexahydro-1H-pyrrolizinyl further substituted by two additional R³⁵; the other two R³⁵ are each independently C1-C3 alkyl.

In an embodiment, R⁶ is -L-heterocyclyl; the heterocyclyl is azetidinyl substituted by one R³⁵, or is azetidinyl in which two hydrogen atoms on the same carbon atom are simultaneously substituted by CH₂CH₂- or -CH₂CH₂CH₂- to form cyclopropyl or cyclobutyl; the R³⁵ is C1-C3 alkyl.

In an embodiment, R⁶ is -L-heterocyclyl; the heterocyclyl is pyrrolidinyl substituted by one R³⁵, or is pyrrolidinyl in which two hydrogen atoms on the same carbon atom are simultaneously substituted by -CH₂CH₂- or -CH₂CH₂CH₂- to form cyclopropyl or cyclobutyl; wherein, R³⁵ is hydroxyalkyl, halogenated alkyl, C1-C3 alkyl, alkoxy, aryl C1-C3 alkyl, -phenyl, -O-phenyl, and-NHC(O)phenyl; wherein, the aryl in the aryl C1-C3 alkyl, the phenyl, or the phenyl in the -O-phenyl or the phenyl in the -NHC(O)phenyl are independently and optionally substituted by one or more (e.g., 1, 2, 3 4 or 5) R³⁶.

In an embodiment, the phenyl, -O-phenylor the phenyl in the -O-phenyl is substituted by -SO₂F.

In an embodiment, R⁶ is -L-heterocyclyl; the heterocyclyl is pyrrolidinyl substituted by two R³⁵, wherein one R³⁵ is C1-C3 alkyl and the other R³⁵ is C1-C3 alkoxy or halogen.

In an embodiment, R⁶ is -L-heterocyclyl; the heterocyclyl is pyrrolidin-2-one substituted by one R³⁵, or is pyrrolidin-2-one, wherein two hydrogen atoms on the same carbon atom are simultaneously substituted by -CH₂CH₂- or -CH₂CH₂CH₂- to form cyclopropyl or cyclobutyl; wherein R³⁵ is C1-C3 alkyl.

In an embodiment, R⁶ is -L-heterocyclyl; the heterocyclyl is piperidinyl substituted by one R³⁵, or is piperidinyl in which two hydrogen atoms on the same carbon atom are simultaneously substituted by -CH₂CH₂- or -CH₂CH₂CH₂- to form cyclopropyl or cyclobutyl; wherein R³⁵ is acetyl, (C1-C3alkoxy) C1-C3 alkoxy or -C(O)CH₂Cl.

In an embodiment, R⁶ is -L-heterocyclyl; the heterocyclyl is morpholinyl or oxa-5-azabicyclo[2.2.1]hept-5-yl.

In an embodiment, R⁶ is -L-heteroaryl; the heteroaryl is optionally substituted by one or more R³⁶.

In an embodiment, R⁶ is -L-heteroaryl; L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl; the heteroaryl is pyridinyl, pyrazolyl, imidazolyl, triazolyl, 4,5,6,7-tetrahydro-1H-indazolyl, benzimidazolyl, imidazo[1,2-a]pyridinyl or pyrimidinyl, each group above is optionally substituted by one or more R³⁶.

In an embodiment, R⁶ is -L-heteroaryl; L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂-CH₂CH₂- to form cyclopropyl or cyclobutyl; the heteroaryl is pyridinyl substituted by one R³⁶; wherein R³⁶ is halogen, C1-C4 alkyl, -N(R⁵)₂ or C1-C4 alkoxy.

In an embodiment, R⁶ is -L-heteroaryl; L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl; the heteroaryl is pyrazolyl substituted by one R³⁶; wherein R³⁶ is C1-C4 alkyl or -N(R³⁴)₂.

In an embodiment, R⁶ is -L-heteroaryl; L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl; the heteroaryl is imidazolyl substituted by one R³⁶, wherein R³⁶ is C1-C4 alkyl, C1-C4 halogenated alkyl or C1-C4 hydroxyalkyl.

In an embodiment, R⁶ is -L-heteroaryl; L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl; the heteroaryl is triazolyl substituted by one R³⁶, wherein R³⁶ is C1-C4 alkyl.

In an embodiment, R⁶ is -L-aryl; the aryl is optionally substituted by one or more R³⁶.

In an embodiment, R⁶ is -L-cycloalkyl; the cycloalkyl is optionally substituted by one or more R³⁵ or two hydrogen atoms on the same carbon atom are optionally and simultaneously substituted by - CH₂CH₂- or -CH₂CH₂CH₂- to form cyclopropyl or cyclobutyl; each R³⁵ is defined as above.

In an embodiment, R⁶ is -L-N(R³⁴)₂; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkyleneare optionally and simultaneously substituted by -CH₂CH₂- or - CH₂CH₂CH₂- to form cyclopropyl or cyclobutyl.

In an embodiment, R⁶ is -L-N(R³⁴)₂; wherein L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl; each R³⁴ is independently selected from C1-C3 alkyl.

In an embodiment, R⁶ is -L-NC(=NH)-NH₂; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkylene are optionally and simultaneously substituted by - CH₂CH₂- or -CH₂CH₂CH₂- to form cyclopropyl or cyclobutyl.

In an embodiment, L is ethylene or propylene.

In an embodiment, R⁶ is -L-C1-C6 halogenated alkyl; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkylene are optionally and simultaneously substituted by - CH₂CH₂- or -CH₂CH₂CH₂- to form cyclopropyl or cyclobutyl.

In an embodiment, R⁶ is -L-OR³⁴; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkylene are optionally and simultaneously substituted by -CH₂CH₂- or - CH₂CH₂CH₂- to form cyclopropyl or cyclobutyl.

In an embodiment, R⁶ is -L-(CH₂OR³⁴)(CH₂)ₙOR³⁴; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkylene are optionally and simultaneously substituted by - CH₂CH₂- or -CH₂CH₂CH₂- to form cyclopropyl or cyclobutyl.

In an embodiment, R⁶ is -L-NR³⁴C(O)-aryl; wherein L is C1-C4 alkylene, wherein one or two hydrogen atoms on any carbon atom or on any same carbon atom of the C1-C4 alkylene are independently and optionally substituted by deuterium or C1-C3 alkyl; or two hydrogen atoms on any same carbon atom of the C1-C4 alkylene are optionally and simultaneously substituted by - CH₂CH₂- or -CH₂CH₂CH₂- to form cyclopropyl or cyclobutyl.

In an embodiment, R⁶ is -L-heterocyclyl; L is C1-C4 alkylene, wherein two hydrogen atoms on any same carbon atom of the C1-C4 alkylene are independently and optionally substituted by deuterium or C1-C6 alkyl; or two hydrogen atoms on any same carbon atom of the C1-C4 alkylene are optionally and simultaneously substituted by -CH₂CH₂- or -CH₂CH₂CH₂- to form cyclopropyl or cyclobutyl.

In an embodiment, R⁶ is -L-6- to 10-membered fused heterocyclyl; two hydrogen atoms on the same carbon atom of any one or two carbon atoms on the 6- to 10-membered fused heterocyclyl are optionally and simultaneously substituted by =CR^{2a}R^{2b}; and/or the 6- to 10-membered fused heterocyclyl is also optionally substituted by one or more R^{6a}; wherein R^{2a}, R^{2b} are each independently hydrogen, halogen, cyano, C1-C4 alkyl or C1-C4 halogenated alkyl; each R^{6a} is respectively and independently halogen.

In an embodiment, L is methylene or ethylene or propylene; wherein two hydrogen atoms on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or - CH₂CH₂CH₂- to form cyclopropyl or cyclobutyl.

In an embodiment, W is CR²⁰; R²⁰ is cyclopropyl, cyclopropyl-O-, cyclobutyl, cyclobutyl-O-, cyclopentyl, cyclopentyl-O-, tetrahydrofuranyl, tetrahydrofuran-O-; wherein the cyclopropyl, cyclobutyl, cyclopentyl, tetrahydrofuranyl are each independently substituted with halogen.

In an embodiment, W is CR²⁰; R²⁰ is hydrogen, halogen, C1-C6 alkyl, C3-C6 cycloalkyl, C3-C6 cycloalkyl-O-, 3- to 6-membered heterocyclyl or 3- to 6-membered heterocyclyl-O-; wherein the C3-C6 cycloalkyl, the 3- to 6-membered heterocyclyl are each independently substituted with halogen.

In an embodiment, R²⁰ is halogen or C1-C3 alkyl.

In an embodiment, R²⁰ is fluorine.

In an embodiment, R²⁰ is methyl.

In an embodiment, L₂ is methylene or ethylene or propylene; wherein two hydrogen atoms on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or - CH₂CH₂CH₂- to form cyclopropyl or cyclobutyl

In an embodiment, 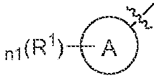 is phenyl substituted with one, two, three or four R¹, wherein each R¹ is respectively and independently halogen, hydroxyl, ethynyl, methyl, methyl-O-, methyl-S-, trifluoromethyl, trifluoromethyl-O-, amino, isopropyl, cyano or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropy; the cyclopropyl is optionally substituted by halogen or methyl.

In an embodiment, 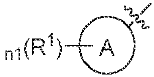 is naphthyl substituted with one, two, three or four R¹, wherein each R¹ is respectively and independently halogen, hydroxyl, ethynyl, methyl, methyl-O-, methyl-S-, trifluoromethyl, trifluoromethyl-O-, amino, isopropyl, cyano or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropy; the cyclopropyl is optionally substituted by halogen or methyl.

In an embodiment, 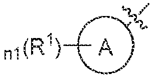 is naphthyl substituted with one, two or three R¹; wherein each R¹ is respectively and independently halogen, hydroxyl, ethynyl, methyl, ethyl, amino, cyano or difluoromethyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl.

In an embodiment, 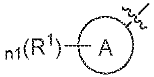 is naphthyl substituted with one or two R¹; wherein each R¹ is respectively and independently halogen, ethynyl or amino.

In an embodiment, 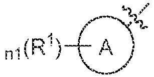 is heteroaryl optionally substituted by one or more R¹.

In an embodiment, 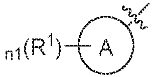 is pyridinyl substituted with one, two, three or four R¹; wherein each R¹ is respectively and independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl.

In an embodiment, 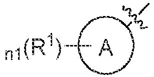 is quinolyl, isoquinolyl, indazolyl or benzo[d][1,3]dioxolane optionally substituted by one or more (e.g., 1, 2, 3, 4 or 5) R¹.

In an embodiment, 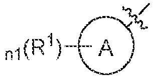 is isoquinolyl substituted by one, two, three or four R¹; wherein each R¹ is respectively and independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl.

In an embodiment, 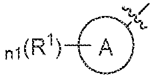 is isoquinolyl substituted by one, two or three R¹; wherein each R¹ is respectively and independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl.

In an embodiment, 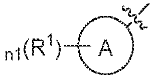 is quinolyl substituted by one, two, three or four R¹; wherein each R¹ is respectively and independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl. Preferably, one of R¹ is ethynyl, the rest R¹ are each independently halogen, hydroxyl, ethynyl, methyl, methyl-O-, methyl-S-, trifluoromethyl, trifluoromethyl-O-, amino, isopropyl or cyclopropyl; the cyclopropyl is optionally substituted by halogen or methyl.

In an embodiment, 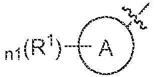 is indazolyl substituted by one, two or three R¹; wherein each R¹ is respectively and independently C1-C3 alkyl.

In an embodiment, 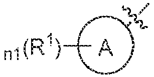 is benzo[d] [1,3]dioxolane substituted by two R¹; wherein each R¹ is respectively and independently selected from halogen.

In an embodiment, 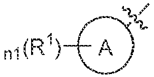 is selected from the group:

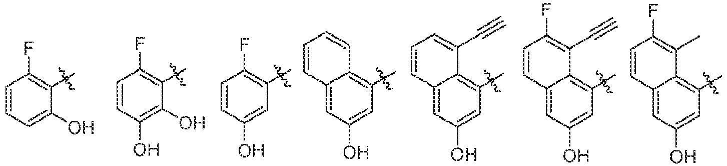

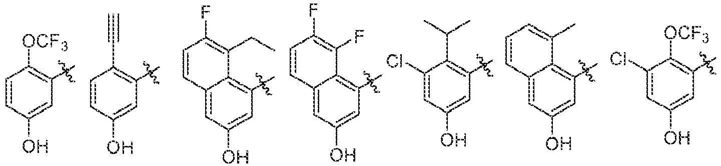

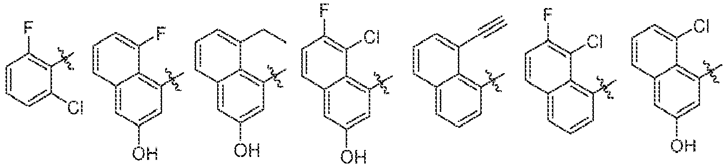

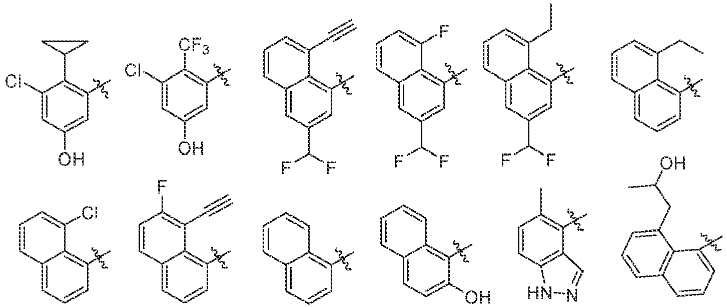

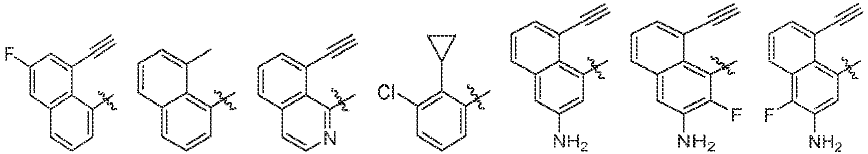

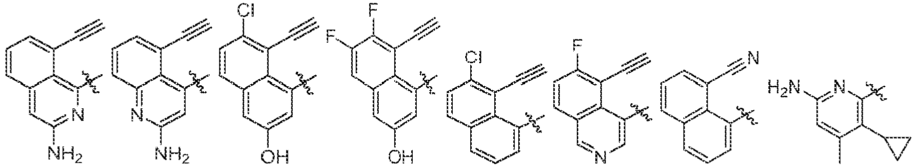

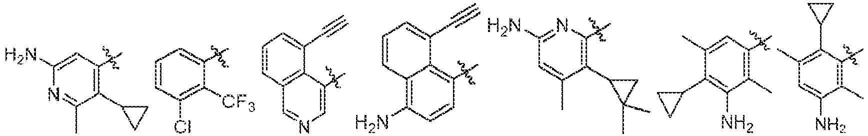

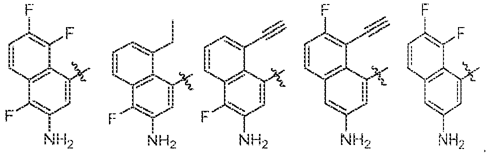

In an embodiment, 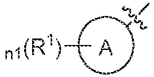 is selected from the group:

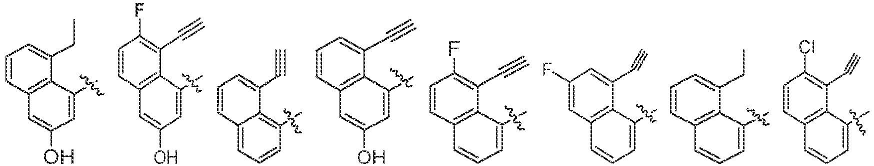

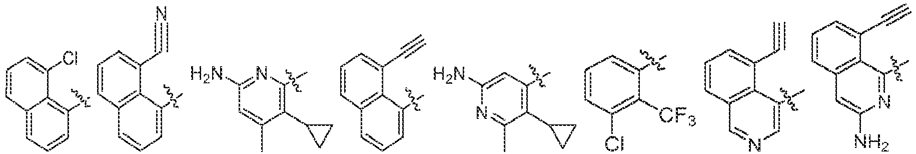

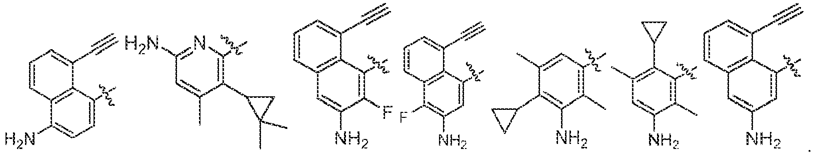

In an embodiment, the -L₃-R⁶ is selected from the group: 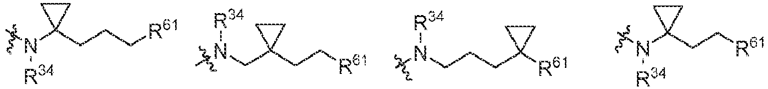 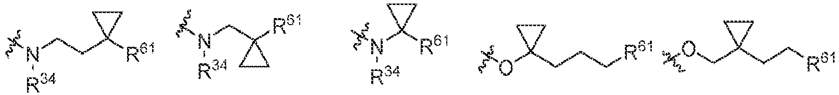 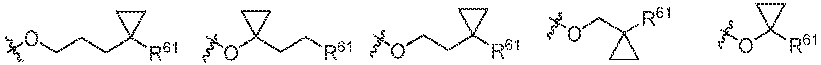 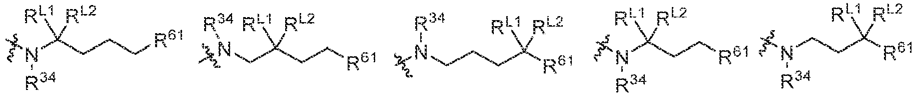 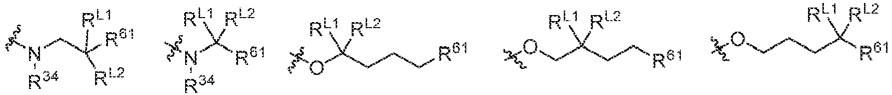 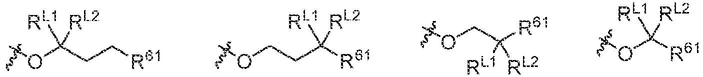 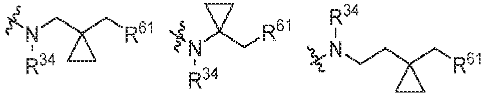 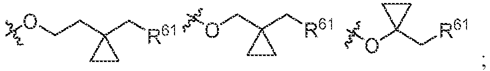 in each formula, R^{L1}, R^{L2} are each independently hydrogen, deuterium, C1-C6 alkyl or C1-C6 halogenated alkyl; R⁶¹ is heterocyclyl, aryl, heteroaryl, cycloalkyl, -N(R³⁴)₂, -NHC(=NH)NH₂, - C(O)N(R³⁴)₂, -C1-C6 halogenated alkyl, -OR³⁴, -(CH₂OR³⁴)(CH₂)ₙOR³⁴, -NR³⁴C(O)-aryl, -COOH or -C(O)OC1-C6 alkyl; wherein the heterocyclyl, the aryl in the -NR³⁴C(O)-aryl, the cycloalkyl each optionally substituted by one or more (e.g., 1, 2, 3 4 or 5) R³⁵ or two hydrogen atoms on the same carbon atom are optionally and simultaneously substituted by -CH₂CH₂- or -CH₂CH₂CH₂- to form cyclopropyl or cyclobutyl; the aryl, the heteroaryl each optionally substituted by one or more (e.g., 1, 2, 3 4 or 5) R³⁶; each R³⁴, each R³⁵, each R³⁶ are each defined as above. Preferably, in each formula above, the R³⁴ in N-R³⁴ is H.

In an embodiment, the -L₃-R⁶ is selected from the group: 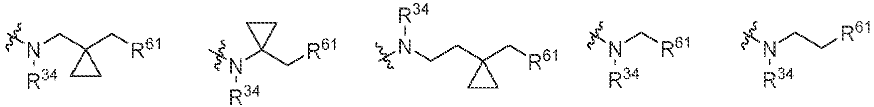 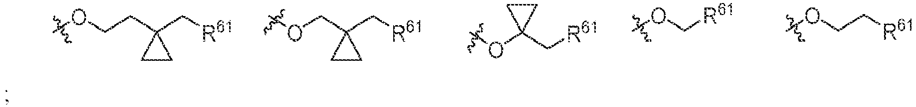 in each formula, R⁶¹ is heterocyclyl, aryl, heteroaryl, cycloalkyl, -N(R³⁴)₂, -NHC(=NH)NH₂, - C(O)N(R³⁴)₂, -C1-C6 halogenated alkyl, -OR³⁴, -(CH₂OR³⁴)(CH₂)ₙOR³⁴, -NR³⁴C(O)-aryl, -COOH or -C(O)OC1-C6 alkyl; wherein the heterocyclyl must be substituted by one or more (e.g., 1, 2, 3 4 or 5) R³⁵ or two hydrogen atoms on the same carbon atom are optionally and simultaneously substituted by -CH₂CH₂- or -CH₂CH₂CH₂- to form cyclopropyl or cyclobutyl; the aryl in the - NR³⁴C(O)-aryl, the cycloalkyl each optionally substituted by one or more (e.g., 1, 2, 3 4 or 5) R³⁵ or two hydrogen atoms on the same carbon atom are optionally and simultaneously substituted by - CH₂CH₂- or -CH₂CH₂CH₂- to form cyclopropyl or cyclobutyl; the aryl, the heteroaryl each optionally substituted by one or more (e.g., 1, 2, 3 4 or 5) R³⁶; each R³⁴, each R³⁵, each R³⁶ are each defined as above. Preferably, in each formula above, the R³⁴ in N-R³⁴ is H.

In an embodiment, the -L₃-R⁶ is -NH-R⁶¹, -O-R⁶¹ or -R⁶¹; orthe -L₃-R⁶ is selected from the group: 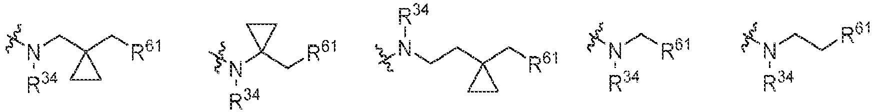 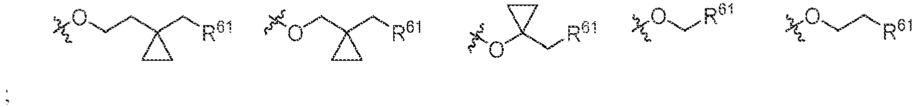 in each formula, the R³⁴ in N-R³⁴ is H; each R⁶¹ is independently selected from the group:

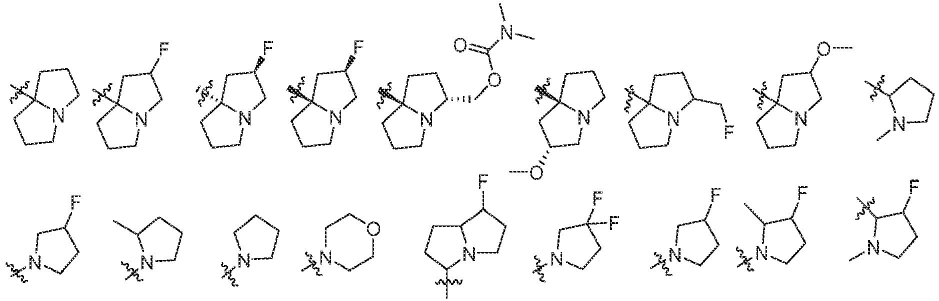

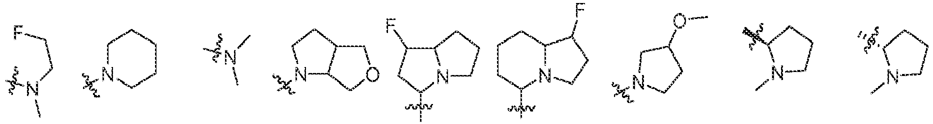

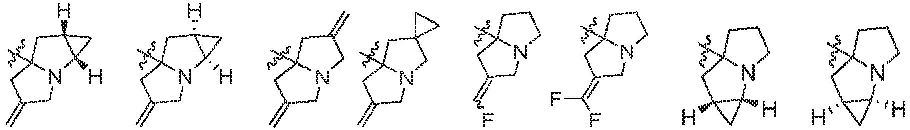

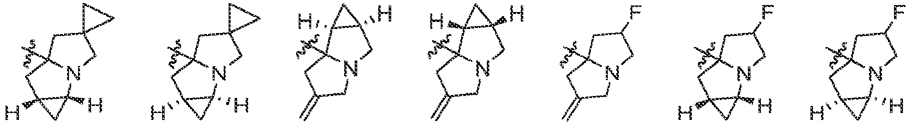

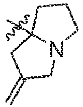

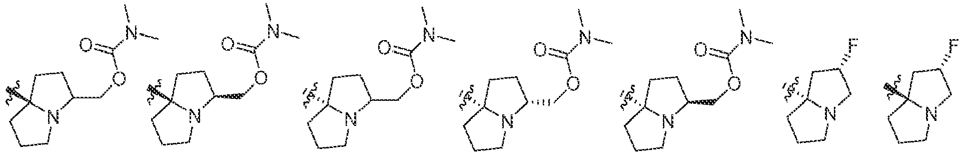

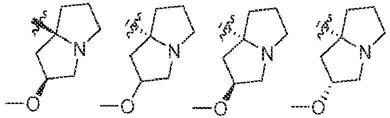

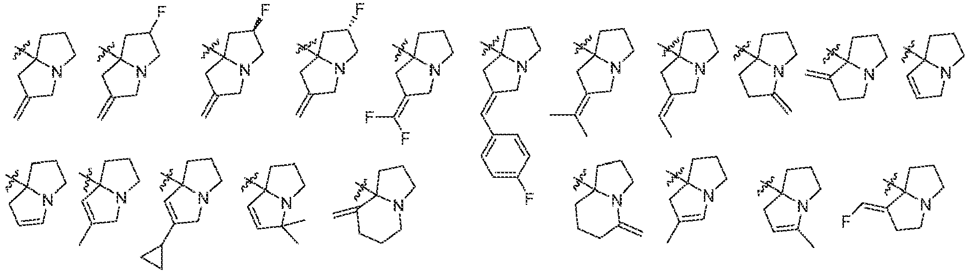

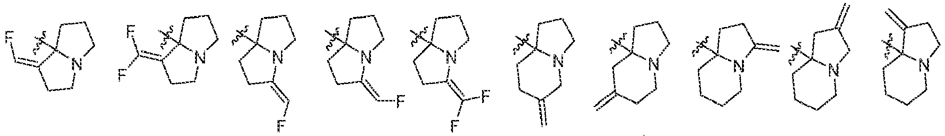

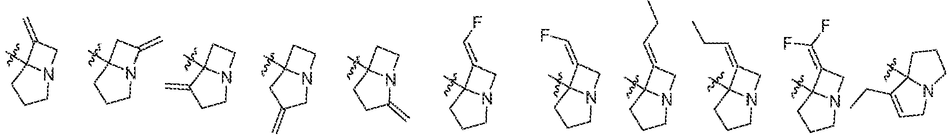

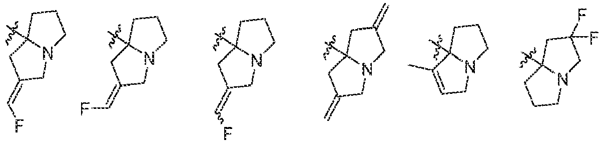

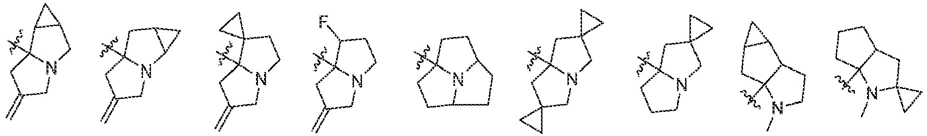

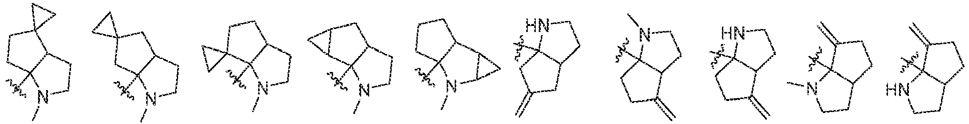

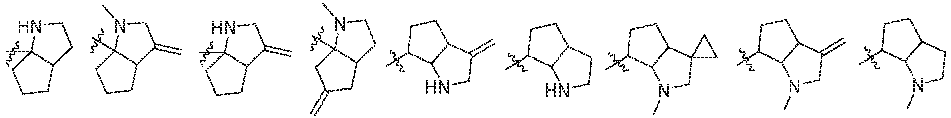

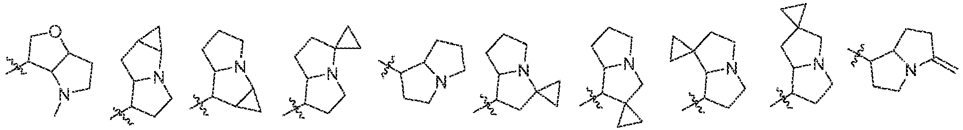

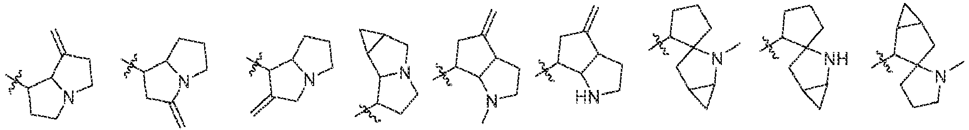

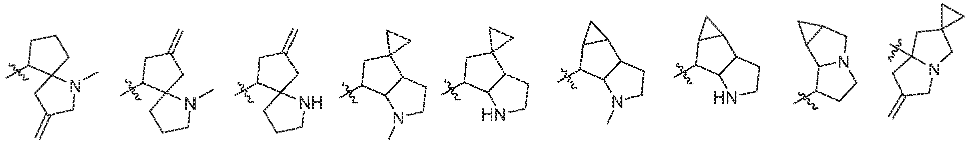

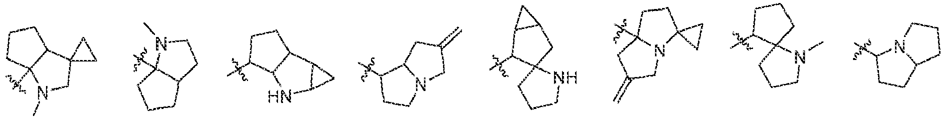

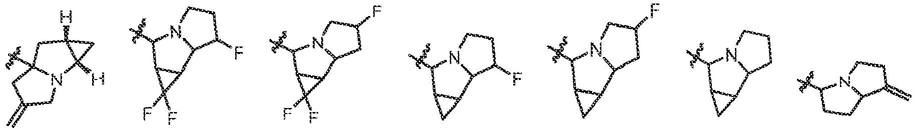

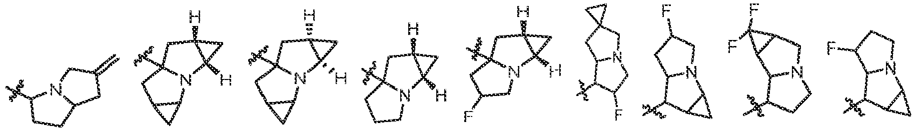

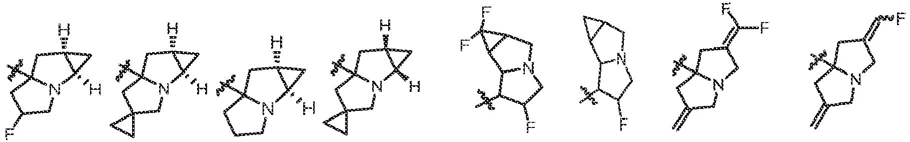

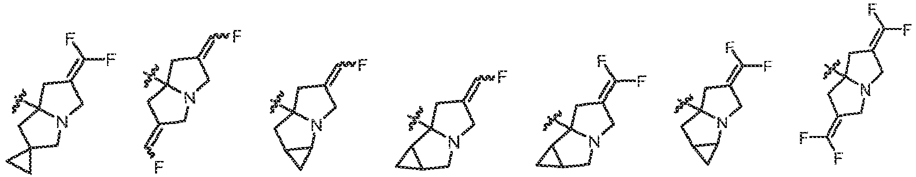

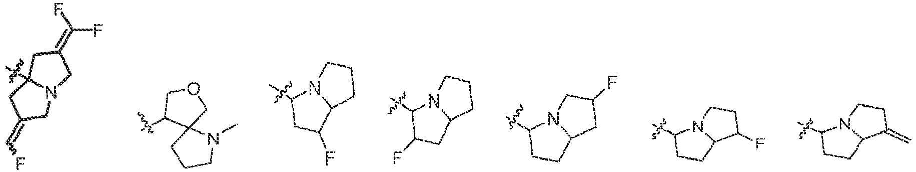

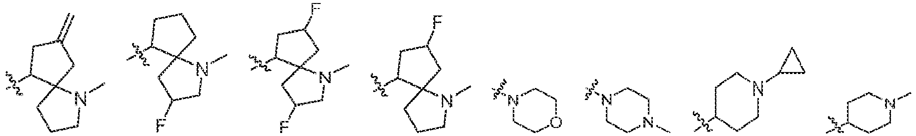

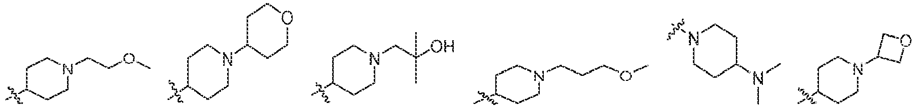

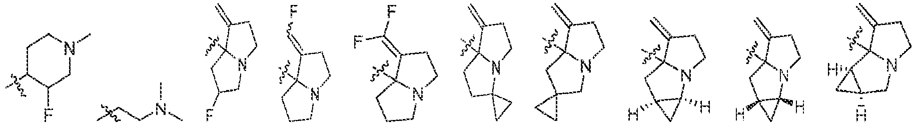

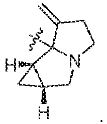

In an embodiment, the -L₃-R⁶ is -O-R⁶¹, 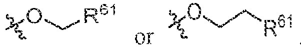 . In an embodiment, R⁶¹ is independently selected from the group:

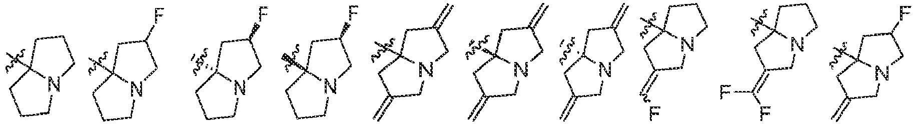

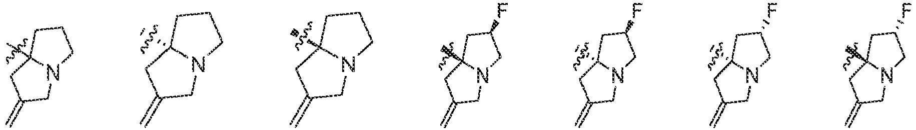

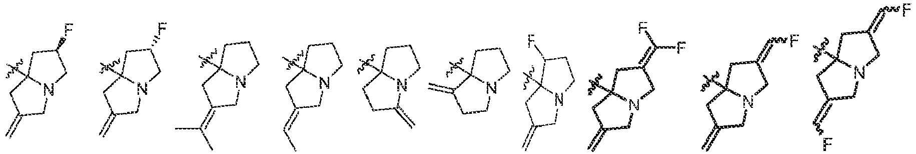

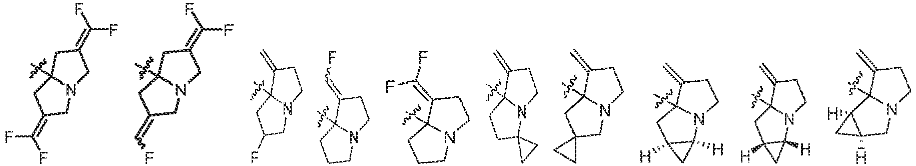

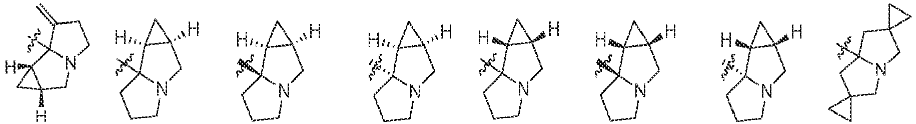

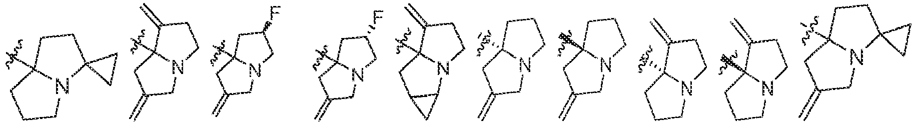

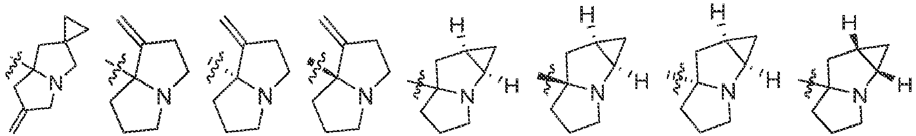

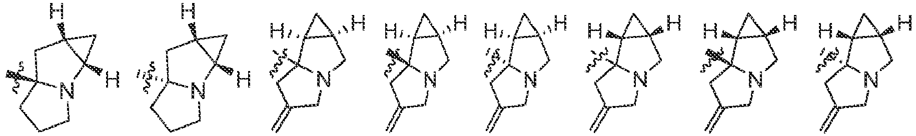

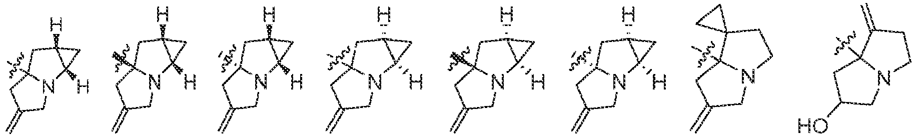

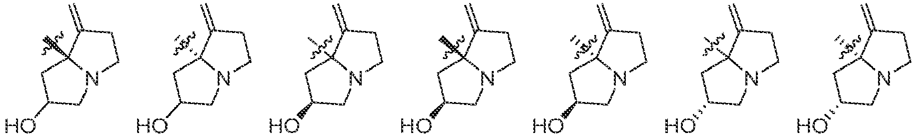

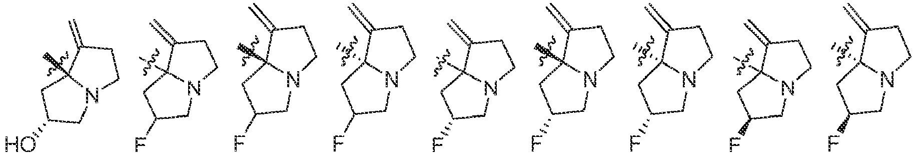

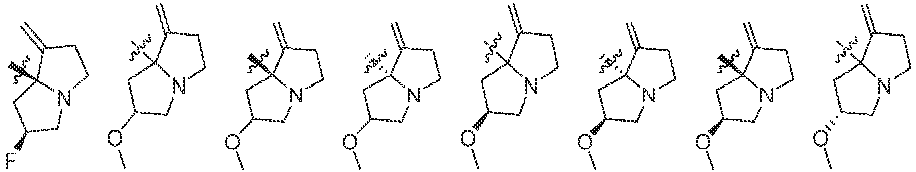

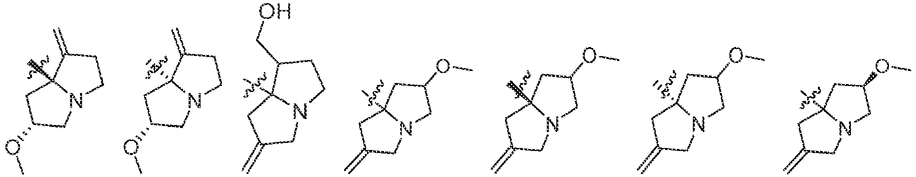

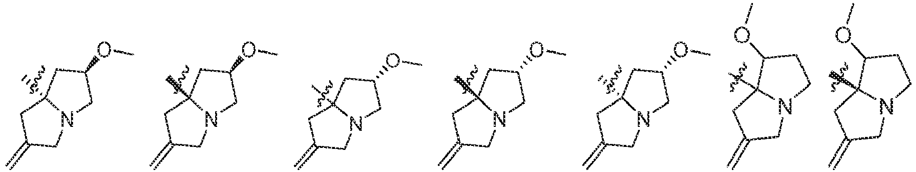

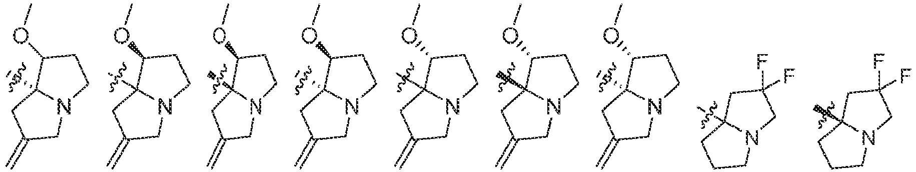

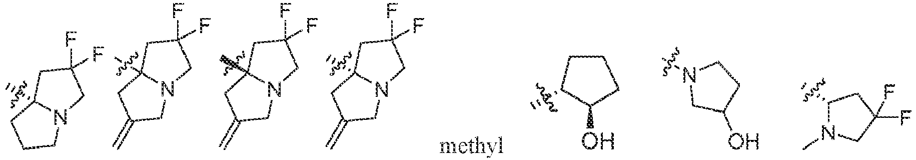

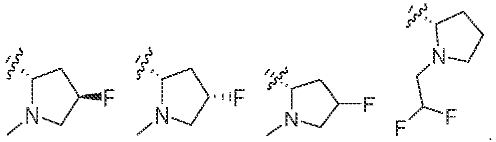

In an embodiment, the -L₃-R⁶ is 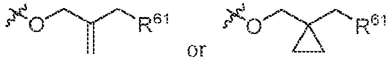 . In an embodiment, R⁶¹ is -OR³⁴, -N(R³⁴)₂ or heterocyclyl; wherein the heterocyclyl optionally substituted by one or more (e.g., 1, 2, 3 4 or 5) R³⁵ or two hydrogen atoms on the same carbon atom are optionally and simultaneously substituted by -CH₂CH₂- or -CH₂CH₂CH₂- to form cyclopropyl or cyclobutyl; each R³⁴, each R³⁵ are each defined as above. Preferably, each R³⁵ is respectively and independently halogen, hydroxyl, C1-C3 alkyl, C2-C4 alkenylene, C2-C4 halogenated alkenylene or C1-C3 halogenated alkyl. Preferably, each R³⁴ is respectively and independently hydrogen, C1-C3 alkyl or C1-C3 halogenated alkyl. In an embodiment, R⁶¹ is independently selected from the group:

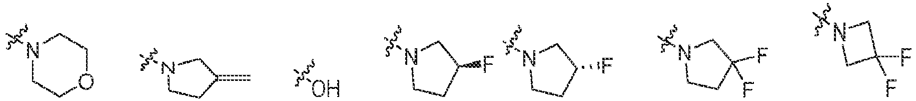

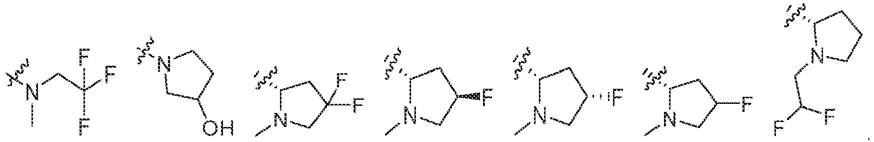

In an embodiment, R⁶¹ is hexahydro-1H-pyrrolizinyl.

In an embodiment, R⁶¹ is hexahydro-1H-pyrrolizinyl substituted by R³⁵; wherein R³⁵ is halogen, hydroxyl, C1-C3 hydroxyalkyl, C1-C3 halogenated alkyl, C1-C3 alkyl, C1-C3 alkoxy, C2-C3 alkenyl, C2-C3 halogenated alkenyl, phenyl, pyrazolyl or -CH₂OC(O)N(R³⁴)₂; each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

In an embodiment, the 6- to 10-membered fused heterocyclyl is selected from the group:

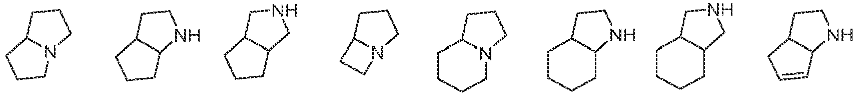

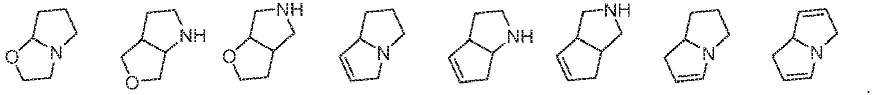

In an embodiment, the 6- to 10-membered fused heterocyclyl is selected from the group: 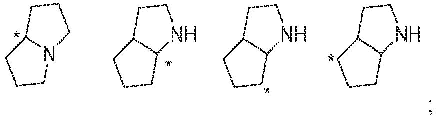 the carbon atoms marked with * are carbon atoms connected to other parts of the molecule.

In an embodiment, the 7- to 11-membered spiro heterocyclyl is selected from the group:

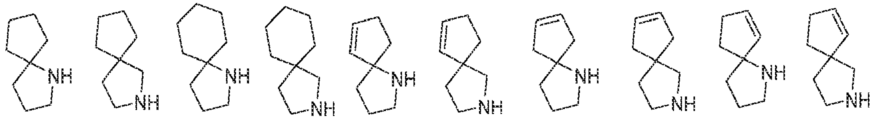

In an embodiment, the 7- to 11-membered spiro heterocyclyl is selected from the group: 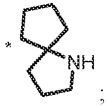 the carbon atoms marked with * are carbon atoms connected to other parts of the molecule.

In an embodiment, the spirocyclic ring substituted 6- to 10-membered fused heterocyclyl is selected from the group:

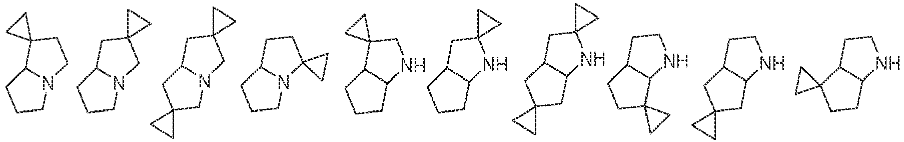

In an embodiment, the spirocyclic ring substituted 6- to 10-membered fused heterocyclyl is selected from the group: 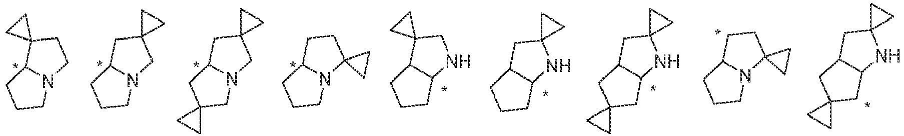 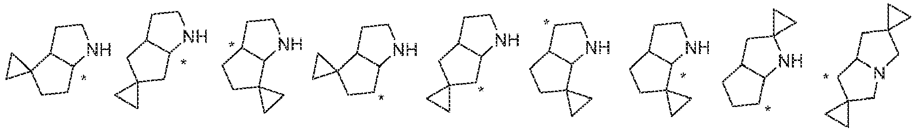 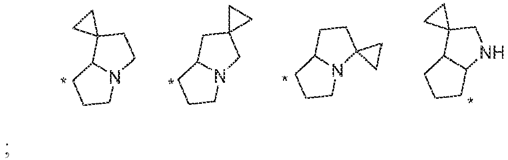 the carbon atoms marked with * are carbon atoms connected to other parts of the molecule.

In an embodiment, the spirocyclic ring substituted 7- to 11-membered spiro heterocyclyl is selected from the group:

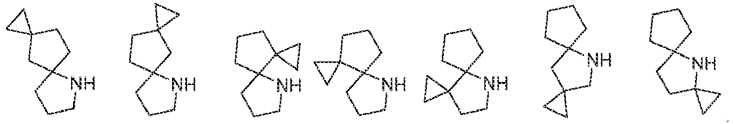

In an embodiment, the fused ring substituted 6- to 10-membered fused heterocyclyl is selected from the group:

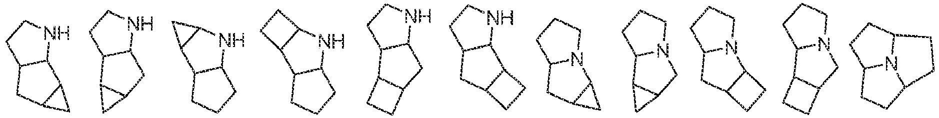

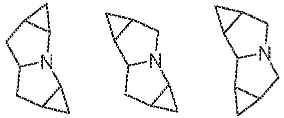

In an embodiment, the fused ring substituted 6- to 10-membered fused heterocyclyl is selected from the group: 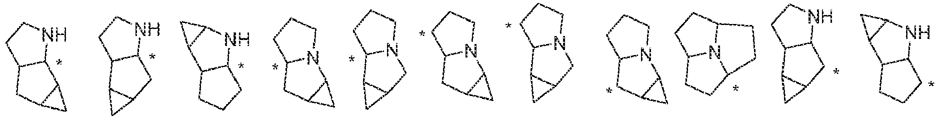 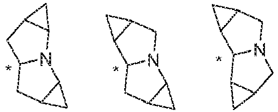 the carbon atoms marked with * are carbon atoms connected to other parts of the molecule.

In an embodiment, the fused ring substituted 7- to 11-membered spiro heterocyclyl is selected from the group:

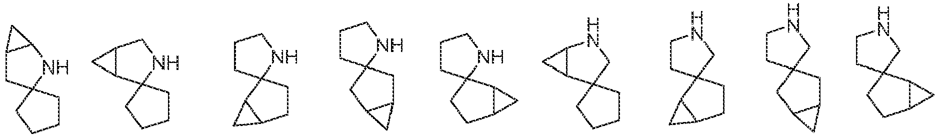

In an embodiment, the fused ring substituted 7- to 11-membered spiro heterocyclyl is selected from the group: 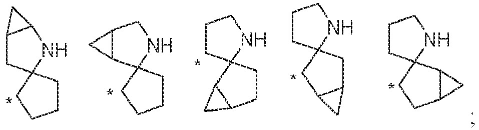 the carbon atoms marked with * are carbon atoms connected to other parts of the molecule.

In an embodiment, the spirocyclic and fused ring substituted 6- to 10-membered fused heterocyclyl is selected from the group:

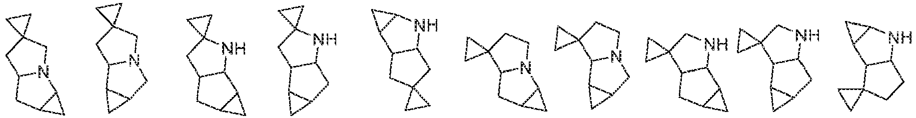

In an embodiment, the spirocyclic and fused ring substituted 6- to 10-membered fused heterocyclyl is selected from the group: 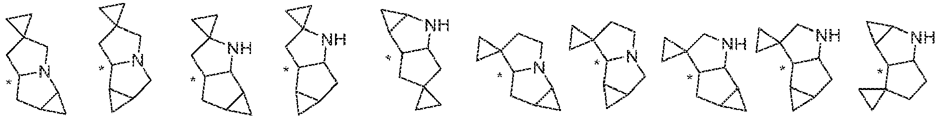 ; the carbon atoms marked with * are carbon atoms connected to other parts of the molecule.

In an embodiment, L₃ is O; R⁶ is -L-hexahydro-1H-pyrrolozinyl; wherein L is a C1-C4 alkylene; two hydrogen atoms on any carbon atom of the hexahydro-1H-pyrrolozinyl are simultaneously substituted by =CR^{2a}R^{2b}; wherein R^{2a}, R^{2b} are each independently hydrogen, halogen, cyano, C1-C4 alkyl, or C1-C4 halogenated alkyl. Preferably, the hexahydro-1H-pyrrolozinyl is also optionally substituted by one or more (e.g., 1, 2, 3, 4, or 5) R^{6a}; wherein each R^{6a} is respectively and independently halogen, hydroxy, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy or cyano.

In an embodiment, L₃ is O; R⁶ is -L-hexahydro-1H-pyrrolozinyl; wherein L is a C1-C4 alkylene; two hydrogen atoms on any carbon atom of the hexahydro-1H-pyrrolozinyl are simultaneously substituted by =CR^{2a}R^{2b}; wherein R^{2a}, R^{2b}, are taken together with the carbon atom to which R^{2a}, R^{2b} are attached, form a C3-C6 monocyclic cycloalkyl or a 3- to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl. Preferably, the hexahydro-1H-pyrrolozinyl is also optionally substituted by one or more (e.g., 1, 2, 3, 4, or 5) R^{6a} substituents; wherein each R^{6a} is respectively and independently halogen, hydroxy, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, or cyano.

In an embodiment, L₃ is O; R⁶ is -L-hexahydro-1H-pyrrolozinyl; wherein L is a C1-C4 alkylene; two hydrogen atoms on one of any two carbon atoms on the hexahydro-1H-pyrrolozinyl are simultaneously substituted by =CR^{2a}R^{2b}; wherein R^{2a}, R^{2b} are each independently hydrogen, halogen, cyano, C1-C4 alkyl, or C1-C4 halogenated alkyl. Preferably, the hexahydro-1H-pyrrolozinyl is also optionally substituted by one or more (e.g., 1, 2, 3, 4, or 5) R^{6a}; wherein each R^{6a} is respectively and independently halogen, hydroxy, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, or cyano.

In an embodiment, L₃ is O; R⁶ is -L-hexahydro-1H-pyrrolozinyl; wherein L is a C1-C4 alkylene; two hydrogen atoms on one of any two carbon atoms on the hexahydro-1H-pyrrolozinyl are simultaneously substituted by =CR^{2a}R^{2b}; wherein one pair of R^{2a}, R^{2b} are each independently hydrogen, halogen, cyano, C1-C4 alkyl, or C1-C4 halogenated alkyl; the other pair of R^{2a}, R^{2b}, are taken together with the attached carbon atom, form a C3-C6 monocyclic cycloalkyl or a 3- to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl. Preferably, the hexahydro-1H-pyrrolozinyl is also optionally substituted by one or more (e.g., 1, 2, 3, 4, or 5) R^{6a}; wherein each R^{6a} is respectively and independently halogen, hydroxy, C1-C6 hydroxyalkyl, C1-C6 alkyl, C2-C6 alkenyl, C2-C6 alkynyl, C 1-C6 halogenated alkyl, C1-C6 alkoxy, C 1-C6 halogenated alkoxy, or cyano.

In an embodiment, L₃ is O; R⁶ is -L-hexahydro-1H-pyrrolozinyl; wherein L is a C1-C4 alkylene; on the hexahydro-1H-pyrrolozinyl, two hydrogen atoms on one of any two carbon atoms are simultaneously substituted by =CR^{2a}R^{2b}; wherein both pairs of R^{2a}, R^{2b}, each independently with the attached carbon atom, form a C3-C6 monocyclic cycloalkyl or a 3- to 6-membered monocyclic heterocyclyl; the C3-C6 monocyclic cycloalkyl or 3- to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl. Preferably, the hexahydro-1H-pyrrolozinyl is also optionally substituted by one or more (e.g., 1, 2, 3, 4, or 5) R^{6a}; wherein each R^{6a} is respectively and independently halogen, hydroxy, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, or cyano.

In an embodiment, L₃ is O; R⁶ is -L-hexahydro-1H-pyrrolozinyl; wherein L is a C1-C4 alkylene; the hexahydro-1H-pyrrolozinyl is substituted by one or more (e.g., 1, 2, 3, 4, or 5) R^{6a}; wherein each R^{6a} is respectively and independently halogen, hydroxy, C1-C6 hydroxyalkyl, C1-C6 alkyl, C2-C6 alkenyl, C2-C6 alkynyl, C1-C6 halogenated alkyl, C1-C6 alkoxy, C1-C6 halogenated alkoxy, or cyano.

In an embodiment, R⁶ is selected from the group:

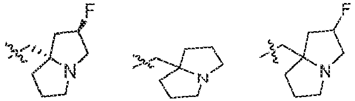

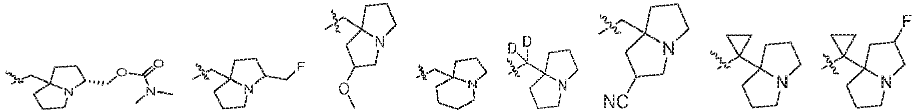

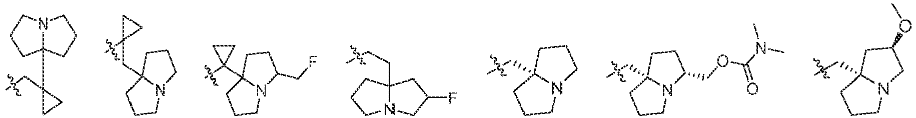

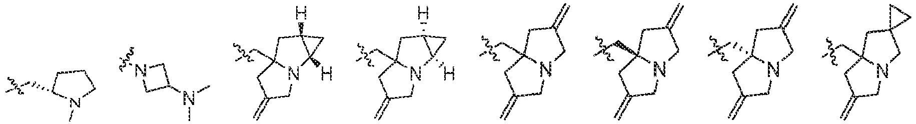

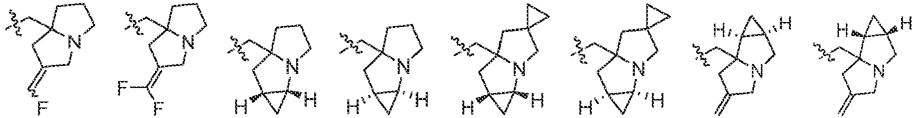

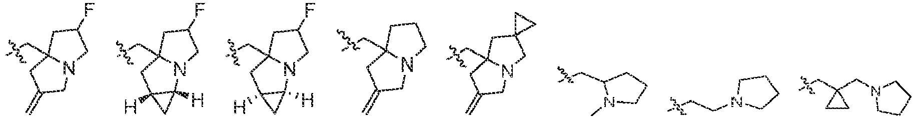

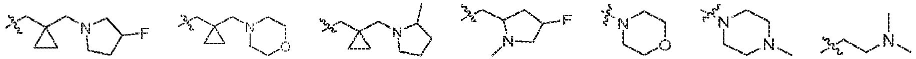

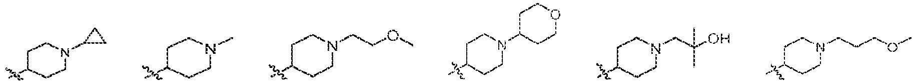

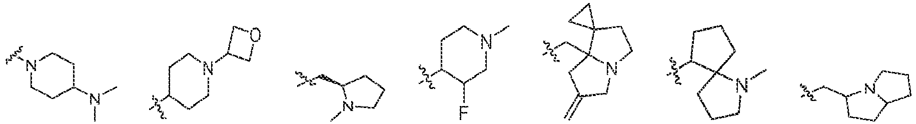

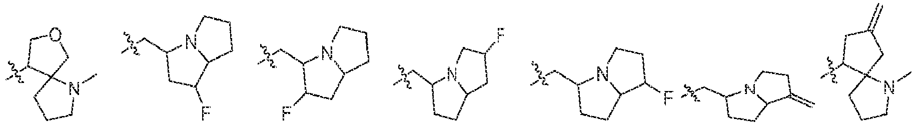

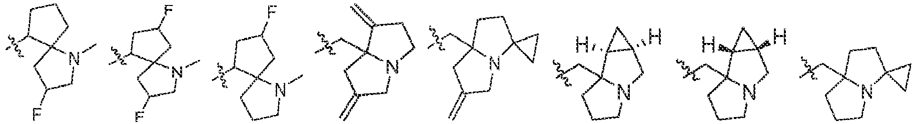

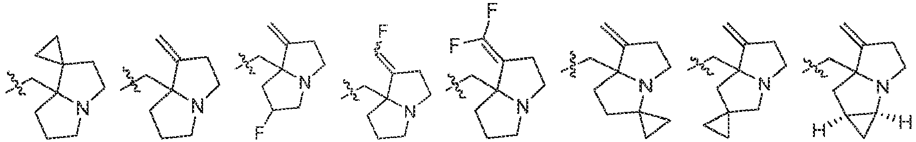

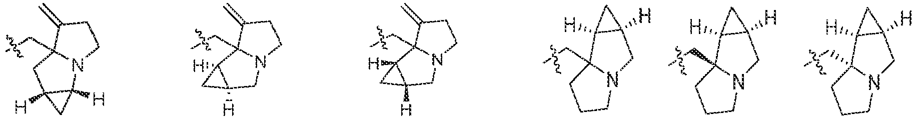

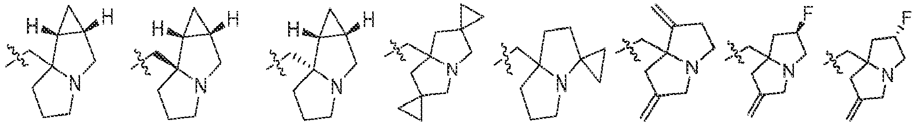

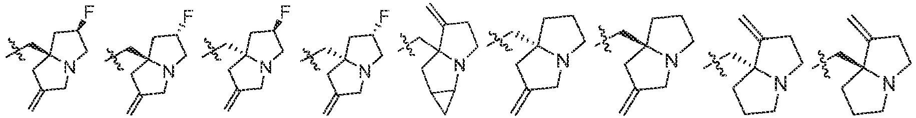

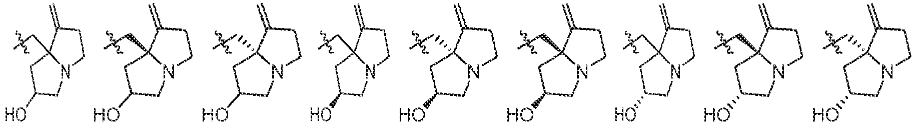

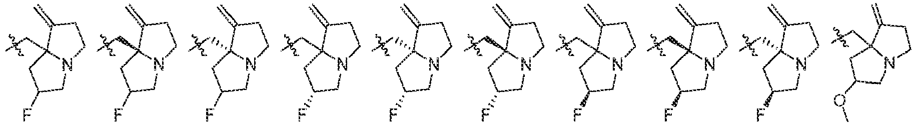

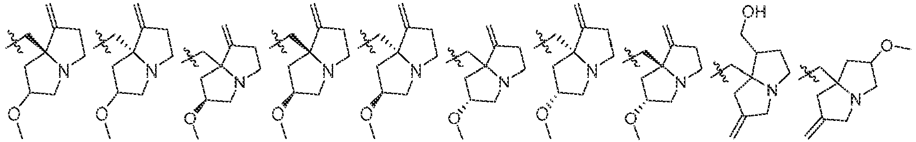

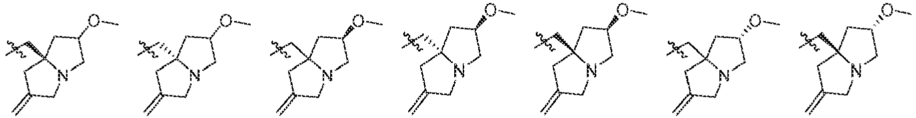

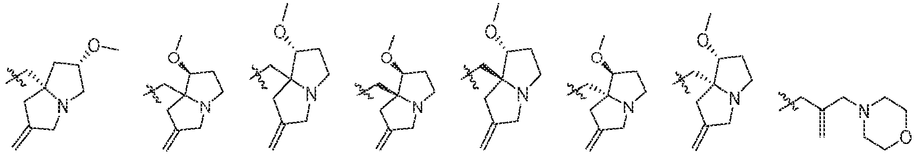

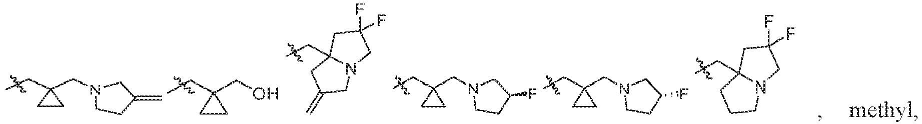

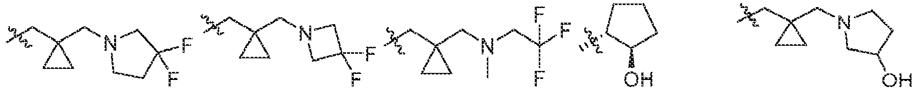

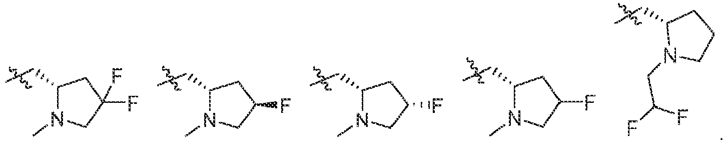

In an embodiment, R⁶ is -L-6- to 10-membered fused heterocyclyl; the 6- to 10-membered fused heterocyclyl containing 1, 2, 3 or 4 heteroatoms selected from N(R^{m}), S, S(=O), S(=O)₂, O as ring atoms; R^{m} is absent, hydrogen, C1-C4 alkyl, deuterated C1-C4 alkyl, C1-C4 halogenated alkyl, C3-C20 cycloalkyl or 3- to 20-membered heterocyclyl; wherein L is a bond, C1-C4 alkylene or heteroaryl; the 6- to 10-membered fused heterocyclyl is saturated; two hydrogen atoms on the same carbon atom of any one or two carbon atoms on the 6- to 10-membered fused heterocyclyl are optionally and simultaneously substituted by =CR^{2a}R^{2b}; the 6- to 10-membered fused heterocyclyl is also optionally substituted by one or more R^{6a}; R^{2a}, R^{2b}, R^{6a} are each defined as above. Preferably, R^{2a}, R^{2b} are each independently hydrogen, halogen or C1-C4 alkyl, C1-C4 halogenated alkyl; R^{6a} is halogen, hydroxyl, or C1-C6 alkyl.

In an embodiment, R⁶ is -L-6- to 10-membered fused heterocyclyl; the 6- to 10-membered fused heterocyclyl is hexahydro-1H-pyrrolizinyl; wherein L is C1-C4 alkylene; two hydrogen atoms on the same carbon atom of any one or two carbon atoms on the 6- to 10-membered fused heterocyclyl are optionally and simultaneously substituted by =CR^{2a}R^{2b}; the 6- to 10-membered fused heterocyclyl is also optionally substituted by one or more R^{6a}; R^{2a}, R^{2b}, R^{6a} are each defined as above. Preferably, R^{2a}, R^{2b} are each independently hydrogen, halogen or C1-C4 alkyl, C1-C4 halogenated alkyl; R^{6a} is halogen, hydroxyl, or C1-C6 alkyl.

In an embodiment, R⁶ is -L-6- to 10-membered fused heterocyclyl; the 6- to 10-membered fused heterocyclyl is hexahydro-1H-pyrrolizinyl; wherein L is C1-C2 alkylene; two hydrogen atoms on the same carbon atom of any one or two carbon atoms on the 6- to 10-membered fused heterocyclyl are optionally and simultaneously substituted by =CR^{2a}R^{2b}; the 6- to 10-membered fused heterocyclyl is also optionally substituted by one or more R^{6a}; R^{2a}, R^{2b} are each independently hydrogen or fluorine; R^{6a} is halogen, hydroxyl or C1-C6 alkyl.

In an embodiment, R⁶ is selected from the group: 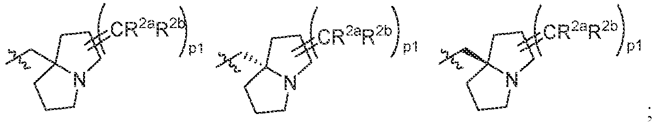 in each formula, each p1 is respectively and independently 1 or 2; R^{2a}, R^{2b} are each defined as above. Preferably, R^{2a}, R^{2b} are each independently hydrogen, halogen or C1-C4 alkyl, C1-C4 halogenated alkyl. Preferably, the 1H-pyrrozine is also optionally substituted by one or more R^{6a}; R^{6a} is defined as above. Preferably, R^{6a} are each independently halogen or C1-C6 alkoxy.

In an embodiment, R⁶ is selected from the group:

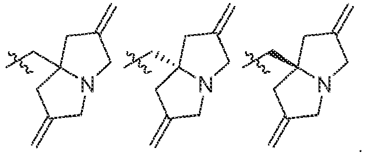

In an embodiment, R⁶ is selected from the group: 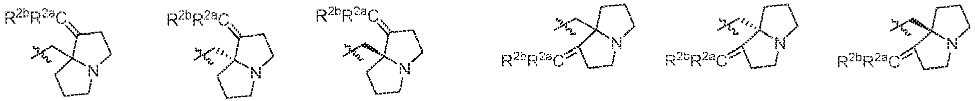 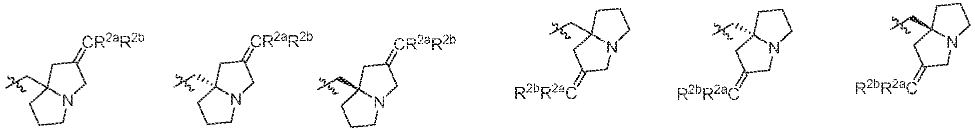 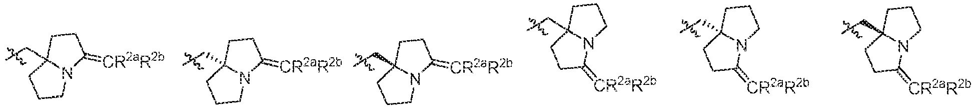 in each formula, R^{2a}, R^{2b} are each defined as above. Preferably, R^{2a}, R^{2b} are each independently hydrogen, halogen or C1-C4 alkyl, C1-C4 halogenated alkyl. Preferably, the 1H-pyrrozine is also optionally substituted by one or more (e.g., 2 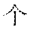 or 3 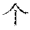 )R^{6a}; R^{6a} is defined as above. Preferably, R^{6a} are each independently halogen or C1-C6 alkoxy.

In an embodiment, the compound is selected from Table (I):

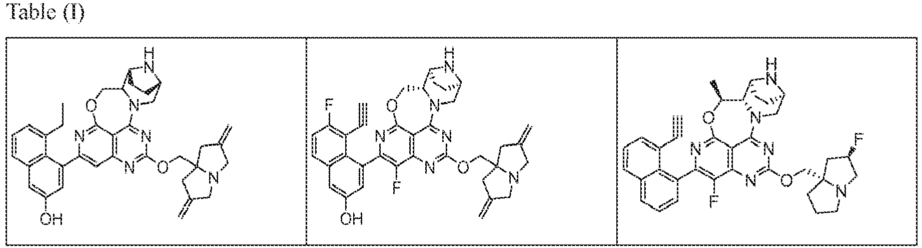

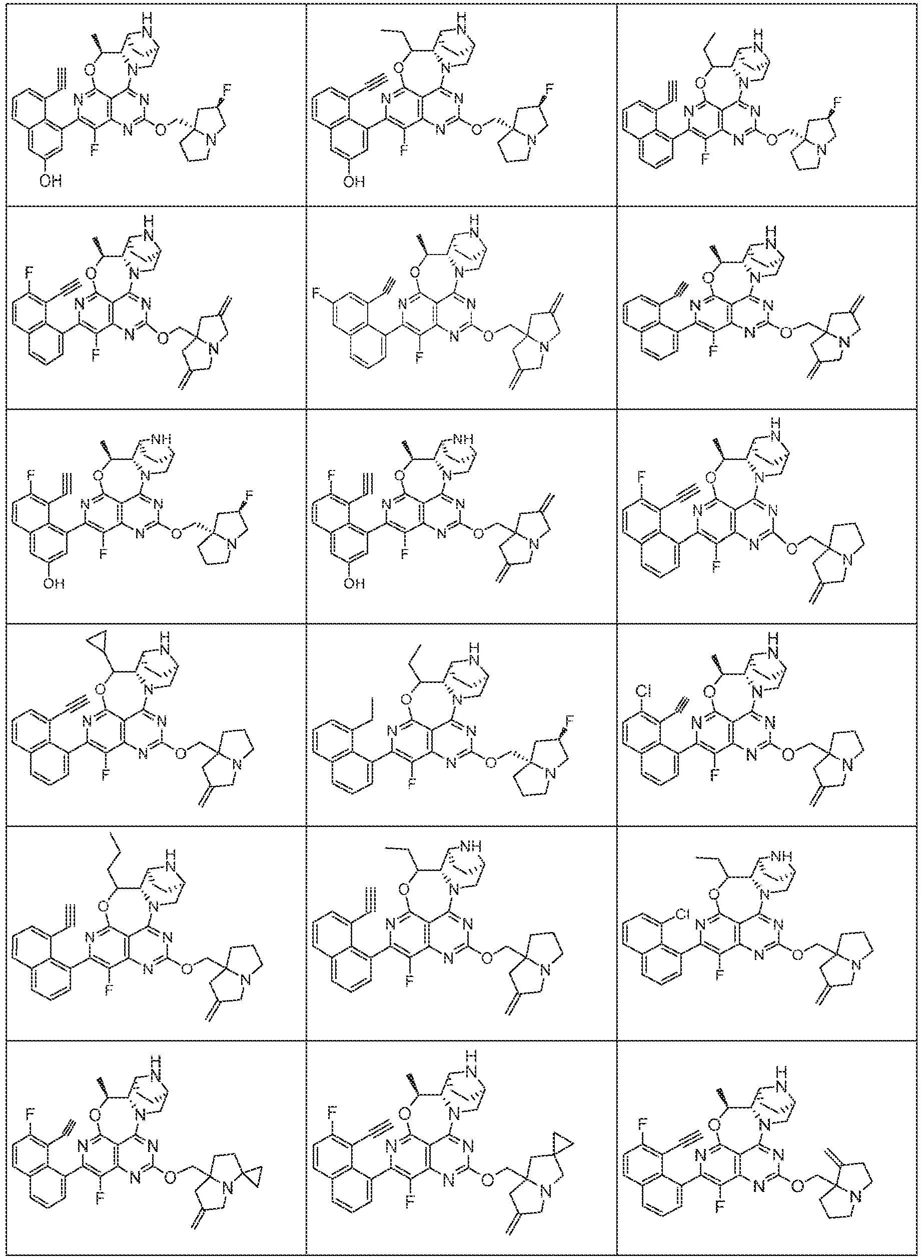

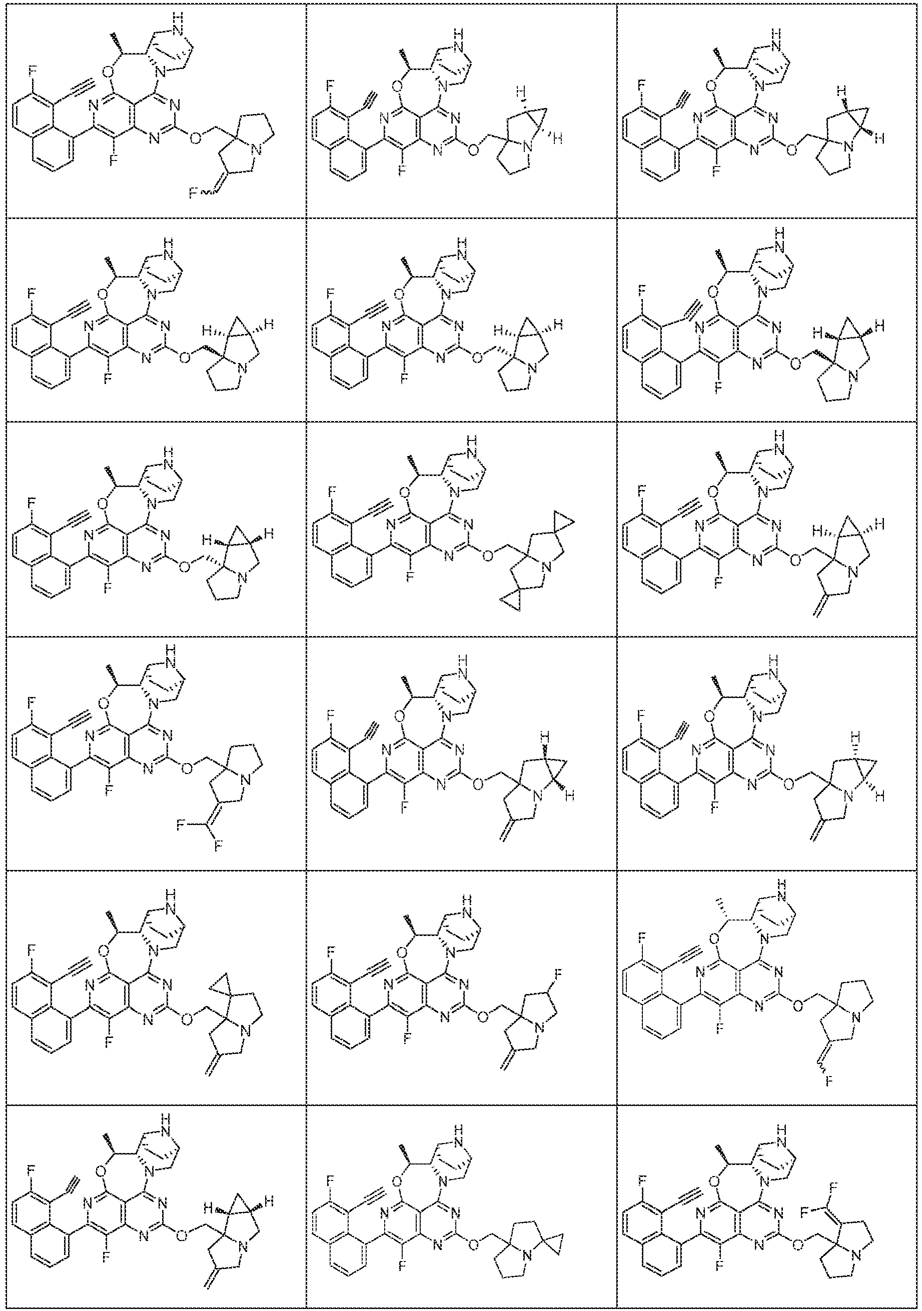

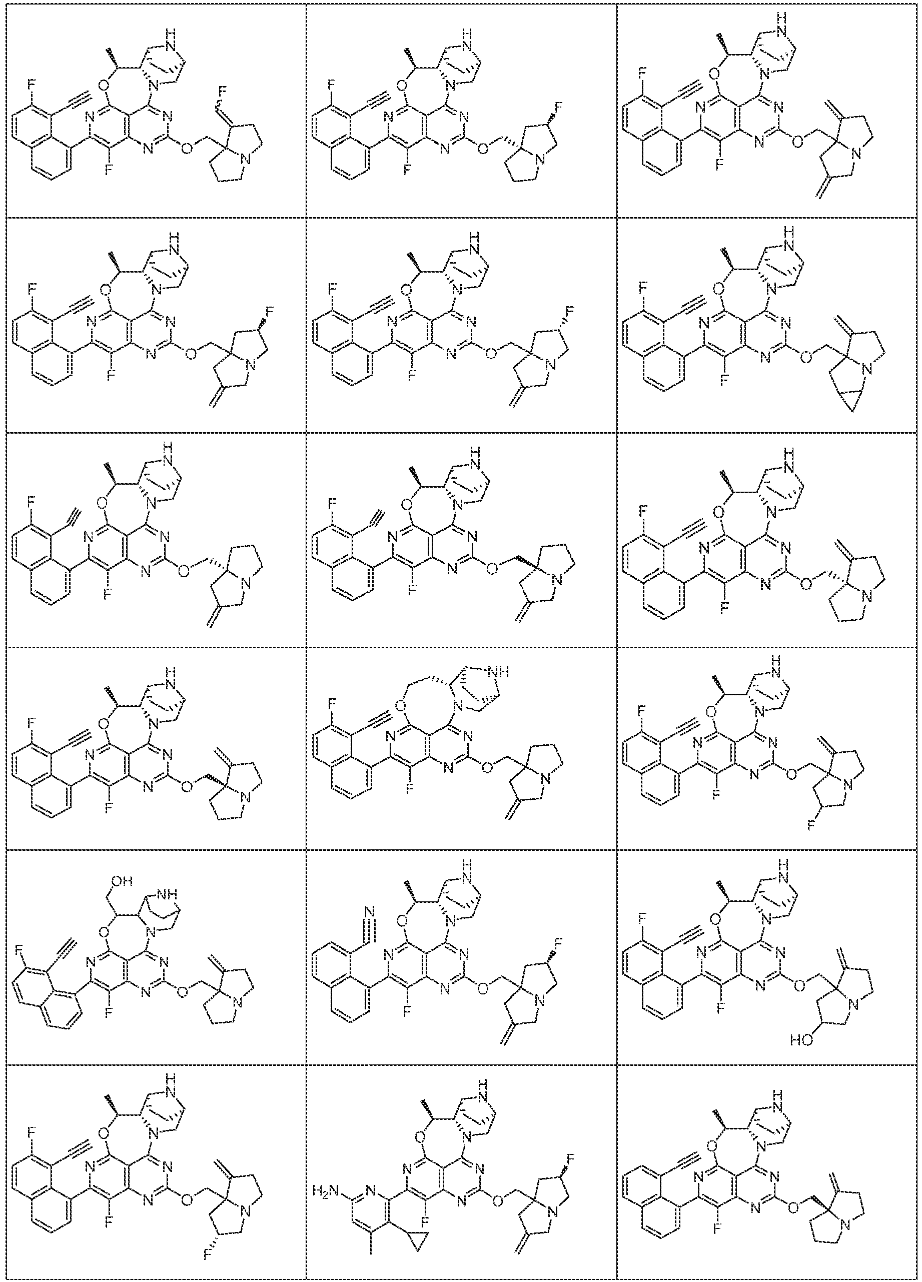

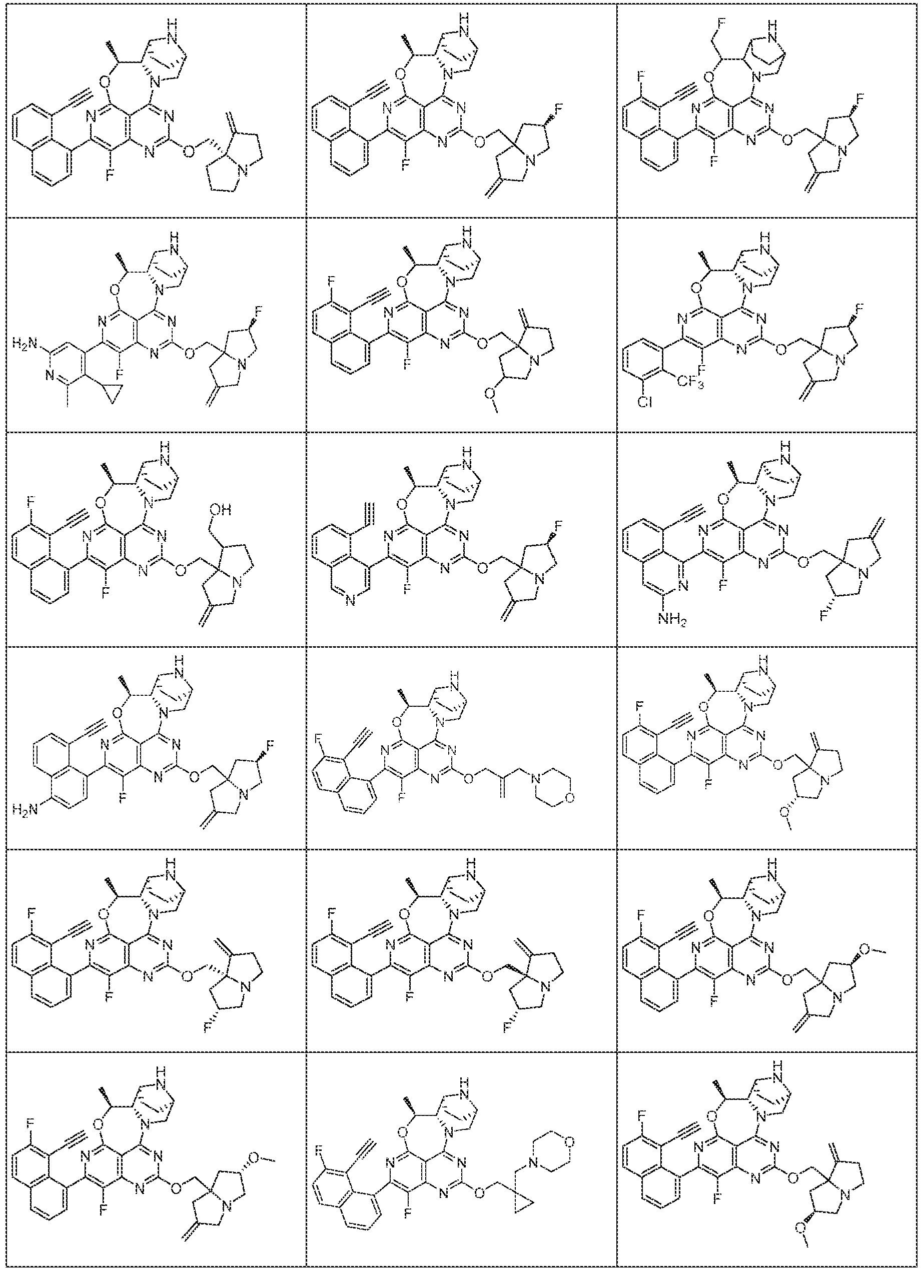

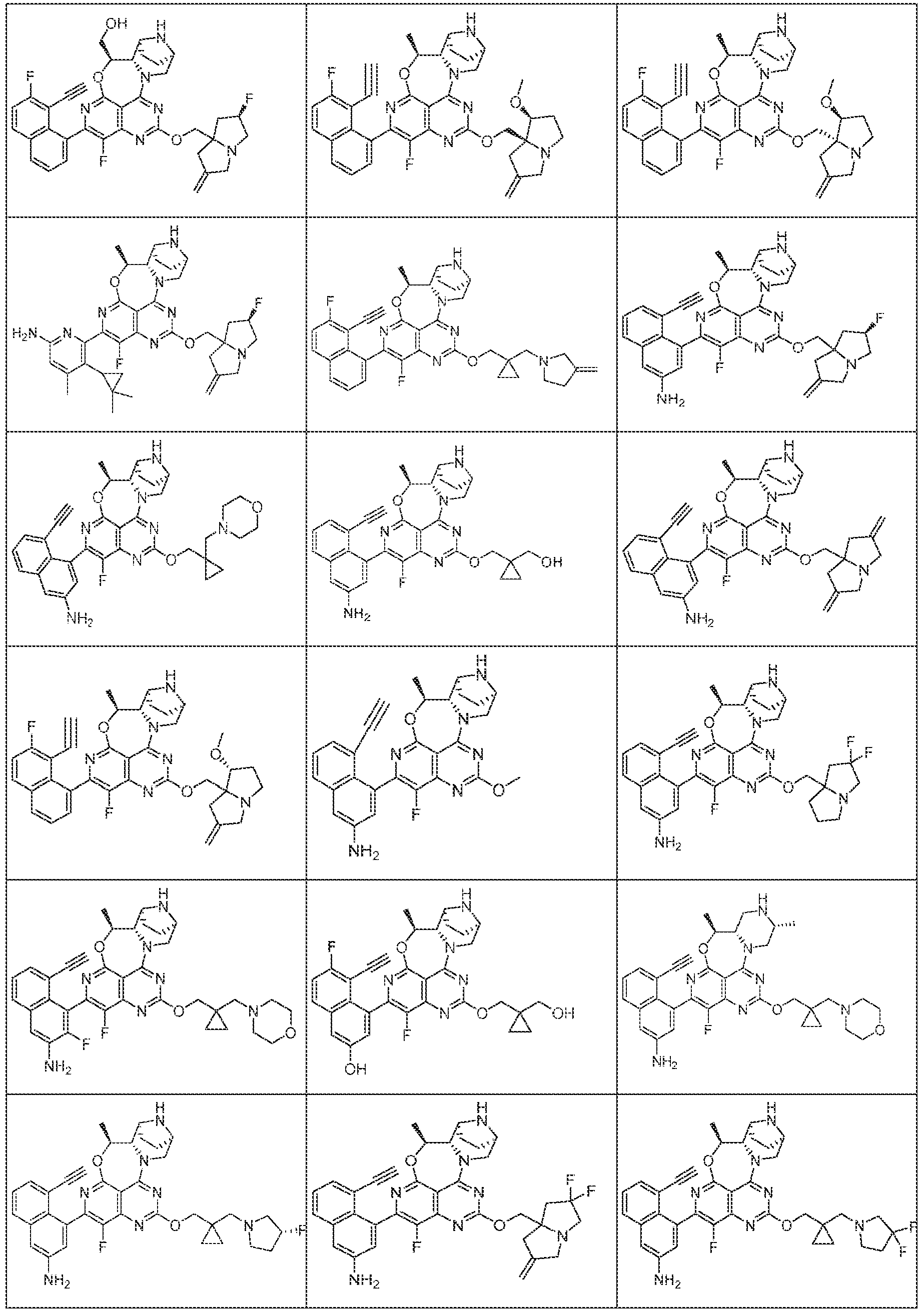

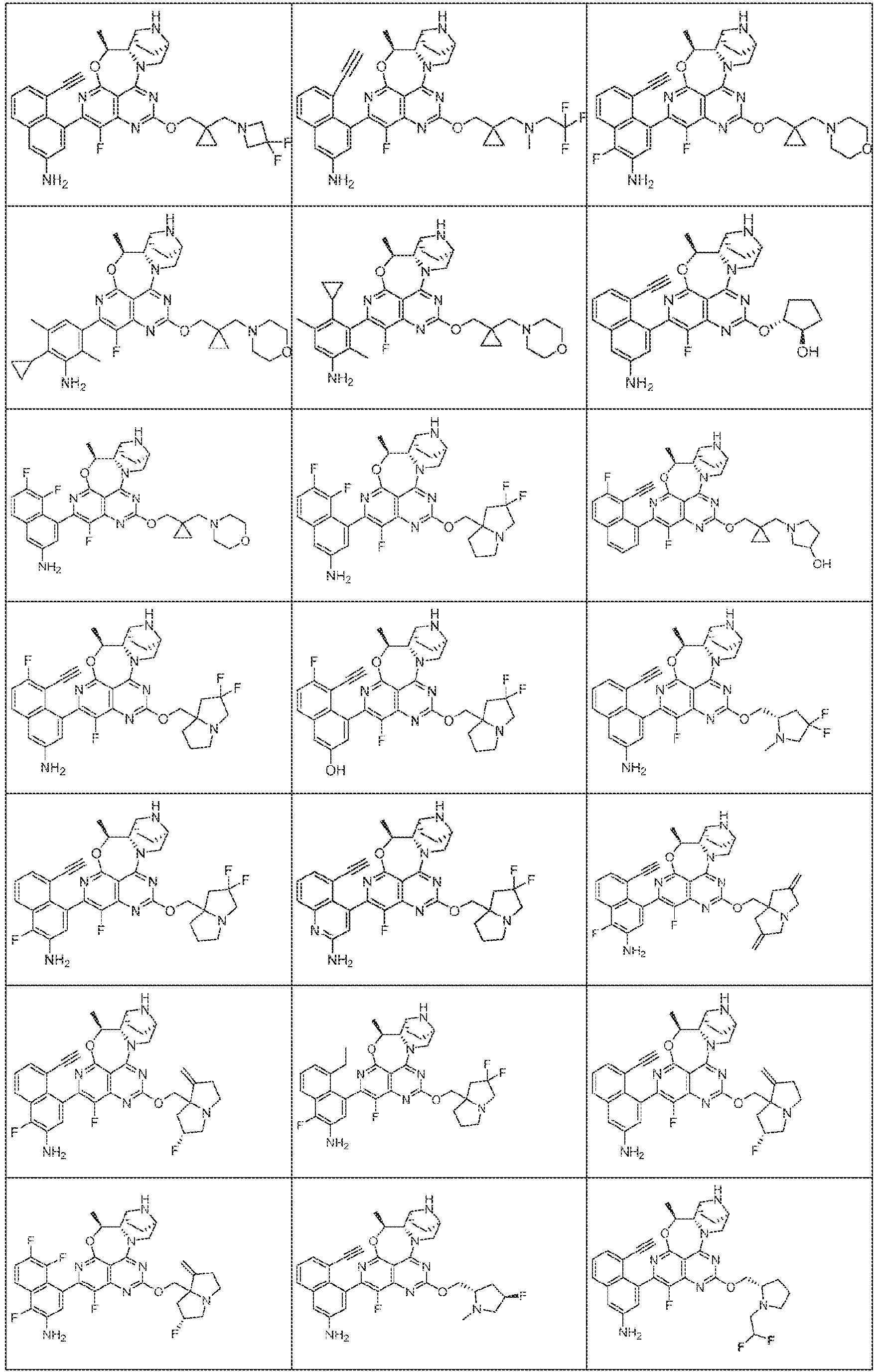

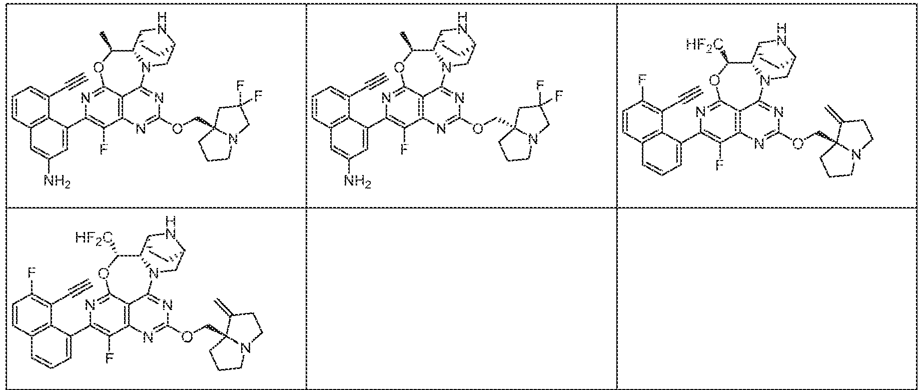

In a second aspect, the present disclosure provides a pharmaceutical composition including the compound, or a pharmaceutically acceptable salt, a stereoisomer or a solvate thereof according to the first aspect above; and a pharmaceutically acceptable carrier.

As used herein, the term "pharmaceutically acceptable carrier" refers to any preparation capable of delivering an effective amount of the active ingredient of the present disclosure without interfering with the biological activity of the active ingredient and without toxic and side effects to a host or subject, or a carrier representative of carrier media, including water, oils, vegetables and minerals, cream bases, lotion bases, ointment bases, etc. Such bases include suspending agents, viscosity enhancers, transdermal enhancers, etc. Their preparations are well known to those skilled in the fields of cosmetics or topical pharmaceutical agents.

In embodiments of the present disclosure, the pharmaceutical composition may be administered in any way selected from: orally, spray inhalation, rectally, nasally, buccally, topically, parenterally, such as subcutaneous, intravenous, intramuscular, intraperitoneal, intrathecal, intraventricular, intrasternal and intracranial injection or infusion, or with the aid of an explanted reservoir. Oral administration, intraperitoneal administration or intravenous administration are preferred. When administered orally, the compounds of the present disclosure may be prepared in any orally acceptable preparation, including but not limited to, tablets, capsules, aqueous solutions or aqueous suspensions. Carriers for tablets generally include lactose and corn starch. In addition, lubricants such as magnesium stearate may be added. Diluents used in capsule formulations typically include lactose and dried corn starch. Aqueous suspensions are generally prepared by mixing the active ingredients with suitable emulsifiers and suspending agents. If desired, some sweeteners, flavoring agents or colorants may also be added to the above-mentioned oral formulations, when administered topically, in particular when to affected surfaces or organs that are readily accessible by topical application, such as eye, skin or lower intestinal neurological diseases, the compounds of the present disclosure may be can be prepared into different topical agents according to different affected surfaces or organs. When administered topically to eyes, the compounds of the present invention can be formulated into micronized suspensions or solutions with isotonic sterile salines at a certain pH with or without the addition of preservatives such as benzyl alkanol chlorides as carriers. For eye use, the compounds can also be prepared into ointments such as Vaseline ointments. When administered topically to the skin, the compounds of the present disclosure can be prepared into suitable ointment, lotion or cream formulations, with the active ingredients being suspended or dissolved in one or more carriers. Carriers that can be used in ointment formulations include, but are not limited to mineral oils, liquid Vaseline, white Vaseline, propylene glycol, polyethylene oxide, polypropylene oxide, emulsified wax, and water. Carriers that can be used in lotions or creams include but are not limited to mineral oils, sorbitan monostearate, Tween 60, cetyl esters wax, 2-octyldodecan-1-ol, 2-octyldodecanol, benzyl alcohol, and water. The compounds of the present invention may also be administered in the form of sterile injections, including sterile injectable water or oil suspensions or sterile injectable solutions. Carriers and solvents that can be used include water, Ringer's solution, and isotonic sodium chloride solution. In addition, sterile non-volatile oils can also be used as solvents or suspending media, e.g., monoglycerides or diglycerides.

Another aspect of the present disclosure provides use of the compound as described above, or the pharmaceutically acceptable salt, the stereoisomer or the solvate thereof according to the first aspect or the pharmaceutical composition according to the second aspect in preparation of medicaments for preventing and/or treating a disease or condition, the disease or condition is a KRAS G12D associated disease or disorder.

As used herein, "KRAS G12D" refers to a mutant type of a mammalian KRAS protein that contains an amino acid substitution of an aspartic acid for a glycine at amino acid position 12. The assignment of amino acid codon and residue positions for human KRAS is based on the amino acid sequence identified by UniProtKB/Swiss-Prot P01116: Variantp.Gly 1 2Asp.

As used herein, "KRAS G12D inhibitor" refers to a compound of the present disclosure, or a pharmaceutically acceptable salt, etc. These compounds can negatively regulate or inhibit all or part of the enzymatic activity of KRAS G12D.

As used herein, a "KRAS G12D associated disease or disorder" refers to a disease or disorder associated with or mediated by, or having a KRAS G12D mutation. A non-limiting example of a KRAS G12D associated disease or disorder is a KRAS G12D associated cancer.

Another aspect of the present disclosure provides a method for treating a KRAS G12D associated cancer comprising the steps of administering to a subject in need thereof a therapeutically effective amount of the compound or pharmaceutically acceptable salt, stereoisomer, or solvate thereof according to the first aspect of the present disclosure; or administering to a subject in need thereof a therapeutically effective amount of the pharmaceutical composition according to the second aspect of the present disclosure.

As herein, in an embodiment, the KRAS G12D associated cancer includes, but is not limited to, lung cancer, prostate cancer, breast cancer, brain cancer, skin cancer, cervical cancer, testicular cancer, etc. In an embodiment, the KRAS G12D associated cancer includes, but is not limited to, astrocytoma, breast cancer, cervical cancer, colorectal cancer, endometrial cancer, esophageal cancer, gastric cancer, head and neck cancer, hepatocellular cancer, laryngeal cancer, lung cancer, oral cancer, ovarian cancer, prostate cancer, thyroid cancer, sarcomas, etc. In an embodiment, the KRAS G12D associated cancer includes, but is not limited to, cancers of cardiac sites such as sarcomas (angiosarcomas, fibrosarcomas, rhabdomyosarcomas, liposarcomas), myxomas, rhabdomyomas, fibromas, lipomas, teratomas, etc; cancers of the lung, for example, bronchial carcinoma (squamous cell, undifferentiated small cell, undifferentiated large cell, adenocarcinoma), alveolar (bronchiolar) carcinoma, bronchial adenoma, sarcoma, lymphoma, chondroma hamartoma, mesothelioma; cancers of the gastrointestinal tract, for example, esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small intestine (adenocarcinoma, carcinoid tumors), kaposi's sarcoma, leiomyoma, hemangioma, lipoma, neurofibroma, fibroma), large intestine (adenocarcinoma, tubular adenoma, villous adenoma, hamartoma, leiomyoma); cancers of the genitourinary tract, for example, kidney (adenocarcinoma, wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratoma), choriocarcinoma, sarcoma, interstitial cell carcinoma, fibroma, fibroadenoma, adenomatoid tumors, lipoma); cancers of the liver, for example liver cancer (hepatocellular carcinoma), cholangiocarcinoma, hepatoblastoma, angiosarcoma, hepatocellular adenoma, hemangioma; biliary: gallbladder cancer, ampulla cancer, bile duct cancer; cancers of bone sites, e.g., osteogenic sarcoma (osteosarcoma), fibrosarcoma, malignant fibrous histiocytoma, chondrosarcoma, ewing's sarcoma, malignant lymphoma (reticulum cell sarcoma), multiple myeloma, malignant giant cell tumor, chordoma, osteochondroma (osteochondral chondroma), benign chondroma, benign chondromatous osteoma, and giant cell tumor; cancers of the nervous system, for example, skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma, glioblastoma multiforme, glioblastoma, congenital tumors), spinal cord neurofibroma, meningioma, glioma, sarcoma); gynecological cancers, for example, uterus (endometrial carcinoma), cervix (cervical carcinoma, precancerous cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); hematologic cancers, e.g., blood (myeloid leukemia (acute) and chronic), acute lymphocytic leukemia, chronic lymphocytic leukemia, myeloproliferative diseases, multiple myeloma, myelodysplastic syndrome), hodgkin's disease, non-Hodgkin' s lymphoma (malignant lymphoma); cancers of the skin site, for example, malignant melanoma, basal cell carcinoma, squamous cell carcinoma, kaposi's sarcoma, moles dysplastic nevi, lipoma, hemangioma, dermatofibroma, keloids, psoriasis; and cancers of the adrenal sites, e.g., neuroblastoma; etc.

In an embodiment, the KRAS G12D associated cancer is lung cancer.

In an embodiment, the KRAS G12D associated cancer is selected from the group: non-small cell lung cancer, small cell lung cancer, colorectal cancer, rectal cancer, or pancreatic cancer.

Another aspect of the present disclosure provides a method for treating cancer in a subject in need thereof, the method including:
(a) determining that the cancer is associated with a KRAS G12D mutation (e.g., a KRAS G12D associated cancer); and
(b) administering to the subject a therapeutically effective amount of the compound, or a pharmaceutically acceptable salt, stereoisomer or solvate thereof according to the first aspect of the present disclosure; or administering to the subject a therapeutically effective amount of the pharmaceutical composition according to the second aspect of the present disclosure.

In an embodiment, the administration is accomplished by a route selected from: parenteral, intraperitoneal, intradermal, intracardiac, intraventricular, intracranial, intracerebrospinal, intraluminal, intrasynovial, intrathecal, intramuscular injection, intravitreal injection, intravenous injection, intraarterial injection, oral, buccal, sublingual, transdermal, topical, intratracheal, rectal, subcutaneous and topical administrations.

Another aspect of the present disclosure provides a method for inhibiting the activity of KRAS G12D in a cell, including the steps of contacting the cell with the compound, or a pharmaceutically acceptable salt, a stereoisomer or a solvate thereof according to the first aspect of the present disclosure; or contacting the cell with the pharmaceutical composition according to the second aspect of the present disclosure.

Another aspect of the present disclosure provides use of the compound, or a pharmaceutically acceptable salt, a stereoisomer or a solvate thereof according to the first aspect described above, or the pharmaceutical composition according to the second aspect described above, in preparation of an KRAS G12D inhibitor.

As used herein, the term "subject" refers to an animal, particularly a mammal, perferably human.

As used herein, the term "effective amount" or "therapeutically effective amount" refers to a sufficient amount of a non-toxic drug or medicament that can achieve the expected effect. In embodiments of the present disclosure, then treating a patient according to the present invention, the amount of a drug is given depending on many factors, such as the specific dosage regimen, the disease or condition type and its severity, and the peculiarity (e.g., body weight) of the subject or host in need of treatment.. However, in accordance with the particular ambient conditions, including, for example, the adopted specific drug and administration route, the treatment condition, and the treated subject or host, the administered dosage may be conventionally determined by the known method in the art. Usually, in terms of dosages used for treating an adult, the administered dosage is typically in a range of 0.02-5000 mg/day, for example, about 1-1500 mg/day. The desired dose can be conveniently shown as a single dose, or divided doses administered simultaneously (or in a short time) or at appropriate intervals, for example, two, three, four or more divided doses per day. It will be understood by those skilled in the art that, although the above-mentioned dosage range was given, the specific effective amount may be appropriately adjusted according to the patient's condition in conjunction with the physician's diagnosis.

As used herein, the term "pharmaceutically acceptable salt" refers to a salt of a compound of the present disclosure that is pharmaceutically acceptable and has the pharmacological activity of the parent compound. Such salts include: acid addictive salts formed with inorganic acids such as nitric acid, phosphoric acid, carbonic acid, etc., or organic acids, such as propionic acid, hexanoic acid, cyclopentanoic acid, glycolic acid, pyruvic acid, gluconic acid, stearic acid, muconic acid, etc., or salts formed by replacing acidic protrons present on the parent compound with metal ions, such as alkali metal ion or alkaline earth metal ion; or a coordination compound formed with organic bases such as ethanolamine, etc. The pharmaceutically acceptable salts of the present disclosure can be synthesized from the parent compound which contains acidic radical or basic base by conventional chemical methods. Generally, such salts are prepared by: reacting these compounds in the form of free acids or bases with a stoichiometric amount of an appropriate base or acid in water or an organic solvent or a mixture of the two. Generally, nonaqueous media such as ether, ethyl acetate, ethanol, isopropanol, or acetonitrile are preferred. In addition to the form of salts, the compounds provided herein exist in the form of prodrug. Prodrugs of the compounds described herein readily undergo chemical changes under physiological conditions and thus transformed into the compounds of the present disclosure. In addition, prodrugs can be converted to the compounds of the present disclosure by chemical or biochemical methods in an in vivo environment.

As used herein, the term "solvate" refers to a substance formed by a compound of the present invention combined with a pharmaceutically acceptable solvent. Pharmaceutically acceptable solvents include acetic acid, etc. Solvates include stoichiometric solvates and non-stoichiometric solvates. Some compounds of the present invention can be present in a non-solvated or solvated form. In general, the solvated form is equivalent to unsolvated form and is intended to be encompassed within the scope of the present disclosure.

As used herein, the term "stereoisomer" includes conformational isomers and configurational isomers, wherein the configurational isomers primarily include cistrans isomers and optical isomers. The compounds described herein may be present in the form of stereoisomers, and thus encompass all possible stereoisomeric forms, including but not limited to cis-trans isomers, tautomers, enantiomers, diastereomers, atropisomers, etc., as well as any combination or any mixture of the aforementioned stereoisomers, such as meso-forms, racemates, equal mixtures of atropisomers, etc. The compounds of the present disclosure may also be present in the form of any combination or any mixture of the aforementioned stereisomers, for example, a mixture of equal amounts of a mesomer, a raceme, and an atropisomer, for example, a single enantiomer, a single diastereomer or a mixture of them, or a single atropisomer or a mixture thereof. When the compounds described herein contain olefinic double bonds, unless specified otherwise, they include cis-isomers, trans-isomers and any combination thereof. Atropisomers of the present disclosure are stereoisomers based on axial or planar chirality resulting from restricted intramolecular rotation. As a drug, a stereoisomer having excellent activity is preferable. The compounds of the present disclosure have optical isomers derived from asymmetric carbon, etc., and if necessary, a single isomer can be resolved by methods known in the art, for example, crystallization or chiral chromatography, etc.

As herein, two hydrogen atoms on the same carbon atom are optionally and simultaneously substituted by =CR^{2a}R^{2b} refers to that the carbon atom is connected to the carbon atom (C) in CR^{2a}R^{2b} through a double bond. That is, the two hydrogen atoms on the same carbon atom are optionally and simultaneously substituted by =CR^{2a}R^{2b} refers to the carbon atom is replaced by .

As used herein, the term "C1-C6 alkyl" refers to a straight and branched aliphatic group consisting of 1 to 6 carbon atoms, preferably, C1-C4 alkyl or C1-C3 alkyl. Examples of alkyl include, but are not limited to, methyl, ethyl, propyl, isopropyl, butyl, isobutyl, sec-butyl, tert-butyl, pentyl, and hexyl.

As used herein, the term "C1-C6 haloalkyl" refers to C1-C6 alkyl in which one or more hydrogen are substituted by halogen. Wherein the definition of alkyl is defined as above. Preferably, the C1-C6 haloalkyl is C1-C3 halogenated alkyl or C1-C4 halogenated alkyl. Examples of halogenated alkyl include, but are not limited to, trifluoromethyl, difluoromethyl, and monofluoromethyl.

As used herein, the term "C1-C6 deuterated alkyl" refers to C1-C6 alkyl in which one or more hydrogen are substituted by a deuterium atom. Wherein the definition of alkyl is defined as above. Preferably, the C1-C6 deuterated alkyl is C1-C3 deuterated alkyl or C1-C4 deuterated alkyl. Examples of halogenated alkyl include, but are not limited to, trifluoromethyl, difluoromethyl, and monofluoromethyl.

As used herein, the term "C1-C6 alkoxy" refers to -O-C1-C6 alkyl. Preferably, the C1-C6 deuterated alkyl is C1-C3 alkoxy or C1-C4 alkoxy. Wherein the definition of alkyl is defined as above.

As used herein, the term "C1-C6 halogenated alkoxy" refers to O-C1-C6 halogenated alkyl, preferably, C1-C3 halogenated alkoxy or C1-C4 halogenated alkoxy. Wherein the definition of halogenated alkyl is defined as above.

As used herein, the term "C1-C6 deuterated alkoxy" refers to O-C1-C6 deuterated alkyl. Preferably, the C1-C6 deuterated alkoxy is C1-C3 deuterated alkoxy or C1-C4 deuterated alkoxy. Wherein the definition of deuterated alkyl is defined as above.

As used herein, the term "C1-C6 hydroxyalkyl" refers to -C1-C6 alkyl-hydroxyl (OH). Preferably, the C1-C6 hydroxyalky is C1-C3 hydroxyalkyl (i.e. -C1-C3 alkyl-hydroxy) or C1-C4 hydroxyalkyl (i.e. -C1-C4 alkyl-hydroxy). Wherein the definition of alkyl is defined as above.

As used herein, the term "C1-C6 cyanoalkyl" refers to -C1-C6 alkyl-cyano. Preferably, the C1-C6 cyanoalkyl is C1-C3 cyanoalkyl or C1-C4 cyanoalkyl. The defintion of alkyl is defined as above. As used herein, the term "C2-C6 alkynyl" refers to a straight and branched aliphatic group consisting of 2-6 carbon atoms containing 1 or 2 carbon-carbon triple bonds, preferably, C2-C4-alkynyl or C2-C3-alkynyl. Examples of alkynyl include, but are not limited to, ethynyl, propynyl, butynyl, etc. As used herein, the term "C2-C6 halogenated alkynyl" refers to C2-C6 alkynyl in which one or more hydrogen are substituted by halogen. Wherein the definition of alkynyl is defined as above, preferably, C2-C3 halogenated alkynyl or C2-C4 halogenated alkynyl. Examples of halogenated alkynyl include, but are not limited to, monofluoroethynyl, difluoroethynyl.

As used herein, the term "C2-C6 alkenyl" refers to straight and branched aliphatic groups consisting of 2-6 carbon atoms having 1 or 2 carbon-carbon double bonds, preferably, C2-C4 alkenyl or C2-C3 alkenyl. Examples of alkenyl include, but are not limited to, ethenyl, propenyl, butenyl, etc.

As used herein, the term "C2-C6 halogenated alkenyl" refers to C2-C6 alkenyl in which one or more hydrogen are substituted by halogen. Wherein the definition of alkynyl is defined as above, preferably, C2-C3 halogenated alkenyl or C2-C4 halogenated alkenyl. Examples of halogenated alkenyl include, but are not limited to, monofluorovinyl, difluorovinyl.

As used herein, the term "C2-C6 hydroxyalkynyl" refers to-C2-C6 alkynyl-OH. Wherein alkynyl is defined as above, preferably, C2-C4 hydroxyalkynyl or C2-C3 hydroxyalkynyl.

As used herein, the term "C2-C6 hydroxyalkenyl" refers to -C2-C6 alkenyl-OH. Wherein alkenyl is defined as above, preferably, C2-C4 hydroxyalkenyl or C2-C3 hydroxyalkenyl.

As used herein, the term "C1-C4 alkylene" refers to C1-C4 alkyl, as defined above, that is positioned between and serves to connect two other portions of the molecule. Typical alkylene includes, but is not limited to, methylene, ethylene, propylene, and butylene.

As used herein, the terms "cycloalkyl" and "cycloalkyl ring" are used interchangeably to refer to saturated monocyclic or polycyclic hydrocarbyls, including, for example, monocyclic cycloalkyl, spiro cycloalkyl, fused cycloalkyl, and bridged cycloalkyl. In the present disclosure, the ring carbon atoms of the cycloalkyl may be optionally substituted by 1, 2 or 3 oxo groups to form a cyclic ketone structure. For example, the term "C3-C20 cycloalkyl" refers to cycloalkyl having 3 to 20 ring carbon atoms, including monocyclic cycloalkyl, spirocycloalkyl, fused cycloalkyl, and bridged cycloalkyl. Preferably C3-C12 cycloalkyl (more preferably C3-C8 monocyclic cycloalkyl), C5-C20 spirocycloalkyl, C5-C20 fused cycloalkyl or C5-C20 bridged cycloalkyl.

The term "C3-C8 monocyclic cycloalkyl" refers to saturated monocyclic hydrocarbyls having 3 to 8 ring carbon atoms. Preferably C3-C6 monocyclic cycloalkyl or C4-C6 monocyclic cycloalkyl, more preferably C3, C4, C5 or C6 monocyclic cycloalkyl. Specific examples of monocyclic cycloalkyl include, but are not limited to, cyclopropyl, cyclobutyl, cyclopentyl, cyclohexyl, cycloheptyl, cyclooctyl, etc.

As used herein, the terms "spirocycloalkyl" and "spirocycloalkyl ring" refer to polycyclic hydrocarbyls formed by sharing one carbon atom (referred to as a spiro atom) between two or more monocyclic rings. Spirocycloalkyl is classified as monospirocycloalkyl, dispirocycloalkyl and polyspirocycloalkyl according to the number of spiro atoms shared from ring to ring. The term "C5-C20 spirocycloalkyl" refers to polycyclic hydrocarbyls having 5 to 20 ring carbon atoms, wherein the monocyclic ring sharing a spiro atom is a C3-C8 monocyclic cycloalkyl ring; preferably, C6-C14 spirocycloalkyl; more preferably, C6-C14 mono spirocycloalky; further preferably C7-C11 spirocycloalkyl; more preferably, a 7- to 11-membered mono spirocycloalkyl; most preferably, C7 (C4 monocyclic cycloalkyl ring/C4 monocyclic cycloalkyl ring), C8 (C4 monocyclic cycloalkyl ring/C5 monocyclic cycloalkyl ring), C9 (C4 monocyclic cycloalkyl ring/C6 monocyclic cycloalkyl ring, C5 monocyclic cycloalkyl ring/C5 monocyclic cycloalkyl ring), C10 (C5 monocyclic cycloalkyl ring/C6 monocyclic cycloalkyl ring) or C11 (C6 monocyclic cycloalkyl ring/C6 monocyclic cycloalkyl ring) monocyclic spirocycloalkyl. Specific examples of spiro cycloalkyl include, but are not limited to: 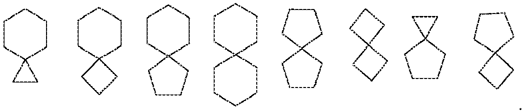 These spiro cycloalkyl may be attached to the rest of the molecule through any one of the ring atoms. As used herein, the terms "fused cycloalkyl" and "fused cycloalkyl ring" refer to two or more monocyclic hydrocarbyls formed by sharing a pair of adjacent carbon atoms. Bicyclic, tricyclic, tetracyclic or polycyclic fused cycloalkyl can be classified according to the number of rings formed. The term "C5-C20 fused cycloalkyl" refers to polycyclic hydrocarbyls having 5 to 20 ring carbon atoms, wherein the monocyclic ring sharing apair of adjacent carbon atoms is a C3-C8 monocyclic cycloalkyl ring, preferably C6-C14 fused cycloalkyl, more preferably C6-C14 doubly fused cycloalkyl, more preferably C7-C10 fused cycloalkyl, more preferably C7-C10 doubly fused cycloalkyl, most preferably C8 (C5 monocyclic cycloalkyl ring is fused to a C5 monocyclic cycloalkyl ring), C9 (C5 monocyclic cycloalkyl ring is fused to a C6 monocyclic cycloalkyl ring) or C10 (C6 monocyclic cycloalkyl ring is fused to a C6 monocyclic cycloalkyl ring) doubly fused cycloalkyl. Specific examples of fused cycloalkyl include, but are not limited to:

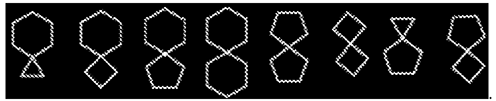

These fused cycloalkyl may be attached to the rest of the molecule through any one of the ring atoms. As used herein, the terms "bridged cycloalkyl" and "bridged cycloalkyl ring" refer to a polycyclic hydrocarbyl formed between two or more monocyclic rings by sharing two carbon atoms that are not directly connected. Bicyclic, tricyclic, tetracyclic or polycyclic bridged cycloalkyl classified according to the number of constituent rings. The term "C5-C20 bridged cycloalkyl" refers to a polycyclic hydrocarbyl having 5 to 20 ring carbon atoms in which any two rings share two carbon atoms that are not directly connected, preferably C6-C14 bridged cycloalkyl, more preferably C7-C10 bridged cycloalkyl. Specific examples of bridged cycloalkyl include, but are not limited to:

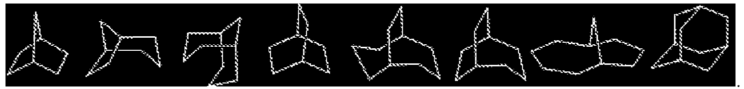

These bridged cycloalkyl may be attached to the rest of the molecule through any one of the ring atoms.

For example, examples of cycloalkyl herein include, but are not limited to, cyclopropyl, cyclobutyl, cyclopentyl, cyclopentenyl, cyclohexyl, cyclohexenyl, cycloheptyl, cyclooctyl, bicyclo[1.1.1]pentyl. As used herein, the term "cycloalkoxy" refers to an -O-cycloalkyl, wherein cycloalkyl is defined as above.

As used herein, the terms "heterocyclyl" and "heterocyclyl ring" are used interchangeably, and refer to saturated or partially unsaturated monocyclic or polycyclic hydrocarbyls, including, for example, monocyclic heterocyclyl, spiro heterocyclyl, fused heterocyclyl, and bridged heterocyclyl. Ring carbon atoms of the heterocyclyl described in the preset disclosure may be optionally substituted by 1, 2, or 3 oxo groups to form a structure of cyclic ketone, cyclic lactone, or cyclic lactam. For example, the term "3- to 20-membered heterocyclyl" refers to a saturated or partially unsaturated monocyclic or polycyclic hydrocarbyls having 3 to 20 ring atoms, wherein the one or more (preferably 1, 2, 3 or 4) ring atoms are heteroatoms selected from nitrogen, oxygen or S(=O)ₘ' (wherein m' is an integer of from 0 to 2), but excludes the ring moieties O-O-, -O-S- or -S-S-, the remaining ring atoms being carbon. When the ring atom is a nitrogen atom, it may be substituted or unsubstituted (i.e. N or NR, R being hydrogen or a further substituent as already defined herein). The 3- to 20-membered heterocyclyl described in the present disclosure includes a monocyclic heterocyclic (e.g., a 3- to 8-membered monocyclic heterocyclyl), a 5- to 20-membered spiro heterocyclyl, a 5- to 20-membered fused heterocyclyl and a 5- to 20-membered bridged heterocyclyl. As used herein, the term "3- to 20-membered nitrogen-containing heterocyclyl" refers to a saturated or partially unsaturated monocyclic hydrocarbyl having 3 to 20 ring atoms containing at least 1 nitrogen atom, wherein the group is attached to the rest of the molecule through the nitrogen atom; and the group optionally also contains one or two other heteroatoms selected from nitrogen, oxygen or S(=O)ₘ' and used as ring atoms, wherein m' is an integer of from 0 to 2. The 3- to 20-membered nitrogen-containing heterocyclyl described herein includes a monocyclic heterocyclyl (e.g., a 3- to 8-membered monocyclic nitrogen-containing heterocyclyl), a 5- to 20-membered nitrogen-containing spiro heterocyclyl, a 5- to 20-membered nitrogen-containing fused heterocyclyl, and a 5-to 20-membered nitrogen-containing bridged heterocyclyl.

As used herein, the terms "3- to 8-membered monocyclic heterocyclyl" and "3- to 8-membered monocyclic heterocyclyl ring" refer to a saturated or partially unsaturated monocyclic hydrocarbyl having 3 to 8 ring atoms, wherein 1, 2 or 3 are heteroatoms selected from nitrogen, oxygen or S(=O)ₘ' , wherein m' is an integer of from 0 to 2, preferably 3- to 6-membered monocyclic heterocyclyl having 3 to 6 ring atoms, wherein 1 or 2 are heteroatoms, more preferably 4 to 6 membered monocyclic heterocyclyl having 4 to 6 ring atoms, wherein 1 or 2 are heteroatoms, more preferably 5- or 6-membered monocyclic heterocyclyl having 5 or 6 ring atoms, wherein 1 or 2 are heteroatoms. When the heteroatom is a nitrogen atom, the nitrogen atom may be substituted or unsubstituted (i.e. N or NR, R being hydrogen or a further substituent as already defined herein). When the heteroatom is a sulfur atom, the sulfur atom may be optionally oxidized (i.e. S(=O)ₘ', m' being an integer of from 0 to 2). Ring carbon atoms of the monocyclic heterocyclyls may be optionally substituted by 1, 2, or 3 oxo groups to form a cyclic ketone, cyclic lactone, or cyclic lactam structure. Specific examples of monocyclic heterocyclyls include, but are not limited to, aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrolidine, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholin-1,1-dioxide, tetrahydropyran, 1,2-dihydroazetidine, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4 (3H)-one, pyrimidin-4 (1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan -2,5-dione, 3,6-dihydropyridin-2(1H)-one, pyridine-2,6-(1H, 3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine etc.

As used herein, the term "3- to 6-membered nitrogen-containing heterocyclyl" refers to a saturated or partially unsaturated monocyclic hydrocarbyl having 3 to 6 ring atoms containing at least 1 nitrogen atom as a ring atom, wherein the group may be attached to other parts of the molecule either through the nitrogen atom or through other ring atoms of the group; and the group optionally also contains one or two other heteroatoms selected from nitrogen, oxygen or S(=O)ₘ' and used as ring atoms, wherein m' is an integer of from 0 to 2. Specific examples may be selected from 3 to 8 membered monocyclic heterocyclyls including, but not limited to, tetrahydropyrrolyl, oxazolidinyl, isoxazolidinyl, piperidinyl, piperazinyl, morpholinyl, thiomorpholinyl, azetidinyl etc.

As used herein, the terms "spiroheterocyclyl" and "spiroheterocyclyl ring" refer to polycyclic heterocyclyls formed by sharing one carbon atom (referred to as a spiro atom) between two or more saturated or partially unsaturated monocyclic rings, wherein one or more (e.g., 1, 2, or 3) ring atoms are heteroatoms selected from nitrogen, oxygen, or S(=O)ₘ' (wherein m' is an integer of from 0 to 2) and the remaining ring atoms are carbon. When the heteroatom is a nitrogen atom, the nitrogen atom may be substituted or unsubstituted (i.e. N or NR, R being hydrogen or a further substituent as already defined herein). Each single ring may contain one or more double bonds, but none of the rings have a completely conjugated π-electron system. Spiroheterocyclyls are classified as mono-, bi- or poly-spiroheterocyclyls depending on the number of spiro atoms shared between the rings. The term "5- to 20-membered spiroheterocyclyl" refers to a spiroheterocyclyl having 5 to 20 ring atoms, wherein one of the monocyclic rings that share a spiro atom is a 3- to 8-membered monocyclic heterocyclyl ring and the other monocyclic ring is a 3- to 8-membered monocyclic heterocyclyl ring or a 3- to 8-membered monocyclic cycloalkyl ring, preferably 6- to 14-membered spiro heterocyclyls having 6 to 14 ring atoms, wherein 1 or 2 are heteroatoms, more preferably 7- to 11-membered spiro heterocyclyls having 7 to 11 ring atoms, wherein 1 or 2 are heteroatoms, most preferably 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring) or 11-membered (6-membered monocyclic heterocyclyl ring/6-membered monocyclic heterocyclyl ring) mono-spiro heterocyclyl. Specific examples of spiroheterocyclyls include, but are not limited to:

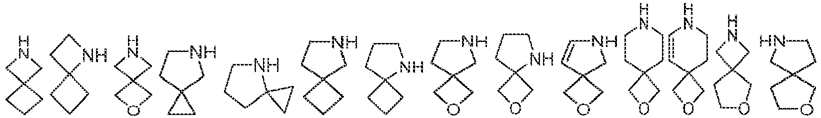

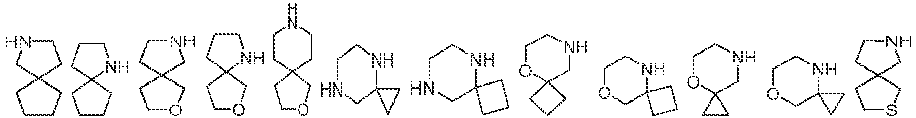

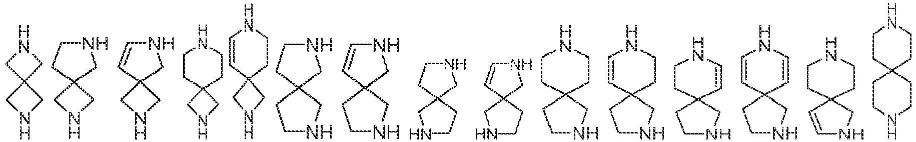

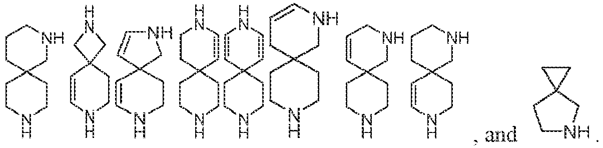

These spiroheterocyclyls may be attached to the rest of the molecule through any suitable ring atom. As used herein, the term "5- to 20-membered nitrogen-containing spiroheterocyclyl" refers to a spiroheterocyclyl having 5 to 20 ring atoms and containing at least 1 nitrogen atom, wherein the group is attached to the rest of the molecule through the nitrogen atom; and the group optionally also containing one or two or three further heteroatoms selected from nitrogen, oxygen or S(=O)ₘ' as ring atoms, wherein m' is an integer of from 0 to 2.

As used herein, the terms "fused heterocyclyl" and "fused heterocyclyl ring" refer to a polycyclic heterocyclyl in which two or more saturated or partially unsaturated monocyclic rings are formed by sharing a pair of adjacent ring atoms, wherein one or more (e.g., 1, 2, or 3) ring atoms are heteroatoms selected from nitrogen, oxygen, or S(=O)ₘ' (wherein m' is an integer of from 0 to 2), the remaining ring atoms being carbon. When the heteroatom is a nitrogen atom, the nitrogen atom may be substituted or unsubstituted (i.e. N or NR, R being hydrogen or a further substituent as already defined herein). Each single ring may contain one or more double bonds, but none of the rings have a completely conjugated π-electron system. The shared a pair of adjacent ring atoms may be C-C or N-C. Bicyclic, tricyclic, tetracyclic or polycyclic fused heterocyclyl can be classified according to the number of constituent rings. The term "5- to 20-membered fused heterocyclyl" refers to fused heterocyclyl having 5 to 20 ring atoms, wherein the single ring sharing a pair of adjacent ring atoms is a 3- to 8-membered monocyclic heterocyclyl ring, preferably 6- to 14-membered fused heterocyclyl having 6 to 14 ring atoms, wherein 1 or 2 are heteroatoms, more preferably 6- to 10-membered fused heterocyclyl having 6 to 10 ring atoms, wherein 1 or 2 are heteroatoms; , more preferably 8- to 10-membered fused heterocyclyl having 8 to 10 ring atoms, wherein1 or 2 are heteroatoms, most preferably 8-membered (a 5-membered monocyclic heterocyclyl ring fused to a 5-membered monocyclic heterocyclyl ring), 9-membered (a 5-membered monocyclic heterocyclyl ring fused to a 6-membered monocyclic heterocyclyl ring) or 10-membered (a 6-membered monocyclic heterocyclyl ring fused to a 6-membered monocyclic heterocyclyl ring) bicyclic fused heterocyclyl. Specific examples of fused heterocyclyl groups include, but are not limited to:

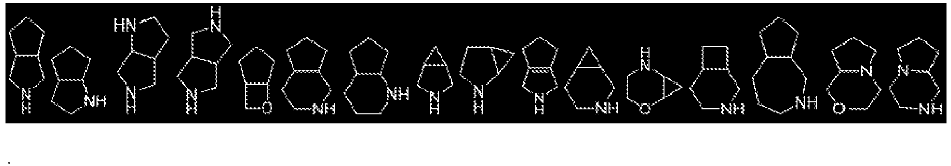

These fused heterocyclic groups may be attached to the rest of the molecule through any suitable ring atom.

As used herein, the term "5- to 20-membered nitrogen-containing fused heterocyclyl" refers to a fused heterocyclyl having 5 to 20 ring atoms and containing at least 1 nitrogen atom, wherein the group is attached to the rest of the molecule through the nitrogen atom; and the group optionally also contains one or two or three further heteroatoms selected from nitrogen, oxygen or S(=O)ₘ' as ring atoms, wherein m' is an integer of from 0 to 2.

As used herein, the terms "bridged heterocyclyl" and "bridged heterocyclyl ring" refer to a polycyclic heterocyclyl in which two or more saturated or partially unsaturated monocyclic rings are formed by sharing two ring atoms that are not directly connected, wherein one or more (e.g., 1, 2, or 3) ring atoms are heteroatoms selected from nitrogen, oxygen, or S(=O)ₘ' (wherein m' is an integer of from 0 to 2) and the remaining ring atoms are carbon. Bicyclic, tricyclic, tetracyclic or polycyclic bridged cycloalkyl classified according to the number of constituent rings. The term "5-to 20-membered bridged heterocyclyl" refers to a saturated or partially unsaturated polycyclic heterocyclic groups having 5 to 20 ring atoms in which any two rings share two ring atoms that are not directly connected, each monocyclic ring may contain one or more double bonds, but no ring has a completely conjugated π-electron system, preferably 6- to 14-membered bridged heterocyclyl, more preferably 7- to 10-membered bridged heterocyclyl. Specific examples of bridged heterocyclyl include, but are not limited to:

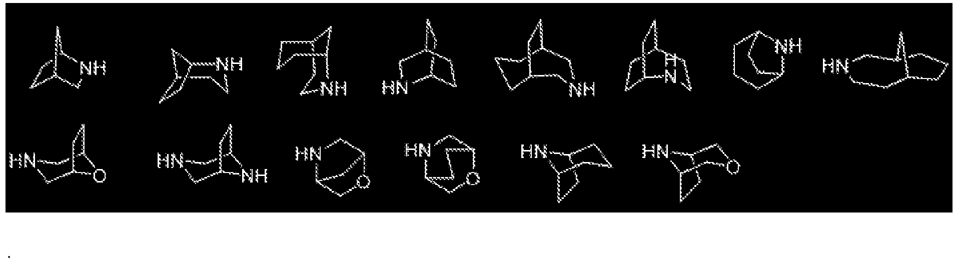

These bridged heterocyclyl may be attached to the rest of the molecule through any suitable ring atom.

As used herein, the term "5- to 20-membered nitrogen-containing bridged heterocyclyl" refers to a bridged heterocyclyl having 5 to 20 ring atoms containing at least 1 nitrogen atom, wherein the group is attached to the rest of the molecule through the nitrogen atom; and the group optionally also contains as ring atoms one or two or three further heteroatoms selected from nitrogen, oxygen or S(=O)ₘ', wherein m' is an integer of from 0 to 2.

In the present disclosure, each of the heterocyclyl above may be optionally substituted, and when substituted, the substituent is preferably one or more of the substituents described in the present disclosure.

Specifically, specific examples of heterocyclyl described herein include, but are not limited to, epoxyl, azetidinyl, tetrahydrofuranyl, tetrahydropyranyl, pyrrolidinyl, pyrrolidonyl, piperidinyl, piperazinyl, imidazolyl, imidazopyridinyl, thiazolyl, oxazolidinyl, oxazolidinedionyl, decahydroquinolinyl, piperidonyl, morphinyl, azabicyclohexyl, azabicycloheptyl, azabicyclooctyl, azabicyclononyl, azabicyclodecyl, azaspiroheptyl, azaspirooctyl, azaspirononyl, azaspirodecyl, tetrahydrospiro[cyclopropane-1,2'-pyrrolizinyl, hexahydro-1H-pyrrolizinyl, hexahydro-1H-pyrrolo[2,1-c][1,4]oxazinyl, octahydroindolizinyl, oxaspiroheptyl, oxaspirooctyl, oxaspirononyl, oxaspirodecyl, diazaspirononyl, oxabicyclohexyl, oxabicycloheptyl, oxabicyclooctyl, hexahydropyrrolizinyl 4(1H)-oxide.

As used herein, the term "aryl" refers to an all-carbon monocyclic, all-carbon non-fused polycyclic (rings being linked by a covalent bond, rather than fused), or all-carbon fused polycyclic (i.e. rings sharing a pair of adjacent carbon atoms) groups, wherein the group has at least aromatic ring, i.e., has a conjugated π-electron system. For example, the term "C6-C14 aryl" refers to aryls having 6 to 14 ring atoms, preferably C6-C10 aryl. The C6-C14 aryl in the present disclosure includes monocyclic aryl, non-fused polycyclic aryl and aromatic fused polycyclices, wherein examples of monocyclic aryl include phenyl, and examples of the non-fused polycyclic aryl include biphenyl, etc.

In the present disclosure, when the C6-C14 aryl is aromatic fused polycycles, the aromatic fused polycycles may be a polycyclic group formed by a monoaryl ring fused with one or more monoaryl rings, non-limiting examples thereof include naphthyl, anthryl, etc.

In some embodiments of the disclosure, when the C6-C14 aryl group is an aromatic fused polycycles, the aromatic fused polycycles may also be a polycyclic group formed by a monoaryl ring (e.g., phenyl) fused with one or more non-aromatic rings, wherein the ring linked to the parent structure is an aromatic ring or a non-aromatic ring. The non-aromatic ring includes, but is not limited to, 3-to 6-membered monocyclic heterocyclyl rings (preferably a 5- or 6-membered monocyclic heterocyclyl ring, wherein the ring carbon atoms may be substituted by 1 to 2 oxo groups to form a cyclic lactam or cyclic lactone structure), a 3- to 6-membered monocyclic cycloalkyl ring (preferably a 5- or 6-membered monocyclic cycloalkyl ring, wherein the ring carbon atoms may be substituted by 1 or 2 oxo groups to form a cyclic ketone structure). Polycycles in which a monoring is fused to one or more non-aromatic rings as described above may be attached to other groups or to the parent structure via a nitrogen or carbon atom, the ring to which the parent structure is attached being a monoaryl ring or a non-aromatic ring.

In the present disclosure, the above-mentioned kinds of aryls may be substituted or unsubstituted, and when substituted, the substituent is preferably one or more of the substituent groups described in the present disclosure.

As used herein, the term "aryl" refers to a C6-C14 aromatic radical comprising 1 to 3 aromatic rings. It may be optionally substituted by one or more (e.g., 1, 2, 3 4 or 5) R⁶ or one or more (e.g., 1, 2, 3 4 or 5) R⁷. In an embodiment, the aryl is C6-C10 aryl. Examples of aryl include, but are not limited to, phenyl, naphthyl, anthracenyl, fluorenyl, and dihydrobenzofuranyl. "Aryl" also refers to a bicyclic or tricyclic ring system wherein one or two rings in the aryl, respectively, may be saturated or partially saturated. If the aryl contains two saturated rings, the two saturated rings may be fused ring systems or spiro ring systems. Examples of aryl comprising two saturated rings (and being a spiro ring system) include the following:

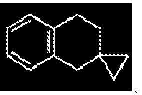

As used herein, the term "aryl C1-C6 alkyl" or "aralkyl" refers to include one aryl covalently linked to one alkyl. Wherein the aryl is defined as above and the alkyl is defined as above, and any or all of aryl or alkyl may be independently and optionally substituted or unsubstituted. An example of the aryl alkyl is (C6-C10) aryl (C1-C6) alkyl-. Specific examples include, but are not limited to, benzyl, phenethyl and naphthylmethyl. An example of a substituted aryl C1-C6 alkyl is wherein the alkyl is substituted by hydroxyalkyl.

As used herein, the term "heteroaryl" refers to a monocyclic or fused polycyclic (i.e. sharing adjacent pairs of ring atoms, which may be C-C or N-C) group wherein the nitrogen and sulfur atoms may optionally be oxidized and the nitrogen atom may optionally be quatemized, with ring atoms being substituted by at least one heteroatom independently selected from nitrogen, oxygen, or sulfur. The heteroaryl has 6, 10 or 14 π electrons shared, with at least one ring in the group being aromatic. For example, the term "5- to 14-membered heteroaryl" refers to a heteroaryl having 5 to 14 ring atoms, wherein 1, 2, 3 or 4 are heteroatoms selected from nitrogen, oxygen or S(=O)ₘ', wherein m' is an integer of from 0 to 2, preferably 5- to 10-membered heteroaryls having 5 to 10 ring atoms, wherein 1, 2, 3 or 4 are heteroatoms. In the present disclosure, the 5- to 10-membered heteroaryl is a monocyclic heteroaryl (e.g., a 5- or 6-membered monocyclic heteroaryl), a fused bicyclic heteroaryl (e.g., an 8- to 10-membered bicyclic heteroaryl), or a fused tricyclic heteroaryl.

As used herein, the term "5- or 6-membered monocyclic heteroaryl" refers to a monocyclic heteroaryl having 5 or 6 ring atoms, wherein 1, 2 or 3 are heteroatoms selected from nitrogen, oxygen or S(=O)ₘ', wherein m' is an integer of from 0 to 2. Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc.

As used herein, the term "8- to 10-membered bicyclic heteraryl" refers to a fused bicyclic heteroaryl having 8 to 10 ring atoms, wherein 1, 2, 3, 4 or 5 are heteroatoms selected from nitrogen, oxygen or S(=O)ₘ', wherein m' is an integer of from 0 to 2. The fused bicyclic heteroaryl can be either a bicyclic group (preferably a 9- or 10-membered bicyclic heteroaryl ring) formed by a monoaryl ring (e.g., phenyl) fused to a monocyclic heteroaryl ring (preferably a 5- or 6-membered monocyclic heteroaryl ring), or a bicyclic group formed by a monocyclic heteroaryl ring (preferably a 5- or 6-membered monocyclic heteroaryl ring) fused with a monocyclic heteroaryl ring (preferably a 5- or 6-membered monocyclic heteroaryl ring).

Any adjacent two ring atoms, including C-C, N-C, N-N, attached on the monocyclic heteroaryl ring described above can be fused to cycloalkyl, heterocyclyl, aryl, or heteroaryl, such as monocyclic cycloalkyl, monocyclic heterocyclyl, monocyclic aryl, 5- or 6-membered monocyclic heteroaryl ring, as defined herein, to form a fused polycyclic ring. The two ring atoms attached on the monocyclic heteroaryl ring forming a fused ring with other rings are preferably C-C, including, but not limited to, the following forms:

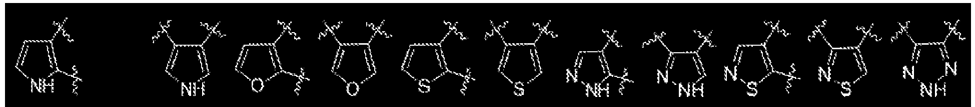

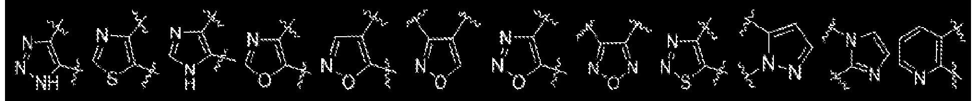

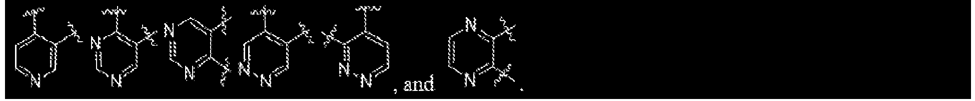

The groups described above are attached to the rest of the molecule through the ring atom identified with

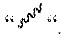

Non-limiting examples of 8- to 10-membered bicyclic heteroaryls include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, etc.

Specific examples of bicyclic heteroaryls include, but are not limited to: 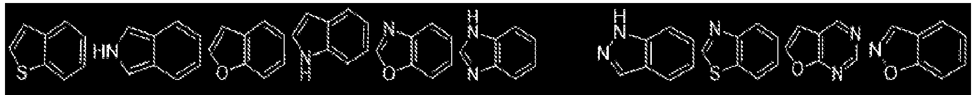 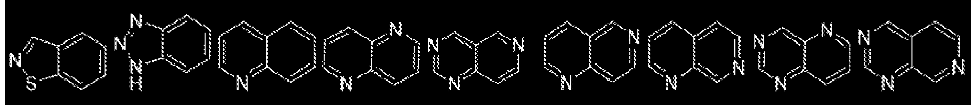 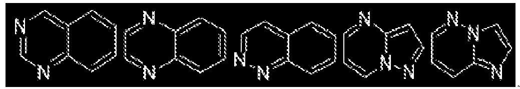 These groups may be attached to the rest of the molecule through any suitable ring atom. The ring linked to the parent structure can be a monocyclic heteroaryl ring or a phenyl ring.

In some embodiments of the present disclosure, the fused bicyclic heteroaryl or fused tricyclic heteroaryl may be a polycyclic group formed by a monocyclic heteroaryl ring (preferably a 5- or 6-membered monocyclic heteroaryl ring) fused with one or more non-aromatic rings, wherein the ring linked to the parent structure is a monocyclic heteroaryl ring or a non-aromatic ring. The non-aromatic ring includes, but not limited to, a 3- to 6-membered monocyclic heterocyclyl ring (preferably a 5- or 6-membered monocyclic heterocyclyl ring, wherein the ring carbon atoms may be substituted by 1 to 2 oxo groups to form a cyclic lactam or cyclic lactone structure), a 3- to 6-membered monocyclic cycloalkyl ring (preferably a 5- or 6-membered monocyclic cycloalkyl ring, wherein the ring carbon atoms may be substituted by 1 or 2 oxo groups to form a cyclic ketone structure) etc. Polycyclic ring formed by a monocyclic heteraryl ring fused to one or more non-aromatic rings as described above may be attached to other groups or to the parent structure via a nitrogen or carbon atom, the ring linked to the parent structure is a monocyclic heteroaryl ring or a non-aromatic ring.

In the present disclosure, the above-mentioned kinds of heteraryls may be substituted or unsubstituted, and when substituted, the substituent is preferably one or more of the substituted groups described in the present disclosure.

As used herein, the term "acetyl" refers to -C(O)CH₃.

### BRIEF DESCRIPTION OF DRAWING

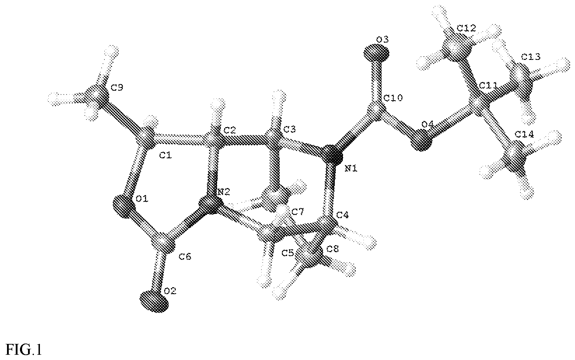

FIG. 1 is the three-dimensional structure diagram of compound B through single crystal diffraction.

### DETAILED DESCRIPTION OF THE INVENTION

The compounds of the present disclosure can be prepared by a plurality of synthesis methods well known to those skilled in the art, including the specific embodiments set forth below, embodiments derived therefrom in combination with other chemical synthesis methods, and equivalents thereof well known to those skilled in the art. Preferred embodiments include, but are not limited to embodiments of the present disclosure. The part relevant to synthesis methods of intermediates or starting compounds herein is present separately in the preparative example illustrated in an example. It is not repeated in the remaining embodiments, if the same intermediate or starting compound is used.

The present disclosure will now be described in more detail by way of embodiments, but not intended to any disadvantageous limitation thereof. While the present disclosure has been described in detail and also disclosed specific embodiments thereof, it will be apparent to those skilled in the art that, various changes and modifications can be made regarding detailed embodiments of the present disclosure therein without departing from the spirits and scope of the present disclosure. Where specific conditions are not illustrated in the examples, they are carried out according to conventional conditions or those conditions suggested by the manufacturer. The used reagents or instruments without indicating the manufacturer, are conventional products commercially available.

For example, the compound of formula (A-4) can be prepared through the following method.

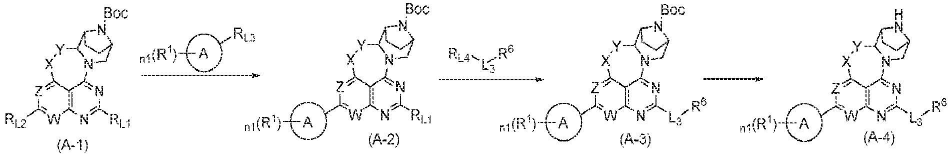

First, compound (A-1), as a reactant, reacts with 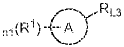 to form compound (A-2). Next, compound (A-2) reacts with 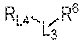 to form compound (A-3). Then, compound (A-3) was subjected to a Boc deprotection reaction to obtain compound (A-4); R_{L1}, R_{L2}, R_{L3}, R_{L4} are each independently leaving groups (e.g., halogens, boronic acid groups (e.g., ), boronate ester groups (e.g., 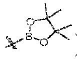 ),-S(=O)CH₃, -S(=O)₂CH₃, -OH, -SnBu₃, phenyltriazole-O- (e.g., 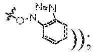 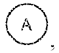 X, Y, Z, W, R', n1, L₃, R⁶ are each defined as above.

As another example, the compound of formula (A-4) can be prepared through the following method.

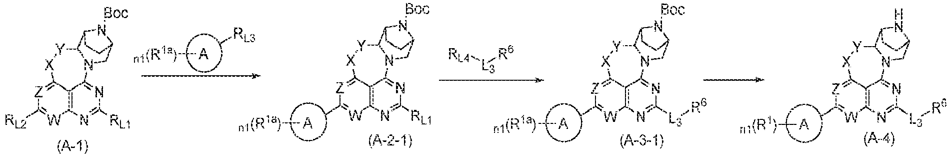

First, compound (A-1), as a reactant, reacts with 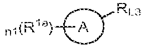 to form compound (A-2-1). Next, compound (A-2-1) reacts with to form compound (A-3-1). Then, compound (A-3-1) was subjected to a Boc deprotection reaction to obtain compound (A-4); R_{L1}, R_{L2}, R_{L3}, R_{L4} are each independently leaving groups (e.g., halogens, boronic acid groups (e.g., 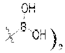 boronate ester groups (e.g., 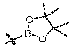 ),-S(=O)CH₃, -S(=O)₂CH₃, -OH, -SnBu₃, phenyltriazole-O- (e.g., 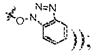 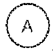 , X, Y, Z, W, R', n1, L₃, R⁶ are each defined as above; R^{1a} refers to R¹ that has been protected with a protecting group. For example, when R¹ contains an alkyne group, that alkyne group can be protected with a TIPS (triisopropylsilyl) protecting group. Similarly, when R¹ contains an amino group, that amino group can be protected with a Boc (tert-butyloxycarbonyl) protecting group.

As another example, the compound of formula (A-4) can be prepared through the following method.

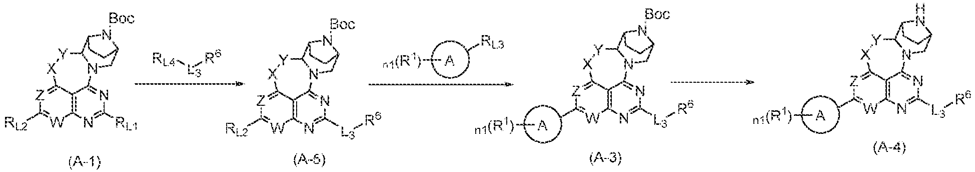

First, compound (A-1), as a reactant, reacts with 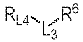 to form compound (A-5). Next, compound (A-5) reacts with 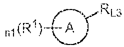 to form compound (A-3). Then, compound (A-3) was subjected to a Boc deprotection reaction to obtain compound (A-4); 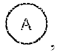 X, Y, Z, W, R¹, n1, L₃, R⁶ are each defined as above.

The structure of the compound was determined using Nuclear Magnetic Resonance (NMR) and Mass Spectrometry (MS) methods, etc.

NMR measurements were conducted using either a Broker Avance NEO 400 or Broker Avance NEO 500 NMR spectrometer. Chemical shifts ( δ ) were reported in parts per million (ppm) units. The solvents used for these measurements are as specified in each example, with tetramethylsilane (TMS) as the internal standard.

MS measurements were performed using an Agilent 1100 Liquid Chromatography system.

High Performance Liquid Chromatography (HPLC) analysis was conducted using a Waters 2695 HPLC system.

For preparative HPLC, a Waters 2767 system was used.

Chiral HPLC analysis was conducted using either a Waters 2695 HPLC system or a Waters Investigator SFC system.

For chiral preparation, a Gilson GX-281 chromatography or a Waters SFC-80 chromatography was utilized.

Thin Layer Chromatography (TLC) was performed using silica gel plates from Qingdao GF254 or Yantai Huanghai HSGF254. The specifications for the silica gel plates used in TLC were 0.15mm-0.2mm, and for TLC separation and purification of products, the specifications were 0.4mm-0.5mm. Column chromatography typically employed the ISCO CombiFlash NextGen 300 system, utilizing Agela Flash Column Silica-Cs series silica columns.

For preparative HPLC used in the following embodiments, unless otherwise specified, the following conditions were used:
- Preparative HPLC (formic acid method 1): Column type: Waters XBridge C18, 19*250mm, Sum; Mobile phase system: A: 0.1% formic acid aqueous solution; B: preparative grade acetonitrile; Flow rate: 15ml/min; B%: 20%-100%; Column temperature: room temperature.
- Preparative HPLC (formic acid method 2): Chromatographic column: Welch Xtimate C18, 21.2* 150mm, Sum; Mobile phase A: 0.1% formic acid aqueous solution, Mobile phase B: acetonitrile; Flow rate: 15ml/min; Column temperature: room temperature.
- Preparative HPLC (ammonium bicarbonate method 1): Column type: Waters XBridge C18, 19*250mm, Sum; Mobile phase system: A: 5mmol/L ammonium bicarbonate aqueous solution; B: preparative grade acetonitrile; Flow rate: 15ml/min; B%: 20%-100%; Column temperature: room temperature.
- Preparative HPLC (ammonium bicarbonate method 2): Chromatographic column: Welch Xtimate C18, 21.2* 150mm, Sum; Mobile phase A: 5mmol/L ammonium bicarbonate aqueous solution, Mobile phase B: acetonitrile; Flow rate: 15mL/min; Column temperature: room temperature.
- Preparative HPLC (ammonia method): Chromatographic column: Waters Xbridge C18, 19*150 mm, 5 µ m; Mobile phase A: 0.1% ammonia solution, Mobile phase B: acetonitrile; Flow rate: 15 mL/min; Column temperature: room temperature.
- Preparative HPLC (trifluoroacetic acid method): Chromatographic column: Welch Xtimate C18, 21.2* 150mm, Sum; Mobile phase A: 0.1% trifluoroacetic acid-water, Mobile phase B: acetonitrile; Flow rate: 15ml/min; Column temperature: room temperature.

### Preparation Example 1: Synthesis of tert-butyl (1R,5S)-2-(hydrozymethyl)-3,8-diazabicyclo[3.2.1]octane-8-carboxylate (A1)

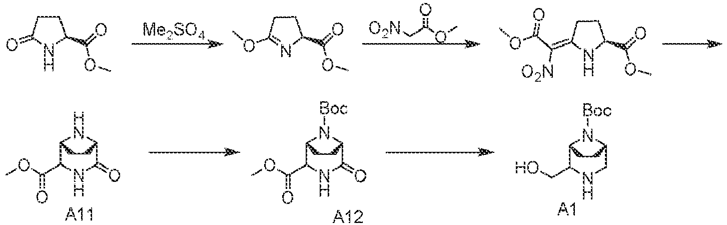

Step 1: In a 1L single-neck flask, methyl (S)-5-oxopyrrolidine-2-carboxylate (200 g, 1397 mmol) and dimethyl sulfate (134 mL) were added. After the addition was complete, the system reacted at 56°C for 18 hours, monitored by TLC until the reaction was complete. The reaction system was then cooled to room temperature, and triethylamine (292 mL) was added dropwise, followed by stirred for 30 minutes. Water (400 mL) was added, and the mixture was extracted with ethyl acetate (400 mL x 3). The organic phase was washed with saturated brine (400 mL), dried over anhydrous sodium sulfate, filtered, and the filtrate was evaporated to obtain methyl (S)-5-methoxy-3,4-dihydro-2H-pyrrole-2-carboxylate (150g, yield: 68.4%), a yellow oily compound. ES-API:[M+1]⁺=158.1.

Step 2: In a 1L single-neck flask, methyl (S)-5-methoxy-3,4-dihydro-2H-pyrrole-2-carboxylate (150 g, 954.3 mmol) and methyl nitroacetate (123.8 g, 1040.2 mmol) were added. After the addition was complete, the system reacted at 60°C for 40 hours, monitored by NMR until the reaction was complete. Column chromatography (dichloromethane/ethyl acetate = 50/1) was performed to obtain methyl (S,Z)-5-(2-methoxy-1-nitro-2-oxoethylidene)pyrrolidine-2-carboxylate (80.0 g, yield: 34.2%), a yellow solid.ES-API:[M+1]⁺=245.1.

Step 3: In a 1L single-neck flask, methyl (S,Z)-5-(2-methoxy-1-nitro-2-oxoethylidene)pyrrolidine-2-carboxylate (20.0 g, 81.9 mmol) palladium on carbon (2.0 g) and methanol (600 mL) were added to. After the addition was complete, the system was purged with hydrogen three times. The reaction was proceeded at 60°C for 5 days, monitored by LC-MS until completion. The process was repeated for four batches. The mixture was filtered, and the filtrate was concentrated to obtain methyl (1R,5S)-4-oxo-3,8-diazabicyclo[3.2.1]octane-2-carboxylate (A11, 60g, crude) as obtained. ES-API:[M+1]⁺=185.1

Step 4: In a 1L single-neck flask, methyl (1R,5S)-4-oxo-3,8-diazabicyclo[3.2.1]octane-2-carboxylate (A1, 60.0 g, 325 mmol), triethylamine (65.9 g, 651.5 mmol), and dichloromethane (600 mL) were added. The mixture was cooled to 0°C and ditert-butyl dicarbonate (106.6 g, 488.6 mmol) was added. After the addition was complete, the system reacted overnight at room temperature, monitored by LC-MS until completion. Water (1L) was added, and the mixture was extracted with dichloromethane (1L). The organic phase was evaporated, and the crude product was purified by column chromatography (dichlorohexane/methanol = 20:1) to obtain 8-(tert-butyl) 2-methyl (lR,5S)-4-oxo-3,8-diazabicyclo[3.2.1]octane-2,8-dicarboxylate (A12, 70.0 g, yield: 75.8%), a yellow solid. ES-API:[M-Boc+1]⁺=285.1.

Step 5: In a 500 mL three-neck flask, the compound 8-(tert-butyl) 2-methyl (1R,5S)-4-oxo-3,8-diazabicyclo[3.2.1]octane-2,8-dicarboxylate (A12, 20.0 g, 70.3 mmol) and tetrahydrofuran (300 mL) were added. The mixture was cooled to 0°C, and lithium aluminum hydride (5.87 g, 154.7 mmol) was added batchwise (the temperature was maintained below 5°C). After addition was complete, the reaction was proceeded overnight at room temperature, monitored by LC-MS until complete. The reaction mixture was quenched with water at 0°C, stirred for 1 hour, and filtered, with the filter cake washed with dichloromethane/methanol (10:1). The filtrate was concentrated to obtain a crude product. The crude product was purified by preparative HPLC (ammonium bicarbonate method 1) to obtain tert-butyl (1R,5S)-2-(hydroxymethyl)-3,8-diazabicyclo[3.2.1]octane-8-carboxylate (A1, 5.8 g, yield: 32.2%), a yellow solid. ES-API:[M-Boc+1]⁺=243.2.

### Preparation Example 2: Synthesis of isomer (A2-1)

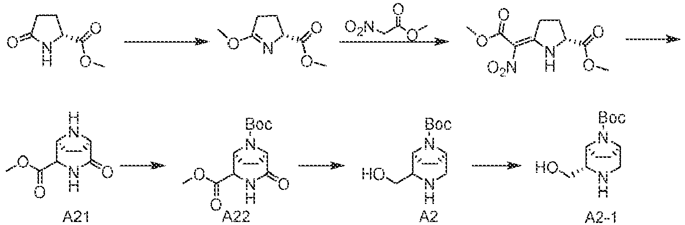

Step 1: methyl (R)-5-oxopyrrolidine-2-carboxylate (395.3 g, 2.762 mmol) was dissolved in 3500 mL of dichloromethane, and trimethyloxonium tetrafluoroborate (612.697 g, 4.142 mol, 1.5 eq) was added batchwise at 5°C. The reaction was proceeded for 24 hours at 25°C and was monitored by TLC until completion. The reaction mixture was then slowly poured into 5000 mL of saturated sodium carbonate solution, maintaining the pH between 8 and 9. The layers were separated, and the aqueous phase was extracted with 1500 mL of dichloromethane. The organic phases were combined, washed with saturated brine, dried over anhydrous magnesium sulfate, filtered, and evaporated to obtain methyl (R)-5-methoxy-3,4-dihydro-2H-pyrrole-2-carboxylate (406.7 g, 2.588 mol, yield: 93.702%) as a pale yellow oily liquid. ES-API:[M+1]⁺=158.1.

Step 2: methyl (R)-5-methoxy-3,4-dihydro-2H-pyrrole-2-carboxylate (406.7 g, 2.588 mol) was added to methyl nitroacetate (308.133 g, 2.588 mol, 1 eq). The reaction was proceeded at 60°C for 48 hours and monitored by NMR until completion. Column chromatography (petroleum ether: ethyl acetate = 4:1~2:1) yielded 250.0 g of a yellow oily crude product, which was then purified by slurring, filtered, and dried to obtain methyl (R)-5-(2-methoxy-1 -nitro-2-oxoethylidene)pyrrolidine-2-carboxylate (146.2 g, 598.687 mmol, yield: 23.136%) as a pale yellow solid. ES-API:[M+1]⁺=245.1.

Step 3: In a 5L autoclave, (R)-5-(2-methoxy-1-nitro-2-oxoethylidene)pyrrolidine-2-carboxylate (146.2 g, 598.687 mmol) was dissolved in methanol (1500 mL) and tetrahydrofuran (1500 mL). Pd(OH)₂/C (15.00 g, 20% purity) was added, and the mixture was purged with hydrogen three times, then reacted under 0.4 MPa at 40°C for 72 hours, with hydrogen replenished multiple times during the reaction. After cooling to room temperature and depressurizing, the reaction mixture was filtered and evaporated to obtain methyl (lR,5S)-4-oxo-3,8-diazabicyclo[3.2.1]octane-2-carboxylate (A21, 110.3 g, crude product) as a yellow oily liquid. ES-API:[M+1]⁺=185.1.

Step 4: The crude product methyl (1R,5S)-4-oxo-3,8-diazabicyclo[3.2.1]octane-2-carboxylate (A21, 10.00 g, 54.291 mmol) was dissolved in water (50 mL) and tetrahydrofuran (50 mL). Na₂CO₃ (5.754 g, 54.291 mmol) was added at 10°C, followed by dropwise addition of (Boc)₂O (11.849 g, 54.291 mmol). After the addition was complete, the reaction was proceeded at 20°C for 12 hours and was monitored by TLC until completion. The reaction mixture was filtered, and the filter cake was washed with 50 mL of ethyl acetate. The layers were separated, and the aqueous phase was extracted with 50 mL of ethyl acetate. The organic phases were combined, evaporated and subjected to column chromatography (petroleum ether: ethyl acetate = 2:1∼1:1) to obtain 8-(tert-butyl)-2-methyl-(1S,5R)-4-oxo-3,8-diazabicyclo[3.2.1]octane-2,8-dicarboxylate (A22, 3.61 g, 12.698 mmol, yield: 23.388%) as a white solid. ES-API:[M-Boc+1]⁺=285.1.

Step 5: 8-(tert-butyl)-2-methyl-(1S,5R)-4-oxo-3,8-diazabicyclo[3.2.1]octane-2,8-dicarboxylate (A22, 2.00 g, 7.035 mmol) was dissolved in tetrahydrofuran (20 mL). Lithium aluminium hydride (400.489 mg, 10.552 mmol, 1.5 eq) was added batchwise at 0°C. After the addition was complete, the reaction was proceeded at 20°C for 12 hours and was monitored by TLC until completion. At 0°C, 0.4 mL of water, 0.4 mL of 15% sodium hydroxide solution, and 1.2 mL of water were successively added to the reaction mixture. After the addition was complete, anhydrous magnesium sulfate was added and the mixture was stirred for 10 minutes, then filtered and concentrated to obtain a crude product. The crude product was purified by reverse phase preparation to obtain tert-butyl (lR,5S)-2-(hydroxymethyl)-3,8-diazabicyclo[3.2.1]octane-8-carboxylate (A2, 0.65 g, yield: 38%) as a pale yellow solid.ES-API:[M-Boc+1]⁺=243.2.

Step 6: tert-butyl (1R,5S)-2-(hydroxymethyl)-3,8-diazabicyclo[3.2.1]octane-8-carboxylate (A2, 100 mg) was subjected to chiral SFC preparative separation [column type: CHIRALPAK-IG, 30*250mm, 10um; mobile phase: carbon dioxide: methanol (0.1% aqueous ammonia) = 80:20(v/v); flow rate: 150 mL/min; column temperature: 35°C; detection wavelength: 210 nm] to obtain an isomeric compound. The structure, identified as tert-butyl (1R,2S,SS)-2-(hydroxymethyl)-3,8-diazabicyclo[3.2.1]octane-8-carboxylate (A2-1, 58 mg, peak 2, retention time 10.391 min), was inferred from single crystal data in Example 14. The isomer was analyzed by chiral HPLC [column type: CHIRALPAK-AY, 4.6*250mm, 5um; mobile phase: hexane: ethanol (0.1% diethylamine) = 95:5(v/v); flow rate: 1 mL/min; column temperature: 37°C; detection wavelength: 2 10 mn]. ¹H NMR of A2-1 (400 MHz, DMSO-*d6*): δ 4.57 (t, J = 5.2 Hz, 1H), 4.01-3.87 (m, 2H), 3.23-3.13 (m, 2H), 2.78-2.66 (m, 2H), 2.59-2.52 (m, 1H), 1.84-1.47 (m, 4H), 1.40 (s, 9H). ES-API:[M+1]⁺=243.2.

### Example 1 Synthesis of 5-ethyl-4-((6R,9S)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z1)

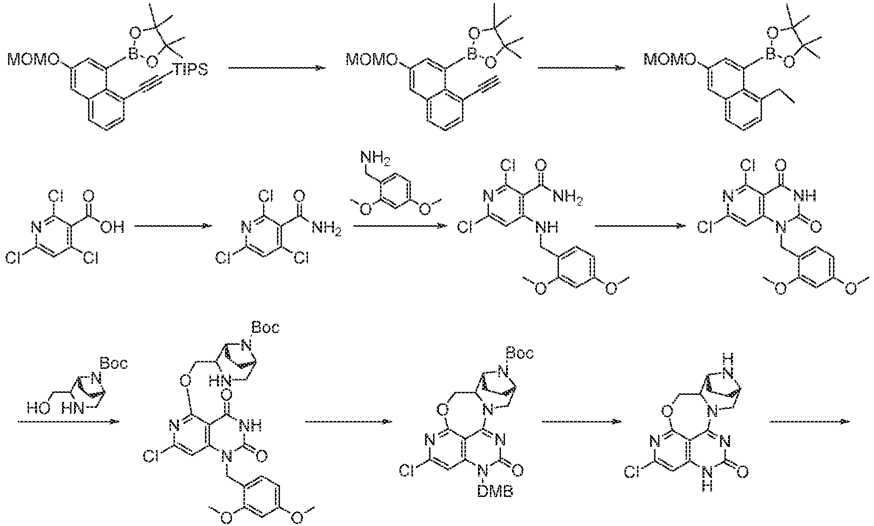

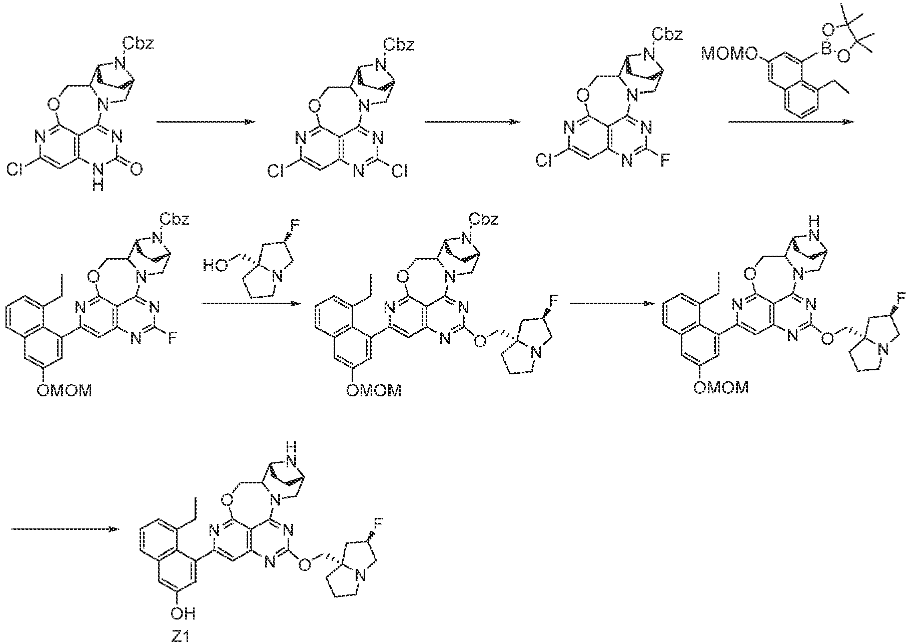

Step 1: To a N,N-dimethylformamide (5.0 mL) solution of triisopropyl((6-(methoxymethoxy)-8-(4,4,5,5-tetramethyl-1,3,2-dioxaborolane-2-yl)naphthalen-1-yl)ethynyl)silane (500 mg, 1.012 mmol), cesium fluoride (3.0 g, 20.24 mmol) was added. The reaction was proceeded at room temperature for 2 hours. Upon completion of the reaction, the mixture was extracted with ethyl acetate (100 mL X 2). The ethyl acetate phases were combined, washed with saturated brine (100 mL X 3), dried over anhydrous sodium sulfate, filtered, and evaporated under reduced pressure to obtain 2-(8-ethynyl-3-(methoxymethoxy)naphthalen-1-yl)-4,4,5,5-tetramethyl-1,3,2-dioxaborolane (558 mg, crude product). ES-API:[M+H]⁺=339.3.

Step 2: To a methanol solution (10.0 mL) of 2-(8-ethynyl-3-(methoxymethoxy)naphthalen-1-yl)-4,4,5,5-tetrainethyl-1,3,2-dioxaborolane (558 mg, crude product), 10% palladium on carbon (60 mg) was added. The mixture was purged four times with hydrogen and reacted under hydrogen atmosphere at room temperature for 2 hours. Upon completion of the reaction, the mixture was filtered, and the solvent was evaporated under reduced pressure to obtain 2-(8-ethyl-3-(methoxymethoxy)naphthalen-1-yl)-4,4,5,5-tetramethyl-1,3,2-dioxaborolane (284 mg, total yield for 2 steps: 82%). ES-API:[M+1]⁺=343.3.

Step 3: At 0°C, 2,4,6-trichloronicotinic acid (5.0 g, 22.22 mmol) was dissolved in dichloromethane (40.0 mL), to which system oxalyl chloride (6.0 mL) was added dropwise, followed by 8 drops of N,N-dimethylformamide. The reaction system was stirred at room temperature and reacted for 1 hour. 1 hour later, the solvent was evaporated under reduced pressure and added to dry tetrahydrofuran (30.0 mL). The solution was cooled to 0-5°C and then added dropwise to a mixed solution of ammonia (30.0 mL) and tetrahydrofuran (30.0 mL), stirred at room temperature for 1 hour. The mixture was extracted with ethyl acetate (100 mL X 2), and the ethyl acetate phase was combined, washed with saturated brine (100 mL X 1), dried over anhydrous sodium sulfate, filtered, and evaporated to obtain 2,4,6-trichloronicotinamide (6.0 g, crude product). ES-API:[M+H]⁺=224.9/226.9.

Step 4: In a 500 mL three-neck round-bottom flask, (2,4-dimethoxyphenyl)methanamine (4.45 g, 26.66 mmol) was dissolved in dry dioxane (90.0 mL). The solution was cooled to 0-5°C in ice-water bath, under nitrogen protection, and N,N-diisopropylethylamine (8.61 g, 66.66 mmol) was added. The reaction was proceeded for about 10-15 minutes at this temperature, then 2,4,6-trichloronicotinamide (6.0 g, crude product) dissolved in dry dioxane (20 mL) was added dropwise to the mixture. The reaction was proceeded at 50°C for 5 hours. Upon completion of the reaction (monitored by LCMS), the reaction mixture was poured into ice water (400 mL), extracted with ethyl acetate (200 mL X 1), dried over anhydrous sodium sulfate, filtered, and evaporated. The crude product was purified by column chromatography [petroleum ether: ethyl acetate = 100:0~50:50, (v/v)] to obtain 2,6-dichloro-4-((2,4-dimethoxybenzyl)amino)nicotinamide (4.16 g, total yield for 2 steps: 52%).ES-API:[M+H]⁺= 356.1/358.1.

Step 5: At room temperature, 2,6-dichloro-4-((2,4-dimethoxybenzyl)amino)nicotinamide (3.723 g, 10.47 mmol) and dry tetrahydrofuran (60 mL) were added to a 500 mL three-neck round-bottom flask. The mixture was cooled to 0-5°C in ice water bath, and sodium hydride (838 mg, 20.95 mmol) was added batchwise. The reaction was proceeded for 20 minutes at this temperature. N,N'-carbonyldiimidazole (5.09 g, 31.41 mmol) was added to dry tetrahydrofuran (30 mL) and then added dropwise to the above solution. After the addition was complete, the reaction was proceeded for 0.5-1 hour at this temperature. Upon completion of the reaction (monitored by LCMS), the reaction mixture was poured into ice water (300 mL). The pH was adjusted to 7-8 with 6M hydrochloric acid solution, and the product was filtered to obtain 5,7-dichloro-1-(2,4-dimethoxybenzyl)pyrido[4,3-d]pyrimidine-2,4(1H,3H)-dione (3.7 g, yield: 92.7%). ES-API:[M+H]⁺=382.0/384.0.

Step 6: At room temperature, dry tetrahydrofuran (100 mL) was added to a 250 mL single-neck flask, followed by (1R,5S)-2-(hydroxymethyl)-3,8-diazabicyclo[3.2.1]octane-8-carboxylate tert-butyl ester (358.0 mg, 1.480 mmol). The mixture was cooled to 0-5°C in an ice-water bath, and sodium hydride (1.20 mg, 2.960 mmol) was added under nitrogen. After 10-20 minutes of reaction, 5,7-dichloro-1-(2,4-dimethoxybenzyl)pyrido[4,3-d]pyrimidine-2,4(1H,3H)-dione (565.0 mg, 1.480 mmol) was added batchwise. The reaction was proceeded for 20-30 minutes at 0-5°C. Upon completion of the reaction (monitored by LCMS), the reaction mixture was poured into ice water (300 mL). The pH was adjusted to 7-8 with 6M hydrochloric acid solution, and the product was extracted with ethyl acetate (100 mL X 2). The ethyl acetate phase were combined, washed with saturated brine (100 mL X 1), dried over anhydrous sodium sulfate, filtered, and evaporated to obtain tert-butyl (1R,SS)-2-(((7-chloro-1-(2,4-dimethoxybenzyl)-2,4-dioxo-1,2,3,4-tetrahydropyrido[4,3-d]pyrimidin-5-yl)oxy)methyl)-3,8-diazabicyclo[3.2.1]octane-8-carboxylate (850 mg, yield: 97%). ES-API:[M+H]⁺=588.3.

Step 7: At room temperature, dry N,N-dimethylformamide (20 mL) and tert-butyl (1R,5S)-2-(((7-chloro-1-(2,4-dimethoxybenzyl)-2,4-dioxo-1,2,3,4-tetrahydropyhdo[4,3-d]pyrimidin-5-yl)oxy)methyl)-3,8-diazabicyclo[3.2.1]octane-8-carboxylate (850 mg, 1.448 mmol) were added to a 250 mL single-neck flask. 1-propylphosphonic anhydride (5.0 g, 7.856 mmol) was added, and after 5 minutes of reaction at room temperature, 1,8-diazabicyclo[5.4.0]undec-7-ene (1.14 g, 7.488 mmol) was added. The reaction was proceeded for 1-2 hours at room temperature. Upon completion of the reaction, the reaction mixture was slowly poured into ice water (100 mL), resulting in the precipitation of a large amount of solid, which was then filtered and evaporated under reduced pressure to obtain the target compound tert-butyl (6R,9S)-2-chloro-13-(3,4-dimethylbenzyl)-12-oxo-5a,6,7,8,9,10,12,13-octahydro-5H-4-oxa-3,10a,11,13,14-pentaaza-6,9-methanonaphtho[1,8-ab]heptalene-14-carboxylate (620 mg, yield: 75.2%). ES-API: [M+H]⁺=570.3.

Step 8: Tert-butyl (6R,9S)-2-chloro-13-(3,4-dimethylbenzyl)-12-oxo-5a,6,7,8,9,10,12,13-octahydro-5H-4-oxa-3,10a,11,13,14-pentaaza-6,9-methanonaphtho[1,8-ab]heptalene-14-carboxylate (420mg, 0.7380mmol) was added to trifluoroacetic acid (5mL). The reaction mixture was stirred at 55°C for 1 hour. Upon completion of the reactiond, the solvent was evaporated under reduced pressure to obtain (6R,9S)-2-chloro-5a,6,7,8,9,10-hexahydro-5H-4-oxa-3,10a,11,13,14-pentazacyclo-6,9-methoxynaphtho[1,8-ab]heptene-12(13H)-one (500mg, crude product). ES-API: [M+H]⁺=320.1.

Step 9: Sodium carbonate (350mg, 3.282mmol) was added to a mixed solution of (6R,9S)-2-chloro-5a,6,7,8,9,10-hexahydro-5H-4-oxa-3,10a,11,13,14-pentazacyclo-6,9-methylenenaphtho[1,8-ab]heptene-12(13H)-one (350mg, 1.094mmol) in tetrahydrofuran (5.0mL) and water (5.0mL). The mixtuure was cooled in ice-water bath, and benzylmethoxycarbonylsuccinimide (354mg, 1.422mmol) was added. The reaction was proceeded at room temperature for 3 hours. Upon completion of the reaction, the reaction mixture was extracted with dichloromethane (80mL), washed with saturated sodium bicarbonate solution (100mL) and saturated brine (80mL), dried over anhydrous sodium sulfate, concentrated, and the crude product was purified by flash silica gel column (ethyl acetate/petroleum ether: 0-50%) to obtain benzyl (6R,9S)-2-chloro-12-oxo-5a,6,7,8,9,10,12,13-octahydro-5H-4-oxa-3,10a,11,13,14-pentaaza-6,9-methanonaphtho[1,8-ab]heptalene-14-carboxylate (460mg, 93% yield). ES-API: [M+H]⁺=454.1.

Step 10: At 0°C, phosphorus oxychloride (465mg, 3.039mmol) and N,N-diisopropylethylamine (392.0mg, 3.039mmol) were successively added to a toluene (3.0mL) solution of benzyl (6R,9S)-2-chloro-12-oxo-5a,6,7,8,9,10,12,13-octahydro-5H-4-oxa-3,10a,11,13,14-pentaaza-6,9-methanonaphtho[1,8-ab]heptalene-14-carboxylate (460mg, 1.013mmol). The reaction was proceeded at 125°C for 12 hours. Upon completion of the reaction, the mixture was cooled to room temperature, and the solvent was evaporated under reduced pressure to obtain benzyl (6R,9S)-2,12-dichloro-5a,6,7,8,9,10-hexahydro-5H-4-oxa-3,10a,11,13,14-pentaaza-6,9-methanonaphtho[1,8-ab]heptalene-14-carboxylate (520mg, crude product). ES-API: [M+H]⁺=472.2.

Step 11: Potassium fluoride (500mg, 8.492mmol) was added to an N,N-dimethylformamide (3.0mL) solution of benzyl (6R,9S)-2,12-dichloro-5a,6,7,8,9,10-hexahydro-5H-4-oxa-3,10a,11,13,14-pentaaza-6,9-methanonaphtho[1,8-ab]heptalene-14-carboxylate (200mg, 0.4246mmol). The reaction was proceeded under nitrogen protection at 120°C for 4 hours. Upon completion of the reaction, the mixture was cooled to room temperature, extracted with ethyl acetate (50mLx2), and the ethyl acetate phases were combined and washed with saturated brine (50mLx4), dried over anhydrous sodium sulfate, filtered, and evaporated to obtain benzyl (6R,9S)-2-chloro-12-fluoro-5a,6,7,8,9,10-hexahydro-5H-4-oxa-3,10a,11,13,14-pentaaza-6,9-methanonaphtho[1,8-ab]heptalene-14-carboxylate (130mg, 67.4% yield). ES-API:[M+H]⁺=456.1.

Step 12: Benzyl (6R,9S)-2-chloro-12-fluoro-5a,6,7,8,9,10-hexahydro-5H-4-oxa-3,10a,11,13,14-pentaaza-6,9-methanonaphtho[1,8-ab]heptalene-14-carboxylate (140mg, 0.3076mmol) was dissolved in a mixture of tetrahydrofuran/water (2 mL/0.5 mL). 2-(8-Ethyl-3-(methoxymethoxy)naphth-1-yl)-4,4,5,5-tetramethyl-1,3,2-dioxaborolane (250mg, 0.7309mmol), potassium phosphate (250mg, 1.179 mmol), and methanesulfonic acid [butylbis(1-adamantyl)phosphine](2-amino-1,1'-biphenyl)palladium(II) (40.0mg, 0.05494mmol) were added, and the mixture was purged four times with nitrogen, then reacted at 80°C for 1 hour using microwave. Cooled to room temperature, the reaction mixture was extracted with ethyl acetate (80mL) and water (60mL). The organic phase was dried over anhydrous sodium sulfate, filtered, and the filtrate was evaporated under reduced pressure. The crude product was purified by flash silica gel column (ethyl acetate/petroleum ether: 0-70%) to obtain benzyl (6R,9S)-2-(8-ethyl-3-(methoxymethoxy)naphthalen- 1-yl)- 12-fluoro-5a,6,7,8,9,10-hexahydro-5H-4-oxa-3,10a, 11,13,14-pentaaza-6,9-methanonaphtho[1,8-ab]heptalene-14-carboxylate (144mg, yield: 73%). ES-API:[M/2+1]⁺=636.3.

Step 13: Under ice-water bath condition, sodium hydride (22mg, 0.5428mmol) was added to a tetrahydrofuran (5.0mL) solution of ((2R,7aS)-2-fluorotetrahydro-1H-pyrrolizin-7a(5H)-yl)methanol (43.0mg, 0.2714mmol) and the mixture reacted at room temperature for 0.5 hours. Benzyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanomphtho[1,8-ab]heptalene-14-carboxylate (145mg, 0.1357mmol) was added to the above reaction system and reacted for 1-2 hours at room temperature. Upon completion of the reaction, ice water (50mL) was added to the system. The mixture was extracted with dichloromethane (50mLx2). The dichloromethane phases were combined, dried over anhydrous sodium sulfate, filtered, and the filtrate was evaporated under reduced pressure. The crude product was purified by flash silica gel column (methanol/dichloromethane: 0-10%) to obtain benzyl (6R,9S)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170mg, crude product). ES-API:[M/2+1]⁺=775.3.

Step 14: To a methanol (10.0mL) solution of benzyl (6R,9S)-2-(8-ethyl-3-(methoxymethoxy)naphthalen-1-yl)-12-(((2R,7aS)-2-fluorotetrahydro-1H-pyrrolizin-7a(SH)-yl)methoxy)-5a,6,7,8,9,10-hexahydro-5H-4-oxa-3,10a,11,13,14-pentaaza-6,9-methanonaphtho[1,8-ab]heptalene-14-carboxylate (170mg, crude product), 10% (mass fraction) palladium on carbon (500mg) was added. The mixture was purged four times with hydrogen and reacted under a hydrogen atmosphere at room temperature for 2 hours to obtain (6R,9S)-2-(8-ethyl-3-(methoxymethoxy)naphthalen-1-yl)-12-(((2R,7aS)-2-fluorotetrahydro-1H-pyrrolizin-7a(5H)-yl)mefhoxy)-5a,6,7,8,9,10-hexahydro-5H-4-oxa-3,10a,11,13,14-pentaaza-6,9-methanonaphtho[1,8-ab]heptalene (162mg, crude product). ES-API:[M/2+1]⁺=7641.3.

Step 15: To a solution of (6R,9S)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (162mg, crude product) in acetonitrile (6.0mL), a 4M hydrochloric acid/dioxane solution (2.0mL, 8.0mmol) was added under ice water bath condition and reacted for 0.5 hours. Upon completion of the reaction, the reaction mixture was concentrated to dryness, dichloromethane (20mL) was added, and triethylamine (3.0mL) was introduced under an ice-water bath. Stirred for 10 minutes, extracted with dichloromethane (100mLx1) and water (50mLx1). The organic phase was dried over anhydrous sodium sulfate, filtered, and the filtrate was evaporated under reduced pressure. The crude product was purified by preparative HPLC (formic acid method 1) to obtain 5-ethyl-4-((6R,9S)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ablheptalen-2-yl)naphthalen-2-ol (Z1, 4.69mg, total yield over 3 steps: 5%, formate salt). ES-API:[M+1]⁺=597.3. ¹H NMR (500 MHz, CD₃OD)δ 8.48 (s, 1H), 7.58 (d, *J* = 8.0 Hz, 1H), 7.34 (t, *J* = 8.0 Hz, 1H), 7.25 -6.91 (m, 4H), 5.50 -5.31 (m, 1H), 5.11 -4.97 (m, 1H), 4.70 -4.30 (m, 4H), 4.20 -4.10 (m, 1H), 3.85 -3.73 (m, 2H), 3.65 -3.40 (m, 3H), 3.29 -3.14 (m, 2H), 2.56 -1.75 (m, 12H), 1.00 -0.85 (m, 3H).

### Example 2 Synthesis of 5-ethyl-4-((5aS,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z2)

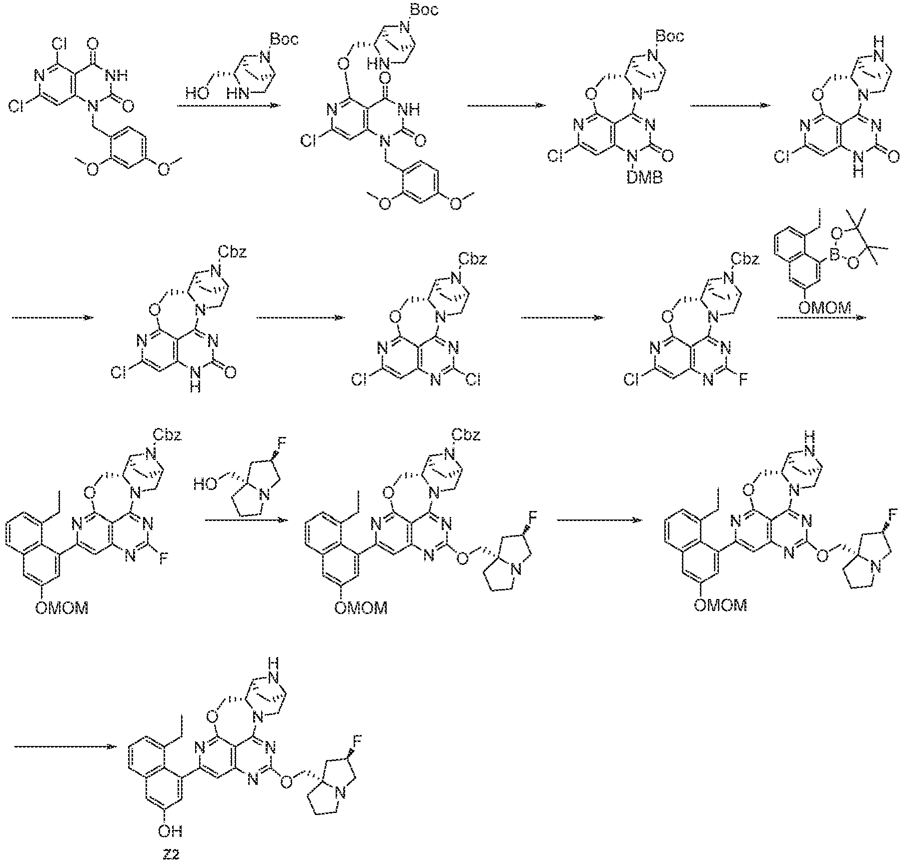

Step 1: At room temperature, dry tetrahydrofuran (100 mL) was added to a 250 mL single-necked flask, followed by tert-butyl (1S,2S,5R)-2-(hydroxymethyl)-3,8-diazabicyclo[3.2.1]octane-8-carboxylate (358.0 mg, 1.480 mmol). The mixture was cooled to 0-5°C in an ice-water bath and sodium hydride (1,20 mg, 2.960 mmol) was added. Under nitrogen protection, the reaction was proceeded for 10-20 minutes before batchwise adding 5,7-dichloro-1-(2,4-dimethoxybenzyl)pyrimido[4,3-d]pyrimidine-2,4(1H,3H)-dione (565.0mg, 1.480mmol) at 0-5°C and further reacted for 20-30 minutes. Upon completion (monitored by LC-MS), the reaction mixture was poured into ice water (300 mL), and the pH was adjusted to 7-8 with 6M hydrochloric acid solution under ice-water bath condition. The mixture was extracted with ethyl acetate (100 mLx2), the ethyl acetate phases were combined and washed with saturated saline solution (100 mLxl). The solution was dried over anhydrous sodium sulfate, filtered, and evaporated to obtain tert-butyl (1S,2S,5R)-2-(((7-chloro-1-(2,4-dimethoxybenzyl)-2,4-dioxacyclo-1,2,3,4-tetrahydropyrimido[4,3-d]pyrimidin-5-yl)oxy)methyl)-3,8-diazabicyclo[3.2.1]octane-8-carboxylate (850 mg, yield: 97%). ES-API:[M+H]⁺=588.3.

Step 2: At room temperature, dry N,N-dimethylformamide (20 mL) was added to a 250 mL single-necked flask containing tert-butyl (1S,25,SR)-2-(((7-chloro-1-(2,4-dimethoxybenzyl)-2,4-dioxacyclo-1,2,3,4-tetrahydropyrimido[4,3-d]pyrimidin-5-yl)oxy)methyl)-3,8-diazabicyclo[3.2.1]octane-8-carboxylate (850 mg, 1.448 mmol). Finally, 1-propylphosphonic anhydride (5.0 g, 7.856 mmol) was added and reacted for 5 minutes at room temperature, followed by the dropwise addition of 1,8-diazabicyclo[5.4.0]undec-7-ene (1.14 g, 7.488 mmol). The reaction was proceeded at room temperature for 1-2 hours. Upon completion of the reaction, the solution was slowly added dropwise to ice water (100 mL) to precipitate a large amount of solid. The mixture was filtered, and the filter cake was evaporated under reduced pressure to obtain tert-butyl (5aS,6S,9R)-2-chloro-13-(3,4-dimethoxybenzyl)-12-oxy-5a,6,7,8,9,10,12,13-octahydro-5H-4-oxa-3,10a,11,13,14-pentazacyclo-6,9-methylenenaphtho[1,8-ab]heptene-14-carboxylate (620 mg, yield: 73%). ES-API: [M+H]⁺=570.3.

Step 3: Trifluoroacetic acid (5.0 mL) was added to tert-butyl (SaS,6S,9R)-2-chloro-13-(2,4-dimethoxybenzyl)-12-oxy-5a,6,7,8,9,10,12,13-octahydro-5H-4-oxa-3,10a, 11,13,14-pentazacyclo-6,9-methylenenaphtho[1,8-ablheptene-14-carboxylate (420 mg, 0.7380 mmol) and stirred at 55°C for 1 hour. Upon completion of the reaction, the solvent was evaporated under reduced pressure to obtain (5aS,6S,9R)-2-chloro-5a,6,7,8,9,10-hexahydro-5H-4-oxa-3,10a,11,13,14-pentazacyclo-6,9-methylenenaphtho[1,8-ab]heptene-12(13H)-one (500 mg, crude product). ES-API: [M+H]⁺=320.1.

Step 4: To a mixed solution of (5aS,6S,9R)-2-chloro-5a,6,7,8,9,10-hexahydro-5H-4-oxa-3,10a,11,13,14-pentazacyclo-6,9-methylenenaphtho[1,8-ab]heptene-12(13H)-one (350 mg, 1.094 mmol) in tetrahydrofuran (5.0 mL) and water (5.0 mL), sodium carbonate (350 mg, 3.282 mmol) was added. The system was cooled with an ice-water bath, followed by the addition of benzylmalonyl imidazolidinone (354 mg, 1.422 mmol), and reacted at room temperature for 3 hours. Upon completion of the reaction, the reaction mixture was extracted with dichloromethane (80 mL), washed with saturated sodium bicarbonate solution (100mL) and saturated saline solution (80 mL), dried over anhydrous sodium sulfate, concentrated, and the crude product was purified by flash silica gel column (ethyl acetate/petroleum ether: 0-50%) to obtain (SaS,6S,9R)-2-chloro-12-oxa-5a,6,7,8,9,10,12,13-octahydro-5H-4-oxa-3,10a,11,13,14-pentazacyclo-6,9-methylenenaphtho[1,8-ab]heptene-14-benzoate (460 mg, yield: 93%). ES-API: [M+H]⁺=454.1.

Step 5: At 0°C, to a solution of (SaS,6S,9R)-2-chloro-12-oxa-Sa,6,7,8,9,10,12,13-octahydro-SH-4-oxa-3,10a,11,13,14-pentazacyclo-6,9-methylenenaphtho[1,8-ab]heptene-14-benzoate (460 mg, 1.013 mmol) in toluene (3.0 mL), phosphorus oxychloride (465 mg, 3.039 mmol) and N,N-diisopropylethylamine (392.0 mg, 3.039 mmol) were successively added and reacted at 125°C for 12 hours. Upon completion of the reaction, the mixture was cooled to room temperature, and the solvent was evaporated under reduced pressure to obtain (SaS,6S,9R)-2,12-dichloro-Sa,6,7,8,9,10-hexahydro-5H-4-axa-3,10a,11,13,14-pentazacyclo-6,9-methylenenaphtho[1,8-ab]heptene-14-benzoate (520 mg, crude product). ES-API: [M+H]⁺=472.2.

Step 6: Potassium fluoride (500 mg, 8.492 mmol) was added to a solution of (5aS,6S,9R)-2,12-dichloro-5a,6,7,8,9,10-hexahydro-5H-4-oxa-3,10a,11,13,14-pentazacyclohexadecene[1,8-ab]naphthene-14-carboxylic acid benzyl ester (200 mg, 0.4246 mmol) in N,N-dimethylfonnamide (3.0 mL). Under nitrogen atmosphere, the reaction was proceeded at 120°C for 4 hours. Upon completion of the reaction, the mixture was cooled to room temperature, extracted with ethyl acetate (50 mLX2), and the ethyl acetate phase was washed with saturated brine (50 mLX4), the ethyl acetate phase was dried over anhydrous sodium sulfate, filtered, and evaporated to obtain benzyl (5aS,6S,9R)-2-chloro-12-fluoro-5a,6,7,8,9,10-hexahydro-5H-4-oxa-3,10a,11,13,14-pentaaza-6,9-methanonaphtho[1,8-ab]heptalene-14-carboxylate (130 mg, yield: 62%). ES-API: [M+H]⁺=456.1

Step 7: Benzyl (5aS,6S,9R)-2-chloro-12-fluoro-5a,6,7,8,9,10-hexahydro-5H-4-oxa-3,10a, 11,13,14-pentaaza-6,9-methanonaphtho[1,8-ab]heptalene-14-carboxylate(140 mg, 0.3076 mmol) was dissolved in a mixture of tetrahydrofuran/water (2 mL/0.5 mL), to which 2-(8-ethyl-3-(methoxymethoxy)naphthalen-1-yl)-4,4,5,5-tetramethyl-1,3,2-dioxaborolane (250 mg, 0.7309 mmol), potassium phosphate (250 mg, 1.179 mmol), and palladium(II) bis[2-(dibutylphosphino)ferrocene] methane sulfonate (40.0 mg, 0.05494 mmol) were added. The mixture was purged four times with nitrogen and then microwave reacted at 80°C for 1 hour. After cooling to room temperature, the reaction mixture was extracted with ethyl acetate (80 mL) and water (60 mL). The organic phase was dried over anhydrous sodium sulfate, filtered, and the filtrate was evaporated. The crude product was purified by flash silica gel column (ethyl acetate/petroleum ether: 0-70%) to obtain benzyl 5aS,6S,9R)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene-14-carboxylate (144 mg, yield: 73%). ES-API:[M/2+1]⁺=636.3.

Step 8: Under ice-water bath condition, sodium hydride (22 mg, 0.5428 mmol) was added to a solution of ((2R,7aS)-2-fluoro tetrahydro-1H-pyrrolo[5H]-7a-yl)methanol (43.0 mg, 0.2714 mmol) in tetrahydrofuran (5.0 mL). After the mixture reacting at room temperature for 0.5 hours, benzyl (5aS,6S,9R)-2-(8-ethyl-3-(methoxymethoxy)naphthalen-1yl)-12-fluoro-5a,6,7,8,9,10-hexahydro-5H-4-oxa-3,10a,11,1 3,14-pentaaza-6,9-methanonaphtho[1,8-ab]heptalene-14-carboxylate (145 mg, 0.1357 mmol) was added to the reaction system and reacted for 1-2 hours at room temperature. Upon completion of the reaction, ice water (50 mL) was added to the system, and the mixture was extracted with dichloromethane (50 mLX2). The dichloromethane phases was combined, dried over anhydrous sodium sulfate, filtered, and the filtrate was concentrated and purified by flash silica gel column (methanol/dichloromethane: 0-10%) to obtain benzyl (SaS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170 mg, crude product). ES-API:[M/2+1]⁺=775.3.

Step 9: To a methanol solution (10.0 mL) of benzyl (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170 mg, crude product), 10% (mass fraction) palladium on carbon (500 mg) was added. Purged four times with hydrogen gas, the reaction was proceeded at room temperature under a hydrogen atmosphere for 2 hours to obtain (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)rnethoxy)-5a,6,7,8,9,10-hexahydro-5H-4-oxa-3,10a,11,13,14-pentaaza-6,9-methanonaphtho[1,8-ab]heptalene (162 mg, crude product). ES-API:[M/2+1]⁺=7641.3.

Step 10: (5aS,6S,9R)-2-(8-ethyl-3-(methoxymethoxy)naphthalen-1-yi)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ablheptalene (162 mg, crude product) was dissolved in acetonitrile (6.0 mL). Under ice water bath condition, a 4M hydrochloric acid/dioxane solution (2.0 mL, 8.0 mmol) was added and reacted for 0.5 hours. Upon completion of the reaction, the reaction mixture was concentrated to dryness and then dichloromethane (20 mL) was added. Under ice water bath condition, triethylamine (3.0 mL) was added. After stirred for 10 minutes, the mixture was extracted with dichloromethane (100 mLX1) and water (50 mLX1). The dichloromethane phase was dried over anhydrous sodium sulfate, filtered, and the filtrate was evaporated under reduced pressure. The crude product was purified by preparative HPLC (ammonium bicarbonate method 1) to obtain 5-ethyl-4-((SaS,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z2, 4.65 mg, total yield for 3 steps: 2.73%). ES-API:[M+1]⁺=597.3. ¹H NMR (500 MHz, CD₃OD)δ 7.57 (d, *J* = 8.2 Hz, 1H), 7.32 (t, *J* = 7.5 Hz, 1H), 7.27 -7.09 (m, 3H), 7.06 -6.90 (m, 1H), 5.43 -5.18 (m, 1H), 5.01 (dd, *J* = 31.4, 13.5 Hz, 1H), 4.58 (d, *J* = 11.5 Hz, 1H), 4.53 -4.38 (m, 1H), 4.25 (d, *J* = 10.4 Hz, 1H), 4.19 (d, *J* = 10.4 Hz, 1H), 4.16 -4.04 (m, 1H), 3.71 (s, 1H), 3.63 (s, 1H), 3.27 -3.17 (m, 4H), 3.01 (td, *J* = 9.6, 5.5 Hz, 1H), 2.55 -2.08 (m, 5H), 2.05 -1.71 (m, 7H), 0.97 (t, *J* = 7.5 Hz, 1H), 0.90 (t, *J* = 7.4 Hz, 2H).

### Example 3 Synthesis of 4-((6R,9S)-1.2-((2,6-dimethylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)-5-ethylnaphthalen-2-ol (Z175-1)

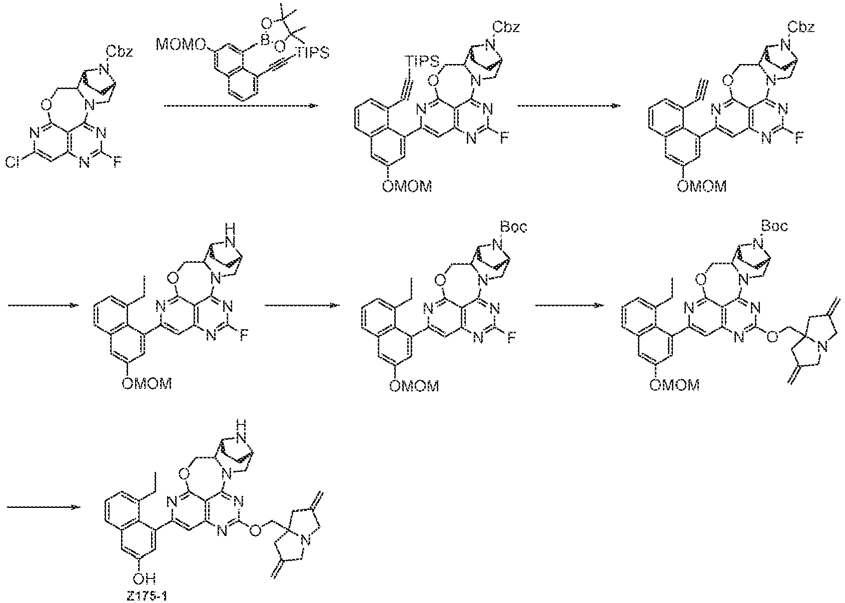

Step 1: Benzyl (6R,9S)-2-chloro-12-fluoro-5a,6,7,8,9,10-hexahydro-5H-4-oxa-3,10a,11,13,14-pentaaza-6,9-methanonaphtho[1,8-ab]heptalene-14-carboxylate (380.0 mg, 0.8351 mmol) was dissolved in tetrahydrofuran/water (12.0 mL/2.0 mL). Triisopropyl((6-(methoxymethoxy)-8-(4,4,5,5-tetramethyl-1,3,2-dioxaborolane-2-yl)naphthalen-1-yl)ethynyl)silane (825.0 mg, 1.670 mmol), potassium phosphate (800 mg, 3.773 mmol), and palladium(II) [bis(butyl)(1-adamantyl)phosphine] (2-amino-1,1'-biphenyl) (80.0 mg, 0.110 mmol) were added. After purged four times with nitrogen, the mixture was microwaved at 80°C and reacted for one hour. The reaction mixture was cooled to room temperature, extracted with ethyl acetate (150 mL) and water (100 mL), and the organic phase was dried over anhydrous sodium sulfate, filtered, and the filtrate was concentrated under reduced pressure. The crude product was purified by flash silica gel column chromatography (ethyl acetate/petroleum ether: 0-70%) to obtain benzyl (6R,9S)-12-fluoro-2-(3-(methoxymethoxy)-8-((trisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (500 mg, yield: 76%). ES-API:[M+1]⁺=788.3.

Step 2: Cesium fluoride (1.93 g, 12.69 mmol) was added to a solution of benzyl (6R,9S)-12-fluoro-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (500 mg, 0.6345 mmol) in N,N-dimethylformamide (20.0 mL). The mixture was stirred at room temperature for one hour. Upon completion of the reaction, the reaction mixture was extracted with ethyl acetate (150 mL) and water (100 mL), and the organic phase was dried over anhydrous sodium sulfate, filtered, and the filtrate was evporated under reduced pressure. The crude product was purified by flash silica gel column chromatography (ethyl acetate/petroleum ether: 0-70%) to obtain benzyl (6R,9S)-2-(8-ethynyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene-14-carboxylate (370 mg, yield: 92%). ES-API:[M+1]⁺=631.3.

Step 3: To a solution of benzyl (6R,9S)-2-(8-ethynyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene-14-carboxylate (370 mg, 0.5863 mmol) in methanol (20.0 mL), 10% (mass fraction) palladium on carbon (500 mg) was added, and the mixture was purged four times with hydrogen and reacted under a hydrogen atmosphere at room temperature for two hours to obtain (6R,9S)-2-(8-ethyl-3-(methoxymethoxy)naphthalen-1-yl)-12-fluoro-5a,6,7,8,9,10-hexahydro-5H-4-axa-3,10a,11,13,14-pentaaza-6,9-methanonaphtho[1,8-ab]heptalene (200 mg, yield: 68%). ES-API:[M+1]⁺=502.3.

Step 4: To a solution of (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene (200 mg, 0.3984 mmol) in dichloromethane (20.0 mL), triethylamine (2.0 mL, 14.41 mmol) and di-tert-butyl dicarbonate (175 mg, 0.7968 mmol) were successively added. The mixture was stirred at room temperature for two hours. Upon completion of the reaction, the reaction mixture was extracted with ethyl acetate (150 mL) and water (100 mL), and the organic phase was dried over anhydrous sodium sulfate, filtered, and the filtrate was evaporated under reduced pressure. The crude product was purified by flash silica gel column chromatography (ethyl acetate/petroleum ether: 0-70%) to obtain tert-butyl (6R,9S)-2-(8-ethyl-3-(methoxymethoxy)naphthalen- 1-yl)- 12-fluoro-5a,6,7,8,9,10-hexahydro-5H-4-oxa-3,10a,11,13,14-pentaaza-6,9-methanonaphtho[1,8-ab]heptalene-14-carboxylate (150 mg, yield: 62%). ES-API:[M+1]⁺=602.3.

Step 5: Under ice-water bath condition, to a solution of (2,6-dimethyltetrahydro-1H-pyrroloquinoline-7a(5H)-yl)methanol (28.0 mg, 0.1661 mmol) in tetrahydrofuran (5.0 mL), sodium hydride (13.28 mg, 0.3322 mmol) was added, and the mixture reacted at room temperature for 0.5 hours. After 0.5 hours, tert-butyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene-14-carboxylate (50 mg, 0.0830 mmol) was added to the reaction system and reacted at room temperature for 1-2 hours. Upon completion of the reaction, ice water (50 mL) was added to the system, and extracted with dichloromethane (50 mLX2). The combined dichloromethane phases were dried over anhydrous sodium sulfate, filtered, and the filtrate was concentrated and purified by flash silica gel column chromatography (methanol/dichloromethane: 0-10%) to obtain tert-butyl (6R,9S)-12-((2,6-dimethylenetetrahydro-1H-pyrrolizin-7a(5H)-yl)methoxy)-2-(8-ethyl-3-(methoxymethoxy)naphthalen-1-yl)-Sa,6,7,8,9,10-hexahydro-5H-4-oxa-3,10a,11,13,14-pentaaza-6,9-metbanonaphtho[1,8-ab]heptalene-14-carboxylate (110 mg, crude product). ES-API:[M+1]⁺=747.3.

Step 6: To a solution of tert-butyl (6R,9S)-12-((2,6-dimethylenetetrahydro-1H-pyrrolizin-7a(5H)-yl)methoxy)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (110 mg, crude product) in acetonitrile (6.0 mL), 4M hydrochloric acid in dioxane (2.0 mL, 8.0 mmol) was added under ice water bath condition, and the mixture reacted for 0.5 hours. Upon completion of the reaction, the reaction mixture was concentrated to dryness and dichloromethane (20 mL) was added, followed by triethylamine (3.0 mL) under ice-water bath condition. After stirred for 10 minutes, the mixture was extracted with dichloromethane (100 mLX1) and water (50 mLX1). The dichloromethane phase was dried over anhydrous sodium sulfate, filtered, and the filtrate was evaporated under reduced pressure to obtain a crude product, which was then purified by preparative HPLC (ammonium bicarbonate method 1) to obtain 4-((6R,9S)-12-((2,6-dimethylenetetrahydro-1H-pyrrolizin-7a(5H)-yl)methoxy)-5a,6,7,8,9, 1 0-hexahydro-5H-4-oxa-3, 10a, 11, 1 3,14-pentaaza-6,9-methanonaphtho[1,8-ab]heptalen-2-yl)-5-ethylnaphthalen-2-ol (Z175-1, 7.47 mg, total yield for 2 steps: 14.9%). ES-API:[M+1]⁺=603.3.

### Example 4: Synthesis of 4-((6R,9S)-12-(3-(dimethylamino)azetidin-1-yl)-5a,6,7,8,9,10-hexahydro-5H-4-oxa-3,10a,11,13,14-pentaaza-6,9-methanonaphtho[1,8-ablheptalen-2-yl)-5-ethylnaphthalen-2-ol (Z308)

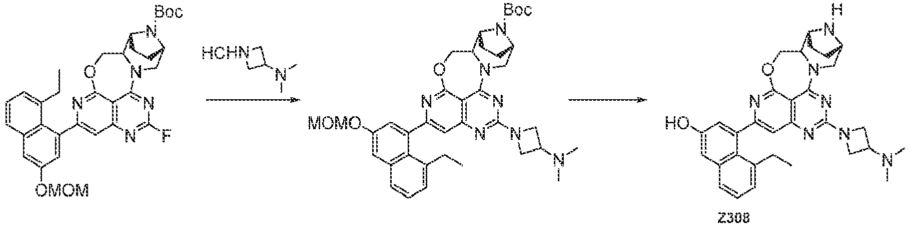

Step 1: Under ice-water bath condition, N,N-diisopropylethylamine (430 mg, 3.322 mmol) was added to a dioxane (5.0 mL) solution of N,N-dimethylazetidine-3-amine hydrochloride (45.0 mg, 0.332 mmol). Finally, tert-butyl (6R,9S)-2-(8-ethyl-3-(metboxymetboxy)naphthalen-1-yl)-12-fluoro-5a,6,7,8,9,10-hexahydro-5H-4-oxa-3,10a,11,13,14-pentaaza-6,9-methanonaphtho[1,8-ab]heptalene-14-carboxylate (100 mg, 0.1660 mmol) was added. The reaction was proceeded at 110°C for two hours. After two hours, ice water (50 mL) was added to the system, and extracted with dichloromethane (50 mLX2). The combined dichloromethane phases were dried over anhydrous sodium sulfate, filtered, and the filtrate was evaporated under reduced pressure. The crude product was purified by flash silica gel column chromatography (methanol/dichloromethane: 0-10%) to obtain tert-butyl (6R,9S)-12-(3-(dimethylamino)azetidin-1-yl)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (110 mg, yield: 97%). ES-API:[M+l]⁺=682.3.

Step 2: Tert-butyl (6R,9S)-12-(3-(dimethylamino)azetidin-1-yl)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (110 mg, 0.1612 mmol) was dissolved in acetonitrile (6.0 mL). Under ice water bath condition, 4M hydrochloric acid in dioxane (2.0 mL, 8.0 mmol) was added, and the mixture reacted under ice water bath condition for 0.5 hours. Upon completion of the reaction, the reaction mixture was concentrated to dryness and dichloromethane (20 mL) was added, followed by triethylamine (3.0 mL) under ice-water bath condition. After stirred for 10 minutes, the mixture was extracted once with dichloromethane (100 mL) and water (50 mL). The dichloromethane phase was dried over anhydrous sodium sulfate, filtered, and the filtrate was evaporated under reduced pressure to obtain a crude product, which was then purified by preparative HPLC (ammonium bicarbonate method 1) to obtain 4-((6R,9S)-12-(3-(dimethylamino)azetidin-1-yl)-5a,6,7,8,9,10-hexahydro-5H-4-axa-3,10a,11,13,14-pentaaza-6,9-methanonaphtho[1,8-ab]heptalen-2-yl)-5-ethylnaphthalen-2-ol (Z308, 14.92 mg, yield: 16.8%). ES-API:[M+1]⁺=538.3.

### Example 5 Synthesis of (1R,2R)-2-(((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)ozy)cyclopentan-1-ol (Z718)

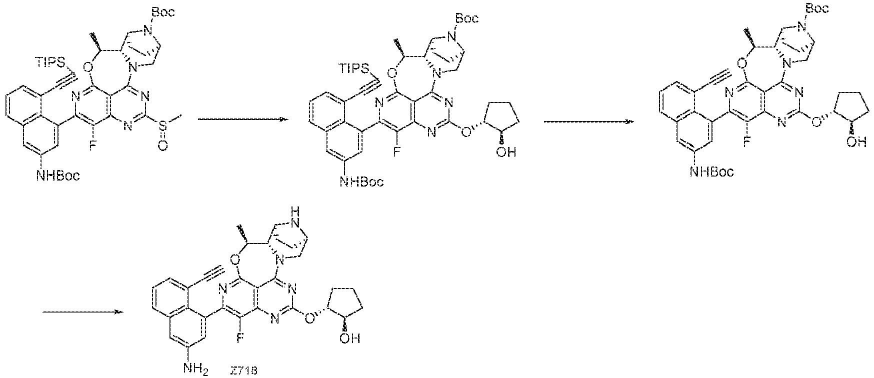

Step 1: trans-Cyclopentane-1,2-diol (46 mg, 0.45 mmol) was dissolved in tetrahydrofuran (5 mL), and at 0°C, 60% sodium hydride (11 mg, 0.27 mmol) was added. The reaction mixture was stirred at this temperature for 30 minutes, after which a tetrahydrofuran solution (2 mL) of tert-butyl (5 S,SaS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (80 mg, 0.092 mmol) was added dropwise and stirred at the same temperature for another 30 minutes. The reaction mixture was added to ethyl acetate (40 mL) and washed successively with water (10 mL) and saturated brine (15 mL). The organic phase was dried over anhydrous sodium sulfate, filtered, and the filtrate was concentrated under reduced pressure. The crude product was purified by flash silica gel column chromatography (tetrahydrofuran/petroleum ether: 0-30%) to obtain the target compound tert-butyl (SS,SaS,6S,9R)-2-(3-((tert-butoxycarbonyl)amino)-8-((triisopropylsilyl)ethynyl)naphthalen-1-yl)-1-fluoro-12-(((1R,2R)-2-hydroxycyclopentyl)oxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, yield 59.9%), a pale yellow solid. ES-API: [M+H]⁺=923.3.

Step 2: tert-butyl (SS,SaS,bS,9R)-2-(3-((tert-butoxycarbonyl)amino)-8-((triisopropylsilyl)ethynyl)naphthalen-1-yl)-1-fluoro-12-(((1R,2R)-2-hydroxycyclopentyl)oxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, 0.054 mmol) was dissolved in N,N-dimethylformamide (3 mL). At room temperature, cesium fluoride (82 mg, 0.54 mmol) was added, and the mixture was stirred at room temperature for 30 minutes. The reaction mixture was added to ethyl acetate (30 mL) and washed successively with water (5 mL), dilute brine (10 mLX3), and saturated brine (10 mL). The organic phase was dried over anhydrous sodium sulfate, filtered, and the filtrate was concentrated under reduced pressure to obtain the target compound tert-butyl (SS,SaS,6S,9R)-2-(3-((tert-butoxycarbonyl)amino)-8-ethynylnaphthalen-1-yl)-1-fluoro-12-(((1R,2R)-2-hydroxycyclopentyl)oxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, yield 96.3%), a pale brown solid. ES-API:[M+H]⁺=767.2.

Step 3: tert-butyl (5S,5aS,6S,9R)-2-(3-((tert-butoxycarbonyl)amino)-8-ethynylnaphthalen-1-yl)-1-fluoro-12-(((1R,2R)-2-hydroxycyclopentyl)oxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.018 mmol) was dissolved in ethyl acetate (1 mL). At 0°C, 4M hydrochloric acid in 1,4-dioxane (1.5 mL) was added, and the mixture was stirred at room temperature for 2 hours. The reaction mixture was concentrated under reduced pressure. The crude product was purified by preparative HPLC (ammonia method) to obtain (1R,2R)-2-(((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)cyclopentan-1-ol (Z718, 6 mg, yield: 20.4%, white solid). ES-API: [M+H]⁺= 567.2.

### Example 6: Synthesis of 5-ethyl-4-((SaS,6S,9R)-1.-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-oI (Z140-1)

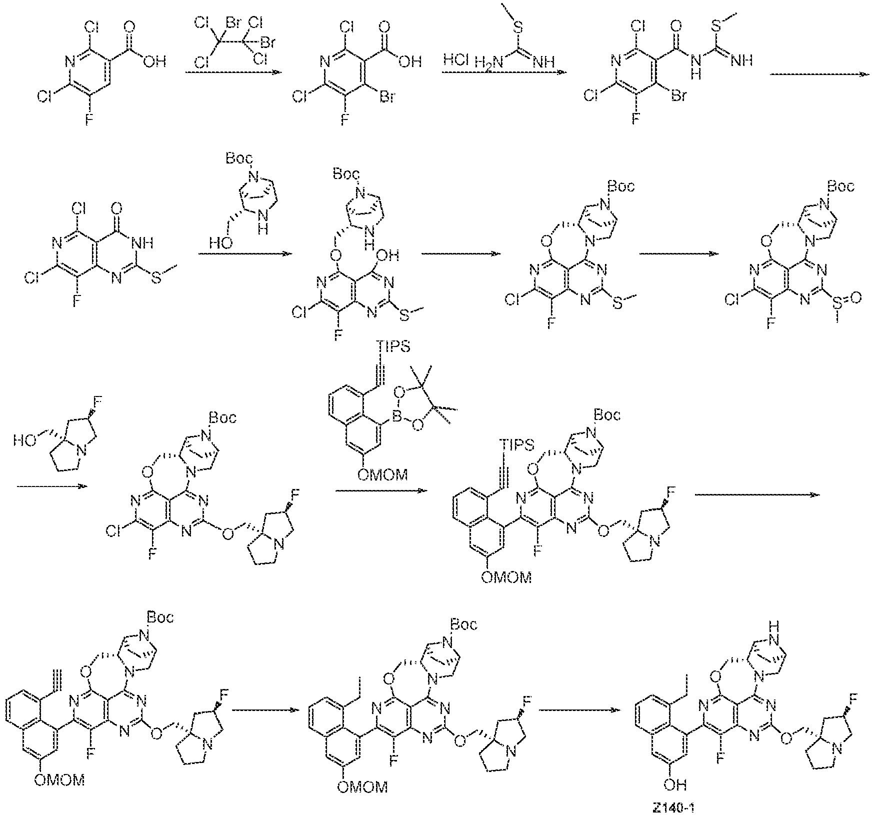

Step 1: Under condition at -78°C, 2,6-dichloro-5-fluoronicotinic acid (8.0 g, 38.095 mmol) was dissolved in dry tetrahydrofuran (150 mL). Methyl lithium (47.6 mL, 76.19 mmol, 1.6 M) was added dropwise. After the addition was completed, the mixture was stirred at -20°C for 2 hours. The system was then cooled again to -78°C, and a tetrahydrofuran solution (30 mL) of 1,2-dibromo-1,1,2,2-tetrachloroethane (12.405 g, 38.095 mmol) was added dropwise over 30 minutes. After the addition was complete, the reaction was proceeded at 0°C for 1.5 hours. Upon completion of the reaction, the reaction mixture was poured into ice water (500 mL) and washed with chloroform (100 mLX1). The aqueous phase was adjusted to a pH of about 3 with 3M hydrochloric acid, evaporated under reduced pressure, and the crude product was purified by column chromatography (dichloromethane:methanol = 100:0-90:10, v/v) to obtain 4-bromo-2,6-dichloro-5-fluoronicotinic acid (7.6 g, yield: 88%). ES-API:[M+H]⁺=287.9.

Step 2: At room temperature, 4-bromo-2,6-dichloro-5-fluoronicotinic acid (6.0 g, 20.90 mmol) was dissolved in sulfolane (40.0 mL). N,N-Dimethylformamide (16 drops) was added to the reaction system, which was then stirred at 100°C for 1 hour. The solvent was evaporated under reduced pressure. At 0°C, the crude product was added to a mixed solution of tetrahydrofuran (100 mL) and saturated sodium bicarbonate (100 mL), followed by the addition of amino(methyliminothio)acetate (12.91 g, 68.607 mmol). The mixture was stirred at the same temperature for 30 minutes, then extracted with ethyl acetate (200 mL). The organic phase was washed with brine, dried over anhydrous sodium sulfate, filtered, and concentrated to obtain a yellow solid, methyl (4-bromo-2,6-dichloro-5-fluorobenzoyl)amino(methyliminothio)acetate (2.23 g, yield: 29.8%). ES-API:[M+1]⁺=359.5.

Step 3: Under nitrogen protection, methyl (4-bromo-2,6-dichloro-5-fluorobenzoyl)amino(methyliminothio)acetate (880 mg, 2.45 mmol) and N,N-diisopropylethylamine (1.4 mL, 8.37 mmol) were sealed in dioxane (12 mL) and heated to 100°C and reacted for 16 hours. The reaction mixture was concentrated until the volume was ~5 mL. Water (15 mL) and 2M hydrochloric acid aqueous solution (3 mL) were added to the residue. After the formation of precipitate, the solution was filtered and the filter cake was washed with water (10 mL), dried under reduced pressure to obtain a yellow solid, 5,7-dichloro-8-fluoro-2-(methylthio)pyrido[4,3-d]pyrimidin-4(3H)-one (350 mg, yield: 51%). ES-API:[M+H]⁺=280.0.

Step 4: At 0°C, tert-butyl (1S,2S,5R)-2-(hydroxymethyl)-3,8-diazabicyclo[3.2.1]octane-8-carboxylate (813.53 mg, 3.357 mmol) was added to a suspension of sodium hydride (427.29 mg, 10.682 mmol) in tetrahydrofuran (20 mL). The mixture was stirred at the same temperature for 10 minutes. 5,7-Dichloro-8-fluoro-2-(methylthio)pyrido[4,3-d]pyrimidin-4(3H)-one (854 mg, 3.052 mmol) was added, and the mixture was heated and stirred at 60°C for 1 hour. Upon completion of the reaction, the mixture was quenched with water and extracted with dichloromethane/methanol (10:1, 25 mLX3). The organic phase was dried over anhydrous sodium sulfate, filtered, and concentrated to obtain tert-butyl (1S,2S,5R)-2-(((7-chloro-8-fluoro-4-hydroxy-2-(methylthio)pyrido[4,3-d]pyrimidin-5-yl)oxy)methyl)-3,8-diazabicyclo[3.2.1]octane-8-carboxylate (1451 mg, 2.992 mmol, yield: 98.02%), a yellow solid. ES-API:[M+H]⁺=486.1. ES-API:[M+H]⁺=287.9.

Step 5: To a dichloromethane (20 mL) solution of tert-butyl (1S,2S,5R)-2-(((7-chloro-8-fluoro-4-hydroxy-2-(methylthio)pyrido[4,3-d]pyrimidin-5-yl)oxy)methyl)-3,8-diazabicyclo[3.2.1]octane-8-carboxylate (1451 mg, 2.992 mmol) and N,N-diisopropylethylamine (4.957 mL, 29.916 mmol), propylphosphonic anhydride (50% in ethyl acetate) (11422.33 mg, 17.949 mmol) was added and stirred at room temperature for 3 hours. The reaction mixture was quenched with water and extracted with dichloromethane. The organic phase was dried over anhydrous sodium sulfate, filtered, and concentrated. The obtained solid was suspended in acetonitrile and stirred at room temperature for 10 minutes. The mixture was filtered, and the filter cake was collected to obtain tert-butyl (5aS,6S,9R)-2-chloro-1-fluoro-12-(methylthio)-5a,6,7,8,9,10-hexahydro-5H-4-oxa-3,10a,11,13,14-pentaza-6,9-methanonaphtho[1,8-ab]heptene-14-carboxylate (623 mg, yield: 45.0%), a white solid. ES-API:[M+H]⁺=468.1.

Step 6: At 0°C, to a dichloromethane (15 mL) solution of tert-butyl (5aS,6S,9R)-2-chloro-1-fluoro-12-(methylthio)-5a,6,7,8,9,10-hexahydro-5H-4-oxa-3,10a, 11,13,14-pentaza-6,9-methanonaphtho[1,8-ab]heptene-14-carboxylate (540 mg, 1.153 mmol), m-chloroperbenzoic acid (322.61 mg, 1.589 mmol) was added. The mixture was stirred at room temperature for 1 hour. The reaction mixture was quenched with saturated sodium bisulfite solution and extracted with dichloromethane (100 mLX2). The organic phase was dried over anhydrous sodium sulfate and concentrated to obtain a crude product, which was then purified by flash silica gel column chromatography (0-10% methanol/dichloromethane) to obtain tert-butyl (5aS,6S,9R)-2-chloro-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (452 mg, yield: 81.0%), a white solid. ES-API:[M+H]⁺=484.1.

Step 7: At 0°C, sodium hydride (45.07 mg, 1.878 mmol) was added to a tetrahydrofuran solution of ((2R,7aS)-2-fluorotetrahydro-1H-pyrroloquinoline-7a(5H)-yl)methanol (239.16 mg, 1.502 mmol). The reaction mixture was stirred at room temperature and reacted for 10 minutes. The mixture was cooled to 0°C, and a tetrahydrofuran solution (5 mL) of tert-butyl (5aS,6S,9R)-2-chloro-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (363.5 mg, 0.751 mmol) was added. The reaction mixture was stirred at 0°C and reacted for 1 hour, quenched with saturated sodium bicarbonate solution, and extracted with ethyl acetate. The organic phase was washed with saturated brine, dried over anhydrous sodium sulfate, filtered, and concentrated. The crude product was purified by flash column chromatography (0-5% methanol/dichloromethane) to obtain tert-butyl (5aS,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (134mg, yield: 30.85%), yellow solid. ES-API: [M+H]⁺=579.2.

Step 8: To a mixture of tert-butyl (5aS,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (134.0 mg, 0.233 mmol), triisopropyl((6-(methoxymethoxy)-8-(4,4,5,5-tetramethyl-1,3,2-dioxaborolane-2-yl)naphthalen-1-yl)ethynyl)silane (230.0 mg, 0.466 mmol) in dioxane (6 mL) and water (1.5 mL), potassium phosphate (147.55 mg, 0.695 mmol), and palladium(II) [bis(butyl)(1-adamantyl)phosphine] (2-amino-1,1'-biphenyl) (1.95 mg, 0.003 mmol) were added. The mixture was bubbled with N₂ and microwave reacted at 80°C for 40 minutes. Upon completion of the reaction, the mixture was extracted with ethyl acetate (30 mLX2) and saturated brine (30 mL). The ethyl acetate phases were combined, dried over anhydrous sodium sulfate, filtered, and concentrated under reduced pressure. The crude product was purified by flash silica gel column chromatography (0-4% methanol/dichloromethane) to obtain tert-butyl (SaS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (164 mg, yield: 77.33%), a yellow solid. ES-API:[M+H]⁺=911.5.

Step 9: To a N,N-dimethylformamide (3.0 mL) solution of tert-butyl (SaS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (165 mg, 0.181 mmol), cesium fluoride (127.55 mg, 0.840 mmol) was added. The reaction mixture was stirred at room temperature and reacted for 0.5 hours. Upon completion of the reaction, the reaction mixture was quenched with water and extracted with ethyl acetate. The organic phase was washed with saturated brine, dried over anhydrous sodium sulfate, filtered, and concentrated to obtain tert-butyl (5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, crude product), yellow oil. ES-API: [M+H]⁺=755.3.

Step 10: To a methanol (5.0 mL) solution of tert-butyl (5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (100 mg, 0.1324 mmol), palladium on carbon (75.0 mg, 0.061 mmol) was added. The mixture was purged with hydrogen and reacted under a hydrogen atmosphere at room temperature for 1 hour. The reaction mixture was filtered and concentrated. The crude product was purified by flash silica gel column chromatography (0-3% methanol/dichloromethane) to obtain tert-butyl (5aS,6S,9R)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (70 mg, yield: 70%), a yellow solid. ES-API: [M+H]⁺=759.4.

Step 11: To a mixture of tert-butyl (5aS,6S,9R)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (80 mg, 0.105 mmol), acetonitrile (2.0 mL), and 4M hydrochloric acid in dioxane solution (0.500 mL, 2.000 mmol) were reacted at 0°C for 1 hour. The reaction mixture was concentrated and the crude product was purified by preparative HPLC (ammonium bicarbonate method 1) to obtain 5-ethyl-4-((SaS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z140-1, 9.65 mg, yield: 14.9%), a yellow solid. ES-API:[M+1]⁺=615.3.

### Example 7: Synthesis of (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,1.1,13,1.4-pentaaza-6,9-methanonaphtho[1,8-ablheptalene (Z350)

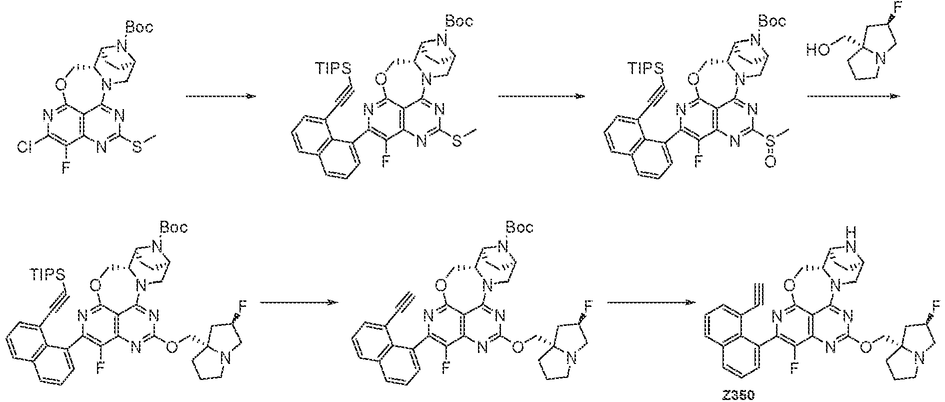

Step 1: To a mixture of tert-butyl (1S,2S,5R)-2-(((7-chloro-8-fluoro-(2-methylthio)-4-oxa-3,4-dihydropyrido[4,3-d]pyrimidin-5-yl)oxy)methyl)-3,8-diazabicyclo[3.2.1]octane-8-carboxylate (150.0 mg, 0.321 mmol) and triisopropyl((8-(4,4,5,5 tetramethyl-1,3,2-dioxaborolane-2-yl)naphthalen-1-yl)ethynyl)silane (220.0 mg, 0.506 mmol) in dioxane (6 mL) and water (1.5 mL), potassium phosphate (300 mg, 1.415 mmol), and palladium(II) [bis(butyl)(1-adamantyl)phosphine] (2-amino-1,1'-biphenyl) (100 mg, 0.137 mmol) were added. The mixture was bubbled with nitrogen and microwave reacted at 80°C for 40 minutes. Upon completion of the reaction, the reaction mixture was extracted with ethyl acetate (30 mLX2) and saturated brine (30 mL). The ethyl acetate phases were combined, dried over anhydrous sodium sulfate, filtered. The filtrate was concentrated under reduced pressure. The crude product was purified by column chromatography (methanol/dichloromethane=0-4%) to obtain tert-butyl (5aS,6S,9R)-1-fluoro-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (272 mg, crude product), a yellow solid. ES-API: [M+H]⁺=800.4.

Step 2: At 0°C, to a dichloromethane (15 mL) solution of tert-butyl (SaS,6S,9R)-1-fluoro-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (272 mg, crude product), m-chloroperbenzoic acid (67.12 mg, 0.389 mmol) was added. The mixture was stirred at room temperature for 1 hour. The reaction mixture was quenched with saturated sodium sulfate solution and extracted with dichloromethane (100 mLX2). The organic phase was dried over anhydrous sodium sulfate and concentrated. The crude product was purified by silica gel column chromatography (methanol/dichloromethane=0-10%) to obtain tert-butyl (5aS,6S,9R)-1-fluoro-12-(mefhylsulfmyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (230 mg, total yield for 2 steps: 93.8%), a white solid. ES-API:[M+H]⁺=756.3.

Step 3: At 0°C, sodium hydride (50.0 mg, 1.25 mmol) was added to a tetrahydrofuran (10.0 mL) solution containing ((2R,7aS)-2-fluorotetrahydro-1H-pyrroloquinoline-7a(5H)-yl)methanol (100.0 mg, 0.628 mmol). After stirred at this temperature for 10 minutes, a tetrahydrofuran (5 mL) solution of tert-butyl (5aS,6S,9R)-1-fluoro-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-medmonaphtho[1,8-ab]heptalene-14-carboxylate (230.0 mg, 0.304 mmol) was added. The mixture was stirred at 0°C and reacted for 1 hour, quenched with saturated sodium bicarbonate solution, and extracted with ethyl acetate. The organic phase was washed with saturated brine, dried over anhydrous sodium sulfate, filtered, and concentrated. The crude product was purified by silica gel column chromatography (methanol/dichloromethane=0-5%) to obtain tert-butyl (SaS,6S,9R)-1-fluoro-12-(((2R,7aS)-2-fluorotetrahydro-1H-pyrrolizin-7a(5H)-yl)methoxy)-2-(8-((triisopropylsilyl)ethynyl)naphthalen-1 -yl)-5a,6,7,8,9,10-hexahydro-5H-4-oxa-3,10a, 11,13,14-pentaaza-6,9-methanonaphtho[1,8-ab]heptalene-14-carboxylate (260 mg, crude product), a yellow solid. ES-API: [M+H]⁺=851.5.

Step 4: To a N,N-dimethylformamide (5.0 mL) solution of tert-butyl (SaS,6S,9R)-1-fluoro-12-(((2R,7aS)-2-fluorotetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (260 mg, crude product), cesium fluoride (1000 mg, 6.583 mmol) was added. The reaction mixture was stirred at room temperature and reacted for 0.5 hours. Upon completion of the reaction, the reaction mixture was quenched with water and extracted with ethyl acetate. The organic phase was washed with saturated brine, dried over anhydrous sodium sulfate, filtered, and concentrated to obtain tert-butyl (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a, 11,13,14-pentaaza-6,9-methanonaphtho[1,8-ab]heptalene-14-carboxylate (120 mg, total yield for 2 steps: 57%), a yellow oil. ES-API: [M+H]⁺=695.3.

Step 5: The reaction mixture containing tert-butyl (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (120 mg, 0.173 mmol), acetonitrile (5.0 mL), and 4M hydrochloric acid/dioxane solution (2.0 mL, 8.000 mmol) reacted at 0°C for 1 hour. The mixture was concentrated, and the crude product was purified by preparative HPLC (ammonium bicarbonate method 1) to obtain (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z350, 34mg, yield: 33%), yellow solid. ES-API:[M+1]⁺=595.3. ¹H NMR (500 MHz, CD₃OD)δ 8.07 (d, *J* = 8.2 Hz, 1H), 8.03 (dd, *J* = 7.8, 3.6 Hz, 1H), 7.73 (ddd, *J* = 13.6, 7.2, 1.2 Hz, 1H), 7.67 -7.47 (m, 3H), 5.30 (d, *J* = 54.1 Hz, 1H), 5.05 (ddd, *J* = 13.5, 6.6, 2.2 Hz, 1H), 4.59 (ddd, *J* = 13.2, 8.9, 2.0 Hz, 1H), 4.44 (ddd, *J* = 13.2, 7.4, 5.9 Hz, 1H), 4.28 (t, *J* = 10.1 Hz, 1H), 4.21 (dd, *J* = 10.5, 5.4 Hz, 1H), 4.12 (d, *J* = 6.2 Hz, 1H), 3.72 (s, 1H), 3.63 (d, *J* = 5.2 Hz, 1H), 3.27 -3.16 (m, 4H), 3.02 (td, *J* = 9.8, 5.9 Hz, 1H), 2.29 -2.21 (m, 1H), 2.16 -2.10 (m, 1H), 1.99 (dq, *J* = 13.5, 6.7 Hz, 2H), 1.95 -1.75 (m, 5H).

### Example 8: Synthesis of 4-((5aS,6S,9R)-12-((2,6-dimethylenetetrahydrn-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethynyl-6-fluoronaphthalen-2-ol (Z380)

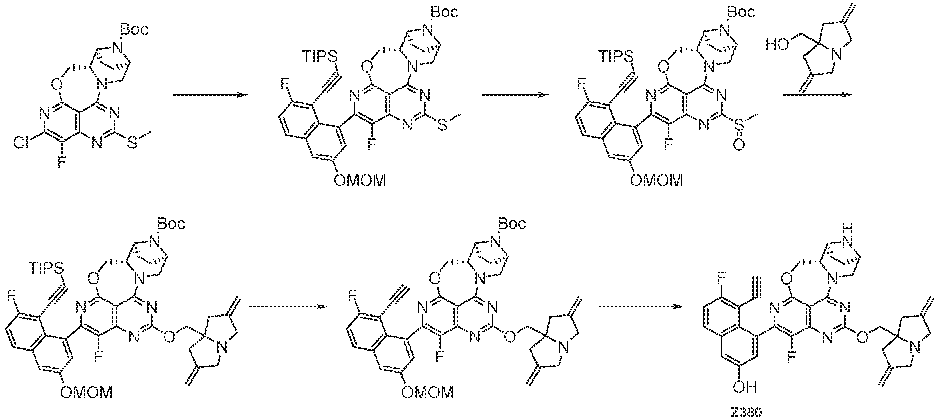

Step 1: To a mixture of tert-butyl (1S,2S,5R)-2-(((7-chloro-8-fluoro-(2-methylthio)-4-oxa-3,4-dihydropyrido[4,3-d]pyrimidin-5-yl)oxy)methyl)-3,8-diazabicyclo[3.2.1]octane-8-carboxylate (100 mg, 0.214 mmol) and ((2-fhioro-6-(methoxymethoxy)-8-(4,4,5,5-tetramethyl-1,3,2-dioxaborolane-2-yl)naphthalen-1-yl)ethynyl)triisopropylsilane (200.0 mg, 0.390 mmol) in tetrahydrofuran (6 mL) and water (1.5 mL), potassium phosphate (200 mg, 0.943 mmol), and palladium(II) [bis(butyl)(1-adamantyl)phosphine] (2-amino-1,1'-biphenyl) (50 mg, 0.069 mmol) were added. The mixture was bubbled with nitrogen and microwave reacted at 80°C for 40 minutes. Upon completion of the reaction, the reaction mixture was extracted with ethyl acetate (30 mLX2) and saturated brine (30 mL). The ethyl acetate phases were combined, dried over anhydrous sodium sulfate, filtered. The filtrate was concentrated under reduced pressure. The crude product was purified by silica gel column chromatography (methanol/dichloromethane=0-4%) to obtain tert-butyl (SaS,6S,9R)-1-fluoro-2-(7-fluoro-3-(methoxymethoxy)-8-((triisopropylsilyl)ethynyl)naphthalen-1-yl)-12-(methylthio)-Sa,6,7,8,9,10-hexahydro-SH-4-oxa.-3,10a,11,13,14-pentaaza-6,9-methanonaphtho[1,8-ab]heptalene-14-carboxylate (210 mg, crude product), a yellow solid. ES-API: [M+H]⁺=818.3.

Step 2: At 0°C, to a dichloromethane (15 mL) solution of tert-butyl (SaS,6S,9R)-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (210 mg, crude product), m-chloroperbenzoic acid (55 mg, 0.320 mmol) was added. The mixture was stirred at room temperature for 1 hour. The reaction mixture was quenched with saturated sodium bisulfite solution and extracted with dichloromethane (100 mLX2). The organic phase was dried over anhydrous sodium sulfate and concentrated. The crude product was purified by silica gel column chromatography (methanol/dichloromethane=0-10%) to obtain tert-butyl (5aS,6S,9R)-1-fluoro-2-(7-fluoro-3-(methoxymethoxy)-8-((triisopropylsilyl)ethynyl)naphthalen-1-yl)-12-(methylsulfinyl)-Sa,6,7,8,9,10-hexahydro-SH-4-oxa-3,10a,11,13,14-pentaaza-6,9-methanonaphtho[1,8-ab]heptalene-14-carboxylate (210 mg, crude product), a white solid. ES-API:[M+H]⁺=834.4.

Step 3: At 0°C, sodium hydride (50.0 mg, 1.25 mmol) was added to a tetrahydrofuran (10.0 mL) solution containing (2,6-dimethyltetrahydro-1H-pyrroloquinoline-7a(5H)-yl)methanol (65.0 mg, 0.393 mmol). After the mixture stirred and reacted for 10 minutes at this temperature, tert-butyl (5aS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (160.0 mg, 0.192 mmol) in tetrahydrofuran (5 mL) was added. The reaction mixture was stirred at 0°C and reacted for 1 hour, quenched with saturated sodium bicarbonate solution, and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (211 mg, crude product), a yellow solid. ES-API: [M+H]⁺=935.5.

Step 4: To a N,N-dimethylformamide (5.0 mL) solution containing tert-butyl (5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (211 mg, crude product), cesium fluoride (1000 mg, 6.583 mmol) was added. The reaction mixture was stirred at room temperature and reacted for 0.5 hours, Upon completion of the reaction, the reaction mixture was quenched with water. The mixture was extracted with ethyl acetate. The organic phase was washed with saturated brine, dried over anhydrous sodium sulfate, filtered, and concentrated to obtain tert-butyl (5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-meffimonaphtho[1,8-ab]heptalene-14-carboxylate (200 mg, crude product), a yellow oil. ES-API: [M+H]⁺=773.3.

Step 5: A reaction solution containing tert-butyl (5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methanonaphtho[1,8-ab]heptalene-14-carboxylate (200 mg, crude product), acetonitrile (5.0 mL), and 4M hydrochloric acid/dioxane solution (2.0 mL, 8.000 mmol) reacted at 0°C for 1 hour. The mixture was concentrated, and the crude product was purified by preparative HPLC (ammonium bicarbonate method 1) to obtain 4-((5aS,6S,9R)-12-((2,6-dimethylenetetrahydro-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethynyl-6-fluoronaphthalen-2-ol (Z380, 8.95mg, total yield for 5 steps: 5.23%), yellow solid. ES-API:[M+1]⁺=635.3. ¹H NMR (500 MHz, CD₃OD) δ 7.86 -7.78 (m, 1H), 7.37 -7.27 (m, 2H), 7.19 (dd, *J* = 35.4, 2.5 Hz, 1H), 5.23 -4.95 (m, 5H), 4.69 -4.01 (m, 5H), 3.86 -3.46 (m, 5H), 3.35 (s, 2H), 3.21 (t, *J* = 12.9 Hz, 1H), 2.78 (d, *J* = 16.5 Hz, 2H), 2.58 (d, *J* = 16.4 Hz, 2H), 2.08 - 1.65 (m, 5H).

### Example 9: Synthesis of 4-((5aS,6S,9R)-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino [5,6,7-de]quinazolin-2-yl)-5-ethylnaphthalen-2-ol (Z381)

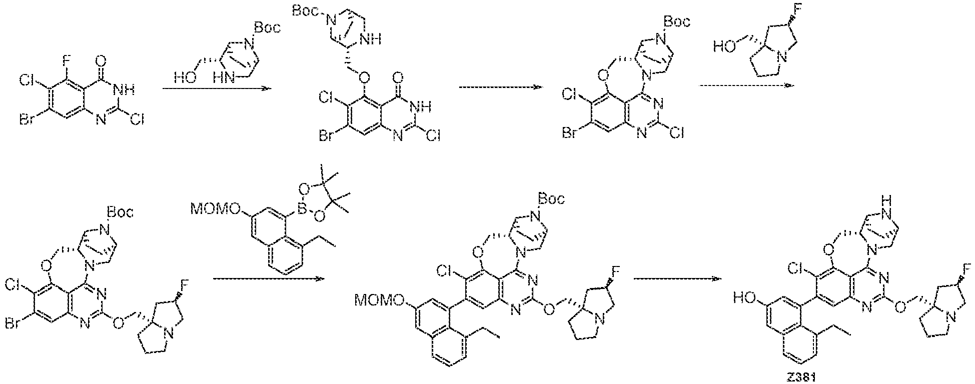

Step 1: At 0°C, to a suspension of sodium hydride (60% dispersed in oil) (32 mg, 0.81 mmol) in tetrahydrofuran (10 mL), tert-butyl (lS,2S,5R)-2-(hydroxymethyl)-3,8-diazabicyclo[3.2.1]octane-8-carboxylate (131 mg, 0.54 mmol) was added, and the mixture was stirred at this temperature for 10 minutes. 7-Bromo-2,6-dichloro-5-fluoroquinazolin-4(3H)-one (170 mg, 0.54 mmol) was then added and the mixture was heated and stirred at 60°C for 1 hour. Upon completion of the reaction, the reaction mixture was quenched with water and extracted with dichloromethane/methanol (10:1, 25 mLX3). The organic phase was dried over anhydrous sodium sulfate, evaporated to obtain tert-butyl (1S,2S,5R)-2-(((7-bromo-2,6-dichloro-4-oxo-3,4-dihydroquinazolin-5-yl)oxy)methyl)-3,8-diazabicyclo[3.2.1]octane-8-carboxylate (150 mg, yield: 51.0%). ES-API:[M+H]⁺=533.2.

Step 2: To a solution of tert-butyl (1S,2S,5R)-2-(((7-bromo-2,6-dichloro-4-oxo-3,4-dihydroquinazolin-5-yl)oxyhnethyl)-3,8-diazabicyclo[3.2.1]octane-8-carboxylate (110 mg, 0.21 mmol) and N,N-diisopropylethylamine (80 mg, 0.62 mmol) in anhydrous dichloromethane (10 mL), 1H-benzotriazole-1-yloxytris(pyrrolidino)phosphonium hexafluorophosphate (86 mg, 0.42 mmol) was added. The mixture was stirred at room temperature for 16 hours. The reaction mixture was quenched with water and extracted with dichloromethane (25 mLX3), washed with saturated brine (10 mL). The organic phase was dried over anhydrous sodium sulfate, filtered, and concentrated. The crude product was purified by reverse-phase preparative HPLC (trifluoroacetic acid method) to obtain tert-butyl (5aS,6S,9R)-2-bromo-3,13-dichloro-5a,6,7,8,9,10-hexahydro-5H-6,9-epiminooxa[2',1':3,4][1,4]oxazonino[5,6,7-de]quinazolin-15-oate (64 mg, yield: 60.3%). ES-API:[M+H]⁺= 515.1.

Step 3: To a solution of tert-butyl (5aS,6S,9R)-2-bromo-3,13-dichloro-5a,6,7,8,9,10-hexahydro-5H-6,9-epiminoazepino[2',1':3,4][1,4]oxazepino[5,6,7-de]quinazoline-15-carboxylate (45 mg, 0.09 mmol) in anhydrous N,N-dimethylformamide (10 mL), ((2R,7aS)-2-fluorotetrahydro-1H-pyrroloquinoline-7a(5H)-yl)methanol (29 mg, 0.18 mmol), potassium fluoride (51 mg, 0.9 mmol), and 4Å molecular sieves were added. Under nitrogen protection, the reaction mixture was stirred at 120°C for 16 hours. Upon completion of the reaction, the reaction mixture was quenched with water and extracted with dichloromethane (25 mLX3), washed with saturated brine (10 mL). The organic phase was dried over anhydrous sodium sulfate, filtered, and concentrated. The crude product was purified by preparative HPLC (trifluoroacetic acid method) to obtain tert-butyl (5aS,6S,9R)-2-bromo-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (22 mg, yield: 38.4%). ES-API:[M+H]⁺= 638.2.

Step 4: To a mixture of tert-butyl (SaS,6S,9R)-2-bromo-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (22 mg, 0.03 mmol) and 2-(8-ethyl-3-(methoxymethoxy)naphthalen-1-yl)-4,4,5,5-tetramethyl-1,3,2-dioxaborolane (17 mg, 0.05 mmol) in tetrahydrofuran (6 mL) and water (1.0 mL), potassium phosphate (21 mg, 0.1 mmol), and palladium(II) [bis(butyl)(1-adamantyl)phosphine] (2-amino-1,1'-biphenyl) (15 mg) were added. Under nitrogen protection, the mixture reacted at 60°C for 2 hours. Upon completion of the reaction, the reaction mixture was extracted with ethyl acetate (30 mLX2) and saturated brine (10 mL). The ethyl acetate phases were combined, dried over anhydrous sodium sulfate, filtered. The filtrate was concentrated under reduced pressure. The crude product was purified by thin-layer chromatography (methanol/dichloromethane=1:15) to obtain tert-butyl (5aS,6S,9R)-3-chloro-2-(8-ethyl-3-(methoxymethoxy)naphthalen-1-yl)-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (10 mg, yield: 43%). ES-API: [M+H]⁺=774.1.

Step 5: To a reaction mixture of tert-butyl (5aS,6S,9R)-3-chloro-2-(8-ethyl-3-(methoxymethoxy)naphthalen-1-yl)-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (10 mg, 0.013 mmol), acetonitrile (2.0 mL), and 4M hydrochloric acid/dioxane solution (0.5 mL, 2.000 mmol) reacted at 0°C for 1 hour. The reaction mixture was concentrated, and the crude product was purified by preparative HPLC (formic acid method 1) to obtain 4-((5aS,6S,9R)-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino [5,6,7-de]quinazolin-2-yl)-5-ethylnaphthalen-2-ol (Z381, 5.0mg, yield: 61.1%, formate), yellow solid. ES-API:[M+l]⁺=630.3. ¹H NMR (400 MHz, CD₃OD)δ 8.51 (s, 2H), 7.59 (d, J = 8.1 Hz, 1H), 7.37 -7.27 (m, 1H), 7.24 -7.07 (m, 2H), 6.97 (d, J = 2.7 Hz, 1H), 6.79 (dd, J = 12.2, 2.6 Hz, 1H), 5.44 (d, J = 52.4 Hz, 1H), 5.09 (dd, J = 29.8, 11.5 Hz, 3H), 4.66 -4.44 (m, 1H), 4.24 -4.08 (m, 1H), 3.92 -3.57 (m, 5H), 3.29 -3.22 (m, 2H), 2.63 -2.31 (m, 5H), 2.27 -2.13 (m, 2H), 1.99 (dd, J = 30.6, 18.8 Hz, 5H), 0.91 (dt, J = 14.5, 7.4 Hz, 3H).

### Example 10: Synthesis of 5-ethynyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z131-1)

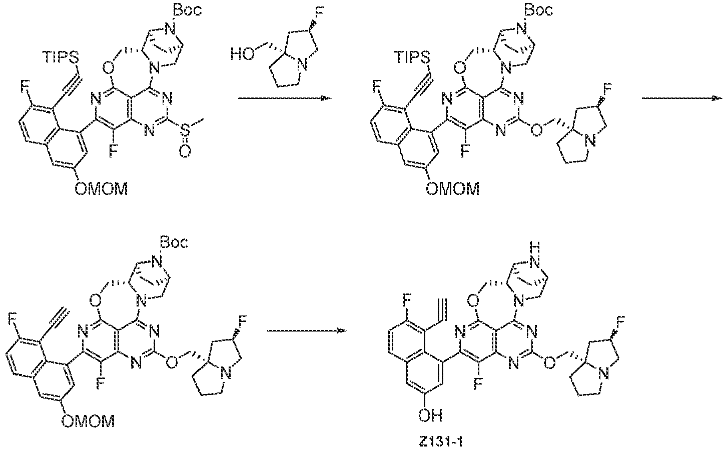

Step 1: At 0°C, sodium hydride (50.0 mg, 1.25 mmol) was added to a tetrahydrofuran (10.0 mL) solution containing ((2R,7aS)-2-fluorotetrahydro-1H-pyrroloquinoline-7a(5H)-yl)methanol (100.0 mg, 0.628 mmol). After the mixture stirred for 10 minutes at this temperature, tert-butyl (5aS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (210.0 mg, 0.252 mmol) in tetrahydrofuran (5 mL) solution was added. The reaction mixture was stirred at 0°C and reacted for 1 hour, quenched with saturated sodium bicarbonate solution, and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170.0 mg, yield: 72.6%), a yellow solid. ES-API: [M+H]⁺=929.3.

Step 2: To a N,N-dimethylformamide (5.0 mL) solution of tert-butyl (SaS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170 mg, 0.183 mmol), cesium fluoride (1000 mg, 6.583 mmol) was added. The reaction mixture was stirred at room temperature and reacted for 0.5 hours, Upon completion of the reaction, the reaction mixture was quenched with water and extracted with ethyl acetate. The organic phase was washed with saturated brine, dried over anhydrous sodium sulfate, filtered, and concentrated to obtain tert-butyl (5aS,6S,9R)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (200 mg, crude product), a yellow oil. ES-API: [M+H]⁺=773.3.

Step 3: A reaction solution of tert-butyl (5aS,6S,9R)-2-(8-ethynyl-7-fluoro-3-(methoxymethoxy)naphthalen-1-yl)-1-fluoro-12-(((2R,7aS)-2-fluorotetrahydro-1H-pyrnolizin-7a(5H)-yl)methoxy)-5a,6,7,8,9,10-hexahydro-5H-4-oxa-3,10a, 11,13,14-pentaaza-6,9-methanonaphtho[1,8-ab]heptalene-14-carboxylate (200 mg, crude product), acetonitrile (10.0 mL), and 4M hydrochloric acid/dioxane solution (2.0 mL, 8.000 mmol) reacted at 0°C for 1 hour. The mixture was concentrated, and the crude product was purified by preparative HPLC (ammonium bicarbonate method 1) to obtain 5-ethynyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z131-1, 8.95mg, total yield for 2 steps: 7.8%), yellow solid. ES-API:[M+1]⁺=629.3.

### Example 11: Synthesis of 5-ethyl-4-((5aS,6S,9R)-12-((1-methyl-1-azaspiro[4.4]nonan-6-yl)oxy)-5a,6,7,8,9,10-hexahydro-5H-4-oxa-3,10a,11,13,14-pentaaza[6,9]methanonaphtho[1,8-ab]heptalen-2-yl)naphthalen-2-ol (Z260-1)

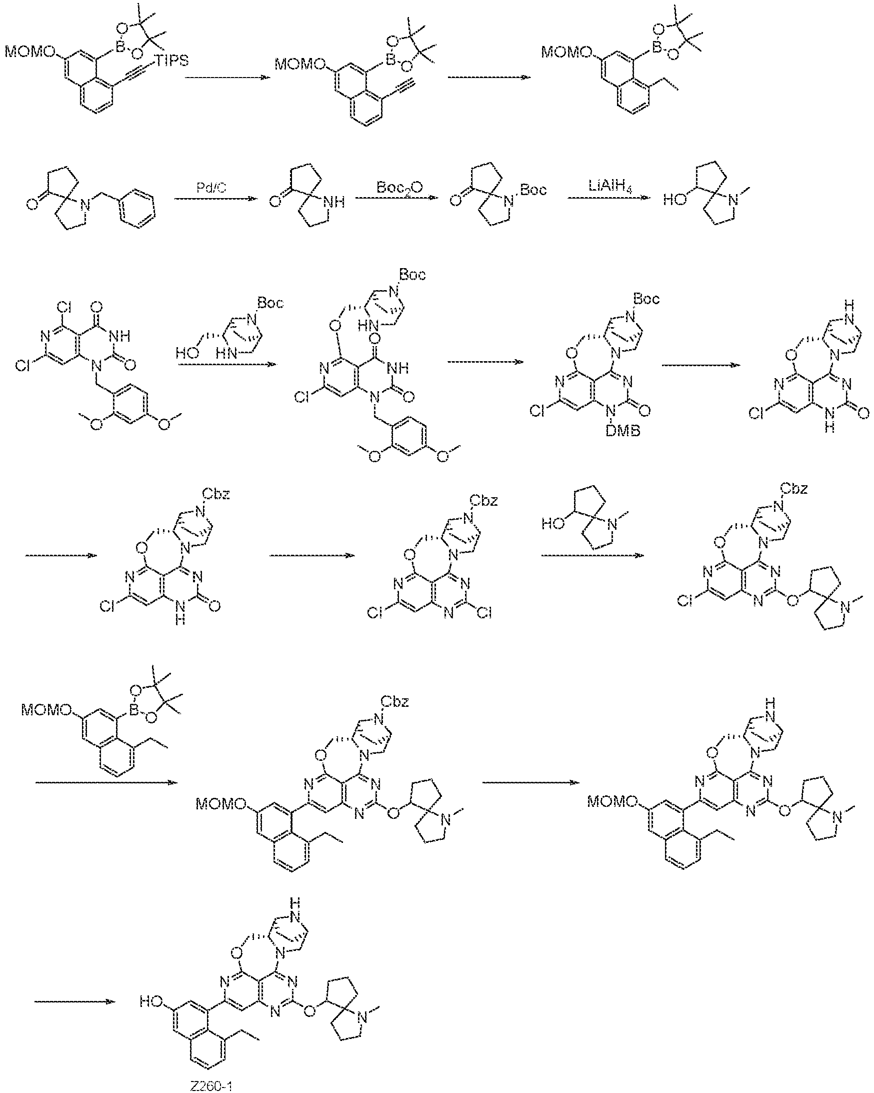

Step 1: To a solution of triisopropyl((6-(methoxymethoxy)-8-(4,4,5,5-tetramethyl-1,3,2-dioxaborolane-2-yl)naphthalen-1-yl)ethynyl)silane (500 mg, 1.012 mmol) in N,N-dimethylformamide (5.0 mL), cesium fluoride (3.0 g, 20.24 mmol) was added and reacted at room temperature for 2 hours. Upon completion of the reaction, the reaction mixture was extracted with ethyl acetate (100 mLX2) and the ethyl acetate phases were combined and washed with saturated brine (100 mLX3). The ethyl acetate phase was dried over anhydrous sodium sulfate, filtered, and evaporated to obtain 2-(8-ethynylnaphthalen-3-(methoxymethoxy)-1-yl)-4,4,5,5 tetramethyl-1,3,2-dioxaborolane (558 mg, crude product). ES-API:[M+H]⁺=339.3.

Step 2: To a methanol (10.0 mL) solution of 2-(8-ethynylnaphthalen-3-(methoxymethoxy)-1-yl)-4,4,5,5-tetramethyl-1,3,2-dioxaborolane (558 mg, crude product), 10% (mass fraction) palladium on carbon was added, and the mixture was purged four times with hydrogen and reacted under a hydrogen atmosphere at room temperature for 2 hours. Upon completion of the reaction, the mixture was filtered, and the solvent was evaporated under reduced pressure to obtain 2-(8-ethylnaphthalen-3-(methoxymethoxy)-1-yl)-4,4,5,5-tetramethyl-1,3,2-dioxaborolane (284 mg, total yield for 2 steps: 82%). ES-API:[M+1]⁺=343.3.

Step 3: 1-Benzyl-1-azaspiro[4.4]nonan-6-one (1.5 g, 6.5 mmol) was dissolved in methanol (10 mL), and palladium on carbon (150 mg) was added. The mixture was purged with hydrogen three times and reacted at room temperature for 4 hours, filtered, washed with methanol, and concentrated to obtain 1-azaspiro[4.4]nonan-6-one (800 mg, yield: 88.5%). ES-API:[M+H]⁺=140.1.

Step 4: 1-Azaspiro[4.4]nonan-6-one (800 mg, 5.7 mmol) was dissolved in tetrahydrofuran (10 mL). At room temperature, triethylamine (0.86 mg, 8.5 mmol) and di-tert-butyl dicarbonate (1.8 g, 8.5 mmol) were added, and the mixture was stirred at room temperature for 8 hours. Upon completion of the reaction (monitored by LCMS), water (10 mL) was added, and the mixture was extracted with ethyl acetate, dried over anhydrous sodium sulfate, and concentrated. The crude product was purified by silica gel column chromatography (ethyl acetate/petroleum ether=0~80%) to obtain tert-butyl 6-oxo-1-azaspiro[4.4]nonane-1-carboxylate (900 mg, yield: 66%). ES-API:[M+H]⁺=240.1.

Step 5: Tert-butyl 6-oxo-1-azaspiro[4.4]nonane-1-carboxylate (900 mg, 3.7 mmol) was dissolved in dry tetrahydrofuran (10 mL), and lithium aluminum hydride (11 mL, 11 mmol, 1M in tetrahydrofuran) was added under an ice water bath. The mixture was stirred at 60°C for 2 hours. Water (10 mL) was added, and the mixture was extracted with ethyl acetate, dried over anhydrous sodium sulfate, and concentrated to obtain 1-methyl-1-azaspiro[4.4]nonan-6-ol (450 mg), which was then used directly in the next step. ES-API:[M+Na]⁺=156.1.

Step 6: At 0°C, sodium hydride (80 mg, 2.0 mmol) was added to a tetrahydrofuran (10 mL) solution of tert-butyl (1S,2S,5R)-2-(hydroxymethyl)-3,8-diazabicyclo[3.2.1]octane-8-carboxylate (242 mg, 1.0 mmol). After the mixture reacting under nitrogen protection for 10-20 minutes, 5,7-dichloro-1-(2,4-dimethoxybenzyl)pyrido[4,3-d]pyrimidme-2,4(1H,3H)-dione (381 mg, 1.0 mmol) was added in batches and the mixture reacted for another 20-30 minutes at 0-5°C. Upon completion of the reaction (monitored by LCMS), the reaction mixture was poured into about 300 mL of ice water, and the pH was adjusted to 7-8 with 6M hydrochloric acid under ice water bath condition. The mixture was then extracted with ethyl acetate (100 mLX2), and the organic phase was washed with saturated brine (100 mLX1). The organic phase was dried over anhydrous sodium sulfate, filtered, and evaporated to obtain the target compound tert-butyl (1S,2S,5R)-2-(((7-chloro-1-(2,4-dimethoxybenzyl)-2,4-dioxo-1,2,3,4-tetrahydropyrido[4,3-d]pyrimidin-5-yl)oxy)methyl)-3,8-diazabicyclo[3.2.1]octane-8-carboxylate (560 mg, yield: 87%). ES-API: [M+H]⁺=588.2.

Step 7: At room temperature, dry N,N-dimethylformamide (15 mL) and tert-butyl (1S,2S,5R)-2-(((7-chloro-1-(2,4-dimethoxybenzyl)-2,4-dioxo-1,2,3,4-tetrahydropyrido[4,3-d]pyrimidin-5-yl)oxy)methyl)-3,8-diazabicyclo[3.2.1]octane-8-carboxylate (510 mg, 0.87 mmol) were added to a 250mL single-neck flask. 1,8-diazabicyclo[5.4.0]undec-7-ene (661 mg, 4.35 mmol) was added dropwise After the mixture reacting with Katt's reagent (1.9 g, 4.35 mmol) for 5 minutes at room temperature. The mixture reacted for 1-2 hours at room temperature. Upon completion of the reaction, the solution was slowly added dropwise to 100 mL of ice water, causing a large amount of solid to precipitate. The solid was filtered, and the filter cake was evaporated under reduced pressure and purified by column chromatography (petroleum ether/ethyl acetate=1/1) to obtain tert-butyl (5aS,6S,9R)-2-chloro-13-(3,4-dimethylbenzyl)-12-oxo-5a,6,7,8,9,10,12,13-octahydro-5H-4-oxa-3,10a,11,13,14-pentaaza-6,9-methanonaphtho[1,8-ab]heptalene-14-carboxylate (300 mg, yield: 61%). ES-API: [M+H]⁺=568.2.

Step 8: To trifluoroacetic acid (5 mL), tert-butyl (5aS,6S,9R)-2-chloro-13-(3,4-dimethylbenzyl)-12-oxo-5a,6,7,8,9,10,12,13-octahydro-5H-4-oxa-3,10a,11,13,14-pentaaza-6,9-methanonaphtho[1,8-ab]heptalene-14-carboxylate (300 mg, 0.53 mmol) was added and stirred at 55°C for 1 hour. Upon completion of the reaction, the solvent was evaporated under reduced pressure to obtain the compound (5aS,6S,9R)-2-chloro-5a,6,7,8,9,10-hexahydro-5H-4-oxy-3,10a, 11,13,14-pentaza-6,9-methanonaphtho[1.8-ab]heptene-12(13H)-one (500 mg, crude product). ES-API: [M+H]⁺=320.1.

Step 9: To a mixed solution of (5aS,6S,9R)-2-chloro-5a,6,7,8,9,10-hexahydro-5H-4-oxy-3,10a,11,13,14-pentaza-6,9-methanonaphtho[1,8-ab]heptene-12(13H)-one (500 mg, crude product) in tetrahydrofuran (5.0 mL) and water (5.0 mL), sodium carbonate (168 mg, 1.59 mmol) was added, and the system was cooled with an ice water bath, then, benzoyloxysuccinimide (171 mg, 0.68 mmol) was added. The reaction mixture was stirred at room temperature and reacted for 3 hours. Upon completion of the reaction, the reaction mixture was added to dichloromethane (80 mL) and washed with saturated bicarbonate solution (100 mL) and saturated brine (80 mL), dried over anhydrous sodium sulfate, concentrated, and the crude product was purified by silica gel column chromatography (ethyl acetate/petroleum ether=0-50%) to obtain the target product benzyl (5aS,6S,9R)-2-chloro-12-oxo-5a,6,7,8,9,10,12,13-octahydro-5H-4-oxa-3,10a,11,13,14-pentaaza-6,9-methanonaphtho[1,8-ab]heptalene-14-carboxylate (396 mg, yield: 93%). ES-API: [M+H]⁺=454.1.

Step 10: At 0°C, to a toluene (5.0 mL) solution of benzyl (5aS,6S,9R)-2-chloro-12-oxo-5a,6,7,8,9,10,12,13-octahydro-5H-4-oxa-3,10a,11,13,14-pentaaza-6,9-methanonaphtho[1,8-ab]heptalene-14-carboxylate (390 mg, 0.8 mmol), phosphorus oxychloride (365 mg, 2.4 mmol) and N,N-diisopropylethylamine (310 mg, 2.4 mmol) were added successively and reacted at 105°C for 12 hours. Upon completion of the reaction, the reaction mixture was cooled to room temperature, and the solvent was evaporated under reduced pressure. The crude product was purified by preparative HPLC (ammonium bicarbonate method 1) to obtain benzyl (5aS,6S,9R)-2,12-dichloro-5a,6,7,8,9,10-hexahydro-5H-4-oxa-3,10a,11,13,14-pentaaza-6,9-methanonaphtho[1,8-ab]heptalene-14-carboxylate (70 mg, yield: 17%). ES-API: [M+H]⁺=472.1.

Step 11: To a dimethyl sulfoxide (3.0 mL) solution of benzyl (SaS,6S,9R)-2,12-dichloro-5a,6,7,8,9,10-hexahydro-5H-4-oxa-3,10a,11,13,14-pentaaza-6,9-methanonaphtho[1,8-ab]heptalene-14-carboxylate (50 mg, 0.1 mmol), potassium fluoride (29 mg, 0.5 mmol), N,N-diisopropylethylenediamine (39 mg, 0.3 mmol), 1-methyl-1-azaspiro[4.4]nonan-6-ol (31 mg, 0.2 mmol), and 4A molecular sieves were added. The mixture reacted under nitrogen protection at 110°C for 4 hours. After cooled to room temperature, the mixture was extracted with ethyl acetate (50mLX2), and the ethyl acetate phase was washed with saturated saline (50mLX4). The ethyl acetate phase was dried over anhydrous sodium sulfate, filtered, and evaporated. The crude product was purified by silica gel column chromatography (petroleum ether/ethyl acetate=2:1) to obtain benzyl (5aS,6S,9R)-2-chloro-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptalene-14-carboxylate (28 mg, yield: 44%). ES-API: [M+H]⁺=591.2.

Step 12: Benzyl (5aS,6S,9R)-2-chloro-12-((1-methyl-1-azaspiro[4.4]nonan-6-yl)oxy)-5a,6,7,8,9,10-hexahydro-5H-4-oxa-3,10a,1113,14-pentaaza-6,9-methanonaphtho[1,8-ab]heptalene-14-carboxylate (28 mg, 0.047 mmol) was dissolved in tetrahydrofuran/water (2 mL/0.5 mL). 2-(8-ethyl-3-(methoxymethoxy)naphth-1-yl)-4,4,5,5-tetramethyl-1,3,2-dioxaborolane (24 mg, 0.07 mmol), potassium phosphate (30 mg, 0.14 mmol), and methanesulfonic acid[tert-butyl di(1-adamantyl)phosphine](2-amino-1,1'-biphenyl)palladium(II) (10 mg, 0.014mmol) were added to the solution. After purged four times with nitrogen, the reaction was proceeded at 60°C for 1 hour. The reaction mixture was cooled to room temperature and extracted with ethyl acetate (80mL) and water (60mL). The organic phase was dried over anhydrous sodium sulfate, filtered, and the filtrate was evaporated under reduced pressure. The crude product was purified by silica gel column chromatography (ethyl acetate/petroleum ether: 0-70%) to obtain benzyl (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptalene-14-carboxylate (26 mg, yield: 73%). ES-API: [M+H]⁺=771.4.

Step 13: To a methanol (10.0 mL) solution of benzyl (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptalene-14-carboxylate (26 mg, 0.034 mmol), 10% (mass fraction) palladium on carbon (50 mg) was added, and the mixture was purged four times with hydrogen and reacted under a hydrogen atmosphere at room temperature for 2 hours. The reaction mixture was filtered to obtain the crude target compound (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptalene (17 mg, yield: 80%). ES-API: [M+H]⁺=637.3.

Step 14: (SaS,6S,9R)-2-(8-ethyl-3-(methoxymethoxy)naphthalen-1-yl)-12-((1-methyl-1-azaspiro[4.4]nonan-6-yl)oxy)-Sa,6,7,8,9,10-hexahydro-SH-4-oxa-3,10a,11,13,14-pentaaza-6,9-methanonaphtho[1,8-ab]heptalene (17 mg, 0.027 mmol) was dissolved in acetonitrile (6.0 mL). Under ice water bath condition, 4M hydrochloric acid/dioxane solution (1.0 mL, 4.0 mmol) was added. The mixture reacted for 0.5 hours in the ice water bath, Upon completion of the reaction, the reaction mixture was concentrated to dryness and then diluted with dichloromethane (20 mL). Triethylamine (3.0mL) was added under ice water bath condition. After stirred for 10 minutes, the mixture was extracted with dichloromethane (100 mLX1) and water (50 mLX1). The dichloromethane phase was dried over anhydrous sodium sulfate, filtered, and the filtrate was evaporated under reduced pressure. The crude product was purified by preparative HPLC (formic acid method 1) to obtain 5-ethyl-4-((5aS,6S,9R)-12-((1-methyl-1-azaspiro[4.4]nonan-6-yl)oxy)-5a,6,7,8,9,10-hexahydro-5H-4-oxa-3,10a,11,13,14-pentaaza[6,9]methanonaphtho[1,8-ab]heptalen-2-yl)naphthalen-2-ol (Z260-1, 1.6 mg, yield: 10%, formate). ES-API: [M+H]⁺=593.3.

### Example 12: Synthesis of (5aS,6S,9R)-1-fluoro-12-(((2R,7aS)-2-fluorotetrahydro-1H-pyrrolizin-7a(5H)-yl)methoxy)-2-(5-methyl-lH-indazol-4-yl)-5a,6,7,8,9,10-hexahydro-5H-4-oxa-3,10a,11,13,14-pentaaza-6,9-methanonaphtho[1,8-ablheptalene (Z382)

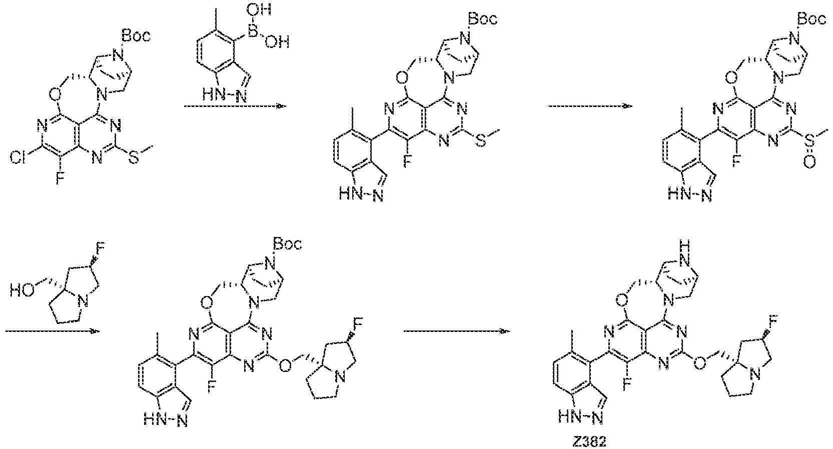

Step 1: To a solution of tert-butyl (1S,2S,5R)-2-[[(7-chloro-8-fluoro-(2-methylthio)-4-oxa-3,4-dihydropyridino[4,3-d]pyrimidin-5-yl)oxy]methyl]-3,8-diazabicyclo[3.2.1]octane-8-carboxylate (200mg, 0.427mmol) and (5-methyl-1H-indol-4-yl)boronic acid (230.0mg, 1.307mmol) in tetrahydrofuran (10.0mL) and water (2.0mL), cesium carbonate (560mg, 1.719mmol) and tris(tribenzylphosphine)palladium (100ing, 0.87mmol) were added. The mixture was bubbled with nitrogen, heated to 115°C and reacted for 6 hours. Upon completion of the reaction, the mixture was extracted with ethyl acetate (30mLX2) and saturated saline (30mL). The ethyl acetate phases were combined, dried over anhydrous sodium sulfate, filtered, and the filtrate was concentrated under reduced pressure. The crude product was purified by silica gel column chromatography (methanol/dichloromethane = 0-4%) to obtain tert-butyl (5aS,6S,9R)-1-fluoro-2-(5-methyl-1H-indazol-4-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (400mg, crude), as a yellow solid. ES-API: [M+H]⁺=564.4.

Step 2: At 0°C, to a dichloromethane (15mL) solution of tert-butyl (SaS,6S,9R)-1-fluoro-2-(5-methyl-1H-indazol-4-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (180mg, 0.319mmol), m-chloroperbenzoic acid (85mg, 0.493mmol) was added. The mixture was stirred at room temperature for 1 hour. The reaction mixture was quenched with saturated sodium bisulfite and the mixture was extracted with dichloromethane (100mLX2). The organic phase was dried over anhydrous sodium sulfate and concentrated. The crude product obtained was purified by silica gel column chromatography (methanol/dichloromethane = 0-10%) to obtain tert-butyl (5aS,6S,9R)-1-fluoro-2-(5-methyl-1H-indazol-4-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (200mg, crude), as a white solid. ES-API:[M+H]⁺=580.3.

Step 3: At 0°C, sodium hydride (55.0mg, 1.382mmol) was added to a tetrahydrofuran (10.0mL) solution containing ((2R,7aS)-2-fluoro-tetrahydro-1H-pyrrolo[3,4-b]pyridin-7a(5H)-yl)methanol (239.16 mg, 1.502 mmol), and the mixture was stirred for 10 minutes at this temperature. Subsequently, a tetrahydrofuran (5 mL) solution of tert-butyl (5aS,6S,9R)-1-fluoro-2-(5-methyl-1H-indazol-4-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (200mg, crude) was added. The reaction mixture was stirred at 0°C and reacted for 1 hour, quenched with saturated sodium bicarbonate solution and extracted with ethyl acetate. The organic phase was washed with saturated saline, dried over anhydrous sodium sulfate, filtered, and concentrated. The crude product was purified by silica gel column chromatography (methanol/dichloromethane = 0-5%) to obtain tert-butyl (SaS,6S,9R)-1-fluoro-12-(((2P,7aS)-2-fluorotetrahydro-1H-pyrrolizin-7a(5H)-yl)methoxy)-2-(5-methyl-1H-indazol-4-yl)-5a,6,7,8,9,10-hexahydro-5H-4-oxa-3,10a,11,13,14-pentaaza-6,9-methanonaphtho[1,8-ab]heptalene-14-carboxylate (220mg, total yield over 3 steps: 94.5%), a yellow solid. ES-API: [M+H]⁺=675.3.

Step 4: At 0°C, to an acetonitrile (5.0mL) solution of tert-butyl (SaS,6S,9R)-1-fluoro-12-(((2R,7aS)-2-fluorotetrahydro-1H-pyrrolizin-7a(5H)-yl)methoxy)-2-(5-methyl-1H-indazol-4-yl)-5a,6,7,8,9,10-hexahydro-5H-4-oxa-3,10a,11,13,14-pentaaza-6,9-methanonaphtho[1,8-ab]heptalene-14-carboxylate (200mg, 0.296mmol), a 4M hydrochloric acid/dioxane solution (2.0mL, 8.000mmol) was added. The reaction mixture was stirred at 0°C and reacted for 1 hour, concentrated, and the crude product was purified by preparative HPLC (ammonium bicarbonate method 1) to obtain (5aS,6S,9R)-1-fluoro-12-(((2R,7aS)-2-fluorotetrahydro-1H-pyrrolizin-7a(5H)-yl)methoxy)-2-(5-methyl-1H-indazol-4-yl)-5a,6,7,8,9,10-hexahydro-5H-4-oxa-3,10a,11,13,14-pentaaza-6,9-methanonaphtho[1,8-ab]heptalene (Z382, 30mg, yield: 17.6%), yellow solid. ES-API:[M+l]⁺=575.3. ¹HNMR (500 MHz, CD₃OD)δ 8.39 (s, 2H), 7.74 (d, *J* = 27.1 Hz, 1H), 7.59 (d, *J* = 8.6 Hz, 1H), 7.40 (dd, *J* = 8.6, 3.2 Hz, 1H), 5.52 (d, *J* = 52.0 Hz, 1H), 5.20 (t, *J* = 12.5 Hz, 1H), 4.82 -4.48 (m, 4H), 4.33 (d, *J* = 5.9 Hz, 1H), 4.20 -3.67 (m, 4H), 3.37 (dd, *J* = 23.7, 13.9 Hz, 2H), 2.73 -1.87 (m, 12H).

### Example 13: Synthesis of 5-ethynyl-6-fluoro-4-((5aS,6S,9R)-1-fluoro-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z383)

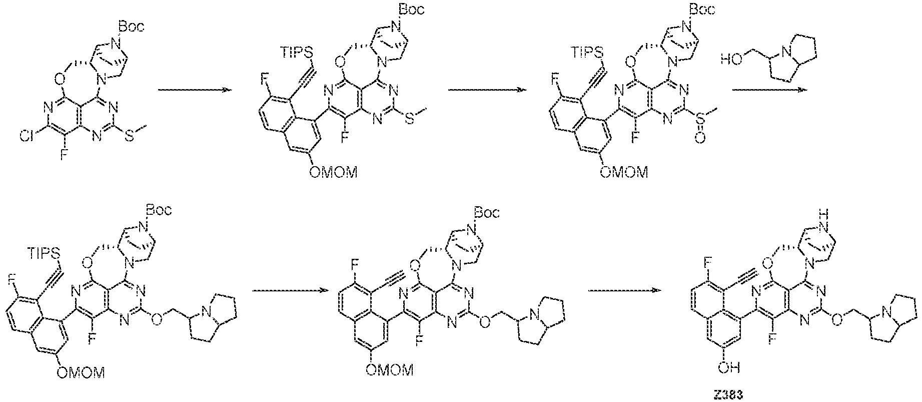

Step 1: To a mixture of tert-butyl (1S,2S,5R)-2-(((7-chloro-8-fluoro-(2-methylthio)-4-oxa-3,4-dihydropyrimido[4,3-d]pyrimidin-5-yl)oxy)methyl)-3,8-diazabicyclo[3.2.1]octane-8-carboxylate (100 mg, 0.214 mmol) and ((2-fluoro-6-(methoxymethoxy)-8-(4,4,5,5 tetramethyl-1,3,2-dioxaborolane-2-yl)naphth-1-yl)ethynyl)triisopropylsilane (200.0 mg, 0.390 mmol) in tetrahydrofuran (6 mL) and water (1.5 mL), potassium phosphate (200 mg, 0.943 mmol) and palladium(II) bis[tert-butyl(1-adamantyl)phosphine] (2-amino-1,1'-biphenyl) (50 mg, 0.069 mmol) were added. The mixture was purged with nitrogen and microwave reacted at 80°C for 40 minutes. Upon completion of the reaction, the reaction mixture was extracted with ethyl acetate (30 mLX2) and saturated brine (30 mL). The ethyl acetate phases were combined, dried over anhydrous sodium sulfate, filtered, and the filtrate was concentrated under reduced pressure. The crude product was purified by silica gel column chromatography (methanol/dichloromethane = 0-4%) to obtain tert-butyl (5aS,6S,9R)-1-fluoro-2-(7-fluoro-3-(methoxymethoxy)-8-((triisopropylsilyl)ethynyl)naphthalen-1-yl)-12-(methylthio)-Sa,6,7,8,9,10-hexahydro-SH-4-oxa.-3,10a,11,13,14-pentaaza-6,9-methanonaphtho[1,8-ab]heptalene-14-carboxylate (210 mg, crude product), a yellow solid. ES-API: [M+H]⁺=818.3.

Step 2: At 0°C, to a dichloromethane solution (15 mL) of tert-butyl (5aS,6S,9R)-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (210 mg, crude product), m-chloroperoxybenzoic acid (55 mg, 0.320 mmol) was added. The mixture was stirred at room temperature for 1 hour. The reaction mixture was quenched with saturated sodium bisulfite and extracted with dichloromethane (100 mLX2). The organic phase was dried over anhydrous sodium sulfate and concentrated, and the crude product was purified by silica gel column chromatography (methanol/dichloromethane = 0-10%) to obtain tert-butyl (SaS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (210 mg, crude product), a white solid. ES-API: [M+H]⁺=834.4.

Step 3: At 0°C, sodium hydride (54.0 mg, 1.34 mmol) was added to a tetrahydrofuran (10.0 mL) solution containing (hexahydro-1H-pyrrolo[3,4-b]pyiazin-3-yl)methanol (95.0 mg, 0.673 mmol). After the mixture stirred at this temperature for 10 minutes, a tetrahydrofuran solution (5 mL) of tert-butyl (SaS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (270.0 mg, 0.324 mmol) was added. The reaction mixture was stirred and reacted for 1 hour at 0°C. The reaction mixture was quenched with saturated sodium bicarbonate solution and the mixture was extracted with ethyl acetate. The organic phase was washed with saturated brine, dried over anhydrous sodium sulfate, filtered, and concentrated. The crude product was purified by silica gel column chromatography (methanol/dichloromethane = 0-5%) to obtain tert-butyl (SaS,6S,9R)-1-fluoro-2-(7-fluoro-3-(methoxymethoxy)-8-((triisopropylsilyl)ethynyl)na.phthalen-1-yl)-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]heptalene-14-carboxylate (300 mg, crude product), a yellow solid. ES-API: [M+H]⁺=911.5.

Step 4: To a N,N-dimethylformamide (5.0 mL) solution of tert-butyl (SaS,6S,9R)-1-fluoro-2-(7-fhioro-3-(methoxymethoxy)-8-((triisopropylsilyl)ethynyl)naphthalen-1 -yl)- 12-((hexahydro- 1H-pyrrolizin-3-yl)methoxy)-5a,6,7,8,9,10-hexahydro-5H-4-oxa-3,10a, 11,13,14-pentaaza-6,9-methanonaphtho[1,8-ab]heptalene-14-carboxylate (300 mg, crude product), cesium fluoride (1000 mg, 6.583 mmol) was added. The reaction mixture was stirred at room temperature and reacted for 0.5 hours. Upon completion of the reaction, the reaction mixture was quenched with water and the mixture was extracted with ethyl acetate. The organic phase was washed with saturated brine, dried over anhydrous sodium sulfate, filtered, and concentrated to obtain tert-butyl (5aS,6S,9R)-2-(8-ethynyl-7-fluoro-3-(methoxymethoxy)naphthalen-1-yl)-1-fluoro-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, total yield over 3 steps: 60.3%), a yellow oil. ES-API: [M+H]⁺=755.3.

Step 5: A reaction solution of tert-butyl (5aS,6S,9R)-2-(8-ethynyl-7-fluoro-3-(methoxymethoxy)naphthalen-1-yl)-1-fluoro-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, 0.199 mmol), acetonitrile (5.0 mL) and 4M hydrochloric acid/dioxane solution (2.0 mL, 8.000 mmol) reacted at 0°C for 1 hour. The mixture was concentrated, and the crude product was purified by preparative HPLC (formic acid method 1) to obtain 5-ethynyl-6-fluoro-4-((5aS,6S,9R)-1-fluoro-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol (Z383, 49.0mg, yield: 41%, formate), yellow solid. ES-API:[M+1]⁺=611.3. ¹H NMR(500 MHz, CD₃OD) δ 8.43 (s, 2H), 7.88 -7.83 (m, 1H), 7.35 -7.16 (m, 3H), 5.07 (d, *J* = 13.7 Hz, 1H), 4.72 -4.60 (m, 2H), 4.57 -4.46 (m, 1H), 4.37 -4.19 (m, 2H), 4.11 (s, 1H), 3.86 (d, *J* = 21.8 Hz, 2H), 3.66 -3.40 (m, 3H), 2.47 -1.66 (m, 13H).

### Example 14: Synthesis of (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ablheptalene (Z360.1)

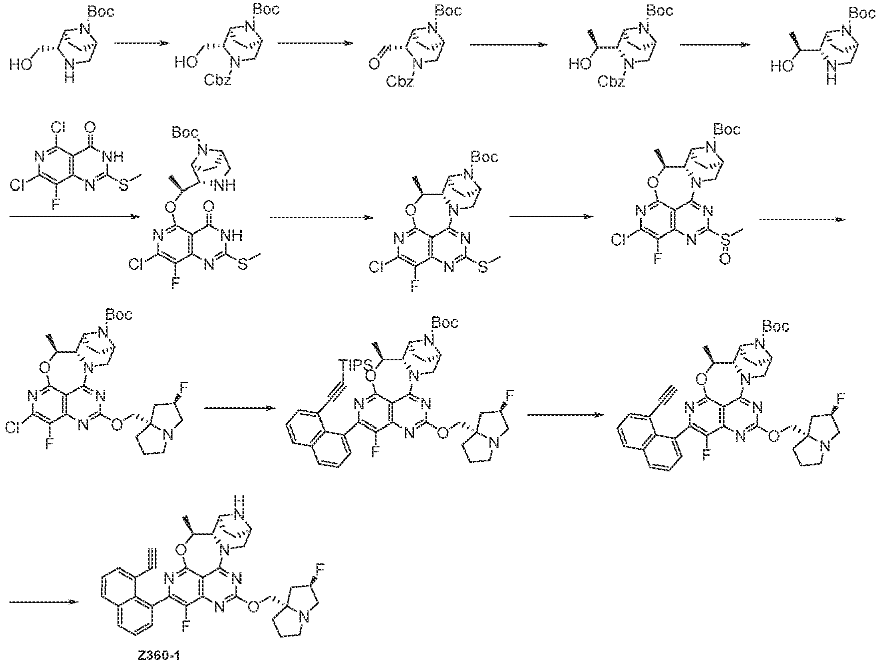

Step 1: Tert-butyl (1S,2S,5R)-2-(hydroxymethyl)-3,8-diazabicyclo[3.2.1]octane-8-carboxylate (3.0 g, 12.40 mmol) was dissolved in a mixed solution of ethyl acetate (30 mL) and water (10 mL). Benzyl chloroformate (2.54 g, 14.88 mmol) and sodium bicarbonate (2.98 g, 32.0 mmol) were added, and the mixture was stirred at room temperature overnight. The mixture was extracted by adding water (50 mL) and ethyl acetate (100 mL × 3), washed with saturated brine (20 mL), dried over anhydrous sodium sulfate, and concentrated. The crude product was purified by silica gel column chromatography (petroleum ether/ethyl acetate = 2:1) to obtain 3-benzyl 8-(tert-butyl)(1S,2S,5R)-2-(hydroxymethyl)-3,8-diazabicyclo[3.2.1]octane-3,8-dicarboxylate (4.35 g, yield: 93.3%).

Step 2: At -78°C and under nitrogen protection, a solution of dimethyl sulfoxide (156 mg, 1.99 mmol) in anhydrous dichloromethane (5.0 mL) was slowly added dropwise to a solution of oxalyl chloride (223 mg, 1.75 mmol) in anhydrous dichloromethane (10 mL). After stirred at this temperature for 15 minutes, a solution of 3-benzyl 8-(tert-butyl)(1S,2S,5R)-2-(hydroxymethyl)-3,8-diazabicyclo[3.2.1]octane-3,8-dicarboxylate (300 mg, 0.80 mmol) in anhydrous dichloromethane (5.0 mL) was slowly added dropwise. The reaction was proceeded for 40 minutes at -78°C. Triethylamine (646 mg, 6.38 mmol) was slowly added. The reaction was proceeded for 1 hour at - 78°C. The reaction mixture was quenched with saturated ammonium chloride (20 mL), extracted with ethyl acetate, dried over anhydrous sodium sulfate, and concentrated to obtain crude 3-benzyl 8-(tert-butyl) (1S,2S,5R)-2-formyl-3,8-diazabicyclo[3.2.1]octane-3,8-dicarboxylate (280 mg, yield: 93.9%).

Step 3: 3-Benzyl 8-(tert-butyl) (1S,2S,5R)-2-formyl-3,8-diazabicyclo[3.2.1]octane-3,8-dicarboxylate (280 mg, 0.75 mmol) was dissolved in dry tetrahydrofuran (5 mL). Under nitrogen protection, the mixture was cooled to -78°C, with 3M methylmagnesium bromide in 2-methyltetrahydrofuran (0.3 mL) added dropwise. The reaction was proceeded for 2 hours at -78°C. The reaction mixture was quenched with saturated ammonium chloride solution (5 mL), extracted with ethyl acetate, dried over anhydrous sodium sulfate, and concentrated. The crude product was purified by thin-layer chromatography (ethyl acetate/petroleum ether = 1:2) to obtain 3-benzyl 8-(tert-butyl)(1S,2S,5R)-2-((S)-1-hydroxyethyl)-3,8-diazabicyclo[3.2.1]octane-3,8-dicarboxylate (180 mg, yield: 61.6%).

Step 4: 3-Benzyl 8-(tert-butyl)(1S,2S,5R)-2-((S)-1-hydroxyethyl)-3,8-diazabicyclo[3.2.1]octane-3,8-dicarboxylate (180 mg, 0.46 mmol) was dissolved in methanol (4 mL) and water (0.5 mL), with ammonium formate (290 mg, 4.6 mmol) and 10% palladium on carbon (20 mg) added. The mixture reacted in a sealed tube at 60°C for 2 hours. The reaction mixture was filtered, concentrated, and purified by preparative thin-layer chromatography (dichloromethane/methanol = 10:1) to obtain tert-butyl (1S,2S,5R)-2-((S)-1-hydroxyethyl)-3,8-diazabicyclo[3.2.1]octane-8-carboxylate (100 mg, yield: 85.5%).

Step 5: tert-butyl (1S,2S,5R)-2-((S)-1-hydroxyethyl)-3,8-diazabicyclo[3.2.1]octane-8-carboxylate (100 mg, 0.39 mmol) was added to a suspension of sodium hydride (60% dispersed in oil) (41 mg, 0.58 mmol) in tetrahydrofuran (10 mL) and stirred at this temperature for 10 minutes. 5,7-Dichloro-8-fluoro-2-(methylthio)pyrimido[4,3-d]pyrimidin-4(3H)-one (112 mg, 0.40 mmol) was added, and the mixture was heated to 60°C and stirred for 1 hour. Upon completion of the reaction, the mixture was quenched with water and extracted with dichloromethane/methanol (10:1, 25 mL × 3). The organic phase was dried over anhydrous sodium sulfate, filtered, and concentrated to obtain tert-butyl (1S,2S,5R)-2-((S)-1-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (160 mg, yield: 82.0%). ES-API:[M+H]⁺=500.1.

Step 6: To a dichloromethane (10 mL) solution of tert-butyl (1S,2S,5R)-2-((S)-1-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (160 mg, 0.32 mmol) and N,N-diisopropylethylenediamine (82 mg, 0.64 mmol), 1-propylphosphonic anhydride (50% solution in ethyl acetate) (265 mg, 0.42 mmol) was added, and the mixture was stirred at room temperature for 3 hours. The reaction mixture was quenched with water and extracted with dichloromethane. The organic phase was dried over anhydrous sodium sulfate, filtered, and concentrated. The obtained solid was suspended in acetonitrile and stirred at room temperature for 10 minutes. The mixture was filtered, and the solid was collected to obtain tert-butyl (5S,SaS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, yield: 65.3%). ES-API:[M+H]⁺=482.1.

Step 7: At 0°C, to a solution of tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (100 mg, 0.21 mmol) in dichloromethane (15 mL), m-chloroperbenzoic acid (40 mg, 0.23 mmol) was added, and the mixture was stirred at room temperature for 1 hour. The reaction mixture was diluted with dichloromethane (30 mL) and washed with 10% sodium hypochlorite solution (5.0 mL × 2). The organic phase was dried over anhydrous sodium sulfate and concentrated to obtain crude tert-butyl (5S,5aS,6S,9R)-2-chloro-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, yield: 95.2%). ES-API:[M+H]⁺=498.1.

Step 8: At 0°C, to a solution of sodium hydride (60% dispersed in oil) (15 mg, 0.36 mmol) in tetrahydrofuran, ((2R,7aS)-2-fluorotetrahydro-1H-pyrrolo[3,4-b]pyrazin-7a(5H)-yl)methanol (40 mg, 0.28 mmol) was added. The reaction mixture was stirred at room temperature and reacted for 10 minutes. With the mixture cooled to 0°C, a solution of tert-butyl (5S,5aS,6S,9R)-2-chloro-1-fluoro-5-methyl-12-(mefhylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, 0.20 mmol) in tetrahydrofuran (5 mL) was added. The reaction mixture was stirred at 0°C and reacted for 1 hour, quenched with saturated sodium bicarbonate solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, yield: 42.4%). ES-API: [M+H]⁺=593.2.

Step 9: To a solution of tert-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50.0 mg, 0.08 mmol) and triisopropyl((8-(4,4,5,5-tetramethyl-1,3,2-dioxaborinane-2-yl)naphth-1-yl)ethynyl)silane (55 mg, 0.13 mmol) in a mixture of tetrahydrofuran (6 mL) and water (1.0 mL), potassium phosphate (34 mg, 0.16 mmol), and bis(butylcyclopentadienyl)diphosphine palladium(II) methanesulfonate [Pd(2-amino-1,1'-biphenyl-2-yl)] (15 mg) were added. The mixture was bubbled with nitrogen and reacted at 60°C for 40 minutes. Upon completion of the reaction, the mixture was extracted with ethyl acetate (30 mL × 2) and saturated brine (10 mL). The ethyl acetate phases were combined, dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-1-fluoro-12-(((2R,7aS)-2-fluorotetrahydro-1H-pyrrolizin-7a(5H)-yl)methoxy)-5methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (45 mg, yield: 65.2%). ES-API: [M+H]⁺=865.1.

Step 10: To a solution of tert-butyl (5S,5aS,6S,9R)-1-fluoro-12-(((2R,7aS)-2-fluorotetrahydro-1H-pyrrolizin-7a(5H)-yl)methoxy)-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (45 mg, 0.052 mmol) in N,N-dimethylformamide (3.0 mL), cesium fluoride (40 mg, 0.26 mmol) was added. The reaction mixture was stirred at room temperature and reacted for 0.5 hours. Upon completion of the reaction, the mixture was purified by preparative HPLC (trifluoroacetic acid method) to obtain tert-butyl (5S,5aS,6S,9R)-2-(8-ethynylnaphthalen-1-yl)-1-fluoro-12-(((2R,7aS)-2-fluorotetrahydro-1H-pynolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (32 mg, yield: 86.5%). ES-API: [M+H]⁺=709.3.

Step 11: A reaction solution of tert-butyl (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (32 mg, 0.045 mmol), acetonitrile (2.0 mL) and 4M hydrochloric acid/dioxane solution (0.500 mL, 2.000 mmol) reacted at 0°C for 1 hour. The reaction solution was concentrated and the crude product was purified by preparative HPLC (formic acid method 1) to obtain (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z360-1, 12mg, yield: 44.4%, formate), yellow solid. ES-API: [M+1]⁺=609.3.

The 3-benzyl 8-(tert-butyl)(1S,2S,5R)-2-((S)-1-hydroxyethyl)-3,8-diazabicyclo[3.2.1]octane-3,8-dicarboxylate obtained in step 3 was converted into compound B for the determination of absolute stereochemistry.

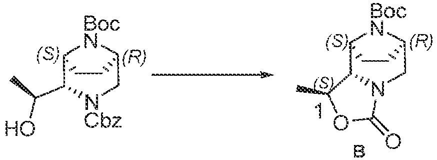

Under nitrogen protection, at 0°C, a 1M solution of potassium tert-butoxide in tetrahydrofuran (0.62 mL, 0.62 mmol) was slowly added to a solution of 3-benzyl 8-(tert-butyl)(1S,2S,5R)-2-((S)-1-hydroxyethyl)-3,8-diazabicyclo[3.2.1]octane-3,8-dicarboxylate (120 mg, 0.31 mmol) in tetrahydrofuran (26.0 mL). After the mixture reacted for 1 hour at 0°C, a saturated aqueous solution of ammonium chloride was slowly added. The mixture was extracted with ethyl acetate three times. The organic phases were combined, washed with saturated brine, dried over anhydrous sodium sulfate, filtered, and the filtrate was evaporated under reduced pressure. The crude product was purified by slurring in petroleum ether to obtain tert-butyl (1S,6S,9R,9aS)-1-methyl-3-oxohexahydro-1H,3H-6,9-epiminooxazolo[3,4-a]azepine-10-carboxylate.

(compound B, 95 mg). ES-API: [M+H]⁺= 283.2. ¹H NMR (500 MHz, CDCl₃) δ 4.44 -3.98 (m, 3H), 3.67 -3.45 (m, 2H), 3.32 -3.06 (m, 1H), 2.11 -1.96 (m, 1H), 1.87 (tdd, *J* = 22.1, 13.1, 4.8 Hz, 2H), 1.77 -1.68 (m, 1H), 1.48 (s, 9H), 1.45 (d, *J* = 6.3 Hz, 3H).¹³C NMR (126 MHz, CDCl₃) δ 157.7, 153.0, 80.6, 72.9, 63.3, 55.0, 53.5, 47.6, 28.4, 27.2, 22.4, 20.7.

### Single Crystal Cultivation

10 milligrams of the crude compound B obtained from the above step were weighed into a sample tube, dissolved in a small amount of ethyl acetate, and placed in a sealed sample bottle. At room temperature, the petroleum ether in the sample bottle slowly diffused into the compound's ethyl acetate solution, thereby obtaining the required single crystals. X-ray single crystal diffraction testing was carried out using a Bruker D8 Venture instrument. The results are shown in Table 3 and Figure 1. The absolute configuration of the substituent at position 1 of compound B was confirmed to be (S).

**Table 3**

| | |
|---|---|
| Empirical formula | C₁₄ H₂₂ N₂ O₄ |
| Formula Weight | 282.33 |
| Temperature | 213.00 K |
| Wavelength | 1.34139 Å |
| Crystal system | Monoclinic |
| Space group | C 1 2 1 |
| Unit cell dimensions | a = 22.9206(4) Å a= 90° |
| | b = 6.62200(3) Å b= |
| | 103.4690(10)° |
| | c = 10.0316(4) Å g = 90° |
| Unit cell volume | 1480.72(5) Å³ |
| Molecule number/unit cell Z | 4 |
| Density (calculated) | 1.266 Mg/m³ |
| Absorption coefficient | 0.488 mm⁻¹ |
| Number of electrons in unit cell F(000) | 608 |
| Crystal size | 0.08 x 0.07 x 0.05 mm³ |
| Theta range for data collection | 3.450 to 54.955° |
| Index ranges 12<=1<=12 | -27<=h<=27, -8<=k<=8, - |
| Reflections collected | 10113 |
| Independent reflections | 2791 [R(int) = 0.0501] |
| Data integrity (Completeness to theta = 53.594°) | 99.2% |
| Correction method (Absorption correction) | Semi-empirical from equivalents |
| Max. and min. transmission | 0.7508 and 0.6140 |
| Refinement method | Full-matrix least-squares on F² |
| Data / restraints / parameters | 2791 / 1 / 185 |
| Goodness-of-fit on F² | 0.993 |
| Final R indices [I>2sigma(I)] | R1 = 0.0411, wR2 = 0.1220 |
| R indices (all data) | R1 = 0.0475, wR2 = 0.1292 |
| Absolute structure parameter | 0.08(14) |
| Largest diff. peak and hole | 0.206 and -0.206 e.Å⁻³ |

### Preparation Example 3 Synthesis of 3-benzyl 8-(tert-butyl)(1S,2S,5R)-2-((R)-1-hydroxyethyl)-3,8-diazabicyclo[3.2.1]octane-3,8-dicarboxylate (A3)

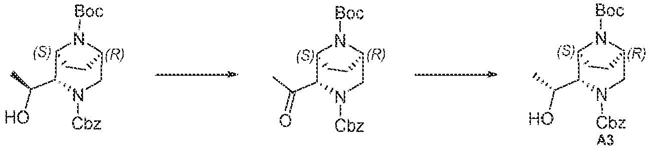

Step 1: 3-Benzyl 8-(tert-butyl)(1S,2S,5R)-2-((S)-1-hydroxyethyl)-3,8-diazabicyclo[3.2.1]octane-3,8-dicarboxylate (120 mg, 0.31 mmol) was dissolved in anhydrous dichloromethane (6 mL). At 0°C, Dess-Martin periodinane (196 mg, 0.46 mmol) was added and the reaction was proceeded for 2 hours at room temperature. Upon completion of the reaction (monitored by LCMS), the reaction mixture was quenched with a saturated solution of sodium thiosulfate (10 mL) and a saturated aqueous solution of sodium bicarbonate (10 mL), and the mixture was extracted with ethyl acetate (20 mL × 2). The organic phase was washed with saturated brine (20 mL), dried over anhydrous sodium sulfate, filtered, and concentrated. The crude product was purified by flash silica gel column chromatography (0-25% ethyl acetate/petroleum ether) to obtain 3-benzyl 8-(tert-butyl) (1S,2S,5R)-2-acetyl-3,8-diazabicyclo[3.2.1]octane-3,8-dicarboxylate (100 mg, yield: 87%). ES-API: [M+H]⁺=389.2. HPLC (column: X-Bridge C18, 4.6* 150mm, 3.5um; mobile phase A: 10mmol/L ammonium bicarbonate aqueous solution, mobile phase B: acetonitrile; flow rate: 0.8ml/min; chromatographic condition: 5%-95% B/A, 15 min; column temperature: 40°C; retention time: 9.57 min).

Step 2: 3-benzyl 8-(tert-butyl) (1S,2S,5R)-2-acetyl-3,8-diazabicyclo[3.2.1]octane-3,8-dicarboxylate (100 mg, 0.26 mmol) was dissolved in anhydrous ethanol (5 mL). At 0°C, a 2 M solution of lithium borohydride in tetrahydrofuran (0.26 mL, 0.52 mmol) was added. The reaction was proceeded for 1 hour at room temperature. Upon completion of the reaction (monitored by LCMS), the reaction mixture was quenched with a saturated solution of ammonium chloride (20 mL) and extracted with ethyl acetate (20 mL × 2). The organic phase was washed with saturated brine (20 mL), dried over anhydrous sodium sulfate, filtered and concentrated. The crude product was purified by flash silica gel column chromatography (0-40% ethyl acetate/petroleum ether) to obtain 3-benzyl 8-(tert-butyl)(1S,2S,5R)-2-((R)-1-hydroxyethyl)-3,8-diazabicyclo[3.2.1]octane-3,8-dicarboxylate (A3, 80 mg, yield: 79.6%), a colorless oily substance, ES-API: [M-55]⁺=335.2. HPLC (column: X-Bridge C18, 4.6* 150mm, 3.5um; mobile phase A: 10mmol/L ammonium bicarbonate aqueous solution, mobile phase B: acetonitrile; flow rate: 0.8ml/min; chromatographic condition: 5%-95% B/A, 15 min; column temperature: 40°C; retention time: 9.46 min).

### Example 15: Synthesis of 5-ethynyl-4-((5S,5aS,6S,9R)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol (Z384)

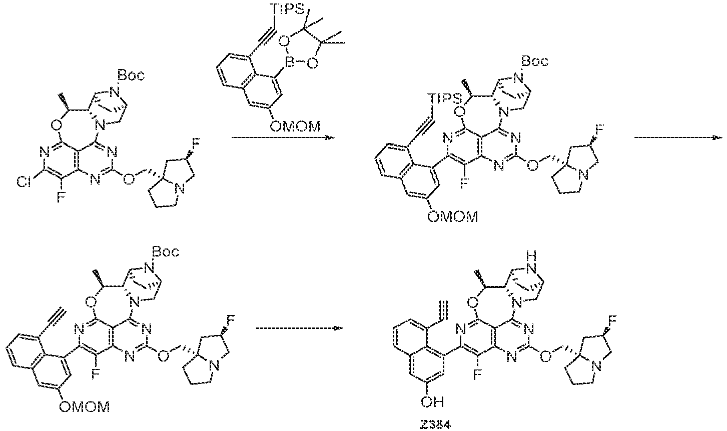

Step 1: To a solution of tert-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,ethanonaphtho[1,8-ab]heptalene-14-carboxylate (40.0mg, 0.067mmol) and triisopropyl(((6-(methoxymethoxy)-8-(4,4,5,5-tetramethyl-1,3,2-dioxaborolane-2-yl)naphthalen-1-yl)ethynyl)silane) (55mg, 0.13mmol) in tetrahydrofuran (6 mL) and water (1.0 mL), potassium phosphate (34 mg, 0.16mmol), methanesulfonic acid [butylbis(1-adamantyl)phosphine](2-amino-1,1'-biphenyl)palladium(II) (15 mg, 0.021mmol) were added, and the mixture was bubbled with nitrogen and reacted at 60°C for 40 minutes. Upon completion of the addition, ethyl acetate (30 mLX2) and saturated brine (10 mL) were used for extraction. The ethyl acetate phases were combined, dried over anhydrous sodium sulfate, filtered, and the filtrate was concentrated under reduced pressure. The crude product was purified by silica gel column chromatography (methanol/dichloromethane=0-4%) to obtain tert-butyl (5S,5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (40mg, yield: 64.5%). ES-API: [M+H]⁺=925.5.

Step 2: To a solution of N,N-dimethylformamide (3.0mL) containing tert-butyl (5S,5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (40mg, 0.043 mmol), cesium fluoride (35mg, 0.22 mmol) was added. The mixture was stirred at room temperature for 0.5 hours. After completion of the reaction, the mixture was filtered and purified by preparative HPLC (trifluoroacetic acid method) to obtain tert-butyl (5S,5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene-14-carboxylate (30mg, yield: 90.9%). ES-API: [M+H]⁺=769.3.

Step 3: A reaction solution of tert-butyl (5S,5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30mg, 0.039 mmol), acetonitrile (2.0mL) and 4M hydrochloric acid/dioxane solution (0.500mL, 2.000mmol) reacted at 0°C for 1 hour. The reaction mixture was concentrated, and the crude product was purified by preparative HPLC (formic acid method 1) to obtain 5-ethynyl-4-((5S,5aS,6S,9R)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol (Z384, 15mg, yield: 62.5%, formate), yellow solid. ES-API: [M+H]⁺=625.3. ¹H NMR (500 MHz, CD₃OD) δ 7.80 (t, *J* = 7.9 Hz, 1H), 7.51 (dd, *J* = 24.3, 6.9 Hz, 1H), 7.39 (dt, *J* = 11.6, 7.7 Hz, 1H), 7.31 (d, *J* = 2.2 Hz, 1H), 7.14 (dd, *J* = 52.3, 2.3 Hz, 1H), 5.49 (d, *J* = 52.1 Hz, 2H), 4.66 -4.42 (m, 3H), 4.21 (d, *J* = 6.5 Hz, 1H), 4.01 -3.56 (m, 5H), 3.35-3.07 (m, 3H), 2.71 -1.73 (m, 10H), 1.59 (t, *J* = 6.1 Hz, 3H).

### Example 16: Synthesis of 1-(8-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-1-yl)propan-2-ol (Z385)

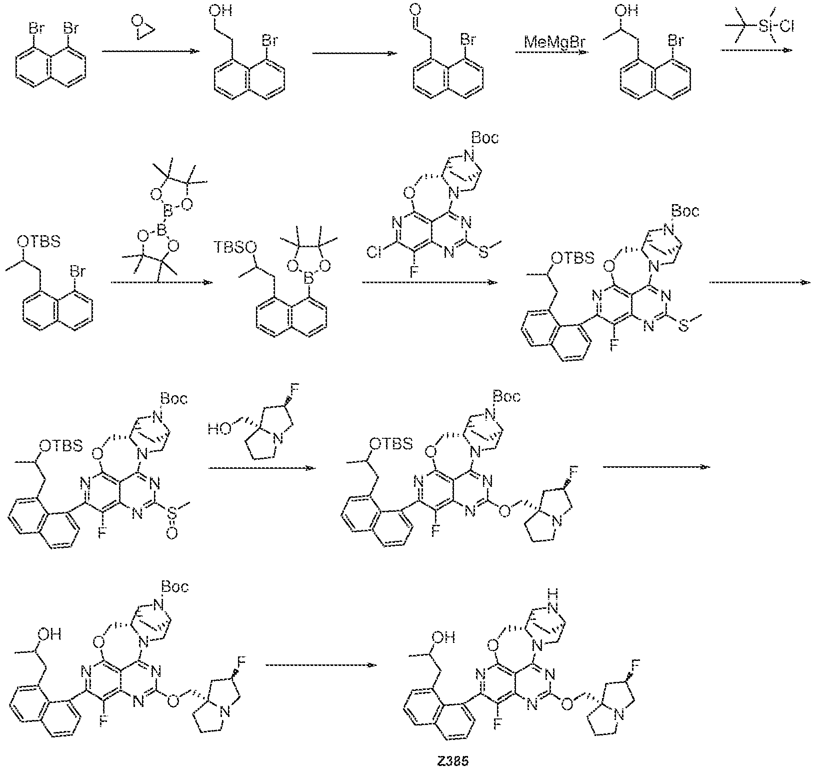

Step 1: Under nitrogen protection, 1,8-dibromonaphthalene (4 g, 13.99 mmol) and tetrahydrofuran (40 mL) were added to a round-bottom flask. The mixture was cooled to -70°C, and a 2.5 M solution of butyllithium in hexane (6.15 mL, 15.39 mmol) was added dropwise. After the addition was complete, the reaction mixture was stirred at -70°C for 30 minutes. A 3M solution of ethylene oxide in tetrahydrofuran (28 mL, 84 mmol) was then added dropwise. After completion of the addition, the reaction mixture was warmed to 0°C and stirred for 1 hour. Ice water (100 mL) was added. The mixture was extracted with ethyl acetate (50 mLX3). The organic phase was washed with saturated brine, dried over anhydrous sodium sulfate, and concentrated to obtain a crude product. The crude product was purified by flash silica gel column chromatography (ethyl acetate/petroleum ether: 0-40%) to obtain 2-(8-bromonaphthalen-1-yl)ethan-1-ol (2 g, yield: 56.9%), a yellow solid. ES-API: [M+Na]⁺=273.1.

Step 2: 2-(8-Bromonaphthalen-1-yl)ethan-1-ol (1.4 g, 5.75 mmol) and dichloromethane (30 mL) were added to a round-bottom flask. Under nitrogen protection, the mixture was cooled to 0°C. With stirred, Dess-Martin periodinane (3.55 g, 8.36 mmol) was added to the reaction mixture. The reaction mixture was stirred at 0°C for 1 hour. Saturated sodium thiosulfate solution (50 mL) and saturated sodium bicarbonate solution (50 mL) were added, and the mixture was stirred at room temperature for 5 minutes. The mixture was extracted with ethyl acetate (100 mLX3). The organic phase was washed with saturated brine, dried over anhydrous sodium sulfate, and concentrated. The crude product was purified by flash silica gel column chromatography (ethyl acetate/petroleum ether: 0-20%) to obtain 2-(8-bromonaphthalen-1-yl)acetaldehyde (1.1 g, yield: 79.2%), a yellow solid. ES-API: [M+H]⁺=249.1.

Step 3: Under nitrogen protection, 2-(8-bromonaphthalen-1-yl)acetaldehyde (1.8 g, 7.23 mmol) and tetrahydrofuran (40 mL) were added to a reaction flask and cooled to -30°C. A 3M solution of methylmagnesium bromide in tetrahydrofuran (2.65mL, 7.95 mmol) was added dropwise to the reaction solution with stirred. The reaction mixture was stirred at -20°C and reacted for 30 minutes. The ice water bath was removed. Saturated ammonium chloride solution (50 mL) was added, and the mixture was extracted with ethyl acetate (50 mLX3). The organic phase was washed with saturated brine, dried over anhydrous sodium sulfate, and concentrated. The crude product was purified by flash silica gel column chromatography (ethyl acetate/petroleum ether: 0-30%) to obtain 1-(8-bromonaphthalen-1-yl)propan-2-ol (1.4 g, yield: 73%). ES-API: [M+H]⁺=287.0.

Step 4: In a reaction flask, to a solution of 1-(8-bromonaphthalen-1-yl)propan-2-ol (1.4 g, 5.28 mmol) and imidazole (0.719 g, 10.56 mmol) in anhydrous N,N-dimethylformamide (15 mL), tert-butyldimethylchlorosilane (1.03 g, 6.86 mmol) was added. The reaction mixture was stirred at room temperature and reacted for 2 hours. Saturated ammonium chloride solution (100 mL) was added, and the mixture was extracted with ethyl acetate (50 mLX2). The organic phase was washed with saturated brine, dried over anhydrous sodium sulfate, and concentrated. The crude product was purified by flash silica gel column chromatography (ethyl acetate/petroleum ether: 0-15%) to obtain ((1-(8-broinonaphthalen-1-yl)propan-2-yl)oxy)(tert-butyl)dimethylsilane (1.9 g, yield: 94.8%). ES-API: [2M+Na]⁺=779.5.

Step 5: Into a round-bottom flask, ((1-(8-bromonaphthyl-1-yl)propyl-2-yl)oxy)(tert-butyl)dimethylsilane (1.8 g, 4.74 mmol), phenylboronic acid pinacol ester (2.41 g, 9.49 mmol), [1,1'-bis(diphenylphosphino)ferrocene]dichloropalladium(II) (347 mg, 0.47 mmol), potassium acetate (1.40 g, 14.23 mmol), and 1,4-dioxane (30 mL) were added. Under nitrogen protection, the system was heated in a 95°C oil bath and stirred for 12 hours. Water (50 mL) was added to the reaction mixture, which was then extracted with ethyl acetate (30 mLX3). The organic phase was dried over anhydrous sodium sulfate, concentrated, and the crude product was purified by flash silica gel column chromatography (ethyl acetate/petroleum ether: 0-10%) to obtain tert-butyldimethyl((1-(8-(4,4,5,5-tetramethyl-1,3,2-dioxaborolan-2-yl)naphthalen-1-yl)propan-2-yl)oxy)silane (1.3 g, crude product), a yellow oil. ES-API: [M+Na]⁺=449.4.

Step 6: Into a round-bottom flask, tert-butyldimethyl(( 1-(8-(4,4,5,5-tetramethyl-1,3,2-dioxaborolan-2-yl)naphthalen-1-yl)propan-2-yl)oxy)silane (260 mg, 0.43 mmol), tert-butyl (SaS,6S,9R)-2-chloro-1-fluoro-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.17 mmol), methane sulfonic acid [butylbis(1-adamantyl)phosphine](2-amino-1,1'-biphenyl)palladium(II) (12.4 mg, 0.017 mmol), potassium phosphate (109 mg, 0.51 mmol), tetrahydrofuran (5 mL), and water (1 mL) were added. Under nitrogen protection, the system was heated in an 80°C microwave reactor for 40 minutes. Water (30 mL) was added to the reaction mixture, which was then extracted with ethyl acetate (30 mLX3). The organic phase was dried over anhydrous sodium sulfate, concentrated, and the crude product was purified by flash silica gel column chromatography (ethyl acetate/petroleum ether: 0-30%) to obtain tert-butyl (5aS,6S,9R)-2-(8-(2-((tert-butyldimethylsilyl)oxy)propyl)naphthalen-1-yl)-1-fluoro-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, yield: 79.9%), a yellow solid. ES-API: [M+H]⁺=732.3.

Step 7: Into a round-bottom flask, tert-butyl (5aS,6S,9R)-2-(8-(2-((tertbutyldimethylsilyl)oxy)propyl)naphthalen-1-yl)-1-fluoro-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.14 mmol) and dichloromethane (8 mL) were added. The mixture was cooled to 0°C, and m-chloroperbenzoic acid (36 mg, 0.20 mmol) was added to the reaction mixture. The reaction mixture was stirred at 0°C for 1 hour. Water (30 mL) was added, and the mixture was extracted with ethyl acetate (30 mLX3). The organic phase was dried over anhydrous sodium sulfate, concentrated, and the crude product was purified by flash silica gel column chromatography (ethyl acetate/petroleum ether: 0-100%) to obtain tert-butyl (5aS,6S,9R)-2-(8-(2-((tertbutyldimethylsilyl)oxy)propyl)naphthalen-1-yl)-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, yield: 97.8%), a yellow solid. ES-API: [M+H]⁺=748.3.

Step 8: Sodium hydride (16 mg, 0.40 mmol) and tetrahydrofuran (5 mL) were added to a round-bottom flask and cooled to 0 °C. ((2R,7aS)-2-Fluoro-tetrahydro-1H-pyrrolo[5H]-pyrazin-7a-yl)methanol (64 mg, 0.40 mmol) was then added to the reaction mixture. The reaction mixture was stirred at 0°C for 10 minutes. A tetrahydrofuran solution (2 mL) of tert-butyl (5aS,6S,9R)-2-(8-(2-((tert-butyldimethylsilyl)oxy)propyl)naphthalen-1-yl)-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, 0.13 mmol) was added dropwise. After the addition was complete, the reaction mixture was stirred at 0°C for 30 minutes. Water (30 mL) was added to the reaction mixture, and the mixture was extracted with ethyl acetate (30 mLX3). The organic phase was dried over anhydrous sodium sulfate, concentrated, and the crude product was purified by thin-layer chromatography (ethyl acetate) to obtain tert-butyl (5aS,6S,9R)-2-(8-(2-((tertbutyldimethylsilyl)oxy)propyl)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (20 mg, yield: 17.7%). ES-API: [M+H]⁺=843.3.

Step 9: Tert-butyl (5aS,6S,9R)-2-(8-(2-((tert-butyldimethylsilyl)oxy)propyl)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (20 mg, 0.024 mmol), tetra-n-butylammonium fluoride (1 mL, 1 mmol, 1M tetrahydrofuran solution), and tetrahydrofuran (1 mL) were added to a round-bottom flask. The reaction mixture was stirred at room temperature for 24 hours. Water (30 mL) was added to the reaction mixture, and the mixture was extracted with ethyl acetate (30 mLX2). The organic phase was washed three times with saturated brine. The organic phase was dried over anhydrous sodium sulfate, concentrated to obtain tert-butyl (5aS,6S,9R)-1-fluoro-12-(((2R,7aS)-2-fluorotetrahydro-1H-pyrrolizin-7a(5H-yl)methoxy)-2-(8-(2-hydroxypropyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (16 mg, crude product), which was then used directly in the next reaction. ES-API: [M+H]⁺=729.3.

Step 10: Tert-butyl (5aS,6S,9R)-1-fluoro-12-(((2R,7aS)-2-fluorotetrahydro-1H-pyrrolizin-7a(5H)-yl)methoxy)-2-(8-(2-hydroxypropyl)naphthalen-1 -yl)-5a,6,7,8,9,10-hexahydro-5H-4-oxa-3,10a,11,13,14-pentaaza-6,9-methanonaphtho[1,8-ab]heptalene-14-carboxylate (16 mg, 0.022 mmol) and acetonitrile (1 mL) were added to a round-bottom flask. A 4M hydrochloric acid/dioxane solution (1 mL) was then added. The reaction mixture was stirred at room temperature for 1 hour. The reaction mixture was concentrated to dryness, and the crude product was purified by preparative HPLC (ammonium bicarbonate method 1) to obtain 1-(8-((SaS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-1-yl)propan-2-ol (Z385, 6 mg, yield: 43.4%), white solid. ES-API:[M+H]⁺=629.3.

### Example 17: Synthesis of 4-((5S,5aS,6S,9R)-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-ol (Z386)

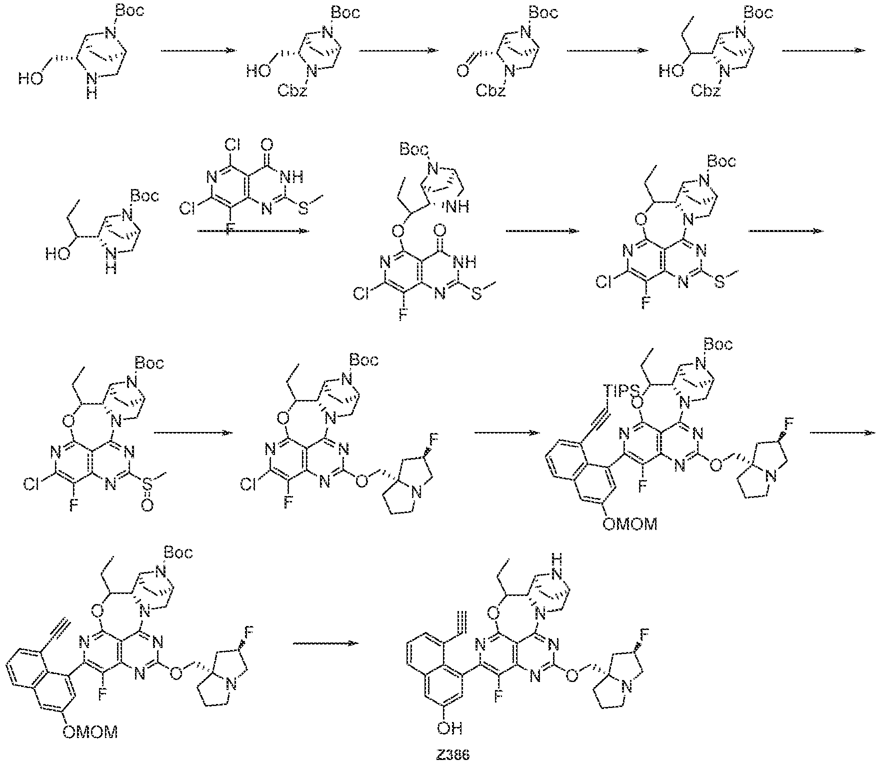

Step 1: (1S,2S,SR)-tert-butyl 2-(hydroxymethyl)-3,8-diazabicyclo[3.2.1]octane-8-carboxylate (3.0 g, 12.40 mmol) was dissolved in a mixture of ethyl acetate (30 mL) and water (10 mL). Benzyl chloroformate (2.54 g, 14.88 mmol) and sodium bicarbonate (2.98 g, 32.0 mmol) were added, and the mixture was stirred overnight at room temperature. Water (50 mL) was added, and the mixture was extracted with ethyl acetate (100 mLX3). The organic phase was washed with saturated brine (20 mL), dried over anhydrous sodium sulfate, concentrated, and the crude product was purified by silica gel column chromatography (petroleum ether/ethyl acetate = 2:1) to obtain 3-benzyl 8-(tert-butyl)(1S,2S,5R)-2-(hydroxymethyl)-3,8-diazabicyclo[3.2.1]octane-3,8-dicarboxylate (4.35 g, yield: 93.3%).

Step 2: Under nitrogen protection and at -78 °C, a solution of dimethyl sulfoxide (156 mg, 1.99 mmol) in anhydrous dichloromethane (5.0 mL) was slowly added dropwise to a solution of oxalyl chloride (223 mg, 1.75 mmol) in anhydrous dichloromethane (10 mL). The mixture was stirred at this temperature for 15 minutes. A solution of 3-benzyl 8-(tert-butyl)(1S,2S,5R)-2-(hydroxymethyl)-3,8-diazabicyclo[3.2.1]octane-3,8-dicarboxylate (300 mg, 0.80 mmol) in anhydrous dichloromethane (5.0 mL) was slowly added dropwise and reacted at -78 °C for 40 minutes. Triethylamine (646 mg, 6.38 mmol) was slowly added. The reaction was proceeded at -78 °C for 1 hour. Saturated ammonium chloride solution (20 mL) was added to quench the reaction, and the mixture was extracted with ethyl acetate. After dried over anhydrous sodium sulfate and concentration, the crude product 3-benzyl 8-(tert-butyl)(1S,2S,5R)-2-formyl-3,8-diazabicyclo[3.2.1]octane-3,8-dicarboxylate (270 mg, yield: 90.2%) was obtained.

Step 3: 3-Benzyl 8-(tert-butyl)(1S,2S,5R)-2-formyl-3,8-diazabicyclo[3.2.1]octane-3,8-dicarboxylate (270 mg, 0.72 mmol) was dissolved in dry tetrahydrofuran (5 mL). Under nitrogen protection, the solution was cooled to -78 °C, and a 3M solution of ethylmagnesium bromide in ether (0.4 mL) was added dropwise. The reaction was proceeded for 2 hours at -78 °C. Saturated ammonium chloride solution (5 mL) was added, and the mixture was extracted with ethyl acetate. After dried over anhydrous sodium sulfate and concentration, the crude product was purified by thin-layer chromatography (ethyl acetate/petroleum ether = 1:3) to obtain 3-benzyl 8-(tert-butyl)(1S,2S,5R)-2-((S)-1-hydroxypropyl)-3,8-diazabicyclo[3.2.1]octane-3,8-dicarboxylate (180 mg, yield: 62.1%).

Step 4: 3-Benzyl 8-(tert-butyl)(1S,2S,5R)-2-((S)-1-hydroxypropyl)-3,8-diazabicyclo[3.2.1]octane-3,8-dicarboxylate (180 mg, 0.45 mmol) was dissolved in methanol (4 mL) and water (0.5 mL). Ammonium formate (290 mg, 4.6 mmol) and 10% palladium on carbon (20 mg) were added, and the mixture reacted in a sealed tube at 60 °C for 2 hours. After filtration and concentration, the crude product was purified by preparative thin-layer chromatography (dichloromethane/methanol = 10:1) to obtain tert-butyl (1S,2S,5R)-2-((S)-1-hydroxypropyl)-3,8-diazabicyclo[3.2.1]octane-8-carboxylate (85 mg, yield: 70.0%).

Step 5: At 0 °C, sodium hydride (60% dispersion in oil) (40 mg, 1.0 mmol) was added to a tetrahydrofuran (10 mL) suspension, tert-butyl (1S,2S,5R)-2-((S)-1-hydroxypropyl)-3,8-diazabicyclo[3.2.1]octane-8-carboxylate (85 mg, 0.31 mmol) was added and stirred at this temperature for 10 minutes. 5,7-Dichloro-8-fluoro-2-(methylthio)pyrido[4,3-d]pyrimidin-4(3H)-one (112 mg, 0.40 mmol) was added and heated at 60 °C for 1 hour. Upon completion of the reaction, water was used to quench the reaction, and the mixture was extracted with dichloromethane/methanol (10:1, 25 mLX3). The organic phase was dried over anhydrous sodium sulfate, filtered, and concentrated to obtain tert-butyl (1S,2S,5R)-2-((S)-1-((7-chloro-8-fluoro-2-(methylthio)-4-oxo-3,4-dihydropyrido[4,3-d]pyrimidin-5-yl)oxy)propyl)-3,8-diazabicyclo[3.2.1]octane-8-carboxylate (120 mg, yield: 75.5%). ES-API: [M+H]⁺=514.2.

Step 6: To a dichloromethane (10 mL) solution of tert-butyl (1S,2S,5R)-2-((S)-1-((7-chloro-8-fluoro-2-(methylthio)-4-oxo-3,4-dihydropyrido[4,3-d]pyrimidin-5-yl)oxy)propyl)-3,8-diazabicyclo[3.2.1]octane-8-carboxylate (120 mg, 0.23 mmol) and N,N-diisopropylethylenediamine (85 mg, 0.69 mmol), propylphosphonic anhydride (50% in ethyl acetate) (280 mg, 0.46 mmol) was added and the mixture was stirred at room temperature for 5 hours. The reaction mixture was quenched with water and extracted with dichloromethane. The organic phase was dried over anhydrous sodium sulfate, filtered, and concentrated. The crude product was purified by silica gel column chromatography (petroleum ether/ethyl acetate = 3:1) to obtain tert-butyl (5S,5aS,6S,9R)-2-chloro-1-fluoro-5-ethyl-12-(methylthio)-5a,6,7,8,9,10-hexahydro-5H-4-oxo-3,10a,11,13,14-pentazacycloundecene[1,8-ab]naphthalene-14-carboxylate (80 mg, yield: 70.1%). ES-API: [M+H]⁺=496.2.

Step 7: At 0°C, m-chloroperoxybenzoic acid (36 mg, 0.20 mmol) was added to a dichloromethane (15 mL) solution of tert-butyl (5S,5aS,6S,9R)-2-chloro-1-fluoro-5-ethyl-12-(methylsulfonyl)-5a,6,7,8,9,10-hexahydro-5H-4-oxa-3,10a,11,13,14-pentazacycloundecene[1,8-ab]naphthalene-14-carboxylate (80 mg, 0.16 mmol). The mixture was stirred at room temperature and reacted for 1 hour, diluted with dichloromethane (30 mL) and washed with 10% sodium hydroxide solution (5.0 mLX2). The organic phase was dried over anhydrous sodium sulfate and concentrated to obtain the crude product tert-butyl (5S,5aS,6S,9R)-2-chloro-1-fluoro-5-ethyl-12-(methylsulfonyl)-5a,6,7,8,9,10-hexahydro-5H-4-oxa-3,10a,11,13,14-pentazacycloundecene[1,8-ab]naphthalene-14-carboxylate (78 mg, yield: 95.2%). ES-API: [M+H]⁺=512.2.

Step 8: At 0°C, ((2R,7aS)-2-fluoro-tetrahydro-1H-pyrrolizin-7a(5H)-yl)methanol (40 mg, 0.28 mmol) was added to a tetrahydrofuran solution of sodium hydride (60% dispersion in oil) (15 mg, 0.36 mmol). The mixture was stirred at room temperature and reacted for 10 minutes, cooled to 0 °C and a tetrahydrofuran solution (5 mL) of tert-butyl (5S,5aS,6S,9R)-2-chloro-1-fluoro-5-ethyl-12-(methylsulfonyl)-5a,6,7,8,9,10-hexahydro-5H-4-oxa-3,10a,11,13,14-pentazacycloundecene[1,8-ab]naphthalene-14-carboxylate (80 mg, 0.16 mmol) was added. The reaction mixture was stirred at 0°C and reacted for 1 hour, quenched with saturated bicarbonate solution and extracted with ethyl acetate. The organic phase was washed with saturated brine, dried over anhydrous sodium sulfate, filtered, and concentrated. The crude product was purified by silica gel column chromatography (methanol/dichloromethane = 0-5%) to obtain tert-butyl (5S,5aS,6S,9R)-2-chloro-5-ethyl-1-fluoro-12-(((2R,7aS)-2-fluoro-tetrahydro-1H-pyrrolizin-7a(5H)-yl)methoxy)-5a,6,7,8,9,10-hexahydro-5H-4-oxa-3,10a,11,13,14-pentazacycloundecene[1,8-ab]naphthalene-14-carboxylate (40 mg, yield: 41.1%). ES-API: [M+H]⁺=607.3.

Step 9: To tert-butyl (5S,5aS,6S,9R)-2-chloro-5-ethyl-1-fluoro-12-(((2R,7aS)-2-fluoro-tetrahydro-1H-pyrrolizin-7a(5H)-yl)methoxy)-5a,6,7,8,9,10-hexahydro-5H-4-oxa-3,10a,11,13,14-pentazacycloundecene[1,8-ab]naphthalene-14-carboxylate (40.0 mg, 0.066 mmol) and triisopropyl((8-(4,4,5,5-tetramethyl-1,3,2-dioxaborolane-2-yl)naphthalen-1-yl)ethynyl)silane (55 mg, 0.13 mmol) in tetrahydrofuran (6 mL) and water (1.0 mL) solution, potassium phosphate (34 mg, 0.16 mmol), methanesulfonic acid[butylbis(1-adamantyl)phosphine](2-amino-1,1'-biphenyl-2-yl)palladium(II) (15 mg, 0.021 mmol) were added. The mixture was bubbled with nitrogen and reacted at 60°C for 40 minutes. Upon completion of the reaction, the mixture was extracted with ethyl acetate (30 mLX2) and saturated brine (10 mL). The ethyl acetate phases were combined, dried over anhydrous sodium sulfate, filtered, and the filtrate was concentrated under reduced pressure. The crude product was purified by silica gel column chromatography (methanol/dichloromethane = 0-4%) to obtain tert-butyl (5S,5aS,6S,9R)-5-ethyl-1-fluoro-12-(((2R,7aS)-2-fluoro-tetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentazacycloundecene[1,8-ab]naphthalene-14-carboxylate (38 mg, yield: 61.3%). ES-API: [M+H]⁺=939.5.

Step 10: To a N,N-dimethylfonnamide (3.0 mL) solution of tert-butyl (5S,5aS,6S,9R)-5-ethyl-1-fluoro-12-(((2R,7aS)-2-fluoro-tetrahydro-1H-pyrrolizin-7a(5H)-yl)methoxy)-2-(3-(methoxymethoxy)-8-(triisopropylsilyl)ethynyl)naphthalen-1-yl)-5a,6,7,8,9,10-hexahydro-5H-4-oxa-3,10a,11,13,14-pentazacycloundecene[1,8-ab]naphthalene-14-carboxylate (38 mg, 0.040 mmol), cesium fluoride (40 mg, 0.26 mmol) was added. The reaction mixture was stirred at room temperature and reacted for 0.5 hours. Upon completion of the reaction, the mixture was filtered and purified by preparative HPLC (trifluoroacetic acid method) to obtain tert-butyl (5S,5aS,6S,9R)-5-ethyl-2-(8-ethynyl-3-(methoxymethoxy)naphthalen-1-yl)-1-fluoro-12-(((2R,7aS)-2-fluoro-tetrahydro-1H-pyrrolizin-7a(5H)-yl)methoxy)-5a,6,7,8,9,10-hexahydro-5H-4-oxa-3,10a,11,13,14-pentazacycloundecene[1,8-ab]naphthalene-14-carboxylate (25 mg, yield: 79.0%). ES-API: [M+H]⁺=783.3.

Step 11: A reaction solution of tert-butyl (5S,5aS,6S,9R)-5-ethyl-2-(8-ethynyl-3-(methoxymethoxy)naphthalen-1-yl)-1-fluoro-12-(((2R,7aS)-2-fluoro-tetrahydro-1H-pyrrolizin-7a(5H)-yl)methoxy)-5a,6,7,8,9,10-hexahydro-5H-4-oxa-3,10a,11,13,14-pentazacycloundecene[1,8-ab]naphthalene-14-carboxylate (25 mg, 0.032 mmol) in acetonitrile (2.0 mL) and 4M hydrochloric acid/dioxane solution (0.500 mL, 2.000 mmol) reacted at 0°C for 1 hour. The reaction mixture was concentrated to obtain the crude product, which was then purified by preparative HPLC (formic acid method) to obtain 4-((SS,SaS,6S,9R)-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-ol (Z386, 8mg, yield: 40.0%, formate), yellow solid. ES-API: [M+H]⁺=639.3. ¹H NMR (400 MHz, CD₃OD) δ 8.44 (s, 2H), 7.81 (d, *J* = 5.2 Hz, 1H), 7.51 (dd, *J* = 14.2, 6.4 Hz, 1H), 7.39 (dd, *J* = 15.1, 6.9 Hz, 1H), 7.31 (s, 1H), 7.12 (dd, *J* = 41.3, 2.4 Hz, 1H), 5.60 -5.36 (m, 2H), 4.63 -4.47 (m, 2H), 4.48 -4.23 (m, 2H), 4.00 -3.44 (m, 5H), 3.39 (s, 0.49H), 3.35-3.30 (m, 2H), 3.08 (s, 0.51H), 2.73 - 1.57 (m, 12H), 1.22 -1.10 (m, 3H).

### Example 18: Synthesis of (5S,5aS,6S,9R)-5-ethyl-2-(8-ethynylnaphthalen-1-yl)-1-fluorn-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z387)

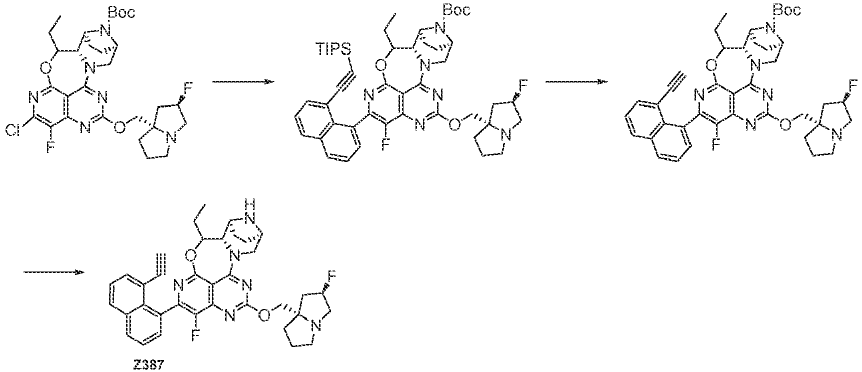

Step 1: To tert-butyl (SaS,6S,9R)-2-chloro-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (40.0 mg, 0.066 mmol) and triisopropyl(((8-(4,4,5,5-tetramethyl-1,3,2-dioxaborolan-2-yl)naphthalen-1-yl)ethynyl)silane) (55 mg, 0.13 mmol) in tetrahydrofuran (6 mL) and water (1.0 mL), potassium phosphate (34 mg, 0.16 mmol) and methanesulfonic acid n-butylbis(1-adamantyl)phosphinepalladium(II) (15 mg, 0.021 mmol) were added. The mixture was bubbled with nitrogen and reacted at 60°C for 40 minutes. Upon completion of the reaction, the reaction mixture was extracted with ethyl acetate (30 mLX2) and saturated brine (10 mL). The ethyl acetate phases were combined, dried over anhydrous sodium sulfate, filtered, and the filtrate was concentrated under reduced pressure. The crude product was purified by silica gel column chromatography (methanol/dichloromethane = 0-4%) to obtain tert-butyl (5aS,6S,9R)-5-ethyl-1-fluoro-12-(((2R,7aS)-2-fluorotetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (40 mg, yield: 69.0%).

Step 2: To a N,N-dimethylformamide (3.0 mL) solution of tert-butyl (5aS,6S,9R)-5-ethyl-1-fluoro-12-(((2R,7aS)-2-fluorotetrahydro-1H-pyrrolizin-7a(5H)-yl)methoxy)-2-(8-((triisopropylsilyl)ethynyl)naphthalen-1 -yl)-5a,6,7,8,9,10-hexahydro-5H-4-oxa-3,10a, 11,13,14-pentaaza-6,9-methanonaphtho[1,8-ab]heptalene-14-carboxylate (40 mg, 0.046 mmol), cesium fluoride (40 mg, 0.23 mmol) was added. The reaction mixture was stirred at room temperature and reacted for 0.5 hours. Upon completion of the reaction, the reaction mixture was filtered and purified by preparative HPLC (trifluoroacetic acid method) to obtain tert-butyl (5aS,6S,9R)-5-ethyl-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (25 mg, yield: 75.7%).

Step 3: A reaction solution of tert-butyl (5aS,6S,9R)-5-ethyl-2-(8-ethynylnaphthalen-1-yl)-1-fluoro-12-(((2R,7aS)-2-fluorotetrahydro-1H-pyrrolizin-7a(5H-yl)methoxy)-5a,6,7,8,9,10-hexahydro-5H-4-oxa-3,10a,11,13,14-pentaaza-6,9-methanonaphtho[1,8-ab]heptalene-14-carboxylate (25 mg, 0.034 mmol) in acetonitrile (2.0 mL) and 4M hydrochloric acid/dioxane solution (0.500 mL, 2.000 mmol) reacted at 0°C for 1 hour. The reaction mixture was concentrated, and the crude product was purified by preparative HPLC (formic acid method 1) to obtain (5S,5aS,6S,9R)-5-ethyl-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z387, 10mg, yield: 46.5%, formate), yellow solid. ES-API: [M+H]⁺=623.2. ¹H NMR (400 MHz, CD₃OD) δ 8.36 (s, 2H), 8.15 -7.96 (m, 2H), 7.81 -7.35 (m, 4H), 5.50 (dd, *J* = 33.0, 19.1 Hz, 2H), 4.69 -4.28 (m, 4H), 4.00-3.65 (m, 5H), 3.45 (s, 0.55H), 3.42 -3.34 (m, 2H), 3.16 (s, 0.46H), 2.65 -1.79 (m, 12H), 1.17 (t, *J* = 6.7 Hz, 3H).

### Example 19: Synthesis of (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z388)

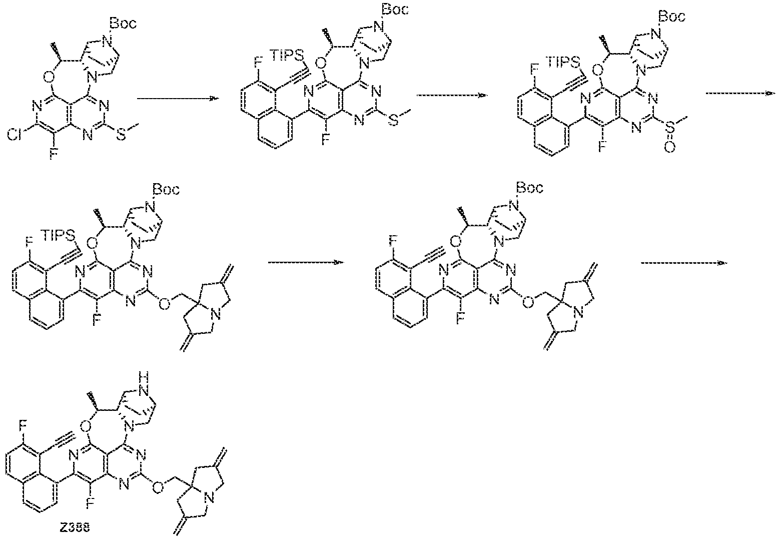

Step 1: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (150 mg, 0.311 mmol), ((2-fluoro-8-(4,4,5,5-tetramethyl-1,3,2-dioxaborolan-2-yl)naphthalen-1-yl)ethynyl)triisopropylsilane (211 mg, 0.466 mmol), potassium phosphate (132 mg, 0.62 mmol) and methanesulfonic acid [n-butylbis(1-adamantyl)phosphine](2-amino-1,1'-biphenyl-2-yl)palladium(II) (23 mg, 0.031 mmol) were dissolved in tetrahydrofuran (6.0 mL) and water (1.5 mL). The reaction mixture was heated and stirred at 80°C under microwave for 40 minutes. Upon completion of the reaction (monitored by LCMS), the reaction mixture was concentrated under reduced pressure, and the crude product was purified by silica gel column chromatography (dichloromethane:methanol = 20:1) to obtain tert-butyl (SS,SaS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (290 mg, crude product).ES-API: [M+H]⁺= 772.3.

Step 2: Tert-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (290 mg, crude product) was dissolved in dichloromethane (10.0 mL). Under an ice-water bath, m-chloroperoxybenzoic acid (70 mg, 0.406 mmol) was added. The reaction was proceeded in an ice-water bath for 30 minutes. Upon completion of the reaction (monitored by LCMS), the reaction mixture was diluted with dichloromethane (20 mL), washed with saturated sodium sulfite solution (15 mLX 2), and saturated brine (15 mLX 3), then dried over anhydrous sodium sulfate, filtered, and concentrated to obtain the crude product, tert-butyl (5S,5aS,6S,9R)-1-fluoro-2-(7-fluoro-8-((triisopropylsilyl)ethynyl)naphthalen-1-yl)-5-methyl- 12-(methylsulfinyl)-5a,6,7,8,9,10-hexahydro-5H-4-oxa-3,10a, 11,13,14-pentaaza-6,9-methanonaphtho[1,8-ab]heptalene-14-carboxylate (280 mg, crude product). ES-API: [M+H]⁺= 788.3.

Step 3: (2,6-Dimethyltetrahydro-1H-pyrrolo[5H]azepin-7a-yl)methanol (175 mg, 1.059 mmol) was dissolved in anhydrous tetrahydrofuran (15 mL). Under an ice-water bath, sodium hydride (85 mg, 2.118 mmol) was added. The reaction was proceeded for 30 minutes in the ice-water bath. A tetrahydrofuran (5 mL) solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (280 mg, crude product) was added dropwise to the above reaction mixture. The reaction was proceeded for 30 minutes at room temperature. Upon completion of the reaction (monitored by LCMS), the reaction mixture was diluted with ethyl acetate (80 mL), washed with saturated ammonium chloride solution (15 mLX2), and saturated brine (60 mLX3), then dried over anhydrous sodium sulfate, filtered, and concentrated to obtain the crude product, tert-butyl (5S,5aS,6S,9R)-12-((2,6-dimethylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (460 mg, crude product). ES-API: [M+H]⁺= 889.3.

Step 4: Cesium fluoride (900 mg, 5.921 mmol) was added to a solution of tert-butyl (5S,5aS,6S,9R)-12-((2,6-dimethylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (460 mg, crude product) in N,N-dimethylformamide (10 mL). The reaction mixture was stirred at room temperature for 2 hours. Upon completion of the reaction (monitored by LCMS), the reaction mixture was diluted with ethyl acetate (60 mL), washed with water (15 mLX2), and saturated brine (60 mLX3), then dried over anhydrous sodium sulfate, filtered, and concentrated to obtain tert-butyl (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (480 mg, crude product). ES-API: [M+H]⁺= 733.3.

Step 5: Under an ice-water bath, a 4 M hydrochloric acid-dioxane solution (5 mL) was slowly added to a solution of tert-butyl (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (480 mg, crude product) in acetonitrile (10.0 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic condition: 15 mL-35-85-13 min) to obtain a pale yellow solid, (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-SH-4-oxa.-3,10a,11,13,14-pentaaza-6,9-methanonaphtho[1,8-ab]heptalene (Z388, 36mg, total yield for 5 steps: 18.2%). ES-API: [M+H]⁺= 633.3. ¹H NMR (500 MHz, CD₃OD) δ 8.13 -8.03 (m, 2H), 7.68 -7.54 (m, 2H), 7.43 (dt, *J* = 11.4, 8.9 Hz, 1H), 5.35 (dd, *J* = 13.3, 2.6 Hz, 1H), 5.02 (d, *J* = 8.3 Hz, 4H), 4.54 (dq, *J* = 12.9, 6.3 Hz, 1H), 4.34 -4.24 (m, 2H), 4.07 (d, *J* = 8.8 Hz, 1H), 3.67 (d, *J* = 5.8 Hz, 1H), 3.59 (s, 1H), 3.50 (s, 0H), 3.35 (s, 1H), 3.32 (s, 1H), 3.17 (t, *J* = 12.8 Hz, 1H), 2.79 (dd, *J* = 16.6, 7.6 Hz, 2H), 2.59 (d, *J* = 16.3 Hz, 2H), 2.09 -1.99 (m, 1H), 1.91 -1.73 (m, 3H), 1.57 (dd, *J* = 9.8, 6.4 Hz, 3H), 1.28 (s, 1H).

### Example 20: Synthesis of (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z550)

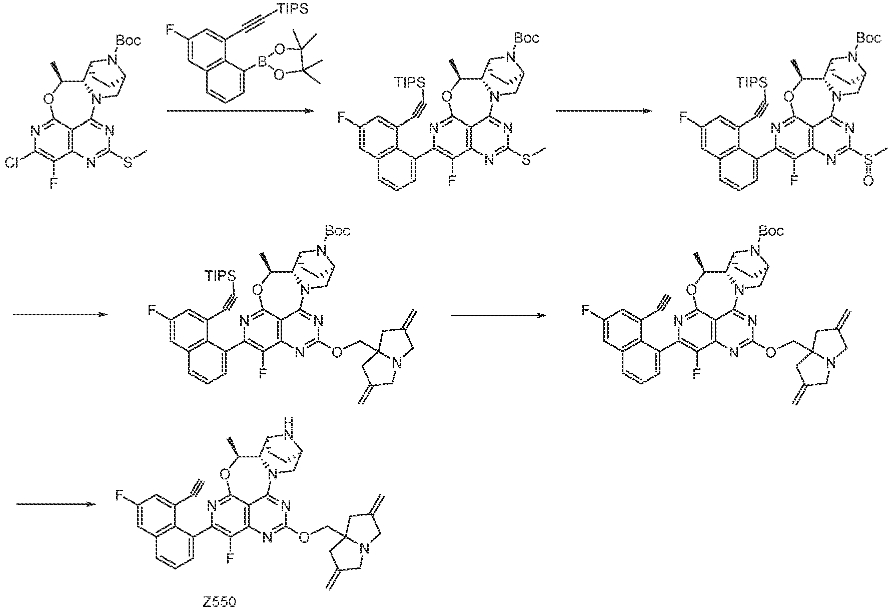

Step 1: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.21 mmol), ((3-fluoro-8-(4,4,5,5-tetramethyl-1,3,2-dioxaborolan-2-yl)naphthalen-1-yl)ethynyl)triisopropylsilane (234 mg, 0.52 mmol), potassium phosphate (132 mg, 0.62 mmol), and methanesulfonic acid [n-butylbis(1-adamantyl)phosphine](2-amino-1,1'-biphenyl-2-yl)palladium(II) (23 mg, 0.031 mmol) were dissolved in tetrahydrofuran (5 mL) and water (1 mL). The reaction mixture was stirred at 80°C under microwave for 40 minutes. Upon completion of the reaction (monitored by LCMS), the reaction mixture was concentrated under reduced pressure, and the crude product was purified by silica gel column chromatography (dichloromethane:methanol = 20:1) to obtain tert-butyl (5S,5aS,6S,9R)-1-fluoro-2-(6-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (128 mg, yield: 80%). ES-API: [M+H]⁺= 772.3.

Step 2: Tert-butyl (5S,5aS,6S,9R)-1-fluoro-2-(6-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (128 mg, 0.17 mmol) was dissolved in dichloromethane (5 mL). Under an ice-water bath, m-chloroperoxybenzoic acid (34 mg, 0.20 mmol) was added. The reaction was proceeded at room temperature for 30 minutes. Upon completion of the reaction (monitored by LCMS), the reaction mixture was diluted with dichloromethane (20 mL), washed with saturated sodium sulfite solution (15 mLX 2), and saturated brine (15 mLX 3), then dried over anhydrous sodium sulfate, filtered, and concentrated to obtain the crude product, tert-butyl (5S,5aS,6S,9R)-1-fluoro-2-(6-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (170 mg, yield: 100%). ES-API: [M+H]⁺= 788.3.

Step 3: (2,6-Dimethyltetrahydro-1H-pyrrolizin-7a(5H)-yl)methanol (36 mg, 0.21 mmol) was dissolved in anhydrous tetrahydrofuran (5 mL). Under an ice-water bath, sodium hydride (22 mg, 0.54 mmol) was added. The reaction was proceeded for 30 minutes in the ice-water bath. A tetrahydrofuran (5 mL) solution of tert-butyl (SS,SaS,6S,9R)-1-fluoro-2-(6-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (170 mg, 0.21 mmol) was added dropwise to the above reaction mixture. The reaction was proceeded at room temperature for 30 minutes. Upon completion of the reaction (monitored by LCMS), the reaction mixture was diluted with ethyl acetate (20 mL), washed with saturated ammonium chloride solution (15 mLX2), and saturated brine (15 mLX3), then dried over anhydrous sodium sulfate, filtered, and concentrated to obtain the crude product, tert-butyl (5S,5aS,6S,9R)-12-((2,6-dimethylenetetrahydro-1H-pyrrolizin-7a(5H)-yl)methoxy)-1-fluoro-2-(6-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (165 mg, yield: 86%). ES-API: [M+H]⁺= 889.3.

Step 4: Cesium fluoride (282 mg, 1.86 mmol) was added to a solution of tert-butyl (5S,5aS,6S,9R)-12-((2,6-dimethylenetetrahydro-1H-pyrrolizin-7a(5H)-yl)methoxy)-1-fluoro-2-(6-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (165 mg, 0.18 mmol) in N,N-dimethylformamide (3 mL). The reaction mixture was stirred at room temperature for 2 hours. Upon completion of the reaction (monitored by LCMS), the reaction mixture was diluted with ethyl acetate (20 mL), washed with water (15 mLX2), and saturated brine (15 mLX3), then dried over anhydrous sodium sulfate, filtered, and concentrated to obtain tert-butyl (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (136 mg, yield: 100%). ES-API: [M+H]⁺= 733.3.

Step 5: Under an ice-water bath, a 4 M hydrochloric acid-dioxane solution (5 mL) was slowly added to a solution of tert-butyl (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6, 7,8,9,10-hexahydro-5H-4-oxa-3,10a,11,13,14-pentaaza-6,9-methanonaphtho[1,8-ab]heptalene-14-carboxylate (136 mg, 0.18 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic condition: 15mL-35-85-13min) to obtain a pale yellow solid, (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z550, 20 mg, yield: 17 %). ES-API: [M+H]⁺= 633.3. ¹H NMR (500 MHz, CD₃OD)δ 8.04 (d, *J* = 7.5 Hz, 1H), 7.77-7.24 (m, 1H), 7.69-7.62 (m, 1H), 7.58-7.54 (m, 1H), 7.54-7.50 (m, 1H), 7.49-7.46 (m, 1H), 5.36-5.33 (m, 1H), 5.02 (d, *J* = 8.0 Hz, 4H), 4.55-4.51 (m, 1H), 4.33 -4.26 (m, 2H), 4.07 (d, *J* = 8.5 Hz, 1H), 3.76 (d, *J* = 14.5 Hz, 2H), 3.68-3.65 (m, 1H), 3.64 -3.55 (m, 1H), 3.35-3.29 (m, 3H), 3.16 (t, *J* = 11.5 Hz, 1H), 2.80-2.76 (m, 2H), 2.59 (d, *J* = 16.0 Hz, 2H), 2.05-2.01 (m, 1H), 1.93 -1.72 (m, 3H), 1.59-1.54 (m, 3H).

### Example 21: Synthesis of (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z551)

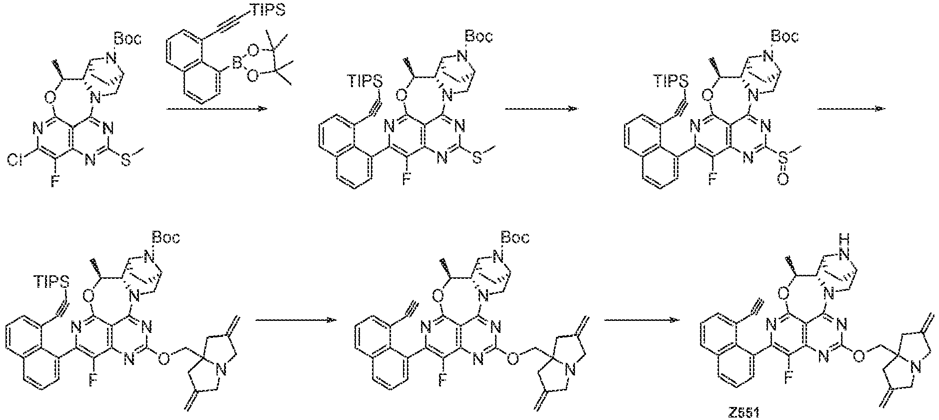

Step 1: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (50 mg, 0.10 mmol), triisopropyl((8-(4,4,5,5-tetramethyl-1,3,2-dioxaborolane-2-yl)naphthalen-1-yl)ethynyl)silane (113 mg, 0.25 mmol), potassium phosphate (66 mg, 0.30 mmol), and methanesulfonic acidbutyl bis(1-adamantyl)phosphinepalladium(II) (11 mg, 0.015 mmol) were dissolved in tetrahydrofuran (5 mL) and water (1 mL). The reaction mixture was stirred at 80°C under microwave for 40 minutes. Upon completion of the reaction (monitored by LCMS), the reaction solution was concentrated under reduced pressure, and the crude product was purified by silica gel column chromatography (dichloromethane:methanol = 20:1) to obtain tert-butyl (5S,5aS,6S,9R)-1-fluoro-5-methyl-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (52 mg, yield: 66%). ES-API: [M+H]⁺= 754.3.

Step 2: Tert-butyl (5S,5aS,6S,9R)-1-fluoro-5-methyl-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (52 mg, 0.07 mmol) was dissolved in dichloromethane (5 mL), and under an ice-water bath, m-chloroperoxybenzoic acid (14 mg, 0.08 mmol) was added. The reaction was proceeded at room temperature for 30 minutes. Upon completion of the reaction (monitored by LCMS), the reaction solution was diluted with dichloromethane (10 mL), washed with saturated sodium bisulfite solution (10 mLX2), saturated brine (10 mLX3), dried over anhydrous sodium sulfate, filtered, and concentrated to obtain the crude product, tert-butyl (5S,5aS,6S,9R)-1-fluoro-5-methyl-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (53 mg, yield: 100%). ES-API: [M+H]⁺= 770.4.

Step 3: (2,6-Dimethyltetrahydro-1H-pyrrolizin-7a(5H)-yl)methanol (23 mg, 0.14 mmol) was dissolved in anhydrous tetrahydrofuran (5 mL), and under an ice-water bath, sodium hydride (8 mg, 0.21 mmol) was added. The reaction was proceeded under an ice-water bath for 30 minutes. A tetrahydrofuran solution (5 mL) of tert-butyl (5S,5aS,6S,9R)-1-fluoro-5-methyl-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (53 mg, 0.07 mmol) was added dropwise to the above reaction mixture. The reaction was proceeded at room temperature for 30 minutes. Upon completion of the reaction (monitored by LCMS), the reaction solution was diluted with ethyl acetate (20 mL), washed with saturated ammonium chloride solution (15 mLX2), saturated brine (15 mLX3), dried over anhydrous sodium sulfate, filtered, and concentrated to obtain the crude product, tert-butyl (5S,5aS,6S,9R)-12-((2,6-dimethylenetetrahydro-1H-pyrrolizin-7a(5H)-yl)methoxy)-1-fluoro-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (60 mg, yield: 100%). ES-API: [M+H]⁺= 871.5.

Step 4: Cesium fluoride (103 mg, 0.7 mmol) was added to a N,N-dimethylformamide (3 mL) solution of tert-butyl (5S,5aS,6S,9R)-12-((2,6-dimethylenetetrahydro-1H-pyrrolizin-7a(5H)-yl)methoxy)-1-fluoro-5-methyl-2-(8-((triisopropylsilyl)ethynyl)naphthalen-1-yl)-Sa,6,7,8,9,10-hexahydro-5H-4-oxa-3,10a,11,13,14-pentaaza-6,9-methanonaphtho[1,8-ab]heptalene-14-carboxylate (60 mg, 0.07 mmol). The reaction mixture was stirred at room temperature for 2 hours. Upon completion of the reaction (monitored by LCMS), the reaction solution was diluted with ethyl acetate (20 mL), washed with water (15 mLX2), saturated brine (15 mLX3), dried over anhydrous sodium sulfate, filtered, and concentrated to obtain the crude product, tert-butyl (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (48 mg, yield: 100%). ES-API: [M+H]⁺= 715.3.

Step 5: Under an ice-water bath, a 4 M hydrochloric acid-dioxane solution (5 mL) was slowly added to a solution of tert-butyl (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-axa-3,10a,11,13,14-pentaaza-6,9-methanonaphtho[1,8-ab]heptalene-14-carboxylate (48 mg, 0.07 mmol) in acetonitrile (2 mL). The reaction mixture was stirred at 0°C for 0.5 hours. Upon completion of the reaction (monitored by LCMS), the reaction solution was concentrated under reduced pressure until dry, and the residue was purified by preparative HPLC (ammonium bicarbonate method 2) to obtain a pale yellow solid, (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-axa-3,10a,11,13,14-pentaaza-6,9-methanonaphtho[1,8-ab]heptalene (Z551, 11 mg, yield: 26 %). ES-API: [M+H]⁺ = 615.3. ¹H NMR (500 MHz, CD₃OD) δ 8.10 -8.00 (m, 2H), 7.79 -7.70 (m, 1H), 7.67 -7.57 (m, 2H), 7.53-7.46 (m, 2H), 5.38-5.34 (m, 1H), 5.02 (d, *J* = 8.0 Hz, 4H), 4.57 -4.48 (m, 1H), 4.32 - 4.26 (m, 2H), 4.07 (d, *J* = 9.0 Hz, 1H), 3.76 (d, *J* = 15.0 Hz, 2H), 3.67 (d, *J* = 5.5 Hz, 1H), 3.59 (d, *J* =5.0 Hz, 1H), 3.36-3.31 (m, 2H), 3.21 -3.12 (m, 2H), 2.79-2.75 (m, 2H), 2.59 (d, *J* = 16.5 Hz, 2H), 2.10 -1.99 (m, 1H), 1.90 -1.73 (m, 3H), 1.63-1.54 (m, 3H).

### Example 22: Synthesis of 5-ethynyl-6-fluoro-4-((5S,5aS,6S,9R)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol (Z552)

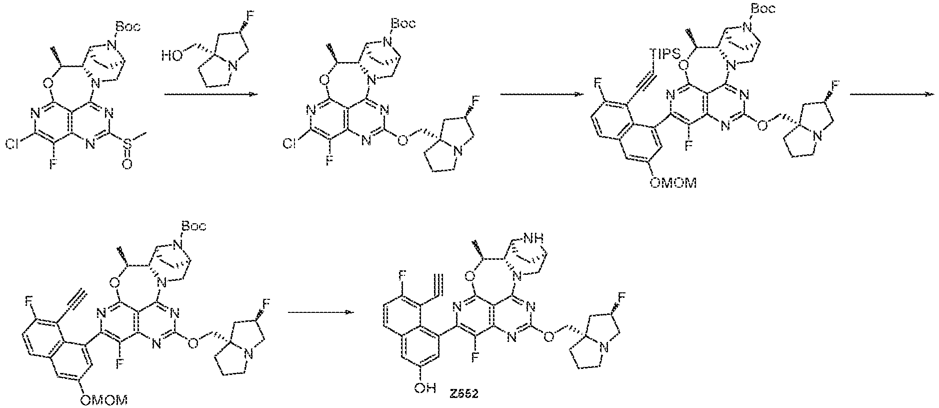

Step 1: At 0°C, ((2R,7aS)-2-fluorotetrahydro-1H-pyrrolizin-7a(5H)-yl)methanol (115 mg, 0.72 mmol) was added to a tetrahydrofuran solution of sodium hydride (60% dispersed in oil) (58 mg, 1.44 mmol). The mixture was stirred at room temperature for 10 minutes, and cooled to 0°C, with a tetrahydrofuran solution (5 mL) of tert-butyl (5S,5aS,6S,9R)-2-chloro-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (200 mg, 0.40 mmol) added. The reaction mixture was stirred at 0°C and reacted for 1 hour. The reaction mixture was quenched with saturated ammonium chloride solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene-14-carboxylate(90 mg, yield: 38.0%).

Step 2: To tert-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene-14-carboxylate (90.0 mg, 0.15 mmol) and ((2-fluoro-6-(methoxymethoxy)-8-(4,4,5,5-tetramethyl-1,3,2-dioxaborolane-2-yl)naphthalen-1-yl)ethynyl)triisopropylsilane (154 mg, 0.30 mmol) in tetrahydrofuran (6 mL) and water (1.0 mL), potassium phosphate (68 mg, 0.30 mmol) and palladium(II) bis(1,1'-biphenyl-2-yl)butylphosphine methanesulfonate (30 mg) were added. Nitrogen was bubbled through and the mixture reacted at 60°C for 40 minutes. Upon completion of the reaction, ethyl acetate (100 mL) was added, washed successively with water (10 mL) and saturated sodium chloride solution (10 mL), dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (95 mg, yield: 67.4%).

Step 3: To a N,N-dimethylformamide solution (3.0 mL) of tert-butyl (5S,5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (95 mg, 0.101 mmol), cesium fluoride (70 mg, 0.50 mmol) was added. The reaction mixture was stirred at room temperature and reacted for 0.5 hours. Upon completion of the reaction, the mixture was filtered and purified by preparative HPLC (trifluoroacetic acid method) to obtain tert-butyl (5S,5aS,6S,9R)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (70 mg, yield: 87.5%).

Step 4: A reaction solution of tert-butyl (5S,5aS,6S,9R)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (70 mg, 0.089 mmol), acetonitrile (2.0 mL), and 4 M hydrochloric acid/dioxane solution (0.500 mL, 2.000 mmol) reacted at 0°C for 1 hour. The pH was adjusted to 8 with saturated sodium bicarbonate. The mixuter was concentrated, filtered, and purified by preparative HPLC (formic acid method 1) to obtain 5-ethynyl-6-fluoro-4-((5S,5aS,6S,9R)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol (Z552, 40mg, yield: 67.8%, formate), yellow solid. ES-API:[M+1]⁺=643.2. ¹H NMR (400 MHz, DMSO-d6) δ 7.14 -6.99 (m, 1H), 6.62 -6.30 (m, 3H), 4.81 -4.55 (m, 2H), 3.89 -3.67 (m, 3H), 3.40 (d, *J* = 8.5 Hz, 1H), 3.13 -2.77 (m, 5H), 2.67 -2.47 (m, 3H), 1.89 -1.02 (m, 10H), 0.81 (t, *J* = 5.7 Hz, 3H).

### Example 23: Synthesis of 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-6-fluoronaphthalen-Z-ol (Z553)

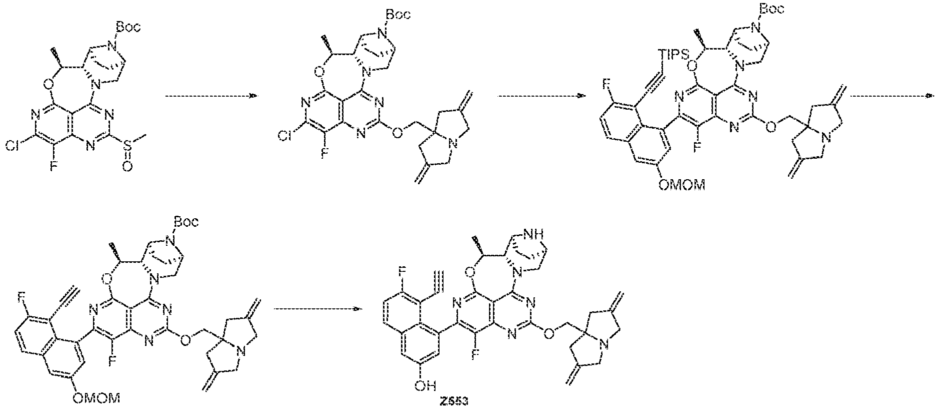

Step 1: At 0°C, (2,6-dimethyltetrahydro-1H-pyrrolo[5H]azepin-7a-yl)methanol (95 mg, 0.57 mmol) was added to a tetrahydrofuran solution of sodium hydride (60% dispersed in oil) (30 mg, 0.76 mmol). The reaction mixture was stirred at room temperature and reacted for 10 minutes. Cooled to 0°C, a tetrahydrofuran solution (5 mL) of tert-butyl (5S,5aS,6S,9R)-2-chloro-1-fluoro-5-methyl-12-(methylsulfmyl)-5a,6,7,8,9,10-hexahydro-5H-4-oxa-3,10a, 11,13,14-pentaaza-6,9-methanonaphtho[1,8-ab]heptalene-14-carboxylate (190 mg, 0.38 mmol) was added. The reaction mixture was stirred at 0°C and reacted for 1 hour, quenched with saturated sodium bicarbonate solution and extracted with ethyl acetate (20mL×3). The organic phase was washed with saturated brine (20 mLX1), dried over anhydrous sodium sulfate, filtered, and concentrated. The crude product was purified by silica gel column chromatography (0-5% methanol/dichloromethane) to obtain the compound tert-butyl (5S,5aS,6S,9R)-2-chloro-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (80 mg, yield: 35.0%). ES-API: [M+H]⁺=599.2.

Step 2: To tert-butyl (5S,5aS,6S,9R)-2-chloro-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (80.0 mg, 0.08 mmol) and ((2-fluoro-6-(methoxymethoxy)-8-(4,4,5,5-tetramethyl-1,3,2-dioxaborolane-2-yl)naphthalen-1-yl)ethynyl)triisopropylsilane (61 mg, 0.12 mmol) in tetrahydrofuran (6 mL) and water (1.0 mL), potassium phosphate (51 mg, 0.24 mmol), methanesulfonic acid butylbis(1-adamantyl)phosphine (2-amino-1,1'-biphenyl-2-yl)palladium(II) (20 mg) were added. Nitrogen was bubbled through, and the mixture reacted at 60°C for 40 minutes. Upon completion of the reaction, the mixture was extracted with ethyl acetate (30 mLX2) and saturated brine (10 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain the compound tert-butyl (5S,5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-axa-3,10a,11,13,14-pentaaza-6,9-methanonaphtho[1,8-ab]heptalene-14-carboxylate (80 mg, yield 63%). ES-API: [M+H]⁺=949.3.

Step 3: To a N,N-dimethylformamide solution (3.0 mL) of tert-butyl (5S,5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (80 mg, 0.084 mmol), cesium fluoride (40 mg, 0.26 mmol) was added and the mixture was stirred at room temperature and reacted for 0.5 hours. Upon completion of the reaction, the mixture was filtered, and purified by preparative HPLC (trifluoroacetic acid method) to obtain tert-butyl (5S,5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5-methyl-Sa,6,7,8,9,10-hexahydro-5H-4-oxa-3,10a,11,13,14-pentaaza-6,9-methanonaphtho[1,8-ab]heptalene-14-carboxylate (60 mg, yield: 90.2%). ES-API: [M+H]⁺=793.1.

Step 4: A reaction solution of tert-butyl (5S,5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1 -yl)-1-fluoro-5-methyl-5a,6,7,8,9, 10-hexahydro-5H-4-oxa-3, 10a, 11, 13,14-pentaaza-6,9-methanonaphtho[1,8-ab]heptalene-14-carboxylate (60 mg, 0.076 mmol), acetonitrile (2.0 mL), and 4 M hydrochloric acid/dioxane solution (0.500 mL, 2.000 mmol) reacted at 0°C for 1 hour. The pH was adjusted to 8 with saturated sodium bicarbonate, and the solution was concentrated, filtered, and purified by preparative HPLC (formic acid method 1) to obtain 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-6-fluoronaphthalen-2-ol (Z553, 37mg, yield: 75.1%, formate). ES-API:[M+1]⁺=649.2. ¹H NMR (400 MHz, CD₃OD) δ 8.41 (s, 1H), 7.85 (dt, J = 9.1, 6.2 Hz, 1H), 7.41 -7.06 (m, 3H), 5.46 (d, J = 12.0 Hz, 1H), 5.15 (s, 4H), 4.68 -4.43 (m, 3H), 4.28 -3.85 (m, 5H), 3.74 -3.38 (m, 3H), 3.33 (d, J = 9.7 Hz, 1H), 3.00 -2.84 (m, 2H), 2.72 (d, J = 16.6 Hz, 2H), 2.02 (dd, J = 56.2, 44.8 Hz, 4H), 1.59 (t, J = 6.2 Hz, 3H).

### Example 24: Synthesis of (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z590)

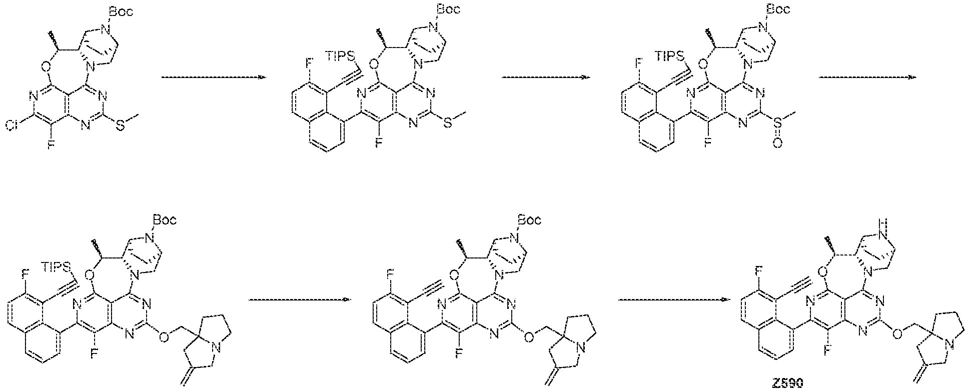

Step 1: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (150 mg, 0.311 mmol), ((2-fluoro-8-(4,4,5,5-tetramethyl-1,3,2-dioxaborolane-2-yl)naphthalen-1-yl)ethynyl)triisopropylsilane (211 mg, 0.466 mmol), potassium phosphate (132 mg, 0.62 mmol) and methanesulfonic acid butylbis(1-adamantyl)phosphine (2-amino-1,1'-biphenyl-2-yl)palladium(II) (23 mg, 0.031 mmol) were dissolved in tetrahydrofuran (6.0 mL) and water (1.5 mL). The reaction mixture was heated and stirred at 80°C under microwave for 40 minutes. Upon completion of the reaction (monitored by LCMS), the reaction mixture was concentrated under reduced pressure, and the crude product was purified by silica gel column chromatography (dichloromethane:methanol=20:1) to obtain tert-butyl (SS,SaS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (290 mg, crude product). ES-API: [M+H]⁺= 772.3.

Step 2: Tert-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (290 mg, crude product) was dissolved in dichloromethane (10.0 mL). Under an ice water bath, m-chloroperbenzoic acid (70 mg, 0.406 mmol) was added. The reaction was proceeded under an ice water bath for 30 minutes. Upon completion of the reaction (monitored by LCMS), the reaction mixture was diluted with dichloromethane (20 mL) and washed with saturated sodium bisulfite solution (15 mLX2) and saturated brine (15 mLX3), dried over anhydrous sodium sulfate, filtered, and concentrated to obtain the crude product tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (280 mg, crude product). ES-API: [M+H]⁺= 788.3.

Step 3: (2,6-Dimethyltetrahydro-1H-pyrrolo[1,2-c]pyrimidin-7a(5H)-yl)methanol (25 mg, 0.163 mmol) was dissolved in anhydrous tetrahydrofuran (5.0 mL). Under an ice water bath, sodium hydride (13 mg, 0.326 mmol) was added and the mixture reacted for 30 minutes under an ice water bath. A solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (41.0 mg, 0.052 mmol) in tetrahydrofuran (2.0 mL) was added dropwise to the above reaction mixture and reacted at room temperature for 30 minutes. Upon completion of the reaction (monitored by LCMS), the reaction mixture was diluted with ethyl acetate (80 mL), washed with saturated ammonium chloride solution (15 mLX2) and saturated brine (60 mLX3), dried over anhydrous sodium sulfate, filtered, and concentrated to obtain the crude product tert-butyl (SS,SaS,6S,9R)-1-fluoro-2-(7-fluom-8-((triisopropylsilyl)ethynyl)naphthalen-1-yl)-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (65 mg, crude product). ES-API: [M+H]⁺= 877.4.

Step 4: Cesium fluoride (160 mg, 1.053 mmol) was added to a solution of tert-butyl (SS,SaS,6S,9R)-1-fluoro-2-(7-fluoro-8-((triisopropylsilyl)ethynyl)naphthalen-1-yl)-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (65 mg, crude product) in N,N-dimethylformamide (3.0 mL). The reaction mixture was stirred at room temperature for 2 hours. Upon completion of the reaction (monitored by LCMS), the reaction mixture was diluted with ethyl acetate (60 mL), washed with water (15 mLX2), saturated brine (60 mLX3), dried over anhydrous sodium sulfate, filtered, and concentrated to obtain tert-butyl (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (70 mg, crude product). ES-API: [M+H]⁺= 721.2. Step 5: Under an ice water bath, 4 M hydrochloric acid-dioxane solution (2 mL) was slowly added to a solution of tert-butyl (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (70 mg, crude product) in acetonitrile (5.0 mL). The reaction mixture was stirred at 0°C for 0.5 hours. Upon completion of the reaction (monitored by LCMS), the reaction mixture was concentrated to dryness under reduced pressure, and the residue was purified by preparative HPLC (ammonium bicarbonate method 2) to obtain a pale yellow solid (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z590, 5.05mg, total yield for 5 steps: 15.6%). ES-API: [M+H]⁺= 621.3.

### Example 25: Synthesis of (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z591)

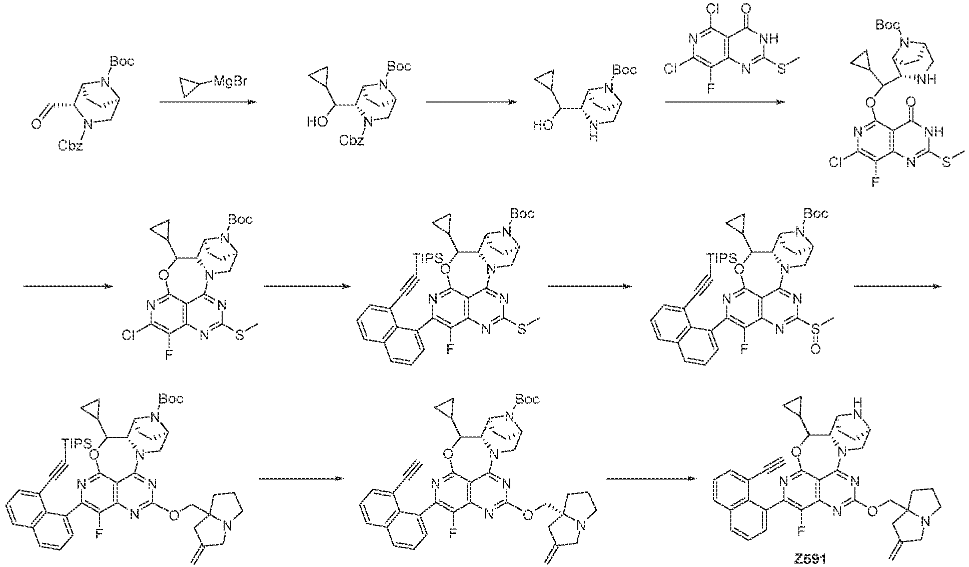

Step 1: 3-Benzyl 8-(tert-butyl) (1S,2S,5R)-2-formyl-3,8-diazabicyclo[3.2.1]octane-3,8-dicarboxylate (1100 mg, 2.938 mmol) was dissolved in dry tetrahydrofuran (15 mL) under nitrogen protection and cooled to -78°C. A 1M cyclopropylmagnesium bromide solution in tetrahydrofuran (4.4 mL) was added dropwise. The reaction was proceeded for 2 hours at -78°C. The reaction mixture was quenched with saturated ammonium chloride solution (5 mL), extracted with ethyl acetate, dried over anhydrous sodium sulfate, concentrated, and purified by silica gel column chromatography (ethyl acetate/petroleum ether = 1:2) to obtain 3-benzyl 8-(tert-butyl) (1S,2S,5R)-2-((S)-cyclopropyl(hydroxy)methyl)-3,8-diazabicyclo[3.2.1]octane-3,8-dicarboxylate (1200 mg, yield: 98.07%). ES-API:[M-18+H]⁺=399.3.

Step 2: 3-Benzyl 8-(tert-butyl)(1S,2S,5R)-2-((S)-cyclopropyl(hydroxy)methyl)-3,8-diazabicyclo[3.2.1]octane-3,8-dicarboxylate ester (1223 mg, 2.938 mmol) was dissolved in methanol (20 mL) and methanolic ammonia (10.0 mL, 7M). Palladium on carbon (10%) (500 mg) was added. The reaction was proceeded under a hydrogen atmosphere at room temperature for 2 hours. The reaction mixture was filtered, concentrated, and the crude product was purified by preparative thin-layer chromatography (dichloromethane/methanol = 10:1) to obtain tert-butyl (1S,2S,5R)-2-((S)-cyclopropyl(hydroxy)methyl)-3,8-diazabicyclo[3.2.1]octane-8-carboxylate (916 mg, crude product).

Step 3: At 0°C, to a suspension of sodium hydride (60% dispersed in oil) (370 mg, 9.25 mmol) in tetrahydrofuran (20 mL), tert-butyl (1S,2S,5R)-2-((S)-cyclopropyl(hydroxy)methyl)-3,8-diazabicyclo[3.2.1]octane-8-carboxylate (516 mg, 1.827 mmol) was added. The mixture was stirred at this temperature for 30 minutes. 5,7-Dichloro-8-fluoro-2-(methylthio)pyrido[4,3-d]pyrimidin-4(3H)-one (1025 mg, 3.66 mmol) was added and the mixture was heated at room temperature and stirred for 1 hour. Upon completion of the reaction, the mixture was quenched with water and extracted with dichloromethane/methanol (10:1, 80 mLX3). The organic phase was dried over anhydrous sodium sulfate and concentrated to obtain the product tert-butyl (1S,2S,5R)-2-((S)-((7-chloro-8-fluoro-2-(methylthio)-4-oxo-3,4-dihydropyrido[4,3-d]pyrimidin-5-yl)oxy)(cyclopropyl)methyl)-3,8-diazabicyclo[3.2.1]octane-8-caiboxylate (700 mg, yield: 72.82%). ES-API:[M+H]⁺=526.0.

Step 4: To a dichloromethane (40 mL) solution of tert-butyl (1S,2S,5R)-2-((S)-((7-chloro-8-fluoro-2-(methylthio)-4-oxo-3,4-dihydropyrido[4,3-d]pyrimidin-5-yl)oxy)(cyclopropyl)methyl)-3,8-diazabicyclo[3.2.1]octane-8-carboxylate (700 mg, 1.331 mmol) and N,N-diisopropylethylenediamine (516 mg, 3.992 mmol), 1-Propylphosphonic anhydride (50% in ethyl acetate) (1.27 g, 3.992 mmol) was added, and the mixture was stirred at room temperature for 3 hours. The reaction mixture was quenched with water and extracted with dichloromethane (100 mLX2). The organic phase was dried over anhydrous sodium sulfate, filtered, and concentrated. The crude product was purified by silica gel column chromatography [petroleum ether: ethyl acetate = 100:0~70:30, (v/v)] to obtain tert-butyl (5S,5aS,6S,9R)-2-chloro-5-cyclopropyl-1-fluoro-12-(methylthio)-5a,6,7,8,9,10-hexahydro-5H-4-oxo-3,10a,11,13,14-pentaaza-6,9-methanonaphtho[1,8-ab]heptalene-14-carboxylate (200 mg, yield: 29.58%). ES-API:[M+H]⁺=508.2.

Step 5: To tert-butyl (5S,5aS,6S,9R)-2-chloro-5-cyclopropyl-1-fluoro-12-(methylthio)-5a,6,7,8,9,10-hexahydro-5H-4-oxo-3,10a,11,13,14-pentaaza-6,9-methanonaphtho[1,8-ab]heptalene-14-carboxylate (80.0 mg, 0.157 mmol) and triisopropyl((8-(4,4,5,5-tetramethyl-1,3,2-dioxaborolane-2-yl)naphth-1-yl)ethynyl)silane (150 mg, 0.345 mmol) were dissolved in tetrahydrofuran (9 mL) and water (2 mL), potassium phosphate (130 mg, 0.613 mmol) and methanesulfonic acid butylbis(1-adamantyl)phosphinepalladium(II) (20 mg, 0.027 mmol) were added. Bubbled with nitrogen for 3 minutes, the mixture reacted at 80°C under microwave for 40 minutes. After completion of the reaction, the mixture was extracted with ethyl acetate (80 mLX2) and saturated brine (60 mL). The combined ethyl acetate phase was dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-5-cyclopropyl-1-fluoro-12-(methylthio)-2-(8-((triisopropylsilyl)ethynyl)naphth-1-yl)-5a,6,7,8,9,10-hexahydro-5H-4-oxo-3,10a,11,13,14-pentaaza-6,9-methanonaphtho[1,8-ab]heptalene-14-carboxylate (150 mg, crude product). ES-API: [M+H]⁺=780.3.

Step 6: At 0°C, m-chloroperbenzoic acid (45 mg, 0.262 mmol) was added to a solution of tert-butyl (5S,5aS,6S,9R)-5-cyclopropyl-1-fluoro-12-(methylthio)-2-(8-((triisopropylsilyl)ethynyl)naphth-1-yl)-5a,6,7,8,9,10-hexahydro-5H-4-oxo-3,10a,11,13,14-pentaaza-6,9-methanonaphtho[1,8-ab]heptalene-14-carboxylate (150 mg, crude product) in dichloromethane (15 mL). The mixture was stirred in an ice-water bath for 1 hour. After diluted with dichloromethane (80 mL), the reaction mixture was washed with 10% sodium hypochlorite solution (10.0 mLX2). The organic phase was dried over anhydrous sodium sulfate and concentrated to obtain the crude product tert-butyl (5S,5aS,6S,9R)-5-cyclopropyl-1-fluoro-12-(methylsulfonyl)-2-(8-((triisopropylsilyl)ethynyl)naphth-1-yl)-5a,6,7,8,9,10-hexahydro-5H-4-oxo-3,10a,11,13,14-pentaaza-6,9-methanonaphtho[1,8-ab]heptalene-14-carboxylate (160 g, crude product). ES-API: [M+H]⁺=796.3.

Step 7: At 0°C, to a tetrahydrofuran solution of sodium hydride (60% dispersion in oil) (40.0 mg, 0.978 mmol), (2-methyltetrahydro-1H-pyrrolizin-7a(5H)-yl)methanol (75 mg, 0.489 mmol) was added. The reaction mixture was stirred at room temperature and reacted for 30 minutes. Cooled to 0°C, a solution of tert-butyl (5S,5aS,6S,9R)-5-cyclopropyl-1-fluoro-12-(methylsulfonyl)-2-(8-((triisopropylsilyl)ethynyl)naphth-1-yl)-5a,6,7,8,9,10-hexahydro-5H-4-oxo-3, 10a, 11, 13,14-pentaaza-6,9-methanonaphtho[1,8-ab]heptalene-14-carboxylate (100 mg, 0.20 mmol) in tetrahydrofuran (5 mL) was added. The mixture was stirred at 0°C and reacted for 1 hour. The reaction mixture was quenched with saturated sodium bicarbonate solution and extracted with ethyl acetate. The organic phase was washed with saturated brine (80 mL), dried over anhydrous sodium sulfate, filtered, and concentrated. The crude product was purified by silica gel column chromatography (0-5% methanol/dichloromethane) to obtain tert-butyl (5S,5aS,6S,9R)-5-cyclopropyl-1-fluoro-12-((2-methyltetrahydro-1H-pyrrolizin-7a(5H)-yl)methoxy)-2-(8-((triisopropylsilyl)ethynyl)naphth-1-yl)-Sa,6,7,8,9,10-hexahydro-5H-4-oxo-3,10a,11,13,14-pentaaza-6,9-methanonaphtho[1,8-ab]heptalene-14-carboxylate (190 mg, crude product). ES-API: [M+H]⁺=885.5.

Step 8: To a solution of tert-butyl (5S,5aS,6S,9R)-5-cyclopropyl-1-fluoro-12-((2-methyltetrahydro-1H-pyrrolizin-7a(5H)-yl)methoxy)-2-(8-((triisopropylsilyl)ethynyl)naphth-1-yl)-5a,6,7,8,9,10-hexahydro-5H-4-oxo-3,10a,11,13,14-pentaaza-6,9-methanonaphtho[1,8-ab]heptalene-14-carboxylate (190 mg, crude product) in N,N-dimethylformamide (10.0 mL), cesium fluoride (480 mg, 3.158 mmol) was added. The reaction mixture was stirred at room temperature and reacted for 1.5 hours. The organic phase was washed with saturated brine (80 mLX5), dried over anhydrous sodium sulfate, filtered, and concentrated to obtain tert-butyl (5S,5aS,6S,9R)-5-cyclopropyl-2-(8-ethynyl naphth-1-yl)-1-fluoro-12-((2-methyltetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]heptalene-14-carboxylate (210 mg, crude product). ES-API: [M+H]⁺=729.3.

Step 9: A reaction solution of tert-butyl (5S,5aS,6S,9R)-5-cyclopropyl-2-(8-ethynyl naphth-1-yl)-1-fluoro-12-((2-methyltetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]heptalene-14-carboxylate (210 mg, crude product), acetonitrile (10.0 mL) and 4M hydrochloric acid/dioxane solution (2.0 mL, 8.000 mmol) reacted at 0°C for 1 hour. The reaction mixture was concentrated, and the crude product was purified by preparative HPLC (formic acid method 1) to obtain (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z591, formate, 22mg, yield: 19.3%), yellow solid. ES-API:[M+1]⁺=629.3.

### Example 26: Synthesis of (5S,5aS,6S,9R)-5-ethyl-2-(8-ethylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z592)

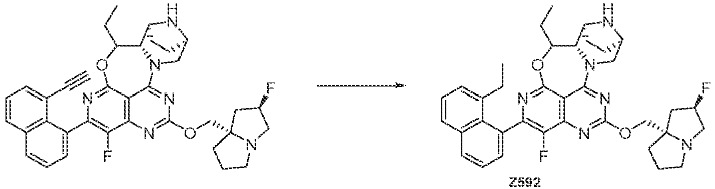

Step 1: (5S,5aS,6S,9R)-5-ethyl-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (20 mg, 2.07mmol) was dissolved in methanol (5.0 mL). Palladium on carbon (10%) (10 mg) was added. The reaction was proceeded under hydrogen for 1 hour. The reaction mixture was filtered and concentrated. The crude product was prepared by preparative HPLC (formic acid method 1) to obtain (5S,5aS,6S,9R)-5-ethyl-2-(8-ethylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z592, 12mg, yield: 61.5%, formate), yellow solid. ES-API: [M+H]⁺=627.3. ¹H NMR (400 MHz, CD₃OD) δ 8.03 (d, *J* = 8.2 Hz, 1H), 7.85 (d, *J* = 8.1 Hz, 1H), 7.63 -7.25 (m, 4H), 5.52 (d, *J* = 51.2 Hz, 2H), 4.69 -4.26 (m, 4H), 4.11 -3.59 (m, 5H), 3.42 -3.23 (m, 2H), 2.79 -1.72 (m, 14H), 1.15 (t, *J* = 7.2 Hz, 3H), 0.95 (dt, *J* = 45.5, 7.5 Hz, 3H).

### Example 27: Synthesis of (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z593)

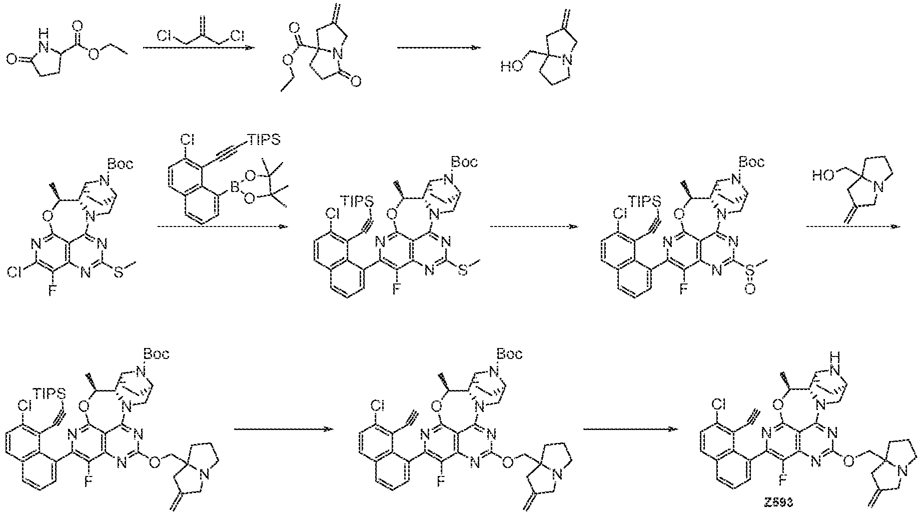

Step 1: Ethyl 5-oxopyrrolidine-2-carboxylate (10g, 63.69 mmol) and 3-chloro-2-(chloromethyl)prop-1-ene (12.64 g, 101.9 mmol) were dissolved in tetrahydrofuran (50 mL), at - 40°C, 1M bis(trimethylsilyl)amine lithium (127 mL) was added. The reaction was proceeded at room temperature for 16 hours. The reaction mixture was quenched with an ammonium chloride solution (50 mL), extracted with ethyl acetate (50 mL × 1), washed with brine, dried over anhydrous sodium sulfate, concentrated, and purified by column chromatography (petroleum ether/ethyl acetate = 1/1) to obtain the product ethyl 2-methylene-5-oxotetrahydro-1H-pyrrolizine-7a(5H)-carboxylate (4.2 g, yield: 31.56%). ES-API: [M+H]⁺= 210.1.

Step 2: Lithium aluminum hydride (1.52 g, 40.15 mmol) was dissolved in tetrahydrofuran (20 mL), cooled to 0°C, and a solution of ethyl 2-methylene-5-oxotetrahydro-1H-pyrrolizine-7a(5H)-carboxylate (4.2 g, 20.07 mmol) in tetrahydrofuran (20 mL) was added dropwise. The reaction solution was heated to 70°C and reacted for 3 hours, with water (1.5 mL),15% sodium hydroxide solution (1.5 mL) added, quenched with water (4.5 mL), filtered, and concentrated to obtain the crude product (2-methylenetetrahydro-1H-pyrrolizin-7a(5H)-yl)methanol (2.5 g, yield: 81.29%). ES-API: [M+H]⁺= 154.1.

Step 3: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.21 mmol), (2-chloro-8-(4,4,5,5-tetramethyl-1,3,2-dioxaborolane-2-yl)naphthalen-1-yl)ethyl(triisopropyl)silane (3589 mg, 0.84 mmol), potassium phosphate (132 mg, 0.62 mmol) and palladium(II) bis(2-amino-1,1'-biphenyl)di-tert-butylphosphine methanesulfonate (23 mg, 0.031 mmol) were dissolved in tetrahydrofuran (5 mL) and water (1 mL). The reaction mixture was stirred at 80°C under microwave for 40 minutes. After completion of the reaction (monitored by LCMS), the reaction mixture was concentrated under reduced pressure, and the crude product was purified by silica gel column chromatography (dichloromethane: methanol = 20:1) to obtain tert-butyl (5S,5aS,6S,9R)-2-(7-chloro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (98 mg, yield: 60 %). ES-API: [M+H]⁺= 788.3

Step 4: Tert-butyl (5S,5aS,6S,9R)-2-(7-chloro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (98 mg, 0.12 mmol) was dissolved in dichloromethane (5 mL). Under an ice-water bath, m-chloroperoxybenzoic acid (25 mg, 0.15 mmol) was added. The reaction was proceeded at room temperature for 30 minutes. Upon completion of the reaction (monitored by LCMS), the reaction mixture was diluted with dichloromethane (20 mL), washed with saturated sodium sulfite solution (15 mL × 2) and saturated brine (15 mL × 3), dried over anhydrous sodium sulfate, filtered, and concentrated to obtain the crude product tert-butyl (5S,5aS,6S,9R)-2-(7-chloro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (60 mg, yield: 60%). ES-API: [M+H]⁺= 804.3.

Step 5: (2-methylenetetrahydro-1H-pyrrolizin-7a(5H)-yl)methanol (34 mg, 0.22 mmol) was dissolved in anhydrous tetrahydrofuran (5 mL). Under an ice-water bath, sodium hydride (11 mg, 0.45 mmol) was added. The reaction was proceeded for 30 minutes under an ice-water bath. A solution of tert-butyl (5S,5aS,6S,9R)-2-(7-chloro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (60 mg, 0.075 mmol) in tetrahydrofuran (2 mL) was added dropwise to the reaction mixture. The reaction was proceeded at room temperature for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous sodium sulfate, filtered, and concentrated to obtain the crude product tert-butyl (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (66 mg, yield: 100%). ES-API: [M+H]⁺= 893.3

Step 6: Cesium fluoride (112 mg, 0.74 mmol) was added to a solution of tert-butyl (SS,SaS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (66 mg, 0.074 mmol) in N,N-dimethylformamide (3 mL). The reaction mixture was stirred at room temperature for 2 hours. Upon completion of the reaction (monitored by LCMS), the reaction mixture was diluted with ethyl acetate (20 mL), washed with water (15 mL × 2), and saturated brine (15 mL × 3), dried over anhydrous sodium sulfate, filtered, and concentrated to obtain the crude product tert-butyl (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a, 11,13,14-pentaaza-6,9-methanonaphtho[1,8-ab]heptalene-14-carboxylate (55 mg, yield: 100%). ES-API: [M+H]⁺= 737.2.

Step 7: Under an ice-water bath, a 4 M hydrochloric acid-dioxane solution (5 mL) was slowly added to a solution of tert-butyl (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (55 mg, 0.074 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) to obtain a pale yellow solid (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a, 11,13,14-pentaaza-6,9-methanonaphtho[1,8-ab]heptalene (Z593, 6 mg, yield: 12 %). ES-API: [M+H]⁺= 637.2.

### Example 28: Synthesis of (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptalene (Z594)

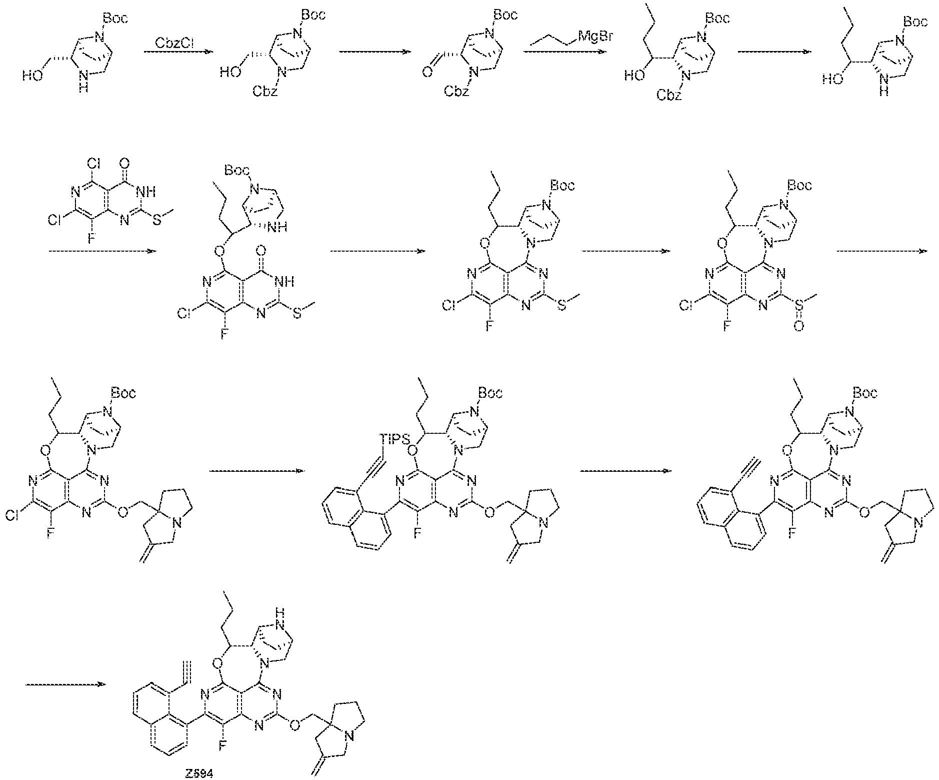

Step 1: tert-butyl (1S,2S,5R)-2-(hydroxymethyl)-3,8-diazabicyclo[3.2.1]octane-8-carboxylate (3.0 g, 12.40 mmol) was dissolved in a mixture of ethyl acetate (30 ml) and water (10 ml). Benzyl chloroformate (2.54 g, 14.88 mmol) and sodium bicarbonate (2.98 g, 32.0 mmol) were added, and the mixture was stirred overnight at room temperature. After adding water (50 ml) and extracting with ethyl acetate (100 ml × 3), the organic phase was washed with saturated brine (20 ml), dried over anhydrous sodium sulfate, concentrated, and purified by column chromatography (petroleum ether/ethyl acetate = 2:1) to obtain 3-benzyl 8-(tert-butyl) (1S,2S,5R)-2-(hydroxymethyl)-3,8-diazabicyclo[3.2.1]octane-3,8-dicarboxylate (4.35 g, yield: 93.3%).

Step 2: Under nitrogen protection, at -78°C, a solution of dimethyl sulfoxide (156 mg, 1.99 mmol) in anhydrous dichloromethane (5.0 ml) was slowly added to a solution of oxalyl chloride (223 mg, 1.75 mmol) in anhydrous dichloromethane (10 ml). The mixture was stirred for 15 minutes at this temperature, then a solution of 3-benzyl 8-(tert-butyl) (1S,2S,5R)-2-(hydroxymethyl)-3,8-diazabicyclo[3.2.1]octane-3,8-dicarboxylate (300 mg, 0.80 mmol) in anhydrous dichloromethane (5.0 ml) was slowly added dropwise. The reaction was proceeded for 40 minutes at -78°C, then triethylamine (646 mg, 6.38 mmol) was slowly added. The reaction was proceeded for 1 hour at - 78°C. The reaction mixture was quenched with saturated ammonium chloride solution (20 ml), extracted with ethyl acetate, dried over anhydrous sodium sulfate, and concentrated to obtain the crude product 3-benzyl 8-(tert-butyl) (1S,2S,5R)-2-formyl-3,8-diazabicyclo[3.2.1]octane-3,8-dicarboxylate (270 mg, yield: 90.2%).

Step 3: 3-benzyl 8-(tert-butyl) (1S,2S,5R)-2-formyl-3,8-diazabicyclo[3.2.1]octane-3,8-dicarboxylate (270 mg, 0.72 mmol) was dissolved in dry tetrahydrofuran (5 mL). Under nitrogen protection The mixture was cooled to -78°C, with a 2M solution of propyl magnesium bromide in ether (0.54 mL) added dropwise. The reaction was proceeded for 2 hours at -78°C, then quenched with saturated ammonium chloride solution (10 ml), extracted with ethyl acetate, dried over anhydrous sodium sulfate, concentrated, and purified by thin-layer chromatography (ethyl acetate/petroleum ether = 1:3) to obtain 3-benzyl 8-(tert-butyl) (1S,2S,5R)-2-((S)-1-hydroxybutyl)-3,8-diazabicyclo[3.2.1]octane-3,8-dicarboxylate (180 mg, yield: 59.8%).

Step 4: 3-benzyl 8-(tert-butyl) (1S,2S,5R)-2-((S)-1-hydroxybutyl)-3,8-diazabicyclo[3.2.1]octane-3,8-dicarboxylate (180 mg, 0.43 mmol) was dissolved in methanol (4 mL), palladium on carbon (10%) (30 mg) was added. The reaction was proceeded under hydrogen at room temperature for 2 hours. After filtration and concentration, tert-butyl (1S,2S,SR)-2-(1-hydroxybutyl)-3,8-diazabicyclo[3.2.1]octane-8-carboxylate (90 mg, yield: 73.8%) was obtained.

Step 5: At 0°C, tert-butyl (1S,2S,5R)-2-((S)-1-hydroxybutyl)-3,8-diazabicyclo[3.2.1]octane-8-carboxylate (90 mg, 0.32 mmol) was added to a suspension of sodium hydride (60% dispersed in oil) (40 mg, 0.95 mmol) in tetrahydrofuran (5.0 mL) and the mixture was stirred for 10 minutes at this temperature. 5,7-Dichloro-8-fluoro-2-(methylthio)pyrido[4,3-d]pyrimidin-4(3H)-one (112 mg, 0.40 mmol) was added, and the mixture was heated and stirred at 40°C for 1 hour. Upon completion of the reaction, the reaction mixture was quenched with water and extracted with dichloromethane/methanol (10:1, 25 mL × 3). The organic phase was dried over anhydrous sodium sulfate, concentrated to obtain tert-butyl (1S,2S,SR)-2-((S)-1-((7-chloro-8-fluoro-2-(methylthio)-4-oxo-3,4-dihydropyrido[4,3-d]pyrimidin-5-yl)oxy)butyl)-3,8-diazabicyclo[3.2.1]octane-8-carboxylate (120 mg, yield: 71.0%).

Step 6: To a solution of tert-butyl (1S,2S,5R)-2-((S)-1-((7-chloro-8-fluoro-2-(methylthio)-4-oxo-3,4-dihydropyrido[4,3-d]pyrimidin-5-yl)oxy)butyl)-3,8-diazabicyclo[3.2.1]octane-8-carboxylate (120 mg, 0.23 mmol) and N,N-diisopropylethylenediamine (85 mg, 0.69 mmol) in dichloromethane (10 mL), tripyrrolidinylphosphonium hexafluorophosphate (215 mg, 0.46 mmol) was added. The mixture was stirred at room temperature for 16 hours. The reaction mixture was quenched with water and extracted with dichloromethane. The organic phase was dried over anhydrous sodium sulfate, filtered, concentrated, and purified by silica gel column chromatography (petroleum ether/ethyl acetate = 3:1) to obtain tert-butyl (5S,5aS,6S,9R)-2-chloro-1-fluoro-5-propyl-12-(methylthio)-5a,6,7,8,9,10-hexahydro-5H-4-oxy-3,10a,11,13,14-pentaaza-6,9-methanonaphtho[1,8-ab]heptalene-14-carboxylate (80 mg, yield: 70.1%).

Step 7: At 0°C, m-chloroperoxybenzoic acid (36 mg, 0.20 mmol) was added to a solution of tert-butyl (5S,5aS,6S,9R)-2-chloro-1-fluoro-5-propyl-12-(methylsulfonyl)-5a,6,7,8,9,10-hexahydro-5H-4-oxy-3,10a,11,13,14-pentaaza-6,9-methanonaphtho[1,8-ab]heptalene-14-carboxylate (80 mg, 0.16 mmol) in dichloromethane (15 mL). The reaction mixture was stirred at room temperature for 1 hour. The reaction was diluted with dichloromethane (30 mL), washed with 10% sodium hydroxide solution (5.0 mL × 2). The organic phase was dried over anhydrous sodium sulfate and concentrated to obtain the crude product tert-butyl (5S,5aS,6S,9R)-2-chloro-1-fluoro-12-(methylsulfinyl)-5-propyl-5a,6,7,8,9,10-hexahydro-5H-4-oxa-3,10a,11,13,14-pentaaza-6,9-methanonaphtho[1,8-ab]heptalene-14-carboxylate (78 mg, yield: 95.0%).

Step 8: At 0°C, (2-methylenetetrahydro-1H-pyrrolizin-7a(5H)-yl)methanol (43 mg, 0.28 mmol) was added to a tetrahydrofuran solution of sodium hydride (60% dispersed in oil) (15 mg, 0.36 mmol). The reaction mixture was stirred at room temperature and reacted for 10 minutes. The reaction was cooled to 0°C, and a tetrahydrofuran solution (5 mL) of tert-butyl (5S,5aS,6S,9R)-2-chloro-1-fluoro-12-(methylsulfinyl)-5-propyl-5a,6,7,8,9,10-hexahydro-5H-4-oxa-3,10a,11,13,14-pentaaza-6,9-methanonaphtho[1,8-ab]heptalene-14-carboxylate (80 mg, 0.16 mmol) was added. The reaction mixture was stirred at 0°C and reacted for 1 hour. The reaction mixture was quenched with saturated bicarbonate solution and extracted with ethyl acetate. The organic phase was washed with saturated brine, dried over anhydrous sodium sulfate, filtered, concentrated, and the crude product was purified by silica gel column chromatography (0-5% methanol/dichloromethane) to obtain tert-butyl (5S,5aS,6S,9R)-2-chloro-1-fluoro-12-((2-methylenetetrahydro-1H-pynolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptalene-14-carboxylate (40 mg, yield: 41.1%). ES-API: [M+H]⁺=607.3.

Step 9: To a solution of tert-butyl (5S,5aS,6S,9R)-2-chloro-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptalene-14-carboxylate (40.0 mg, 0.066 mmol) and triisopropyl[(8-(4,4,5,5-tetramethyl-1,3,2-dioxaborolane-2-yl)naphthalen-1-yl)ethynyl]silane (56 mg, 0.13 mmol) in tetrahydrofuran (6 mL) and water (1.0 mL), potassium phosphate (34 mg, 0.16 mmol) and palladium(II) [bis(di-tert-butylphosphino)ferrocene]msulfonate [2-aminobiphenyl] (15 mg) were added. The reaction was bubbled with nitrogen and heated at 60°C for 2 hours. Upon completion of the reaction, the reaction mixture was extracted with ethyl acetate (30 mL × 2) and saturated brine (10 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-1-fluoro-12-((2-methylenetetrahydro-1H-pyrrolizin-7a(5H)-yl)methoxy)-5-propyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (38 mg, yield: 61.3%).

Step 10: To a solution of tert-butyl (5S,5aS,6S,9R)-1-fluoro-12-((2-methylenetetrahydro-1H-pyrrolizin-7a(5H)-yl)methoxy)-5-propyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (38 mg, 0.043 mmol) in N,N-dimethylformamide (3.0 mL), cesium fluoride (40 mg, 0.26 mmol) was added and the mixture was stirred at room temperature and reacted for 0.5 hours. Upon completion of the reaction, the reaction mixture was filtered and purified by preparative HPLC (trifluoroacetic acid method) to obtain tert-butyl (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptalene-14-carboxylate (25 mg, yield: 79.0%).

Step 11: A reaction solution of tert-butyl (5S,5aS,6S,9R)-2-(8-ethynylnaphthalen-1-yl)-1-fluoro-12-((2-methylenetetrahydro-1H-pyrrolizin-7a(5H-yl)methoxy)-5-propyl-5a,6,7,8,9,10-hexahydro-5H-4-oxa-3,10a,11,13,14-pentaaza-6,9-methanonaphtho[1,8-ab]heptalene-14-carboxylate (25 mg, 0.034 mmol) in acetonitrile (2.0 mL) and 4 M hydrochloric acid/dioxane solution (0.500 mL, 2.000 mmol) reacted at 0°C for 1 hour. The reaction mixture was concentrated, and the crude product was purified by preparative HPLC (formic acid method 1) to obtain (SS,SaS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptalene (Z594, 12mg, yield: 55.6%, formate), yellow solid. ES-API: [M+H]⁺=631.3.

### Example 29: Synthesis of (5S,5aS,6S,9R)-5-ethyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ablheptalene (Z595)

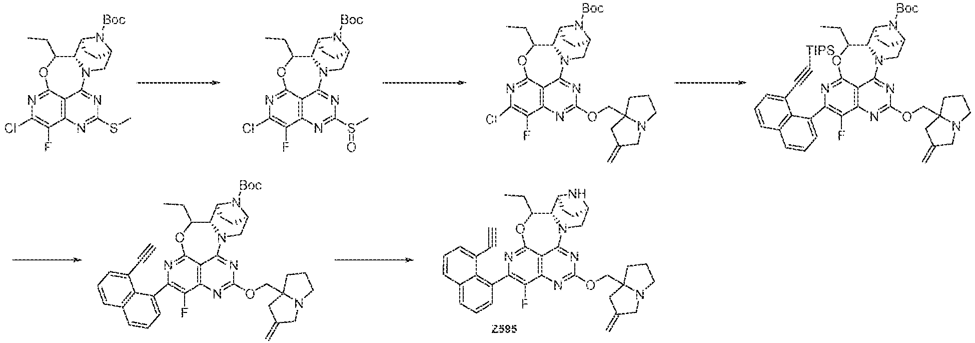

Step 1: At 0°C, m-chloroperoxybenzoic acid (61 mg, 0.30 mmol) was added to a solution of tert-butyl (5S,5aS,6S,9R)-2-chloro-5-ethyl-1-fluoro-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.20 mmol) in dichloromethane (15 mL). The mixture was stirred at room temperature for 1 hour. The reaction mixture was diluted with dichloromethane (50 mL) and washed with 10% sodium hydroxide solution (5.0 mL × 2). The organic phase was dried over anhydrous sodium sulfate and concentrated to obtain a crude product of tert-butyl (5S,5aS,6S,9R)-2-chloro-5-ethyl-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, yield: 94.8%).

Step 2: At 0°C, (2-methylene-4-hydro-1H-pyrrolizine-7a(5H)-yl)methanol (52 mg, 0.34 mmol) was added to a solution of sodium hydride (60% dispersed in oil) (20 mg, 0.51 mmol) in tetrahydrofuran. The mixture was stirred at room temperature and reacted for 10 minutes. With the mixture cooled to 0°C, a solution of tert-butyl (5S,5aS,6S,9R)-2-chloro-5-ethyl-1-fluoro-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (100 mg, 0.19 mmol) in tetrahydrofuran (5 mL) was added. The reaction mixture was stirred at 0°C and reacted for 1 hour. The reaction mixture was quenched with saturated sodium bicarbonate solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-chloro-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (40mg, yield: 35.0%).

Step 3: To tert-butyl (5S,5aS,6S,9R)-2-chloro-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (40.0mg,0.067mmol) and triisopropyl((8-(4,4,5,5-tetramethyl-1,3,2-dioxaborolane-2-yl)naphthalene-1-yl)ethynyl)silane (56mg,0.13mmol) in a mixture of tetrahydrofuran (6 mL) and water (1.0 mL) were added to potassium phosphate (34 mg, 0.16mmol) and palladium(II) 2-amino-1,1'-biphenyl-2-yl bis[1,1'-biphenyl]-4-yl phosphine (15 mg). The reaction was bubbled with nitrogen and heated at 60°C for 2 hours. Upon completion of the reaction, the reaction mixture was extracted with ethyl acetate (30 mL × 2) and saturated brine (10 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-1-fluoro-12-((2-methylene-tetrahydro-1H-pyrrolizine-7a(5H)-yl)methoxy)-5-ethyl-2-(8-((triisopropylsilyl)ethynyl)naphthalene-1-yl)-5a,6,7,8,9,10-hexahydro-5H-4-oxa-3,10a,11,13,14-pentaaza-6,9-methanonaphtho[1,8-ab]heptalene-14-carboxylate (38mg, yield: 64.4%).

Step 4: To a solution of tert-butyl (5S,5aS,6S,9R)-1-fluoro-12-((2-methylene-tetrahydro-1H-pyrrolizine-7a(5H)-yl)methoxy)-5-ethyl-2-(8-((triisopropylsilyl)ethynyl)naphthalene-1-yl)-5a,6,7,8,9,10-hexahydro-5H-4-oxa-3,10a,11,13,14-pentaaza-6,9-methanonaphtho[1,8-ab]heptalene-14-carboxylate (38 mg, 0.043 mmol) in N,N-dimethylformamide (3.0 mL), cesium fluoride (40 mg, 0.26 mmol) was added. The reaction mixture was stirred at room temperature and reacted for 0.5 hours. Upon completion of the reaction, the mixture was filtered and purified by preparative HPLC (trifluoroacetic acid method) to obtain tert-butyl (5S,5aS,6S,9R)-2-(8-ethynyl naphthalene-1-yl)-1-fluoro-12-((2-methylene-tetrahydro-1H-pyrrolizine-7a(5H)-yl)methoxy)-5-ethyl-5a,6,7,8,9,10-hexahydro-5H-4-oxa-3,10a,11,13,14-pentaaza-6,9-methanonaphtho[1,8-ab]heptalene-14-carboxylate (25 mg, yield: 79.0%).

Step 5: A solution of tert-butyl (5S,5aS,6S,9R)-2-(8-ethynyl naphthalene-1-yl)-1-fluoro-12-((2-methylene-tetrahydro-1H-pyrrolizine-7a(5H)-yl)methoxy)-5-ethyl-5a,6,7,8,9,10-hexahydro-5H-4-oxa-3,10a,11,13,14-pentaaza-6,9-methanonaphtho[1,8-ab]heptalene-14-carboxylate (25 mg, 0.035 mmol) in acetonitrile (2.0 mL) and 4M hydrochloric acid/dioxane solution (0.500 mL, 2.000 mmol) was prepared. The reaction solution reacted at 0°C for 1 hour. After concentration, the crude product was purified by preparative HPLC (formic acid method 1) to obtain (5S,5aS,6S,9R)-5-ethyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z595, 12mg, yield: 55.6%, formate), yellow solid. ES-API: [M+H]⁺=617.3. ¹H NMR (400 MHz, CD₃OD) δ 8.07 (dd, *J* = 17.3, 9.4 Hz, 2H), 7.83 -7.43 (m, 4H), 5.37 (d, *J* = 84.9 Hz, 3H), 4.78 -4.50 (m, 2H), 4.32 (dd, *J* = 38.6, 24.6 Hz, 3H), 3.76 (dd, *J* = 64.3, 10.2 Hz, 4H), 3.54 -3.11 (m, 2H), 3.02 (d, *J* = 16.6 Hz, 1H), 2.76 (d, *J* = 16.4 Hz, 1H), 2.45 -1.70 (m, 10H), 1.16 (t, *J* = 6.5 Hz, 3H).

### Example 30: Synthesis of (5S,5aS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z596)

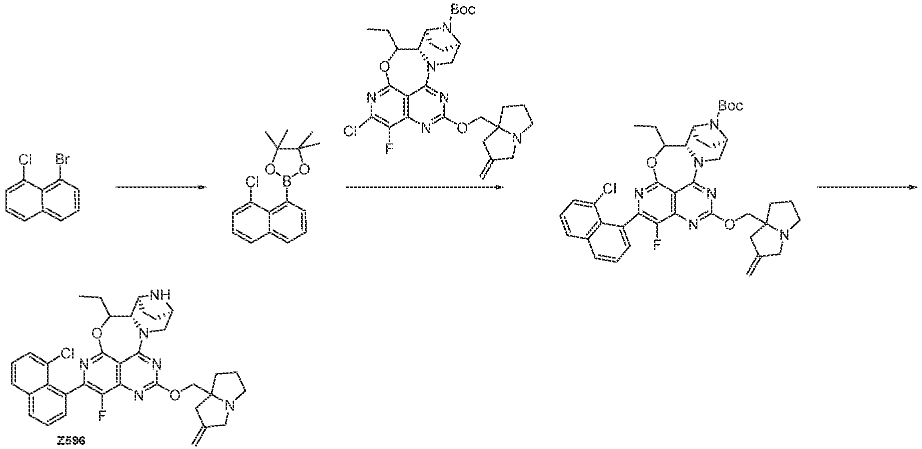

Step 1: 1-Bromo-8-chloronaphthalene (500 mg, 2.07 mmol) was dissolved in anhydrous dioxane (5.0 mL), and then pinacol boronate (631 mg, 2.48 mmol), 1,1'-bis(diphenylphosphino)ferrocene palladium(II) dichloride (151 mg, 0.207 mmol), and potassium acetate (282 mg, 4.14 mmol) were added. The mixture reacted at 90°C for 12 hours under nitrogen protection. The reaction mixture was filtered, concentrated, and the crude product was purified by silica gel column chromatography (0-20% ethyl acetate/petroleum ether) to obtain 2-(8-chloronaphthalen-1-yl)-4,4,5,5-tetramethyl-1,3,2-dioxaborolane (300 mg, yield: 50.2%).

Step 2: To a solution of tert-butyl (5S,5aS,6S,9R)-2-chloro-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (40.0 mg, 0.067 mmol) and 2-(8-chloronaphthalen-1-yl)-4,4,5,5-tetramethyl-1,3,2-dioxaborolane (56 mg, 0.13 mmol) tetrahydrofuran (6 mL) and water (1.0 mL), potassium phosphate (34 mg, 0.16 mmol) and methanesulfonic acid [n-butyl-di(1-adamantyl)phosphine](2-amino-1,1'-biphenyl-2-yl)palladium(II) (15 mg) were added. The mixture was bubbled with nitrogen and reacted at 60°C for 2 hours. Upon completion of the reaction, the mixture was extracted with ethyl acetate (30 mLX2) and saturated brine (10 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain tert-butyl (SS,SaS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (38 mg, yield: 64.4%).

Step 3: A reaction solution of tert-butyl (5S,5aS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (38 mg, 0.052 mmol) in acetonitrile (2.0 mL) and 4M hydrochloric acid/dioxane solution (0.500 mL, 2.000 mmol) reacted at 0°C for 1 hour, then concentrated, and the crude product was purified by preparative HPLC (formic acid method 1) to obtain (5S,5aS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z596, 12mg, yield: 36.8%, formate), yellow solid. ES-API: [M+H]⁺=627.3. ¹H NMR (400 MHz, CD₃OD) δ 8.13 (d, *J* = 8.0 Hz, 1H), 8.00 (d, *J* = 7.6 Hz, 1H), 7.82 -7.40 (m, 4H), 5.49 (d, *J* = 13.2 Hz, 1H), 5.28 (s, 2H), 4.57-4.37 (m, 4H), 4.16 -3.59 (m, 4H), 3.52 -3.13 (m, 2H), 3.06 (d, *J* = 16.3 Hz, 1H), 2.78 (d, *J* = 16.0 Hz, 1H), 2.40 (s, 1H), 2.45 -1.70 (m, 10H), 1.16 (t, *J* = 6.5 Hz, 3H).

### Example 31: Synthesis of (5S,5aS,6S,9R)-2-(&ethynyl-7-fluoronaphthalen-1-yl)-1-fluoro-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z597)

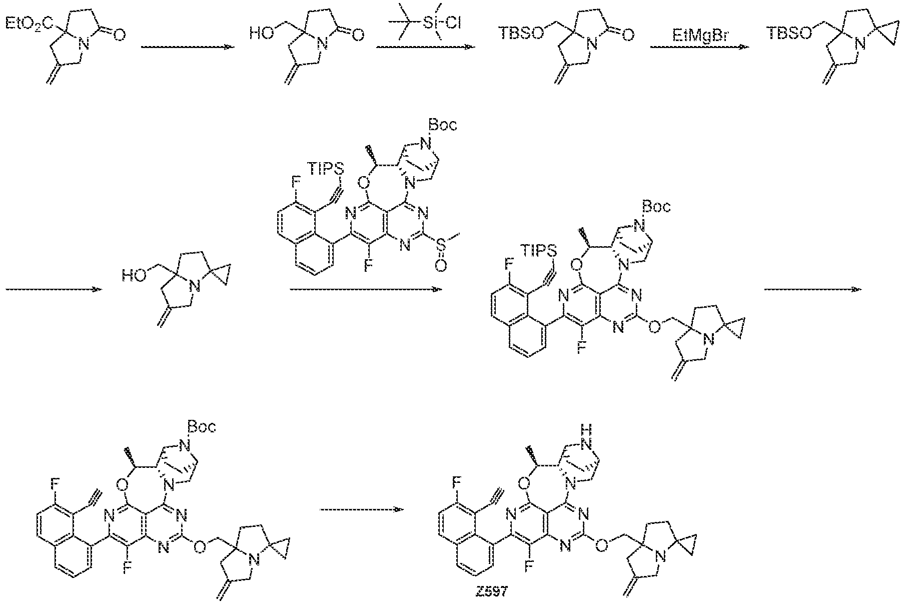

Step 1: Under nitrogen protection, ethyl 2-methylene-5-oxotetrahydro-1H-pyrrolizine-7a(5H)-carboxylate (1.5 g, 7.17 mmol) and tetrahydrofuran (15 mL) were added to a round-bottom flask. Cooled to 0°C, lithium borohydride solution (9 mL, 18 mmol, 2M in tetrahydrofuran) was added dropwise to the above solution. After the addition was complete, the mixture was stirred for 30 minutes at 0°C. The mixture was stirred at room temperature for another 20 minutes, cooled to 0°C, and 2M hydrochloric acid (10mL) was added dropwise until no more bubbles formed. 2M sodium hydroxide aqueous solution (30 mL) was added until the solution cleared. The solution was extracted with dichloromethane/isopropanol (3:1, 50 mLX3). The organic phase was dried over anhydrous sodium sulfate and concentrated to obtain 7a-(hydroxymethyl)-6-methylenehexahydro-3H-pyrrolizin-3-one (1.05 g, yield: 87%), a colorless oily substance. ES-API: [M+H]⁺=168.1.

Step 2: Sodium hydride (595 mg, 14.87 mmol) and tetrahydrofuran (20 mL) were added to a round-bottom flask. Under nitrogen protection, cooled to 0°C. Under stirred, a solution of 7a-(hydroxymethyl)-6-methylenehexahydro-3H-pyrrolizin-3-one (1g, 5.95 mmol) in tetrahydrofuran (2 mL) was added dropwise to the reaction mixture. The mixture was stirred at 0°C for 10 minutes, tert-butyldimethylchlorosilane (1.3 g, 8.93 mmol) was added and stirred at room temperature for 1 hour. Saturated ammonium chloride aqueous solution (50 mL) was added to the reaction mixture, and the mixture was extracted with ethyl acetate (50 mLX3). The organic phase was washed with saturated brine, dried over anhydrous sodium sulfate, concentrated, and the crude product was purified by flash silica gel column chromatography (dichloromethane/petroleum ether: 0-100%) to obtain 7a-(((tert-butyldimethylsilyl)oxy)methyl)-6-methylenehexahydro-3H-pyrrolizin-3-one (750 mg, yield: 44%), a colorless oil. ES-API: [M+H]⁺=282.3.

Step 3: Under nitrogen protection, tetraisopropoxytitanium (1.3g, 4.62 mmol) and tetrahydrofuran (10 mL) were added to a reaction flask. Cooled to -70°C, under stirred, ethylmagnesium bromide solution (9.24mL, 9.24 mmol, 1M in tetrahydrofuran) was added dropwise to the above reaction mixture. The mixtrure stirred at -70°C for 5 minutes. A solution of 7a-(((tertbutyldimethylsilyl)oxy)methyl)-6-methylenehexahydro-3H-pyrrolizin-3-one (650 mg, 2.31 mmol) in tetrahydrofuran (3 mL) was added dropwise, stirred for 20 minutes, then the ice water bath was removed. The mixture was stirred at room temperature for 2 hours. Saturated sodium bicarbonate aqueous solution (50 mL) was added to the reaction mixture, and extracted with ethyl acetate (50 mLX3). The organic phase was washed with saturated brine, dried over anhydrous sodium sulfate, concentrated, and the crude product was purified by flash silica gel column chromatography (ethyl acetate/petroleum ether: 0-50%) to obtain 7a'-(((tert-butyldimethylsilyl)oxy)methyl)-6'-methylenehexahydrospiro[cyclopropane-1,3'-pyrrolizine] (200 mg, yield: 29%), a yellow oily substance. ES-API: [M+H]⁺=294.3.

Step 4: Into the reaction flask, 7a'-(((tert-butyldimethylsilyl)oxy)methyl)-6'-methylene hexahydrospiro[cyclopropane-1,3'-pyrrolizine] (200 mg, 0.68 mmol), methanol (1 mL), and 4M hydrochloric acid/dioxane solution (3 mL) were added. The mixture was stirred at room temperature for one hour. The reaction mixture was then concentrated to obtain (6'-methylenetetrahydrospiro[cyclopropane-1,3'-pyrrolizin]-7a'(5'H)-yl)methanol (122 mg, crude product), which was then used directly in the next step without purification. ES-API: [M+H]⁺=180.2.

Step 5: (6'-Methylene tetrahydrospiro[cyclopropane-1,3'-pyrrolizine]-7a'(5'H)-methyl) methanol (34 mg, 0.19 mmol) was dissolved in anhydrous tetrahydrofuran (5 mL) under an ice-water bath. Sodium hydride (15 mg, 0.38 mmol) was added, and the mixture reacted for 30 minutes under the ice-water bath. A solution of tetrahydrofuran (2 mL) containing tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (50 mg, 0.063 mmol) was then added dropwise to the reaction mixture, which reacted at 40°C for 30 minutes. Upon completion of the reaction (monitored by LCMS), the reaction mixture was diluted with ethyl acetate (20 mL), washed with saturated ammonium chloride solution (15 mLX2) and saturated brine (15 mLX3), dried over anhydrous sodium sulfate, filtered, and concentrated to obtain the crude product tert-butyl (SS,Sa5,6S,9R)-1-fluoro-2-(7-fluoro-8-((triisopropylsilyl)ethynyl)naphthalen-1-yl)-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (26 mg, yield: 46%). ES-API: [M+H]⁺= 903.4.

Step 6: Cesium fluoride (44 mg, 0.29 mmol) was added to a solution of N,N-dimethylformamide (1.5 mL) containing tert-butyl (5S,5aS,6S,9R)-1-fluoro-2-(7-fluoro-8-((triisopropylsilyl)ethynyl)naphthalen-1-yl)-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (26 mg, 0.029 mmol). The reaction mixture was stirred at room temperature for two hours. Upon completion of the reaction (monitored by LCMS), the reaction mixture was diluted with ethyl acetate (20 mL), washed with water (15 mLX2) and saturated brine (15 mLX3), dried over anhydrous sodium sulfate, filtered, and concentrated to obtain the crude product tert-butyl (5S,5aS,6S,9R)-2-(8-ethynyl-7-fluoronaphthalen-1-yl)-1-fluoro-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (22 mg, yield: 100%). ES-API: [M+H]⁺= 747.3.

Step 7: Under an ice-water bath, a 4M hydrochloric acid-dioxane solution (3 mL) was slowly added to a solution of acetonitrile (2 mL) containing tert-butyl (5S,5aS,6S,9R)-2-(8-ethynyl-7-fluoronaphthalen-1-yl)-1-fluoro-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (22 mg, 0.029 mmol). The reaction mixture was stirred at 0°C for 0.5 hour. Upon completion of the reaction (monitored by LCMS), the reaction mixture was concentrated under reduced pressure until dry, and the residue was purified by preparative HPLC (Ammonium bicarbonate method 2) to obtain a pale yellow solid, (5 S,5aS,6S,9R)-2-(8-ethynyl-7-fluoronaphthalen-1 -yl)-1 -fluoro-5-methyl-12-((6'-methylenetetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z597, 5 mg, yield: 26 %). ES-API: [M+H]⁺= 647.3. ¹H NMR (400 MHz, CD₃OD) δ 8.13 -8.03 (m, 2H), 7.68 - 7.54 (m, 2H), 7.49 -7.36 (m, 1H), 5.42 -5.33 (m, 1H), 4.97 (d, *J* = 5.2 Hz, 2H), 4.57-4.51 (m, 1H), 4.45 -4.31 (m, 2H), 4.10-4.05 (m, 1H), 3.68 (d, *J* = 5.6 Hz, 1H), 3.64 -3.58 (m, 2H), 3.54 (s, 1H), 3.49 (d, *J* = 4.0 Hz, 1H), 3.23 -3.14 (m, 1H), 2.84-2.79 (m, 1H), 2.63 (d, *J* = 15.8 Hz, 1H), 2.39 - 2.28 (m, 1H), 2.27 -2.16 (m, 1H), 2.13 -1.97 (m, 2H), 1.93 -1.70 (m, 3H), 1.61 -1.53 (m, 4H), 1.03 -0.95 (m, 1H), 0.95 -0.85 (m, 1H), 0.70-0.60 (m, 2H), 0.53-0.49 (m, 1H).

### Example 32: Synthesis of (5S,5aS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizinl-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z599)

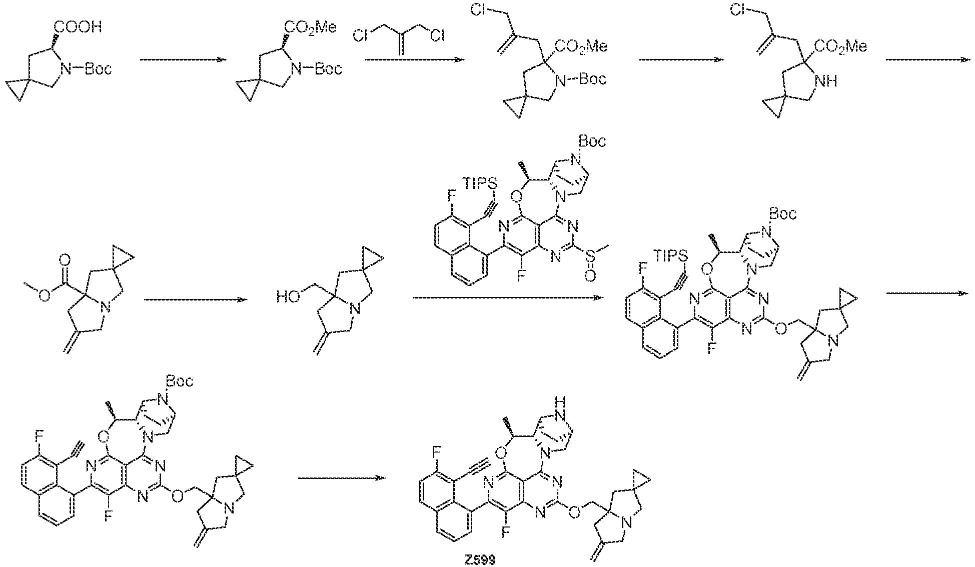

Step 1: In an ice-water bath, potassium carbonate (8.6 g, 62.25 mmol) and iodomethane (3.8 mL, 62.25 mmol) were added to a solution of (S)-5-(tert-butoxycarbonyl)-5-azaspiro[2.4]heptane-6-carboxylic acid (10 g, 41.5 mmol) in N,N-dimethylformamide (50 mL). The mixture was stirred at room temperature for 1.5 hours. Upon completion of the reaction, as monitored by LCMS, the reaction mixture was filtered and washed with ethyl acetate. The filtrate was then concentrated under reduced pressure to obtain 5-(tert-butyl)-6-methyl-(S)-5-azaspiro[2.4]heptane-5,6-dicarboxylate (11.2 g, yield: 71%). ES-API: [M-55]⁺= 200.1.

Step 2: Under nitrogen protection, 5-(tert-butyl)-6-methyl-(S)-5-azaspiro[2.4]heptane-5,6-dicarboxylate (5.0 g, 20 mmol) was dissolved in anhydrous tetrahydrofuran (50 mL), and the mixture was placed in a dry ice-acetone bath. A solution of bis(trimethylsilyl)aminolithium (30 mL, 30 mmol, 1 M in THF) was slowly added dropwise to the mixture, which was then stirred at the same temperature for 1 hour. A solution of 3-chloro-2-chloromethylpropene (12.4 g, 100 mmol) in anhydrous tetrahydrofuran (20 mL) was slowly added to the mixture via syringe. The reaction mixture was further stirred at -78 °C for 1 hour. Upon completion of the reaction, monitored by LCMS, the reaction mixture was quenched with saturated ammonium chloride solution (50 mL), extracted with ethyl acetate (50 mL × 3). The organic phase was washed with saturated brine (50 mL × 3), dried over anhydrous sodium sulfate, filtered, and concentrated. The crude product was purified by flash silica gel chromatography (petroleum ether: ethyl acetate = 20:1) to obtain 5-(tert-butyl)-6-methyl-(R)-6-(2-(chloromethyl)allyl)-5-azaspiro[2.4]heptane-5,6-dicarboxylate (5.2 g, yield: 75%). ES-API: [M-55]⁺= 288.1.

Step 3: At room temperature, a 4M solution of hydrochloric acid in dioxane (15 mL) was slowly added to a solution of 5-(tert-butyl)-6-methyl-(R)-6-(2-(chloromethyl)allyl)-5-azaspiro[2.4]heptane-5,6-dicarboxylate (5.2 g, 15 mmol) in acetonitrile (15 mL). The mixture was stirred at room temperature for 0.5 hours. Upon completion of the reaction, monitored by LCMS, the reaction mixture was concentrated under reduced pressure to obtain methyl (R)-6-(2-(chloromethyl)allyl)-5-azaspiro[2.4]heptane-6-carboxylate (3.65 g, yield: 100%). ES-API: [M+H]⁺= 244.1.

Step 4: At room temperature, sodium bicarbonate (6.3 g, 75 mmol) and potassium iodide (250 mg, 1.5 mmol) were added to a solution of methyl (R)-6-(2-(chloromethyl)allyl)-5-azaspiro[2.4]heptane-6-carboxylate (3.65 g, 15 mmol) in acetonitrile (50 mL). The mixture was stirred at room temperature for 1.5 hours. Upon completion of the reaction, monitored by LCMS, the reaction mixture was filtered and washed with acetonitrile. The filtrate was concentrated under reduced pressure and the crude product was purified by flash silica gel chromatography (petroleum ether: ethyl acetate = 15:1) to obtain methyl 6'-methylenedihydro-1'H,3'H-spiro[cyclopropane-1,2'-pyrrolizine]-7a'(5'H)-carboxylate (3.1 g, yield: 100%). ES-API: [M+H]⁺= 208.2.

Step 5: Under nitrogen protection, methyl 6'-methylenedihydro-1'H,3'H-spiro[cyclopropane-1,2'-pyrrolizine]-7a'(5'H)-carboxylate (3.1 g, 15 mmol) was dissolved in anhydrous tetrahydrofuran (50 mL), and the mixture was placed in an ice-water bath. A solution of lithium aluminium hydride (30 mL, 30 mmol, 1 M in THF) was slowly added dropwise to the mixture, which was then stirred at this temperature for 0.5 hours. Upon completion of the reaction, monitored by LCMS, sodium sulfate decahydrate (3.2 g) was added to quench the reaction, and the mixture was stirred at room temperature for 0.5 hours, then filtered. The filtrate was concentrated under reduced pressure to obtain (6'-methylenedihydro-1'H,3'H-spira[cyclopropane-1,2'-pyrrolizin]-7a'(5'H)-yl)methanol (2.7 g, yield: 100%). ES-API: [M+H]⁺= 180.3.

Step 6: (6'-Methylenedihydro-1'H,3'H-spiro[cyclopropane-1,2'-pyrrolizin]-7a'(5'H)-yl)methanol (34 mg, 0.19 mmol) was dissolved in anhydrous tetrahydrofuran (5 mL). Under an ice-water bath, sodium hydride (15 mg, 0.38 mmol) was added. The reaction was proceeded in the ice-water bath for 30 minutes. A solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (50 mg, 0.063 mmol) in tetrahydrofuran (2 mL) was slowly added to the reaction mixture, which was then reacted at 40°C for 30 minutes. Upon completion of the reaction, monitored by LCMS, the reaction mixture was diluted with ethyl acetate (20 mL), washed with saturated ammonium chloride solution (15 mL × 2), saturated brine (15 mL × 3), dried over anhydrous sodium sulfate, filtered, and concentrated to obtain a crude product of tert-butyl (5S,5aS,6S,9R)-1-fluoro-2-(7-fluoro-8-((triisopropylsilyl)ethynyl)naphthalen-1-yl)-5-methyl-12-((6'-methylenedihydro-1'H,3'H-spiro[cyclopropane-1,2'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (57 mg, yield: 100%). ES-API: [M+H]⁺= 903.4

Step 7: Cesium fluoride (94 mg, 0.62 mmol) was added to a solution of tert-butyl (SS,SaS,6S,9R)-1-fluoro-2-(7-fluoro-8-((triisopropylsilyl)ediynyl)naphthalen-1-yl)-5-methyl-12-((6'-methylenedihydro-1'H,3'H-spiro[cyclopropane-1,2'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (57 mg, 0.062 mmol) in N,N-dimethylformamide (1.5 mL). The reaction mixture was stirred at room temperature for 2 hours. Upon completion of the reaction, monitored by LCMS, the reaction mixture was diluted with ethyl acetate (20 mL), washed with water (15 mL × 2), saturated brine (15 mL × 3), dried over anhydrous sodium sulfate, filtered, and concentrated to obtain a crude product of tert-butyl (SS,SaS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (46 mg, yield: 100%). ES-API: [M+H]⁺= 747.3.

Step 8: Under an ice-water bath, a 4M solution of hydrochloric acid in dioxane (3 mL) was slowly added to a solution of tert-butyl (5S,5aS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizin]-7a'(5'H)-yl)methoxy)-5a,6,7,8,9,10-hexahydro-5H-4-oxa-3,10a, 11,13,14-pentaaza-6,9-methanonaphtho[1,8-ab]heptalene-14-carboxylate (46 mg, 0.062 mmol) in acetonitrile (2 mL). The reaction mixture was stirred at 0°C for 0.5 hours. Upon completion of the reaction, monitored by LCMS, the reaction mixture was concentrated to dryness, and the residue was purified by preparative HPLC (ammonium bicarbonate method 2) to obtain a pale yellow solid of (SS,SaS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizin]-7a'(5H)-yl)methoxy)-5a,6,7,8,9,10-hexahydro-5H-4-oxa-3,10a,11,13,14-pentaaza-6,9-methanonaphtho[1,8-ab]heptalene (Z599, 5 mg, yield: 12 %). ES-API: [M+H]⁺= 647.3.

### Example 33: Synthesis of (5S,5aS,6S,9R)-2-(&ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z600)

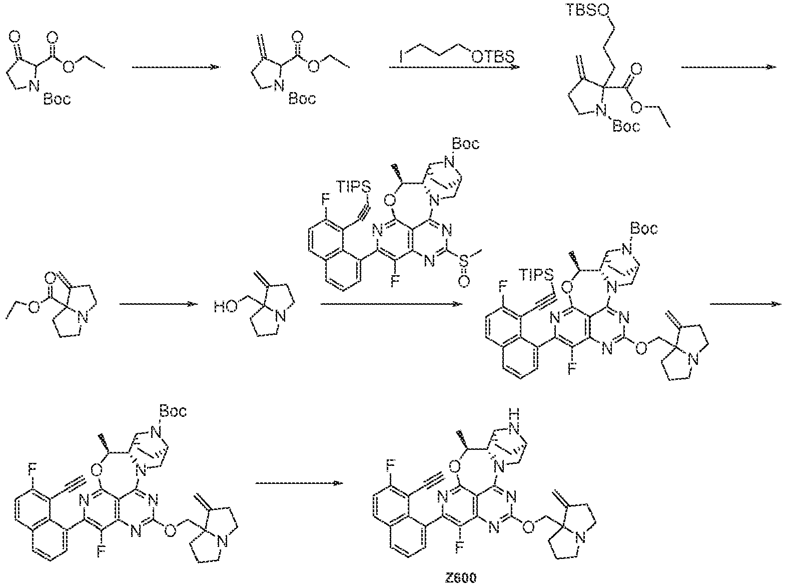

Step 1: Methyltriphenylphosphonium bromide (10.41 g, 29.15 mmol) was added to tetrahydrofuran (200 mL) and cooled to 0°C. Under a nitrogen atmosphere and at 0°C, a solution of potassium tert-butoxide in tetrahydrofuran (29.15 mL, 29.15 mmol, 1M) was added to the mixture. After the dropwise addition was complete, the reaction mixture was heated to room temperature and stirred for 1 hour. 1-(tert-butyl) 2-ethyl 3-oxopyrrolidine-1,2-dicarboxylate (3.0 g, 12.82 mmol) was dissolved in tetrahydrofuran (10 mL) and slowly added to the reaction mixture under a nitrogen atmosphere. After the addition was complete, the mixture was stirred at room temperature for 3 hours. Saturated ammonium chloride solution (30 mL) was added to the reaction mixture, and the mixture was extracted with ethyl acetate (100 mL × 3). The organic phase was washed with saturated brine (200 mL), dried over anhydrous sodium sulfate, concentrated, and the crude product was purified by flash silica gel column (ethyl acetate/petroleum ether: 0%-8%) to obtain 1-(tert-butyl)-2-ethyl-3-methylene pyrrolidine-1,2-dicarboxylate (2.0 g, yield: 61.6%), a colorless and transparent liquid. ES-API: [M-Boc+H]⁺=200.1.

Step 2: A mixture of 1-(tert-butyl)-2-ethyl-3-methylene pyrrolidine-1,2-dicarboxylate (0.5 g, 1.96 mmol), tert-butyl (3-iodopropoxy)dimethylsilane (3.0 g, 9.99 mmol), potassium carbonate (1.61 g, 11.61 mmol), andN,N-dimethylformamide (40 mL) was stirred overnight at room temperature under a nitrogen atmosphere. Water (100 mL) was added to the reaction mixture, and the mixture was extracted with ethyl acetate (100 mL × 4). The organic phase was washed with saturated brine (200 mL × 3), dried over anhydrous sodium sulfate, concentrated, and the crude product was purified by flash silica gel column (ethyl acetate/petroleum ether: 0-10%) to obtain 1-(tert-butyl)-2-ethyl-2-(3-((tert-butyldimethylsilyl)oxy)propyl)-3-methylene pyrrolidine-1,2-dicarboxylate (720 mg, yield: 86%), a colorless and transparent oily liquid. ES-API: [M-Boc+H]⁺=350.2.

Step 3: Under an ice-water bath, 1-(tert-butyl)-2-ethyl-2-(3-((tert-butyldimethylsilyl)oxy)propyl)-3-methylenepyrrolidine-1,2-dicarboxylate (720 mg, 1.682 mmol) was dissolved in dichloromethane (20 mL), and sulfonyl chloride (7.0 mL, 96.47 mmol) was added. The mixture was stirred overnight at room temperature. The solvent was evaporated under reduced pressure, and the crude product was treated with a mixed solvent (30 mL, dichloromethane: methanol = 10:1) and finally potassium carbonate (464 mg, 3.36 mmol) was added and stirred at room temperature for 1 hour. After filtration, the filtrate was evaporated under reduced pressure to obtain ethyl 1-methylenetetrahydro-1H-pyrrolizine-7a(5H)-carboxylate (190 mg, yield: 57%), a colorless and transparent liquid. ES-API: [M-Boc+H]⁺=196.1.

Step 4: Ethyl 1-methylenetetrahydro-1H-pyrrolizine-7a(5H)-carboxylate (190 mg, 0.969 mmol) was dissolved in tetrahydrofuran (10 mL) and cooled to 0°C. Under a nitrogen atmosphere, a solution of lithium aluminium hydride (2.0 mL, 2.0 mmol, 1M in tetrahydrofuran) was added to the solution. The reaction mixture was stirred at 0°C for 1 hour. Water (0.03 mL) was added to quench the reaction, followed by 15% sodium hydroxide solution (0.03 mL) and water (0.09 mL). Tetrahydrofuran (10 mL) was added, the mixture was filtered, and the filtrate was concentrated to obtain (1-methylenetetrahydro-1H-pyrrolizin-7a(5H)-yl)methanol (120 mg, yield: 80%), a colorless and transparent liquid. ES-API: [M +H]⁺=154.1.

Step 5: (1-Methylenetetcahydro-1H-pyrrolizin-7a(5H)-ylhnethanol (60 mg, 0.3896 mmol) was dissolved in anhydrous tetrahydrofuran (5 mL), and under an ice-water bath, sodium hydride (30 mg, 0.780 mmol) was added. The reaction was proceeded under the ice-water bath for 30 minutes. Then, a solution of tert-butyl (5S,5aS,6S,9R)-1-fluoro-2-(7-fluoro-8-((triisopropylsilyl)ethγnyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (50 mg, 0.063 mmol) in tetrahydrofuran (2 mL) was slowly added dropwise to the reaction mixture. The reaction was proceeded at 40°C for 30 minutes. Upon completion of the reaction, monitored by LCMS, the reaction mixture was diluted with ethyl acetate (50 mL), washed with saturated ammonium chloride solution (15 mL × 2), saturated brine (50 mL x 3), dried over anhydrous sodium sulfate, filtered, and the crude product was purified by silica gel column chromatography (dichloromethane: methanol = 15:1) to obtain tert-butyl (5S,5aS,6S,9R)-1-fluoro-2-(7-fluoro-8-((triisopropylsilyl)ethynyl)naphthalen-1-yl)-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (160 mg, crude product). ES-API: [M+H]⁺= 877.4.

Step 6: Cesium fluoride (96.0 mg, 0.63 mmol) was added to a N,N-dimethylformamide (5.0 mL) solution of tert-butyl (5S,5aS,6S,9R)-1-fluoro-2-(7-fluoro-8-((triisopropylsilyl)ethynyl)naphthalen-1-yl)-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (160 mg, crude product). The reaction mixture was stirred at room temperature for 2 hours. Upon completion of the reaction, monitored by LCMS, the reaction mixture was diluted with ethyl acetate (60 mL), washed with water (30 mL × 2), saturated brine (50 mL × 3), dried over anhydrous sodium sulfate, filtered, and concentrated to obtain a crude product of tert-butyl (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (200 mg, crude product). ES-API: [M+H]⁺= 721.3.

Step 7: Under an ice-water bath, a 4 M hydrochloric acid-dioxane solution (3 mL) was slowly added to a solution of tert-butyl (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (200 mg, crude product) in acetonitrile (5.0 mL). The reaction mixture was stirred at 0°C for 0.5 hours. Upon completion of the reaction, monitored by LCMS, the reaction mixture was concentrated to dryness, and the residue was purified by preparative HPLC (bicarbonate method 2) to obtain a pale yellow solid of (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z600, 6.0 mg, total yield for 3 steps: 2.4 %). ES-API: [M+H]⁺= 621.3. ¹H NMR (400 MHz, CD₃OD) δ 8.10 -8.05 (m, 2H), 7.68 - 7.52 (m, 2H), 7.46-7.39(m, 1H), 5.38-5.34(m, 1H), 5.10-5.08 (m, 2H), 4.59 -4.47 (m, 1H), 4.39 - 4.27 (m, 2H), 4.08-4.06(m, 1H), 3.80 -3.42 (m, 3H), 3.26 -3.06 (m, 3H), 2.89 -2.77 (m, 1H), 2.74 - 2.59 (m, 3H), 2.20-2.13(m, 1H), 2.10-2.00(m, 1H), 1.99 -1.70 (m, 6H), 1.58-1.55 (m, 3H).

### Example 34: Synthesis of (5S,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z601)

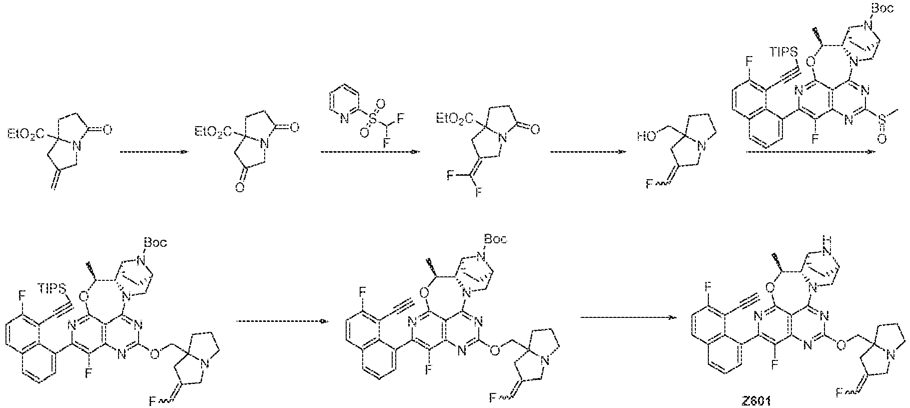

Step 1: Under nitrogen protection, ethyl 2-methylene-5-oxotetrahydro-1H-pyrrolizine-7a(5H)-carboxylate (2g, 9.57mmol), potassium osmate dihydrate (176mg, 0.48mmol), water (40mL), and tetrahydrofuran (20mL) were added to a round-bottomed flask and cooled to 0°C. Under stirring, sodium periodate (6.14g, 28.71mmol) was added in batches to the mixture. The system was stirred at room temperature for 2 hours. Saturated sodium bisulfite aqueous solution (50mL) was added to the reaction mixture, which was then extracted with dichloromethane/methanol (10/1) (50mL × 5). The organic phase was dried over anhydrous sodium sulfate and concentrated. The crude product was purified by flash silica gel column (ethyl acetate/petroleum ether: 0-80%) to obtain ethyl 2,5-dioxotetrahydro-1H-pyrrolizine-7a(5H)-carboxylate (1.4g, yield: 69%), a brown oily substance. ES-API: [M+H]⁺=212.1.

Step 2: Ethyl 2,5-dioxotetrahydro-1H-pyrnolizine-7a(5H)-carboxylate (1g, 4.74mmol), difluoromethyl(2-pyridyl)sulfone (1.65g, 8.52mmol), and N,N-dimethylformamide (24mL) were added to a round-bottomed flask. Under nitrogen protection and cooled to -60°C, with stirred, a tetrahydrofuran solution of potassium tert-butoxide (11.8mL, 11.84mmol) was slowly added dropwise to the reaction mixture. After 30 minutes dropwise addition was complete, the reaction mixture was naturally warmed to room temperature, stirred at room temperature for 4 hours. Saturated sodium bicarbonate aqueous solution (100mL) was added to the reaction mixture, which was then extracted with ethyl acetate (50mL × 3). The organic phase was washed with saturated brine, dried over anhydrous sodium sulfate, and the crude product was purified by flash silica gel column (ethyl acetate/petroleum ether: 0-60%) to obtain ethyl 2-(difluoromethylene)-5-oxotetrahydro-1H-pyrrolizine-7a(5H)-carboxylate (130mg, yield: 11.1%), a yellow oil. ES-API: [M+H]⁺=246.1.

Step 3: Under nitrogen protection, ethyl 2-(difluoromethylene)-5-oxotetrahydro-1H-pyrrolizine-7a(5H)-carboxylate (130mg, 0.53mmol) and tetrahydrofuran (5mL) were added to a reaction flask and cooled to 0°C. Under stirring, a 1M tetrahydrofuran solution of lithium aluminum hydride (2.1mL, 2. 10mmol) was added dropwise to the reaction mixture. After removing the cooling bath, the reaction mixture was stirred at 65°C for 2 hours. Cooled to 0°C, the reaction mixture was quenched with sodium sulfate decahydrate, filtered, and the filter cake was washed with tetrahydrofuran. The filtrate was dried over anhydrous sodium sulfate and concentrated to obtain (2-(fluoromethylene)tetrahydro-1H-pyrrolizin-7a(5H)-yl)methanol (90 mg, crude product), a yellow oily substance. ES-API: [M+H]⁺=172.1.

Step 4: (2-(fluoromethylene)tetrahydro-1H-pyrrolizin-7a(5H)-yl)methanol (54 mg, 0.32 mmol) was dissolved in anhydrous tetrahydrofuran (3 mL) and, under an ice water bath, sodium hydride (12 mg, 0.31 mmol) was added. The reaction was proceeded for 20 minutes under an ice water bath, after which a tetrahydrofuran solution (1 mL) of tert-butyl (5S,5aS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (50 mg, 0.063 mmol) was slowly added dropwise to the reaction mixture and reacted at 0°C for 20 minutes. Upon completion of the reaction (monitored by LCMS), saturated ammonium chloride solution (30 mL) was added to the reaction mixture. The reaction mixture was extracted with ethyl acetate (20 mL × 2). The organic phase was washed with saturated brine (15 mL × 3), dried over anhydrous sodium sulfate, filtered, and concentrated to obtain crude tert-butyl (5S,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (55 mg, crude product). ES-API: [M+H]⁺=895.3

Step 5: Cesium fluoride (55 mg, 0.61 mmol) was added to a N,N-dimethylformamide solution (2mL) of tert-butyl (5S,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (55 mg, crude product). The reaction mixture was stirred at room temperature for 1 hour. Upon completion of the reaction (monitored by LCMS), the reaction mixture was diluted with ethyl acetate (20 mL), washed with water (15 mL × 2), and saturated brine (15 mL × 3), dried over anhydrous sodium sulfate, filtered, and concentrated to obtain crude tert-butyl (5S,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, crude product). ES-API: [M+H]⁺= 739.3.

Step 6: A 4M hydrochloric acid-dioxane solution (0.68 mL) was slowly added under an ice water bath to a nitrile solution (3 mL) of tert-butyl (5S,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-Sa,6,7,8,9,10-hexahydro-SH-4-oxa-3,10a,11,13,14-pentaaza-6,9-methanonaphtho[1,8-ab]heptalene-14-carboxylate (40 mg, crude product). The reaction mixture was stirred at 0°C for 1 hour. Upon completion of the reaction (monitored by LCMS), the reaction mixture was concentrated to dryness, and the residue was purified by preparative HPLC (ammonium bicarbonate method 2) to obtain (5S,5aS,6S,9R)-2-(8-ethynyl-7-fluoronaphthalen-1-yl)-1-fluoro-12-((2-(fluoromethylene)tetrahydro-1H-pyrrolizin-7a(5H)-ylhnethoxy)-5-methyl-5a,6,7,8,9,10-hexahydro-5H-4-oxa-3,10a,11,13,14-pentaaza-6,9-methanonaphtho[1,8-ab]heptalene (Z601, 5 mg, yield: 14.4 %), white solid. ES-API: [M+H]⁺= 639.3. ¹H NMR (400MHz, CD₃OD): 8.11-8.05(m, 2H), 7.66-7.54(m, 2H), 7.45-7.19(m, 1H), 6.82-6.54(m, 1H), 5.38-5.35(m, 1H), 4.57-4.50(m, 1H), 4.30-4.20(m, 2H), 4.09-4.07(m, 1H), 3.86-3.69(m, 2H), 3.59-3.57(m, 1H), 3.47-3.44(m, 1H), 3.35-3.34(m, 1H), 3.21-3.10(m, 2H), 2.85-2.66(m, 2H), 2.57-2.42(m, 1H), 2.15-1.77(m, 8H), 1.59-1.56(m, 3H).

### Example 35: Synthesis of 5S,5aS,6S,9R)-2-(8-ethynyl-7-fluoronaphthalen-1-yl)-1-fluoro-12-(((1aR,6aR)-hexahydrocyclopropa[b]pyrrolizin-5a(3H)-yl)methoxy)-5-methyl-5a,6,7,8,9,10-hezahydro-5H-4-oxa-3,10a,11,13,14-pentaaza-6,9-methanonaphtho[1,8-ab]heptalene (Z624)

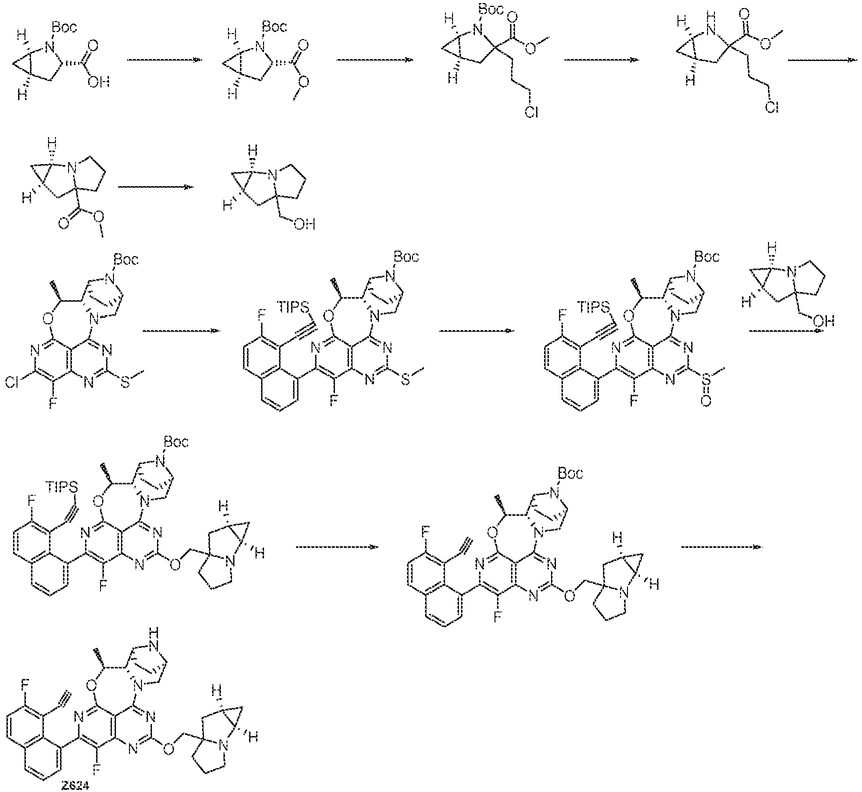

Step 1: Under an ice water bath, potassium carbonate (3.65 g, 26.4 mmol) and iodomethane (1.6 mL, 26.4 mmol) were added to a solution of (1R,3S,SR)-2-(tert-butoxycarbonyl)-2-azabicyclo[3.1.0]hexane-3-carboxylic acid (2 g, 8.8 mmol) in N,N-dimethylformamide (50 mL). The mixture was stirred at room temperature for 1.5 hours. Upon completion of the reaction (monitored by LCMS), the mixture was filtered and washed with ethyl acetate. The filtrate was concentrated under reduced pressure to obtain 2-(tert-butyl) 3-methyl(1R,3S,5R)-2-azabicyclo[3.1.0]hexane-2,3-dicarboxylate (2.01 g, yield: 95%). ES-API: [M-100]⁺= 142.1.

Step 2: Under nitrogen protection, 2-(tert-butyl) 3-methyl(1R,3S,SR)-2-azabicyclo[3.1.0]hexane-2,3-dicarboxylate (1.0 g, 4.1 mmol) was dissolved in anhydrous tetrahydrofuran (10 mL). The mixture was placed in a dry ice-acetone bath. A solution of bis(trimethylsilyl)aminolithium in tetrahydrofuran (8.3 mL, 8.3 mmol, 1 M) was slowly added dropwise, stirred for 1 hour at this temperature, and then a solution of 1-chloro-3-iodopropane (4.24 g, 20.7 mmol) in anhydrous tetrahydrofuran (10 mL) was slowly added. The reaction mixture was further stirred at -78°C for 1 hour. Upon completion of the reaction (monitored by LCMS), saturated ammonium chloride solution (50 mL) was added to quench the reaction. The mixture was extracted with ethyl acetate (50 mL × 3), washed with saturated brine (50 mL × 3), dried over anhydrous sodium sulfate, filtered, and concentrated. The crude product was purified by flash silica gel column chromatography (petroleum ether: ethyl acetate = 20:1) to obtain 2-(tert-butyl) 3-methyl(1R,5R)-3-(3-chloropropyl)-2-azabicyclo[3.1.0]hexane-2,3-dicarboxylate (1.1 g, yield: 83.5%). ES-API: [M-100]⁺= 218.2.

Step 3: At room temperature, a 4M hydrochloric acid/dioxane solution (15 mL) was slowly added to a solution of 2-(tert-butyl)3-methyl(1R,5R)-3-(3-chloropropyl)-2-azabicyclo[3.1.0]hexane-2,3-dicarboxylate (1.1 g, 3.46 mmol) in acetonitrile (3 mL). The mixture was stirred at room temperature for 0.5 hours. Upon completion of the reaction (monitored by LCMS), the reaction mixture was concentrated under reduced pressure to obtain methyl (1R,5R)-3-(3-chloropropyl)-2-azabicyclo[3.1.0]hexane-3-carboxylate (0.75 g, yield: 100%). ES-API: [M+H]⁺= 218.2.

Step 4: At room temperature, sodium bicarbonate (1.4 g, 17.2 mmol) and potassium iodide (57 mg, 0.34 mmol) were added to a solution of methyl (1R,5R)-3-(3-chloropropyl)-2-azabicyclo[3.1.0]hexane-3-carboxylate (0.75 g, 3.4 mmol) in acetonitrile (10 mL). The mixture was stirred at room temperature for 1.5 hours. Upon completion of the reaction (monitored by LCMS), the mixture was filtered and washed with acetonitrile. The filtrate was concentrated under reduced pressure and the crude product was purified by flash silica gel column chromatography (petroleum ether: ethyl acetate = 15:1) to obtain methyl (1aR,6aR)-hexahydrocyclopropa[b]pyrrolizine-5a(3H)-carboxylate (500 mg, yield: 80%). ES-API: [M+H]⁺=182.2.

Step 5: Under nitrogen protection, methyl (1aR,6aR)-hexahydrocyclopropa[b]pyrrolizine-5a(3H)-carboxylate (500 mg, 2.76 mmol) was dissolved in anhydrous tetrahydrofuran (10 mL), and the mixture was placed in an ice water bath. A solution of lithium aluminum hydride in tetrahydrofuran (5.5 mL, 5.5 mmol, 1 M) was slowly added dropwise, and the mixture was stirred at this temperature for 0.5 hours. Upon completion of the reaction (monitored by LCMS), dodecahydrate sodium sulfate (500 mg) was added to quench the reaction, the mixture was stirred at room temperature for 0.5 hours, and then filtered. The filtrate was concentrated under reduced pressure to obtain ((1aR,6aR)-hexahydrocyclopropa[b]pyrrolizin-5a(3H)-yl)methanol (300 mg, yield: 71%). ES-API: [M+H]⁺= 154.1.

Step 6: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (150 mg, 0.311 mmol), (2-fluoro-8-(4,4,5,5-tetramethyl-1,3,2-dioxaborolane-2-yl)naphth-1-yl)ethynyltriisopropylsilane (211 mg, 0.466 mmol), potassium phosphate (132 mg, 0.62 mmol), and methanesulfonic acid [butylbis(1-adamantyl)phosphine](2-amino-1,1'-biphenyl)palladium(II) (23 mg, 0.031 mmol) were dissolved in tetrahydrofuran (6.0 mL) and water (1.5 mL). The reaction mixture was heated to 80°C under microwave and stirred for 40 minutes. Upon completion of the reaction (monitored by LCMS), the reaction mixture was concentrated under reduced pressure, and the crude product was purified by silica gel column chromatography (dichloromethane: methanol = 20:1) to obtain tert-butyl (5 S,5aS,6S,9R)-1 -fluoro-2-(7-fluoro-8-((triisopropylsilyl)ethynyl)naphthalen-1 -yl)-5-methyl-12-(methylthio)-5a,6,7,8,9, 10-hexahydro-5H-4-oxa-3, 10a, 11, 13,14-pentaaza-6,9-methanonaphtho[1,8-ab]heptalene-14-carboxylate (290 mg, crude product). ES-API: [M+H]⁺= 772.3.

Step 7: Tert-butyl (SS,SaS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (290 mg, crude product) was dissolved in dichloromethane (10.0 mL). Under an ice water bath, m-chloroperbenzoic acid (70 mg, 0.406 mmol) was added. The reaction was proceeded for 30 minutes in the ice water bath. Upon completion of the reaction (monitored by LCMS), the reaction mixture was diluted with dichloromethane (20 mL), washed with saturated sodium bisulfite solution (15 mL × 2) and saturated brine (15 mL × 3), dried over anhydrous sodium sulfate, filtered, and concentrated to obtain tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (280 mg, crude product). ES-API: [M+H]⁺= 788.3.

Step 8: ((1aR,6aR)-hexahydrocyclopropa[b]pyrrolizin-5a(3H)-yl)methanol (29 mg, 0.19 mmol) was dissolved in anhydrous tetrahydrofuran (5 mL), and under an ice water bath, sodium hydride (15 mg, 0.38 mmol) was added. The reaction was proceeded for 30 minutes in the ice water bath, then a tetrahydrofuran solution (2 mL) of tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (50 mg, 0.063 mmol) was added dropwise to the reaction mixture, which was then reacted at 40°C for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous sodium sulfate, filtered, and the crude product was purified by silica gel column chromatography (dichloromethane: methanol = 15:1) to obtain tert-butyl (5S,5aS,6S,9R)-1-fluoro-2-(7-fluoro-8-((triisopropylsilyl)ethynyl)aphthalen-1-yl)-12-(((1aR,6aR)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, yield: 36%). ES-API: [M+H]⁺= 877.5

Step 9: Cesium fluoride (34 mg, 0.22 mmol) was added to a solution of tert-butyl (5S,5aS,6S,9R)-1-fluoro-2-(7-fluoro-8-((triisopropylsilyl)ethynyl)naphthalen-1-yl)-12-(((1aR,6aR)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, 0.023 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL), washed with water (15 mL × 2) and saturated brine (15 mL × 3), dried over anhydrous sodium sulfate, filtered, and concentrated to obtain tert-butyl (SS,SaS,6S,9R)-2-(8-ethynyl-7-fluoronaphthalen-1-yl)-1-fluoro-12-(((1aR,6aR)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (16 mg, yield: 100%). ES-API: [M+H]⁺= 721.3 Step 10: A 4 M solution of hydrochloric acid in dioxane (3 mL) was slowly added to a solution of tert-butyl (5S,5aS,6S,9R)-2-(8-ethynyl-7-fluoronaphthalen-1-yl)-1-fluoro-12-(((1aR,6aR)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (16 mg, 0.023 mmol) in acetonitrile (1 mL) under an ice water bath. The reaction mixture was stirred at 0°C for 0.5 hours. Upon completion of the reaction (monitored by LCMS), the reaction mixture was concentrated to dryness, and the residue was purified by preparative HPLC (ammonium bicarbonate method 2) (chromatographic condition: 15mL-35-85-13min) to obtain a pale yellow solid of (5S,5aS,6S,9R)-2-(8-ethynyl-7-fluoronaphthalen-1-yl)-1-fluoro-12-(((1aR,6aR)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene (Z624, 1.2 mg, yield: 8.4 %). ES-API: [M+H]⁺= 621.3.

### Example 36: Synthesis of (5S,5aS,6S,9R)-2-(8-ethynyl-7-fluoronaphthalen-1-yl)-1-fluoro-12-(((1aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene (Z625)

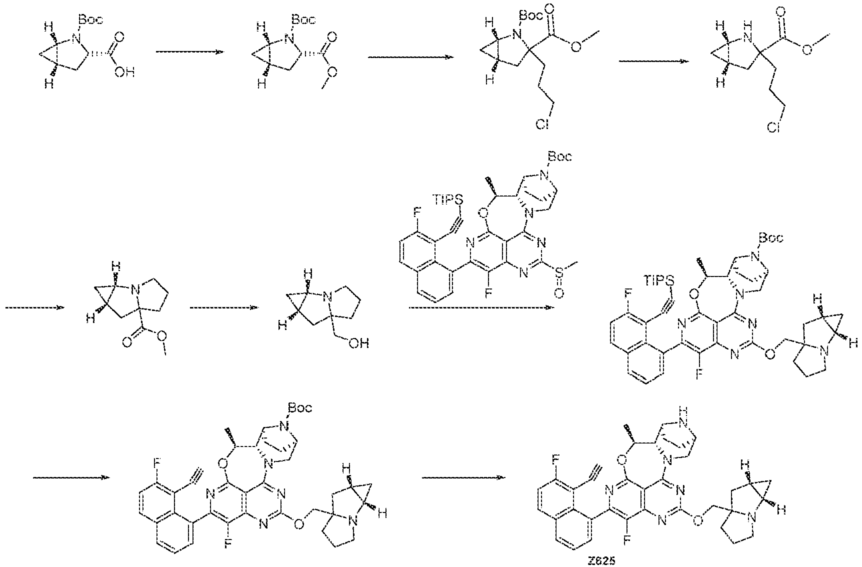

Step 1: Under an ice-water bath, potassium carbonate (3.65 g, 26.4 mmol) and iodomethane (1.6 mL, 26.4 mmol) were added to a solution of (1S,3S,5S)-2-(tert-butoxycarbonyl)-2-azabicyclo[3.1.0]hexane-3-carboxylic acid (2 g, 8.8 mmol) in N,N-dimethylformamide (50 mL). The mixture was stirred at room temperature for 1.5 hours. Upon completion of the reaction, as monitored by LCMS, the mixture was filtered and washed with ethyl acetate. The filtrate was concentrated under reduced pressure to obtain 2-(tert-butyl) 3-methyl(1S,3S,SS)-2-azabicyclo[3.1.0]hexane-2,3-dicarboxylate (2.05 g, yield: 96%). ES-API: [M-100]⁺= 142.1.

Step 2: Under nitrogen protection, 2-(tert-butyl) 3-methyl(lS,3S,5S)-2-azabicyclo[3.1.0]hexane-2,3-dicarboxylate (1.0 g, 4.1 mmol) was dissolved in anhydrous tetrahydrofuran (10 mL) and the mixture was placed in a dry ice-acetone bath. A solution of bis(trimethylsilyl)aminolithium (8.3 mL, 8.3 mmol, 1 M in tetrahydrofuran) was slowly added dropwise to the above solution, which was then stirred at this temperature for 1 hour. A solution of 1-chloro-3-iodopropane (4.24 g, 20.7 mmol) in anhydrous tetrahydrofuran (10 mL) was slowly added via syringe to the reaction mixture, which was then further stirred at -78 °C for 1 hour. Upon completion of the reaction, as monitored by LCMS, saturated ammonium chloride solution (50 mL) was added to quench the reaction, and the mixture was extracted with ethyl acetate (50 mLx3). The organic phases were washed with saturated brine (50 mLx3), dried over anhydrous sodium sulfate, filtered, and concentrated. The crude product was purified by flash silica gel column chromatography (petroleum ether: ethyl acetate = 20:1) to obtain 2-(tert-butyl)3-methyl(1S,5S)-3-(3-chloropropyl)-2-azabicyclo[3.1.0]hexane-2,3-dicarboxylate (1.2g, yield: 91%). ES-API: [M-100]⁺= 218.2.

Step 3: At room temperature, a 4M solution of hydrochloric acid/dioxane (15 mL) was slowly added to a solution of 2-(tert-butyl)3-methyl(1S,5S)-3-(3-chloropropyl)-2-azabicyclo[3.1.0]hexane-2,3-dicarboxylate (1.2g, 3.7 mmol) in acetonitrile (3 mL), and the mixture was stirred at room temperature for 0.5 hours. Upon completion of the reaction, as monitored by LCMS, the reaction mixture was concentrated under reduced pressure to obtain methyl (1S,5S)-3-(3-chloropropyl)-2-azabicyclo[3.1.0]hexane-3-carboxylate (0.82 g, yield: 100%). ES-API: [M+H]⁺= 218.2.

Step 4: At room temperature, sodium bicarbonate (1.5 g, 18.8 mmol) and potassium iodide (63 mg, 0.37 mmol) were added to a solution of methyl (1S,SS)-3-(3-chloropropyl)-2-azabicyclo[3.1.0]hexane-3-carboxylate (0.82 g, 3.7mmol) in acetonitrile (10 mL), and the mixture was stirred at room temperature for 1.5 hours. Upon completion of the reaction, as monitored by LCMS, the mixture was filtered and washed with acetonitrile. The filtrate was concentrated under reduced pressure, and the crude product was purified by flash silica gel column chromatography (petroleum ether: ethyl acetate = 15:1) to obtain methyl (laS,6aS)-hexahydrocyclopropa[b]pyrrolizine-5a(3H)-carboxylate (521 mg, yield: 76%). ES-API: [M+H]⁺=182.1.

Step 5: Under nitrogen protection, methyl (1aS,6aS)-hexahydrocyclopropa[b]pyrrolizine-5a(3H)-carboxylate (521 mg, 2.87 mmol) was dissolved in anhydrous tetrahydrofuran (10 mL), and the mixture was placed in an ice-water bath. A solution of lithium aluminium hydride (5.74 mL, 5.74 mmol, 1 M in tetrahydrofuran) was slowly added dropwise to the solution, and the mixture was stirred at this temperature for 0.5 hours. Upon completion of the reaction, as monitored by LCMS, sodium sulfate decahydrate (521 mg) was added to quench the reaction, and the mixture was stirred at room temperature for 0.5 hours, then filtered. The filtrate was concentrated under reduced pressure to obtain ((1aS,6aS)-hexahydrocyclopropa[b]pyrrolizin-5a(3H-yl)methanol (410 mg, yield: 93%). ES-API: [M+H]⁺= 154.1.

Step 6: ((1aS,6aS)-hexahydrocyclopropa[b]pyrrolizin-5a(3H)-yl)methanol (29 mg, 0.19 mmol) was dissolved in anhydrous tetrahydrofuran (5 mL). Under an ice-water bath, sodium hydride (15 mg, 0.38 mmol) was added. The reaction was proceeded for 30 minutes in an ice-water bath. Then a solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (50 mg, 0.063 mmol) in tetrahydrofuran (2 mL) was slowly added dropwise to the reaction mixture, which was then reacted at 40°C for 30 minutes. Upon completion of the reaction, as monitored by LCMS, the reaction mixture was diluted with ethyl acetate (20 mL), washed with saturated ammonium chloride solution (15 mLx2) and saturated brine (15 mLx3), dried over anhydrous sodium sulfate, filtered, and the crude product was purified by silica gel column chromatography (dichloromethane: methanol = 15:1) to obtain tert-butyl (5 S,5aS,6S,9R)-1 -fluoro-2-(7-fluoro-8-((triisopropylsilyl)ethynyl)naphthalen-1 -y 1)-12-(((1aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, yield: 53%). ES-API: [M+H]⁺= 877.5.

Step 7: Cesium fluoride (52 mg, 0.34 mmol) was added to a solution of tert-butyl (5S,5aS,6S,9R)-1-fluoro-2-(7-fluoro-8-((triisopropylsilyl)ethynyl)naphthalen-1-yl)-12-(((1aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.034 mmol) in N,N-dimethylformamide (1.5 mL). The reaction mixture was stirred at room temperature for 2 hours. Upon completion of the reaction, as monitored by LCMS, the reaction mixture was diluted with ethyl acetate (20 mL), washed with water (15 mLx2) and saturated brine (15 mLx3), dried over anhydrous sodium sulfate, filtered, and concentrated to obtain a crude product tert-butyl (5S,5aS,6S,9R)-2-(8-ethynyl-7-fluoronaphthalen-1-yl)-1-fluoro-12-(((1aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, yield: 100%). ES-API: [M+H]⁺= 721.3.

Step 8: Under an ice-water bath, a 4 M solution of hydrochloric acid in dioxane (3 mL) was slowly added to a solution of tert-butyl (SS,SaS,6S,9R)-2-(8-ethynyl-7-fluoronaphthalen-1-yl)-1-fluoro-12-(((1 aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.034 mmol) in acetonitrile (1 mL). The reaction mixture was stirred at 0°C for 0.5 hours. Upon completion of the reaction, as monitored by LCMS, the reaction mixture was concentrated to dryness, and the residue was purified by preparative HPLC (ammonium bicarbonate method 2) (chromatographic condition: 15mL-35-85-13min) to obtain a pale yellow solid (5S,5aS,6S,9R)-2-(8-ethynyl-7-fluoronaphthalen-1-yl)-1-fluoro-12-(((1aS,6aS)-hexahydrocyclopropa[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptalene (Z625, 4 mg, yield: 18.9 %). ES-API: [M+H]⁺= 621.3.

### Example 37: Synthesis of (5S,5aS,6S,9R)-2-(8-ethynyl-7-fluoronaphthalen-1-yl)-1-fluoro-12-(((1aR,6aR,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene (Z626-1) and (5S,5aS,6S,9R)-2-(8-ethynyl-7-fluoronaphthalen-1-yl)-1-fluoro-12-(((1aR,6aS,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene (Z626-2)

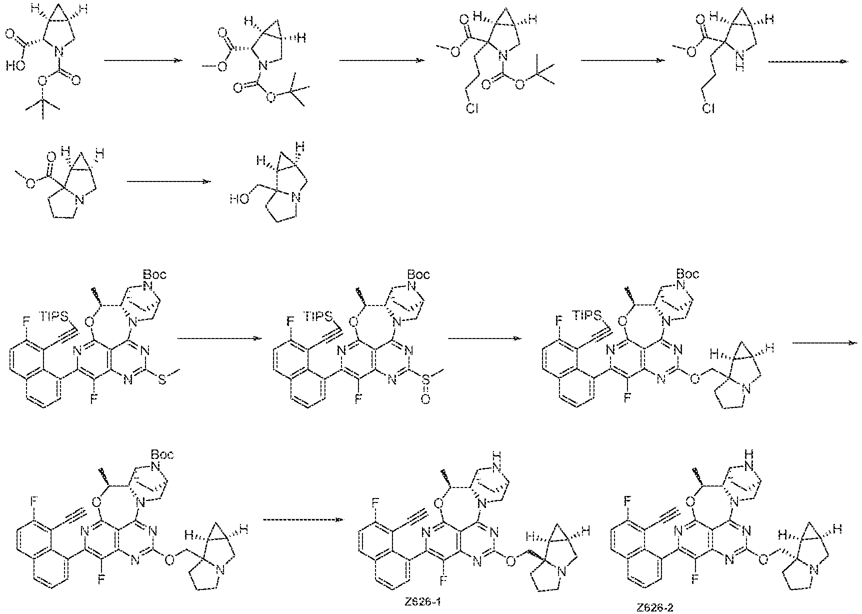

Step 1: In a sealed tube, potassium carbonate (276 mg, 2.0 mmol) and iodomethane (0.19 mL, 3.0 mmol) were added to a solution of (1S,2S,5R)-3-(tert-butoxycarbonyl)-3-azabicyclo[3.1.0]hexane-2-carboxylic acid (227 mg, 1.0 mmol) in N,N-dimethylformamide (10 mL). The reaction mixture was stirred at room temperature for 2 hours, then diluted with water and extracted three times with petroleum ether. The organic phase was washed with saturated brine, dried over anhydrous sodium sulfate, filtered, and concentrated to obtain a crude product of 3-(tert-butyl) 2-methyl(1S,2S,5R)-3-azabicyclo[3.1.0]hexane-2,3-dicarboxylate, which was then directly used in the next step. ES-API: [M-55]⁺= 186.1.

Step 2: Under nitrogen protection, the crude product of 3-(tert-butyl) 2-methyl(1S,2S,5R)-3-azabicyclo[3.1.0]hexane-2,3-dicarboxylate was dissolved in anhydrous tetrahydrofuran (5.0 mL) and the mixture was placed in a dry ice-ethanol bath. Bis(trimethylsilyl)aminolithium (1.7 mL, 1.7 mmol, 1 M in tetrahydrofuran) was slowly added dropwise to the solution, and the mixture was stirred at this temperature for 15 minutes, then 1-chloro-3-iodopropane (529 mg, 2.6 mmol) was slowly added. After stirred at -78°C for 1 hour, the reaction mixture was quenched with saturated ammonium chloride solution, extracted three times with ethyl acetate, washed with saturated brine, dried over anhydrous sodium sulfate, filtered, and concentrated. The crude product was purified by flash silica gel column (ethyl acetate/petroleum ether: 0-15%) to obtain 3-(tert-butyl) 2-methyl(lS,5R)-2-(3-chloropropyl)-3-azabicyclo[3.1.0]hexane-2,3-dicarboxylate (236 mg, yield: 86%). ES-API: [M-99]⁺= 218.1.

Step 3: At room temperature, a 4 M solution of hydrochloric acid-dioxane (1.0 mL) was slowly added to a solution of 3-(tert-butyl) 2-methyl(1S,SR)-2-(3-chloropropyl)-3-azabicyclo[3.1.0]hexane-2,3-dicarboxylate (209 mg, 0.66 mmol) in acetonitrile (10.0 mL). After the reaction mixture stirred at room temperature for 1 hour, the reaction solution was concentrated under reduced pressure to dryness to obtain methyl (1S,5R)-2-(3-chloropropyl)-3-azabicyclo[3.1.0]hexane-2-carboxylate. The crude product was not purified and was directly used in the next step. ES-API: [M+H]⁺= 218.1.

Step 4: At room temperature, sodium bicarbonate (276 mg, 3.3 mmol) and potassium iodide (11 mg, 0.07 mmol) were successively added to a solution of the crude product of methyl (1S,5R)-2-(3-chloropropyl)-3-azabicyclo[3.1.0]hexane-2-carboxylate in acetonitrile (30.0 mL). The reaction mixture was heated to reflux and stirred for 2 hours, then filtered. The filtrate was concentrated under reduced pressure, and the obtained crude product was purified by flash silica gel column (methanol/dichloromethane: 0-10%) to obtain methyl (1aR,6bS)-hexahydrocyclopropa[a]pyrrolizine-6a(4H)-carboxylate (101 mg, yield: 85%). ES-API: [M+H]⁺= 182.2.

Step 5: Under nitrogen protection, methyl (1aR,6bS)-hexahydrocyclopropa[a]pyrrolizine-6a(4H)-carboxylate (101 mg, 0.56 mmol) was dissolved in anhydrous tetrahydrofuran (10.0 mL), and the mixture was placed in an ice-water bath. Lithium aluminium hydride (0.84 mL, 0.84 mmol, 1 M in tetrahydrofuran) was slowly added dropwise to the solution, and the mixture was stirred at this temperature for 0.5 hours. Sodium sulfate decahydrate was then slowly added to quench the reaction. After warmed to room temperature and stirred for 0.5 hours, the mixture was filtered. The filtrate was concentrated under reduced pressure to obtain ((1aR,6bS)-hexahydrocyclopropa[a]pyrrolizin-6a(4H)-yl)methanol (83 mg, yield: 97%). ES-API: [M+H]+= 154.3.

Step 6: At 0°C, m-chloroperoxybenzoic acid (17 mg, 0.08 mmol, 85% purity) was added to a dichloromethane solution (5.0 mL) of tert-butyl (SS,SaS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (50 mg, 0.065 mmol), and the mixture was stirred at 0°C for 0.5 hours. Saturated sodium bicarbonate and sodium sulfite solutions were then added, and after vigorous stirring for 15 minutes, the organic phase was separated. The aqueous phase was extracted twice with dichloromethane, the organic phases were combined, washed with saturated brine, dried over anhydrous sodium sulfate, filtered, and evaporated to dryness to obtain tert-butyl (SS,Sa5,6S,9R)-1-fluoro-2-(7-fluoro-8-((triisopropylsilyl)ethγnyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate. The crude product was used directly in the next step without purification. ES-API: [M+H]⁺= 788.3.

Step 7: At 0°C, sodium hydride (60% dispersed in oil) (10 mg, 0.26 mmol) was added to a solution of ((1aR,6bS)-hexahydrocyclopropa[a]pyrrolizin-6a(4H)-yl)methanol (25 mg, 0.16 mmol) in tetrahydrofuran (5.0 mL). After the mixture reacting for 15 minutes, a tetrahydrofuran solution (1.0 mL) of the crude product of tert-butyl (SS,SaS,65,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate was added dropwise. After the mixture reacting at 0°C for 0.5 hours, saturated ammonium chloride solution was slowly added, the reaction mixture was extracted three times with dichloromethane. The organic phases were combined, washed with saturated brine, dried over anhydrous sodium sulfate, filtered, and evaporated to dryness. The crude product was purified by a preparative thin-layer chromatography (ethyl acetate/petroleum ether 80%) to obtain tert-butyl (5S,5aS,6S,9R)-1-fluoro-2-(7-fluoro-8-((triisopropylsilyl)ethynyl)naphthalen-1-yl)-12-(((1aR,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (23 mg, yield: 40%). ES-API: [M+H]⁺= 877.4.

Step 8: Tert-butyl (5S,5aS,6S,9R)-1-fluoro-2-(7-fluoro-8-((triisopropylsilyl)ethynyl)naphthalen-1-yl)-12-(((1aR,6bS)-hexahydrocyclopropa[a]pyrrolizin-6a(4H)-yl)methoxy)-5-methyl-5a,6,7,8,9,10-hexahydro-5H-4-oxa-3,10,11,13,14-pentaaza-6,9-methanonaphtho[1,8-ab]heptalene-14-carboxylate (25 mg, 0.026 mmol) was dissolved in N,N-dimethylformamide (1.0 mL). Cesium fluoride (40 mg, 0.26 mmol) was added. The reaction mixture was stirred at room temperature for 1 hour. Upon completion of the reaction, water was added to the reaction solution. The reaction mixture was extracted three times with ethyl acetate. The organic phases were combined, washed with saturated brine, dried over anhydrous sodium sulfate, and filtered. The filtrate was concentrated under reduced pressure to obtain tert-butyl (SS,SaS,6S,9R)-2-(8-ethynyl-7-fluoronaphthalen-1-yl)-1-fluoro-12-(((1aR,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate, which was then used directly in the next step without purification. ES-API: [M+H]⁺= 721.3.

Step 9: The crude product of tert-butyl (5S,5aS,6S,9R)-2-(8-ethynyl-7-fluoronaphthalen-1-yl)-1-fluoro-12-(((1aR,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate was dissolved in acetonitrile (5.0 mL). Under an ice water bath, 4M hydrochloric acid/dioxane solution (1.0 mL, 4.0 mmol) was added. The reaction mixture was stirred at 0°C for 0.5 hours and then heated to room temperature and stirred for another 0.5 hours. Upon completion of the reaction, the solvent was removed under reduced pressure. The crude product was purified by preparative HPLC (ammonium bicarbonate method 1) (chromatographic condition: 15mL-55-95-13min) to obtain two isomers. One isomer was arbitrarily designated as (5S,5aS,6S,9R)-2-(8-ethynyl-7-fluoronaphthalen-1-yl)-1-fluoro-12-(((1aR,6aR,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene (Z262-1, retention time 7.03 min, 0.40 mg, yield: 2.5%), ES-API: [M+H]⁺= 621.3. The other isomer was arbitrarily designated as (5S,5aS,6S,9R)-2-(8-ethynyl-7-fluoronaphthalen-1-yl)-1-fluoro-12-(((1aR,6aS,6bS)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene(Z262-2, retention time 8.22 min, 1.30 mg, yield: 8.1%), ES-API: [M+H]⁺= 621.3.

### Example 38: Synthesis of (5S,5aS,6S,9R)-2-(8-ethynyl-7-fluoronaphthalen-1-yl)-1-fluoro-12-(((1aS,6aR,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene (Z627-1) and (5S,5aS,6S,9R)-2-(8-ethynyl-7-fluoronaphthalen-1-yl)-1-fluoro-12-(((1aS,6aS,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene (Z627-2)

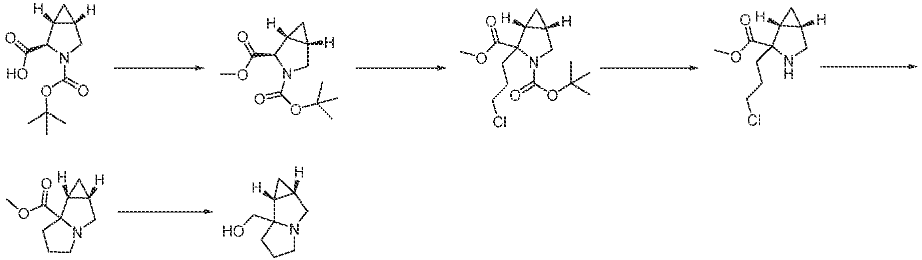

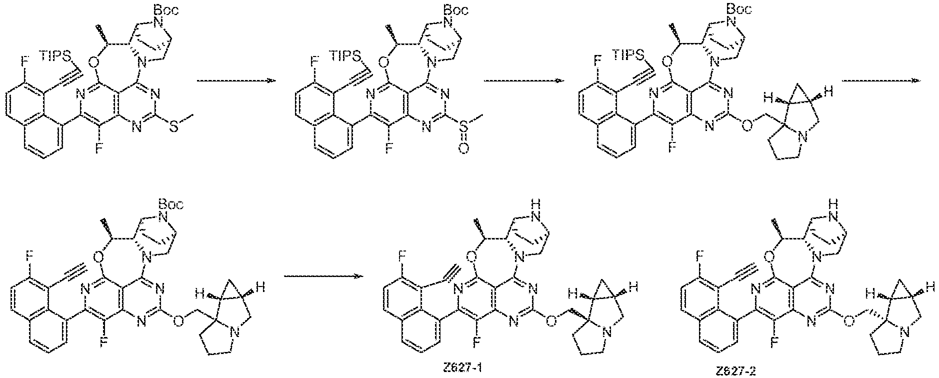

Step 1: Inside a sealed tube, potassium carbonate (276 mg, 2.0 mmol) and iodomethane (0.19 mL, 3.0 mmol) were added to a solution of (1R,2R,SS)-3-(tert-butoxycarbonyl)-3-azabicyclo[3.1.0]hexane-2-carboxylic acid (227 mg, 1.0 mmol) in N,N-dimethylformamide (10.0 mL). After stirred at room temperature for 2 hours, the reaction mixture was diluted with water and extracted three times with petroleum ether. The organic phase was washed with saturated brine, dried over anhydrous sodium sulfate, filtered, and concentrated to obtain a crude product of 3-(tert-butyl) 2-methyl (1R,2R,SS)-3-azabicyclo[3.1.0]hexane-2,3-dicarboxylate, which was then directly used for the next step. ES-API: [M-55]⁺= 186.1.

Step 2: Under nitrogen protection, the crude product of 3-(tert-butyl) 2-methyl (1R,2R,5S)-3-azabicyclo[3.1.0]hexane-2,3-dicarboxylate was dissolved in anhydrous tetrahydrofuran (5.0 mL) and placed in a dry ice-ethanol bath. Bis(trimethylsilyl)aminolithium (2.5 mL, 2.5 mmol, 1 M in tetrahydrofuran) was slowly added dropwise to the solution, which was then stirred at this temperature for 15 minutes, followed by slow addition of 1-chloro-3-iodopropane (613 mg, 3.0 mmol). The reaction mixture was stirred at -78 °C for 2 hours, quenched with saturated ammonium chloride solution, extracted three times with ethyl acetate, washed with saturated brine, dried over anhydrous sodium sulfate, filtered, and concentrated. The crude product was purified by flash silica gel column (ethyl acetate/petroleum ether: 0-15%) to obtain 3-(tert-butyl) 2-methyl (1R,5S)-2-(3-chloropropyl)-3-azabicyclo[3.1.0]hexane-2,3-dicarboxylate (191 mg, yield: 60%). ES-API: [M-99]⁺= 218.1.

Step 3: At room temperature, 4 M hydrochloric acid-dioxane solution (1.0 mL) was slowly added to a solution of 3-(tert-butyl) 2-methyl (1R,5S)-2-(3-chloropropyl)-3-azabicyclo[3.1.0]hexane-2,3-dicarboxylate (191 mg, 0.60 mmol) in acetonitrile (10 mL). After stirred at room temperature for 1 hour, the reaction mixture was concentrated under reduced pressure to obtain methyl (1R,5S)-2-(3-chloropropyl)-3-azabicyclo[3.1.0]hexane-2-carboxylate as a crude product used for the next step. ES-API: [M+H]⁺= 218.2.

Step 4: At room temperature, sodium bicarbonate (252 mg, 3.0 mmol) and potassium iodide (10 mg, 0.06 mmol) were successively added to a solution of the crude product of methyl (IR,5S)-2-(3-chloropropyl)-3-azabicyclo[3.1.0]hexane-2-carboxylate in acetonitrile (30 mL). The reaction mixture was heated to reflux and stirred for 2 hours, then filtered. The filtrate was concentrated under reduced pressure and the obtained crude product was purified by flash silica gel column (methanol/dichloromethane: 0-10%) to obtain methyl (1aS,6bR)-hexahydrocyclopropa[a]pyrrolizine-6a(4H)-carboxylate (96 mg, yield: 88%). ES-API: [M+H]⁺= 182.1.

Step 5: Under nitrogen protection, methyl (1aS,6bR)-hexahydrocyclopropa[a]pyrrolizine-6a(4H)-carboxylate (96 mg, 0.53 mmol) was dissolved in anhydrous tetrahydrofuran (5.0 mL). The mixture was placed in an ice-water bath, and lithium aluminum hydride (0.80 mL, 0.80 mmol, 1 M in tetrahydrofuran) was slowly added dropwise to the solution. After stirred at this temperature for 0.5 hours, the reaction mixture was quenched with decahydrate sodium sulfate and stirred at room temperature for 0.5 hours. After filtering, the filtrate was concentrated under reduced pressure to obtain ((1aS,6bR)-hexahydrocyclopropa[a]pyrrolizin-6a(4H)-yl)methanol (63 mg, yield: 78%). ES-API: [M+H]⁺= 154.3.

Step 6: At 0 °C, m-chloroperbenzoic acid (17 mg, 0.08 mmol, 85% purity) was added to a solution of tert-butyl (5 S,5aS,6S,9R)-1 -fluoro-2-(7-fluoro-8-((triisopropylsilyl)ethynyl)naphthalen-1 -yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (50 mg, 0.065 mmol) in dichloromethane (5.0 mL). After the mixture stirred at 0 °C for 0.5 hours, saturated sodium bicarbonate solution and saturated sodium sulfite solution were added, and the mixture was vigorously stirred for 15 minutes. The organic phase was separated, the aqueous phase was extracted twice with dichloromethane, and the organic phases were combined, washed with saturated brine, dried over anhydrous sodium sulfate, filtered, and evaporated. The obtained tert-butyl (5S,5aS,6S,9R)-1-fluoro-2-(7-fluoro-8-((triisopropylsilyl)ethγnyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate was used directly for the next step without purification. ES-API: [M+H]⁺= 788.3.

Step 7: At 0 °C, sodium hydride (60% dispersed in oil) (10 mg, 0.26 mmol) was added to a solution of ((laS,6bR)-hexahydrocyclopropa[a]pyrrolizin-6a(4H)-yl)methanol (25 mg, 0.16 mmol) in tetrahydrofuran (5.0 mL). After the mixture reacting for 15 minutes, a solution of the crude product of tert-butyl (5 S,5aS,6S,9R)-1 -fluoro-2-(7-fluoro-8-((triisopropylsilyl)ethynyl)naphthalen-1 -yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate in tetrahydrofuran (1.0 mL) was slowly added dropwise to the reaction. After the mixture reacting at 0 °C for 0.5 hours, saturated ammonium chloride solution was added, and the mixture was extracted three times with dichloromethane. The organic phases were combined, washed with saturated brine, dried over anhydrous sodium sulfate, filtered, and evaporated. The crude product was purified by preparative thin-layer chromatography (ethyl acetate/petroleum ether 80%) to obtain the product tert-butyl (SS,SaS,6S,9R)-1-fluoro-2-(7-fluoro-8-((triisopropylsilyl)ethynyl)naphthalen-1-yl)-12-(((1aS,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, yield: 53%). ES-API: [M+H]⁺= 877.4.

Step 8: Tert-butyl (5S,5aS,6S,9R)-1-fluoro-2-(7-fluoro-8-((triisopropylsilyl)ethynyl)naphthalen-1-yl)-12-(((1aS,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.034 mmol) was dissolved in N,N-dimethylformamide (1.0 mL) and cesium fluoride (52 mg, 0.34 mmol) was added. The reaction was proceeded at room temperature for 1 hour. After the reaction was complete, water was added to the reaction mixture, and the reaction mixture was extracted three times with ethyl acetate. The combined organic phases were washed with saturated brine, dried over anhydrous sodium sulfate, filtered, and the filtrate was concentrated under reduced pressure to obtain tert-butyl (5S,5aS,6S,9R)-2-(8-ethynyl-7-fluoronaphthalen-1-yl)-1-fluoro-12-(((1aS,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate. The crude product was used directly for the next step without purification. ES-API:[M+H]⁺= 721.3.

Step 9: The crude product of tert-butyl (SS,SaS,6S,9R)-2-(8-ethynyl-7-fluoronaphthalen-1-yl)-1-fluoro-12-(((1aS,6bR)-hexahydrocyclopropa[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate was dissolved in acetonitrile (5.0 mL). 4M hydrochloric acid/dioxane solution (1.0 mL, 4.0 mmol) was added under an ice water bath. The reaction was proceeded at 0 °C for 0.5 hours, then warmed to room temperature and reacted for another 0.5 hours. After completion of the reaction, the reaction mixture was concentrated to dryness. The crude product was purified by preparative HPLC (Ammonium bicarbonate method 1) (chromatographic condition: 15mL-55-95-13min) to obtain two isomers. One isomer was arbitrarily designated as (SS,SaS,6S,9R)-2-(8-ethynyl-7-fluoronaphthalen-1-yl)-1-fluoro-12-(((1aS,6aR,6bR)-hexahydrocyclopropa[a]pyrrolizin-6a(4H)-yl)methoxy)-5-methyl-5a,6,7,8,9,10-he-xahydro-5H-4-oxa-3,10a,11,13,14-pentaaza-6,9-methanonaphtho[1,8-ab]heptalene (Z627-1, retention time: 7.12 min, 1.15 mg, yield: 5.5%), ES-API: [M+H]⁺= 621.3. The other isomer was arbitrarily designated as (SS,SaS,6S,9R)-2-(8-ethynyl-7-fluoronaphthalen-1-yl)-1-fluoro-12-(((1aS,6aS,6bR)-hexahydrocyclopropa[a]pyrrolizin-6a(4H)-yl)methoxy)-5-methyl-5a,6,7,8,9,10-hexahydro-SH-4-oxa-3,10a,11,13,14-pentaaza-6,9-methanonaphtho[1,8-ab]heptalene (Z627-2, retention time: 8.37 min, 1.65 mg, yield: 7.8%), ES-API: [M+H]⁺= 621.3.

### Example 39: Synthesis of (5S,5aS,6S,9R)-12-((1'H,3'H,5'H-dispiro[cyclopropane-1,2'-pyrrolizine-6',1"-cyclopropan]-7a'(7'H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z628)

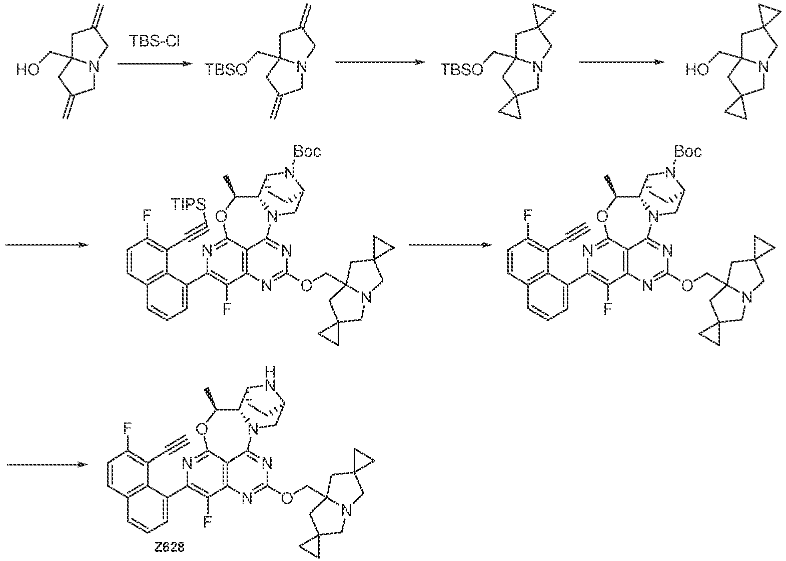

Step 1: To a dichloromethane (20.0mL) solution of (2,6-dimethyltetrahydro-1H-pyrrolizin-7a(5H)-yl)methanol (1.0g, 6.052mmol), tert-butyldimethylchlorosilane (912.13mg, 6.052mmol) and imidazole (823mg, 12.1mmol) were added, and the mixture was stirred at room temperature for 3 hours. Upon completion of the addition, the mixture was added to saturated ammonium chloride (30 mL), extracted with ethyl acetate (100 mL X 3). The organic phase was washed with saturated brine (200mL), dried over anhydrous sodium sulfate, filtered, and concentrated. The crude product was purified by flash silica gel column (methanol/dichloromethane: 0%-5%) to obtain 7a-(((tertbutyldimethylsilyl)oxy)methyl)-2,6-dimethyltetrahydro-1H-pyrrolizine (1.5g, 88% yield), a colorless and transparent liquid. ES-API: [M+H]⁺=280.3.

Step 2: At -20°C, diethyl zinc (5.3mL, 5.30mmol) was added to a dichloromethane (10.0 mL) solution of 7a-(((tert-butyldimethylsilyl)oxy)methyl)-2,6-dimethyltetrahydro-1H-pyrrolizine (0.3g, 71.073mmol), and the mixture stirred for 20 minutes at this temperature. After 20 minutes, diiodomethane (2.87g, 10.73mmol) was added, slowly warmed to an ice-water bath, and reacted for 3 hours. Upon completion of the reaction, with saturated ammonium chloride (30 mL) added, the mixture was extracted with ethyl acetate (80 mL X 2). The organic phase was washed with saturated brine (60mL), dried over anhydrous sodium sulfate, filtered, and concentrated to obtain the crude product 7a'-(((tert-butyldimethylsilyl)oxy)methyl)dihydro-1H,3'H,5'H-dispiro[cyclopropane-1,2'-pyrrolizine-6',1"-cyclopropane] (0.40g). ES-API: [M+H]⁺=308.2.

Step 3: To a solution of 7a'-(((tert-butyldimethylsilyl)oxy)methyl)dihydro-1'H,3'H,5'H-dispiro[cyclopropane-1,2'-pyrrolizine-6',1"-cyclopropane] (0.40g, crude) in acetonitrile (10.0mL), dioxane hydrochloride (4.0mL, 4M, 16.0mmol) was added, and the mixture reacted at room temperature for 3 hours. Upon completion of the reaction, the solvent was evaporated under reduced pressure, and acetonitrile (20.0mL) and dry potassium carbonate (1000mg, 7.24mmol) were added to the crude product, the mixture stirred at room temperature for 1 hour, filtered, and the filtrate was evaporated under reduced pressure to obtain (1'H,3'H,5'H-dispiro[cyclopropane-1,2'-pyrrolizine-6',1"-cyclopropanel-7a'(7'H)-yl)methanol (240mg, crude). ES-API: [M+H]⁺=194.1.

Step 4: (1'H,3'H,5'H-Dispiro[cyclopropane-1,2'-pyrrolizine-6',1"-cyclopropane]-7a'(7'H)-yl)methanol (240mg, crude) was dissolved in anhydrous tetrahydrofuran (10.0 mL), and sodium hydride (100mg, 2.50 mmol) was added under an ice-water bath. The mixture reacted for 30 minutes under ice-water bath condition. Then, a tetrahydrofuran (2 mL) solution of tert-butyl (5S,5aS,6S,9R)-1-fluoro-2-(7-fluoro-8-((tripropylsilyl)ethynyl)naphthalen-1-yl)-5-methyl-12-(methylsulfonyl)-5a,6,7,8,9,10-hexahydro-5H-4-oxygen-3,10a,11,13,14-pentaaza-6,9-methanonaphtho[1,8-ab]heptalene-14-carboxylate (80mg, 0.414 mmol) was added dropwise to the above reaction mixture, and reacted at 0°C for 30 minutes. Upon completion of the reaction (monitored by LCMS), the reaction mixture was diluted with ethyl acetate (50 mL), washed with saturated ammonium chloride solution (15 mL X 2) and saturated brine (50 mL X 3), dried over anhydrous sodium sulfate, filtered, and the crude product was purified by silica gel column chromatography (dichloromethane: methanol = 15:1) to obtain tert-butyl (SS,SaS,6S,9R)-12-((1'H,3'H,5'H-dispiro[cyclopropane-1,2'-pyrrolizine-6',1"-cyclopropan]-7a'(7'H)-yl)methoxy)-1-fluoro-2-(7-fluoro-8-((triisopropylsilyl)ethynyl)naphthalen-1-yl)-5-methyl-Sa,6,7,8,9,10-hexahydro-5H-4-oxa-3,10a,11,13,14-pentaaza-6,9-methanonaphtho[1,8-ab]heptalene-14-carboxylate (20mg, total yield for 3 steps 2.0%). ES-API: [M+H]⁺= 917.4

Step 5: Cesium fluoride (96.0 mg, 0.63mmol) was added to a solution of tert-butyl (SS,SaS,6S,9R)-12-((1'H,3'H,5'H-dispiro[cyclopropane-1,2'-pyrrolizine-6',1"-cyclopropan]-7a'(7'H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,1 1,1 3,14-pentaaza-6,9-methanonaphtho[1,8-ab]heptalene-14-carboxylate (20mg, 0.02183mmol) in N,N-dimethylformamide (2.0mL). The reaction mixture was stirred at room temperature for 2 hours. Upon completion of the reaction (monitored by LCMS), the reaction mixture was diluted with ethyl acetate (60mL), washed with water (30 mL X 2), saturated brine (50 mL X 3), dried over anhydrous sodium sulfate, filtered, and concentrated to obtain the crude product tert-butyl (5S,5aS,6S,9R)-12-((1'H,3'H,5'H-dispiro[cyclopropane-1,2'-pyrrolizine-6',1"-cyclopropan]-7a'(7'H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (27mg, crude). ES-API: [M+H]⁺= 761.3.

Step 6: Under ice-water bath condition, 4 M hydrochloric acid-dioxane solution (3 mL) was slowly added to a solution of tert-butyl (5S,5aS,6S,9R)-12-((1'H,3'H,5'H-dispiro[cyclopropane-1,2'-pyrrolizine-6',1"-cyclopropan]-7a'(7'H)-yl)methoxy)-2-(8-ethynyl-7-fluoronaphthalen-1-yl)-1-fluoro-5-mediyl-5a,6,7,8,9,10-hexahydro-5H-4-oxa-3,10a,11,13,14-pentaaza-6,9-methanonaphtho[1,8-ab]heptalene-14-carboxylate (27mg, crude) in acetonitrile (5.0mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic condition: 15ml-35-85-13min) to obtain a pale yellow solid (5S,5aS,6S,9R)-12-((1'H,3'H,5'H-dispiro[cyclopropane-1,2'-pyrrolizine-6',1"-cyclopropan]-7a'(7H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z628, 2.68mg, total yield for 2 steps: 18.6 %). ES-API: [M+H]⁺= 661.3.

### Example 40 Synthesis of 4-cycloprnpyl-3-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)-2,5-dimethylaniline (Z717)

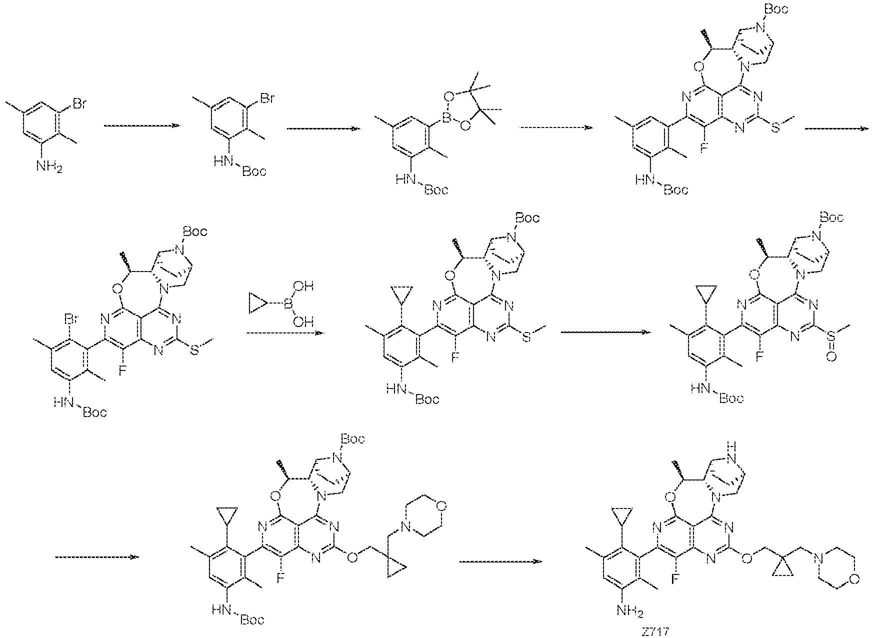

Step 1: At room temperature, 3-bromo-2,5-dimethylaniline (1.0 g, 4.998 mmol) and di-tert-butyl dicarbonate (1.42 g, 6.497 mmol) were successively added to a solution of sodium hydroxide (2M, 20 mL). The reaction was proceeded at 100°C for 1 hour. Upon completion of the reaction, the mixture was extracted with ethyl acetate (200 mL × 2) and saturated brine (80 mL). The combined ethyl acetate phases were dried over anhydrous sodium sulfate, filtered, and the filtrate was concentrated under reduced pressure. The crude product was purified by silica gel column chromatography (0-50% ethyl acetate/petroleum ether) to obtain (3-bromo-2,5-dimethylphenyl)tert-butyl carbamate (1.40 g, yield 93.31%). ES-API: [M-55+H]⁺=245.9.

Step 2: (3-bromo-2,5-dimethylphenyl)tert-butyl carbamate (0.9 g, 2.998 mmol), pinacol boronic ester (1.52 g, 5.996 mmol), potassium acetate (1.173 g, 11.99 mmol), and [1,1'-bis(diphenylphosphino)ferrocene]dichloropalladium(II) (0.323 g, 0.45 mmol) were added to N,N-dimethylformamide (30 mL). The mixture was purged four times with nitrogen and reacted at 80°C for 2 hours. Upon completion of the reaction, the mixture was extracted with ethyl acetate (200 mL X 2) and saturated brine (80 mL). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain (2,5-dimethyl-3-(4,4,5,5-tetramethyl-1,3,2-dioxaborolane-2-yl)phenyl)tert-butyl carbamate (490 mg, yield 47%). ES-API: [M-55+H]⁺=292.2.

Step 3: To a solution of tert-butyl (5S,5aS,6S,9R)-2-chloro-1-fluoro-5-methyl-12-(methylthio)-5a,6,7,8,9,10-hexahydro-5H-4-oxygen-3,10a,11,13,14-pentaaza-6,9 methanonaphtho[1,8-ab]heptalene-14-carboxylate (350 mg, 0.726 mmol) and (2,5-dimethyl-3-(4,4,5,5-tetramethyl-1,3,2-dioxaborolane-2-yl)phenyl)tert-butyl carbamate (0.490 g, 1.411 mmol) in tetrahydrofuran (30 mL) and water (3 mL), potassium phosphate (0.616 g, 2.90 mmol) and methanesulfonic acid [butylbis(1-adamantyl)phosphine](2-amino-1,1'-biphenyl)palladium(II) (79.3 mg, 0.109 mmol) were added. The mixture was bubbled with nitrogen and reacted in an oil bath at 80°C for 3 hours. Upon completion of the reaction, the mixture was extracted with ethyl acetate (30 mL X 2) and saturated brine (30 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-2-(3-((tert-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (570 mg, crude), a yellow solid. ES-API: [M+H]⁺=667.2.

Step 4: At 0°C, tert-butyl (5S,5aS,6S,9R)-2-(3-((tert-butoxycarbonyl)amino)-2,5-dimethylphenyl)-1-fluoro-5-methyl-12-(methylthio)-5a,6,7,8,9, 10-hexahydro-5H-4-oxa-3, 10a, 11, 13,14-pentaaza-6,9-methanonaphtho[1,8-ab]heptalene-14-carboxylate (483.0 mg, 0.726 mmol) was added to dichloromethane (20 mL), followed by N-bromosuccinimide (168.0 mg, 0.944 mmol). The reaction was proceeded at this temperature for 0.5 hours. Upon completion of the reaction, the mixture was extracted with ethyl acetate (100 mL × 2) and saturated brine (100 mL). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain tert-butyl (5S,5aS,6S,9R)-2-(2-bromo-5-((tert-butoxycarbonyl)amino)-3,6-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, yield 74.0%). ES-API: [M+H]⁺=745.1.

Step 5: To a mixture of tert-butyl (SS,SaS,6S,9R)-2-(2-bromo-5-((tert-butoxycarbonyl)amino)-3,6-dimethylphenyl)-1 -fluoro-5-methyl- 12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, 0.536 mmol) and cyclopropylboronic acid (230.0 mg, 2.68 mmol) in toluene (50 mL) and water (5 mL), potassium phosphate (455.4 mg, 2.146 mmol), 2-dicyclohexylphosphino-2',6'-dimethoxybiphenyl (66.06 mg, 0.161 mmol), and palladium acetate (24.04 mg, 0.107 mmol) were added. The mixture was bubbled with nitrogen and reacted in an oil bath at 105°C for 2 hours. Upon completion of the reaction, the mixture was extracted with ethyl acetate (200 mL X 2) and saturated brine (100 mL X 4). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain tert-butyl (SS,SaS,6S,9R)-2-(3-((tert-butoxycarbonyl)amino)-6-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (520 mg, crude), a yellow solid. ES-API: [M+H]⁺=707.2.

Step 6: At 0°C, m-chloroperoxybenzoic acid (188.3 mg, 1.09 mmol) was added to a solution of tert-butyl (5S,5aS,6S,9R)-2-(3-((tert-butoxycarbonyl)amino)-6-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (379.0 mg, 0.537 mmol) in dichloromethane (30.0 mL). The reaction mixture was stirred under an ice-water bath for 1 hour. The reaction mixture was quenched with saturated sodium bisulfite solution and extracted with dichloromethane (100 mL X 2). The organic layer was dried over anhydrous sodium sulfate, filtered, and concentrated to obtain a crude product, which was then purified by silica gel column chromatography (0-10% methanol/dichloromethane) to obtain tert-butyl (SS,SaS,6S,9R)-2-(3-((tert-butoxycarbonyl)amino)-6-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, crude), a yellow solid. ES-API: [M+H]⁺=723.3.

Step 7: At 0°C, sodium hydride (70.0 mg, 1.754 mmol) was added to a solution of (1-(morpholinomethyl)cyclopropyl)methanol (100 mg, 0.5850 mmol) in tetrahydrofuran (10.0 mL). After the mixture stirred at this temperature for 10 minutes, a solution of tert-butyl (SS,SaS,6S,9R)-2-(3-((tert-butoxycarbonyl)amino)-6-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (390 mg, crude) in tetrahydrofuran (5 mL) was added. The reaction mixture was stirred at 0°C for 10 minutes, then at room temperature for 0.5 hours. Upon completion of the reaction, the reaction mixture was quenched with saturated ammonium chloride solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-(3-((tert-butoxycarbonyl)amino)-6-cyclopropyl-2,5-dimethylphenyl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, crude), a yellow solid. ES-API: [M+H]⁺=830.3.

Step 8: Under an ice-water bath, 4 M hydrochloric acid dioxane solution (5.0 mL, 4M, 20.0 mmol) was slowly added to a dichloromethane (10.0 mL) solution of tert-butyl (5S,5aS,6S,9R)-2-(3-((tertbutoxycarbonyl)amino)-6-cyclopropyl-2,5-dimethylphenyl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, crude). The reaction mixture was stirred at room temperature for 1.0 hour. Upon completion of the reaction (monitored by LCMS), the reaction mixture was concentrated to dryness under reduced pressure, and the residue was purified by preparative HPLC (ammonium bicarbonate method 2) to obtain a white solid 4-cyclopropyl-3-((5S,5aS,6S,9R)-1-fluoro-5-methyl-12-((1-(morpholinomediyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)-2,5-dimethylaniline (Z717, 5.0 mg, yield: 1.5%). ES-API: [M+H]⁺= 630.4.

### Example 41: Synthesis of (SS,SaS,bS,9R)-2-(&ethynyl-7-fluoronaphthalen-1-yl)-1-fluoro-5-methyl-12-(((1aR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene (Z605)

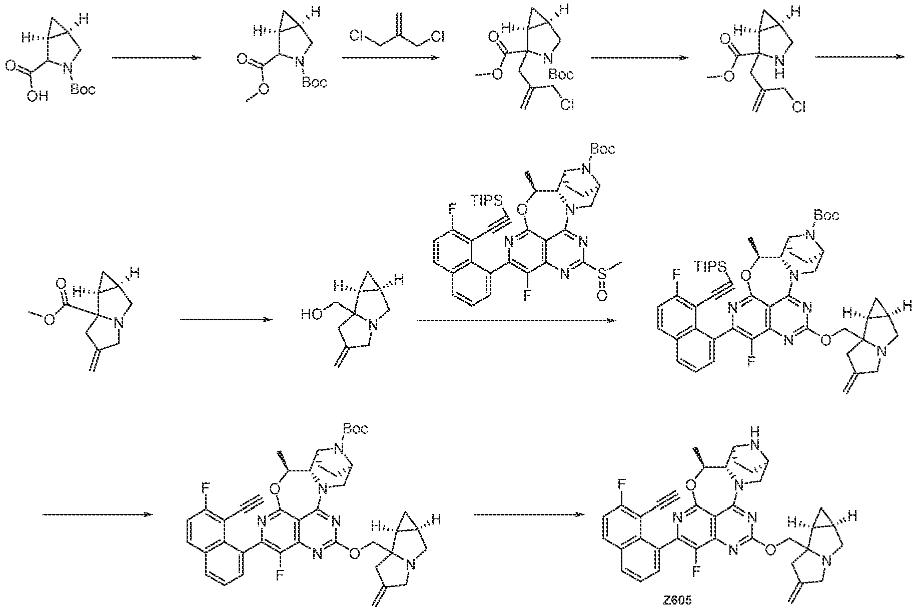

Step 1: Potassium carbonate (548 mg, 3.960 mmol) and iodomethane (375 mg, 2.640 mmol) were added to a solution of (1S,SR)-3-(tert-butoxycarbonyl)-3-azabicyclo[3.1.0]hexane-2-carboxylic acid (300 mg, 1.320 mmol) in N,N-dimethylformamide (10 mL), and the mixture was stirred at room temperature for 2 hours. Upon completion of the reaction, as monitored by LCMS, ethyl acetate (20 mL) and water (20 mL) were added. The separated organic phase was washed three times with saturated brine, dried over anhydrous sodium sulfate, filtered, and the filtrate was concentrated under reduced pressure to obtain the crude product 3-(tert-butyl)-2-methyl(1S,5R)-3-azabicyclo[3.1.0]hexane-2,3-dicarboxylate (318 mg). ES-API: [M-55]⁺= 186.1.

Step 2: Under nitrogen protection, 3-(tert-butyl)-2-methyl(1S,5R)-3-azabicyclo[3.1.0]hexane-2,3-dicarboxylate (318 mg, 1.318 mmol) was dissolved in anhydrous tetrahydrofuran (10 mL) and cooled in a dry ice-acetone bath. A solution of bis(trimethylsilyl)aminolithium (2.6 mL, 2.636 mmol, 1 M in tetrahydrofuran) was slowly added dropwise to the solution, and stirred at this temperature for 1 hour. Then, a solution of 3-chloro-2-(chloromethyl)allylaldehyde (823 mg, 6.590 mmol) in anhydrous tetrahydrofuran (5 mL) was slowly added through a syringe, and the mixture was stirred at -78 °C for 1 hour and then warm to room temperature and stirred overnight. Upon completion of the reaction, monitored by LCMS, saturated ammonium chloride solution (20 mL) was added to quench the reaction. The mixture was extracted with ethyl acetate (15 mL X 3), washed with saturated brine (15 mL X 3), dried over anhydrous sodium sulfate, filtered, and concentrated under reduced pressure. The crude product was purified by flash silica gel column chromatography (ethyl acetate: petroleum ether = 0-15%) to obtain 3-(tert-butyl)-2-methyl(1S,SR)-2-(2-(chloromethyl)allyl)-3-azabicyclo[3.1.0]hexane-2,3-dicarboxylate (180 mg, yield: 41%). ES-API: [M-55]⁺= 274.

Step 3: Under an ice-water bath, a 4M hydrochloric acid/dioxane solution (2 mL) was slowly added to a solution of 3-(tert-butyl)-2-methyl(1S,5R)-2-(2-(chloromethyl)allyl)-3-azabicyclo[3.1.0]hexane-2,3-dicarboxylate (180 mg, 0.546 mmol) in acetonitrile (2 mL). The mixture was stirred at 0 °C for 1 hour. Upon completion of the reaction, monitored by LCMS, the reaction mixture was concentrated under reduced pressure to obtain the crude product methyl (1S,5R)-2-(2-(chloromethyl)allyl)-3-azabicyclo[3.1.0]hexane-2-carboxylate (125 mg). ES-API: [M+H]⁺= 230.2.

Step 4: At room temperature, sodium bicarbonate (229 mg, 2.721 mmol) and potassium iodide (26 mg, 0.054 mmol) were added to a solution of methyl (1S,SR)-2-(2-(chloromethyl)allyl)-3-azabicyclo[3.1.0]hexane-2-carboxylate (125 mg, 0.544 mmol) in acetonitrile (10 mL). The mixture was stirred overnight at room temperature. Upon completion of the reaction, monitored by LCMS, the mixture was filtered and washed with acetonitrile. The filtrate was concentrated under reduced pressure, and the crude product was purified by flash silica gel column chromatography (methanol: dichloromethane = 0~10%) to obtain methyl (1aR,6bS)-5-methylenehexahydrocyclopropa[a]pyrrolizine-6a(4H)-carboxylate (89 mg, yield: 85 %). ES-API: [M+H]⁺= 194.2.

Step 5: Under nitrogen protection, methyl (1aR,6bS)-5-methylenehexahydrocyclopropa[a]pyrrolizine-6a(4H)-carboxylate (89 mg, 0.461 mmol) was dissolved in anhydrous tetrahydrofuran (5 mL) and placed in an ice-water bath. A solution of lithium aluminium hydride (1 mL, 0.921 mmol, 1 M in tetrahydrofuran) was slowly added dropwise to the solution, and stirred at this temperature for 0.5 hours. Upon completion of the reaction, monitored by LCMS, sodium dodecyl sulfate was added to quench the reaction, and the mixture was stirred at room temperature for 0.5 hours, filtered, and the filtrate concentrated under reduced pressure to obtain the crude product ((1aR,6bS)-5-methylenehexahydrocyclopropa[a]pyrrolizin-ba(4H)-yl)methanol (76 mg). ES-API: [M+H]⁺= 166.1.

Step 6: ((1aR,6bS)-5-methylenehexahydrocyclopropa[a]pyrrolizin-6a(4H)-yl)methanol (31 mg, 0.190 mmol) was dissolved in anhydrous tetrahydrofuran (5 mL), and under an ice-water bath, sodium hydride (7.6 mg, 0.190 mmol) was added. The reaction was proceeded for 30 minutes in the ice-water bath. Then, a solution of tert-butyl (SS,SaS,6S,9R)-1-fluoro-2-(7-fluoro-8-((triisopropylsilyl)ethγnyl)naphthalen-1-yl)-5-methyl-12-(methylsulfinyl)-Sa,6,7,8,9,10-hexahydro-5H-4-oxa-3,10a,11,1 3,14-pentaaza-6,9-methanonaphtho[1,8-ab]heptalene-14-carboxylate (50 mg, 0.063 mmol) in tetrahydrofuran (2 mL) was slowly added dropwise to the reaction mixture, and stirred at 0°C for 30 minutes. Upon completion of the reaction (monitored by LCMS), ethyl acetate (10 mL) was added to dilute the reaction mixture, which was then washed with saturated ammonium chloride solution (10 mL × 2) and saturated brine (10 mL × 3), dried over anhydrous sodium sulfate, filtered, and concentrated under reduced pressure to obtain the crude product tert-butyl (5S,5aS,6S,9R)-1-fluoro-2-(7-fluoro-8-((triisopropylsilyl)ethynyl)naphthalen-1-yl)-5-methyl-12-(((1aR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (48 mg). ES-API: [M+H]⁺= 889.3.

Step 7: Cesium fluoride (82 mg, 0.540 mmol) was added to a solution of tert-butyl (5S,5aS,6S,9R)-1-fluoro-2-(7-fluoro-8-((triisopropylsilyl)ethynyl)naphthalen-1-yl)-5-methyl-12-(((1aR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (48 mg, 0.054 mmol) in N,N-dimethylformamide (2 mL). The reaction mixture was stirred at room temperature for 1 hour. Upon completion of the reaction (monitored by LCMS), the reaction mixture was diluted with ethyl acetate (15 mL), washed with water (15 mL X 2), saturated brine (15 mL X 3), dried over anhydrous sodium sulfate, filtered, and concentrated to obtain the crude product tert-butyl (5S,5aS,6S,9R)-2-(8-ethynyl-7-fluoronaphthalen-1-yl)-1-fluoro-5-methyl-12-(((1aR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (39 mg). ES-API: [M+H]⁺= 733.3.

Step 8: Under an ice-water bath, a 4M hydrochloric acid-dioxane solution (2 mL) was slowly added to a solution of tert-butyl (5S,5aS,6S,9R)-2-(8-ethynyl-7-fluoronaphthalen-1-yl)-1-fluoro-5-methyl-12-(((laR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate ((39 mg, 0.053 mmol) in acetonitrile (1 mL). The reaction mixture was stirred at 0°C for 0.5 hours. Upon completion of the reaction (monitored by LCMS), the reaction mixture was concentrated to dryness, and the residue was purified by preparative HPLC (bicarbonate method 2) (chromatographic condition: 15ml-50-70-13min) to obtain a pale white solid (SS,SaS,6S,9R)-2-(8-ethynyl-7-fluoronaphthalen-1-yl)-1-fluoro-5-methyl-12-(((1aR,6bS)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene (Z605, 1.05 mg, yield: 3 %). ES-API: [M+H]⁺= 633.3.

### Example 42: Synthesis of (5S,5aS,6S,9R)-12-((2-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z608)

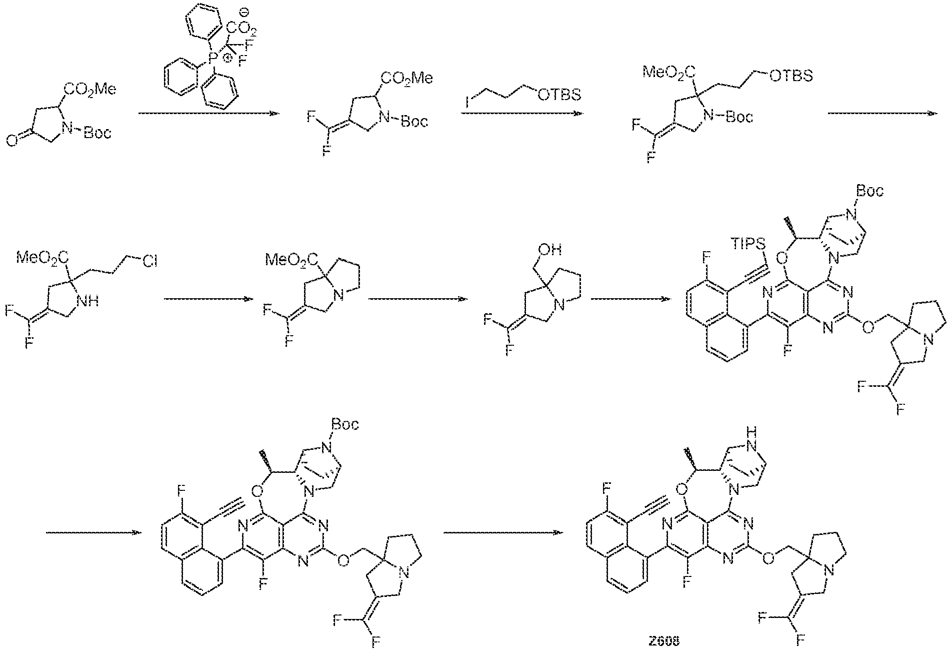

Step 1: Under nitrogen protection, 1-(tert-butyl)-2-methyl-4-oxopyrrolidine-1,2-dicarboxylate (2.5 g, 10.28 mmol), (triphenylphosphonium) difluoroacetate inner salt (9.5 g, 26.75 mmol), and N,N-dimethylformamide (30 mL) were added to a round-bottom flask. The mixture was stirred at 80°C for 6 hours. Water (100 mL) and ethyl acetate (50 mL X 2) were added for extraction. The organic phase was washed with saturated brine (50 mL X 2), dried over anhydrous sodium sulfate, concentrated, and the crude product was purified by flash silica gel column chromatography (ethyl acetate/petroleum ether: 0-8%) to obtain 1-(tert-butyl) 2-methyl 4-(difluoromethylene)pyrrolidine-1,2-dicarboxylate (1.47 g, yield 51%), a colorless oily substance. ES-API: [M+Na]⁺=300.1.

Step 2: To a round-bottom flask, 1-(tert-butyl) 2-methyl 4-(difluorornothylene)pyrrolidine-1,2-dicarboxylate (1.4 g, 5.05 mmol), tert-butyl (3-iodopropoxy)dimethylsilane (3.03 g, 10.11 mmol), and tetrahydrofuran (20 mL) were added. Under nitrogen protection, the mixture was cooled to - 78°C. While stirring, bis(trimethylsilyl)amine potassium solution (10.11 mL, 10.11 mmol, 1M in tetrahydrofuran) was slowly added dropwise to the reaction mixture. After the addition was complete, the reaction mixture was stirred at -78°C for 30 minutes. The ice water bath was removed. The reaction mixture was poured into a saturated ammonium chloride solution (100 mL), extracted with ethyl acetate (50 mL × 3), washed with saturated brine, dried over anhydrous sodium sulfate, filtered, concentrated, and purified by flash silica gel column chromatography (ethyl acetate/petroleum ether: 0-7%) to obtain 1-(tert-butyl) 2-methyl 2-(3-((tert-butyldimethylsilanyl)oxy)propyl)-4-(difluoromethylene)pyrrolidine-1,2-dicarboxylate (900 mg, yield: 40%), a colorless oil. ES-API: [M+Na]⁺=472.3.

Step 3: Under nitrogen protection, 1-(tert-butyl) 2-methyl 2-(3-((tertbutyldimethylsilanyl)oxy)propyl)-4-(difluoromethylene)pyrrolidine-1,2-dicarboxylate (200 mg, 0.44 mmol), dichloromethane (1 mL), 4M hydrochloric acid/dioxane solution (0.6 mL), and dichlorosulfoxide (3 mL) were successively added to a reaction flask. The reaction mixture was stirred at 50°C for 4 hours. The reaction mixture was concentrated, and dichloromethane (30 mL) was added, washed with saturated bicarbonate solution (50 mL). The organic phase was dried over anhydrous sodium sulfate, and concentrated to obtain methyl 2-(3-chloropropyl)-4-(difluoromethylene)pyrrolidine-2-carboxylate (111 mg, crude product), a yellow oily substance. ES-API: [M+H]⁺=254.1.

Step 4: Methyl 2-(3-chloropropyl)-4-(difluoromethylene)pyrrolidine-2-carboxylate (220 mg, crude product), potassium carbonate (119 mg, 0.86 mmol), potassium iodide (143 mg, 0.86 mmol), and N,N-dimethylformamide (5 mL) were added to a reaction flask. The reaction mixture was stirred at 85°C for 2 hours. Water (50 mL) was added to the reaction mixture, and the mixture was extracted with ethyl acetate (30 mL X 3). The organic phase was washed with saturated brine, dried over anhydrous sodium sulfate, filtered, and concentrated. The crude product was purified by flash silica gel column chromatography (ethyl acetate/petroleum ether: 0-40%) to obtain methyl 2-(difluoromethylene)tetrahydro-1H-pyrrolizine-7a(5H)-carboxylate (120 mg, total yield for 2 steps: 64%). ES-API: [M+H]⁺=218.1.

Step 5: Under nitrogen protection, methyl 2-(difluoromethylene)tetrahydro-1H-pyrrolizine-7a(5H)-carboxylate (100 mg, 0.46 mmol) and tetrahydrofuran (15 mL) were added to a round-bottom flask. The mixture was cooled to 0°C, and lithium borohydride solution (0.46 mL, 0.92 mmol, 2M in tetrahydrofuran) was slowly added dropwise. After the addition was complete, the reaction mixture was stirred at 0°C for 30 minutes. 4M hydrochloric acid (1 mL) was slowly added, and the mixture was stirred at room temperature for 30 minutes. 2M sodium hydroxide solution (5 mL) and water (20 mL) were added. The mixture was extracted with dichloromethane/methanol (10:1, 30 mL X 3). The organic phase was dried over anhydrous sodium sulfate and concentrated to obtain (2-(difluoromethylene)tetrahydro-1H-pyrrolizin-7a(5H)-yl)methanol (40 mg, yield: 46%), a yellow oily substance. ES-API: [M+H]⁺=190.1.

Step 6: (2-(difluoromethylene)tetrahydro-1H-pyrrolizin-7a(5H)-yl)methanol (44.0 mg, 0.233 mmol) was dissolved in anhydrous tetrahydrofuran (5 mL), under an ice water bath, sodium hydride (20 mg, 0.50 mmol) was added. The reaction was proceeded at 0°C for 30 minutes. tert-butyl (5 S,5aS,6S,9R)-1 -fluoro-2-(7-fluoro-8-((triisopropylsilyl)ethynyl)naphthalen-1 -yl)-5-methyl-12-(methylsulfonyl)-5a,6,7,8,9,10-hexahydro-5H-4-oxygen-3,10a,11,13,14-pentaaza-6,9-methanonaphtho[1,8-ab]heptalene-14-carboxylate (50 mg, 0.063 mmol) in tetrahydrofuran (2 mL) was slowly added to the reaction mixture at 40°C for 30 minutes. Upon completion of the reaction (monitored by LCMS), the reaction mixture was diluted with ethyl acetate (50 mL), washed with saturated ammonium chloride solution (15 mL X 2) and saturated brine (50 mL X 3), dried over anhydrous sodium sulfate, filtered, and purified by silica gel column chromatography (dichloromethane: methanol = 15:1) to obtain tert-butyl (5S,5aS,6S,9R)-12-((2-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate. ES-API: [M+H]⁺= 913.3.

Step 7: Cesium fluoride (96.0 mg, 0.63 mmol) was added to a solution of tert-butyl (5S,5aS,6S,9R)-12-((2-(difluoromethylene)tetrahydro-1H-pyrrolizin-7a(5H-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, crude product) in N,N-dimethylformamide (5.0 mL). The reaction mixture was stirred at room temperature for 2 hours. Upon completion of the reaction (monitored by LCMS), the reaction mixture was diluted with ethyl acetate (60 mL), washed with water (30 mL X 2) and saturated brine (50 mL X 3), dried over anhydrous sodium sulfate, filtered, and concentrated to obtain a crude product of tert-butyl (5S,5aS,6S,9R)-12-((2-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (65 mg, crude product). ES-API: [M+H]⁺= 757.3.

Step 8: A solution of 4 M hydrochloric acid in dioxane (3 mL) was slowly added to a solution of tert-butyl (5S,5aS,6S,9R)-12-((2-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (65 mg, crude product) in acetonitrile (5.0 mL) under an ice water bath. The reaction mixture was stirred at 0°C for 0.5 hours. Upon completion of the reaction (monitored by LCMS), the reaction mixture was concentrated to dryness, and the residue was purified by preparative HPLC (ammonium bicarbonate method 2) (chromatographic condition: 15 ml-35-85-13 min) to obtain a light yellow solid of (5S,5aS,6S,9R)-12-((2-(difhioromethylene)tetrahydro-1H-pyrrolizin-7a(5H)-yl)methoxy)-2-(8-ethynyl-7-fluoronaphthalen-1-yl)-1-fluoro-5-methyl-Sa,6,7,8,9,10-hexahydro-SH-4-oxa.-3,10a,11,13,14-pentaaza-6,9-methanonaphtho[1,8-ab]heptalene (Z608, 1.0mg, total yield for 3 steps: 2.4 %). ES-API: [M+H]⁺= 657.3.

### Example 43: Synthesis of (5S,5aS,6S,9R)-2-(&ethynyl-7-fluoronaphthalen-1-yl)-1-fluoro-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z604)

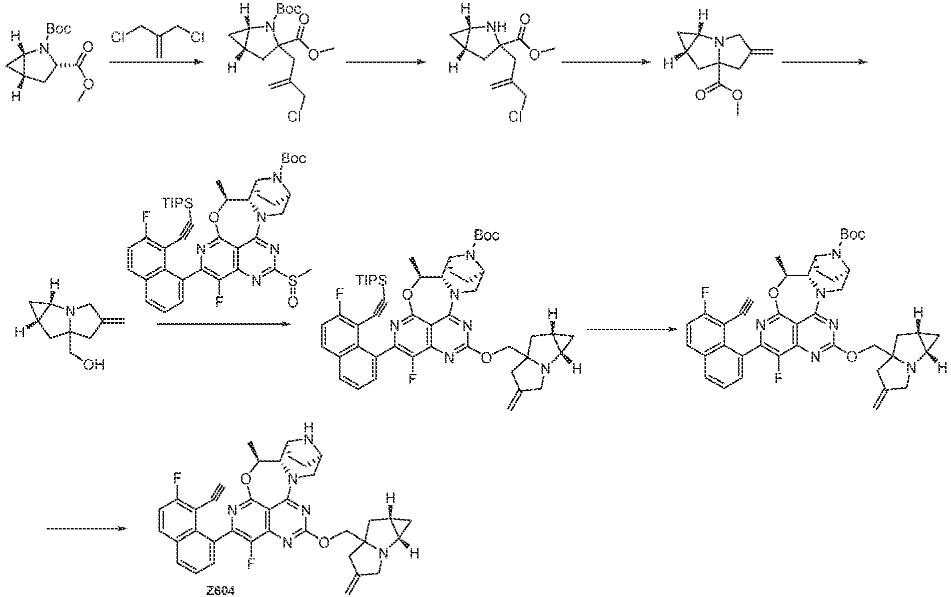

Step 1: Under nitrogen protection, 2-(tert-butyl) 3-methyl (1S,3S,5S)-2-azabicyclo[3.1.0]hexane-2,3-dicarboxylate (1.0 g, 4.1 mmol) was dissolved in anhydrous tetrahydrofuran (10 mL), and the mixture was cooled in a dry ice-acetone bath. Bis(trimethylsilyl)aminolithium solution (8.3 mL, 8.3 mmol, 1 M in tetrahydrofuran) was slowly added to the above solution and stirred at this temperature for 1 hour. A solution of 3-chloro-2-(chloromethyl)propen- 1-yne (2.59 g, 20.7 mmol) in anhydrous tetrahydrofuran (10 mL) was slowly added to the above solution using a syringe, and the reaction mixture was further stirred at -78°C for 1 hour. Upon completion of the reaction (monitored by LCMS), the reaction mixture was quenched with saturated ammonium chloride solution (50 mL), extracted with ethyl acetate (50 mL × 3), and the organic phase was washed with saturated brine (50 mL X 3), dried over anhydrous sodium sulfate, filtered, and concentrated. The crude product was purified by flash silica gel column chromatography (petroleum ether: ethyl acetate = 20:1) to obtain 2-(tert-butyl) 3-methyl (1S,5S)-3-(2-(chloromethyl)allyl)-2-azabicyclo[3.1,0]hexane-2,3-dicarboxylate (1.28 g, yield: 93%). ES-API: [M-56]⁺= 274.1.

Step 2: At room temperature, a solution of 4 M hydrochloric acid in dioxane (15 mL) was slowly added to a solution of 2-(tert-butyl) 3-methyl (1S,5S)-3-(2-(chloromethyl)allyl)-2-azabicyclo[3.1.0]hexane-2,3-dicarboxylate (1.28 g, 3.7 mmol) in acetonitrile (3 mL). The mixture was stirred at room temperature for 0.5 hours. Upon completion of the reaction (monitored by LCMS), the reaction mixture was concentrated under reduced pressure to obtain methyl (1S,5S)-3-(2-(chloromethyl)allyl)-2-azabicyclo[3.1.0]hexane-3-carboxylate (0.89 g, yield: 100%). ES-API: [M+H]⁺= 230.2.

Step 3: At room temperature, sodium bicarbonate (1.6 g, 19.5 mmol) and potassium iodide (65 mg, 0.39 mmol) were added to a solution of methyl (1S,5S)-3-(2-(chloromethyl)allyl)-2-azabicyclo[3.1.0]hexane-3-carboxylate (0.89 g, 3.9 mmol) in acetonitrile (10 mL). The mixture was stirred at room temperature for 1.5 hours. Upon completion of the reaction (monitored by LCMS), the mixture was filtered, washed with acetonitrile. The filtrate was concentrated under reduced pressure, and the crude product was purified by flash silica gel column chromatography (petroleum ether: ethyl acetate = 15:1) to obtain methyl (1aS,6aS)-4-methylenehexahydrocyclopropa[b]pyrrolizine-5a(3H)-carboxylate (510 mg, yield: 68%). ES-API: [M+H]⁺=194.1.

Step 4: Under nitrogen protection, methyl (1aS,6aS)-4-methylenehexahydrocyclopropa[b]pyrrolizine-5a(3H)-carboxylate (510 mg, 2.6 mmol) was dissolved in anhydrous tetrahydrofuran (10 mL), and the mixture was placed in an ice water bath. Lithium aluminium hydride solution (5.4 mL, 5.4 mmol, 1 M in tetrahydrofuran) was slowly added dropwise to the above solution and stirred at this temperature for 0.5 hours. Upon completion of the reaction (monitored by LCMS), sodium sulfate decahydrate (510 mg) was added to quench the reaction, and the mixture was stirred at room temperature for 0.5 hours, then filtered. The filtrate was concentrated under reduced pressure to obtain ((1aS,6aS)-4-methylenehexahydrocyclopropa[b]pyrrolizin-5a(3H)-yl)methanol (410 mg, yield: 93%). ES-API: [M+H]⁺= 166.2.

Step 5: ((1aS,6aS)-4-methylenehexahydrocyclopropa[b]pyrrolizin-5a(3H)-yl)methanol (47 mg, 0.29 mmol) was dissolved in anhydrous tetrahydrofuran (5 mL), and under an ice water bath, sodium hydride (11 mg, 0.29 mmol) was added. The reaction was proceeded under an ice water bath for 30 minutes. A solution of tert-butyl (SS,SaS,6S,9R)-1-fluoro-2-(7-fluoro-8-((triisopropylsilyl)ethγnyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (75 mg, 0.095 mmol) in tetrahydrofuran (2 mL) was added dropwise to the reaction mixture, and the reaction was proceeded at 40°C for 30 minutes. Upon completion of the reaction (monitored by LCMS), the reaction mixture was diluted with ethyl acetate (20 mL), washed with saturated ammonium chloride solution (15 mL X 2) and saturated brine (15 mL X 3), dried over anhydrous sodium sulfate, filtered, and concentrated under reduced pressure to obtain a crude product of tert-butyl (5S,5aS,6S,9R)-1-fluoro-2-(7-fluoro-8-((triisopropylsilyl)ethynyl)naphthalen-1-yl)-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a, 11,13,14-pentaaza-6,9-methanonaphtho[1,8-ab]heptalene-14-carboxylate (85 mg, crude product). ES-API: [M+H]⁺= 889.5.

Step 6: Cesium fluoride (145 mg, 0.9 mmol) was added to a solution of tert-butyl (5S,5aS,6S,9R)-1-fluoro-2-(7-fluoro-8-((triisopropylsilyl)ethynyl)naphthalen-1-yl)-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (85 mg, 0.09 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL), washed with water (15 mL × 2), and saturated brine (15 mL X 3), dried over anhydrous sodium sulfate, filtered, and concentrated to obtain a crude product of tert-butyl (5S,5aS,6S,9R)-2-(8-ethynyl-7-fluoronaphthalen-1-yl)-1-fluoro-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (69 mg, crude product). ES-API: [M+H]⁺= 733.3.

Step 7: Under an ice water bath, 4 M hydrochloric acid-dioxane solution (3 mL) was slowly added to a solution of tert-butyl (5S,5aS,6S,9R)-2-(8-ethynyl-7-fluoronaphthalen-1-yl)-1-fluoro-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (69 mg, 0.09 mmol) in acetonitrile (1 mL). The reaction mixture was stirred at 0°C for 0.5 hours. Upon completion of the reaction (monitored by LCMS), the reaction mixture was concentrated to dryness, and the residue was purified by preparative HPLC (bicarbonate method 2) (chromatographic condition: 15 mL - 35-85 - 13 min) to obtain a pale yellow solid of (5S,5aS,6S,9R)-2-(8-ethynyl-7-fhioronaphthalen-1-yl)-1-fluoro-5-methyl-12-(((1aS,6aS)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z604, 10 mg, yield: 16 %). ES-API: [M+H]⁺= 633.3.

### Example 44: Synthesis of (5S,5aS,6S,9R)-2-(8-ethynyl-7-fluoronaphthalen-1-yl)-1-fluoro-5-methyl-12-(((1aR,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z603)

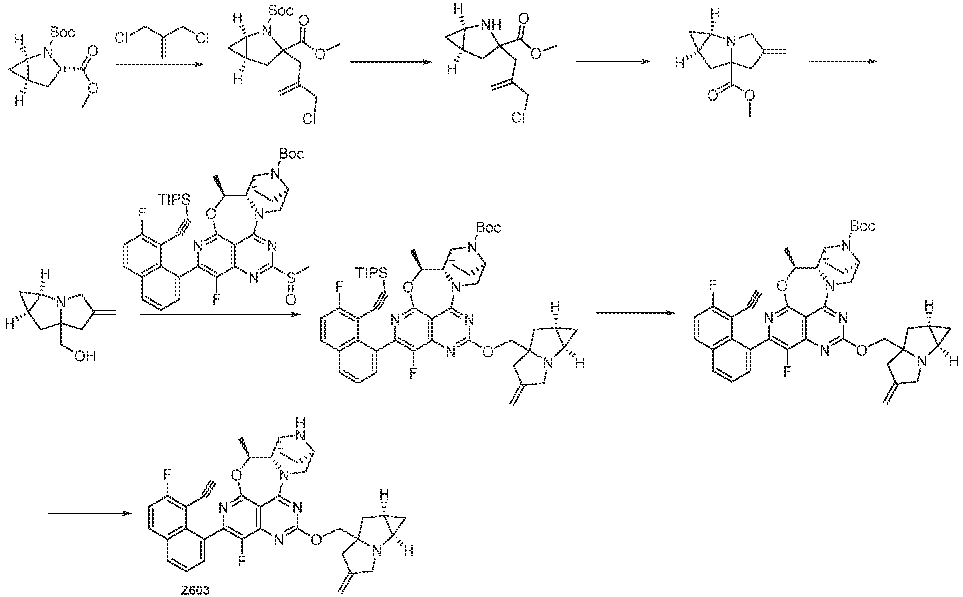

Step 1: Under nitrogen protection, 2-(tert-butyl) 3-methyl (1R,3S,5R)-2-azabicyclo[3.1.0]hexane-2,3-dicarboxylate (1.0 g, 4.1 mmol) was dissolved in anhydrous tetrahydrofuran (10 mL), and the mixture was placed in a dry ice-acetone bath. Bis(trimethylsilyl)amine lithium solution (8.3 mL, 8.3 mmol, 1M in tetrahydrofuran) was slowly added dropwise to the solution, stirred at this temperature for 1 hour, then 3-chloro-2-(chloromethyl)propen-1-ene (2.59 g, 20.7 mmol) in anhydrous tetrahydrofuran (10 mL) was slowly added via syringe, and the reaction mixture was stirred at -78°C for another 1 hour. Upon completion of the reaction (monitored by LCMS), the reaction was quenched with saturated ammonium chloride solution (50 mL), extracted with ethyl acetate (50 mL X 3), washed with saturated brine (50 mL X 3), dried over anhydrous sodium sulfate, filtered, and concentrated. The crude product was purified by flash silica gel column chromatography (petroleum ether: ethyl acetate = 20: 1) to obtain 2-(tert-butyl) 3-methyl (1R,5R)-3-(2-(chloromethyl)allyl)-2-azabicyclo[3.1.0]hexane-2,3-dicarboxylate (1.3g, yield: 95%). ES-API: [M-56]⁺= 274.1.

Step 2: At room temperature, 4 M hydrochloric acid-dioxane solution (10 mL) was slowly added to a solution of 2-(tert-butyl) 3-methyl (1R,SR)-3-(2-(chloromethyl)allyl)-2-azabicyclo[3.1.0]hexane-2,3-dicarboxylate (1.3g, 3.7 mmol) in acetonitrile (3 mL), the mixture was stirred at room temperature for 0.5 hours. Upon completion of the reaction (monitored by LCMS), the reaction mixture was concentrated under reduced pressure to obtain methyl (1R,5R)-3-(2-(chloromethyl)allyl)-2-azabicyclo[3.1.0]hexane-3-carboxylate (0.91 g, yield: 100%). ES-API: [M+H]⁺= 230.2.

Step 3: At room temperature, sodium bicarbonate (1.6 g, 19.5 mmol) and potassium iodide (65 mg, 0.39 mmol) were added to a solution of methyl (1R,SR)-3-(2-(chloromethyl)allyl)-2-azabicyclo[3.1.0]hexane-3-carboxylate (0.91 g, 3.9 mmol) in acetonitrile (10 mL), the mixture was stirred at room temperature for 1.5 hours. Upon completion of the reaction (monitored by LCMS), the mixture was filtered and washed with acetonitrile. The filtrate was concentrated under reduced pressure and the crude product was purified by flash silica gel column chromatography (petroleum ether: ethyl acetate = 15:1) to obtain methyl (1aR,6aR)-4-methylenehexahydrocyclopropa[b]pyrrolizine-5a(3H)-carboxylate (500 mg, yield: 65%). ES-API: [M+H]⁺=194.1.

Step 4: Under nitrogen protection, methyl (1aR,6aR)-4-methylenehexahydrocyclopropa[b]pyrrolizine-5a(3H)-carboxylate (500 mg, 2.6 mmol) was dissolved in anhydrous tetrahydrofuran (10 mL). The mixture was placed in an ice-water bath, and a solution of lithium aluminum hydride (5.4 mL, 5.4 mmol, 1 M in tetrahydrofuran) was slowly added dropwise. The mixture was stirred at this temperature for 0.5 hours. Upon completion of the reaction (monitored by LCMS), sodium sulfate decahydrate (500 mg) was added to quench the reaction, and the mixture was stirred at room temperature for 0.5 hours, and then filtered. The filtrate was concentrated under reduced pressure to obtain ((1aR,6aR)-4-methylenehexahydrocyclopropa[b]pyrrolizin-5a(3H)-yl)methanol (400 mg, yield: 93%). ES-API: [M+H]⁺= 166.2.

Step 5: ((1aR,6aR)-4-methylenehexahydrocyclopropa[b]pyrrolizin-5a(3H)-yl)methanol (47 mg, 0.29 mmol) was dissolved in anhydrous tetrahydrofuran (5 mL). Under an ice-water bath, sodium hydride (11 mg, 0.29 mmol) was added and the mixture reacted for 30 minutes. A solution of tert-butyl (5S,5aS,6S,9R)-1-fluoro-2-(7-fhioro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (75 mg, 0.095 mmol) in tetrahydrofuran (2 mL) was added dropwise to the reaction mixture and reacted at 40°C for 30 minutes. Upon completion of the reaction (monitored by LCMS), the reaction mixture was diluted with ethyl acetate (20 mL), washed with saturated ammonium chloride solution (15 mL X 2) and saturated brine (15 mL X 3), dried over anhydrous sodium sulfate, filtered, and concentrated under reduced pressure to obtain the crude product, tert-butyl (5S,5aS,6S,9R)-1-fluoro-2-(7-fluoro-8-((triisopropylsilyl)ethynyl)naphthalen-1-yl)-5-methyl-12-(((1aR,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (89 mg, crude product). ES-API: [M+H]⁺= 889.5.

Step 6: Cesium fluoride (145 mg, 0.9 mmol) was added to a solution of tert-butyl (5S,5aS,6S,9R)-1-fluoro-2-(7-fluoro-8-((triisopropylsilyl)ethynyl)naphthalen-1-yl)-5-methyl-12-(((1aR,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (89 mg, 0.1 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL) and washed with water (15 mL X 2) and saturated brine (15 mL X 3), dried over anhydrous sodium sulfate, filtered, and concentrated to obtain the crude product, tert-butyl (SS,SaS,6S,9R)-2-(8-ethynyl-7-fluoronaphthalen-1-yl)-1-fluoro-5-methyl-12-(((lalt,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (71 mg, crude product). ES-API: [M+H]⁺= 733.3.

Step 7: Under an ice-water bath, a solution of 4 M hydrochloric acid-dioxane (3 mL) was slowly added to a solution of tert-butyl (5S,5aS,6S,9R)-2-(8-ethynyl-7-fluoronaphthalen-1-yl)-1-fluoro-5-methyl-12-(((1aR,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (75 mg, 0.1 mmol) in acetonitrile (1 mL). The reaction mixture was stirred at 0°C for 0.5 hours. Upon completion of the reaction (monitored by LCMS), the reaction mixture was concentrated to dryness, and the residue was purified by preparative HPLC (Ammonium bicarbonate method 2) (chromatographic condition: 15 mL-35-85-13 min) to obtain a pale yellow solid, (5S,5aS,6S,9R)-2-(8-ethynyl-7-fluoronaphthalen-1-yl)-1-fluoro-5-methyl-12-(((1aR,6aR)-4-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z603, 4.5 mg, yield: 7 %). ES-API: [M+H]⁺= 633.3.

### Example 45: Synthesis of (5S,5aS,6S,9R)-2-(8-ethynyl-7-fluoronaphthalen-1-yl)-1-fluoro-5-methyl-12-((6'-methylenetetrahydrospiro[cyclopropane-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z602)

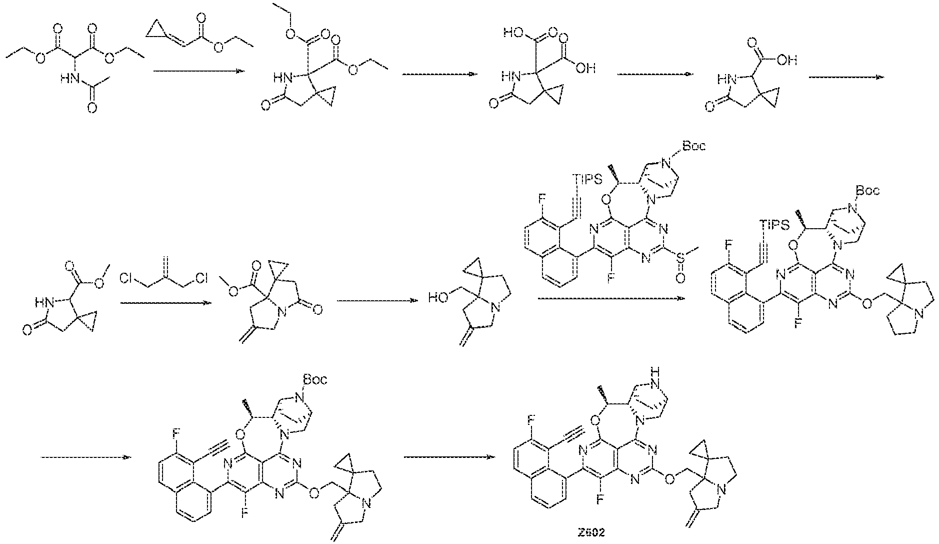

Step 1: Ethyl 2-cyclopropylacetate (1261 mg, 10.0 mmol) and diethyl 2-acetamidomalonate (1671 mg, 7.69 mmol) were dissolved in ethanol (20 mL). Freshly prepared sodium ethoxide (157 mg, 2.31 mmol) was added under nitrogen protection, and the mixture reacted at 80°C for 3 hours. Upon completion of the reaction, the mixture was concentrated, diluted with ethyl acetate (200 mL), washed successively with water (20 mL X 2) and saturated sodium chloride (20 mL), dried over anhydrous sodium sulfate, filtered, concentrated, and purified by silica gel column chromatography (10-50% petroleum ether/ethyl acetate) to obtain diethyl 6-oxo-5-azaspiro[2.4]heptane-4,4-dicarboxylate (1099 mg, yield 56%).

Step 2: Diethyl 6-oxo-5-azaspiro[2.4]heptane-4,4-dicarboxylate (1099 mg, 4.30 mmol) was dissolved in methanol (20 mL), 10% sodium hydroxide solution (5.0 mL) was added, and the mixture reacted at 60°C for 1 hour. The reaction mixture was concentrated, diluted with water (20 mL), adjusted to pH 4-5 with 2N hydrochloric acid to precipitate a solid, which was then filtered and dried to obtain 6-oxo-5-azaspiro[2.4]heptane-4,4-dicarboxylic acid (770 mg, yield 90.0%).

Step 3: 6-oxo-5-azaspiro[2.4]heptane-4,4-dicarboxylic acid (770 mg, 3.87 mmol) was heated directly to 120°C for 1 hour. After the reaction was complete, 6-oxo-5-azaspiro[2.4]heptane-4-carboxylic acid was obtained (550 mg, yield 91.7%).

Step 4: 6-oxo-5-azaspiro[2.4]heptane-4-carboxylic acid (550 mg, 3.55 mmol) was dissolved in methanol (20 mL), concentrated sulfuric acid (0.2 mL) was added, and the mixture reacted at 60°C for 16 hours. Upon completion of the reaction (monitored by LCMS), the reaction mixture was concentrated, diluted with ethyl acetate (200 mL), washed successively with saturated sodium bicarbonate (20 mL X 2) and saturated sodium chloride (10 mL), dried over anhydrous sodium sulfate, filtered, evaporated, and purified by silica gel column chromatography (10-70% petroleum ether/ethyl acetate) to obtain methyl 6-oxo-5-azaspiro[2.4]heptane-4-carboxylate (500 mg, yield 83.3%).

Step 5: Methyl 6-oxo-5-azaspiro[2.4]heptane-4-carboxylate (500 mg, 2.96 mmol) and 3-chloro-2-(chloromethyl)prop-1-ene (592 mg, 4.74 mmol) were dissolved in tetrahydrofuran (20 mL). At - 40°C, bis(trimethylsilyl)aminolithium (6.0 mL, 1M) was added, the mixture was react at room temperature for 16 hours. The reaction was quenched with an ammonium chloride solution (40 mL), extracted with ethyl acetate (50 mL X 2), the organic phase was washed with brine, dried over anhydrous sodium sulfate, filtered, concentrated, and purified by silica gel column chromatography (petroleum ether/ethyl acetate = 1:1) to obtain methyl 6'-methylene-3'-oxotetrahydrospiro[cyclopropane-1,1'-pyrrolizine]-7a'(5'H)-carboxylate (207 mg, yield: 31.56%).

Step 6: Methyl 6'-methylene-3'-oxotetrahydrospiro[cyclopropane-1,1'-pyrrolizine]-7a'(5'H)-carboxylate (207 mg, 0.93 mmol) was dissolved in tetrahydrofuran (10 mL), and lithium aluminium hydride (4.5 mL, 1M) was added dropwise under an ice water bath. The mixture was heated to 70°C and reacted for 3 hours. The reaction mixture was quenched with water (0.2 mL), 15% sodium hydroxide solution (0.2 mL), and water (0.6 mL), then filtered and concentrated to obtain a crude product of (6'-methylenetetrahydrospiro[cyclopropane-1,1'-pyrrolizin]-7a'(5'H)-yl)methanol (130 mg, yield: 78.3%).

Step 7: (6'-methylenetetrahydrospiro[cyclopropane-1,1'-pyrrolizin]-7a'(5'H)-yl)methanol (25 mg, 0.14 mmol) was dissolved in dry tetrahydrofuran (5 mL). Sodium hydride (28 mg, 0.70 mmol) was added at 0°C, and the mixture was stirred at this temperature for 15 minutes. Then tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (50 mg, 0.063 mmol) was added, and the reaction was proceeded at room temperature for 16 hours. Upon completion of the reaction, the reaction mixture was diluted with water (10 mL), extracted with ethyl acetate (15 mL X 3), dried over anhydrous sodium sulfate, filtered, concentrated, and purified by silica gel column chromatography (50-100% ethyl acetate/petroleum ether) to obtain the compound tert-butyl (5S,5aS,6S,9R)-1-fluoro-2-(7-fluoro-8-((triisopropylsilyl)ethynyl)naphthalen-1-yl)-5-methyl-12-((tetrahydrospiro[cyclopropane-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (35 mg, yield: 62.4%). ES-API:[M+H]⁺= 902.1.

Step 8: Tert-butyl (5S,5aS,6S,9R)-1-fluoro-2-(7-fluoro-8-((triisopropylsilyl)ethynyl)naphthalen-1-yl)-5-methyl-12-((tetrahydrospiro[cyclopropane-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (35 mg, 0.039 mmol) was dissolved in N,N-dimethylformamide (2 mL), cesium fluoride (60 mg, 0.39 mmol) was added, and the mixture was stirred at room temperature for 1 hour. The reaction mixture was extracted with ethyl acetate (10 mL X 3), dried over anhydrous sodium sulfate, filtered, and concentrated. The crude product was purified by preparative HPLC (trifluoroacetic acid method) to obtain the product tert-butyl (5S,5aS,6S,9R)-2-(8-ethynyl-7-fluoronaphthalen-1-yl)-1-fluoro-5-methyl-12-((6'-methylenetetrahydrospiro[cyclopropane-1,1'-pyrrolizinl-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (20 mg, yield 68.7%). ES-API:[M+H]⁺=746.3.

Step 9: Tert-butyl (5S,5aS,6S,9R)-2-(8-ethynyl-7-fluoronaphthalen-1-yl)-1-fluoro-5-methyl-12-((6'-methylenetetrahydrospiro[cyclopropane-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (20 mg, 0.027 mmol) was dissolved in acetonitrile (5 mL). A 4M hydrochloric acid/dioxane solution (1 mL) was added under an ice water bath, and the reaction was proceeded for 1 hour under an ice water bath. The reaction mixture was adjusted to pH = 8 with saturated sodium bicarbonate, then extracted with ethyl acetate (15 mL X 3), concentrated, and purified by preparative HPLC (trifluoroacetic acid method) to obtain the product (5S,5aS,6S,9R)-2-(8-ethynyl-7-fluoronaphthalen-1-yl)-1-fluoro-5-methyl-12-((6'-methylenetetrahydrospiro[cyclopropane-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z602, 9.5 mg, yield: 54.4%, trifluoroacetate). ES-API:[M+H]⁺=647.4. ¹H NMR (400 MHz, CD₃OD) δ 8.44 (s, 2H), 8.20 -8.01 (m, 2H), 7.71 -7.36 (m, 3H), 5.41 (d, J = 13.6 Hz, 1H), 5.23 (s, 2H), 4.70 -4.40 (m, 3H), 4.34 -4.08 (m, 2H), 3.98 -3.44 (m, 5H), 3.26 (d, J = 8.6 Hz, 2H), 2.81 (s, 1H), 2.58 (d, J = 15.8 Hz, 1H), 2.36 -2.22 (m, 1H), 1.99 (ddd, J = 63.6, 17.5, 6.1 Hz, 5H), 1.69 -1.50 (m, 3H), 0.99 -0.77 (m, 3H), 0.78 -0.68 (m, 1H).

### Example 46: Synthesis of (5S,5aS,6S,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a,6,7,8,9,10-hezahydro-5H-4-oza-3,10a,11,13,14-pentaaza-6,9-methanonaphtho[1,8-ab]heptalene (Z607)

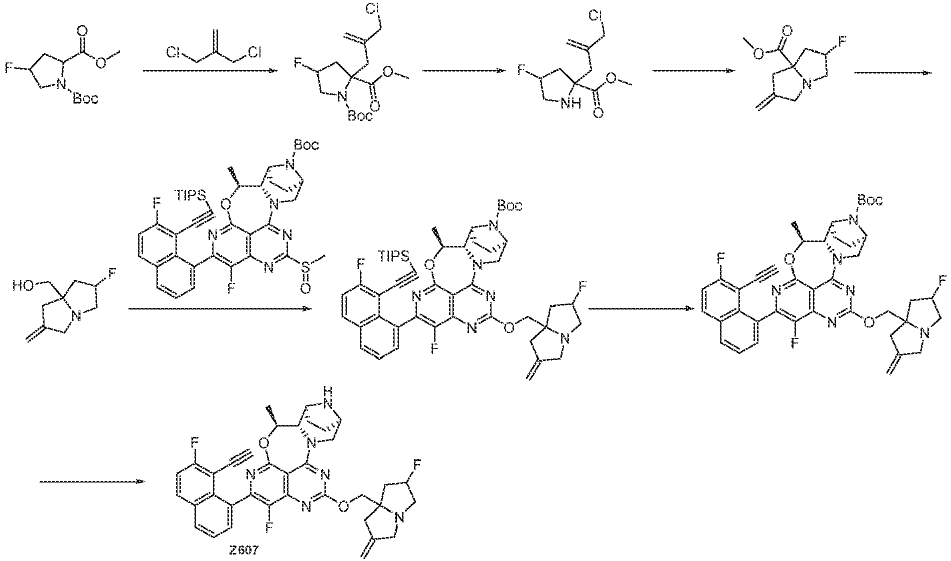

Step 1: Under nitrogen protection, 1-(tert-butyl) 2-methyl 4-fluoropyrrolidin-1,2-dicarboxylate (1 g, 4.044 mmol) was dissolved in anhydrous tetrahydrofuran (15 mL), and the mixture was placed in a dry ice-acetone bath. Bis(trimethylsilyl)aminolithium (6.1 mL, 6.066 mmol) was added dropwise to the above solution, and the mixture was stirred at this temperature for 1 hour. Then, a solution of 3-chloro-2-chloromethylacrylate (2.53 g, 20.222 mmol) in anhydrous tetrahydrofuran (10 mL) was slowly added to the above solution via a syringe, and the reaction mixture continued to stir at -78 °C for 1 hour. Upon completion of the reaction (monitored by LCMS), the reaction was quenched with saturated ammonium chloride solution (30 mL), extracted with ethyl acetate (20 mL X 3), the organic phase was washed with saturated brine (15 mL X 3), dried over anhydrous sodium sulfate, filtered, concentrated, and the crude product was purified by silica gel column chromatography to obtain 1-(tert-butyl) 2-methyl 2-(2-(chloromethyl)vinylpropyl)-4-fluoropyrrolidin-1,2-dicarboxylate (1354 mg, yield: 100%). ES-API: [M-55]⁺= 280.1.

Step 2: At room temperature, 4 M hydrochloric acid-dioxane solution (10 mL) was slowly added to a solution of 1-(tert-butyl) 2-methyl 2-(2-(chloromethyl)vinylpropyl)-4-fluoropyrrolidin-1,2-dicarboxylate (1354 mg, 4.044 mmol) in acetonitrile (6 mL), and the reaction mixture was stirred at room temperature for 0.5 hours. Upon completion of the reaction (monitored by LCMS), the reaction mixture was concentrated under reduced pressure to dryness to obtain the crude product methyl 2-(2-(chloromethyl)allyl)-4-fluoropyrrolidine-2-carboxylate (1.2 g). ES-API: [M+H]⁺= 236.2.

Step 3: At room temperature, sodium bicarbonate (1.93 g, 22.979 mmol) and potassium iodide (76 mg, 0.460 mmol) were added to a solution of methyl 2-(2-(chloromethyl)allyl)-4-fluoropyrrolidine-2-carboxylate (1.08 g, 4.596 mmol) in acetonitrile (60 mL), and the reaction mixture was stirred at room temperature for 1.5 hours. The reaction was monitored by LCMS, the mixture was filtered, and the insoluble material was washed with acetonitrile. The filtrate was concentrated under reduced pressure, and the residue was purified by silica gel column chromatography to obtain methyl 2-fhioro-6-methylenetetrahydro-1H-pyrrolizine-7a(5H)-carboxylate (980 mg, yield: 96%). ES-API: [M+H]⁺= 200.1.

Step 4: Under nitrogen protection, methyl 2-fluoro-6-methylenetetrahydro-1H-pyrrolizine-7a(5H)-carboxylate (573 mg, 2.879 mmol) was dissolved in anhydrous tetrahydrofuran (10 mL), and the mixture was placed in an ice-water bath. Lithium aluminum hydride (5.8 mL, 219 mg, 5.76 mmol, 1 M in tetrahydrofuran) was added dropwise to the above solution, and the mixture was stirred at this temperature for 0.5 hours. Upon completion of the reaction (monitored by LCMS), the reaction mixture was quenched with sodium dodecahydrate (438 mg) and stirred at room temperature for 0.5 hours, then filtered to remove insoluble material. The filtrate was concentrated under reduced pressure to obtain (2-fluoro-6-methylenetetrahydro-1H-pyrrolizin-7a(5H)-yl)methanol (370 mg, yield: 56%). ES-API: [M+H]⁺= 172.1.

Step 5: (2-fluoro-6-methylenetetrahydro-1H-pyrrolizin-7a(5H)-yl)methanol (53 mg, 0.308 mmol) was dissolved in anhydrous tetrahydrofuran (5 mL). Under an ice-water bath, sodium hydride (12 mg, 0.308 mmol) was added, and the reaction was proceeded for 30 minutes under an ice-water bath. A tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (81 mg, 0.103 mmol) was then added dropwise to the above reaction mixture, and the reaction was proceeded for 30 minutes at 0°C. Upon completion of the reaction (monitored by LCMS), the reaction mixture was diluted with ethyl acetate (10 mL), washed with saturated ammonium chloride solution (10 mL X 2) and saturated brine (10 mL X 3), dried over anhydrous sodium sulfate, filtered, and concentrated under reduced pressure to obtain the crude product tert-butyl (5S,5aS,6S,9R)-1-fluoro-12-((2-fluoro-6-methylenetetrahydro-1H-pyrrolizin-7a(5H)-yl)methoxy)-2-(7-fluoro-8-((triisopropylsilyl)ethynyl)naphthalen-l-yl)-5-methyl-5a,6,7,8,9,10-hexahydro-5H-4-oxa-3,10a,11,13,14-pentaaza-6,9-methanonaphtho[1,8-ab]heptalene-14-carboxylate (88 mg). ES-API: [M+H]⁺= 895.4.

Step 6: Cesium fluoride (150 mg, 0.983 mmol) was added to a solution of tert-butyl (5S,5aS,6S,9R)-1-fluoro-12-((2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (88 mg, 0.098 mmol) in N,N-dimethylformamide (5 mL). The reaction mixture was stirred at room temperature for 1 hour. Upon completion of the reaction (monitored by LCMS), the reaction mixture was diluted with ethyl acetate (15 mL), washed with water (15 mL X 2), and saturated brine (15 mL X 3), dried over anhydrous sodium sulfate, filtered, and concentrated to obtain the crude product of tert-butyl (5S,5aS,6S,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (72 mg). ES-API: [M+H]⁺= 739.3.

Step 7: Under an ice-water bath, 4 M hydrochloric acid-dioxane solution (5 mL) was slowly added to a solution of tert-butyl (5S,5aS,6S,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (72 mg, 0.097 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (bicarbonate method 2) (chromatographic condition: 15 mL-50-65-13 min) to obtain an off-white solid of (5 S,5aS,6S,9R)-2-(8-ethynyl-7-fluoronaphthalen- 1-yl)-1 -fhioro-12-((2-fluoro-6-methylenetetrahydro-1H-pyrrolizin-7a(5H)-yl)methoxy)-5-methyl-5a,6,7,8,9,10-hexahydro-5H-4-oxa-3,10a,1,13,14-pentaaza-6,9-methanonaphtho[1,8-ab]heptalene (Z607, 11.3 mg, yield: 18 %). ES-API: [M+H]⁺= 639.3.

### Example 47: Synthesis of (5R,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hezahydrn-5H-4-oza-3,10a,11,13,14-pentaaza-6,9-methanonaphtho[1,8-ab]heptalene (Z630)

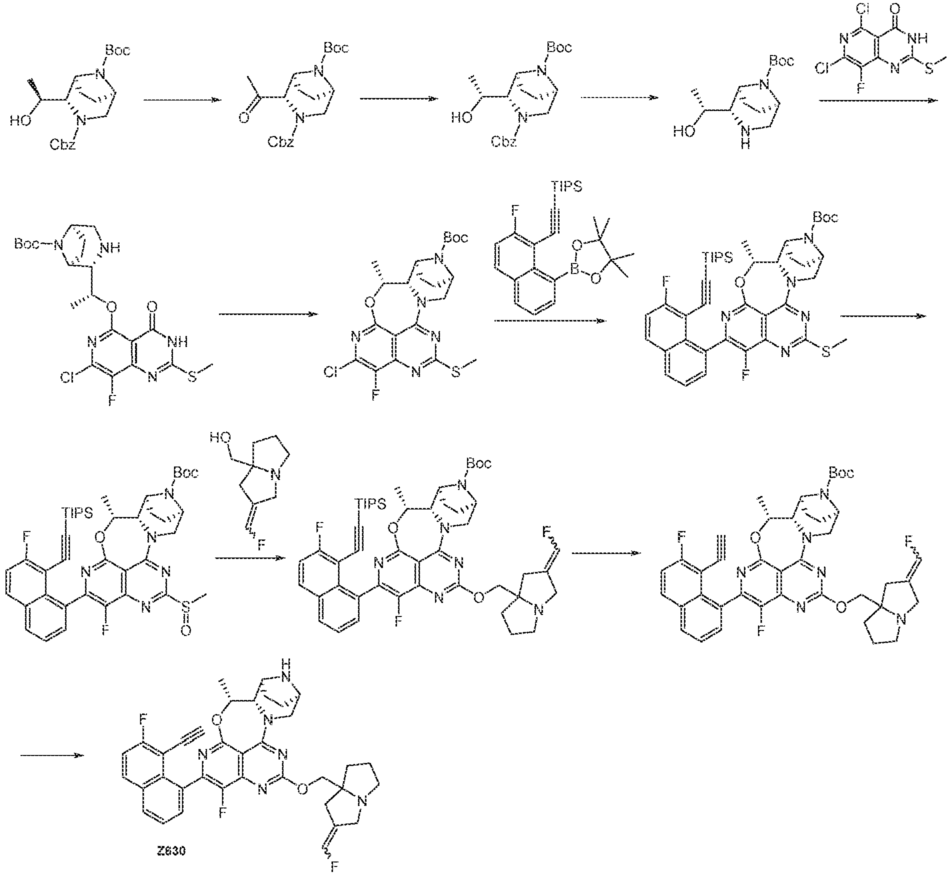

Step 1: 3-Benzyl-8-(tert-butyl)(1S,2S,5R)-2-((S)-1-hydroxyethyl)-3,8-diazabicyclo[3.2.1]octane-3,8-dicarboxylate (120 mg, 0.31 mmol) was dissolved in anhydrous dichloromethane (6 mL). At 0°C, Dess-Martin periodinane (196 mg, 0.46 mmol) was added, and the reaction was proceeded at room temperature for 2 hours. Upon completion of the reaction (monitored by LCMS), saturated sodium thiosulfate solution (10 mL) and saturated sodium bicarbonate solution (10 mL) were added to the reaction mixture. The mixture was extracted with ethyl acetate (20 mL × 2). The organic phase was washed with saturated brine (20 mL), dried over anhydrous sodium sulfate, filtered, concentrated, and the crude product was purified by flash silica gel column (0-25% ethyl acetate/petroleum ether) to obtain 3-benzyl-8-(tert-butyl)(1S,2S,SR)-2-acetyl-3,8-diazabicyclo[3.2.1]octane-3,8-dicarboxylate (100 mg, yield 87%). ES-API: [M+H]⁺=389.2.

Step 2: 3-Benzyl-8-(tert-butyl)(1S,2S,5R)-2-acetyl-3,8-diazabicyclo[3.2.1]octane-3,8-dicarboxylate (100 mg, 0.26 mmol) was dissolved in anhydrous ethanol (5 mL). At 0°C, 2 M lithium borohydride in tetrahydrofuran solution (0.26 mL, 0.52 mmol) was added. The reaction was proceeded at room temperature for 1 hour. Upon completion of the reaction (monitored by LCMS), saturated ammonium chloride solution (20 mL) was added. The mixture was extracted with ethyl acetate (20 mL × 2). The organic phase was washed with saturated brine (20 mL), dried over anhydrous sodium sulfate, filtered, concentrated, and the crude product was purified by flash silica gel column (0-40% ethyl acetate/petroleum ether) to obtain 3-benzyl-8-(tert-butyl)(1S,2S,5R)-2-((R)-1-hydroxyethyl)-3,8-diazabicyclo[3.2.1]octane-3,8-dicarboxylate (80 mg, yield 79%), a colorless oily substance. ES-API: [M-55]⁺=335.2.

Step 3: 3-Benzyl-8-(tert-butyl)(1S,2S,SR)-2-((R)-1-hydroxyethyl)-3,8-diazabicyclo[3.2.1]octane-3,8-dicarboxylate (260 mg, 0.67 mmol), palladium on carbon (142 mg, 0.13 mmol), acetic acid (249 mg, 1.33 mmol), and 2,2,2-trifluoroethanol (12 mL) were successively added to a round-bottom flask. The reaction mixture was stirred at 30°C under hydrogen atmosphere for 16 hours. Upon completion of the reaction (monitored by LCMS), the reaction mixture was filtered through diatomaceous earth, and the filtrate was concentrated. The crude product was dissolved in water and adjusted to pH 5 with 1 M hydrochloric acid. The aqueous phase was extracted with ethyl acetate (30 mL × 1), the organic phase was discarded. The aqueous phase was basified with 2 M sodium hydroxide and extracted with dichloromethane/isopropanol (3:1, 30 mL × 3). The organic phase was dried over anhydrous sodium sulfate, filtered, and concentrated to obtain tert-butyl (1S,2S,5R)-2-((R)-1-hydroxyethyl)-3,8-diazabicyclo[3.2.1]octane-8-carboxylate (130 mg, yield 76%), a yellow oil. ES-API: [M+H]⁺=257.1.

Step 4: At 0°C, sodium hydride (61 mg, 2.54 mmol), tert-butyl (1S,2S,SR)-2-((R)-1-hydroxyethyl)-3,8-diazabicyclo[3.2.1]octane-8-carboxylate (130 mg, 0.51 mmol), and tetrahydrofuran (8 mL) were added to a round-bottom flask. The mixture was stirred at room temperature for 10 minutes. Cooled to 0°C, 5,7-dichloro-S-fluoro-2-(methylthio)pyrido[4,3-d]pyrimidin-4(3H)-one (284 mg, 1.01 mmol) was added. The reaction mixture was stirred in a 60°C oil bath for 60 minutes. Upon completion of the reaction (monitored by LCMS), saturated ammonium chloride solution (50 mL) was added. The mixture was extracted with ethyl acetate (50 mL × 2). The organic phase was washed with saturated brine (50 mL), dried over anhydrous sodium sulfate, filtered, concentrated, and the crude product was purified by flash silica gel column (0-10% methanol/petroleum ether) to obtain tert-butyl (1S,2S,5R)-2-((R)-1-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (200 mg, yield 79%), a yellow solid. ES-API: [M+H]⁺=500.2.

Step 5: Tert-butyl (1S,2S,SR)-2-((R)-1-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (170 mg, 0.34 mmol), N,N-diisopropylethylamine (0.21 mL, 1.19 mmol), and dichloromethane (15 mL) were added to a 100 mL round-bottom flask. Cooled to 0°C, 2-(7-azabenzotriazol)-N,N,N',N'-tetramethyluronium hexafluorophosphate (259 mg, 0.68 mmol) was added, and the reaction mixture was stirred at 30°C for 16 hours. Upon completion of the reaction (monitored by LCMS), dichloromethane (50 mL) was added, and the reaction mixture was washed with water (30 mL) and saturated sodium bicarbonate solution (30 mL). The organic phase was dried over anhydrous sodium sulfate, filtered, and concentrated. The crude product was purified by flash silica gel column (ethyl acetate/petroleum ether: 0-30%) to obtain tert-butyl (5R,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (120 mg, yield 73%), a yellow solid.

Step 6: Tert-butyl (5R,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanomphtho[1,8-ab]heptalene-14-carboxylate (110 mg, 0.23 mmol), ((2-fluoro-8-(4,4,5,5-tetramethyl-1,3,2-dioxaborolan-2-yl)naphthyl-1-yl)ethynyl)triisopropylsilane (206 mg, 0.46 mmol), palladium(II) bis[butyldi(1-adamantyl)phosphine](2-amino-1,1'-biphenyl) methanesulfonate (17 mg, 0.023 mmol), potassium phosphate (145 mg, 0.68 mmol), tetrahydrofuran (6 mL), and water (1.5 mL) were added to a round-bottom flask. Under nitrogen protection, the system was heated in a microwave reactor at 75°C for 40 minutes. With water (30 mL) added, the reaction mixture was extracted with ethyl acetate (30 mL × 3), and the organic phase was dried over anhydrous sodium sulfate, filtered, and concentrated. The crude product was purified by flash silica gel column (ethyl acetate/petroleum ether: 0-25%) to obtain tert-butyl (5R,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (130 mg, yield 74%), a yellow solid. ES-API: [M+H]⁺=772.3.

Step 7: Tert-butyl (5R,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (130 mg, 0.17 mmol) and dichloromethane (6 mL) were added to a round-bottom flask. Cooled to 0°C, m-chloroperbenzoic acid (44 mg, 0.25 mmol) was added to the reaction mixture, which was then stirred at room temperature for 1 hour. Saturated sodium thiosulfate solution (30 mL) and saturated sodium bicarbonate solution were added to the reaction mixture, and the mixture was extracted with ethyl acetate (30 mL × 3). The organic phase was dried and concentrated to obtain tert-butyl (SR,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (130 mg, yield 97.8%), a yellow solid. ES-API: [M+H]⁺=788.3.

Step 8: Sodium hydride (15 mg, 0.38 mmol) and tetrahydrofuran (4 mL) were added to a round-bottom flask. Cooled to 0°C, (2-(fluoromethyl)tetrahydro-1H-pyrrolo[7a(5H)]-yl)methanol (33 mg, 0.19 mmol) dissolved in anhydrous tetrahydrofuran (1 mL) was added to the reaction mixture. The reaction was proceeded at room temperature for 20 minutes. Tert-butyl (SR,SaS,6S,9R)-1-fluoro-2-(7-fluoro-8-((triisopropylsilyl)ethynyl)naphthalen-l-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (60 mg, 0.076 mmol) was added to the reaction mixture, and the reaction mixture was stirred at 0°C for 20 minutes. Upon completion of the reaction (monitored by LCMS), saturated ammonium chloride solution (30 mL) was added. The mixture was extracted with ethyl acetate (20 mL × 2). The organic phase was washed with saturated brine (30 mL), dried over anhydrous sodium sulfate, filtered, and concentrated to obtain tert-butyl (SR,SaS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, crude product). ES-API: [M+H]⁺=895.5.

Step 9: Cesium fluoride (102 mg, 0.67 mmol) was added to N,N-dimethylformamide (3 mL) containing tert-butyl (5R,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, crude product). The reaction mixture was stirred at room temperature for 1 hour. Upon completion of the reaction (monitored by LCMS), ethyl acetate (50 mL) was added to dilute the reaction mixture, which was then washed with water (15 mL × 2) and saturated brine (15 mL × 3), dried over anhydrous sodium sulfate, filtered, and concentrated to obtain tert-butyl (SR,SaS,6S,9R)-2-(8-ethynyl-7-fluoronaphthyl-1-yl)-1-fluoro-12-((2-(fluoromethyl)tetrahydro-1H-pyrrolo[7a(5H)]-yl)methoxy)-5-methyl-5a,6,7,8,9,10-hexahydro-5H-4-oxygen-3,10a,11,13,14-pentazacyclo-6,9-methylenenaphth[1,8-ab]heptene-14-carboxylate (49 mg, crude product). ES-API: [M+H]⁺= 739.3.

Step 10: Under an ice-water bath, 4 M hydrochloric acid-dioxane solution (2 mL) was slowly added to the acetonitrile (2 mL) solution of tert-butyl (SR,SaS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (49 mg, crude product). The reaction mixture was stirred at room temperature for 1 hour. Upon completion of the reaction (monitored by LCMS), the reaction mixture was concentrated under reduced pressure to dryness, and the residue was purified by preparative HPLC (ammonium bicarbonate method 1) to obtain (SR,SaS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene (Z630, 10 mg, yield: 23 %), white solid. ES-API: [M+H]⁺= 639.2.

### Example 48: Synthesis of (5S,5aS,6S,9R)-2-(8-ethynyl-7-fluoronaphthalen-1-yl)-1-fluoro-5-methyl-12-(((1aS,6bR)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene (Z606)

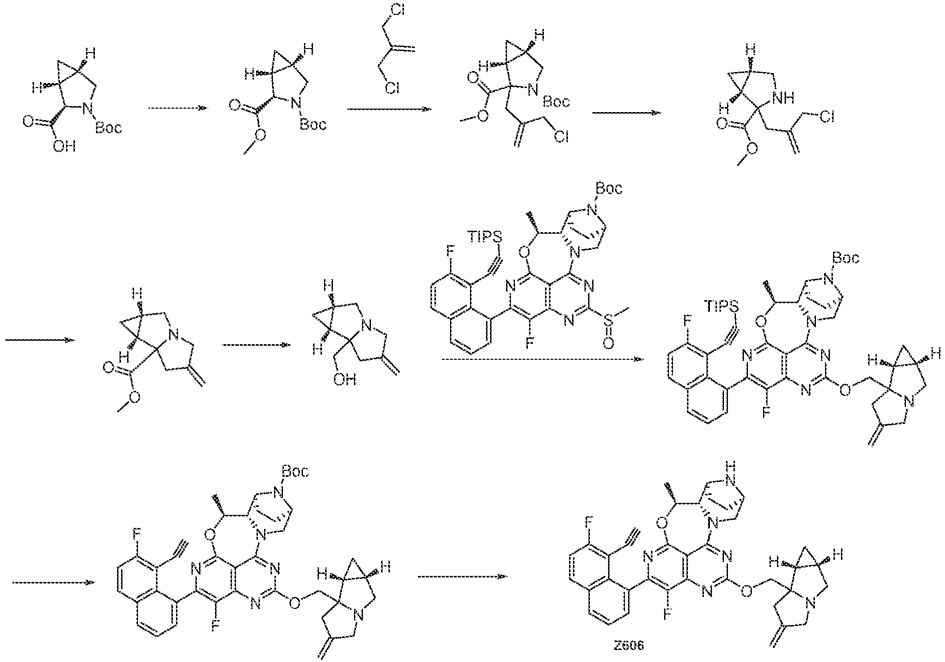

Step 1: Under an ice-water bath, potassium carbonate (456 mg, 3.3 mmol) and iodomethane (312 mg, 2.2 mmol) were added to a N,N-dimethylformamide (5 mL) solution of (1R,2R,5S)-3-(tert-butoxycarbonyl)-3-azabicyclo[3.1.0]hexane-2-carboxylic acid (250 mg, 1.1 mmol). The mixture was stirred at room temperature for 1.5 hours. Upon completion of the reaction (monitored by LCMS), the reaction mixture was filtered and washed with ethyl acetate. The filtrate was concentrated under reduced pressure to obtain 3-(tert-butyl)-2-methyl-(1R,2R,SS)-3-azabicyclo[3.1.0]hexane-2,3-dicarboxylate (265 mg, yield: 100%). ES-API: [M-100]⁺= 186.1.

Step 2: Under nitrogen protection, 3-(tert-butyl)-2-methyl-(1R,2R,5S)-3-azabicyclo[3.1.0]hexane-2,3-dicarboxylate (220 mg, 0.9 mmol) was dissolved in anhydrous tetrahydrofuran (10 mL), and the mixture was placed in a dry ice-acetone bath. Bis(trimethylsilyl)aminolithium solution (1.8 mL, 1.8 mmol, 1 M in tetrahydrofuran) was slowly added dropwise to the solution, and the mixture was stirred at this temperature for 1 hour. A solution of 3-chloro-2-(chloromethyl)propen-1-ene (570 mg, 4.6 mmol) in anhydrous tetrahydrofuran (10 mL) was slowly added via syringe, and the reaction mixture was further stirred at -78°C for 1 hour. Upon completion of the reaction (monitored by LCMS), the reaction was quenched with saturated ammonium chloride solution (50 mL). The mixture was extracted with ethyl acetate (50 mL × 3), washed with saturated brine (50 mL × 3), dried over anhydrous magnesium sulfate, filtered, and concentrated. The crude product was purified by flash silica gel column chromatography (petroleum ether: ethyl acetate = 20:1) to obtain 3-(tert-butyl) 2-methyl (1R,5S)-2-(2-(chloromethyl)allyl)-3-azabicyclo[3.1.0]hexane-2,3-dicarboxylate (160 mg, yield: 53%). ES-API: [M-56]⁺= 274.1.

Step 3: At room temperature, 4 M hydrochloric acid/dioxane solution (5 mL) was slowly added to the acetonitrile (2 mL) solution of 3-(tert-butyl) 2-methyl (1R,5S)-2-(2-(chloromethyl)allyl)-3-azabicyclo[3.1.0]hexane-2,3-dicarboxylate (160 mg, 0.5 mmol). The mixture was stirred at room temperature for 0.5 hours. Upon completion of the reaction (monitored by LCMS), the reaction mixture was concentrated under reduced pressure to obtain methyl (1R,5S)-2-(2-(chloromethyl)allyl)-3-azabicyclo[3.1.0]hexane-2-carboxylate (111 mg, yield: 100%). ES-API: [M+H]⁺= 230.2.

Step 4: At room temperature, sodium bicarbonate (203 mg, 2.4 mmol) and potassium iodide (8 mg, 0.05 mmol) were added to the acetonitrile (10 mL) solution of methyl (1R,5S)-2-(2-(chloromethyl)allyl)-3-azabicyclo[3.1.0]hexane-2-carboxylate (111 mg, 0.5 mmol), and the mixture was stirred for 1.5 hours. Upon completion of the reaction (monitored by LCMS), the reaction mixture was filtered and washed with acetonitrile. The filtrate was concentrated under reduced pressure to obtain methyl (1aS,6bR)-5-methylenehexahydrocyclopropa[a]pyrrolizine-6a(4H)-carboxylate (94 mg, crude product). ES-API: [M+H]⁺=194.1.

Step 5: Under nitrogen protection, methyl (1aS,6bR)-5-methylenehexahydrocyclopropa[a]pyrrolizine-6a(4H)-carboxylate (94 mg, 0.5 mmol) was dissolved in anhydrous tetrahydrofuran (5 mL), and the mixture was placed in an ice-water bath. Lithium aluminium hydride solution (1.0 mL, 1.0 mmol, 1 M in tetrahydrofuran) was slowly added dropwise to the above solution, and the mixture was stirred at this temperature for 0.5 hours. Upon completion of the reaction (monitored by LCMS), sodium sulfate decahydrate (100 mg) was added to quench the reaction, and the mixture was stirred at room temperature for 0.5 hours. The reaction mixture was filtered. The filtrate was concentrated under reduced pressure to obtain ((1aS,6bR)-5-methylene hexahydrocyclopropa[a]pyrrolizin-6a(4H)-yl)methanol (43 mg, yield: 54%). ES-API: [M+H]⁺= 166.1.

Step 6: ((1aS,6bR)-5-Methylene hexahydrocyclopropa[a]pyrrolizin-6a(4H)-yl)methanol (34 mg, 0.2 mmol) was dissolved in anhydrous tetrahydrofuran (5 mL). Under an ice-water bath, sodium hydride (10 mg, 0.4 mmol) was added, and the mixture reacted for 30 minutes at the ice-water bath temperature. A tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (81 mg, 0.1 mmol) was added dropwise to the above reaction mixture and reacted at 40°C for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous magnesium sulfate, filtered, and concentrated. The crude product was purified by silica gel column chromatography (dichloromethane: methanol = 15:1) to obtain tert-butyl (SS,SaS,6S,9R)-1-fluoro-2-(7-fluoro-8-((triisopropylsilyl)ethynyl)naphthalen-1-yl)-5-methyl-12-(((1aS,6bR)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (17 mg, yield: 20%). ES-API: [M+H]⁺= 889.5.

Step 7: Cesium fluoride (30 mg, 0.2 mmol) was added to N,N-dimethylformamide (1.5 mL) containing tert-butyl (SS,SaS,6S,9R)-1-fluoro-2-(7-fluoro-8-((triisopropylsilyl)ethynyl)naphthalen-1-yl)-5-methyl-12-(((1aS,6bR)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (17 mg, 0.02 mmol). The reaction mixture was stirred at room temperature for 2 hours. Upon completion of the reaction (monitored by LCMS), the reaction mixture was diluted with ethyl acetate (20 mL) and washed with water (15 mL × 2) and saturated brine (15 mL × 3), dried over anhydrous magnesium sulfate, filtered, and concentrated to obtain a crude product of tert-butyl (SS,SaS,6S,9R)-2-(8-ethynyl-7-fluoronaphthalen-1-yl)-1-fluoro-5-methyl-12-(((1aS,6bR)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, crude product). ES-API: [M+H]⁺= 733.3.

Step 8: Under an ice-water bath, 4 M hydrochloric acid-dioxane solution (3 mL) was slowly added to the acetonitrile (1 mL) solution of tert-butyl (5S,5aS,6S,9R)-2-(8-ethynyl-7-fluoronaphthalen-1-yl)-1-fluoro-5-methyl-12-(((1aS,6bR)-5-methylenehexahydrocyclopropa[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, 0.02 mmol), and the reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic condition: 15ml-35-85-13min) to obtain a pale yellow solid of (SS,SaS,6S,9R)-2-(8-ethynyl-7-fluoronaphthalen-1-yl)-1-fluoro-5-methyl-12-(((1aS,6bR)-5-methylenehexahydrocyclopropa[a]pyrrolizin-6a(4H-yl)methoxy)-5a,6,7,8,9,10-hexahydro-5H-4-oxa-3,10a, 11,13,14-pentaaza-6,9-methanonaphtho[1,8-ab]heptalene (Z606, 1.2 mg, yield: 9.5 %). ES-API: [M+H]⁺= 633.3.

### Example 49: Synthesis of (5S,5aS,6S,9R)-2-(8-ethynyl-7-fluoronaphthalen-1-yl)-1-fluoro-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z631)

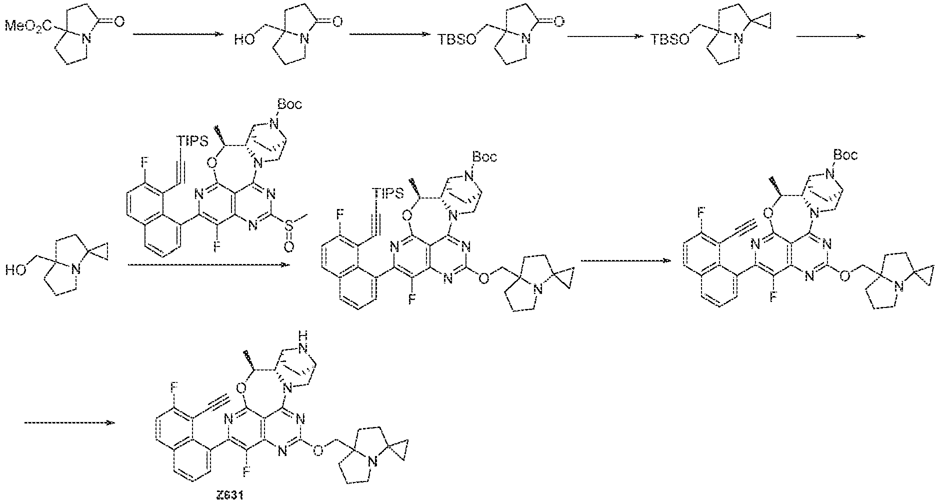

Step 1: Methyl 3-oxo-tetrahydro-1H-pyrrolizine-7a(5H)-carboxylate (900 mg, 4.9 mmol) was dissolved in methanol (10 mL) and cooled to 0°C. Sodium borohydride (373 mg, 9.8 mmol) was added, and the reaction was proceeded at room temperature for 16 hours. Upon completion of the reaction, the reaction mixture was quenched with saturated ammonium chloride aqueous solution, extracted with ethyl acetate (20 mL × 3), washed with saturated sodium chloride (20 mL), dried over anhydrous sodium sulfate, filtered and evaporated to obtain a crude product of 7a-(hydroxymethyl)hexahydro-3H-pyrrolizin-3-one (600 mg, yield 79%).

Step 2: 7a-(hydroxymethyl)hexahydro-3H-pyrrolizin-3-one (600 mg, 3.87 mmol) was dissolved in dichloromethane, imidazole (394 mg, 5.80 mmol) was added, followed by the slow addition of tert-butyldimethylchlorosilane (870 mg, 5.80 mmol). The reaction mixture reacted at room temperature for 16 hours. Upon completion of the reaction, water was added, and the mixture was extracted with ethyl acetate (30 mL × 3), washed with saturated sodium chloride (30 mL), dried over anhydrous sodium sulfate, filtered, evaporated and purified by silica gel column chromatography (petroleum ether/ethyl acetate = 0-50%) to obtain 7a-(((tert-butyldimethylsilyl)oxy)methyl)hexahydro-3H-pyrrolizin-3-one (900 mg, yield 82.7%).

Step 3: 7a-(((Tert-butyldimethylsilyl)oxy)methyl)hexahydro-3H-pyrrolizin-3-ane (900 mg, 3.2 mmol) and tetraisopropoxy titanium (2.73g, 9.6 mmol) were dissolved in anhydrous tetrahydrofuran. At 0°C, ethyl formate reagent (0.64 mL, 2.5 M, 16 mmol) was slowly added dropwise, and the reaction mixture reacted at room temperature for 16 hours. Upon completion of the reaction, the reaction mixture was quenched with saturated ammonium chloride aqueous solution, extracted with ethyl acetate (30 mL × 3), washed with saturated sodium chloride (30 mL), dried over anhydrous sodium sulfate, filtered, evaporated and purified by silica gel column chromatography (petroleum ether/ethyl acetate = 0-30 %) to obtain 7a'-(((tertbutyldimethylsilyl)oxy)methyl)hexahydrospiro[cyclopropane-1,3'-pyrrolizine] (350 mg, yield 38.9%).

Step 4: 7a'-(((tert-butyldimethylsilyl)oxy)methyl)hexahydrospiro[cyclopropane-1,3'-pyrrolizine] (150 mg, 0.53 mmol) was dissolved in tetrahydrofuran (20 mL), tetra-n-butylammonium fluoride (2 mL) was added, and the reaction mixture reacted at room temperature for 2 hours. Upon completion of the reaction, the reaction mixture was concentrated and purified by column chromatography (petroleum ether/ethyl acetate = 10-100%) to obtain (tetrahydrospiro[cyclopropane-1,3'-pyrrolizin]-7a'(5'H)-yl)methanol (40 mg, yield 45.2%).

Step 5: (Tetrahydrospiro[cyclopropane-1,3'-pyrrolizin]-7a'(5'H)-yl)methanol (40 mg, 0.24 mmol) was dissolved in dry tetrahydrofuran (5 mL), sodium hydride (48 mg, 1.2 mmol) was added at 0°C and the mixture was stirred 15 minutes. Then, tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (50 mg, 0.063 mmol) was added and the reaction was proceeded at room temperature for 16 hours. Upon completion of the reaction, water (10 mL) was added, extracted with ethyl acetate (15 mL × 3), dried over anhydrous sodium sulfate, filtered, concentrated, and purified by silica gel column chromatography (50-100% ethyl acetate/petroleum ether) to obtain the compound tert-butyl (5S,5aS,6S,9R)-1-fluoro-2-(7-fluoro-8-((triisopropylsilyl)ethynyl)naphthalen-1-yl)-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (30 mg, yield: 53.5%). ES-API: [M+H]⁺= 890.3.

Step 6: Tert-butyl (5S,5aS,6S,9R)-1-fluoro-2-(7-fluoro-8-((triisopropylsilyl)ethynyl)naphthalen-1-yl)-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (30 mg, 0.034 mmol) was dissolved in N,N-dimethylformamide (2 mL), cesium fluoride (52 mg, 0.34 mmol) was added, and the reaction mixture reacted at room temperature for 1 hour. The reaction mixture was extracted with ethyl acetate (10 mL × 3), dried over anhydrous sodium sulfate, filtered, evaporated, and purified by preparative HPLC (trifluoroacetic acid method) to obtain the product tert-butyl (5S,5aS,6S,9R)-2-(8-ethynyl-7-fluoronaphthalen-1-yl)-1-fluoro-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, yield 60.1%). ES-API: [M+H]⁺= 734.3.

Step 7: Tert-butyl (5S,5aS,6S,9R)-2-(8-ethynyl-7-fluoronaphthalen-1-yl)-1-fluoro-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, 0.020 mmol) was dissolved in acetonitrile (5 mL), 4 M hydrochloric acid/dioxane solution (1 mL) was added under an ice water bath, and the reaction mixture reacted for 1 hour under an ice water bath. The reaction mixture was adjusted to pH 8 with saturated sodium bicarbonate, extracted with ethyl acetate (15 mL × 3), evaporated, and purified by preparative HPLC (trifluoroacetic acid method) to obtain the product (5S,5aS,6S,9R)-2-(8-ethynyl-7-fluoronaphthalen-1-yl)-1-fluoro-5-methyl-12-((tetrahydrospiro[cyclopropane-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptalene (Z631, 5.0 mg, yield: 39.4%, trifluoroacetate). ES-API: [M+H]⁺= 634.4. ¹H NMR (400 MHz, CD₃OD) δ 8.52 (s, 1H), 8.10 (dd, J = 6.1, 3.0 Hz, 2H), 7.74 -7.24 (m, 3H), 5.39 (d, J = 10.9 Hz, 1H), 4.53 (dd, J = 23.5, 13.1 Hz, 2H), 4.12 (d, J = 9.0 Hz, 1H), 3.81 -3.37 (m, 4H), 3.18 (dd, J = 33.4, 23.2 Hz, 2H), 2.63 -1.69 (m, 12H), 1.59 (t, J = 6.3 Hz, 3H), 1.04 (s, 2H), 0.89 (s, 1H), 0.74 (s, 1H).

### Example 50: Synthesis of (5S,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z612)

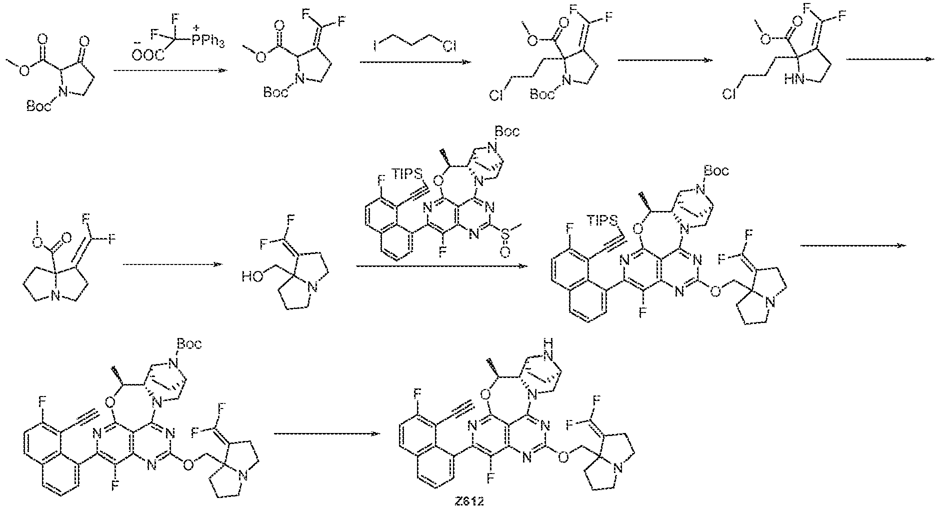

Step 1: Under nitrogen protection, (triphenylphosphonium) difluoroacetate inner salt (10 g, 28.066 mmol) was added to 1-(tert-butyl)-2-methyl-3-oxopyrrolidine-1,2-dicarboxylate (2.6 g, 10.688 mmol) in N,N-dimethylformamide (50 mL). The reaction mixture was heated to 80 °C and stirred for 2 hours. Upon completion of the reaction, as monitored by LCMS, the reaction was quenched with water (500 mL) and extracted with ethyl acetate (3x30 mL). The organic phase was washed with saturated brine (3x15 mL), dried over anhydrous sodium sulfate, filtered, and concentrated. The crude product was purified by silica gel column chromatography (ethyl acetate: petroleum ether = 0~5%) to obtain 1-(tert-butyl) 2-methyl 3-(difluoromethylene)pyrrolidine-1,2-dicarboxylate (1.2 g, yield: 46%). ES-API: [M-55]⁺= 222.1.

Step 2: Under nitrogen protection, 1-(tert-butyl) 2-methyl 3-(difluoromethylene)pyrrolidine-1,2-dicarboxylate (1.2 g, 4.328 mmol) was dissolved in anhydrous tetrahydrofuran (15 mL). The mixture was placed in a dry ice-acetone bath. Diisopropylamine lithium (8.6 mL, 8.656 mmol) was slowly added dropwise to the solution, which was then stirred at this temperature for 1 hour. Then, a solution of 1-chloro-3-iodopropane (2.654 g, 12.984 mmol) in anhydrous tetrahydrofuran (10 mL) was slowly added via syringe. The reaction mixture continued to be stirred at -78°C for 1 hour, and then was slowly warmed to room temperature and stirred overnight. Upon completion of the reaction, as monitored by LCMS, the reaction was quenched with saturated ammonium chloride solution (30 mL) and the mixture was extracted with ethyl acetate (3x20 mL). The organic phase was washed with saturated brine (3x15 mL), dried over anhydrous sodium sulfate, filtered, and concentrated. The crude product was purified by silica gel column chromatography (ethyl acetate: petroleum ether = 0∼15%) to obtain 1-(tert-butyl) 2-methyl 2-(3-chloropropyl)-3-(difluoromethylene)pyrrolidine-1,2-dicarboxylate (1128 mg, yield: 74%). ES-API: [M-55]⁺= 298.1.

Step 3: At room temperature, 4 M hydrochloric acid-dioxane solution (10 mL) was slowly added to a solution of 1-(tert-butyl) 2-methyl 2-(3-chloropropyl)-3-(difluoromethylene)pyrrolidine-1,2-dicarboxylate (581 mg, 1.642 mmol) in acetonitrile (2 mL). The reaction mixture was stirred at room temperature for 0.5 hours. Upon completion of the reaction, as monitored by LCMS, the reaction mixture was concentrated under reduced pressure to obtain methyl 2-(3-chloropropyl)-3-(difluoromethylene)pyrrolidine-2-carboxylate (416 mg). ES-API: [M+H]⁺= 254.6.

Step 4: At room temperature, sodium bicarbonate (689 mg, 8.20 mmol) and potassium iodide (27 mg, 0.164 mmol) were added to a solution of methyl 2-(3-chloropropyl)-3-(difluoromethylene)pyrrolidine-2-carboxylate (416 mg, 1.640 mmol) in acetonitrile (15 mL). The reaction mixture was stirred overnight at room temperature. Upon completion of the reaction, as monitored by LCMS, the reaction mixture was filtered and the insoluble material was washed with acetonitrile. The filtrate was concentrated under reduced pressure to obtain methyl 1-(difluoromethylene)tetrahydro-1H-pyrrolizine-7a(5H)-carboxylate (356 mg). ES-API: [M+H]⁺= 218.6.

Step 5: Under nitrogen protection, methyl 1-(difluoromethylene)tetrahydro-1H-pyrrolizine-7a(5H)-carboxylate (356 mg, 1.639 mmol) was dissolved in anhydrous tetrahydrofuran (10 mL), and the mixture was placed in an ice-water bath. Lithium aluminium hydride (3.3 mL, 3.278 mmol, 1 M in tetrahydrofuran) was slowly added dropwise to the solution, and the mixture was stirred for 0.5 hours at this temperature. Upon completion of the reaction, as monitored by LCMS, the reaction was quenched with decahydrate sodium sulfate added to the solution, and the solution was stirred at room temperature for 0.5 hours, and filtered to remove insoluble materials. The filtrate was concentrated under reduced pressure, and the crude product was purified by silica gel column chromatography (methanol: dichloromethane = 0∼40%) to obtain (1-(difluoromethylene)tetrahydro-1H-pyrrolizin-7a(5H)-yl)methanol (180 mg, yield: 58%). ES-API: [M+H]⁺= 190.1

Step 6: (1-(Difluoromethylene)tetrahydro-1H-pyrrolizin-7a(5H)-yl)methanol (58 mg, 0.305 mmol) was dissolved in anhydrous tetrahydrofuran (5 mL) and under an ice-water bath, sodium hydride (12 mg, 0.305 mmol) was added. The reaction was proceeded for 30 minutes in the ice-water bath. A solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (80 mg, 0.102 mmol) in tetrahydrofuran (2 mL) was added dropwise to the reaction mixture above, and the reaction was proceeded at 0°C for 30 minutes. Upon completion of the reaction, as monitored by LCMS, the reaction mixture was diluted with ethyl acetate (10 mL), washed with saturated ammonium chloride solution (2x10 mL) and saturated brine (3x10 mL), dried over anhydrous sodium sulfate, filtered, and concentrated under reduced pressure to obtain the crude product tert-butyl (5S,5aS,6S,9R)-12-((1-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (92 mg). ES-API: [M+H]⁺= 913.4.

Step 7: Cesium fluoride (153 mg, 1.008 mmol) was added to a solution of tert-butyl (5S,5aS,6S,9R)-12-((1-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (92 mg, 0.101 mmol) in N,N-dimethylformamide (5 mL). The reaction mixture was stirred at room temperature for 1 hour. Upon completion of the reaction, as monitored by LCMS, the reaction mixture was diluted with ethyl acetate (15 mL), washed with water (2x15 mL), and saturated brine (3x15 mL), dried over anhydrous sodium sulfate, filtered, and concentrated to obtain the crude product tert-butyl (5S,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-Sa,6,7,8,9,10-hexahydro-SH-4-oxa.-3,10a,11,13,14-pentaaza-6,9-methanonaphtho[1,8-ab]heptalene-14-carboxylate (76 mg). ES-API: [M+H]⁺= 757.2.

Step 8: Under an ice-water bath, 4 M hydrochloric acid-dioxane solution (5 mL) was slowly added to a solution of tert-butyl (5S,5aS,6S,9R)-12-((1-(difluoromethyl)tetrahydro-1H-pyrazine-7a(5H)-yl)methoxy)-2-(8-ethynyl-7-fluoronaphth-1-yl)-1-fluoro-5-methyl-5a,6,7,8,9,10-hexahydro-5H-4-oxa-3,10a,11,13,14-pentaza-6,9-methanenaphtho[1,8-ab]heptene-14-carboxylate (76 mg, 0.100 mmol) in acetonitrile (2 mL). The reaction mixture was stirred at 0°C for 0.5 hours. Upon completion of the reaction, as monitored by LCMS, the reaction mixture was concentrated to dryness, and the residue was purified by preparative HPLC (trifluoroacetic acid method) (chromatographic condition: 15ml-10-30-13min) to obtain a white solid (SS,SaS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene (Z612, 27.1 mg, yield: 41 %, trifluoroacetate). ES-API: [M+H]⁺= 657.2. ¹H NMR (400 MHz, DMSO-*d6*) δ 8.24-8.16 (m, 3 H), 7.70-7.53 (m, 3 H), 5.16-5.13 (m, 1 H), 4.53-4.46 (m, 1 H), 4.31-4.21 (m, 2 H), 4.03-3.99 (m, 2 H), 3.63-3.62 (m, 1 H), 3.52-3.49 (m, 1 H), 3.12-3.05 (m, 2 H), 2.98-2.92 (m, 1 H), 2.78-2.55 (m, 3 H), 2.07-2.01 (m, 1 H), 1.93-1.57 (m, 8 H), 1.46-1.42 (m, 3 H).

### Example 51: Synthesis of (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z611)

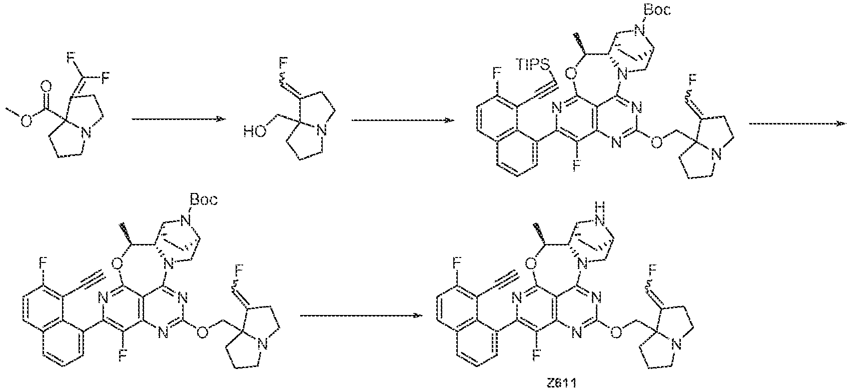

Step 1: Under nitrogen protection, methyl 1-(difluoromethylene)tetrahydro-1H-pyrrolizine-7a(5H)-carboxylate (319.3 mg, 1.47 mmol) and tetrahydrofuran (15 mL) were added to a round-bottom flask and cooled to 0°C. Lithium borohydride solution (10.0 mL, 10.0 mmol, 1M in tetrahydrofuran) was then added dropwise to the solution. After the addition was completed, the reaction mixture was stirred at 0°C for 30 minutes. 4M hydrochloric acid (1 mL) was added dropwise, and the mixture was stirred at room temperature for 30 minutes. 2M sodium hydroxide solution (5 mL) and water (20 mL) were added. The mixture was extracted with dichloromethane/methanol (10:1, 3x30 mL). The organic phase was dried over anhydrous sodium sulfate, filtered, and concentrated to obtain (1-(fluoromethylene)tetrahydro-1H-pyrrolizin-7a(5H)-yl)methanol (130 mg, yield: 46%), a yellow oily substance. ES-API: [M+H]⁺=172.1.

Step 2: (1-(Fluoromethylene)tetrahydro-1H-pyrrolizin-7a(5H)-yl)methanol (50.0 mg, 0.292 mmol) was dissolved in anhydrous tetrahydrofuran (5 mL) and under an ice-water bath, sodium hydride (48 mg, 1.20 mmol) was added. The reaction was proceeded for 30 minutes in the ice-water bath. A solution of tert-butyl (5S,5aS,6S,9R)-1-fluoro-2-(7-fluoro-8-((triisopropylsilyl)ethynyl)naphth-1-yl)-5-methyl-12-(methylsulfonyl)-5a,6,7,8,9,10-hexahydro-5H-4-oxa-3,10a,11,13,14-pentaza-6,9-methanenaphtho[1,8-ab]heptene-14-carboxylate (70.0 mg, 0.089 mmol) in tetrahydrofuran (2 mL) was added dropwise to the reaction mixture and reacted at 40°C for 30 minutes. Upon completion of the reaction, as monitored by LCMS, the reaction mixture was diluted with ethyl acetate (50 mL), washed with saturated ammonium chloride solution (2x15 mL), and saturated brine (3x50 mL), dried over anhydrous sodium sulfate, filtered, and the crude product was purified by silica gel column chromatography (dichloromethane: methanol = 15:1) to obtain tert-butyl (SS,SaS,6S,9R)-1-fluoro-2-(7-fluoro-8-((triisopropylsilyl)ethynyl)naphthalen-1-yl)-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (130 mg, crude product). ES-API: [M+H]⁺= 895.3.

Step 3: Cesium fluoride (96.0 mg, 0.63 mmol) was added to a solution of tert-butyl (5S,5aS,6S,9R)-1-fluoro-2-(7-fluoro-8-((triisopropylsilyl)ethynyl)naphthalen-1-yl)-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (130 mg, crude product) in N,N-dimethylformamide (5.0 mL). The reaction mixture was stirred at room temperature for 2 hours. Upon completion of the reaction, as monitored by LCMS, the reaction mixture was diluted with ethyl acetate (60 mL), washed with water (2x30 mL), and saturated brine (3x50 mL), dried over anhydrous sodium sulfate, filtered, and concentrated to obtain the crude product tert-butyl (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (100 mg, crude product). ES-API: [M+H]⁺= 739.2.

Step 4: Under an ice-water bath, 4 M hydrochloric acid-dioxane solution (3 mL) was slowly added to a solution of tert-butyl (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (100 mg, crude product) in acetonitrile (5.0 mL). The reaction mixture was stirred at 0°C for 0.5 hours. Upon completion of the reaction, as monitored by LCMS, the reaction mixture was concentrated under reduced pressure to dryness, and the residue was purified by preparative HPLC (ammonium bicarbonate method 2) (chromatographic condition: 15 ml-35-85-13 min) to obtain a pale yellow solid, (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z611, 4.8mg, total yield for 3 steps: 2.4 %). ES-API: [M+H]⁺= 639.3.

### Example 52: Synthesis of (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z632)

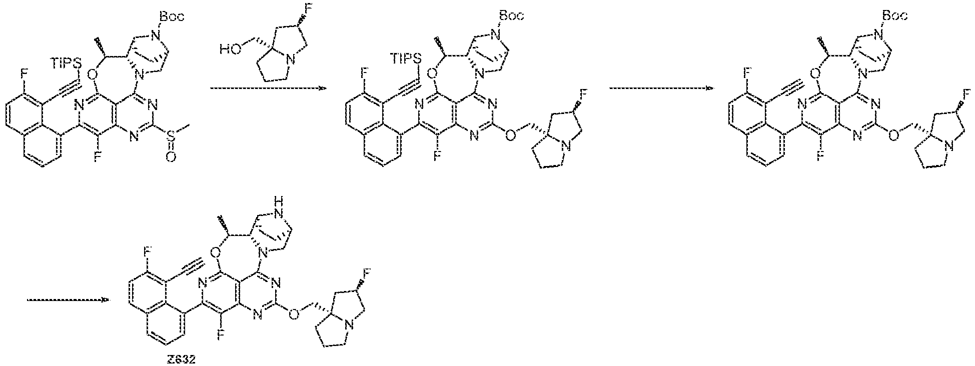

Step 1: ((2R,7aS)-2-fluorotetrahydro-1H-pyrrolizin-7a(5H)-yl)methanol (24 mg, 0.15 mmol) was dissolved in dry tetrahydrofuran (5 mL) at 0°C. Sodium hydride (15 mg, 0.38 mmol) was added, and the reaction mixture was stirred at 0°C for 15 minutes. Then, tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (60 mg, 0.076 mmol) was added, and the reaction was proceeded at 0°C for 1 hour. Upon completion of the reaction, the reaction mixture was quenched with water (10 mL), extracted with ethyl acetate (3x15 mL), dried over anhydrous sodium sulfate, filtered, and evaporated. The crude product was purified by silica gel column chromatography (50-100% ethyl acetate/petroleum ether) to obtain the compound tert-butyl (SS,SaS,6S,9R)-1-fluoro-2-(7-fluoro-8-((triisopropylsilyl)ethynyl)naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, yield: 60.4%). ES-API:[M+H]⁺=882.3.

Step 2: Tert-butyl (5S,5aS,6S,9R)-1-fluoro-2-(7-fluoro-8-((triisopropylsilyl)ethynyl)naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.045 mmol) was dissolved in N,N-dimethylformamide (2 mL), and cesium fluoride (68 mg, 0.45 mmol) was added. The reaction was proceeded at room temperature for 1 hour. The reaction mixture was extracted with ethyl acetate (3x10 mL), dried over anhydrous sodium sulfate, filtered, evaporated, and the product was obtained by preparative HPLC (trifluoroacetic acid method) as tert-butyl (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, yield: 76.4%). ES-API:[M+H]⁺=727.2.

Step 3: Tert-butyl (SS,SaS,bS,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.034 mmol) was dissolved in acetonitrile (5 mL). Under ice water bath, 4 M hydrochloric acid-dioxane solution (1 mL) was added, and the reaction mixture reacted under ice water bath for 2 hours. The reaction mixture was adjusted to pH 8 with saturated sodium bicarbonate solution, extracted with ethyl acetate (3x15 mL), evaporated, and the crude product was purified by preparative HPLC (trifluoroacetic acid method) to obtain the product (5S,5aS,6S,9R)-2-(8-ethynyl-7-fluoronaphthalen-1-yl)-1-fluoro-12-(((2R,7aS)-2-fluorotetrahydro-1H-pyrrolizin-7a(SH)-yl)methoxy)-5-methyl-5a,6,7,8,9,10-hexahydro-5H-4-oxa-3,10a, 11,13,14-pentaaza-6,9-methanonaphtho[1,8-ab]heptalene (Z632, 10.5mg, yield: 47.9%, trifluoroacetate). ES-API:[M+H]⁺=627.3. ¹H NMR (400 MHz, CD₃OD) δ 8.38 (s, 1H), 8.21 -7.90 (m, 2H), 7.76 -7.26 (m, 3H), 5.63 -5.35 (m, 2H), 4.71 -4.47 (m, 3H), 4.24 (s, 1H), 3.78 (dt, J = 29.2, 23.7 Hz, 6H), 3.33 (s, 2H), 2.29 (ddd, J = 136.4, 66.9, 25.3 Hz, 12H), 1.60 (t, J = 6.0 Hz, 3H).

### Example 53: Synthesis of (5S,5aS,6S,9R)-12-((1,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ablheptalene (Z610)

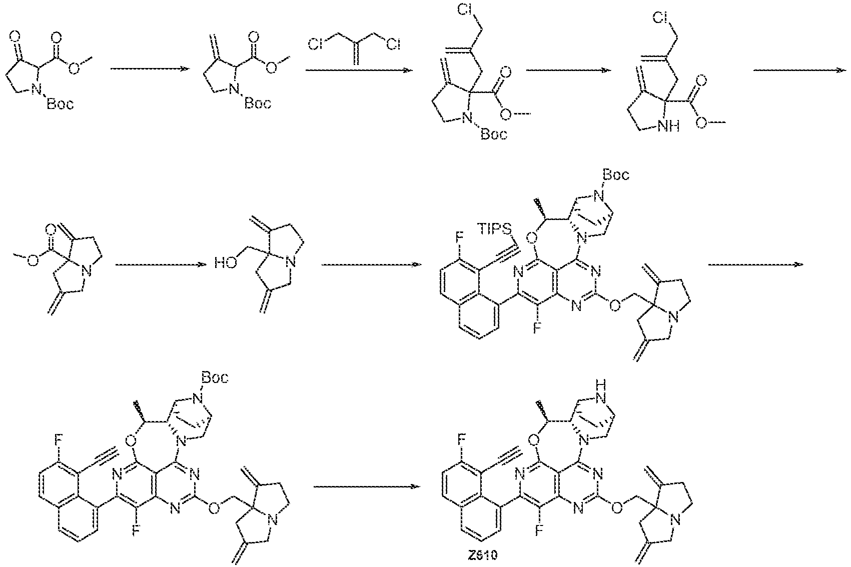

Step 1: Methyltriphenylphosphonium bromide (18.35 g, 51.38 mmol) was added to tetrahydrofuran (150 mL) and cooled to 0°C. Under a nitrogen atmosphere, at 0°C, a tetrahydrofuran solution of potassium tert-butoxide (51.38 mL, 51.38 mmol, 1M) was added to the mixture. After the addition was complete, the reaction mixture was stirred at room temperature for 1 hour. 1-(tert-butyl) 2-methyl 3-oxopyrrolidine-1,2-dicarboxylate (5.0 g, 20.55 mmol) was dissolved in tetrahydrofuran (20 mL) and slowly added to the above reaction mixture under a nitrogen atmosphere. After the addition was complete, the reaction was further stirred at room temperature for 3 hours. Saturated ammonium chloride (30 mL) was added to the reaction mixture, which was then extracted with ethyl acetate (3x100 mL). The organic phase was washed with saturated brine (200 mL), dried over anhydrous sodium sulfate, filtered, concentrated, and the crude product was purified by fast silica gel column (ethyl acetate/petroleum ether: 0%-8%) to obtain 1-(tert-butyl) 2-methyl 3-methylenepyrrolidine-1,2-dicarboxylate (1.2 g, yield 24.2%) as a colorless transparent liquid. ES-API: [M-Boc+H]⁺=186.1.

Step 2: At -78°C, bis(trimethylsilyl)aminolithium (9.2 mL, 9.2 mmol) was added dropwise to a dry tetrahydrofuran (15.0 mL) solution of 1-(tert-butyl) 2-methyl 3-methylenepyrrolidine-1,2-dicarboxylate (1.1 g, 4.56 mmol), and the mixture was stirred at this temperature for 1 hour. 1 hour later, 3-chloro-2-chloromethylpropene (2.85 g, 22.794 mmol) was slowly added dropwise to the above solution, and the reaction was slowly warmed to room temperature and reacted for 2 hours. Upon completion of the reaction, water (100 mL) was added to the reaction mixture, which was then extracted with ethyl acetate (4x100 mL). Washed with saturated brine (3x200 mL), dried over anhydrous sodium sulfate, filtered, concentrated, and the crude product was purified by fast silica gel column (ethyl acetate/petroleum ether: 0-10%) to obtain 1-(tert-butyl) 2-methyl 2-(2-(chloromethyl)allyl)-3-methylenepyrrolidine-1,2-dicarboxylate (899 mg, yield 57.8%) as a colorless transparent oily liquid. ES-API: [M-Boc+H]⁺=274.1.

Step 3: At 0°C, a dioxane solution of hydrochloric acid (10 mL, 10.000 mmol) was added to a solution of 1-(tert-butyl) 2-methyl 2-(2-(chloromethyl)allyl)-3-methylenepyrrolidine-1,2-dicarboxylate (900 mg, 2.729 mmol) in acetonitrile (5.0 mL). The reaction mixture was stirred at this temperature for 1.5 hours. Upon completion of the reaction, the solvent was evaporated under reduced pressure to obtain the crude product methyl 2-(2-(chloromethyl)allyl)-3-methylenepyrrolidine-2-carboxylate (626.80 mg, crude), which was then used directly in the next step. ES-API: [M+H]⁺=274.1.

Step 4: At 0°C, potassium iodide (45.24 mg, 0.273 mmol) and sodium bicarbonate (1144.76 mg, 13.626 mmol) were added to a solution of methyl 2-(2-(chloromethyl)allyl)-3-methylenepyrrolidine-2-carboxylate (626.80 mg, 2.725 mmol) in acetonitrile (5.0 mL). The reaction was proceeded overnight at room temperature. Upon completion of the reaction, with water (100 mL) added, the reaction mixture was extracted with ethyl acetate (4×100 mL), washed with saturated brine (3x200 mL), dried over anhydrous sodium sulfate, filtered, concentrated, and the crude product was purified by fast silica gel column (methanol/dichloromethane: 0-10%) to obtain methyl 1,6-dimethylenetetrahydro-1H-pyrrolizine-7a(5H)-carboxylate (400 mg, 75.96%). ES-API: [M+H]⁺=194.2.

Step 5: Methyl 1,6-dimethylenetetrahydro-1H-pyrrolizine-7a(5H)-carboxylate (200 mg, 1.036 mmol) was dissolved in tetrahydrofuran (10 mL) and cooled to 0°C. Under a nitrogen atmosphere, a solution of lithium aluminium hydride (2.0 mL, 2.0 mmol, 1M in tetrahydrofuran) was added to the above solution. The reaction mixture was stirred at 0°C for 1 hour. Water (0.03 mL) was added to quench the reaction, followed by 15% sodium hydroxide solution (0.03 mL) and water (0.09 mL), then tetrahydrofuran (10 mL), filtered, and the filtrate was concentrated to obtain (1,6-dimethylenetetrahydro-1H-pyrrolizin-7a(5H)-yl)methanol (130 mg, yield 77%) as a colorless transparent liquid. ES-API: [M +H]⁺=166.1.

Step 6: (1,6-dimethylenetetrahydro-1H-pyrrolizin-7a(5H)-yl)methanol (60 mg, 0.3610 mmol) was dissolved in anhydrous tetrahydrofuran (5 mL). Under a ice water bath, sodium hydride (58.0 mg, 1.444 mmol) was added and the mixture reacted under a ice water bath for 30 minutes. A solution of tert-butyl (5S,5aS,6S,9R)-1-fluoro-2-(7-fluoro-8-((triisopropylsilyl)ethynyl)naphthalene-1-yl)-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (90 mg, 0.113 mmol) in tetrahydrofuran (2 mL) was added dropwise to the above reaction mixture and the mixture reacted at 40°C for 30 minutes. Upon completion of the reaction (monitored by LCMS), the reaction mixture was diluted with ethyl acetate (50 mL), washed with saturated ammonium chloride solution (2x15 mL), and saturated brine (3x50 mL), dried over anhydrous sodium sulfate, filtered, concentrated, and the crude product was purified by silica gel column chromatography (dichloromethane: methanol = 15:1) to obtain tert-butyl (5S,5aS,6S,9R)-12-((1,6-dimethylenetatrahydro-1H-pyrrolizin-7a(5H)-yl)methoxy)-1-fluoro-2-(7-fluoro-8-((triisopropylsilyl)ethynyl)naphthalen-1-yl)-5-methyl-Sa,6,7,8,9,10-hexahydro-SH-4-oxa-3,10a,11,13,14-pentaaza-6,9-methanonaphtho[1,8-ab]heptalene-14-carboxylate (130 mg, crude product). ES-API: [M+H]⁺= 889.3.

Step 7: Cesium fluoride (96.0 mg, 0.63 mmol) was added to a solution of tert-butyl (5S,5aS,6S,9R)-12-((1,6-dimethylenetetrahydro-1H-pyrrolizin-7a(5H)-yl)methoxy)-1 -fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (130 mg, crude product) in N,N-dimethylformamide (5.0 mL). The reaction mixture was stirred at room temperature for 2 hours. Upon completion of the reaction (monitored by LCMS), the reaction mixture was diluted with ethyl acetate (60 mL), washed with water (2x30 mL) and saturated brine (3x50 mL), dried over anhydrous sodium sulfate, filtered, and concentrated to obtain the crude product tert-butyl (5S,5aS,6S,9R)-12-((1,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (110 mg, crude product). ES-API: [M+H]⁺= 733.2.

Step 8: Under an ice-water bath, 4 M hydrochloric acid-dioxane solution (3 mL) was slowly added to a solution of tert-butyl (5S,5aS,6S,9R)-12-((1,6-dimethylenetetrahydro-1H-pyrrolizin-7a(5H)-yl)methoxy)-2-(8-ethynyl-7-fluoronaplxthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (110 mg, crude product) in acetonitrile (5.0 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic condition: 15ml-35-85-13min) to obtain a light yellow solid, (5S,5aS,6S,9R)-12-((1,6-dimethylenetetrahydro-1H-pyrrolizin-7a(5H)-yl)methoxy)-2-(8-ethynyl-7-fluoronaphthalen-1 -yl)-1 -fluoro-5-methyl-5a,6,7,8,9,10-hexahydro-5H-4-oxa-3,10a,11,13,14-pentaaza-6,9-methanonaphtho[1,8-ab]heptalene (Z610, 15.0mg, total yield for 3 steps: 20.8 %). ES-API: [M+H]⁺= 633.3.

### Example 54: Synthesis of (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z633)

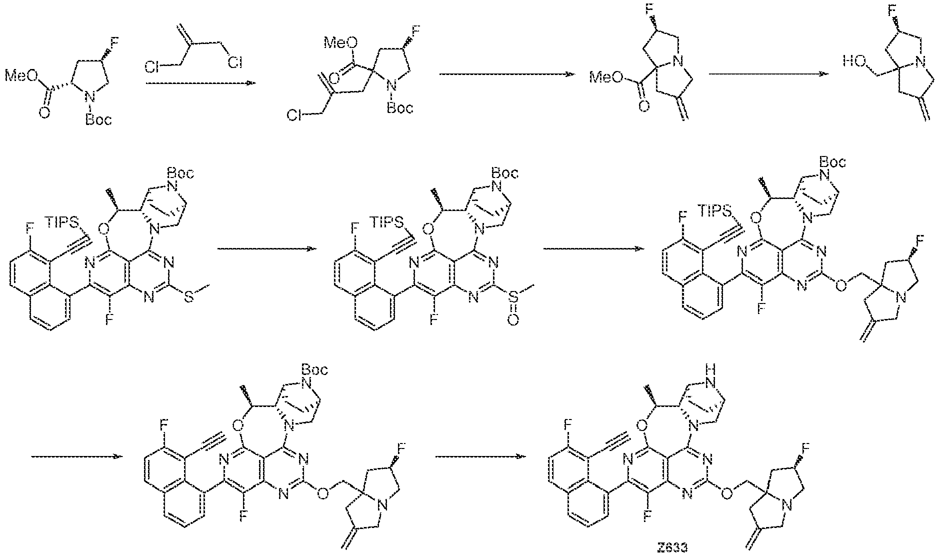

Step 1: Under nitrogen protection, 1-(tert-butyl) 2-methyl (2S,4R)-4-fluoropyrrolidine-1,2-dicarboxylate (1.24 g, 5.0 mmol) was dissolved in anhydrous tetrahydrofuran (20.0 mL). The mixture was placed in a dry ice-ethanol bath. Bis(trimethylsilyl)aminolithium (10.0 mL, 10.0 mmol, 1 M in tetrahydrofuran) was slowly added dropwise to the mixture above, and stirred for 15 minutes at this temperature. Then, 3-chloro-2-chloromethylacrylate (1.39 mL, 15.0 mmol) was slowly added to the mixture above. After stirred at -78 °C for 1 hour, the reaction mixture was quenched with saturated ammonium chloride solution, extracted three times with ethyl acetate, washed with saturated brine, dried over anhydrous sodium sulfate, filtered, concentrated, and the crude product was purified by flash silica gel column (ethyl acetate/petroleum ether: 0-20%) to obtain 1-(tert-butyl) 2-methyl (4R)-2-(2-(chloromethyl)allyl)-4-fluoropyrrolidine-1,2-dicarboxylate (1.43 g, yield: 85%). ES-API: [M-55]⁺= 280.0.

Step 2: At 0 °C, 4 M hydrochloric acid-dioxane solution (2.5 mL) was slowly added to a solution of 1-(tert-butyl) 2-methyl (4R)-2-(2-(chloromethyl)allyl)-4-fluoropyrrolidine-1,2-dicarboxylate (336 mg, 1.0 mmol) in acetonitrile (10.0 mL). After the reaction mixture stirred at room temperature for 1 hour, sodium bicarbonate (5 g) was added and the mixture was stirred at room temperature for another 1 hour, filtered, and the filtrate was taken and concentrated under reduced pressure. The crude product was purified by flash silica gel column (methanol/dichloromethane: 0-8%) to obtain methyl (2R)-2-fluoro-6-methylenetetrahydro-1H-pyrrolizine-7a(5H)-carboxylate (200 mg, yield: 100%). ES-API: [M+H]⁺= 200.1.

Step 3: Under nitrogen protection, methyl (2R)-2-fluoro-6-methylenetetrahydro-1H-pyrrolizine-7a(5H)-carboxylate (99 mg, 0.50 mmol) was dissolved in anhydrous tetrahydrofuran (5.0 mL). The mixture was placed in an ice-water bath, with lithium aluminum hydride (0.60 mL, 0.60 mmol, 1 M in tetrahydrofuran) slowly added, stirred for 0.5 hours at this temperature. The reaction mixture was quenched with sodium sulfate decahydrate, warmed to room temperature, and stirred for another 0.5 hours. The mixture was filtered. The filtrate was concentrated under reduced pressure to obtain ((2R)-2-fluoro-6-methylenetetrahydro-1H-pyrrolizin-7a(5H)-yl)methanol (74 mg, yield: 86%). ES-API: [M+H]⁺= 172.0.

Step 4: At 0°C, m-chloroperbenzoic acid (17 mg, 0.08 mmol, 85% purity) was added to a dichloromethane (5.0 mL) solution of tert-butyl (SS,SaS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate(50 mg, 0.065 mmol). After the mixture stirred at 0°C for 0.5 hours, saturated sodium bicarbonate solution and saturated sodium sulfite solution were added. After vigorous stirred for 15 minutes, the organic phase was separated, the aqueous phase was extracted twice with dichloromethane, and the organic phases were collected, washed with saturated brine, dried over anhydrous sodium sulfate, filtered, and evaporated to obtain a crude product of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (crude product), which was then directly used in the next reaction without purification. ES-API: [M+H]⁺= 788.3.

Step 5: At 0°C, sodium hydride (60% dispersion in oil) (10 mg, 0.26 mmol) was added to a tetrahydrofuran (5.0 mL) solution of ((2R)-2-fluoro-6-methylenetetrahydro-1H-pyrrolizin-7a(5H)-yl)methanol (crude product) from Step 3. After the mixture reacting for 15 minutes, a tetrahydrofuran solution (1.0 mL) of the crude product from Step 4, tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (crude product), was slowly added to the reaction mixture. After the mixture reacting at 0°C for 0.5 hours, saturated ammonium chloride solution was slowly added. The mixture was extracted three times with dichloromethane, and the organic phases were collected, washed with saturated brine, dried over anhydrous sodium sulfate, filtered, and evaporated. The crude product was purified by preparative thin-layer chromatography (ethyl acetate/petroleum ether 80%) to obtain tert-butyl (5S,5aS,6S,9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (18 mg, yield: 31%). ES-API: [M+H]⁺= 895.3.

Step 6: Tert-butyl (5S,5aS,6S,9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.034 mmol) was dissolved in N,N-dimethylformamide (2.0 mL), and cesium fluoride (31 mg, 0.20 mmol) was added. The reaction mixture reacted at room temperature for 0.5 hours. Upon completion of the reaction, water was added to the reaction mixture, which was then extracted three times with ethyl acetate. The combined organic phases were washed with saturated brine, dried over anhydrous sodium sulfate, filtered, and evaporated to obtain tert-butyl (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (crude product), which was then directly used in the next reaction without purification. ES-API:[M+H]⁺= 739.0.

Step 7: Tert-butyl (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (crude product) was dissolved in acetonitrile (5.0 mL). Under an ice water bath, 4 M hydrochloric acid/dioxane solution (3.0 mL, 13.0 mmol) was added and the reaction mixture and the mixture was warmed to room temperature and reacted for 3 hours. Upon completion of the reaction, the reaction mixture was concentrated to dryness. The crude product was purified by preparative HPLC (ammonium bicarbonate method 1) to obtain (SS,SaS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene (Z633, 3.69 mg, & yield: 29%). ES-API: [M+H]⁺= 639.2.

### Example 55: Synthesis of (5S,5aS,6S,9R)-2-(8-ethynyl-7-fluoronaphthalen-1-yl)-1-fluoro-12-(((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z634)

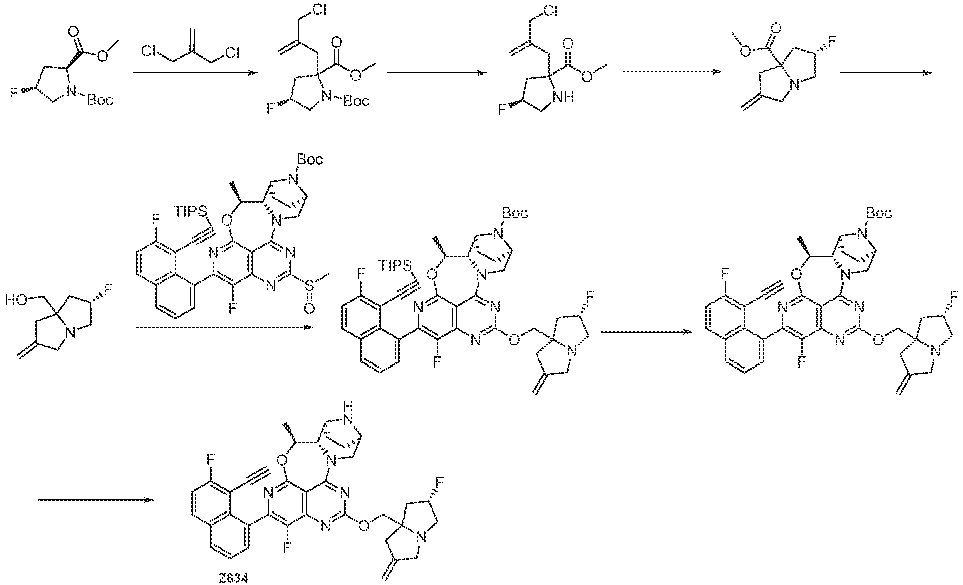

Step 1: Under nitrogen protection, 1-(tert-butyl) 2-methyl (2S,4S)-4-fluoropyrrolidine-1,2-dicarboxylate (1 g, 4.044 mmol) was dissolved in anhydrous tetrahydrofuran (20 mL) and the mixture was cooled in a dry ice-acetone bath. Diisopropylamine lithium (8.0 mL, 8.088 mmol) was slowly added dropwise to the solution. After the solution stirred for 1 hour at this temperature, then 3-chloro-2-chloromethylpropene (1.516 g, 12.132 mmol) in anhydrous tetrahydrofuran (10 mL) was slowly added to the above solution via a syringe. The reaction mixture was stirred at -78 °C for 1 hour, then slowly warmed to room temperature and stirred for 2 hours. Upon completion of the reaction (monitored by LCMS), the reaction mixture was quenched with saturated ammonium chloride solution (30 mL), extracted with ethyl acetate (20 mL × 3), washed with saturated brine (15 mL × 3), dried over anhydrous sodium sulfate, filtered, concentrated, and the crude product was purified by silica gel column chromatography (ethyl acetate:petroleum ether = 0∼15%) to obtain 1-(tert-butyl) 2-methyl (4S)-2-(2-(chloromethyl)allyl)-4-fluoropyrrolidine-1,2-dicarboxylate (1190 mg, yield: 88%). ES-API: [M-55]⁺= 280.1.

Step 2: At room temperature, 4 M hydrochloric acid-dioxane solution (15 mL) was slowly added to 1-(tert-butyl) 2-methyl (4S)-2-(2-(chloromethyl)allyl)-4-fluoropyrrolidine-1,2-dicarboxylate (1190 mg, 3.544 mmol) in acetonitrile (3 mL). The reaction mixture was stirred at room temperature for 3 hours. Upon completion of the reaction (monitored by LCMS), the reaction mixture was concentrated under reduced pressure to obtain crude methyl (4S)-2-(2-(chloromethyl)allyl)-4-fluoropyrrolidine-2-carboxylate (835 mg). ES-API: [M+H]⁺= 236.1.

Step 3: At room temperature, sodium bicarbonate (1.488 g, 17.715 mmol) and potassium iodide (59 mg, 0.354 mmol) were added to methyl (4S)-2-(2-(chloromethyl)allyl)-4-fluoropyrrolidine-2-carboxylate (835 mg, 3.543 mmol) in acetonitrile (25 mL). The reaction mixture was stirred at room temperature for 2 hours. The reaction was monitored by LCMS, filtered, and the insoluble material was washed with acetonitrile. The filtrate was concentrated under reduced pressure and the residue was purified by silica gel column chromatography (ethyl acetate:petroleum ether = 0∼45%) to obtain methyl (2S)-2-fluoro-6-methylenetetrahydro-1H-pyrrolizine-7a(5H)-carboxylate (611 mg, yield: 87%). ES-API: [M+H]⁺= 200.2.

Step 4: Under nitrogen protection, methyl (2S)-2-fluoro-6-methylenetetrahydro-1H-pyrrolizine-7a(5H)-carboxylate (200 mg, 1.004 mmol) was dissolved in anhydrous tetrahydrofuran (5 mL) and the mixture was cooled in an ice water bath. Lithium aluminum hydride (2 mL, 2.008 mmol, 1 M in tetrahydrofuran) was slowly added dropwise to the solution, and the mixture was stirred for 0.5 hours at this temperature. Upon completion of the reaction (monitored by LCMS), the reaction mixture was quenched with sodium thiosulfate decahydrate, stirred at room temperature for 0.5 hours, and the insoluble material was filtered. The filtrate was concentrated under reduced pressure to obtain crude ((2S)-2-fluoro-6-methylenetetrahydro-1H-pyrrolizin-7a(5H)-yl)methanol (171 mg). ES-API: [M+H]⁺= 172.1.

Step 5: ((2S)-2-fluoro-6-methylenetetrahydro-1H-pyrrolizin-7a(5H)-yl)methanol (52 mg, 0.305 mmol) was dissolved in anhydrous tetrahydrofuran (5 mL). Under an ice water bath, sodium hydride (12 mg, 0.305 mmol) was added and the reaction was proceeded for 30 minutes under ice water bath. The solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (81 mg, 0.103 mmol) in tetrahydrofuran (2 mL) was added dropwise to the reaction mixture, and the mixture reacted at 0°C for 30 minutes. Upon completion of the reaction (monitored by LCMS), the reaction mixture was diluted by adding ethyl acetate (10 mL), washed with saturated ammonium chloride solution (10 mL × 2) and saturated brine (10 mL × 3), dried over anhydrous sodium sulfate, filtered, and concentrated under reduced pressure to obtain the crude tert-butyl (5S,5aS,6S,9R)-1-fluoro-12-(((2S)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (90 mg). ES-API: [M+H]⁺= 895.3.

Step 6: Cesium fluoride (153 mg, 1.010 mmol) was added to N,N-dimethylformamide (5 mL) containing tert-butyl (5S,5aS,6S,9R)-1-fluoro-12-(((2S)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (90 mg, 0.101 mmol). The reaction mixture was stirred at room temperature for 1 hour. Upon completion of the reaction (monitored by LCMS), the reaction mixture was diluted with ethyl acetate (15 mL), washed with water (15 mL × 2), and saturated brine (15 mL × 3), dried over anhydrous sodium sulfate, filtered, and concentrated under reduced pressure to obtain the crude tert-butyl (5S,5aS,6S,9R)-2-(8-ethynyl-7-fluoronaphthalen-1-yl)-1-fluoro-12-(((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (74 mg). ES-API: [M+H]⁺= 739.3.

Step 7: Under an ice water bath, 4 M hydrochloric acid-dioxane solution (5 mL) was slowly added to a acetonitrile solution (2 mL) of tert-butyl (SS,SaS,6S,9R)-2-(8-ethynyl-7-fluoronaphthalen-1-yl)-1-fluoro-12-(((2S)-2-fluoro-6-methylenetetrahydro-1H-pyrrolizin-7a(SH)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (74 mg, 0.100 mmol). The reaction mixture was stirred at 0°C for 0.5 hours. Upon completion of the reaction (monitored by LCMS), the reaction mixture was concentrated under reduced pressure, and the residue was purified by preparative HPLC (ammonium bicarbonate method 2) (chromatographic condition: 15ml-50-60-13min) to obtain a off-white solid (5S,5aS,6S,9R)-2-(8-ethynyl-7-fluoronaphthalen-1-yl)-1-fluoro-12-(((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z634, 11.36 mg, yield: 18 %). ES-API: [M+H]⁺= 639.3. ¹H NMR (400 MHz, DMSO-*d6*) δ 8.24-8.16 (m, 2 H), 7.70-7.52 (m, 3 H), 5.45-5.29 (m, 1 H), 5.15-5.12 (m, 1 H), 4.94-4.93 (m, 2 H), 4.53-4.45 (m, 1 H), 4.10-3.97 (m, 3 H), 3.61-3.58 (m, 2 H), 3.50-3.47 (m, 1 H), 3.27-3.18 (m, 2 H), 3.09-3.03 (m, 1 H), 2.93-2.80 (m, 1 H), 2.67-2.63 (m, 1 H), 2.45-2.00 (m, 5 H), 1.85-1.55 (m, 4 H), 1.46-1.40 (m, 3 H).

### Example 56: Synthesis of (5S,5aS,6S,9R)-2-(8-ethynyl-7-fluoronaphthalen-1-yl)-1-fluoro-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z635)

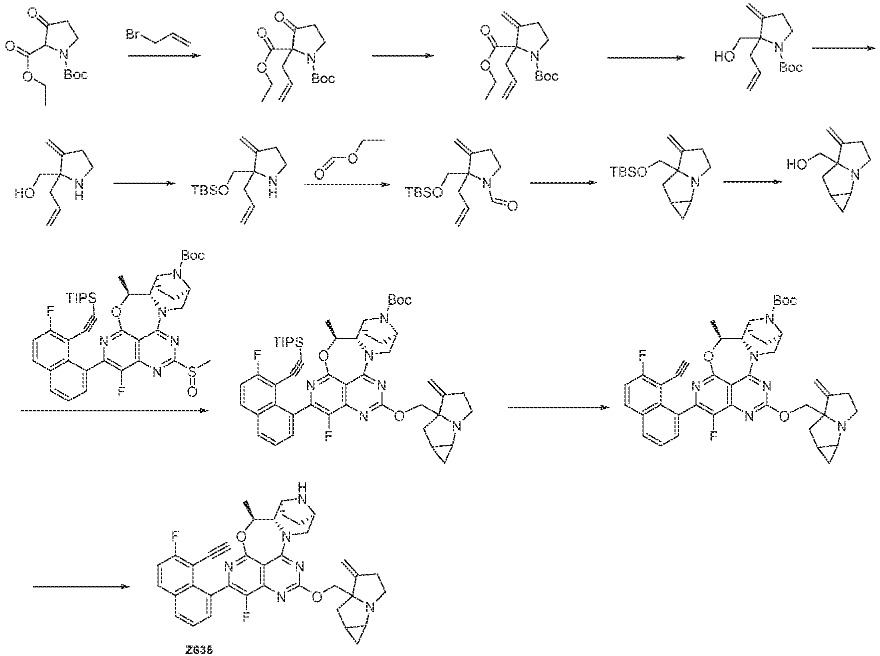

Step 1: Under nitrogen protection, 1-(tert-butyl) 2-ethyl 3-oxopyrrolidine-1,2-dicarboxylate (2000 mg, 7.773 mmol), 3-bromoprop-1-ene (1410.63 mg, 11.660 mmol), potassium carbonate (2148.55 mg, 15.547 mmol), and potassium iodide (2580.75 mg, 15.547 mmol) were mixed with N,N-dimethylformamide (50 mL) and stirred overnight at room temperature. Water (100 mL) was added to the reaction mixture, and the mixture was extracted with ethyl acetate (100 mL × 2). The organic phase was washed with saturated brine (200 mL × 3), dried over anhydrous sodium sulfate, filtered, concentrated, and the crude product was purified by flash silica gel column chromatography (ethyl acetate/petroleum ether: 0-15%) to obtain 1-(tert-butyl) 2-ethyl 2-allyl-3-oxopyrrolidine-1,2-dicarboxylate (1700 mg, yield 73.55%), a colorless transparent liquid. ES-API: [M-C₄H₈+H]⁺ =242.2

Step 2: Methyltriphenylphosphonium bromide (10.21 g, 28.586 mmol) was added to tetrahydrofuran (100 mL) and cooled to 0°C. Under nitrogen atmosphere at 0°C, a solution of potassium tert-butoxide in tetrahydrofuran (1 M, 22.869 mL, 22.869 mmol) was added to the mixture. After the addition was complete, the reaction was warm to room temperature and stirred for 0.5 hours. 1-(tert-butyl) 2-ethyl 2-allyl-3-oxopyrrolidine-1,2-dicarboxylate (1700 mg, 5.717 mmol) dissolved in tetrahydrofuran (5 mL) was then slowly added to the reaction solution under nitrogen atmosphere. After the dropwise addition was complete, the reaction continued to stir at 50°C for 5 hours. Saturated ammonium chloride solution (30 mL) was added, and the reaction mixture was extracted with ethyl acetate (60 mL × 3). The organic phase was washed with saturated brine (200 mL), dried over anhydrous sodium sulfate, filtered, concentrated, and the crude product was purified by flash silica gel column chromatography (ethyl acetate/petroleum ether: 0%-8%) to obtain 1-(tert-butyl) 2-ethyl 2-allyl-3-methylenepyrrolidine-1,2-dicarboxylate (1200 mg, yield 71.06%), a colorless transparent liquid. ES-API: [M-Boc+H]⁺=196.0.

Step 3: 1-(tert-butyl) 2-ethyl 2-allyl-3-methylenepyrrolidine-1,2-dicarboxylate (1000 mg, 3.385 mmol) was dissolved in tetrahydrofuran (10 mL) and ethanol (2 mL) and cooled to 0°C. Under a nitrogen atmosphere, a solution of lithium borohydride in tetrahydrofuran (2M, 3.385 mL, 6.771 mmol) was added. After the addition was complete, the reaction mixture was stirred at 40°C for 1 hour. Dilute hydrochloric acid solution (2 M) was added to quench the reaction, followed by the addition of 15% sodium hydroxide solution to adjust the pH to 8. The obtained mixture was extracted with dichloromethane/methanol (10: 1, v/v, 30 mL × 3). The organic phase was washed with saturated brine (30 mL), dried over anhydrous sodium sulfate, filtered, concentrated, and the crude product was purified by flash silica gel column chromatography (methanol/dichloromethane: 0%-8%) to obtain tert-butyl 2-allyl-2-(hydroxymethyl)-3-methylenepyrrolidine-1-carboxylate (650 mg, yield 75.79%), a colorless transparent liquid. ES-API: [M-C₄H₈+H]⁺=198.1.

Step 4: Tert-butyl 2-allyl-2-(hydroxymethyl)-3-methylenepyrrolidine-1-carboxylate (650 mg, 2.566 mmol) was dissolved in a hydrochloric acid-dioxane solution (4 M, 10 mL) and the mixture was stirred at room temperature for 3 hours. The reaction mixture was concentrated to obtain (2-allyl-3-methylenepyrrolidin-2-yl)methanol (486.67 mg, yield 100%), ES-API: [M+H]⁺=154.1.

Step 5: (2-allyl-3-methylenepyrrolidin-2-yl)methanol (390 mg, 2.056 mmol) was dissolved in dichloromethane and cooled to 0°C. Under nitrogen atmosphere at 0°C, triethylamine (0.857 mL, 6.168 mmol) and tert-butyldimethylchlorosilane (402.86 mg, 2.673 mmol) were added to the solution. After the addition was complete, the reaction mixture was warm to room temperature and stirred for 17 hours. After completion of the reaction, the reaction mixture was concentrated to obtain a crude product. The crude product was purified by flash silica gel column (methanol/dichloromethane: 0%-2%) to obtain 2-allyl-2-(((tert-butyldimethylsilyl)oxy)methyl)-3-methylenepyrrolidine (500 mg, 90.91%), a colorless transparent liquid. ES-API: [M+H]⁺=268.1.

Step 6: 2-allyl-2-(((tert-butyldimethylsilyl)oxy)methyl)-3-methylenepyrrolidine (400 mg, 1.495 mmol) and ethyl formate (5 mL) were added to a sealed tube reactor. The reactor was closed and the mixtrue was stirred at 100°C for 17 hours. After completion of the reaction, the reaction mixture was concentrated to obtain 2-allyl-2-(((tert-butyldimethylsilyl)oxy)methyl)-3-methylenepyrrolidine-1-carbaldehyde (400 mg, 90.52%), a colorless transparent liquid. ES-API: [M+H]⁺=296.1.

Step 7: 2-allyl-2-(((tert-butyldimethylsilyl)oxy)methyl)-3-methylenepyrrolidine-1-carbaldehyde (400 mg, 1.36 mmol) was dissolved in anhydrous tetrahydrofuran and cooled to 0°C. Under nitrogen atmosphere at 0°C, tetrakis(triphenylphosphine)palladium(0) (425 mg, 1.5 mmol) and cyclohexylmagnesium bromide (4.76 mL, 4.76 mmol, 1.0 M solution in THF) were added. After the addition was complete, the reaction mixture was warm to room temperature and stirred for 17 hours. After completion of the reaction, the reaction mixture was diluted with ethyl acetate (50 mL), washed with saturated ammonium chloride solution (15 mL × 2), saturated brine (15 mL × 3), dried over anhydrous sodium sulfate, filtered, and the filtrate concentrated under reduced pressure. The crude product was purified by flash silica gel column (methanol/dichloromethane: 0%-5%) to obtain 5a-(((tert-butyldimethylsilyl)oxy)methyl)-5-methyleneoctahydrocyclopropa[b]pyrrolizine (260 mg, yield 61%), a colorless transparent liquid. ES-API: [M+H]⁺=280.3.

Step 8: At room temperature, a hydrochloric acid-dioxane solution (4 M, 10 mL) was slowly added to a solution of 5a-(((tert-butyldimethylsilyl)oxy)methyl)-5-methyleneoctahydrocyclopropa[b]pyrrolizine (260 mg, 0.93 mmol) in acetonitrile (2 mL). The reaction mixture was stirred at room temperature for 0.5 hours. After completion of the reaction (monitored by LCMS), the reaction mixture was concentrated under reduced pressure to obtain crude (5-methylenehexahydrocyclopropa[b]pyrrolizin-5a(3H)-yl)methanol (153 mg, yield: 100 %). ES-API: [M+H]⁺=166.2.

Step 9: (5-methylenehexahydrocyclopropa[b]pyrrolizin-5a(3H)-yl)methanol (44 mg, 0.26 mmol) was dissolved in anhydrous tetrahydrofuran (2 mL). Under an ice-water bath, sodium hydride (14 mg, 0.36 mmol) was added, and the mixture reacted for 30 minutes under the ice-water bath. A solution of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (102 mg, 0.13 mmol) in tetrahydrofuran (2 mL) was added dropwise to the reaction mixture, and the reaction was proceeded at room temperature for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous sodium sulfate, filtered, and the filtrate concentrated under reduced pressure to obtain the crude product tert-butyl (SS,SaS,6S,9R)-1-fluoro-2-(7-fluoro-8-((triisopropylsilyl)ethynyl)naphthalen-1-yl)-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (115 mg, yield: 100%). ES-API: [M+H]⁺= 889.3.

Step 10: Cesium fluoride (196 mg, 1.3 mmol) was added to N,N-dimethylformamide (2.0 mL) containing tert-butyl (5S,5aS,6S,9R)-1-fluoro-2-(7-fluoro-8-((triisopropylsilyl)ethynyl)naphthalen-1-yl)-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (115 mg, 0.13 mmol). The reaction mixture was stirred at room temperature for 2 hours. Upon completion of the reaction (monitored by LCMS), the reaction mixture was diluted with ethyl acetate (20 mL), washed with water (15 mL × 2), saturated brine (15 mL × 3), dried over anhydrous sodium sulfate, filtered, and concentrated to obtain the crude product tert-butyl (5S,5aS,6S,9R)-2-(8-ethynyl-7-fluoronaphthalen-1-yl)-1-fluoro-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (94 mg, yield 100%). ES-API: [M+H]⁺= 733.2.

Step 11: A hydrochloric acid-dioxane solution (4 M, 5 mL) was slowly added to a solution of tert-butyl (5S,5aS,6S,9R)-2-(8-ethynyl-7-fluoronaphthalen-1-yl)-1-fluoro-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene-14-carboxylate (94 mg, 0.13 mmol) in acetonitrile (2 mL) under an ice-water bath. The reaction mixture was stirred at 0°C for 0.5 hours. Upon completion of the reaction (monitored by LCMS), the reaction mixture was concentrated to dryness, and the residue was purified by preparative HPLC (ammonium bicarbonate method 2) (Chromatographic condition: 15ml-35-85-13min) to obtain a pale yellow solid (5S,5aS,6S,9R)-2-(8-ethynyl-7-fluoronaphthalen-1-yl)-1-fluoro-5-methyl-12-((5-methylenehexahydrocyclopropa[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptalene (Z635, 8 mg, yield: 9 %). ES-API: [M+H]⁺= 633.3

### Example 57 Synthesis of (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z636)

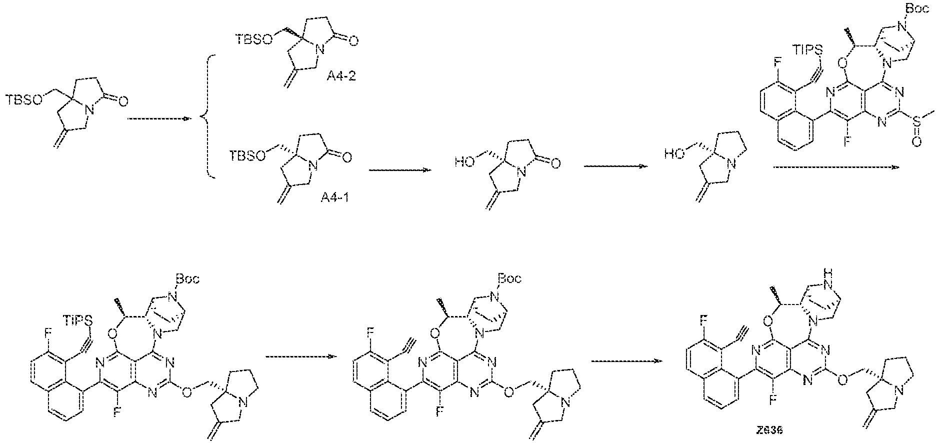

Step 1: 7a-(((tert-butyldimethylsilyl)oxy)methyl)-6-methylenehexahydro-3H-pyrrolizin-3-one (3.1 g) was subjected to chiral preparative separation (separation column IC, 250mm*4.6mm*5um, mobile phase: n-hexane: ethanol = 90:10:0.2, flow rate: 1ml/min, column temperature: 30°C) yielding two isomeric compounds. One of the structures was arbitrarily designated as (R)-7a-(((tertbutyldimethylsilyl)oxy)methyl)-6-methylenehexahydro-3H-pyrrolizin-3-one (A4-1, 1.2 g, peak 1, retention time 7.385min, 100%), a colorless liquid. ES-API: [M+H]+= 282.2. The other structure was arbitrarily designated as (S)-7a-(((tert-butyldimethylsilyl)oxy)methyl)-6-methylenehexahydro-3H-pyrrolizin-3-one (A4-2, 1.3 g, peak 2, retention time 9.205min, 100%), a colorless liquid. ES-API: [M+H]⁺= 282.2.

Step 2: At room temperature, a 4M hydrochloric acid/dioxane solution (10 mL) was added slowly to the acetonitrile (2 mL) solution of (R)-7a-(((tert-butyldimethylsilyl)oxy)methyl)-6-methylenehexahydro-3H-pyrrolizin-3-one (A4-1, retention time 7.385min, 1.2 g, 4.3 mmol) and stirred at room temperature for 0.5 hours. Upon completion of the reaction (monitored by LCMS), the reaction mixture was concentrated under reduced pressure to obtain a crude product, (R)-7a-(hydroxymethyl)-6-methylenehexahydro-3H-pyrrolizin-3-one (712 mg, yield: 100%). ES-API: [M+H]⁺=168.1.

Step 3: Under nitrogen protection, (R)-7a-(hydroxymethyl)-6-methylenehexahydro-3H-pyrrolizin-3-one (712 mg, 4.3 mmol) was dissolved in anhydrous tetrahydrofuran (5mL). The mixture was placed in an ice water bath, and lithium aluminium hydride solution (4.3 mL, 4.3 mmol, 1 M in tetrahydrofuran) was added dropwise to the above solution, and stirred at 60°C for 3 hours. Upon completion of the reaction (monitored by LCMS), sodium sulfate decahydrate (430 mg) was added to quench the reaction, and the mixture was stirred at room temperature for 0.5 hours and filtered. The filtrate was concentrated under reduced pressure to obtain a crude product, (R)-(2-methylenetetrahydro-1H-pyrrolizin-7a(5H)-yl)methanol (650 mg, yield: 99%). ES-API: [M+H]⁺= 154.1.

Step 4: (R)-(2-methylenetetrahydro-1H-pyrrolizin-7a(5H)-yl)methanol (27 mg, 0.18 mmol) was dissolved in anhydrous tetrahydrofuran (2 mL). Under an ice-water bath, sodium hydride (14 mg, 0.36 mmol) was added, and the reaction was proceeded for 30 minutes in the ice-water bath. Then, a tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (70 mg, 0.09 mmol) was added dropwise to the reaction mixture, and the reaction was proceeded at room temperature for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous sodium sulfate, filtered, and the filtrate was concentrated under reduced pressure. This yielded a crude product of tert-butyl (SS,SaS,6S,9R)-1-fluoro-2-(7-fluoro-8-((triisopropylsilyl)ethynyl)naphthalen-1-yl)-S-methyl-12-(((R)-2-methylenetetiahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (77 mg, yield: 100%). ES-API: [M+H]⁺= 877.3.

Step 5: Cesium fluoride (135 mg, 0.9 mmol) was added to a solution of tert-butyl (SS,SaS,6S,9R)-1-fluoro-2-(7-fluoro-8-((triisopropylsilyl)ethynyl)naphthalen-1-yl)-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (77 mg, 0.09 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL), washed with water (15 mL × 2) and saturated brine (15 mL × 3), dried over anhydrous sodium sulfate, filtered, and concentrated to obtain a crude product of tert-butyl (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, yield: 100%). ES-API: [M+H]⁺= 721.3.

Step 6: Under an ice-water bath, a 4 M hydrochloric acid-dioxane solution (5 mL) was slowly added to a solution of tert-butyl (SS,SaS,6S,9R)-2-(8-ethynyl-7-fluoronaphthalen-1-yl)-1-fluoro-S-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, 0.09 mmol) in acetonitrile (1 mL), and the reaction mixture was stirred at 0°C for 0.5 hours. Upon completion of the reaction (monitored by LCMS), the reaction mixture was concentrated to dryness. The residue was purified by preparative HPLC (ammonium bicarbonate method 2) (chromatographic condition: 15ml-35-85-13min) to obtain a pale yellow solid (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,1 0-hexahydro-5H-4-oxa-3,10a,11,13,14-pentaaza-6,9-methanonaphtho[1,8-ab]heptalene (Z636, 10 mg, yield: 18 %). ES-API: [M+H]⁺= 621.3.

### Example 58 Synthesis of (5S,5aS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z637)

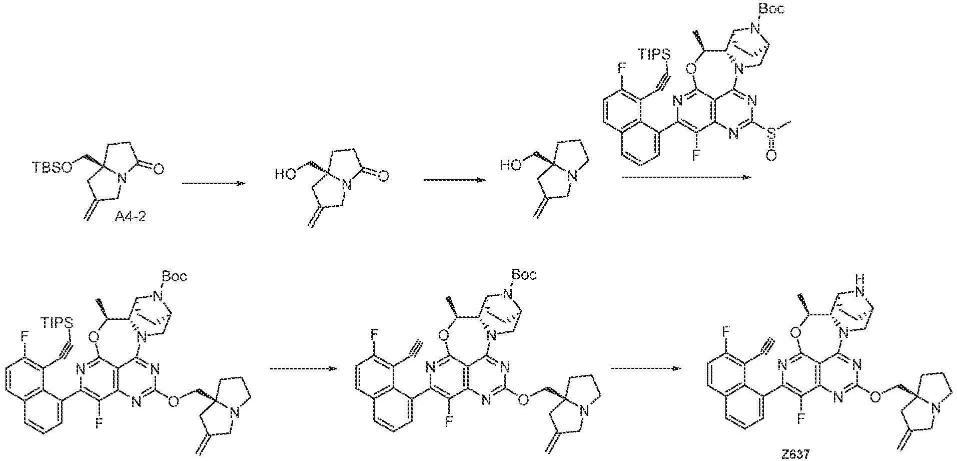

Step 1: At room temperature, a 4M hydrochloric acid/dioxane solution (10 mL) was slowly added to a acetonitrile (2 mL) solution of (S)-7a-(((tert-butyldimethylsilyl)oxy)methyl)-6-methylenehexahydro-3H-pyrrolizin-3-one (A4-2, retention time 9.205mm, 1.3 g, 4.6 mmol), and the mixture was stirred at room temperature for 0.5 hours. Upon completion of the reaction (monitored by LCMS), the reaction mixture was concentrated under reduced pressure to obtain a crude product, (S)-7a-(hydroxymethyl)-6-methylenehexahydro-3H-pyrrolizin-3-one (772 mg, yield: 100%). ES-API: [M+H]⁺=168.1.

Step 2: Under nitrogen protection, (S)-7a-(hydroxymethyl)-6-methylenehexahydro-3H-pyrrolizin-3-one (772 mg, 4.6 mmol) was dissolved in anhydrous tetrahydrofuran (5 mL). The mixture was placed in an ice water bath, and lithium aluminium hydride solution (4.6 mL, 4.6 mmol, 1 M in tetrahydrofuran) was slowly added dropwise to the solution, and the mixture was stirred at 60°C for 3 hours. Upon completion of the reaction (monitored by LCMS), sodium sulfate decahydrate (460 mg) was added to quench the reaction, and the mixture was stirred at room temperature for 0.5 hours, and then filtered. The filtrate was concentrated under reduced pressure to obtain a crude product, (S)-(2-methylenetetrahydro-1H-pyrrolizin-7a(5H)-yl)methanol (700 mg, yield: 99%). ES-API: [M+H]⁺= 154.1.

Step 3: (S)-(2-methylenetetrahydro-1H-pyrrolizin-7a(5H)-yl)methanol (27 mg, 0.18 mmol) was dissolved in anhydrous tetrahydrofuran (2 mL), and under an ice-water bath, sodium hydride (14 mg, 0.36 mmol) was added, and the reaction was proceeded for 30 minutes in the ice-water bath. Then, a tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (70 mg, 0.09 mmol) was added dropwise to the reaction mixture, which reacted at room temperature for 30 minutes. Upon completion of the reaction (monitored by LCMS), the reaction mixture was diluted with ethyl acetate (20 mL), washed with saturated ammonium chloride solution (15 mL × 2), and saturated brine (15 mL × 3), dried over anhydrous sodium sulfate, filtered, and the filtrate concentrated under reduced pressure. This yielded a crude product of tert-butyl (SS,SaS,6S,9R)-1-fluoro-2-(7-fluoro-8-((triisopropylsilyl)ethynyl)naphthalen-1-yl)-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (77 mg, yield: 100%). ES-API: [M+H]⁺= 877.3.

Step 4: Cesium fluoride (135 mg, 0.9 mmol) was added to a solution of tert-butyl (SS,SaS,6S,9R)-1-fluoro-2-(7-fluoro-8-((triisopropylsilyl)ethynyl)naphthalen-1-yl)-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (77 mg, 0.09 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL), washed with water (15 mL × 2) and saturated brine (15 mL × 3), dried over anhydrous sodium sulfate, filtered, and concentrated to obtain a crude product of tert-butyl (5S,5aS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, yield: 100%). ES-API: [M+H]⁺= 721.3.

Step 5: Under an ice-water bath, a 4 M hydrochloric acid-dioxane solution (5 mL) was slowly added to a solution of tert-butyl (SS,SaS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, 0.09 mmol) in acetonitrile (1 mL). The reaction mixture was stirred at 0°C for 0.5 hours. Upon completion of the reaction (monitored by LCMS), the reaction mixture was concentrated to dryness. The residue was purified by preparative HPLC (ammonium bicarbonate method 2) (chromatographic condition: 15ml-35-85-13min) to obtain a pale yellow solid, (SS,SaS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)nethoxy)-5a,6,7,8,9,10-hexahydro-5H-4-oxa-3,10a,11,13,14-pentaaza-6,9-methanonaphtho[1,8-ab]heptalene (Z637, 12 mg, yield: 22 %). ES-API: [M+H]⁺= 621.3.

### Example 59 Synthesis of (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z638)

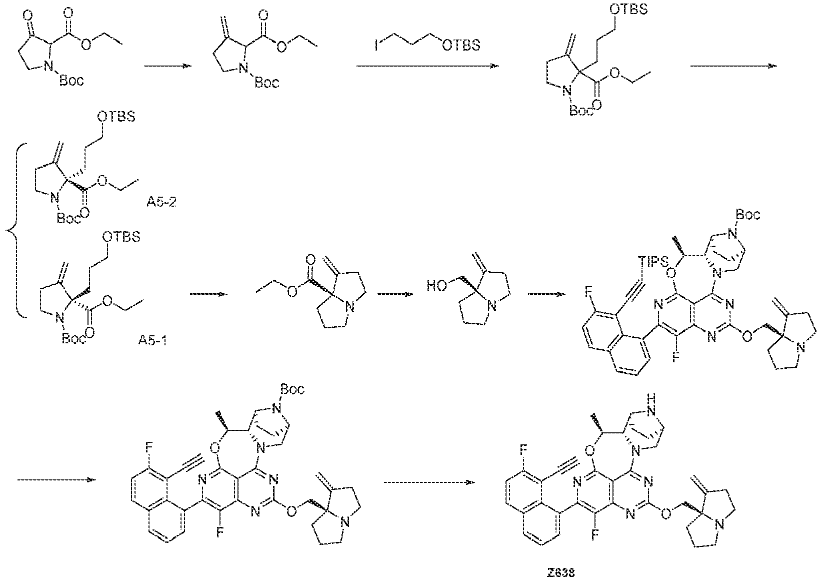

Step 1: Methyltriphenylphosphonium bromide (10.41g, 29.15 mmol) was added to tetrahydrofuran (200 mL) and cooled to 0°C. Under a nitrogen atmosphere, at 0°C, a tetrahydrofuran solution of potassium tert-butoxide (29.15mL, 29.15 mmol, 1M) was added to the mixture. After the addition was complete, the reaction mixture was stirred at room temperature for 1 hour. 1-(tert-butyl) 2-ethyl 3-oxopyrrolidine-1,2-dicarboxylate (3.0g, 12.82 mmol) was dissolved in tetrahydrofuran (10 mL) and slowly added to the above reaction mixture under a nitrogen atmosphere. After the dropwise addition was complete, the mixture was stirred at room temperature for 3 hours. Saturated ammonium chloride (30 mL) was added to the reaction mixture, and the mixture was extracted with ethyl acetate (100 mL × 3). The organic phase was washed with saturated brine (200mL), dried over anhydrous sodium sulfate, filtered, and concentrated. The crude product was purified by flash silica gel column chromatography (ethyl acetate/petroleum ether: 0%-8%) to obtain 1-(tert-butyl) 2-ethyl 3-methylenepyrrolidine-1,2-dicarboxylate (2.0g, yield 61.6%), a colorless and transparent liquid. ES-API: [M-Boc+H]⁺=200.1.

Step 2: At -78°C, bis(trimethylsilyl)amine lithium (4.0mL, 4.0mmol) was added dropwise to a dry tetrahydrofuran (20.0mL) solution of 1-(tert-butyl) 2-ethyl 3-methylenepyrrolidine-1,2-dicarboxylate (0.50g, 1.96 mmol), and the mixture was stirred at this temperature for 1 hour. hour later, tert-butyl(3-iodopropoxy)dimethylsilane (3.0 g, 9.99 mmol) was slowly added to the solution, which was then slowly warmed to room temperature and reacted for 2 hours. Upon completion of the reaction, saturated ammonium chloride solution (100 mL) was added to the reaction mixture, and the mixture was extracted with ethyl acetate (100 mL × 4). The mixture was washed with saturated brine (200 mL × 1), dried over anhydrous sodium sulfate, filtered, and concentrated. The crude product was purified by flash silica gel column chromatography (ethyl acetate/petroleum ether: 0-10%) to obtain 1-(tert-butyl) 2-ethyl 2-(3-((tert-butyldimethylsilyl)oxy)propyl)-3-methylenepyrrolidine-1,2-dicarboxylate (720 mg, yield 86%), a colorless and transparent oily liquid. ES-API: [M-Boc+H]⁺=328.3.

Step 3: 1-(tert-butyl) 2-ethyl 2-(3-((tert-butyldimethylsilyl)oxy)propyl)-3-methylenepyrrolidine-1,2-dicarboxylate (4.4g, 10.30 mmol) was separated under the following condition: (Column: Daicel CHIRALPAK^{®} IB 250*30 mm, 10µm; Mobile phase A: n-hexane; Mobile phase B: ethanol; Detection wavelength: 254nm/214nm; Flow rate: 25mL/min; Isocratic elution program: Mobile phase A: Mobile phase B=99.5:0.5 (V/V); Column temperature: room temperature) to obtain two isomers. One structure was arbitrarily designated as 1-(tert-butyl) 2-ethyl (R)-2-(3-((tertbutyldimethylsilyl)oxy)propyl)-3-methylenepyrrolidine-1,2-dicarboxylate (A5-1, 2.01g, peak 1, retention time 7.0 min, yield: 40%), a pale yellow oily liquid. ES-API: [M-Boc+H]⁺=350.2. The other structure was arbitrarily designated as 1-(tert-butyl) 2-ethyl (S)-2-(3-((tertbutyldimethylsilyl)oxy)propyl)-3-methylenepyrrolidine-1,2-dicarboxylate (A5-2, 1.91g, peak 2, retention time 9.2 min, yield: 38%), a pale yellow oily liquid. ES-API: [M-Boc+H]⁺=328.3.

Step 4: Under an ice water bath, 1-(tert-butyl) 2-ethyl (R)-2-(3-((tertbutyldimethylsilyl)oxy)propyl)-3-methylenepyrrolidine-1,2-dicarboxylate (A5-1, retention time 7.0 min, 2.01g, 4.70mmol) was dissolved in dichloromethane (20 mL), and sulfoxide chloride (15.0mL, 207.0mmol) was added. The mixture was stirred overnight at room temperature. The reaction mixture was evaporated under reduced pressure to remove the solvent, and mixed solvent (dichloromethane: methanol = 10: 1, 30 mL) was added to the crude product, followed by potassium carbonate (1.30g, 9.40mmol). The mixture was stirred at room temperature for 1 hour. After filtration, the filtrate was evaporated under reduced pressure to obtain ethyl (R)-1-methylenetetrahydro-1H-pyrrolizine-7a(5H)-carboxylate (650mg, yield 70.83%), a colorless transparent liquid. ES-API: [M+H]⁺=196.1.

Step 5: Ethyl (R)-1-methylenetetrahydro-1H-pyrrolizine-7a(SH)-carboxylate (100mg, 0.512mmol) was dissolved in tetrahydrofuran (10 mL) and cooled to 0°C. Under a nitrogen atmosphere, a solution of lithium aluminium hydride (2.0 mL, 2.0mmol, 1M in tetrahydrofuran) was added. The reaction mixture was stirred at 0°C for 1 hour. Water (0.03 mL) was added to quench the reaction, followed by 15% sodium hydroxide solution (0.03 mL) and water (0.09 mL). Tetrahydrofuran (10 mL) was added, and the mixture was filtered. The filtrate was concentrated to obtain (R)-(1-methylenetetrahydro-1H-pyrrolizin-7a(5H)-yl)methanol (70mg, yield 89%), a colorless transparent liquid. ES-API: [M+H]⁺=154.1.

Step 6: (R)-(1-methylenetetrahydro-1H-pyrrolizin-7a(5H)-yl)methanol (70mg, 0.457 mmol) was dissolved in anhydrous tetrahydrofuran (5 mL). Under an ice water bath, sodium hydride (73.0mg, 1.83 mmol) was added, and the reaction was proceeded for 30 minutes under the ice water bath. A tetrahydrofuran (2 mL) solution of tert-butyl(SS,SaS,6S,9R)-1-fluoro-2-(7-fluoro-8-((triisopropylsilyl)ethynyl)naphthalen-1-yl)-S-methyl-12-(methylsulfinyl)-Sa,6,7,8,9,10-hexahydro-5H-4-oxygen-3,10a,11,13,14-pentazacyclohexadeca[1,8-ab]naphthalene-14-carboxylate (100mg, 0.127 mmol) was added dropwise to the reaction mixture, which was then stirred at 0°C for 30 minutes. Upon completion of the reaction (monitored by LCMS), the reaction mixture was diluted with ethyl acetate (50 mL), washed with saturated ammonium chloride solution (15 mL × 2), and saturated brine (50 mL × 3), dried over anhydrous sodium sulfate, and filtered. The crude product was purified by automated flash chromatography (dichloromethane: methanol = 15:1) through silica gel to obtain tert-butyl (SS,SaS,6S,9R)-1-fluoro-2-(7-fluoro-8-((triisopropylsilyl)ethynyl)naphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (150mg, crude product). ES-API: [M+H]⁺= 877.4.

Step 7: Cesium fluoride (1500mg, 9.86mmol) was added to a solution of tert-butyl (5S,5aS,6S,9R)-1-fluoro-2-(7-fluoro-8-((triisopropylsilyl)efihynyl)naphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (150mg, crude product) in N,N-dimethylformamide (5.0mL). The reaction mixture was stirred at room temperature for 2 hours. Upon completion of the reaction (monitored by LCMS), the reaction mixture was diluted with ethyl acetate (60mL), washed with water (30 mL × 2), and saturated brine (50 mL × 3), dried over anhydrous sodium sulfate, filtered, and concentrated to obtain a crude product of tert-butyl (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170mg, crude product). ES-API: [M+H]⁺= 721.3.

Step 8: Under an ice water bath, 4 M hydrochloric acid-dioxane solution (3 mL) was slowly added to a solution of tert-butyl (SS,SaS,6S,9R)-2-(8-ethynyl-7-fluoronaphthalen-1-yl)-1-fluoro-S-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (170mg, crude product) in acetonitrile (5.0mL). The reaction mixture was stirred at 0°C for 0.5 hours. Upon completion of the reaction (monitored by LCMS), the reaction mixture was evaporated to dryness, and the residue was purified by preparative HPLC (ammonium bicarbonate method 2) to obtain a pale yellow solid of (SS,SaS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z638, 10.0mg, total yield for 3 steps: 12.6 %). ES-API: [M+H]⁺= 621.3. ¹HNMR(400MHz, DMSO-*d₆*): 8.20-8.17(m, 2H), 7.66-7.55 (m, 3H), 5.49-5.07 (m, 2H), 4.60-4.20(m, 3H), 4.12-3.66(m, 4H), 3.35-3.10 (m, 5H), 2.78-2.50(m, 2H), 2.01-1.65(m, 9H), 1.58-1.45 (m, 3H).

### Example 60 Synthesis of (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z639)

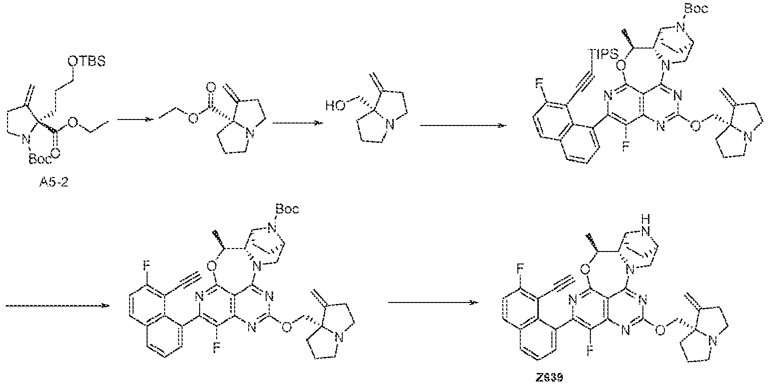

Step 1: In an ice water bath, 1-(tert-butyl) 2-ethyl (S)-2-(3-((tert-butyldimethylsilyl)oxy)propyl)-3-methylenepyrrolidine-1,2-dicarboxylate (A5-2, retention time 9.2 min, 1.91g, 4.46mmol) was dissolved in dichloromethane (20 mL), and thionyl chloride (15.0mL, 207.0mmol) was added. The mixture was stirred overnight at room temperature. The reaction mixture was then evaporated under reduced pressure to remove the solvent, and a mixed solvent (dichloromethane:methanol = 10:1, 30mL) was added to the crude product, followed by the addition of potassium carbonate (1.30g, 9.40mmol). The reaction mixture was stirred for 1 hour at room temperature. After filtration, the filtrate was concentrated under reduced pressure to obtain ethyl (S)-1-methylenetetrahydro-1H-pyrrolizine-7a(5H)-carboxylate (850mg, yield 97.0%), a colorless and transparent liquid. ES-API: [M+H]⁺=196.1.

Step 2: Ethyl (S)-1-methylenetetrahydro-1H-pyrrolizine-7a(5H)-carboxylate (150mg, 0.768mmol) was dissolved in tetrahydrofuran (10 mL) and cooled to 0°C. Under a nitrogen atmosphere, lithium aluminum hydride solution (2.0 mL, 2.0mmol, 1M in tetrahydrofuran) was added to the solution. The reaction mixture was stirred for 1 hour at 0°C. Water (0.03 mL) was added to quench the reaction, followed by the addition of 15% sodium hydroxide solution (0.03 mL) and water (0.09 mL). Tetrahydrofuran (10 mL) was added, and the mixture was filtered. The filtrate was concentrated to obtain (S)-(1-methylenetetrahydro-1H-pyrrolizin-7a(5H)-yl)methanol (88mg, yield 74.5%), a colorless and transparent liquid. ES-API: [M +H]⁺=154.1.

Step 3: (S)-(1-methylenetetrahydro-1H-pyrrolizin-7a(5H)-yl)methanol (80mg, 0.522 mmol) was dissolved in anhydrous tetrahydrofuran (5 mL). Under an ice water bath, sodium hydride (83.0mg, 2.088 mmol) was added, and the reaction was proceeded for 30 minutes under the ice water bath. A tetrahydrofuran (2 mL) solution of tert-butyl(SS,SaS,6S,9R)-1-fluoro-2-(7-fluoro-8-((triisopropylsilyl)ethynyl)naphtbalen-1-yl)-5-methyl-12-(methylsulfonyl)-5a,6,7,8,9,10-hexahydro-5H-4-oxa-3,10a,11,13,14-pentaza-6,9-methylenenaphtho[1,8-ab]heptene-14-carboxylate (100mg, 0.127 mmol) was then added dropwise to the reaction mixture and reacted for 30 minutes at 0°C. Upon completion of the reaction (monitored by LCMS), the reaction mixture was diluted with ethyl acetate (50 mL), washed with saturated ammonium chloride solution (15 mLX 2) and saturated brine (50 mLX3), dried over anhydrous sodium sulfate, filtered, and the crude product was purified by flash silica gel column chromatography (dichloromethane:methanol = 15:1) to obtain tert-butyl (5S,5aS,6S,9R)-1-fluoro-2-(7-fluoro-8-((triisopropylsilyl)ethynyl)naphthalen-1-yl)-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-b,9-methanonaphtho[1,8-ab]heptalene-14-carboxylate (150mg, crude product). ES-API: [M+H]⁺= 877.4.

Step 4: Cesium fluoride (1500mg, 9.86mmol) was added to a solution of tert-butyl (SS,SaS,6S,9R)-1-fluoro-2-(7-fluoro-8-((triisopropylsilyl)ethynyl)naphthalen-1-yl)-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (150mg, crude product) in N,N-dimethylformamide (5.0mL). The reaction mixture was stirred at room temperature for 2 hours. Upon completion of the reaction (monitored by LCMS), the reaction mixture was diluted with ethyl acetate (60mL), washed with water (30 mLX 2) and saturated brine (50 mLX 3), dried over anhydrous sodium sulfate, filtered, and concentrated to obtain the crude product tert-butyl (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (175mg, crude product). ES-API: [M+H]⁺= 721.3.

Step 5: Under an ice water bath, a 4 M solution of hydrochloric acid-dioxane (3 mL) was slowly added to a solution of tert-butyl (5S,5aS,6S,9R)-2-(8-ethynyl-7-fluoronaphthalen-1-yl)-1-fluoro-5-methyl-12-(((S)-1-methylenetetrahydro-1H-pynolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (175mg, crude product) in acetonitrile (5.0mL). The reaction mixture was stirred at 0°C for 0.5 hours. Upon completion of the reaction (monitored by LCMS), the reaction mixture was concentrated to dryness, and the residue was purified by preparative HPLC (formic acid method 2) (chromatographic condition: 15ml-35-85-13min) to obtain a pale yellow solid (SS,SaS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z639, 11.0mg, total yield for 3 steps: 13.9 %, formate). ES-API: [M+H]⁺= 621.3.

### Example 61 Synthesis of (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5)cycloocta[1,2,3-de]naphthalene (Z640)

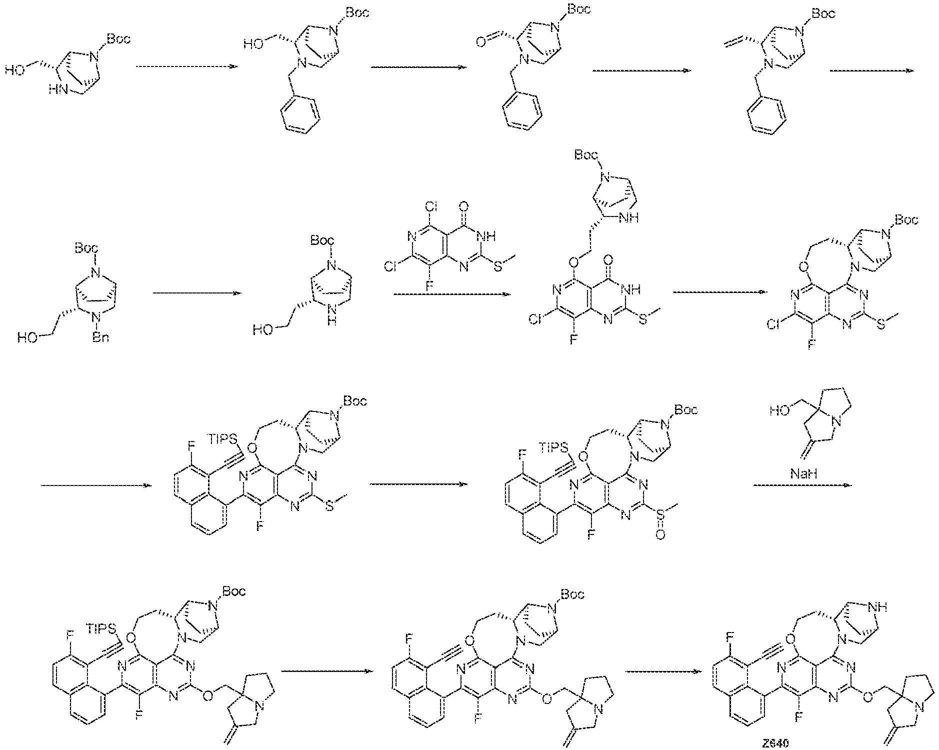

Step 1: Tert-butyl (1S,2S,5R)-2-(hydroxymethyl)-3,8-diazabicyclo[3.2.1]octane-8-carboxylate (2.00 g, 8.25 mmol) was dissolved in acetonitrile (50 mL), followed by successive addition of benzyl bromide (1.18 mL, 9.90 mmol) and anhydrous potassium carbonate (1.71 g, 12.38 mmol). The mixture was stirred at room temperature for 1 hour, then the solvent was evaporated. Water was added to the residue, and the mixture was extracted three times with dichloromethane. The organic phase was collected, washed with saturated brine, dried over anhydrous sodium sulfate, filtered, and evaporated. The crude product was purified by flash silica gel column chromatography (tetrahydrofuran/petroleum ether: 0-30%) to obtain tert-butyl (1S,2S,SR)-3-benzyl-2-(hydroxymethyl)-3,8-diazabicyclo[3.2.1]octane-8-carboxylate (2.74 g, yield: 100%). ES-API: [M+H]⁺= 333.3.

Step 2: Tert-butyl (1S,2S,5R)-3-benzyl-2-(hydroxymethyl)-3,8-diazabicyclo[3.2.1]octane-8-carboxylate (2.74 g, 8.12 mmol) in dichloromethane (80 mL) was cooled to 0°C, and Dess-Martin periodinane (4.46 g, 10.56 mmol) was added in batches. The mixture was stirred at 0°C for 1 hour, then saturated sodium bicarbonate solution and saturated sodium bisulfite solution were added, and the mixture was stirred at room temperature for 15 minutes. The organic phase was separated, and the aqueous phase was extracted twice with dichloromethane. The organic phase was collected, washed with saturated brine, dried over anhydrous sodium sulfate, filtered, and evaporated to obtain crude tert-butyl (1S,2S,5R)-3-benzyl-2-formyl-3,8-diazabicyclo[3.2.1]octane-8-carboxylate (2.6 g), which was then used directly in the next step without further purification. ES-API: [M+H]⁺= 331.2.

Step 3: Under nitrogen protection and at 0°C, a 1 M solution of potassium tert-butoxide in tetrahydrofuran (19.7 mL, 19.7 mmol) was slowly added to a suspension of methyltriphenylphosphonium bromide (5.6 g, 15.74 mmol) in tetrahydrofuran (60 mL). After 15 minutes, a solution of the crude tert-butyl (1S,2S,SR)-3-benzyl-2-formyl-3,8-diazabicyclo[3.2.1]octane-8-carboxylate (2.6 g, 7.87 mmol) in tetrahydrofuran (20 mL) was added dropwise. After completion of the addition, the reaction mixture was warm to room temperature and stirred overnight. Saturated ammonium chloride solution was then slowly added, and the mixture was extracted with ethyl acetate three times. The organic phase was collected, washed with saturated brine, dried over anhydrous sodium sulfate, filtered, and evaporated. The crude product was purified by flash silica gel column chromatography (ethyl acetate/petroleum ether: 0-8%) to obtain tert-butyl (1S,2R,5R)-3-benzyl-2-vinyl-3,8-diazabicyclo[3.2.1]octane-8-carboxylate (2.10 g, total yield for 2 steps 79%). ES-API: [M+H]⁺= 329.3.

Step 4: Under nitrogen protection and at 0°C, a 0.5 M solution of 9-borabicyclo[3.3.1]nonane in tetrahydrofuran (42.00 mL, 21.00 mmol) was slowly added dropwise to a solution of tert-butyl (lS,2R,5R)-3-benzyl-2-vinyl-3,8-diazabicyclo[3.2.1]octane-8-carboxylate (2.3 g, 7.00 mmol) in tetrahydrofuran (20.0 mL). The reaction mixture was warm to room temperature and stirred overnight. The reaction mixture was then cooled to 0°C, and a 3 M solution of sodium hydroxide (15.0 mL) and 30% hydrogen peroxide (15.0 mL) were slowly added, followed by stirred at room temperature for 3 hours. After diluted with water, the reaction mixture was extracted twice with ethyl acetate. The organic phase was collected, washed with saturated brine, dried over anhydrous sodium sulfate, filtered, and evaporated. The crude product was purified by flash silica gel column chromatography (tetrahydrofuran/petroleum ether: 0-30%) to obtain tert-butyl (1R,2R,5S)-3-benzyl-2-(2-hydroxyethyl)-3,8-diazabicyclo[3.2.1]octane-8-carboxylate (1.85 g, yield: 76%). ES-API: [M+H]⁺= 347.3.

Step 5: In a two-neck flask, tert-butyl (1R,2R,SS)-3-benzyl-2-(2-hydroxyethyl)-3,8-diazabicyclo[3.2.1]octane-8-carboxylate (1.85 g, 5.34 mmol) from previous step was dissolved in anhydrous methanol (20.0 mL) and 10% palladium on carbon (500 mg) was added. The mixture was purged with hydrogen three times, and under hydrogen protection, 7.0 M ammonia in methanol (4.0 mL) was added. After stirred overnight at room temperature, the mixture was filtered and the filtrate was evaporated to obtain tert-butyl (1R,2R,5S)-2-(2-hydroxyethyl)-3,8-diazabicyclo[3.2.1]octane-8-carboxylate (1.36 g, crude product), which was then used directly in the next step without further purification. ES-API: [M+H]⁺= 257.2.

Step 6: Under nitrogen protection and at 0°C, sodium hydride (60% dispersion in oil) (94 mg, 2.34 mmol) was added in batches to a solution of crude tert-butyl (1R,2R,5S)-2-(2-hydroxyethyl)-3,8-diazabicyclo[3.2.1]octane-8-carboxylate (150 mg, 0.59 mmol) in anhydrous tetrahydrofuran (20.0 mL). After 15 minutes reaction, 5,7-dichloro-8-fluoro-2-(methylthio)pyrido[4,3-d]pyrimidin-4(3H)-one (492 mg, 1.76 mmol) was added. The reaction mixture was heated to 40°C for 6 hours, then slowly added with saturated ammonium chloride solution and extracted three times with dichloromethane. The organic phase was washed with saturated brine, dried over anhydrous sodium sulfate, filtered, and evaporated. The crude product was purified by flash silica gel column chromatography (dichloromethane/methanol: 0-15%) to obtain tert-butyl (1S,2R,5R)-2-(2-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (220 mg, yield: 75%). ES-API: [M+H]'= 500.1.

Step 7: At room temperature, 2-(7-azabenzotriazol)-N,N,N',N'-tetramethyluronium hexafluorophosphate (335 mg, 0.88 mmol) was added to a solution of tert-butyl (1S,2R,5R)-2-(2-((7-chloro-8-fluoro-2-(methylthio)-4-oxo-3,4-dihydropyrido[4,3-d]pyrimidin-5-yl)oxy)ethyl)-3,8-diazabicyclo[3.2.1]octane-8-carboxylate (220 mg, 0.44 mmol) in N,N-dimethylformamide (22.0 mL). After the mixture stirred for 5 minutes, N,N-diisopropylethylamine (0.38 mL, 2.20 mmol) was added. The mixture reacted overnight, then diluted with ethyl acetate. The organic phase was washed three times with saturated brine, dried over anhydrous sodium sulfate, filtered, and evaporated. The crude product was purified by flash silica gel column chromatography (ethyl acetate/petroleum ether: 0-30%) to obtain tert-butyl (6aR,7S,10R)-2-chloro-1-fluoro-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate (73 mg, yield: 34%). ES-API: [M+H]⁺= 482.1.

Step 8: In a microwave tube, tert-butyl (6aR,7S,10R)-2-chloro-1-fluoro-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate (73 mg, 0.15 mmol), (2-fluoro-8-(4,4,5,5 tetramethyl-1,3,2-dioxaborolane-2-yl)-1-naphthyl)ethynyl)triisopropylsilane (171 mg, 0.38 mmol), potassium phosphate (96 mg, 0.45 mmol), methanesulfonic acid [n-butylbis(1-adamantyl)phosphine](2-amino-1,1'-biphenyl)palladium(II) (22 mg, 0.03 mmol), tetrahydrofuran (2.0 mL), and water (0.4 mL) were added. The mixture was purged four times with nitrogen and reacted in an 80°C oil bath for 2 hours. After cooling to room temperature, with water added, and the reaction mixture was extracted three times with ethyl acetate. The combined organic phases were washed with saturated brine, dried over anhydrous sodium sulfate, filtered, and the filtrate was evaporated under reduced pressure. The crude product was purified by flash silica gel column chromatography (ethyl acetate/petroleum ether: 0-40%) to obtain tert-butyl (6aR,7S,10R)-1-fluoro-2-(7-fluoro-8-((triisopropylsilyl)ethynyl)naphthalen-1 -yl)-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate (61 mg, yield: 52%). ES-API: [M+H]⁺= 772.3.

Step 9: At 0°C, m-chloroperbenzoic acid (24 mg, 0.12 mmol) was added to a solution of tert-butyl (6aR,7S,10R)-1-fluoro-2-(7-fluoro-8-((triisopropylsilyl)ethynyl)naphthalen-1-yl)-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate (61 mg, 0.08 mmol) in dichloromethane (5.0 mL). After the mixture stirred for 0.5 hours, saturated sodium bicarbonate solution and saturated sodium bisulfite solution were added, and the mixture was vigorously stirred for 15 minutes. The organic phase was separated, and the aqueous phase was extracted twice with dichloromethane. The collected organic phase was washed with saturated brine, dried over anhydrous sodium sulfate, filtered, and evaporated to obtain crude tert-butyl (6aR,7S,10R)-1-fluoro-2-(7-fluoro-8-((triisopropylsilyl)ethynyl)naphthalen-1-yl)-13-(methylsulfinyl)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate, which was then used directly in the next step without further purification. ES-API: [M+H]⁺= 788.3.

Step 10: At 0°C, sodium hydride (60% dispersed in oil) (19 mg, 0.47 mmol) was added to a solution of (2-methylene tetrahydro-1H-pyrrozin-7a(5H)-yl)methanol (61 mg, 0.40 mmol) in tetrahydrofuran (5.0 mL). After 15 minutes, a tetrahydrofuran solution (2.0 mL) of tert-butyl (6aR,7S,10R)-1-fluoro-2-(7-fluoro-8-((triisopropylsilyl)ethynyl)naphthalen-1-yl)-13-(methylsulfinyl)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylatetert-butyl (6aR,7S,10R)-1-fluoro-2-(7-fluoro-8-((triisopropylsilyl)ethynyl)naphthalen-1-yl)-13-(methylsulfinyl)-5,6,6a,7,8,9,10,1-octahydro-4-oxa-3,11a,12,14,15-pentaaza-7,10-methanocyclohepta[4,5]cycloocta[1,2,3-de]naphthalene-15-carboxylate crude product was added dropwise. After the mixture reacting for 0.5 hours at 0°C, saturated ammonium chloride solution was slowly added. The reaction mixture was extracted three times with dichloromethane, and the organic phase was collected, washed with saturated brine, dried over anhydrous sodium sulfate, filtered, and evaporated. The crude product was purified by flash silica gel column chromatography (dichloromethane/methanol: 0-10%) to obtain tert-butyl (6aR,7S,10R)-1-fluoro-2-(7-fluoro-8-((triisopropylsilyl)ethynyl)naphthalen-1-yl)-13-((2-methylenetetrahydro-1H-pyrrolizin-7a(5H)-yl)methoxy)-S,6,6a,7,8,9,10,11-octahydro-4-oxa-3,11a,12,14,15-pentaaza-7,10-methanocyclohepta[4,5]cycloocta[1,2,3-de]naphthalene-15-carboxylate (16 mg, yield: 23%). ES-API: [M+H]⁺= 877.3.

Step 11: Tert-butyl (6aR,7S,10R)-1-fluoro-2-(7-fluoro-8-((triisopropylsilyl)ethynyl)naphthalen-1-yl)-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate (16 mg, 0.018 mmol) was dissolved in N,N-dimethylformamide (1.0 mL) and cesium fluoride (28 mg, 0.18 mmol) was added. The reaction was proceeded at room temperature for 1 hour. Upon completion of the reaction, water was added, and the reaction mixture was extracted three times with ethyl acetate. The combined organic phases were washed with saturated brine, dried over anhydrous sodium sulfate, filtered, and the filtrate was evaporated under reduced pressure to obtain crude tert-butyl (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate, which was then used directly in the next step without further purification. ES-API:[M+H]⁺=721.2.

Step 12: Tert-butyl (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalene-15-carboxylate crude product was dissolved in acetonitrile (2.0 mL). Under an ice water bath, a 4M solution of hydrochloric acid in dioxane (2.0 mL, 8.0 mmol) was added, and the reaction was proceeded at room temperature for 1 hour. Upon completion of the reaction, the reaction mixture was concentrated to dryness, and the crude product was purified by preparative HPLC (bicarbonate method 1) to obtain the target compound (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalene (Z640, 1.60 mg, yield: 14%). ES-API: [M+H]⁺=621.3.

### Example 62 Synthesis of (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hezahydro-5H-4-oza-3,10a,11,13,14-pentaaza-6,9-methanonaphtho[1,8-ab]heptalene (Z609)

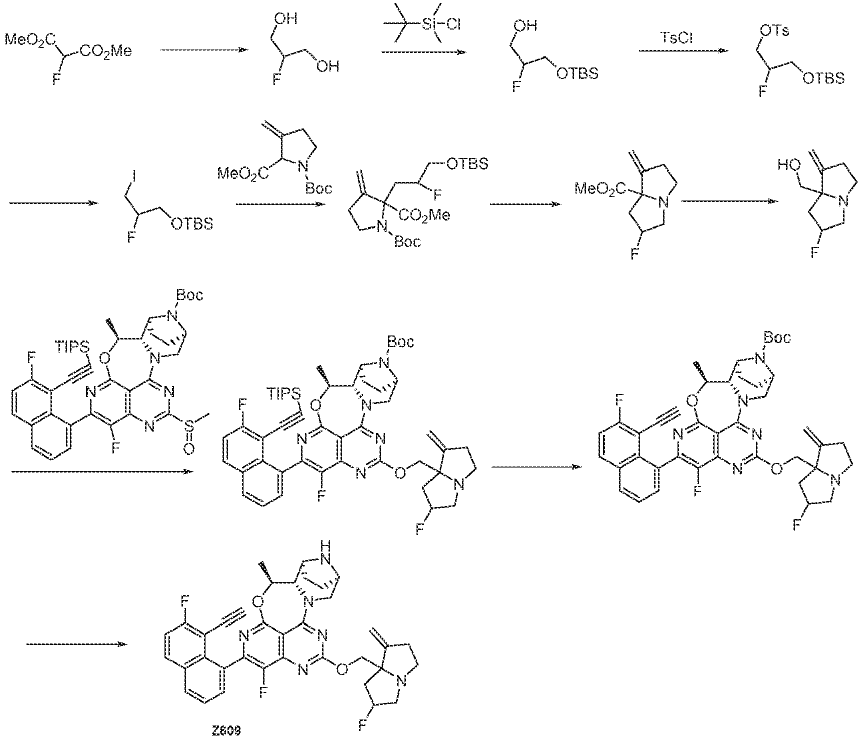

Step 1: At room temperature, sodium borohydride (10.08 g, 266.472 mmol) and lithium chloride (2.82 g, 66.618 mmol) were added to a mixture of anhydrous ethanol (80 mL) and tetrahydrofuran (100 mL). A solution of dimethyl 2-fluoromalonate (10 g, 66.62 mmol) in tetrahydrofuran (100 mL) was then added dropwise. After the addition, the reaction mixture was stirred at room temperature for 16 hours. The reaction mixture was then cooled to 0°C and quenched with concentrated hydrochloric acid (10 mL). Ethyl acetate (100 mL) was added, and the mixture was filtered through diatomaceous earth. The filtrate was concentrated, and the residue was suspended in dichloromethane/methanol (10:1, 150 mL) and stirred at room temperature for 30 minutes. After filtration and concentration of the filtrate, the crude product was purified by flash silica gel column chromatography (0-10% methanol/dichloromethane) to obtain 2-fluoropropane-1,3-diol (2 g, yield 32%). ¹H NMR(400M, CDCl₃): 4.65(m, 1 H), 3.84-3.62 (m, 4H).

Step 2: At 0°C, tert-butyldimethylchlorosilane (3.2 g, 21.26 mmol) was added to a solution of 2-fluoropropane-1,3-diol (2 g, 21.25 mmol) and imidazole (2.17 g, 31.88 mmol) in dichloromethane (50 mL). The reaction mixture was stirred at room temperature for 1 hour. The reaction mixture was then quenched with water (100 mL) and extracted with dichloromethane (50 mL × 2). The organic phase was washed with saturated brine (50 mL), dried over anhydrous sodium sulfate, filtered, and concentrated. The residue was purified by flash silica gel column chromatography (0-15% ethyl acetate/petroleum ether) to obtain 3-((tert-butyldimethylsilyl)oxy)-2-fluoropropan-1-ol (1 g, yield 22.5%). ES-API: [M+H]⁺=209.2.

Step 3: At 0°C, 4-dimethylaminopyridine (322 mg, 2.64 mmol) and triethylamine (0.55 mL, 3.96 mmol) were successively added to a solution of 3-((tert-butyldimethylsilyl)oxy)-2-fluoropropan-1-ol (500 mg, 2.64 mmol) in dichloromethane (10 mL). P-toluenesulfonyl chloride (754 mg, 3.96 mmol) was then added, and the mixture was stirred at room temperature for 2 hours. The reaction was quenched with 1M hydrochloric acid (20 mL) and the mixture was extracted with dichloromethane (30 mL × 2). The organic layer was washed with saturated sodium bicarbonate. The organic phase was dried over anhydrous sodium sulfate, filtered, and concentrated to obtain 3-((tert-butyldimethylsilyl)oxy)-2-fluoropropyl 4-toluenesulfonate (900 mg, yield 86.2%). ES-API: [M+H]⁺=363.1.

Step 4: Sodium iodide (3.72 g, 24.83 mmol) was added to a solution of 3-((tertbutyldimethylsilyl)oxy)-2-fluoropropyl 4toluenesulfonate (900 mg, 2.48 mmol) in acetone (15 mL). The reaction mixture was sealed and heated at 75°C for 5 hours. The reaction mixture was then quenched with water (50 mL) and extracted with ethyl acetate (30 mL × 2). The organic phase was washed with saturated brine, dried over anhydrous sodium sulfate, filtered, and concentrated. The residue was purified through flash silica gel column chromatography (0-5% ethyl acetate/petroleum ether) to obtain tert-butyldimethyl(2-fluoro-3-iodopropoxy)silane (700 mg, yield 88.6%).

Step 5: At -78°C, bis(trimethylsilyl)aminolithium (2.6 mL, 2.6 mmol) was added to a solution of 1-(tert-butyl) 2-methyl 3-methylenepyrrolidine-1,2-dicarboxylate (350 mg, 1.45 mmol) in tetrahydrofuran (10 mL). The reaction mixture was stirred at -78°C for 1 hour. A solution of tert-butyldimethyl(2-fluoro-3-iodopropoxy)silane (692 mg, 2.17 mmol) in tetrahydrofuran (1.5 mL) was then added dropwise. The mixture was stirred for 30 minutes at -78°C, then warm slowly to room temperature and stirred for 2 hours. The reaction mixture was cooled to 0°C and quenched with saturated ammonium chloride (30 mL), and extracted with ethyl acetate (30 mL × 2). The organic phase was washed with saturated brine, dried over anhydrous sodium sulfate, filtered, and concentrated. The residue was purified by flash silica gel column chromatography (0-8% ethyl acetate/petroleum ether) to obtain 1-(tert-butyl) 2-methyl 2-(3-((tert-butyldimethylsilyl)oxy)-2-fluoropropyl)-3-methylenepyrrolidine-1,2-dicarboxylate (300 mg, yield 47.9%). ES-API: [M+Na]⁺=454.3.

Step 6: To a solution of 1-(tert-butyl) 2-methyl 2-(3-((tert-butyldimethylsilyl)oxy)-2-fluoropropyl)-3-methylenepyrrolidine-1,2-dicarboxylate (290 mg, 0.67 mmol) in dichloromethane (10 mL), thionyl chloride (2.4 mL, 33.59 mmol) was added dropwise, and the reaction mixture was stirred at 35°C for 12 hours. The reaction was then heated at 55°C for 48 hours. The reaction mixture was concentrated, and the residue was dissolved in acetonitrile. Sodium bicarbonate (282 mg, 3.36 mmol) and potassium iodide (111 mg, 0.67 mmol) were added, and the mixture was stirred at 60°C for 1 hour. After cooling to room temperature, the mixture was filtered. The filtrate was concentrated, and the residue was purified by flash silica gel column chromatography (0-5% methanol/dichloromethane) to obtain methyl 6-fluoro-1-methylenetetrahydro-1H-pyrrolizine-7a(5H)-carboxylate (130 mg, yield 97.1%). ES-API: [M+H]⁺=200.1.

Step 7: To a solution of methyl 6-fluoro-1-methylenetetrahydro-1H-pyrrolizine-7a(5H)-carboxylate (130 mg, 0.65 mmol) in tetrahydrofuran (5 mL), a solution of lithium aluminum hydride in tetrahydrofuran (1.3 mL, 1.3 mmol) was added dropwise. The mixture was stirred at 0°C for 1 hour. The reaction was quenched succesively with 3 drops of water and 15% sodium hydroxide solution (0.1 mL). The mixture was stirred at room temperature for 30 minutes and filtered through diatomaceous earth. The filtrate was concentrated to obtain crude (6-fluoro-1-methylenetetrahydro-1H-pyrrolizin-7a(5H)-yl)methanol (100 mg, yield 89.4%), a yellow oil. ES-API: [M+H]⁺=172.1.

Step 8: Sodium hydride (9 mg, 0.23 mmol) and tetrahydrofuran (3 mL) was added to a round-bottom flask. The reaction mixture was cooled to 0°C, and a solution of (6-fluoro-1-methylene tetrahydro-1H-pyrrozin-7a(5H)-yl)methanol (26 mg, 0.15 mmol) in anhydrous tetrahydrofuran (1 mL) was added. After the mixture stirred for 20 minutes at room temperature, tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (60 mg, 0.076 mmol) was added and the reaction mixture was stirred for 20 minutes at 0°C. Upon completion of the reaction (monitored by LCMS), the reaction mixture was quenched with saturated ammonium chloride solution (30 mL) and extracted with ethyl acetate (20 mL × 2). The organic phase was washed with saturated brine (30 mL), dried over anhydrous sodium sulfate, filtered, and concentrated to obtain tert-butyl (SS,SaS,6S,9R)-1-fluoro-12-((6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (65 mg, crude product). ES-API: [M+H]⁺=895.3.

Step 9: Cesium fluoride (110 mg, 0.73 mmol) was added to a solution of tert-butyl (SS,SaS,6S,9R)-1-fluoro-12-((6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[l,8-ab]heptalene-14-carboxylate (65 mg, crude product) in N,N-dimethylformamide (3 mL). The reaction mixture was stirred at room temperature for 1 hour. Upon completion of the reaction (monitored by LCMS), the reaction mixture was diluted with ethyl acetate (50 mL), washed with water (15 mL × 2) and saturated brine (15 mL × 3), dried over anhydrous sodium sulfate, filtered, and concentrated to obtain tert-butyl (SS,SaS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, crude product). ES-API: [M+H]⁺= 739.3.

Step 10: Under an ice water bath, a 4 M solution of hydrochloric acid in dioxane (1 mL) was slowly added to a solution of tert-butyl (5S,SaS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, crude product) in acetonitrile (2 mL). The reaction mixture was stirred at room temperature for 1 hour. Upon completion of the reaction (monitored by LCMS), the reaction mixture was concentrated to dryness, and the residue was purified by preparative HPLC (bicarbonate method 2) to obtain the target compound (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z609, 1 mg, diastereoisomer ratio = 65:35, retention time: 6.31 min and 6.44min, yield: 2%). ES-API: [M+H]⁺= 639.2.

### Example 63 Synthesis of (2-(8-ethynyl-7-fluoronaphthalen-1-yl)-1-fluoro-12-((1-methylenetetrahydro-1H-pyrrolizin-7a(5H)-yl)methox)-5a,6,7,8,9,10-hexahydro-5H-4-oxa-3,10a,11,13,14-pentaaza-6,9-methanonaphtho[1,8-ab]heptalen-5-yl)methanol (Z672)

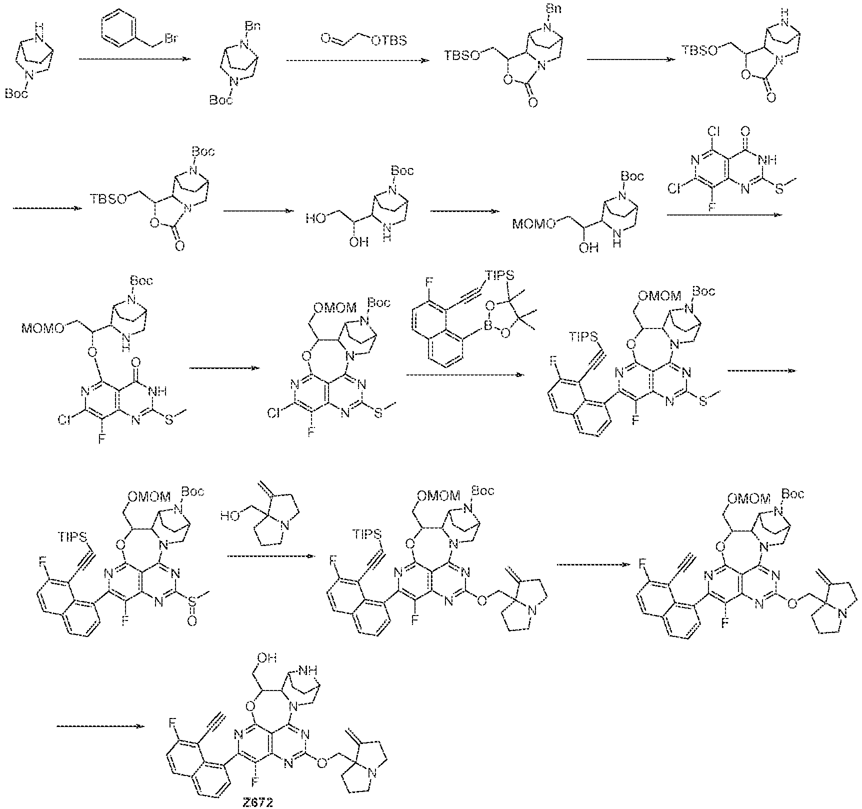

Step 1: Tert-butyl 3,8-diazabicyclo[3.2.1]octane-3-carboxylate (2 g, 9.421 mmol) was dissolved in acetonitrile (20 mL) at room temperature, followed by successive addition of potassium carbonate (1.432 g, 10.363 mmol) and benzyl bromide (1.934 g, 11.305 mmol). The reaction mixture was heated to 60°C and stirred for 5 hours. Upon completion of the reaction (monitored by LCMS), water (30 mL) was added to quench the reaction, then the mixture was extracted with ethyl acetate (30 mL × 3). The combined organic phase was washed with saturated brine (20 mL × 2), dried over anhydrous sodium sulfate, filtered, and concentrated. The crude product was purified by automated flash chromatography through silica gel (ethyl acetate:petroleum ether = 0-40%) to obtain tert-butyl 8-benzyl-3,8-diazabicyclo[3.2.1]octane-3-carboxylate (2.748 g, yield: 96%). ES-API: [M+ H]⁺= 303.2.

Step 2: Under nitrogen protection, tert-butyl 8-benzyl-3,8-diazabicyclo[3.2.1]octane-3-carboxylate (2.3 g, 7.605 mmol) and N,N,N',N'-tetramethylethylenediamine (4.419 g, 38.027 mmol) were dissolved in anhydrous tetrahydrofuran (30 mL). The mixture was cooled to -78°C using a dry ice-acetone bath, and sec-butyllithium (17.5 mL, 22.816 mmol, 1.3 M in hexane) was slowly added. After the mixture stirred for 1 hour at -78°C, a solution of (tert-butyldimethylsilyl)oxy)acetaldehyde (3.977 g, 22.816 mmol) in tetrahydrofuran (10 mL) was added, and the mixture was warm to room temperature and stirred overnight. Upon completion of the reaction (monitored by LCMS), saturated ammonium chloride solution (50 mL) was added to quench the reaction, and the mixture was extracted with ethyl acetate (30 mL × 3). The combined organic phase was washed with saturated brine (15 mL × 2), dried over anhydrous sodium sulfate, filtered, and concentrated. The crude product was purified by automated flash chromatography through silica gel (ethyl acetate:petroleum ether = 0-15%) to obtain (6S,9R)-10-benzyl-1-(((tert-butyldimethylsilyl)oxy)methyl)hexahydro-1H,3H-6,9-epiminooxazolo[3,4-a]azepin-3-one (806 mg, yield: 26%). ES-API: [M+ H]⁺= 403.3.

Step 3: Under hydrogen protection, (6S,9R)-10-benzyl-1-(((tertbutyldimethylsilyl)oxy)methyl)hexahydro-1H,3H-6,9-epiminooxazolo[3,4-a]azepin-3-one (806 mg, 2.002 mmol) was dissolved in dichloromethane (30 mL) and ammonia-methanol solution (6 mL, 7M in methanol). Palladium on carbon (10%, 300 mg) was added, and the mixture was stirred at room temperature overnight. Upon completion of the reaction (monitored by LCMS), the mixture was filtered through diatomaceous earth and concentrated under reduced pressure to obtain crude (6S,9R)-1-(((tert-butyldimethylsilyl)oxy)methyl)hexahydro-1H,3H-6,9-epiminooxazolo[3,4-a]azepin-3-one (625 mg). ES-API: [M+ H]⁺= 313.3.

Step 4: (6S,9R)-1-(((tert-butyldimethylsilyl)oxy)methyl)hexahydro-1H,3H-6,9-epiminooxazolo[3,4-a]azepin-3-one (625 mg, 2.000 mmol) was dissolved in dichloromethane (20 mL) and the mixture was placed in an ice-water bath. Di-t-butyl dicarbonate (0.70 mL, 3.000 mmol) and N,N-diisopropylethylamine (0.7 mL, 4.000 mmol) were added, and the reaction mixture was slowly warmed to room temperature and stirred for 1 hour. Upon completion of the reaction (monitored by LCMS), the reaction mixture was concentrated and the crude product was purified by automated flash chromatography through silica gel (ethyl acetate:petroleum ether = 0-20%) to obtain tert-butyl (6S,9R)-1-(((tert-butyldimethylsilyl)oxy)methyl)-3-oxohexahydro-1H,3H-6,9-epiminooxazolo[3,4-a]azepine-10-carboxylate (558 mg, yield: 68%). ES-API: [M+ H]⁺= 413.2.

Step 5: Sodium hydroxide (1.082 g, 27.048 mmol) was added to a solution of tert-butyl (6S,9R)-1-(((tert-butyldimethylsilyl)oxy)methyl)-3-oxohexahydro-1H,3H-6,9-epiminooxazolo[3,4-a]azepine-10-carboxylate (558 mg, 1.352 mmol) in ethanol (30 mL) and water (6 mL) at room temperature. The reaction mixture was heated to 80°C and stirred overnight. Upon completion of the reaction (monitored by LCMS), the mixture was concentrated under reduced pressure to dryness, and the crude product was dissolved in water (1.5 mL) and extracted with dichloromethane and isopropanol (15 mL × 4, V/V= 5:1). The combined organic phase was washed with saturated brine (10 mL × 2), dried over anhydrous sodium sulfate, filtered, and concentrated to obtain crude tert-butyl (1R,5S)-2-(1,2-dihydroxyethyl)-3,8-diazabicyclo[3.2.1]octane-8-carboxylate (388 mg). ES-API: [M+ H]⁺= 273.2.

Step 6: N,N-Diisopropylethylamine (342 mg, 2.644 mmol) was added to a solution of tert-butyl (1R,5S)-2-(1,2-dihydroxyethyl)-3,8-diazabicyclo[3.2.1]octane-8-carboxylate (360 mg, 1.322 mmol) in dichloromethane (20 mL) under an ice-water bath. The reaction mixture was stirred at 0°C for 0.5 hours. Bromomethyl methyl ether (198 mg, 1.586 mmol) was added, and the reaction mixture was stirred for an additional 2.5 hours at 0°C. Upon completion of the reaction (monitored by LCMS), the mixture was concentrated and the crude product was purified by automated flash chromatography through silica gel (methanol:dichloromethane = 0-10%) to obtain tert-butyl (1R,5S)-2-(1-hydroxy-2-(methoxymethoxy)ethyl)-3,8-diazabicyclo[3.2.1]octane-8-carboxylate (420 mg). ES-API: [M+ H]⁺= 317.2.

Step 7: Sodium hydride (212 mg, 5.310 mmol, 60% dispersed in oil) was added to a solution of tert-butyl (1R,5S)-2-(1-hydroxy-2-(methoxymethoxy)ethyl)-3,8-diazabicyclo[3.2.1]octane-8-carboxylate (420 mg, 1.327 mmol) in tetrahydrofuran (10 mL) under an ice-water bath. The reaction mixture was stirred at 0°C for 30 minutes. Then, 5,7-dichloro-8-fluoro-2-(methylthio)pyrido[4,3-d]pyrimidin-4(3H)-one (743 mg, 2.655 mmol) was added, and the mixture was slowly warmed to 60°C and stirred for 1.5 hours. Upon completion of the reaction (monitored by LCMS), saturated ammonium chloride solution (100 mL) was added, and the mixture was extracted with dichloromethane (50 mL × 3). The organic phase was dried over anhydrous sodium sulfate, filtered, and concentrated to obtain tert-butyl (1R,5S)-2-(1-((7-chloro-8-fluoro-2-(methylthio)-4-oxo-3,4-dihydropyrido[4,3-d]pyrimidin-5-yl)oxy)-2-(methoxymethoxy)ethyl)-3,8-diazabicyclo[3.2.1]octane-8-carboxylate (520 mg, yield: 70%). ES-API: [M+ H]⁺= 560.2.

Step 8: Under an ice-water bath, 2-(7-azabenzotriazolyl)-N,N,N',N'-tetramethyluronium hexafluorophosphate (706 mg, 1.857 mmol) was added to a solution of tert-butyl (1R,5S)-2-(1-((7-chloro-8-fluoro-2-(methylthio)-4-oxo-3,4-dihydropyrido[4,3-d]pyrimidin-5-yl)oxy)-2-(methoxymethoxy)ethyl)-3,8-diazabicyclo[3.2.1]octane-8-carboxylate (520 mg, 0.929 mmol) and N,N-diisopropylethylamine (480 mg, 3.714 mmol) in dichloromethane (20 mL). The reaction mixture was stirred at 0°C for 0.5 hours, then warmed to room temperature and stirred for 1.5 hours. Upon completion of the reaction (monitored by LCMS), the mixture was concentrated and purified by automated flash chromatography through silica gel (ethyl acetate:petroleum ether = 0-20%) to obtain tert-butyl 2-chloro-1 -fluoro-5-((methoxymethoxy)methyl)- 12-(methylthio)-5a,6,7,8,9,10-hexahydro-5H-4-oxa-3,10a,11,13,14-pentaaza-6,9-methanonaphtho[1,8-ab]heptalene-14-carboxylate (225 mg, yield: 45%). ES-API: [M+ H]⁺= 542.1.

Step 9: Under nitrogen protection, tert-butyl 2-chloro-1-fluoro-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (60 mg, 0.111 mmol), ((2-fluoro-8-(4,4,5,5-tetramethyl-1,3,2-dioxaborolane-2-yl)naphthalen-1-yl)ethynyl)triisopropylsilane (125 mg, 0.277 mmol), potassium phosphate (70 mg, 0.332 mmol), and methanesulfonic acid [n-butylbis(1-adamantyl)phosphine](2-wnino-1,1'-biphenyl-2-yl)palladium(II) (16 mg, 0.022 mmol) were dissolved in tetrahydrofuran (5 mL) and water (1 mL). The reaction mixture was stirred at 80°C for 0.5 hours in a microwave reactor. Upon completion of the reaction (monitored by LCMS), the mixture was concentrated and purified by automated flash chromatography through silica gel (petroleum ether/ethyl acetate = 0-20%) to obtain tert-butyl 1-fluoro-2-(7-fluoro-8-((triisopropylsilyl)ethynyl)naphthalen-1-yl)-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (49 mg, yield: 53%). ES-API: [M+ H]⁺= 832.3.

Step 10: Tert-butyl 1-fluoro-2-(7-fluoro-8-((triisopropylsilyl)ethynyl)naphthalen-1-yl)-5-((methoxymethoxy)methyl)-12-(methylthio)-Sa,6,7,8,9,10-hexahydro-SH-4-oxa-3,10a,11,13,14-pentaaza-6,9-methanonaphtho[1,8-ab]heptalene-14-carboxylate (49 mg, 0.059 mmol) was dissolved in anhydrous dichloromethane (10 mL). M-chloroperbenzoic acid (15 mg, 0.088 mmol) was added to the solution, and the reaction mixture was stirred at room temperature for 0.5 hours. Upon completion of the reaction (monitored by LCMS), the mixture was diluted with dichloromethane (15 mL), quenched with saturated sodium bisulfite solution (15 mL), then washed with sodium bicarbonate solution (15 mL) and saturated brine (15 mL). The organic phase was dried over anhydrous sodium sulfate, filtered, and concentrated to obtain crude tert-butyl 1-fluoro-2-(7-fluoro-8-((triisopropylsilyl)ethynyl)naphthalen-1-yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (49 mg). ES-API: [M+ H]⁺= 848.3.

Step 11: (1-Methylene tetrahydro-1H-pyrrozin-7a(5H)-yl)methanol (27 mg, 0.173 mmol) was dissolved in anhydrous tetrahydrofuran (5 mL). Under an ice-water bath, sodium hydride (7 mg, 0.173 mmol) was added and the reaction was proceeded for 10 minutes at 0°C. A tetrahydrofuran solution (5 mL) of tert-butyl 1-fluoro-2-(7-fluoro-8-((triisopropylsilyl)ethynyl)naphthalen-1-yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[l,8-ab]heptalene-14-carboxylate (49 mg, 0.058 mmol) was then added dropwise, and the mixture was stirred for 30 minutes at 0°C. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure. The crude product was purified by automated flash chromatography through silica gel (methanol/dichloromethane = 0~3%) to obtain tert-butyl 1-fluoro-2-(7-fluoro-8-((triisopropylsilyl)ethynyl)naphthalen-1-yl)-5-((methoxymethoxy)methyl)-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[l,8-ab]heptalene-14-carboxylate (50 mg, yield: 92%). ES-API: [M+ H]⁺= 937.4 .

Step 12: Cesium fluoride (52 mg, 0.341 mmol) was added to a solution of tert-butyl 1-fluoro-2-(7-fluoro-8-((triisopropylsilyl)ethynyl)naphthalen-1-yl)-5-((methoxymethoxy)methyl)-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (32 mg, 0.034 mmol) in N,N-dimethylformamide (3 mL). The reaction mixture was stirred at room temperature for 1 hour. Upon completion of the reaction (monitored by LCMS), the mixture was diluted with ethyl acetate (15 mL), washed with water (15 mL × 2), saturated brine (15 mL × 3), dried over anhydrous sodium sulfate, filtered, and concentrated. The crude product was purified by thin-layer chromatography (dichloromethane/methanol = 20:1) to obtain tert-butyl 2-(8-ethynyl-7-fluoronaphthalen-1-yl)-1-fluoro-5-((methoxymethoxy)methyl)-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (26 mg, yield: 98%). ES-API: [M+ H]⁺= 781.2.

Step 13: Under an ice-water bath, a 4 M solution of hydrochloric acid in dioxane (3 mL) was slowly added to a solution of tert-butyl 2-(8-ethynyl-7-fluoronaphthalen-1-yl)-1-fluoro-5-((methoxymethoxy)methyl)-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (26 mg, 0.033 mmol) in acetonitrile (2 mL). The reaction mixture was stirred at 0°C for 1 hour. Upon completion of the reaction (monitored by LCMS), the mixture was concentrated under reduced pressure to dryness, and the residue was neutralized with saturated sodium bicarbonate solution (3 mL) until the solution pH was above 7.0. The solution was extracted with dichloromethane (5 mL × 3), and the combined organic phase was concentrated to obtain a white-like solid (2-(8-ethynyl-7-fluoronaphthalen-1-yl)-1-fluoro-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1.8-ab]heptalen-5-yl)methanol (Z672, 17 mg, yield: 80 %). ES-API: [M+H]⁺= 637.2.

### Example 64 Synthesis of 8-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oza-3,10a,11,13,14-pentaaza-6,9-methanonaphtho[1,8-ab]heptalen-2-yl)-1-naphthonitrile (Z673)

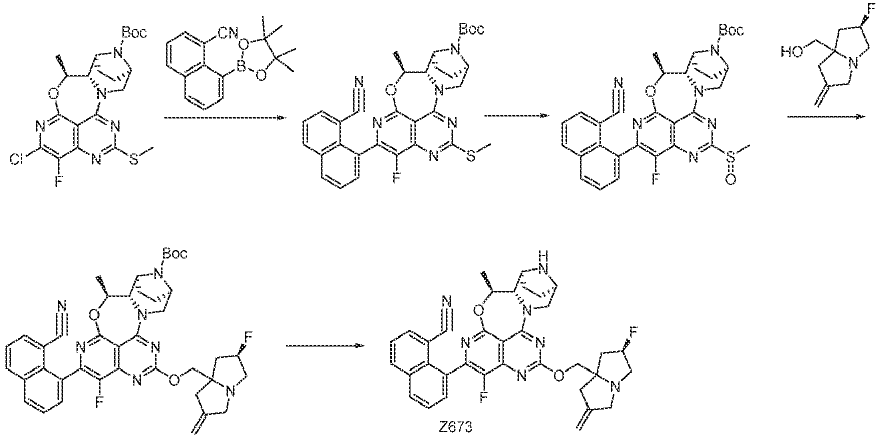

Step 1: Under nitrogen protection, tert-butyl (5S,SaS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (220 mg, 0.46 mmol), 8-(4,4,5,5-tetramethyl-1,3,2-dioxaborolane-2-yl)-1-naphthonitrile (319 mg, 1.14 mmol), potassium phosphate (291 mg, 1.37 mmol), and CataXium A Pd G3 (50 mg, 0.068 mmol) were dissolved in tetrahydrofuran (2.5 mL) and water (0.5 mL). The reaction mixture was heated to 80°C and stirred for 3 hours. Upon completion of the reaction (monitored by LCMS), the mixture was concentrated, and the crude product was purified by automated flash chromatography through silica gel (petroleum etherethyl acetate = 20:1) to obtain tert-butyl (SS,SaS,6S,9R)-2-(8-cyanonaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (190 mg, yield: 69.5%). ES-API: [M+H]⁺=599.2.

Step 2: Tert-butyl (SS,SaS,6S,9R)-2-(8i,yanonaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.17 mmol) was dissolved in dichloromethane (5 mL). M-chloroperbenzoic acid (43 mg, 0.25 mmol) was added, and the reaction mixture was stirred at room temperature for 1 hour. Upon completion of the reaction (monitored by LCMS), the mixture was diluted with ethyl acetate (20 mL), washed with saturated sodium bisulfite solution (15 mL × 2) and saturated brine (15 mL × 3), dried over anhydrous sodium sulfate, filtered, and concentrated to obtain crude tert-butyl (SS,5aS,6S,9R)-2-(8-cyanonaphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (102.7 mg, yield: 100%). ES-API: [M+H]⁺=615.2.

Step 3: ((2R)-2-fluoro-6-methylenetetrahydro-1H-pyrrolizin-7a(5H)-yl)methanol (56.8 mg, 0.33 mmol) was dissolved in anhydrous tetrahydrofuran (5 mL). Under an ice-water bath, sodium hydride (16 mg, 0.67 mmol) was added, and the reaction was proceeded for 30 minutes. Then, a tetrahydrofuran solution (2 mL) of tert-butyl (SS,SaS,6S,9R)-2-(8-cyanonaphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (102.7 mg, 0.17 mmol) was added dropwise. The mixture was stirred at room temperature for 30 minutes. Upon completion of the reaction (monitored by LCMS), the reaction mixture was diluted with ethyl acetate (20 mL), washed with saturated ammonium chloride solution (15 mL × 2), and saturated brine (15 mL × 3), dried over anhydrous sodium sulfate, filtered, and concentrated. The crude product was purified by automated flash chromatography through silica gel (dichloromethane:methanol = 20:1) to obtain tert-butyl (SS,SaS,6S,9R)-2-(8-cyanonaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, yield: 42%). ES-API: [M+H]⁺= 722.2.

Step 4: Under an ice-water bath, a 4 M solution of hydrochloric acid-dioxane (3 mL) was slowly added to a solution of tert-butyl (5S,5aS,6S,9R)-2-(8-cyanonaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, 0.069 mmol) in acetonitrile (2 mL). The reaction mixture was stirred at 0°C for 0.5 hours. Upon completion of the reaction (monitored by LCMS), the mixture was concentrated under reduced pressure to dryness, and the residue was purified by preparative HPLC using a bicarbonate method (chromatographic condition: 15 ml-35-85-13 min) to obtain a light yellow solid 8-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalen-2-yl)-1-naphthonitrile (Z673, 10 mg, yield: 23 %). ES-API: [M+H]⁺= 647.3. ¹H NMR (400 MHz, CD₃OD) δ 8.35 (t, J = 7.8 Hz, 1H), 8.20 (d, J = 8.0 Hz, 1H), 8.08-8.01 (m, 1H), 7.78 (d, J = 4.0 Hz, 1H), 7.76 -7.69 (m, 1H), 7.68 (d, J = 8.0 Hz, 1H), 5.45-5.36 (m, 2H), 5.24 (d, J = 16.0 Hz, 1H), 5.07-5.01 (m, 2H), 4.54-5.48 (m, 2H), 4.45-4.36 (m, 1H), 4.31-4.27 (m, 1H), 4.12 (d, J = 8.0 Hz, 1H), 3.78-3.70 (m, 2H), 3.67-3.59 (m, 1H), 3.51 (s, 1H), 3.49-3.45 (m, 1H), 3.41-3.39 (m, 1H), 3.25-3.19 (m, 1H), 3.05-2.93 (m, 1H), 2.92-2.89 (m, 1H), 2.86-2.83 (m, 1H), 2.81-2.78 (m, 1H), 2.55-2.47 (m, 2H), 2.42-2.37 (m, 1H), 2.23 -2.15 (m, 1H), 2.10-2.04 (m, 2H).

### Example 65 Synthesis of 7a-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)-7-methylenehexahydro-1H-pyrrolizin-2-ol (Z674)

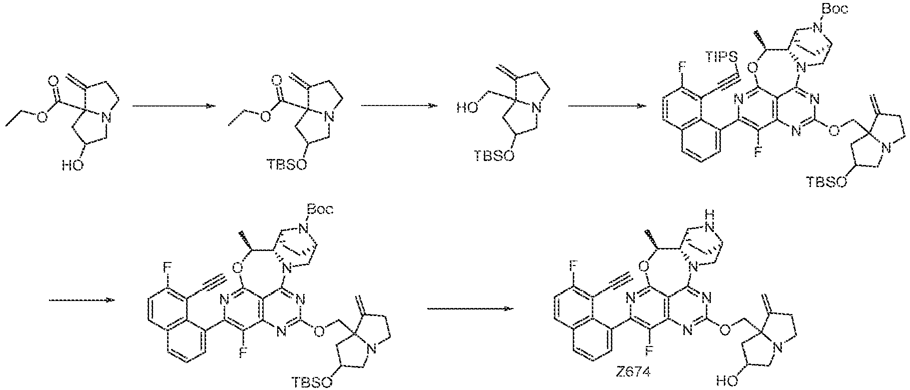

Step 1: At 0°C, imidazole (87.0 mg, 1.278 mmol) and tert-butyldimethylchlorosilane (256.84 mg, 1.704 mmol) were added to a solution of ethyl 6-hydroxy-1-methylenetetrahydro-1H-pyrrolizine-7a(5H)-carboxylate (180 mg, 0.852 mmol) in N,N-dimethylformamide (5.0 mL). The mixture was stirred overnight at room temperature. Upon completion of the reaction (monitored by LCMS), the mixture was diluted with ethyl acetate (60 mL), washed with water (30 mL × 2), saturated brine (50 mL × 3), dried over anhydrous sodium sulfate, filtered, and concentrated to obtain ethyl 6-((tertbutyldimethylsilyl)oxy)-1-methylenetetrahydro-1H-pyrrolizine-7a(5H)-carboxylate (200 mg, yield 72%). ES-API: [M+H]⁺= 326.2.

Step 2: Ethyl 6-((tert-butyldimethylsilyl)oxy)-1-methylenetetrahydro-1H-pyrrolizine-7a(5H)-carboxylate (200 mg, 0.614 mmol) was dissolved in tetrahydrofuran (10 mL) and cooled to 0°C. Under nitrogen atmosphere, a solution of lithium aluminum hydride (1.3 mL, 1.3 mmol, 1 M in tetrahydrofuran) was added. The reaction mixture was stirred at 0°C for 1 hour. Water (0.03 mL) was added to quench the reaction, followed by 15% sodium hydroxide solution (0.03 mL) and water (0.09 mL). The mixture was filtered, and the filtrate was concentrated to obtain (6-((tertbutyldimethylsilyl)oxy)-1-methylenetetrahydro-1H-pyrrolizin-7a(5H)-yl)methanol (140 mg, yield 80.38%), a colorless transparent liquid. ES-API: [M +H]⁺=284.2.

Step 3: (6-((tert-butyldimethylsilyl)oxy)-1-methylenetetrahydro-1H-pyrrolizin-7a(5H)-yl)methanol (140 mg, 0.494 mmol) was dissolved in anhydrous tetrahydrofuran (5 mL). Under an ice-water bath, sodium hydride (40.0 mg, 0.988 mmol) was added and the reaction was proceeded for 30 minutes. A tetrahydrofuran solution (2 mL) of tert-butyl (5S, 5aS, 6S, 9R)-1-fluoro-2-(7-fluoro-8-((triisopropylsilyl)ethynyl)naphthalen-1-yl)-5-methyl-12-(methylsulfonyl)-5a,6,7,8,9,10-hexahydro-5H-4-oxa-3,10a,11,13,14-pentaza-6,9-methylenenaphtho[1,8-ab]heptene-14-carboxylate (600 mg, 0.761 mmol) was added dropwise, and the reaction mixture was stirred for 30 minutes at 0°C. Upon completion of the reaction (monitored by LCMS), the mixture was diluted with ethyl acetate (50 mL), washed with saturated ammonium chloride solution (15 mL × 2), saturated brine (50 mL × 3), dried over anhydrous sodium sulfate, filtered, and the crude product was purified by automated flash chromatography through silica gel (dichloromethane:methanol = 15:1) to obtain tert-butyl (5S,5aS,6S,9R)-12-((6-((tert-butyldimethylsilyl)oxy)-1-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[l,8-ab]heptalene-14-carboxylate (160 mg, yield 32.16%). ES-API: [M+H]⁺= 1007.5.

Step 4: Cesium fluoride (100.0 mg, 0.658 mmol) was added to a solution of tert-butyl (5S,5aS,6S,9R)-12-((6-((tert-butyldimethylsilyl)oxy)-1-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, 0.060 mmol) in N,N-dimethylformamide (5.0 mL). The reaction mixture was stirred at room temperature for 2 hours. Upon completion of the reaction (monitored by LCMS), the mixture was diluted with ethyl acetate (60 mL), washed with water (30 mL × 2), saturated brine (50 mL × 3), dried over anhydrous sodium sulfate, filtered, and concentrated to obtain crude tert-butyl (5S,5aS,6S,9R)-12-((6-((tert-butyldimethylsilyl)oxy)-1-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9, 10-hexahydro-5H-4-oxa-3, 10a, 11, 13,14-pentaaza-6,9-methanonaphtho[1,8-ab]heptalene-14-carboxylate (108 mg, crude product). ES-API: [M+H]⁺= 851.2.

Step 5: Under an ice-water bath, a 4 M hydrochloric acid solution in dioxane (1.0 mL) was slowly added to a solution of tert-butyl (5S,5aS,6S,9R)-12-((6-((tert-butyldimethylsilyl)oxy)-1-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (51.06 mg, 0.060 mmol) in acetonitrile (5.0 mL). The reaction mixture was stirred at 0°C for 0.5 hours. Upon completion of the reaction (monitored by LCMS), the mixture was concentrated under reduced pressure to dryness, and the residue was purified by preparative HPLC using a bicarbonate method (chromatographic condition: 15ml-35-85-13min) to obtain a pale yellow solid 7a-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)-7-methylenehexahydro-1H-pyrrolizin-2-ol (Z674, 3.0mg, yield: 7.85%). ES-API: [M+H]⁺= 637.2.

### Example 66 Synthesis of (5S,5aS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z675)

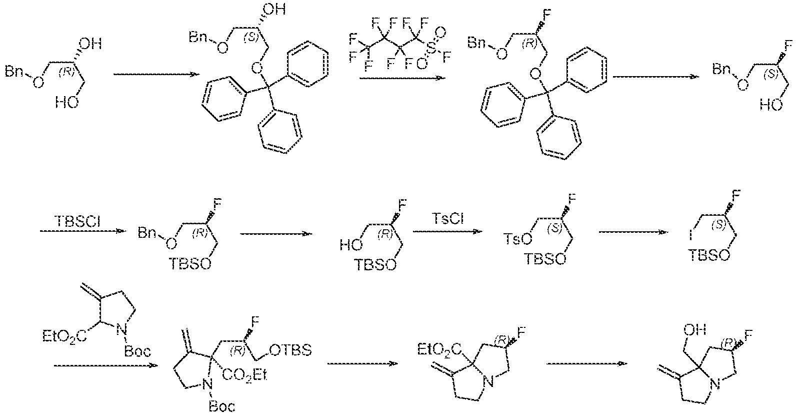

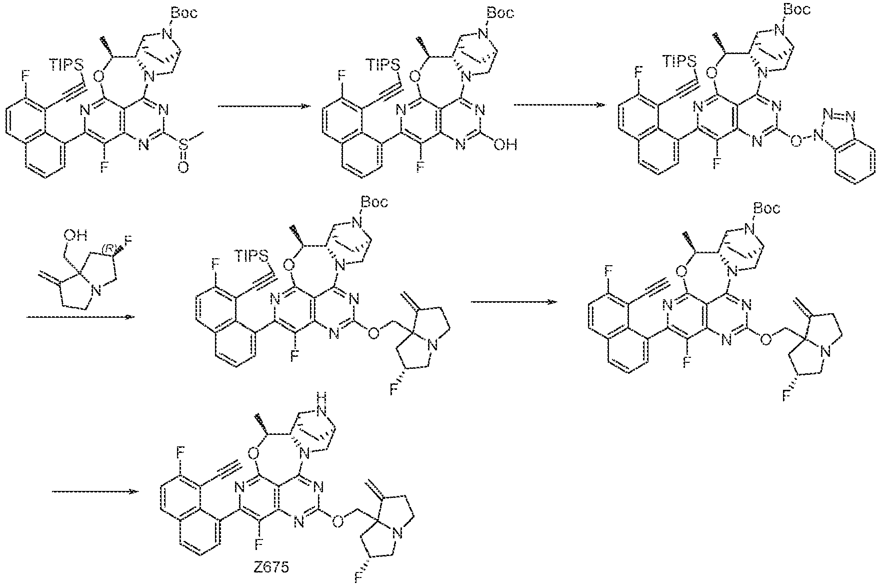

Step 1: To a round-bottom flask, 4-dimethylaminopyridine (1.01 g, 8.23 mmol), triphenylchloromethane (18.36 g, 65.85 mmol), triethylamine (30.5 mL, 219.51 mmol), (R)-(+)-1-benzylglycerol (10 g, 54.879 mmol), and tetrahydrofuran (300 mL) were added successively. Under nitrogen protection, the reaction mixture was stirred in a 65°C oil bath for 17 hours. The reaction was quenched with water, extracted with ethyl acetate (150 mL × 2), dried over anhydrous sodium sulfate, filtered, and concentrated. The residue was purified through flash silica gel chromatography (0-10% ethyl acetate/petroleum ether) to obtain (S)-1-(benzyloxy)-3-(trityloxy)propan-2-ol (19 g, yield 81%). ES-API:[M+Na]⁺=447.3.

Step 2: At 0°C, (S)-1-(benzyloxy)-3-(trityloxy)propan-2-ol (19 g, 44.75 mmol) and toluene (200 mL) were added to a round-bottom flask. Perfluorobutanesulfonyl fluoride (14.47 mL, 80.56 mmol) was added dropwise, followed by the addition of 1,8-diazabicyclo[5.4.0]undec-7-ene (12 mL, 80.56 mmol) in toluene (20 mL). The reaction mixture was stirred at 0°C for 30 minutes and then brought to room temperature and stirred for 3 hours. The reaction was quenched with water, and the mixture was extracted with ethyl acetate (150 mL × 2), dried over anhydrous sodium sulfate, filtered, and concentrated. The residue was purified through flash silica gel chromatography (0-15% ethyl acetate/petroleum ether) to obtain (R)-((3-(benzyloxy)-2-fluoropropoxy)methanetriyl)tribenzene (15 g, yield 78%), a yellow oil. ES-API:[M+Na]⁺=449.2.

Step 3: (R)-((3-(benzyloxy)-2-fluoropropoxy)methanetriyl)tribenzene (15 g, 35.17 mmol), water (20 mL), 1,4-dioxane (20 mL), and acetic acid (100 mL) were added successively to a round-bottom flask. The reaction mixture was stirred at 90°C for 5 hours. The reaction mixture was concentrated to remove acetic acid, and the residue was added to water (100 mL) and extracted with ethyl acetate (100 mL × 2). The organic layer was washed with saturated sodium bicarbonate, dried over anhydrous sodium sulfate, filtered, and concentrated. The residue was purified through flash silica gel chromatography (0-35% ethyl acetate/petroleum ether) to obtain (S)-3-(benzyloxy)-2-fluoropropan-1-ol (5 g, yield 77%), a yellow oil. ES-API:[M+Na]⁺=207.1.

Step 4: At 0°C, to a dichloromethane (90 mL) solution of (S)-3-(benzyloxy)-2-fluoropropan-1-ol (5 g, 27.14 mmol), 4-dimethylaminopyridine (0.33 g, 2.71 mmol), and imidazole (2.77 g, 40.71 mmol), tert-butyldimethylchlorosilane (4.91 g, 32.57 mmol) was added. The reaction mixture was stirred at room temperature for 5 hours. The reaction was quenched with water (100 mL), and the mixture was extracted with ethyl acetate (100 mL × 2), washed with saturated brine, dried over anhydrous sodium sulfate, filtered, and concentrated. The residue was purified through flash silica gel chromatography (0-12% ethyl acetate/petroleum ether) to obtain (R)-(3-(benzyloxy)-2-fluoropropoxy)(tert-butyl)dimethylsilane (8 g, yield 98%), a colorless oil. ES-API:[M+H]⁺=299.1.

Step 5: To a methanol (30 mL) solution of (R)-(3-(benzyloxy)-2-fluoropropyloxy)(tert-butyl)dimethylsilane (8 g, 26.80 mmol), 10% palladium on carbon (1 g, 50% water content) was added. Under hydrogen protection, the reaction mixture was stirred at room temperature for 48 hours. The reaction mixture was filtered through diatomaceous earth. The filtrate was concentrated to obtain (R)-3-((tert-butyldimethylsilyl)oxy)-2-fluoropropan-1-ol (5.4 g, yield 96%). ES-API: [M+H]⁺=209.2.

Step 6: To a round-bottom flask, 4-dimethylaminopyridine (0.63 g, 5.18 mmol), (R)-3-((tertbutyldimethylsilyl)oxy)-2-fluoropropan-1-ol (5.4 g, 25.92 mmol), triethylamine (7.2 mL, 51.84 mmol), and dichloromethane (100 mL) were added successively. The reaction mixture was cooled to 0°C, and p-toluenesulfonyl chloride (7.41 g, 38.88 mmol) was added. The reaction mixture was stirred at 0°C for 2 hours. The reaction was quenched with water (60 mL) and 1M hydrochloric acid (60 mL), then the mixture was extracted with dichloromethane (100 mL × 2). The organic layer was washed with saturated sodium bicarbonate, dried over anhydrous sodium sulfate, filtered, and concentrated to obtain (S)-3-((tert-butyldimethylsilyl)oxy)-2-fluoropropyl 4-methylbenzenesulfonate (9.2 g, yield 97%). ES-AP1:[M+H]⁺=363.2.

Step 7: (S)-3-((tert-butyldimethylsilyl)oxy)-2-fluoropropyl 4-methylbenzenesulfonate (9.2 g, 25.38 mmol) was dissolved in acetone (100 mL), and sodium iodide (19.02 g, 126.89 mmol) was added. The reaction mixture was stirred at 75°C for 5 hours. The reaction mixture was concentrated, and with ethyl acetate (100 mL) added, the residue was diluted with water (50 mL) and saturated sodium sulfite (30 mL). The solution was extracted with ethyl acetate (100 mL). The organic layer was washed with saturated brine, dried over anhydrous sodium sulfate, filtered, and concentrated. The residue was purified through flash silica gel chromatography (0-5% ethyl acetate/petroleum ether) to obtain (S)-tert-butyl (2-fluoro-3-iodopropyloxy)dimethylsilane (7.7 g, yield 95%). ¹H NMR: (CDCl₃, 400MHz) 4.50-4.35 (m, 1H), 3.80-3.75 (m, 2H), 3.41-3.15 (m, 2H), 0.81 (s, 9H), 0.01(s, 6H).

Step 8: To a round-bottom flask, 1-(tert-butyl) 2-ethyl 3-methylenepyrrolidine-1,2-dicarboxylate (800 mg, 3.13 mmol) and tetrahydrofuran (15 mL) were added. The mixture was cooled to -78°C, and a tetrahydrofuran solution of bis(trimethylsilyl)amino sodium (6.27 mL, 6.27 mmol) was added dropwise. After the dropwise addition was complete, the reaction mixture was stirred at -78°C for 1 hour. A tetrahydrofuran solution of (S)-tert-butyl (2-fluoro-3-iodopropyloxy)dimethylsilane (1.6 g, 5.02 mmol) in tetrahydrofuran (3 mL) was added. After the dropwise addition was complete, the reaction continued to stir at -78°C for 30 minutes and then was stirred at room temperature for 2 hours. The reaction mixture was quenched with saturated ammonium chloride solution (100 mL), extracted with ethyl acetate (100 mL × 2), washed with saturated brine, dried over anhydrous sodium sulfate, filtered, and concentrated. The residue was purified through flash silica gel chromatography (0-8% ethyl acetate/petroleum ether) to obtain 1-(tert-butyl) 2-ethyl 2-((R)-3-((tertbutyldimethylsilyl)oxy)-2-fluoropropyl)-3-methylenepyrrolidine-1,2-dicarboxylate (700 mg, yield 50%). ES-API:[M+Na]⁺=468.3.

Step 9: To 1-(tert-butyl) 2-ethyl 2-((R)-3-((tert-butyldimethylsilyl)oxy)-2-fluoropropyl)-3-methylenepyrrolidine-1,2-dicarboxylate (700 mg, 1.57 mmol), thionyl chloride (10 mL, 137.86 mmol) was added. The reaction mixture was stirred at 45°C for 24 hours. With thionyl chloride (10 mL, 137.86 mmol) added, the reaction mixture was stirred at 65°C for 2 days. The reaction mixture was concentrated. With dichloromethane (5 mL) added to the residue, the mixture was cooled to 0°C. Triethylamine (0.22 mL, 1.57 mmol) was added, and the reaction mixture was stirred at room temperature for 30 minutes, quenched with water (50 mL), extracted with dichloromethane (30 mL × 2), washed with saturated brine (30 mL), dried over anhydrous sodium sulfate, filtered, and concentrated. The residue was purified through flash silica gel chromatography (0-60% ethyl acetate/petroleum ether) to obtain ethyl (6R)-6-fluoro-1-methylenetetrahydro-1H-pyrrolizine-7a(5H)-carboxylate (170 mg, yield 50%). ES-API:[M+H]₊=214.2.

Step 10: To a tetrahydrofuran (5 mL) solution of ethyl (6R)-6-fluoro-1-methylenetetrahydro-1H-pyrrolizine-7a(5H)-carboxylate (170 mg, 0.80 mmol), a tetrahydrofuran solution of lithium aluminum hydride (1.59 mL, 1.59 mmol) was added dropwise at 0°C and stirred for 1 hour, quenched successively with 3 drops of water and 15% sodium hydroxide solution (0.1 mL). The mixture was stirred at room temperature for 30 minutes and filtered through diatomaceous earth. The filtrate was concentrated to obtain crude ((6R)-6-fluoro-1-methylenetetrahydro-1H-pyrrolizin-7a(5H)-yl)methanol (130 mg, yield 95%), a yellow oil. ES-API: [M+H]⁺=172.1.

Step 11: To a water (3 mL) solution of potassium hydroxide (142 mg, 2.54 mmol), tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (400 mg, 0.51 mmol) and tetrahydrofuran (8 mL) were added. The reaction mixture was stirred at 60°C for 1 hour, quenched with water (50 mL), extracted with ethyl acetate (100 mL), washed with saturated brine, dried over anhydrous sodium sulfate, filtered, and concentrated to obtain tert-butyl (SS,SaS,6S,9R)-1-fluoro-2-(7-fluoro-8-((triisopropylsilyl)ethynyl)naphthalen-1-yl)-12-hydroxy-5-methyl-5a,6,7,8,9,10-hexahydro-5H-4-oxa-3,10a,11,13,14-pentaaza-6,9-methanonaphtho[1,8-ab]heptalene-14-carboxylate (376 mg, yield 99%). ES-API: [M+H]⁺=742.3.

Step 12: To tert-butyl (SS,SaS,6S,9R)-1-fluoro-2-(7-fluoro-8-((triisopropylsilyl)ethynyl)naphthalen-1-yl)-12-hydroxy-5-methyl-5a,6,7,8,9,10-hexahydro-5H-4-oxa-3,10a,11,13,14-pentaaza-6,9-methanonaphtho[1,8-ab]heptalene-14-carboxylate (376 mg, 0.51 mmol), cesium carbonate (330 mg, 1.01 mmol), and tetrahydrofuran (15 mL) were added to a round-bottom flask along with a Carter coupling reagent (448 mg, 1.01 mmol). The reaction mixture was stirred at room temperature for 1 hour, quenched with water (50 mL), extracted with ethyl acetate (100 mL), washed with saturated brine, dried over anhydrous sodium sulfate, filtered, and concentrated. The residue was purified through flash silica gel chromatography (0-50% ethyl acetate/petroleum ether) to obtain tert-butyl (5S,5aS,6S,9R)-12-((1H-benzo[d][1,2,3]triazol-1-yl)oxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (390 mg, yield 89%). ES-API: [M+H]⁺=859.2.

Step 13: To a round-bottom flask, sodium hydride (21 mg, 0.52 mmol) and tetrahydrofuran (8 mL) were added. Cooled to 0°C, ((6R)-6-fluoro-1-methylenetetrahydro-1H-pyrrolizin-7a(5H)-yl)methanol (60 mg, 0.35 mmol) in anhydrous tetrahydrofuran (2 mL) was added to the reaction mixture. The reaction mixture was stirred at room temperature for 10 minutes. Cooled to 0°C, tert-butyl (5S, 5aS, 6S, 9R)-12-(1H-benzotriazole-1-yl)oxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanenaphthacene[1,8-ab]heptene-14-carboxylate (150 mg, 0.17 mmol) was added to the reaction mixture, and the reaction mixture was stirred at room temperature for 1 hour. Water (30 mL) was added to the reaction mixture, which was then extracted with ethyl acetate (20 mL × 2). The organic layer was washed with saturated brine (30 mL), dried over anhydrous sodium sulfate, filtered, and concentrated to obtain tert-butyl (SS,SaS,6S,9R)-1-fluoro-12-(((6R)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (150 mg, yield 95%). ES-API: [M+H]⁺=895.3.

Step 14: To a solution of tert-butyl (5S,5aS,6S,9R)-1-fluoro-12-(((6R)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (150 mg, crude product) in N,N-dimethylformamide (5 mL), cesium fluoride (509 mg, 3.35 mmol) was added. The reaction mixture was stirred at room temperature for 2 hours. Upon completion of the reaction (monitored by LCMS), ethyl acetate (100 mL) was added to dilute the reaction mixture, which was then washed with water (30 mL) and saturated brine (30 mL × 2). The organic layer was dried over anhydrous sodium sulfate, filtered, and concentrated. The residue was purified through flash silica gel chromatography (0-5% methanol/dichloromethane) to obtain tert-butyl (SS,SaS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene-14-carboxylate (120 mg, yield 96%). ES-API: [M+H]⁺= 739.2.

Step 15: Under an ice water bath, 4 M hydrochloric acid-dioxane solution (3 mL) was slowly added to a solution of tert-butyl (SS,SaS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (120 mg, 0.16 mmol) in acetonitrile (2 mL). The reaction mixture was stirred at room temperature for 1 hour. Upon completion of the reaction (monitored by LCMS), the reaction mixture was concentrated under reduced pressure, and the residue was dissolved in saturated bicarbonate solution and dichloromethane, and the mixture was extracted with dichloromethane/methanol (10:1, 50 mL). The organic phase was washed with saturated brine (30 mL), dried over anhydrous sodium sulfate, filtered, and concentrated to obtain (SS,SaS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z675, 80mg, yield: 77%), yellow solid. ES-API: [M+H]⁺= 639.2.

### Example 67 Synthesis of 5-cyclopropyl-6-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)-4-methylpyridin-2-amine (Z676)

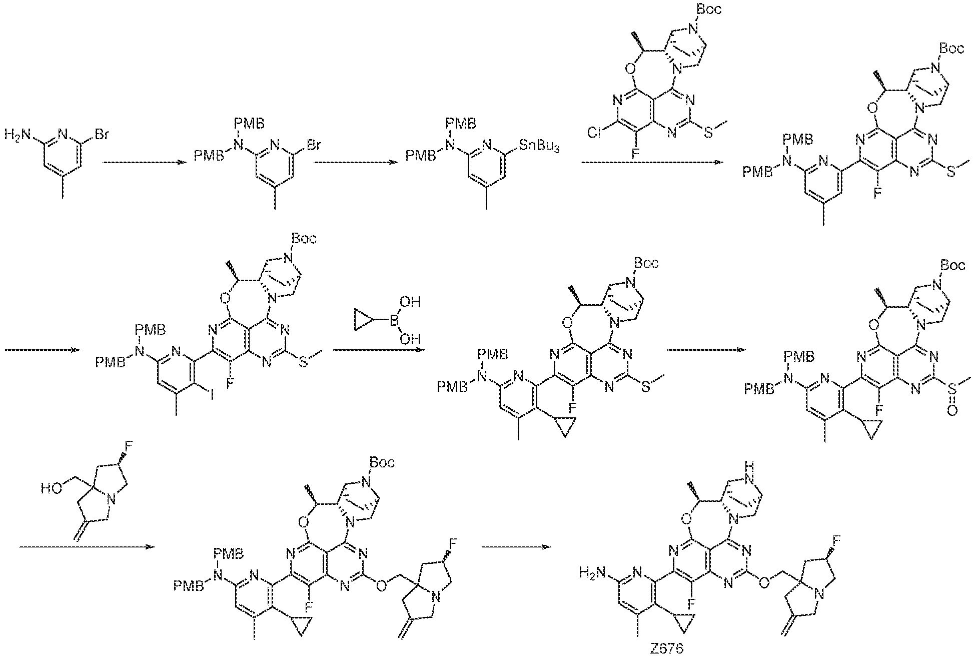

Step 1: 6-Bromo-4-methylpyridin-2-amine (2 g, 10.69 mmol) was dissolved in N,N-dimethylformamide (40 mL) and 60% sodium hydride (2.14 g, 53.46 mmol) was added at 0°C. After the mixture stirred at room temperature for 1 hour, p-methoxybenzyl chloride (3.68 g, 23.52 mmol) was added dropwise, and the reaction mixture was stirred at room temperature for 2 hours. The reaction mixture was quenched with saturated ammonium chloride solution (50 mL) and extracted with ethyl acetate (100 mL). The organic phase was washed with saturated brine (50 mL × 3), dried over anhydrous sodium sulfate, and filtered. The filtrate was concentrated under reduced pressure. The crude product was purified by flash silica gel column chromatography (ethyl acetate/petroleum ether: 0-15%) to obtain the target product 6-bromo-N,N-bis(4-methoxybenzyl)-4-methylpyridin-2-amine (3.8 g, yield 83.2%) as a white solid. ES-API: [M+H]⁺= 427.1, 428.9.

Step 2: To a 100 mL round-bottom flask, 6-bromo-N,N-bis(4-methoxybenzyl)-4-methylpyridin-2-amine (1.20 g, 2.81 mmol), hexabutylditin (4.89 g, 8.42 mmol), tris(dibenzylideneacetone)dipalladium (257 mg, 0.28 mmol), tri(cyclohexyl)phosphine (157 mg, 0.56 mmol), lithium chloride (595 mg, 14.04 mmol), and 1,4-dioxane (20 mL) were added. The mixture was purged with nitrogen three times and reacted at 110°C for 5 hours. The cooled reaction mixture was concentrated under reduced pressure. The crude product was purified by flash silica gel column chromatography (ethyl acetate/petroleum ether: 0-10%) to obtain the target product N,N-bis(4-methoxybenzyl)-4-methyl-6-(tributylstannyl)pyridin-2-amine (1.60 g, yield 89.4%) as a pale yellow liquid. ES-API: [M+H]⁺=639.3.

Step 3: To a 100 mL round-bottom flask, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (450 mg, 0.93 mmol), N,N-bis(4-methoxybenzyl)-4-methyl-6-(tributylstannyl)pyridin-2-amine (1.20 g, 1.88 mmol), copper(1) iodide (178 mg, 0.93 mmol), lithium chloride (158 mg, 3.74 mmol), and N,N-dimethylacetamide (20 mL) were added. The mixture was purged with nitrogen three times and reacted at 120°C for 3 hours. The cooled reaction mixture was added to ethyl acetate (60 mL), and washed successively with dilute brine (30 mL × 3), and saturated brine (30 mL). The organic phase was dried over anhydrous sodium sulfate, filtered, and the filtrate was concentrated under reduced pressure. The crude product was purified by flash silica gel column chromatography (ethyl acetate/petroleum ether: 0-10%) to obtain the target product tert-butyl (SS,SaS,6S,9R)-2-(6-(bis(4-methoxybenzyl)amino)-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (500 mg, yield 67.4%) as an off-white solid. ES-API: [M+H]⁺=794.2.

Step 4: Tert-butyl (SS,SaS,6S,9R)-2-(6-(bis(4-methoxybenzyl)amino)-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (450 mg, 0.57 mmol) and p-toluenesulfonic acid monohydrate (11 mg, 0.057 mmol) were dissolved in N,N-dimethylformamide (10 mL). At room temperature, N-iodosuccinimide (638 mg, 2.83 mmol) was added, and the reaction mixture was stirred at room temperature for 2 hours. The reaction mixture was added to ethyl acetate (60 mL), and washed successively with saturated sodium thiosulfate (40 mL), and saturated brine (30 mL). The organic phase was dried over anhydrous sodium sulfate, filtered, and the filtrate was concentrated under reduced pressure. The crude product was purified by flash silica gel column chromatography (ethyl acetate/petroleum ether: 0-40%) to obtain the target product tert-butyl (5S,5aS,6S,9R)-2-(6-(bis(4-methoxybenzyl)amino)-3-iodo-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (450 mg, yield 86.37%) as a yellow solid. ES-API: [M+H]⁺=920.0.

Step 5: To tert-butyl (SS,SaS,6S,9R)-2-(6-(bis(4-methoxybenzyl)amino)-3-iodo-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, 0.44 mmol) and cyclopropylboronic acid (187 mg, 2.17 mmol) in a mixture of toluene (10 mL) and water (1 mL), potassium phosphate (277 mg, 1.31 mmol), 2-dicyclohexylphosphino-2',6'-dimethoxybiphenyl (36 mg, 0.088 mmol), and palladium acetate (10 mg, 0.043 mmol) were added. The mixture was purged with nitrogen three times and then reacted at 105°C for 3 hours. The reaction mixture was added to ethyl acetate (60 mL) and washed with saturated brine (30 mL). The organic phase was dried over anhydrous sodium sulfate, filtered, and the filtrate was concentrated under reduced pressure. The crude product was purified by flash silica gel column chromatography (ethyl acetate/petroleum ether: 0-40%) to obtain the target product tert-butyl (SS,SaS,6S,9R)-2-(6-(bis(4-methoxybenzyl)amino)-3-cyclopropyl-4-methylpyridin-2-yl)-1-fluoro-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (350 mg, yield 96.5%) as a pale yellow solid. ES-API: [M+H]⁺=834.2.

Step 6: Tert-butyl (SS,SaS,6S,9R)-2-(6-(bis(4-methoxybenzyl)amino)-3-cyclopropyl-4-methylpyridin-2-yl)-1-fluoro-5-methyl-12-(methylthio)-Sa,6,7,8,9,10-hexahydro-SH-4-oxa-3,10a,11,13,14-pentaaza-6,9-methanonaphtho[1,8-ab]heptalene-14-carboxylate (250 mg, 0.30 mmol) was dissolved in dichloromethane (10 mL). At room temperature, m-chloroperbenzoic acid (91 mg, 0.45 mmol) was added, and the reaction mixture was stirred at room temperature for 1 hour. The reaction mixture was added to dichloromethane (50 mL) and washed successively with saturated sodium thiosulfate (15 mL), saturated sodium bicarbonate (15 mL), and saturated brine (30 mL). The organic phase was dried over anhydrous sodium sulfate, filtered, and the filtrate was concentrated under reduced pressure to obtain the target product tert-butyl (SS,SaS,6S,9R)-2-(6-(bis(4-methoxybenzyl)amino)-3-cyclopropyl-4-methylpyridin-2-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (250 mg, yield 98.1%) as a white solid. ES-API: [M+H]⁺=850.2.

Step 7: Tert-butyl (SS,SaS,6S,9R)-2-(6-(bis(4-methoxybenzyl)amino)-3-cyclopropyl-4-methylpyridin-2-yl)-1-fluoro-5-methyl-12-(methylsulfinyl)-5a,6,7,8,9,10-hexahydro-5H-4-oxa-3,10a,11,13,14-pentaaza-6,9-methanonaphtho[l,8-ab]heptalene-14-carboxylate (250 mg, 0.29 mmol) and ((2R)-2-fluoro-6-methylenetetrahydro-1H-pyrrolizin-7a(5H)-yl)methanol (76 mg, 0.44mmol) were dissolved in tetrahydrofuran (10mL). At 0°C, 1.3M solution of bis(trimethylsilyl)amine lithium in tetrahydrofuran (0.45 mL, 0.59 mmol) was added dropwise, and the reaction mixture was stirred at this temperature for 30 minutes. The reaction mixture was added to ethyl acetate (60 mL), and washed successively with water (20 mL), and saturated brine (30 mL). The organic phase was dried over anhydrous sodium sulfate, filtered, and the filtrate was concentrated under reduced pressure. The crude product was purified by preparative thin-layer chromatography (dichloromethane/7.0M ammonium hydroxide=25:1) to obtain the target product tert-butyl (5S,5aS,6S,9R)-2-(6-(bis(4-methoxybenzyl)amino)-3-cyclopropyl-4-methylpyridin-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (80 mg, yield 28.4%) as a pale yellow solid. ES-API: [M+H]⁺=957.3.

Step 8: Tert-butyl (SS,SaS,6S,9R)-2-(6-(bis(4-methoxybenzyl)amino)-3-cyclopropyl-4-methylpyridin-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalene-14-carboxylate (75 mg, 0.078 mmol) was dissolved in trifluoroacetic acid (4 mL). The reaction mixture was stirred at 50°C for 2 hours. The reaction mixture was concentrated under reduced pressure to dryness, then dichloromethane (5 mL) and 7.0M ammonium hydroxide (5 mL) were added, and the mixture was concentrated under reduced pressure. The crude product was purified by flash silica gel column chromatography (dichloromethane/7.0M ammonium hydroxide: 0-15%) to obtain the target product 5-cyclopropyl-6-((SS,SaS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)-4-methylpyridin-2-amine (Z676, 35 mg, yield: 72.4%). ES-API: [M+H]⁺= 617.2.

### Example 68 Synthesis of (SS,SaS,6S,9R)-2-(8-ethynylnaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z677) and (5S,5aS,6S,9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z702)

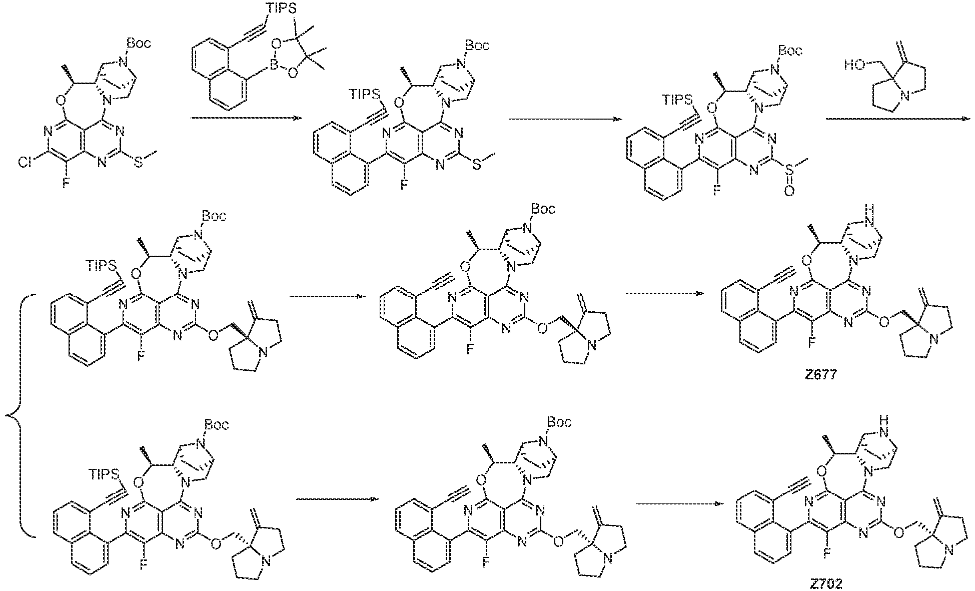

Step 1: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (100 mg, 0.207 mmol), triisopropyl((8-(4,4,5,5-tetramethyl-1,3,2-dioxaborolane-2-yl)naphth-1-yl)ethynyl)silane (180 mg, 0.415 mmol), potassium phosphate (132 mg, 0.622 mmol), and palladium(II) (2-amino-1,1'-biphenyl-2-yl)bis(1-kadamantylphosphine) methanesulfonate (23 mg, 0.031 mmol) were dissolved in tetrahydrofuran (12 mL) and water (2.5 mL). The reaction mixture was heated to 80°C in a sealed tube and stirred for 3 hours. Upon completion of the reaction (monitored by LCMS), the reaction mixture was concentrated, and the crude product was purified through silica gel using automatic flash chromatography (petroleum ether/ethyl acetate = 0 - 20 %), yielding tert-butyl (5S,5aS,6S,9R)-1-fluoro-5-methyl-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (156 mg, yield: 99 %). ES-API: [M+H]⁺= 754.3.

Step 2: Tert-butyl (5S,5aS,6S,9R)-1-fluoro-5-methyl-12-(methylthio)-2-(8-((triisopropylsilyl)ethynyl)naphthalen-1 -yl)-5a,6,7,8,9,10-hexahydro-5H-4-oxa-3,10a, 11,13,14-pentaaza-6,9-methanonaphtho[1,8-ab]heptalene-14-carboxylate (156 mg, 0.207 mmol) was dissolved in anhydrous dichloromethane (10 mL), and m-chloroperbenzoic acid (54 mg, 0.310 mmol) was added to the solution. The reaction mixture was stirred at room temperature for 0.5 hours. Upon completion of the reaction (monitored by LCMS), dichloromethane (15 mL) was added to dilute the reaction mixture, which was then quenched with saturated sodium sulfite solution (15 mL), then washed with sodium bicarbonate solution (15 mL) and saturated brine (15 mL). The organic phase was dried over anhydrous sodium sulfate, filtered, and concentrated to obtain the crude product tert-butyl (5S,5aS,6S,9R)-1-fluoro-5-methyl-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (159 mg). ES-API: [M+ 1]⁺= 770.3.

Step 3: ((1-Methylene tetrahydro-1H-pyrrolo[7a(5H)]-yl)methanol) (63 mg, 0.413 mmol) was dissolved in anhydrous tetrahydrofuran (5 mL), and under an ice-water bath, sodium hydride (25 mg, 0.619 mmol) was added. The reaction was proceeded for 10 minutes under the ice-water bath. A solution of tert-butyl (5S,5aS,6S,9R)-1-fluoro-5-methyl-12-(methylsulfinyl)-2-(8-((triisopropylsilyl)ethynyl)naphthalen-1 -yl)-5a,6,7,8,9,10-hexahydro-5H-4-oxa-3,10a, 11,13,14-pentaaza-6,9-methanonaphtho[1,8-ab]heptalene-14-carboxylate (159 mg, 0.206 mmol) in tetrahydrofuran (5 mL) was added dropwise to the reaction mixture and the mixture reacted at 0°C for 30 minutes. Upon completion of the reaction (monitored by LCMS), the reaction mixture was diluted with ethyl acetate (10 mL) and washed with saturated ammonium chloride solution (10 mL x2), and saturated brine (10 mL x3). The organic phase was dried over anhydrous sodium sulfate, filtered, and concentrated under reduced pressure. The crude product was purified through silica gel using automatic flash chromatography (methanol/dichloromethane = 0 - 3 %) to obtain two isomeric compounds. One isomer was arbitrarily assigned as tert-butyl (SS,SaS,6S,9R)-1-fluoro-5-methyl-12-(((R)-1-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (47 mg, yield: 27 %). ES-API: [M+ H]⁺= 859.4 (retention time: 1.787 min, analytical method: '1-POS-3MIN-2.7', mobile phase: A: water (0.05% trifluoroacetic acid) B: acetonitrile (0.05% trifluoroacetic acid), gradient: 5%-95% B in 1.6min, flow rate: 1.8 ml/min, column: SunFire C18,4.6*50mm,3.5um, column temperature: 40°C). The other isomer was arbitrarily assigned as tert-butyl (5S,5aS,6S,9R)-1-fluoro-5-methyl-12-(((S)-1-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (72 mg, yield: 41 %). ES-API: [M+ H]+= 859.4 (retention time: 1.749 min, analytical method: '1-POS-3MIN-2.7', mobile phase: A: water (0.05% trifluoroacetic acid) B: acetonitrile (0.05% trifluoroacetic acid), gradient: 5%-95% B in 1.6min, flow rate: 1.8 ml/min, column: SunFire C18,4.6*50mm,3.5um, column temperature: 40°C).

Step 4: Cesium fluoride (83 mg, 0.547 mmol) was added to a solution of tert-butyl (SS,SaS,6S,9R)-1-fluoro-5-methyl-12-(((R)-1-methylenetetrahydro-1H-pyrrolizin-7a(5H)-yl)methoxy)-2-(8-((triisopropylsilyl)ethynyl)naphthalen-1 -yl)-5a,6,7,8,9,10-hexahydro-5H-4-oxa-3,10a, 11,13,14-pentaaza-6,9-methanonaphtho[1,8-ab]heptalene-14-carboxylate (47 mg, 0.055 mmol) in N,N-dimethylformamide (2 mL). The reaction mixture was stirred at room temperature for 1 hour. Upon completion of the reaction (monitored by LCMS), the reaction mixture was diluted with ethyl acetate (15 mL), washed with water (15 mL × 2), and saturated brine (15 mL × 3). The organic phase was dried over anhydrous sodium sulfate, filtered, and concentrated, then purified through silica gel using automatic flash chromatography (methanol/dichloromethane = 0 ~ 5 %), yielding tert-butyl (5S,5aS,6S,9R)-2-(8-ethynylnaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (28 mg, yield: 73 %). ES-API: [M+ H]⁺= 703.3.

Step 5: Under an ice-water bath, 4 M hydrochloric acid-dioxane solution (3 mL) was slowly added to a solution of tert-butyl (5S,5aS,6S,9R)-2-(8-ethynylnaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (28 mg, 0.040 mmol) in acetonitrile (2 mL). The reaction mixture was stirred at 0°C for 1 hour. Upon completion of the reaction (monitored by LCMS), the reaction mixture was concentrated under reduced pressure until dry. The residue was added to saturated sodium bicarbonate solution (3 mL) until the solution pH > 7.0, extracted with dichloromethane (5 mL × 3). The combined organic phases were concentrated under reduced pressure to obtain a whitish solid: (SS,SaS,6S,9R)-2-(8-ethynylnaphthalen-1-yl)-1-fluoro-5-methyl-12-(((R)-1-methylenetetrahydro-1H-pyrrolizin-7a(SH)-yl)methoxy)-5a,6,7,8,9,10-hexahydro-5H-4-oxa-3,10a, 11,13,14-pentaaza-6,9-methanonaphtho[1,8-ab]heptalene (Z677, 9 mg, yield: 37 %). ES-API: [M+H]⁺= 603.3.

Step 6: Cesium fluoride (127 mg, 0.838 mmol) was added to a solution of tert-butyl (SS,SaS,6S,9R)-1-fluoro-5-methyl-12-(((S)-1-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (72 mg, 0.084 mmol) in N,N-dimethylformamide (2 mL). The reaction mixture was stirred at room temperature for 1 hour. Upon completion of the reaction (monitored by LCMS), the reaction mixture was diluted with ethyl acetate (15 mL), washed with water (15 mL × 2), and saturated brine (15 mL × 3). The organic phase was dried over anhydrous sodium sulfate, filtered, and concentrated. The crude product was purified through silica gel using automatic flash chromatography (methanol/dichloromethane = 0 - 5 %) to obtain tert-butyl (SS,SaS,6S,9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (43 mg, yield: 73%). ES-API: [M+ H]⁺= 703.3.

Step 7: Under an ice-water bath, 4 M hydrochloric acid-dioxane solution (3 mL) was slowly added to a solution of tert-butyl (SS,SaS,6S,9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (43 mg, 0.061 mmol) in acetonitrile (2 mL). The reaction mixture was stirred at 0°C for 1 hour. Upon completion of the reaction (monitored by LCMS), the reaction mixture was concentrated under reduced pressure until dry. The residue was added to saturated sodium bicarbonate solution (3 mL) until the solution pH > 7.0, extracted with dichloromethane (5 mL × 3). The combined organic phases were concentrated under reduced pressure to obtain a whitish solid: (SS,SaS,6S,9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z702, 29 mg, yield: 79 %). ES-API: [M+H]⁺= 603.3.

### Example 69 Synthesis of (5S,5aS,6S,9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z678)

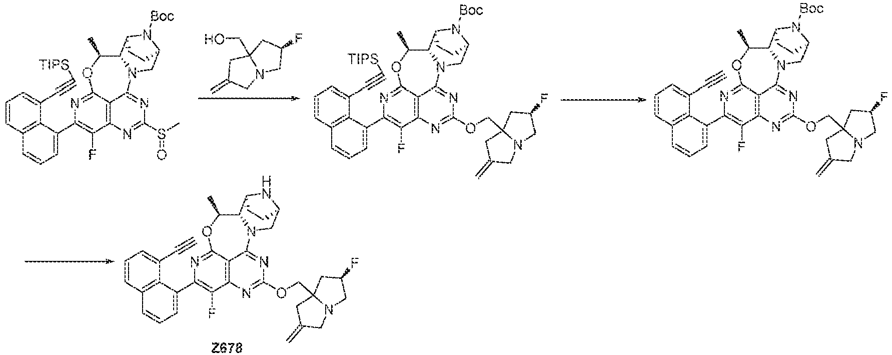

Step 1: ((2R)-2-fluoro-6-methylenetetrahydro-1H-pyrrolizin-7a(5H)-yl)methanol (62 mg, 0.362 mmol) was dissolved in anhydrous tetrahydrofuran (5 mL). Under an ice-water bath, sodium hydride (25 mg, 0.619 mmol) was added, and the reaction was proceeded for 10 minutes under an ice-water bath. Then, a solution of tert-butyl (5S,5aS,6S,9R)-1-fluoro-5-methyl-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (159 mg, 0.206 mmol) in tetrahydrofuran (5 mL) was added dropwise to the reaction mixture. The reaction was proceeded for 30 minutes at 0°C. Upon completion of the reaction (monitored by LCMS), the reaction mixture was diluted with ethyl acetate (10 mL), washed with saturated ammonium chloride solution (10 mL × 2), and saturated brine (10 mL × 3). The organic phase was dried over anhydrous sodium sulfate, filtered, and concentrated. The crude product was purified through silica gel using automatic flash chromatography (methanol/dichloromethane = 0 - 3 %) to obtain tert-butyl (SS,Sa5,6S,9R)-1-fluoro-12-(((2R)-2-fluoro-6-methylenetetrahydro-1H-pyrrolizin-7a(5H)-yl)methoxy)-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptalene-14-carboxylate (88 mg, yield: 49%). ES-API: [M+ H]⁺= 877.3.

Step 2: Cesium fluoride (152 mg, 1.003 mmol) was added to a solution of tert-butyl (5S, 5aS, 6S, 9R)-1-fluoro-12-((2R)-2-fluoro-6-methylene tetrahydro-1H-pyrrolo[7a(5H)]-yl)methoxy)-5-methyl-2-(8-(triisopropylsilyl)ethynyl)naphth-1-yl)-5a,6,7,8,9,10-hexahydro-5H-4-oxy-3,10a,11,13,14-pentaaza-6,9-methanonaphtho[1,8-ab]heptalene-14-carboxylate (88 mg, 0.100 mmol) in N,N-dimethylformamide (3 mL). The reaction mixture was stirred at room temperature for 1 hour. Upon completion of the reaction (monitored by LCMS), the reaction mixture was diluted with ethyl acetate (15 mL), washed with water (15 mL × 2), and saturated brine (15 mL × 3). The organic phase was dried over anhydrous sodium sulfate, filtered, and concentrated. The crude product was purified on a thin-layer chromatography plate (dichloromethane/methanol = 15:1) to obtain tert-butyl (5S,5aS,6S,9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (68 mg, yield: 94%). ES-API: [M+ H]⁺= 721.2.

Step 3: Under an ice-water bath, 4 M hydrochloric acid-dioxane solution (3 mL) was slowly added to a solution of tert-butyl (5S,5aS,6S,9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (68 mg, 0.094 mmol) in acetonitrile (3 mL). The reaction mixture was stirred at 0°C for 1 hour. Upon completion of the reaction (monitored by LCMS), the reaction mixture was concentrated under reduced pressure until dry. The residue was adjusted by adding saturated sodium bicarbonate solution (3 mL) until the solution pH> 7.0, extracted with dichloromethane (5 mL × 3). The combined organic phases were concentrated under reduced pressure to obtain a whitish solid: (5S,5aS,6S,9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z678, 44 mg, yield: 75 %). ES-API: [M+H]⁺= 621.3. ¹H NMR (400 MHz, CD₃OD) δ 8.08-8.01 (m, 2 H), 7.77-7.48 (m, 4 H), 5.44-5.27 (m, 2 H), 5.02 (s, 2 H), 4.59-4.54 (m, 1 H), 4.43-4.38 (m, 1 H), 4.30 (d, J= 13.0 Hz, 1 H), 4.15 (d, J= 10.5 Hz, 1 H), 3.82-3.73 (m, 3 H), 3.70-3.36 (m, 3 H), 3.28-3.15 (m, 2 H), 3.01-2.85 (m, 2 H), 2.55-2.46 (m, 2 H), 2.23-2.05 (m, 2 H), 1.98-1.85 (m, 3 H), 1.58 (t, J= 8.5 Hz, 3 H).

### Example 70 Synthesis of 2-(8-ethynyl-7-fluornnaphthalen-1-yl)-1-fluoro-12-(((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptalene (Z679)

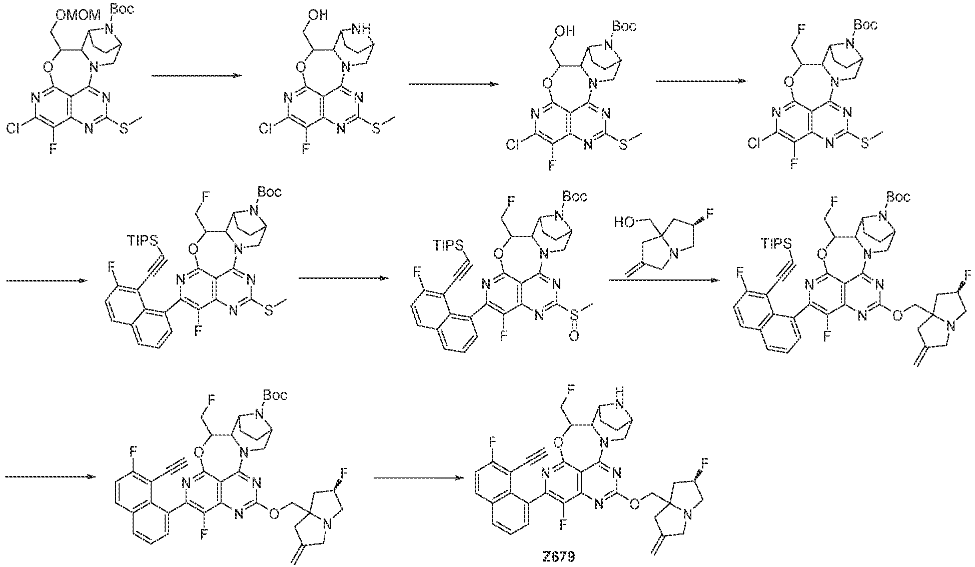

Step 1: Under an ice-water bath, tert-butyl 2-chloro-1-fluoro-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (148.5 mg, 0.275 mmol) was dissolved in hydrochloric acid methanol solution (10 mL, 4 M). The reaction mixture was stirred at 0°C for 3 hours. Upon completion of the reaction (monitored by LCMS), the reaction mixture was concentrated under reduced pressure to obtain the crude product: (2-chloro-1-fluoro-12-(methylthio)-5a,6,7,8,9,10-hexahydro-5H-4-oxa-3,10a,11,13,14-pentaaza-6,9-methanonaphtho[1,8-ab]heptalen-5-yl)methanol (104.5 mg). ES-API: [M+ H]⁺= 398.0.

Step 2: Under an ice-water bath, (2-chloro-1-fluoro-12-(methylthio)-5a,6,7,8,9,10-hexahydro-5H-4-oxa-3,10a,11,1 3,14-pentaaza-6,9-methanonaphtho[1,8-ab]heptalen-5-yl)methanol (100 mg, 0.250 mmol) was dissolved in dichloromethane (20 mL). Di-tert-butyl dicarbonate (165 mg, 0.755 mmol) and N,N-diisopropylethylamine (195 mg, 1.510 mmol) were added to the reaction mixture, which was then stirred at 0°C for 3 hours. Upon completion of the reaction (monitored by LCMS), the reaction mixture was concentrated. The crude product was purified through silica gel using automatic flash chromatography (ethyl acetate:petroleum ether = 0-20%) to obtain tert-butyl 2-chloro-1-fluoro-5-(hydroxymethyl)-12-(methylthio)-5a,6,7,8,9,10-hexahydro-5H-4-oxa-3,10a,11,13,14-pentaaza-6,9-methanonaphtho[l,8-ab]heptalene-14-carboxylate (125 mg, yield: 99%). ES-API: [M+ H]⁺= 498.1.

Step 3: Under an ice-water bath, tert-butyl 2-chloro-1-fluoro-5-(hydroxymethyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (125 mg, 0.250 mmol) was dissolved in dichloromethane (15 mL). Bis(2-diethylamino)trifluorosulfonium (160 mg, 1.005 mmol) was added dropwise to the reaction mixture, which was then stirred at 0°C for 3 hours. Upon completion of the reaction (monitored by LCMS), the reaction mixture was diluted with dichloromethane (20 mL), then washed with saturated sodium bicarbonate solution (20 mL × 2) and saturated brine (20 mL × 2). The organic phase was dried over anhydrous sodium sulfate, filtered, and concentrated to obtain the crude product: tert-butyl 2-chloro-1-fluoro-5-(fluoromethyl)-12-(methylthio)-5a,6,7,8,9,10-hexahydro-5H-4-oxa-3,10a,11,13,14-pentaaza-6,9-methanonaphtho[l,8-ab]heptalene-14-carboxylate (120 mg). ES-API: [M+H]⁺= 500.0.

Step 4: Under nitrogen protection, tert-butyl 2-chloro-1-fluoro-5-(fluoromethyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.200 mmol), {[2-fluoro-8-(4,4,5,5-tetramethyl-1,3,2-dioxaborolane-2-yl)-1-naphthyl]ethynyl}[3-propyl]silane (226 mg, 0.500 mmol), potassium phosphate (127 mg, 0.600 mmol) and methanesulfonic acid [butylbis(1-adamantyl)phosphine](2-amino-1,1'-biphenyl)palladium(II) (30 mg, 0.040 mmol) were dissolved in tetrahydrofuran (12 mL) and water (2.5 mL). The reaction mixture was stirred in a microwave generator at 80°C for 0.5 hours. Upon completion of the reaction (monitored by LCMS), the reaction mixture was concentrated. The crude product was purified through silica gel using automatic flash chromatography (petroleum ether/ethyl acetate = 0-20%) to obtain tert-butyl 1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (60 mg, yield: 38%). ES-API: [M+ H]⁺= 790.2.

Step 5: Tert-butyl 1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (60 mg, 0.076 mmol) was dissolved in anhydrous dichloromethane (10 mL). M-chloroperbenzoic acid (20 mg, 0.114 mmol) was added to the solution, and the reaction mixture was stirred at room temperature for 0.5 hours. Upon completion of the reaction (monitored by LCMS), dichloromethane (15 mL) was added to dilute the reaction mixture, which was then quenched with saturated sodium sulfite solution (15 mL), then washed with sodium bicarbonate solution (15 mL) and saturated brine (15 mL). The organic phase was dried over anhydrous sodium sulfate, filtered, and concentrated to obtain the crude product: tert-butyl 1-fluoro-2-(7-fluoro-8-((triisopropylsilyl)ethynyl)naphthalen-1-yl)-5-(fluoromethyl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (61 mg). ES-API: [M+H]⁺= 822.1.

Step 6: Sodium hydride (9 mg, 0.228 mmol) was added to a solution of ((2R)-2-fluoro-6-methyltetrahydro-1H-pyrrolo[2,1-b][1,3]oxazine-7a(5H)-yl)methanol (26 mg, 0.152 mmol) in anhydrous tetrahydrofuran (5 mL) under an ice-water bath. The reaction was proceeded for 10 minutes under the ice-water bath. Then, a solution of tert-butyl 1-fluoro-2-(7-fluoro-8-((triisopropylsilyl)ethynyl)naphthalen-1-yl)-5-(fluoromethyl)-12-(methylsulfinyl)-5a,6,7,8,9,10-hexahydro-5H-4-oxa-3,10a, 11,13,14-pentaaza-6,9-methanonaphtho[1,8-ab]heptalene-14-carboxylate (61 mg, 0.076 mmol) in tetrahydrofuran (5 mL) was added dropwise to the reaction mixture, which reacted for 30 minutes at 0°C. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure. The crude product was purified through silica gel using automatic flash chromatography (methanol/dichloromethane = 0 - 3%) to obtain tert-butyl 1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-(fluoromethyl)-5a,6,7,8,9,10-hexahydro-5H-4-oxa-3,10a,11,13,14-pentaaza-6,9-methanonaphtho[1,8-ab]heptalene-14-carboxylate (69 mg, yield: 99%). ES-API: [M+ H]⁺= 913.3.

Step 7: Cesium fluoride (115 mg, 0.756 mmol) was added to a solution of tert-butyl 1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-(fluoromethyl)-5a,6,7,8,9,10-hexahydro-5H-4-oxa-3,10a,11,13,14-pentaaza-6,9-methanonaphtho[l,8-ab]heptalene-14-carboxylate (69 mg, 0.076 mmol) in N,N-dimethylformamide (5 mL). The reaction mixture was stirred at room temperature for 1 hour. Upon completion of the reaction (monitored by LCMS), the reaction mixture was diluted with ethyl acetate (15 mL), washed with water (15 mL × 2) and saturated brine (15 mL × 3), dried over anhydrous sodium sulfate, filtered, and concentrated under reduced pressure. The crude product was purified on thin-layer chromatography plates (dichloromethane/methanol = 50: 1) to obtain tert-butyl 2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptalene-14-carboxylate (60 mg). ES-API: [M+ H]⁺= 757.2.

Step 8: A solution of 4 M hydrochloric acid-dioxane (5 mL) was slowly added to a solution of tert-butyl 2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptalene-14-carboxylate (60 mg, 0.079 mmol) in acetonitrile (2 mL) under an ice-water bath. The reaction mixture was stirred at 0°C for 1 hour. Upon completion of the reaction (monitored by LCMS), the reaction mixture was concentrated under reduced pressure to dryness. The residue was adjusted by adding saturated sodium bicarbonate solution (3 mL) until the solution pH> 7.0, extracted with dichloromethane (5 mL × 3), and the combined organic phases were concentrated under reduced pressure to obtain an off-white solid: 2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptalene (Z679, 29 mg, yield: 56 %). ES-API: [M+H]⁺= 657.2.

### Example 71 Synthesis of 5-cyclopropyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)-6-methylpyridin-2-amine (Z680)

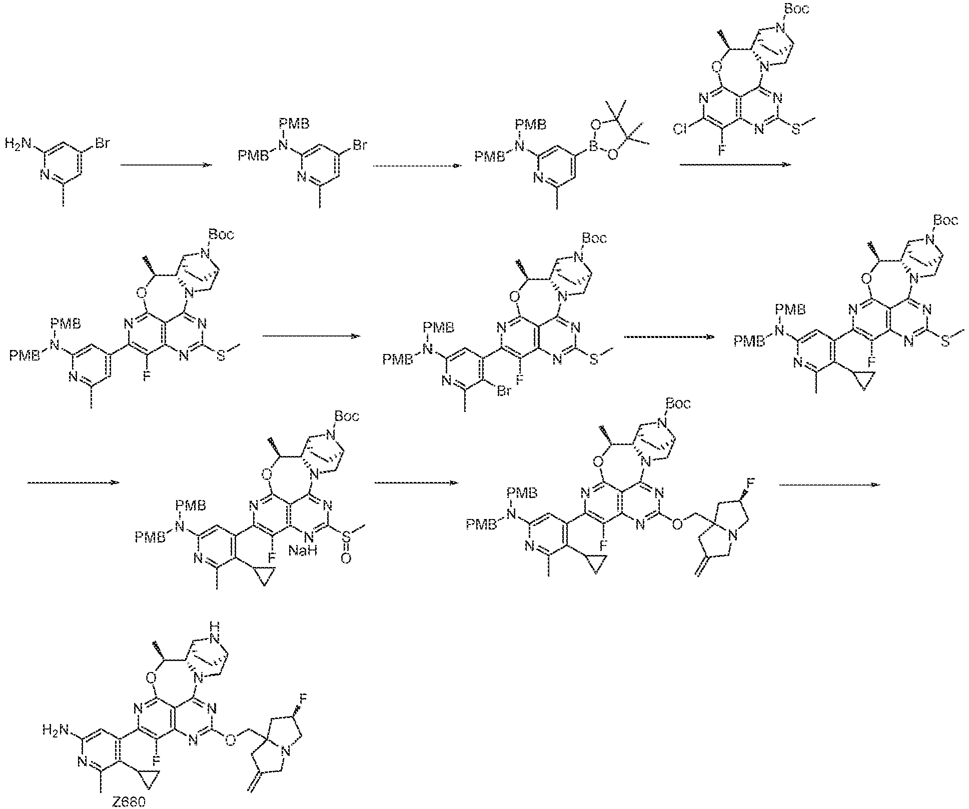

Step 1: At 0°C, sodium hydride (256.0 mg, 6.415 mmol) was added to a solution of 4-bromo-6-methylpyridine-2-amine (1000 mg, 5.346 mmol) in NN-dimethylformamide (10 mL). After the mixture stirred at this temperature for 0.5 hours, 4-methoxybenzyl chloride (921 mg, 5.881 mmol) was added. The reaction was proceeded overnight at room temperature. Upon completion of the reaction, the mixture was extracted with ethyl acetate (200 mL × 2) and saturated brine (80 mL). The ethyl acetate phases were combined, dried over anhydrous sodium sulfate, and filtered. The filtrate was concentrated under reduced pressure. The crude product was purified by silica gel column chromatography (0-50% ethyl acetate/petroleum ether) to obtain 4-bromo-N,N-bis(4-methoxybenzyl)-6-methylpyridin-2-amine (1.56 g, yield 68.28%). ES-API: [M+H⁺=427.1.

Step 2: 4-bromo-N,N-bis(4-methoxybenzyl)-6-methylpyridin-2-amine (1.20 g, 2.808 mmol), pinacolborane (1.43 g, .616 mmol), potassium acetate (0.93 g, 11.232 mmol), and [1,1'-bis(diphenylphosphino)ferrocene]dichloropalladium(II) (0.31 g, 0.421 mmol) were added to N,N-dimethylformamide (20 mL). After purged four times with nitrogen, the mixture reacted at 120°C for 1.5 hours. Upon completion of the reaction, the mixture was extracted with ethyl acetate (200 mL × 2) and saturated brine (80 mL). The ethyl acetate phases were combined, dried over anhydrous sodium sulfate, and filtered. The filtrate was concentrated under reduced pressure. The crude product was purified by silica gel column chromatography (0-70% ethyl acetate/petroleum ether) to obtain N,N-bis(4-methoxybenzyl)-6-methyl-4-(4,4,5,5 tetramethyl-1,3,2-dioxaborolan-2-yl)pyridin-2-amine (1.4 g, crude product). ES-API: [M+H]⁺=475.3.

Step 3: To a mixture of tert-butyl (5S,5aS,6S,9R)-2-chloro-1-fhioro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (500 mg, 1.037 mmol) and N,N-bis(4-methoxybenzyl)-6-methyl-4-(4,4,5,5-tetramethyl-1,3,2-dioxaborolan-2-yl)pyridin-2-amine (1.40 g, 2.95 mmol) in dioxane (30 mL) and water (3 mL), potassium phosphate (2.50 g, 11.80 mmol), tricyclohexylphosphine (827 mg, 2.95 mmol), and tris(dibenzylideneacetone)dipalladium(0) (405 mg, 0.443 mmol) were added. The mixture was bubbled with nitrogen and reacted in an oil bath at 100°C for 5 hours. Upon completion of the reaction, the mixture was extracted with ethyl acetate (30 mL × 2) and saturated brine (30 mL). The ethyl acetate phases were combined, dried over anhydrous sodium sulfate, and filtered. The filtrate was concentrated under reduced pressure. The crude product was purified by silica gel column chromatography (0-4% methanol/dichloromethane) to obtain tert-butyl (5S,5aS,6S,9R)-2-(2-(bis(4-methoxybenzyl)amino)-6-methylpyridin-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (860 mg, crude product), a yellow solid. ES-API: [M+H]⁺=794.2.

Step 4: At 0°C, to a dichloromethane solution (20 mL), tert-butyl (5S,5aS,6S,9R)-2-(2-(bis(4-methoxybenzyl)amino)-6-methylpyridin-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (800 mg, 1.008 mmol) was added, followed by the addition of N-bromosuccinimide (233.32 mg, 1.311 mmol). The reaction was proceeded for 0.5 hours at this temperature. Upon completion of the reaction, the mixture was extracted with ethyl acetate (100 mL × 2) and saturated brine (100 mL). The ethyl acetate phases were combined, dried over anhydrous sodium sulfate, and filtered. The filtrate was concentrated under reduced pressure. The crude product was purified by silica gel column chromatography (0-70% ethyl acetate/petroleum ether) to obtain tert-butyl (SS,SaS,6S,9R)-2-(6-(bis(4-methoxybenzyl)amino)-3-bromo-2-methylpyridin-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (840 mg, yield 95.57%). ES-API: [M+H]⁺=872/874.

Step 5: To a mixture of tert-butyl (5S,5aS,6S,9R)-2-(6-(bis(4-methoxybenzyl)amino)-3-bromo-2-methylpyridin-4-yl)-1-fluoro-5-methyl-12-(methylthio)-Sa,6,7,8,9,10-hexahydro-SH-4-oxa-3,10a,11,13,14-pentaaza-6,9-methanonaphtho[1,8-ab]heptalene-14-carboxylate (860 mg, 0.985 mmol) and cyclopropylboronic acid (516 mg, 6.007 mmol) in toluene (50 mL) and water (5 mL), potassium phosphate (860 mg, 4.0523 mmol), 2-dicyclohexylphosphino-2',6'-dimethoxybiphenyl (120 mg, 0.293 mmol), and palladium acetate (34.4 mg, 6.007 mmol) were added. The mixture was bubbled with nitrogen and reacted in an oil bath at 105°C for 2 hours. Upon completion of the reaction, the mixture was extracted with ethyl acetate (200 mL × 2) and saturated brine (100 mL × 4). The ethyl acetate phases were combined, dried over anhydrous sodium sulfate, and filtered. The filtrate was concentrated under reduced pressure. The crude product was purified by silica gel column chromatography (0-70% ethyl acetate/petroleum ether) to obtain tert-butyl (5S,5aS,6S,9R)-2-(6-(bis(4-methoxybenzyl)amino)-3-cyclopropyl-2-methylpyridin-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (830 mg, crude product), a yellow solid. ES-API: [M+H]⁺=834.3.

Step 6: At 0°C, m-chloroperoxybenzoic acid (188.3 mg, 1.09 mmol) was added to a dichloromethane solution (30.0 mL) of tert-butyl (SS,SaS,6S,9R)-2-(6-(bis(4-methoxybenzyl)amino)-3-cyclopropyl-2-methylpyridin-4-yl)-1-fluoro-5-methyl-12-(methylthio)-5a,6,7,8,9,10-hexahydro-5H-4-oxa-3,10a,11,13,14-pentaaza-6,9-methanonaphtho[l,8-ab]heptalene-14-carboxylate (700 mg, 0.839 mmol). The mixture was stirred for 1 hour in an ice-water bath. The reaction mixture was quenched with saturated sodium sulfite and extracted with dichloromethane (100 mL × 2). The organic layer was dried over anhydrous sodium sulfate and concentrated to obtain a crude product, which was then purified by silica gel column chromatography (0-10% methanol/dichloromethane) to obtain tert-butyl (SS,SaS,6S,9R)-2-(6-(bis(4-methoxybenzyl)amino)-3-cyclopropyl-2-methylpyridin-4-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (520 mg, yield 72.89%), a white solid. ES-API:[M+H]⁺=850.2.

Step 7: At 0°C, sodium hydride (56.48 mg, 1.412 mmol) was added to a tetrahydrofuran solution (10.0 mL) containing ((2R)-2-fluoro-6-methylene tetrahydro-1H-pyrrolo[3,4-b]pyrazine-7a(5H)-yl)methanol (120 mg, 0.701 mmol), and the mixture stirred for 10 minutes at this temperature. Subsequently, a tetrahydrofuran solution (5 mL) of tert-butyl (5S,5aS,6S,9R)-2-(6-(bis(4-methoxybenzyl)amino)-3-cyclopropyl-2-methylpyridin-4-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (300 mg, 0.353 mmol) was added. The reaction mixture was stirred for 10 minutes at 0°C and then for 0.5 hours at room temperature. Upon completion of the reaction, the mixture was quenched with saturated ammonium chloride (20 mL) solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-(6-(bis(4-methoxybenzyl)amino)-3-cyclopropyl-2-methylpyridin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (175 mg, yield 51.8%), a yellow solid. ES-API: [M+H]⁺=957.3.

Step 8: Under an ice-water bath, tert-butyl (5S,5aS,6S,9R)-2-(6-(bis(4-methoxybenzyl)amino)-3-cyclopropyl-2-methylpyridin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (175 mg, 0.183 mmol) was slowly added to trifluoroacetic acid (10.0 mL) . The reaction mixture was stirred at 50°C for 1.0 hour. Upon completion of the reaction (monitored by LCMS), the reaction solution was concentrated under reduced pressure to dryness. The residue was purified by preparative HPLC (ammonium bicarbonate method 2) (chromatographic conditions: 15ml-35-85-13min) to obtain a pale yellow solid, 5-cyclopropyl-4-((SS,SaS,6S,9R)-1-fluoro-12-(((2R)-2-fluoro-6-methylenetetrahydro-1H-pyrrolizin-7a(5H)-yl)methoxy)-5-methyl-5a,6,7,8,9, 10-hexahydro-5H-4-oxa-3, 10a, 11, 13,14-pentaaza-6,9-methanonaphtho[1,8-ab]heptalen-2-yl)-6-methylpyridin-2-amine(Z680, 15 mg, yield: 13%). ES-API: [M+H]⁺= 617.2.

### Example 72 Synthesis of (5S,5aS,6S,9R)-2-(8-ethynyl-7-fluoronaphthalen-1-yl)-1-fluoro-12-((6-methosy-1-methylenetetrahydrn-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z681)

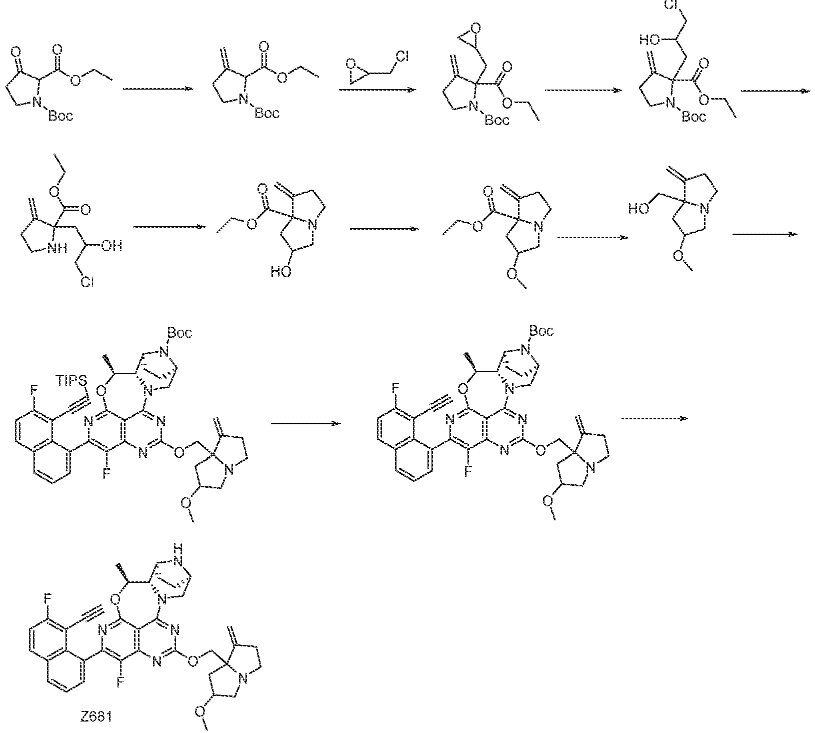

Step 1: Methyltriphenylphosphonium bromide (20.82 g, 58.30 mmol) was added to tetrahydrofuran (400 mL) and the mixture was cooled to 0°C. Under a nitrogen atmosphere, at 0°C, a tetrahydrofuran solution of potassium tert-butoxide (58.30 mL, 58.30 mmol, 1M) was added to the mixture. After the addition was complete, the reaction mixture was warmed to room temperature and stirred for 1 hour. 1-(tert-butyl) 2-ethyl 3-oxopyrrolidine-1,2-dicarboxylate (6.0 g, 25.64 mmol) was dissolved in tetrahydrofuran (20.0 mL), and was slowly added to the solution above under a nitrogen atmosphere. After the dropwise addition was complete, the mixture was further stirred at room temperature for 3 hours. Saturated ammonium chloride (60 mL) was added to the reaction mixture, which was then extracted with ethyl acetate (200 mL × 3). The organic phase was washed with saturated brine (400 mL), dried over anhydrous sodium sulfate, filtered, and concentrated. The crude product was purified by flash silica gel column chromatography (ethyl acetate/petroleum ether: 0%-8%) to obtain 1-(tert-butyl) 2-ethyl 3-methylenepyrrolidine-1,2-dicarboxylate (4.0 g, yield 61.6%), a colorless and transparent liquid. ES-API: [M-Boc+H]⁺=200.1.

Step 2: At -70°C, bis(trimethylsilyl)aminolithium (54.8 mL, 1M, 54.8 mmol) was added to a dry tetrahydrofuran solution (200.0 mL) of 1-(tert-butyl) 2-ethyl 3-methylenepyrrolidine-1,2-dicarboxylate (7.0 g, 27.418 mmol), and the mixture was stirred for 1 hour at this temperature. 1 hour later, 2-(chloromethyl)oxirane (7.61 g, 82.25 mmol) was added to the reaction solution, and the mixture was stirred overnight at room temperature. Upon completion of the reaction, saturated ammonium chloride (30 mL) was added to the solution, followed by extraction with ethyl acetate (200 mL × 4). The organic phases were washed with saturated brine (200 mL × 3), dried over anhydrous sodium sulfate, concentrated, and the crude product was purified by flash silica gel column chromatography [dichloromethane:methanol=100:0~90:10,(v/v)] to obtain the target compound 1-(tert-butyl) 2-ethyl 3-methylene-2-(oxiran-2-ylmethyl)pyrrolidine-1,2-dicarboxylate (6.0 g, yield 70.28%). ES-API: [M-56+H]⁺=256.1.

Step 3: At room temperature, acetic acid (6.0 mL, 104.813 mmol) and lithium chloride (5.0 g, 117.952 mmol) were added to a dry tetrahydrofuran solution (30.0 mL) of 1-(tert-butyl) 2-ethyl 3-methylene-2-(oxiran-2-ylmethyl)pyrrolidine-1,2-dicarboxylate (2.0 g, 6.423 mmol), and the mixture was stirred for 12 hours at this temperature. Upon completion of the reaction, saturated ammonium chloride (100 mL) was added to the solution, followed by extraction with ethyl acetate (200 mL × 2). The organic phases were combined, dried over anhydrous sodium sulfate, filtered, and the solvent was evaporated under reduced pressure to obtain the crude product 1-(tert-butyl) 2-ethyl 2-(3-chloro-2-hydroxypropyl)-3-methylenepyrrolidine-1,2-dicarboxylate (2.04 g, yield 91%). ES-API: [M-56+H]⁺=256.1.

Step 4: To a dichloromethane solution (20.0 mL) of 1-(tert-butyl) 2-ethyl 2-(3-chloro-2-hydroxypropyl)-3-methylenepyrrolidine-1,2-dicarboxylate (2.0 g, 6.423 mmol), a hydrochloric acid dioxane solution (16.0 mL, 4M, 64.00 mmol) was added, and the mixture was stirred at room temperature for 2 hours. Upon completion of the reaction, the solvent was evaporated under reduced pressure to obtain the crude target compound ethyl 2-(3-chloro-2-hydroxypropyl)-3-methylenepyrrolidine-2-carboxylate (1.95 g, crude product). ES-API: [M+H]⁺=248.1.

Step 5: To a solution of ethyl 2-(3-chloro-2-hydroxypropyl)-3-methylenepyrrolidine-2-carboxylate (1.45 g, 5.865 mmol) in acetonitrile (30.0 mL), sodium bicarbonate (4.87 g, 58.65 mmol) was added, and the reaction was proceeded at 70°C for 0.5 hours. Upon completion of the reaction, the mixture was cooled to room temperature, filtered, and the filtrate was concentrated under reduced pressure. The crude product was purified by flash silica gel column chromatography (methanol/dichloromethane: 0%-10%) to obtain ethyl 6-hydroxy-1-methylenetetrahydro-1H-pyrrolizine-7a(5H)-carboxylate (780 mg, yield 62.95%). ES-API: [M+H]⁺=212.1.

Step 6: At 0°C, sodium hydride (147 mg, 3.692 mmol) was added to a solution of ethyl 6-hydroxy-1-methylenetetrahydro-1H-pyrrolizine-7a(5H)-carboxylate (390 mg, 1.846 mmol) in N,N-dimethylformamide (5.0 mL), and the mixture was stirred for 10 minutes at this temperature, then warmed to room temperature and stirred for 0.5 hours. Upon completion of the reaction, the mixture was cooled to 0°C, with iodomethane (340 mg, 2.40 mmol) added, and reacted for 0.5 hours. Upon completion of the reaction, the reaction mixture was diluted with ethyl acetate (60 mL × 2), washed with water (30 mL × 2) and saturated brine (50 mL × 3), dried over anhydrous sodium sulfate, filtered, and concentrated. The crude product was purified by flash silica gel column chromatography (methanol/dichloromethane: 0%-8%) to obtain ethyl 6-methoxy-1-methylenetetrahydro-1H-pyrrolizine-7a(5H)-carboxylate (120 mg, yield 28.85%). ES-API: [M+H]⁺= 226.1.

Step 7: Ethyl 6-methoxy-1-methylenetetrahydro-1H-pyrrolizne-7a(5H)-carboxylate (120 mg, 0.533 mmol) was dissolved in tetrahydrofuran (10 mL) and the mixture was cooled to 0°C. Under a nitrogen atmosphere, lithium aluminium hydride solution (1.0 mL, 1.0 mmol, 1M in tetrahydrofuran) was added. The reaction mixture was stirred at 0°C for 1 hour. Water (0.03 mL) was added to quench the reaction, followed by the addition of 15% sodium hydroxide solution (0.03 mL) and water (0.09 mL). Tetrahydrofuran (10 mL) was added, and the mixture was filtered. The filtrate was concentrated to obtain (6-methoxy-1-methylenetetrahydro-1H-pyrrolizin-7a(5H)-yl)methanol (85 mg, yield 87%), a colorless and transparent liquid. ES-API: [M +H]⁺=184.1.

Step 8: (6-methoxy-1-methylenetetrahydro-1H-pyrrolizin-7a(5H)-yl)methanol (85 mg, 0.464 mmol) was dissolved in anhydrous tetrahydrofuran (5 mL). Under an ice water bath, sodium hydride (37.25 mg, 0.931 mmol) was added, and the reaction was proceeded for 30 minutes under the ice water bath. Then a solution of tert-butyl (5S,5aS,6S,9R)-1-fluoro-2-(7-fluoro-8-(triisopropylsilyl)ethynyl)naphthalen-1-yl)-5-methyl-12-(methylsulfonyl)-5a,6,7,8,9,10-hexahydro-5H-4-oxygen-3,10a,11,13,14-pentazacyclononadec[1,8-ab]heptene-14-carboxylate (180 mg, 0.233 mmol) in tetrahydrofuran (2 mL) was added dropwise. The reaction was proceeded at 40°C for 30 minutes. Upon completion of the reaction (monitored by LCMS), the reaction mixture was diluted with ethyl acetate (50 mL), washed with saturated ammonium chloride solution (15 mL × 2) and saturated brine (50 mL × 3), dried over anhydrous sodium sulfate, filtered, and purified by automatic flash chromatography (dichloromethane: methanol = 15:1) through silica gel to obtain tert-butyl (5S,5aS,6S,9R)-1-fluoro-2-(7-fluoro-8-((triisopropylsilyl)ethynyl)naphthalen-1-yl)-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (120 mg, yield 56.8%). ES-API: [M+H]⁺= 907.1.

Step 9: Cesium fluoride (100.0 mg, 0.658 mmol) was added to a solution of tert-butyl (SS,SaS,6S,9R)-1-fluoro-2-(7-fluoro-8-((triisopropylsilyl)ethynyl)naphthalen-1-yl)-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (120 mg, 0.132 mmol) in N,N-dimethylformamide (5.0 mL). The reaction mixture was stirred at room temperature for 2 hours. Upon completion of the reaction (monitored by LCMS), the mixture was diluted with ethyl acetate (60 mL), washed with water (30 mL × 2), and saturated brine (50 mL × 3), dried over anhydrous sodium sulfate, filtered, and concentrated to obtain tert-butyl (5 S,5aS,6S,9R)-2-(8-ethynyl-7-fluoronaphthalen-1 -yl)-1 -fluoro- 12-((6-methoxy-1 - methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (80.0 mg, yield 80.5%). ES-API: [M+H]⁺= 751.1.

Step 10: Under an ice water bath, a 4 M hydrochloric acid-dioxane solution (1.0 mL) was slowly added to a solution of tert-butyl (SS,SaS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (80.0 mg, 0.107 mmol) in acetonitrile (5.0 mL). The reaction mixture was stirred at 0°C for 0.5 hours. Upon completion of the reaction (monitored by LCMS), the mixture was concentrated under reduced pressure, and the residue was purified by preparative HPLC (ammonium bicarbonate method 2) (chromatographic conditions: 15ml-35-85-13min) to obtain a pale yellow solid, (5S,5aS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z681, 60.0mg, yield: 86.54%). ES-API: [M+H]⁺= 651.2.

### Example 73 Synthesis of (5S,5aS,6S,9R)-2-(3-chloro-2-(trifluoromethyl)phenyl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z682)

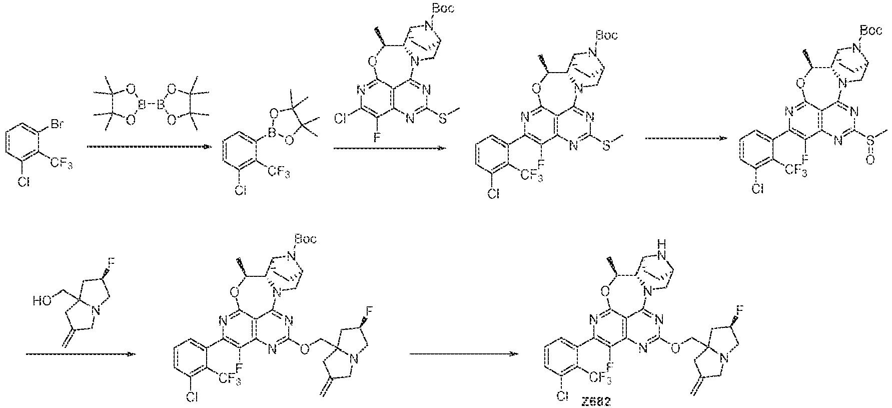

Step 1: 2-Chloro-6-bromotrifluoromethylbenzene (778.35 mg, 3 mmol), allyl boronic acid pinacol ester (1523.64 mg, 6.000 mmol), potassium acetate (735.00 mg, 7.500 mmol), and 1,1'-bis(diphenylphosphino)ferrocene dichloropalladium(II) (219.51 mg, 0.300 mmol) were dissolved in 1,4-dioxane (5 mL) and stirred at 110°C for 2 hours. The mixture was then concentrated under reduced pressure and purified by column chromatography (petroleum ether/ethyl acetate = 90/10) to obtain 2-(3-chloro-2-(trifluoromethyl)phenyl)-4,4,5,5-tetramethyl-1,3,2-dioxaborolane (450 mg, 1.468 mmol, yield 48.94%).

Step 2: 2-(3-Chloro-2-(trifluoromethyl)phenyl)-4,4,5,5-tetramethyl-1,3,2-dioxaborolane (127.19 mg, 0.415 mmol) and tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.207 mmol) were dissolved in tetrahydrofuran (2 mL) and water (0.5 mL). Methanesulfonic acid [butylbis(1-adamantyl)phosphine](2-amino-1,1'-biphenyl)palladium(II) (15.10 mg, 0.021 mmol) and potassium phosphate (132.12 mg, 0.622 mmol) were added. After the addition was complete, the reaction mixture was stirred at 65°C for 5 hours. Upon completion of the reaction, water (15 mL) was added, and the mixture was extracted with ethyl acetate (15 mL × 3). The organic phase was washed with saturated brine (30 mL), dried over anhydrous sodium sulfate, filtered, concentrated, and purified by flash silica gel column chromatography (ethyl acetate/petroleum ether: 0-30%) to obtain tert-butyl (5S,5aS,6S,9R)-2-(3-chloro-2-(trifluoromethyl)phenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (45 mg, 0.072 mmol, yield 34.64%), a white solid. ES-API: [M +H]⁺= 626.1.

Step 3: Tert-butyl (5S,5aS,6S,9R)-2-(3-chloro-2-(trifluoromethyl)phenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (42 mg, 0.067 mmol) was dissolved in dichloromethane (5 mL). At room temperature, m-chloroperoxybenzoic acid (15.05 mg, 0.087 mmol) was added to the solution. After the addition was complete, the reaction mixture was stirred at room temperature for 1 hour. Sulfuric acid sodium thiosulfate solution (5 mL) was then added, and the mixture was extracted with dichloromethane (10 mL × 3). The combined organic phases were washed first with saturated bicarbonate solution (20 mL) and then with saturated sodium chloride (20 mL). The organic phase was dried over anhydrous sodium sulfate, filtered, and concentrated to obtain tert-butyl (5 S,5aS,6S,9R)-2-(3-chloro-2-(trifluoromethyl)phenyl)-1 -fhioro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (40 mg, 0.062 mmol, yield 92.87%). ES-API: [M +H]⁺= 642.0.

Step 4: Tert-butyl (5S,5aS,6S,9R)-2-(3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (40 mg, 0.062 mmol, yield 92.87%) was dissolved in tetrahydrofuran (3 mL) and cooled to 0°C. Under a nitrogen atmosphere, sodium hydride (10.47 mg, 0.262 mmol) was added and the solution was stirred for 30 minutes. Then, ((2R)-2-fluoro-6-methylenetetrahydro-1H-pyrrolizin-7a(5H)-yl)methanol (22.40 mg, 0.131 mmol) was slowly added to the solution, which was then stirred at 0°C for 2 hours. Water (5 mL) was added to quench the reaction and the mixture was extracted with ethyl acetate (20 mL × 3). The organic phase was washed with saturated brine (30 mL × 3), dried over anhydrous sodium sulfate, filtered, and concentrated to obtain a crude product. The crude product was purified by flash silica gel column chromatography (ethyl acetate/petroleum ether: 0-70%) to obtain tert-butyl (5S,5aS,6S,9R)-2-(3-chloro-2-(trifluoromethyl)phenyl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.033 mmol, yield 51.01%). ES-API: [M+H]⁺=749.0.

Step 5: Tert-butyl (5S,5aS,6S,9R)-2-(3-chloro-2-(trifluoromethyl)phenyl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate was dissolved in dichloromethane and trifluoroacetic acid was added. The reaction mixture was stirred at room temperature for 2 hours. Upon completion of the reaction, the crude product was purified by preparative HPLC (ammonium bicarbonate method 1) to obtain (5 S,5aS,6S,9R)-2-(3-chloro-2-(trifluoromethyl)phenyl)-1 -fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z682, 10 mg, 0.015 mmol, yield: 76.92%). ES-API: [M+H]⁺=649.2.

### Example 74 Synthesis of (7a-((((5S,5aS,6S,9R)-2-(8-ethynyl-7-fluoronaphthalen-1-yl)-1-fluoro-5-methyl-Sa,6,7,8,9,10-hexahydro-5H-4-oxa-3,10a,11,13,14-pentaaza-6,9-methanonaphtho[1,8-ab]heptalen-12-yl)oxy)methyl)-6-methylenehexahydro-1H-pyrrolizin-1-yl)methanol (Z683)

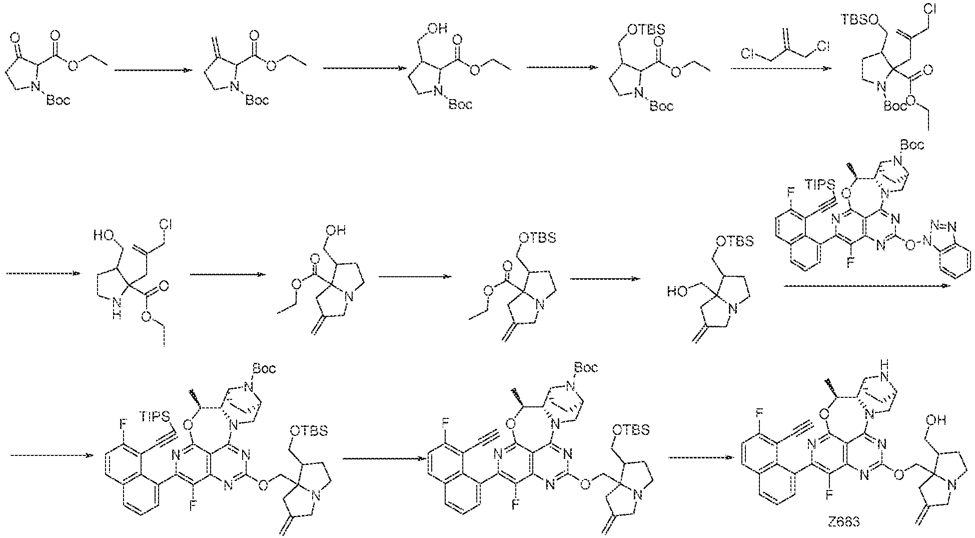

Step 1: Methyltriphenylphosphonium bromide (18.34 g, 51.385 mmol) was dissolved in tetrahydrofuran (220 mL) and cooled to 0°C in an ice water bath. A solution of potassium tert-butoxide (51.4 mL, 51.385 mmol) was slowly added to the reaction mixture. After completion of the addition, the reaction mixture was warm to room temperature and stirred for 1 hour. A tetrahydrofuran solution of 1-(tert-butyl) 2-ethyl 3-oxopyrrolidine-1,2-dicarboxylate (5.0 g, 20.544 mmol) was added to the above solution, and the reaction mixture was stirred at room temperature for 3 hours. Upon completion of the reaction (monitored by LCMS), the reaction mixture was extracted with ethyl acetate (200 mL × 2), washed with saturated ammonium chloride solution (60 mL × 2), dried over anhydrous sodium sulfate, filtered, and concentrated. The crude product was purified by flash silica gel column chromatography (ethyl acetate/petroleum ether = 0-10%) to obtain 1-(tert-butyl) 2-ethyl 3-methylenepyrrolidine-1,2-dicarboxylate (2240 mg, yield: 43%). ES-API: [M-55]⁺= 200.1.

Step 2: 1-(tert-butyl) 2-ethyl 3-methylenepyrrolidine-1,2-dicarboxylate (670 mg, 2.624 mmol) was dissolved in tetrahydrofuran (20 mL) and cooled to 0°C in an ice water bath. A tetrahydrofuran solution of borane (13 mL, 13.121 mmol, 1 M in tetrahydrofuran) was slowly added, and then the reaction mixture was warmed to room temperature and stirred for 3 hours. Upon completion of the reaction (monitored by LCMS), 3 M sodium hydroxide solution (4 mL) and hydrogen peroxide (3 mL) were successively added, and the reaction mixture was stirred at room temperature for 1 hour. Water (30 mL) was added, and the mixture was extracted with dichloromethane (20 mL × 3). The organic phase was washed with saturated brine (15 mL × 2), dried over anhydrous sodium sulfate, filtered, and concentrated to obtain a crude product of 1-(tert-butyl) 2-ethyl 3-(hydroxymethyl)pyrrolidine-1,2-dicarboxylate (859 mg). ES-API: [M-55]⁺= 218.1.

Step 3: 1-(tert-butyl) 2-ethyl 3-(hydroxymethyl)pyrrolidine-1,2-dicarboxylate (859 mg, 3.143 mmol) was dissolved in dichloromethane (20 mL). Imidazole (1070 mg, 15.714 mmol) and tert-butyldimethylchlorosilane (710 mg, 4.714 mmol) were successively added to the reaction mixture, which was then stirred at room temperature overnight. Upon completion of the reaction (monitored by LCMS), the reaction mixture was diluted with dichloromethane (30 mL), washed with water (10 mL × 2) and saturated brine (15 mL). The organic phase was dried over anhydrous sodium sulfate, filtered, and concentrated. The crude product was purified by flash silica gel column chromatography (ethyl acetate/petroleum ether = 0-4%) to obtain 1-(tert-butyl) 2-ethyl 3-(((tertbutyldimethylsilyl)oxy)methyl)pyrrolidine-1,2-dicarboxylate (553 mg, yield: 45%). ES-API: [M-99]⁺= 288.2.

Step 4: Under nitrogen protection, 1-(tert-butyl) 2-ethyl 3-(((tertbutyldimethylsilyl)oxy)methyl)pyrrolidine-1,2-dicarboxylate (553 mg, 1.427 mmol) was dissolved in anhydrous tetrahydrofuran (20 mL), and the mixture was placed in a dry ice-acetone bath. Diisopropylamine lithium (1.5 mL, 2.854 mmol) was slowly added dropwise to the solution, followed by stirring at this temperature for 1 hour. Then, a solution of 3-chloro-2-chloromethylpropene (892 mg, 7.134 mmol) in anhydrous tetrahydrofuran (3 mL) was slowly added via syringe, and the reaction mixture was stirred at -78°C for another hour, then slowly warmed to room temperature and stirred overnight. Upon completion of the reaction (monitored by LCMS), the reaction was quenched with saturated ammonium chloride solution (30 mL), and the mixture was extracted with ethyl acetate (20 mL × 3), washed with saturated brine (15 mL × 3), dried over anhydrous sodium sulfate, filtered, and concentrated. The crude product was purified by flash silica gel column chromatography (ethyl acetate/petroleum ether = 0-3%) to obtain 1-(tert-butyl) 2-ethyl 3-(((tert-butyldimethylsilyl)oxy)methyl)-2-(2-(chloromethyl)allyl)pyrrolidine-1,2-dicarboxylate (176 mg, yield: 26%). ES-API: [M-55]⁺= 420.2.

Step 5: At room temperature, 4 M hydrochloric acid-dioxane solution (5 mL) was slowly added to a solution of 1-(tert-butyl) 2-ethyl 3-(((tert-butyldimethylsilyl)oxy)methyl)-2-(2-(chloromethyl)allyl)pyrrolidine-1,2-dicarboxylate (176 mg, 0.370 mmol) in acetonitrile (2 mL), and the reaction mixture was stirred at room temperature for 2 hours. Upon completion of the reaction (monitored by LCMS), the reaction mixture was concentrated under reduced pressure to dryness to obtain a crude product of ethyl 2-(2-(chloromethyl)allyl)-3-(hydroxymethyl)pyrrolidine-2-carboxylate (96 mg). ES-API: [M+ H]⁺= 262.1.

Step 6: At room temperature, sodium bicarbonate (276 mg, 3.286 mmol) and potassium iodide (11 mg, 0.066 mmol) were added to a solution of ethyl 2-(2-(chloromethyl)allyl)-3-(hydroxymethyl)pyrrolidine-2-carboxylate (86 mg, 0.329 mmol) in acetonitrile (10 mL), and the reaction mixture was stirred at room temperature for 1 hour. The reaction was monitored by LCMS, and the mixture was filtered, washing the insoluble material with acetonitrile. The filtrate was concentrated under reduced pressure to obtain a crude product of ethyl 1-(hydroxymethyl)-6-methylenetetrahydro-1H-pyrrolizine-7a(5H)-carboxylate (127 mg). ES-API: [M+H]⁺= 226.1.

Step 7: Ethyl 1-(hydroxymethyl)-6-methylenetetrahydro-1H-pyrrolizine-7a(5H)-carboxylate (127 mg, 0.564 mmol) was dissolved in dichloromethane (10 mL). Imidazole (192 mg, 2.819 mmol) and tert-butyldimethylchlorosilane (110 mg, 0.733 mmol) were successively added to the reaction mixture, which was then stirred at room temperature overnight. Upon completion of the reaction (monitored by LCMS), the reaction mixture was diluted with dichloromethane (30 mL), washed with water (10 mL × 2) and saturated brine (15 mL), dried over anhydrous sodium sulfate, filtered, and concentrated. The crude product was purified by flash silica gel column chromatography (ethyl acetate/petroleum ether = 0-4%) to obtain ethyl 1-(((tert-butyldimethylsilyl)oxy)methyl)-6-methylenetetrahydro-1H-pyrrolizine-7a(5H)-carboxylate (20 mg, yield: 10%). ES-API: [M+ 1]⁺= 340.3.

Step 8: Under nitrogen protection, ethyl 1-(((tert-butyldimethylsilyl)oxy)methyl)-6-methylenetetrahydro-1H-pyrrolizine-7a(5H)-carboxylate (20 mg, 0.059 mmol) was dissolved in anhydrous tetrahydrofuran (5 mL) and the mixture was placed in an ice water bath. Lithium aluminium hydride (0.2 mL, 0.20 mmol, 1 M tetrahydrofuran solution) was slowly added dropwise to the solution, and the mixture was then stirred at this temperature for 0.5 hours. Upon completion of the reaction (monitored by LCMS), the reaction mixture was quenched with sodium sulfate decahydrate (2.0 eq) and stirred at room temperature for 0.5 hours, then filtered to remove insoluble material. The filtrate was concentrated under reduced pressure to obtain a crude product of (1-(((tertbutyldimethylsilyl)oxy)methyl)-6-methylenetetrahydro-1H-pyrrolizin-7a(5H)-yl)methanol (17.5 mg). ES-API: [M+H]⁺= 298.2.

Step 9: (1-(((tert-butyldimethylsilyl)oxy)methyl)-6-methylenetetrahydro-1H-pyrrolizin-7a(5H)-yl)methanol (17 mg, 0.058 mmol) was dissolved in anhydrous tetrahydrofuran (5 mL). Under an ice water bath, sodium hydride (2.3 mg, 0.058 mmol) was added and the mixture reacted for 10 minutes. A solution of tert-butyl (5S,5aS,6S,9R)-12-((1H-benzo[d][1,2,3]triazol-1-yl)axy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.029 mmol) in tetrahydrofuran (2 mL) was added dropwise to the reaction mixture, reacted at 0 °C for 30 minutes, then heated to 35 °C and stirred for 2 hours. Upon completion of the reaction (monitored by LCMS), the reaction mixture was diluted with ethyl acetate (10 mL), washed with saturated ammonium chloride solution (10 mL × 2), and saturated brine (10 mL × 3), dried over anhydrous sodium sulfate, filtered, and concentrated to obtain a crude product of tert-butyl (5S,5aS,6S,9R)-12-((1-(((tert-butyldimethylsilyl)oxy)methyl)-6-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (86 mg). ES-API: [M+H]⁺= 1021.4.

Step 10: Cesium fluoride (128 mg, 0.842 mmol) was added to tert-butyl (SS,SaS,6S,9R)-12-((1-(((tert-butyldimethylsilyl)oxy)methyl)-6-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (86 mg, 0.084 mmol) in N,N-dimethylformamide (2 mL), and the mixture was stirred at room temperature for 0.5 hours. Upon completion of the reaction (monitored by LCMS), the reaction mixture was diluted with ethyl acetate (15 mL), washed with water (15 mL × 2), and saturated brine (15 mL × 3), dried over anhydrous sodium sulfate, filtered, and concentrated to obtain a crude product of tert-butyl (5S,5aS,6S,9R)-12-((1-(((tert-butyldimethylsilyl)oxy)methyl)-6-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (72 mg). ES-API: [M+H]⁺= 865.3.

Step 11: Under an ice water bath, 4 M hydrochloric acid-dioxane solution (5 mL) was slowly added to a solution of tert-butyl (SS,SaS,6S,9R)-12-((1-(((tert-butyldimethylsilyl)oxy)methyl)-6-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (72 mg, 0.083 mmol) in acetonitrile (2 mL), and the mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic conditions: 15mL-40-60-13 min) to obtain a pale white solid, (7a-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)-6-methylenehexahydro-1H-pyrrolizin-1-yl)methanol (Z683, 6.73 mg, total yield for 3 steps: 18 %). ES-API: [M+H]⁺= 651.3. ¹H NMR (400 MHz, CD₃OD) δ 8.11-8.06 (m, 2 H), 7.66-7.54 (m, 2 H), 7.46-7.39 (m, 1 H), 5.39-5.35 (m, 1 H), 5.03 (s, 2 H), 4.57-4.52 (m, 1 H), 4.33-4.07 (m, 4 H), 3.78-3.60 (m, 4 H), 3.47-3.35 (m, 2 H), 3.21-3.12 (m, 2 H), 2.69-2.65 (m, 1 H), 2.53-2.48 (m, 2 H), 2.05-2.03 (m, 1 H), 1.93-1.78 (m, 4 H), 1.57 (t, J= 8.5 Hz, 3 H), 1.18-1.16 (m, 3 H), 1.00-0.98 (m, 1 H).

### Example 75 Synthesis of (5S,5aS,65,9R)-2-(5-ethynylisoquinolin-4-yl)-1-fluoro-12(((2R)-2-fluoro-6-methylenetetrahydro-1H-pyrrolizin-7a(5H)-yl)methoxy)-5-methyl-5a,6,7,8,9,10-hexahydro-SH-4-oxa-3,10a,11,13,14-pentaaza-6,9-methanonaphtho[1.,8-ab]heptalene (Z684)

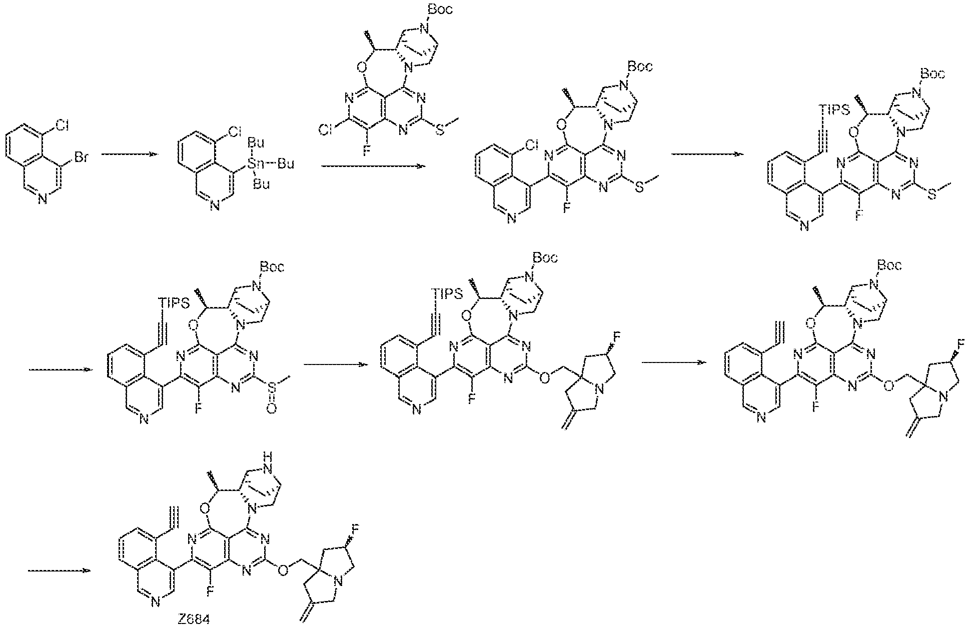

Step 1: Under nitrogen protection, 4-bromo-5-chloroisoquinoline (900 mg, 3.7 mmol), hexabutylditin (4.3 g, 7.4 mmol), tris(dibenzylideneacetone)dipalladium (340 mg, 0.37 mmol), tricyclohexylphosphine (208 mg, 0.02 mmol), and anhydrous lithium chloride (787 mg, 18.6 mmol) were dissolved in dioxane (5 mL). The reaction mixture was heated to 110 °C and stirred for 16 hours. Upon completion of the reaction (monitored by LCMS), the reaction mixture was concentrated, and the crude product was purified by flash chromatography through silica gel (petroleum ether: ethyl acetate = 30:1) to obtain 5-chloro-4-(tributylstannyl)isoquinoline (800 mg, yield: 47.6 %). ES-API: [M+H]⁺=454.1.

Step 2: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (290 mg, 0.6 mmol), 5-chloro-4-(tributylstannyl)isoquinoline (681 mg, 1.5 mmol), bis(dibenzylideneacetone)palladium(0) (44 mg, 0.06 mmol), 1,1'-binaphthyl-2,2'-bis(diphenylphosphine) (75 mg, 0.12 mmol), and copper(I) iodide (34 mg, 0.18 mmol) were dissolved in toluene (10 mL). The reaction mixture was heated to 90 °C and stirred for 16 hours. Upon completion of the reaction (monitored by LCMS), the reaction mixture was concentrated, and the crude product was purified by flash chromatography through silica gel (petroleum ether: ethyl acetate = 20:1) to obtain tert-butyl (5S,5aS,6S,9R)-2-(5-chloroisoquinolin-4-yl)-1-fluoro-5-methyl-12-(methylthio)-Sa,6,7,8,9,10-hexahydro-SH-4-oxa-3,10a,11,13,14-pentaaza-6,9-methanonaphtho[l,8-ab]heptalene-14-carboxylate (240 mg, yield: 65 %). ES-API: [M+H]⁺=609.1.

Step 3: Under nitrogen protection, tert-butyl (SS,SaS,6S,9R)-2-(5-chloroisoquinolin-4-yl)-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (190 mg, 0.3 mmol), triisopropylsilyl acetylene (284 mg, 1.56 mmol), cesium carbonate (203 mg, 0.6 mmol), 2-dicyclohexylphosphino-2',4',6'-triisopropylbiphenyl (89 mg, 0.19 mmol), and palladium(II) chloride acetonitrile complex (16 mg, 0.06 mmol) were dissolved in acetonitrile (10 mL). The reaction mixture was heated to 85 °C and stirred for 16 hours. Upon completion of the reaction (monitored by LCMS), the reaction mixture was concentrated, and the crude product was purified by flash chromatography through silica gel (dichloromethane: methanol = 20:1) to obtain tert-butyl (SS,SaS,6S,9R)-1-fluoro-5-methyl-12-(methylthio)-2-(5-((triisopropylsilyl)ethynyl)isoquinolin-4-yl)-5a,6,7,8,9,10-hexahydro-5H-4-oxa-3,10a,11,13,14-pentaaza-6,9-methanonaphtho[1,8-ab]heptalene-14-carboxylate (130 mg, yield: 55 %). ES-API: [M+H]⁺=755.2.

Step 4: Under nitrogen protection, m-chloroperoxybenzoic acid (36 mg, 0.21 mmol) was added to a dichloromethane (5 mL) solution of tert-butyl (SS,SaS,6S,9R)-1-fluoro-5-methyl-12-(methylthio)-2-(5-((triisopropylsilyl)ethynyl)isoquinolin-4-yl)-5a,6,7,8,9,10-hexahydro-5H-4-oxa-3,10a,11,13,14-pentaaza-6,9-methanonaphtho[1,8-ab]heptalene-14-carboxylate (130 mg, 0.17 mmol). The reaction mixture was stirred at room temperature for 1 hour. Upon completion of the reaction (monitored by LCMS), the reaction mixture was diluted with ethyl acetate (20 mL), washed with saturated sodium sulfite solution (15 mL × 2) and saturated brine (15 mL × 3), dried over anhydrous sodium sulfate, filtered, and concentrated to obtain the crude product tert-butyl (5S,5aS,6S,9R)-1-fluoro-5-methyl-12-(methylsulfinyl)-2-(5-((triisopropylsilyl)ethynyl)isoquinolin-4-yl)-5a,6,7,8,9, 10-hexahydro-5H-4-oxa-3, 10a, 11, 13,14-pentaaza-6,9-methanonaphtho[1,8-ab]heptalene-14-carboxylate (132.7 mg, yield: 100 %). ES-API: [M+H]⁺=771.2.

Step 5: ((2R)-2-Fluoro-6-methylene tetrahydro-1H-pyrrolo-7a(5H)-yl)methanol (59 mg, 0.34 mmol) was dissolved in anhydrous tetrahydrofuran (5 mL), and sodium hydride (27 mg, 0.69 mmol) was added under an ice water bath. The mixture reacted for 30 minutes. Then, a tetrahydrofuran (2 mL) solution of tert-butyl (5S,5aS,6S,9R)-1-fluoro-5-methyl-12-(methylsulfinyl)-2-(5-((triisopropylsilyl)ethynyl)isoquinolin-4-yl)-5a,6,7,8,9,10-hexahydro-5H-4-oxa-3,10a,11,13,14-pentaaza-6,9-methanonaphtho[1,8-ab]heptalene-14-carboxylate (132.7 mg, 0.17 mmol) was added dropwise to the solution. The reaction was continued at room temperature for 30 minutes. Upon completion of the reaction (monitored by LCMS), the reaction mixture was diluted with ethyl acetate (20 mL), washed with saturated ammonium chloride solution (15 mL × 2), saturated brine (15 mL × 3), dried over anhydrous sodium sulfate, filtered, and concentrated. The crude product was purified by flash chromatography through silica gel (dichloromethane: methanol = 20:1) to obtain tert-butyl (5S,5aS,6S,9R)-1-fluoro-12-(((2R)-2-fluoro-6-methylenetetrahydro-1H-pyrrolizin-7a(5H)-yl)methoxy)-5-methyl-2-(5-((triisopropylsilyl)ethynyl)isoquinolin-4-yl)-5a,6,7,8,9,10-hexahydro-5H-4-oxa-3,10a,11,13,14-pentaaza-6,9-methanonaphtho[1,8-ab]heptalene-14-carboxylate (40 mg, yield: 26.6%). ES-API: [M+H]⁺=878.2.

Step 6: Cesium fluoride (69 mg, 0.46 mmol) was added to a solution of tert-butyl (5S,5aS,6S,9R)-1-fluoro-12-(((2R)-2-fluoro-6-methylenetetrahydro-1H-pyrnolizin-7a(SH)-yl)methoxy)-5-methyl-2-(5-((triisopropylsilyl)ethynyl)isoquinolin-4-yl)-5a,6,7,8,9,10-hexahydro-5H-4-oxa-3,10a,11,13,14-pentaaza-6,9-methanonaphtho[1,8-ab]heptalene-14-carboxylate (40 mg, 0.046 mmol) in N,N-dimethylformamide (3 mL). The reaction mixture was stirred at room temperature for 2 hours. Upon completion of the reaction (monitored by LCMS), the reaction mixture was diluted with ethyl acetate (20 mL), washed with water (15 mL × 2), saturated brine (15 mL × 3), dried over anhydrous sodium sulfate, filtered, and concentrated to obtain tert-butyl (5S,5aS,6S,9R)-2-(5-ethynylisoquinolin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (32 mg, yield: 97 %). ES-API: [M+H]⁺=722.2.

Step 7: Under an ice water bath, 4 M hydrochloric acid-dioxane solution (5 mL) was slowly added to a solution of tert-butyl (SS,SaS,6S,9R)-2-(5-ethynylisoquinolin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (32 mg, 0.044 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic conditions: 15 ml-35-85-13 min) to obtain a light yellow solid, (5S,5aS,6S,9R)-2-(5-ethynylisoquinolin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z684, 2 mg, yield: 7 %). ES-API: [M+H]⁺= 622.2.

### Example 76 Synthesis of 8-ethynyl-1-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)isoquinolin-3-amine (Z685)

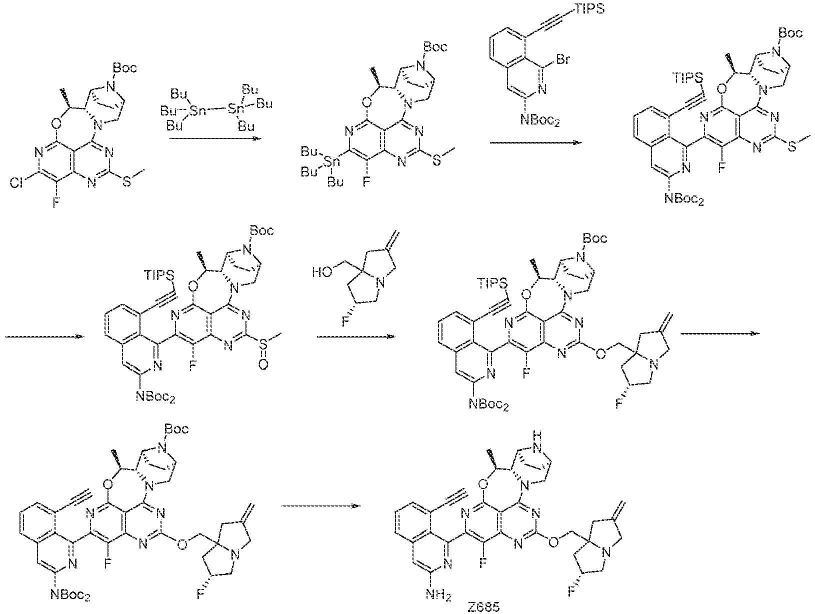

Step 1: Tert-butyl (SS,SaS,6S,9R)-2-chloro-1-fluoro-5 methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (500 mg, 1.037 mmol), hexabutylditin (1.046 mL, 2.074 mmol), tris(dibenzylideneacetone)dipalladium (95.00 mg, 0.104 mmol), trihexylphosphine (58.19 mg, 0.207 mmol), lithium chloride (220 mg, 5.19 mmol) and 1,4-dioxane (15 mL) were added to a round-bottom flask. The reaction mixture was stirred for 18 hours in an oil bath at 110 degrees under nitrogen protection. After concentration of the reaction mixture, the residue was purified by a flash silica gel column (0-15% ethyl acetate/petroleum ether) to obtain tert-butyl (5S,5aS,6S,9R)-1-fluoro-5-methyl-12-(methylthio)-2-(tributylstannyl)-5a,6,7,8,9,10-hexahydro-5H-4-oxa-3,10a,11,13,14-pentaaza-6,9-methanonaphtho[1,8-ab]heptalene-14-carboxylate (370 mg, yield 48%). ES-API:[M+Na]⁺=738.1.

Step 2: A mixture of tert-butyl (SS,SaS,6S,9R)-1-fluoro-5-methyl-12-(methylthio)-2-(tributylstannyl)-5a,6,7,8,9,10-hexahydro-5H-4-oxa-3,10a,11,13,14-pentaaza-6,9-methanonaphtho[1,8-ab]heptalene-14-carboxylate (146 mg, 0.20 mmol), tert-butyl (1-bromo-8-((triisopropylsilyl)ethynyl)isoquinolin-3-yl)(tert-butoxycarbonyl)carbamate (80 mg, 0.13 mmol), copper(I) iodide (25 mg, 0.13 mmol), tetrakis(triphenylphosphine)palladium (23 mg, 0.02 mmol), and anhydrous lithium chloride (22 mg, 0.53 mmol) in N,N-dimethylacetamide (4 mL) was added to a round-bottom flask. The reaction was proceeded under a nitrogen atmosphere at 130 degrees in an oil bath for 4 hours. Water (30 mL) was added to the reaction mixture, which was then extracted with ethyl acetate (20 mL X 2). The organic phase was washed with saturated brine (30 mL), dried over anhydrous sodium sulfate, filtered, and concentrated. The residue was purified by a flash silica gel column (0-30% ethyl acetate/petroleum ether) to obtain tert-butyl (5S,5aS,6S,9R)-2-(3-(bis(tert-butoxycarbonyl)amino)-8-((triisopropylsilyl)ethynyl)isoquinolin-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (25 mg, yield 21%). ES-API:[M+Na]⁺=870.3.

Step 3: To a round-bottom flask, tert-butyl (SS,SaS,6S,9R)-2-(3-(bis(tert-butoxycarbonyl)amino)-8-((triisopropylsilyl)ethynyl)isoquinolin-1-yl)-1-fluoro-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (25 mg, 0.029 mmol) and dichloromethane (3 mL) were added. The mixture was cooled to 0 degrees, and then m-chloroperoxybenzoic acid (10 mg, 0.057 mmol) was added to the reaction mixture. The reaction mixture was stirred at room temperature for 1 hour. Water (30 mL) was added to the reaction mixture, followed by extraction with ethyl acetate (20 mL × 2). The organic phase was washed with saturated brine (30 mL), dried over anhydrous sodium sulfate, filtered, and concentrated to obtain tert-butyl (SS,SaS,6S,9R)-2-(3-(bis(tert-butoxycarbonyl)amino)-8-((triisopropylsilyl)ethynyl)isoquinolin-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (25 mg, crude). ES-API:[M+Na]⁺=886.3.

Step 4: To a round-bottom flask, 60% sodium hydride (4 mg, 0.14 mmol) and tetrahydrofuran (3 mL) were added. The mixture was cooled to 0 degrees and then a solution of ((2R)-2-fluoro-6-methylenetetrahydro-1H-pynolizin-7a(5H)-yl)methanol (14 mg, 0.085 mmol) in anhydrous tetrahydrofuran (1 mL) was added. The reaction was proceeded for 10 minutes at room temperature. The reaction mixture was cooled to 0 degrees, and tert-butyl (5S,5aS,6S,9R)-2-(3-(bis(tert-butoxycarbonyl)amino)-8-((triisopropylsilyl)ethynyl)isoquinolin-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (25 mg, crude) was added and the mixture reacted for 0.5 hours at 0 degrees. Water (30 mL) was added to the reaction mixture, followed by extraction with ethyl acetate (20 mL × 2). The organic phase was washed with saturated brine (30 mL), dried over anhydrous sodium sulfate, filtered, and concentrated. The residue was purified by a flash silica gel column (0-3% methanol/dichloromethane) to obtain tert-butyl (5S,5aS,6S,9R)-2-(3-(bis(tert-butoxycarbonyl)amino)-8-((triisopropylsilyl)ethynyl)isoquinolin-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (15 mg, yield 53%). ES-API: [M+H]⁺=993.3.

Step 5: Cesium fluoride (23 mg, 0.15 mmol) was added to a solution of tert-butyl (5S,5aS,6S,9R)-2-(3-(bis(tert-butoxycarbonyl)amino)-8-((triisopropylsilyl)ethynyl)isoquinolin-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (15 mg, crude) in N,N-dimethylformamide (2 mL). The reaction mixture was stirred at room temperature for 1 hour. Upon completion of the reaction (monitored by LCMS), the reaction mixture was diluted with ethyl acetate (30 mL) and washed with water (30 mL) and saturated brine (30 mL × 2). The organic phase was dried over anhydrous sodium sulfate, filtered, and concentrated to obtain tert-butyl (5S,5aS,6S,9R)-2-(3-(bis(tert-butoxycarbonyl)amino)-8-ethynylisoquinolin-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (12 mg, crude). ES-API: [M+H]⁺= 837.1.

Step 6: Under an ice-water bath, 4 M hydrochloric acid-dioxane solution (0.5 mL) was slowly added to a solution of tert-butyl (SS,SaS,6S,9R)-2-(3-(bis(tert-butoxycarbonyl)amino)-8-ethynylisoquinolin-1-yl)-1-fluoro-12-(((2R)-2-fluoro-b-methylenetetrahydro-1H-pyrrolizin-7a(5H)-yl)methoxy)-5-methyl-5a,6,7,8,9, 10-hexahydro-5H-4-oxa-3, 10a, 11, 13,14-pentaaza-6,9-methanonaphtho[1,8-ab]heptalene-14-carboxylate (10 mg, crude) in acetonitrile (1 mL). The reaction mixture was stirred at room temperature for 1 hour. Upon completion of the reaction (monitored by LCMS), the reaction mixture was concentrated under reduced pressure to dryness. The crude product was purified by preparative HPLC (ammonium bicarbonate method 1) to obtain 8-ethynyl-1-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)isoquinolin-3-amine (Z685, 0.67 mg, yield: 8%), white solid. ES-API: [M+H]⁺= 637.2.

### Example 77 Synthesis of 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-1-amine (Z686)

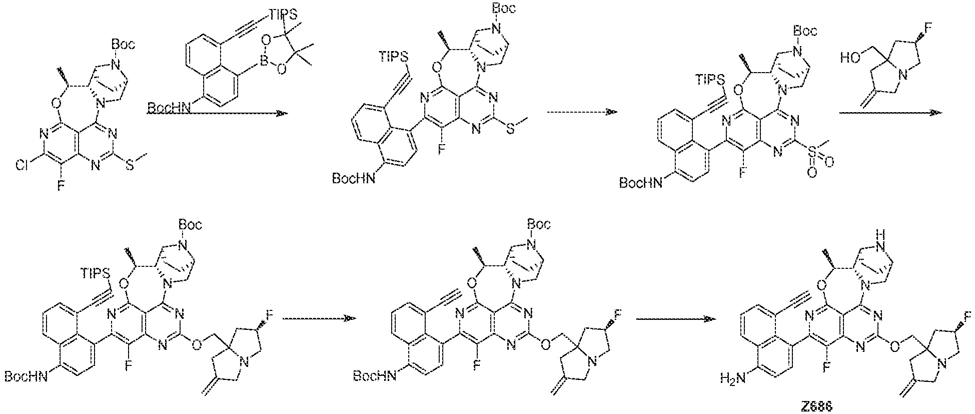

Step 1: tert-butyl (SS,SaS,6S,9R)-2-chloro-1-fluoro-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (120 mg, 0.249 mmol) and tert-butyl (4-(4,4,5,5-tetramethyl-1,3,2-dioxaborolan-2-yl)-5-((triisopropylsilyl)efihynyl)naphthalen-1-yl)carbamate (164.22 mg, 0.299 mmol) were dissolved in tetrahydrofuran (10 mL) and water (1 mL). To this solution, palladium(II) bis(2-amino-1,1'-biphenyl) [tetrakis(butyl(dicyclohexylphosphine))] methanesulfonate (18.13 mg, 0.025 mmol) and potassium phosphate (158.55 mg, 0.747 mmol) were added. After the addition was complete, the reaction mixture was stirred at 65 degrees Celsius for 5 hours. Upon completion of the reaction, water (15 mL) was added to the reaction mixture, which was then extracted with ethyl acetate (15 mL × 3). The organic phase was washed with saturated brine (30 mL), dried over anhydrous sodium sulfate, filtered, concentrated, and the crude product was purified by a flash silica gel column (ethyl acetate/petroleum ether: 0-30%) to obtain tert-butyl (5S,5aS,6S,9R)-2-(4-((tert-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (130 mg, 0.150 mmol, yield 60.07%), a white solid. ES-API: [M+H]⁺= 869.3.

Step 2: Tert-butyl (5S,5aS,6S,9R)-2-(4-((tert-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (130 mg, 0.150 mmol) was dissolved in dichloromethane (5 mL). At room temperature, m-chloroperoxybenzoic acid (85% purity, 91.10 mg, 0.449 mmol) was added to the solution. After the addition was complete, the reaction mixture was stirred at room temperature for 1 hour. Saturated sodium thiosulfate solution (5 mL) was added to the reaction mixture, followed by extraction with dichloromethane (10 mL × 3). The combined organic phases were successively washed with saturated bicarbonate solution (20 mL) and saturated sodium chloride (20 mL). The organic phase was dried and concentrated to obtain tert-butyl (5S,5aS,6S,9R)-2-(4-((tert-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (120 mg, 0.133 mmol, yield 89.03%). Light yellow solid. ES-API: [M +H]⁺= 901.0.

Step 3: ((2R)-2-fluoro-6-methylenetetrahydro-1H-pyrrolizin-7a(5H)-yl)methanol (26.60 mg, 0.155 mmol) was dissolved in tetrahydrofuran (3 mL) and cooled to 0 degrees Celsius. Under a nitrogen atmosphere, sodium hydride (60%, 12.43 mg, 0.311 mmol) was added to the solution and stirred for 30 minutes. Subsequently, tert-butyl (SS,SaS,6S,9R)-2-(4-((tert-butoxycarbonyl)amino)-8-((triisopropylsilyl)ethynyl)naphthalen-1 -yl)-1 -fluoro-5-methyl- 12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (70 mg, 0.078 mmol) was slowly added to the solution, which was then stirred for 2 hours at 0 degrees Celsius. Water (5 mL) was added to quench the reaction, and the mixture was extracted with ethyl acetate (20 mL × 3). The organic phase was washed with saturated brine (30 mL × 3), dried over anhydrous sodium sulfate, filtered, and concentrated to obtain a crude product. The crude product was purified by a flash silica gel column (ethyl acetate/petroleum ether: 0-70%) to obtain tert-butyl (5S,5aS,6S,9R)-2-(4-((tert-butoxycarbonyl)amino)-8-((triisopropylsilyl)ethynyl)naphthalen-1 -yl)-1 -fluoro- 12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.030 mmol, yield 38.92%), a yellow solid. ES-API: [M+H]⁺=992.1.

Step 4: A mixed solution of tert-butyl (SS,SaS,6S,9R)-2-(4-((tert-butoxycarbonyl)amino)-8-((triisopropylsilyl)ethynyl)naphthalen-1 -yl)-1 -fluoro- 12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.030 mmol), cesium fluoride (45.92 mg, 0.302 mmol), and N, N-dimethylformamide (2 mL) was stirred at room temperature for 2 hours. Water (10 mL) was added to the reaction mixture, which was then extracted with ethyl acetate (15 mL × 2). The organic phase was washed with saturated brine (15 mL × 3), dried over anhydrous sodium sulfate, filtered, and concentrated to obtain tert-butyl (5S,5aS,6S,9R)-2-(4-((tert-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (18 mg, 0.022 mmol, yield 71.22%), a yellow oily liquid. ES-API: [M+H]⁺=835.1.

Step 5: Tert-butyl (5S,5aS,6S,9R)-2-(4-((tert-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (15 mg, 0.018 mmol) was dissolved in acetonitrile (2 mL) and cooled to 0 degrees Celsius. Hydrochloric acid in dioxane solution (4 M, 1 mL) was added to the solution, and the reaction mixture was stirred at 0 degrees Celsius for 2 hours. The reaction mixture was concentrated, redissolved in dichloromethane (3 mL), and basified to pH 8 with an ammonium hydroxide solution in methanol (7 M). The solution was concentrated, and the crude product was purified by preparative HPLC (ammonium bicarbonate method 1) to obtain 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a, 11,13,14-pentaaza-6,9-methanonaphtho[1,8-ab]heptalen-2-yl)naphthalen-1-amine (Z686, 2.5 mg, 0.004 mmol, yield: 20.82%), brown solid. ES-API: [M+H]⁺=636.2.

### Example 78 Synthesis of (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptalene (Z687)

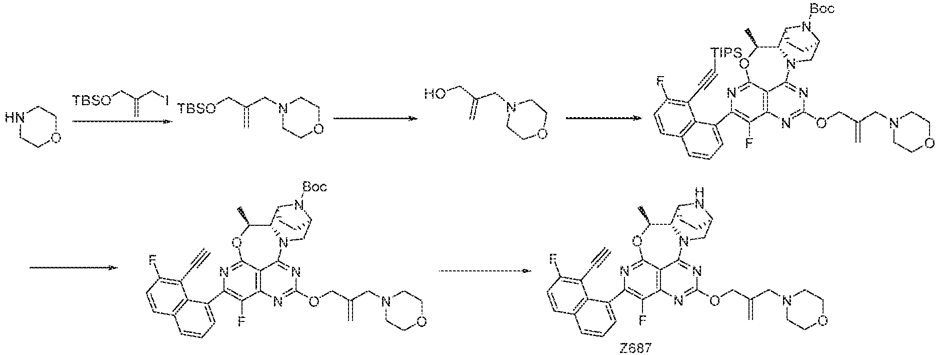

Step 1: At room temperature, to a solution of morpholine (1.0 g, 11.478 mmol) in acetonitrile (50.0 mL), tert-butyl((2-(iodomethyl)allyl)oxy)dimethylsilane (3.58 g, 11.478 mmol) and potassium carbonate (4.0 g, 28.94 mmol) were added successively, and the mixture was stirred for 3 hours. After the reaction was complete, saturated ammonium chloride (30 mL) was added to the reaction mixture, followed by extraction with ethyl acetate (200 mL) and saturated brine (50 mL × 3). The organic phase was dried over anhydrous sodium sulfate, filtered, and the crude product was purified by silica gel column chromatography [petroleum ether: ethyl acetate = 100:0 - 50:50, (v/v)] to obtain 4-(2-(((tert-butyldimethylsilyl)oxy)methyl)allyl)morpholine (2.67 g, yield 85.8%). ES-API: [M+H]⁺=272.2.

Step 2: Under an ice-water bath, a 4 M hydrochloric acid-dioxane solution (4.0 mL, 4 M, 16.0 mmol) was slowly added to a solution of 4-(2-(((tert-butyldimethylsilyl)oxy)methyl)allyl)morpholine (700 mg, 2.578 mmol) in acetonitrile (10.0 mL). The reaction mixture was stirred at 0°C for 0.5 hours. Upon completion of the reaction (monitored by LCMS), the reaction mixture was concentrated under reduced pressure to dryness, and the residue was added to methanol (50 mL), followed by addition of sodium bicarbonate (2.0 g). The mixture was stirred at room temperature for 1 hour. The mixture was filtered, and the filtrate was evaporated under reduced pressure to obtain 2-(morpholinomethyl)prop-2-en-1-ol (0.4 g, yield 98%). ES-API: [M+H]⁺=158.2.

Step 3: 2-(morpholinomethyl)prop-2-en-1-ol (180 mg, 1.145 mmol) was dissolved in anhydrous tetrahydrofuran (10 mL). Under an ice-water bath, sodium hydride (90.8 mg, 2.27 mmol) was added, and the reaction mixture was stirred for 30 minutes under the ice-water bath. Then, a solution of tert-butyl (5S,5aS,6S,9R)-1-fluoro-2-(7-fluoro-8-((triisopropylsilyl)ethynyl)naphthalen-1-yl)-5-methyl-12-(methylsulfonyl)-5a,6,7,8,9,10-hexahydro-5H-4-oxy-3,10a,11,13,14-pentaaza-6,9-methanonaphtho[1,8-ab]heptalene-14-carboxylate (137.7 mg, 0.175 mmol) in tetrahydrofuran (2 mL) was added dropwise to the reaction mixture, which then reacted at 25°C for 30 minutes. Upon completion of the reaction (monitored by LCMS), the reaction mixture was diluted with ethyl acetate (50 mL), washed with saturated ammonium chloride solution (15 mL × 2), and saturated brine (50 mL × 3). The organic phase was dried over anhydrous sodium sulfate, filtered, and the crude product was purified by silica gel column chromatography (dichloromethane: methanol = 15:1) to obtain tert-butyl (5 S,5aS,6S,9R)-1 -fhioro-2-(7-fluoro-8-((triisopropylsilyl)ethynyl)naphthalen-1 -yl)-5-methyl-12-((2-(morpholinornethyl)allyl)oxy)-5a,6,7,8,9,10-hexahydro-5H-4-oxa-3,10a,11,13,14-pentaaza-6,9-methanonaphtho[1,8-ab]heptalene-14-carboxylate (400 mg, crude product). ES-API: [M+H]⁺= 881.2.

Step 4: Cesium fluoride (600.0 mg, 3.947 mmol) was added to a solution of tert-butyl (SS,SaS,6S,9R)-1-fluoro-2-(7-fluoro-8-((triisopropylsilyl)ethynyl)naphthalen-1-yl)-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptalene-14-carboxylate (154.0 mg, 0.175 mmol) in N,N-dimethylformamide (10.0 mL), and the reaction mixture was stirred at room temperature for 2 hours. Upon completion of the reaction (monitored by LCMS), the reaction mixture was diluted with ethyl acetate (60 mL), washed with water (30 mL × 2), and saturated brine (50 mL × 3). The organic phase was dried over anhydrous sodium sulfate, filtered, and concentrated to obtain tert-butyl (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptalene-14-carboxylate (180 mg, crude product). ES-API: [M+H]⁺= 725.2.

Step 5: Under an ice-water bath, a 4 M hydrochloric acid-dioxane solution (2.0 mL, 4 M, 8.0 mmol) was slowly added to a solution of tert-butyl (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptalene-14-carboxylate (127 mg, 0.175 mmol) in acetonitrile (10.0 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic conditions: 15ml-35-85-13min) to obtain a pale yellow solid, (SS,SaS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptalene (Z687, 60 mg, yield: 54.88 %). ES-API: [M+H]⁺= 625.2. ¹H NMR (400 MHz, CD₃OD) δ 8.10-8.05 (m, 2H), 7.66 -7.55 (m, 2H), 7.45-7.38 (m, 1H), 5.39-5.31 (m, 2H), 5.20 (s, 1H), 5.06 -4.97(m, 2H), 4.56-4.49 (m, 1H), 4.09-4.04 (m, 1H), 3.79 -3.48 (m, 7H), 3.31-3.30(m, 2H), 3.20-3.10(m, 3H), 2.44 (s, 3H), 2.08-2.04(m, 1H), 1.86-1.76(m, 3H), 1.59-1.55(m, 3H).

### Example 79 Synthesis of (5S,5aS,6S,9R)-2-(8-ethynyl-7-fluoronaphthalen-1-yl)-1-fluoro-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z688)

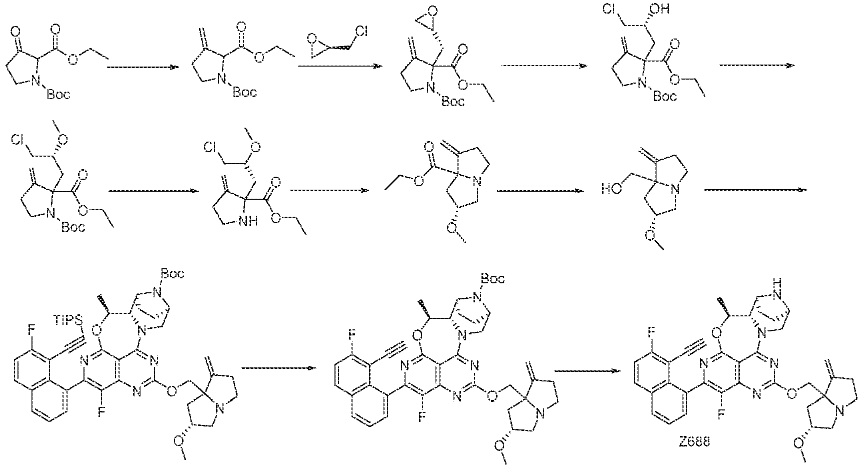

Step 1: Methyl triphenylphosphonium bromide (22.6 g, 63.3 mmol) was added to tetrahydrofuran (200 mL) and cooled to 0°C. Under a nitrogen atmosphere, at 0°C, a solution of potassium tertbutoxide in tetrahydrofuran (63 mL, 63 mmol, 1M) was added to the mixture. After the addition was complete, the reaction mixture was warmed to room temperature and stirred for 1 hour. 1-(tert-butyl) 2-ethyl 3-oxopyrrolidine-1,2-dicarboxylate (6.5 g, 25.3 mmol) was dissolved in tetrahydrofuran (20 mL) and slowly added dropwise to the above reaction mixture under a nitrogen atmosphere. After the dropwise addition, the mixture was further stirred at room temperature for 3 hours. Saturated ammonium chloride solution (60 mL) was added, followed by extraction with ethyl acetate (200 mL × 3). The organic layer was washed with saturated brine (400 mL), dried over anhydrous sodium sulfate, filtered, and concentrated. The crude product was purified by a flash silica gel column (ethyl acetate/petroleum ether: 0%-8%) to obtain 1-(tert-butyl) 2-ethyl 3-methylpyrrolidine-1,2-dicarboxylate (2.8 g, 10.9 mmol, yield 43.4%), a colorless transparent liquid. ES-API: [M-55]⁺= 200.1.

Step 2: At -70°C, bis(trimethylsilyl)aminolithium (22 mL, 1M, 21.8 mmol) was added to a dry tetrahydrofuran solution (20 mL) of 1-(tert-butyl) 2-ethyl 3-methylpyrrolidine-1,2-dicarboxylate (2.8 g, 10.9 mmol). The mixture was stirred at this temperature for 1 hour, then (R)-2-(chloromethyl)oxirane (3.0 g, 32.7 mmol) was added and the mixture was stirred overnight at room temperature. Upon completion of the reaction, saturated ammonium chloride solution (30 mL) was added, followed by extraction with ethyl acetate (200 mL × 3). The organic phase was washed with saturated brine (200 mL × 3), dried over anhydrous sodium sulfate, filtered, and concentrated. The crude product was purified by a flash silica gel column (dichloromethane:methanol = 100:0~90:10, v/v) to obtain the target compound 1-(tert-butyl) 2-ethyl 3-methylene-2-(((R)-oxiran-2-yl)methyl)pyrrolidine-1,2-dicarboxylate (2.4 g, 7.72 mmol, yield 70.8%). ES-API: [M-55]⁺=256.1.

Step 3: At room temperature, acetic acid (3 mL) and lithium chloride (3.2 g, 77.2 mmol) were added to a dry acetonitrile solution (30 mL) of 1-(tert-butyl) 2-ethyl 3-methylene-2-(((R)-oxiran-2-yl)methyl)pyrrolidine-1,2-dicarboxylate (2.4 g, 7.72 mmol). The mixture was stirred at this temperature for 12 hours. Upon completion of the reaction, saturated ammonium chloride solution (20 mL) was added to the solution, followed by extraction with ethyl acetate (30 mL × 2). The combined organic phases were dried over anhydrous sodium sulfate, filtered, and the filtrate was concentrated under reduced pressure to obtain the crude product target compound 1-(tert-butyl) 2-ethyl 2-((R)-3-chloro-2-hydroxypropyl)-3-methylenepyrrolidine-1,2-dicarboxylate (1.8 g, 5.17 mmol, yield 67.0%). ES-API: [M-99]⁺=248.1.

Step 4: 1-(tert-butyl) 2-ethyl 2-((R)-3-chloro-2-hydroxypropyl)-3-methylenepyrrolidine-1,2-dicarboxylate (420 mg, 1.21 mmol) was dissolved in dichloromethane (10 mL). Then 1,8-bis(dimethylamino)naphthalene (906 mg, 4.23 mmol) and trimethyloxonium tetrafluoroborate (572 mg, 3.86 mmol) were added, and the mixture was stirred at room temperature for 16 hours. Upon completion of the reaction, the reaction mixture was diluted with dichloromethane (30 mL × 2), washed with water (10 mL × 2) and saturated brine (50 mL × 3), dried over anhydrous sodium sulfate, filtered, and concentrated. The crude product was purified by a flash silica gel column (ethyl acetate/petroleum ether: 0%-30%) to obtain 1-(tert-butyl) 2-ethyl 2-((R)-3-chloro-2-methoxypropyl)-3-methylenepyrrolidine-1,2-dicarboxylate (278 mg, 0.77 mmol, yield 63.6%). ES-API: [M-99]⁺= 262.1.

Step 5: Hydrochloric acid dioxane solution (5 mL, 4M, 20 mmol) was added to a dichloromethane solution (5 mL) of 1-(tert-butyl) 2-ethyl 2-((R)-3-chloro-2-methoxypropyl)-3-methylenepyrrolidine-1,2-dicarboxylate (278 mg, 0.77 mmol). The reaction mixture was stirred at room temperature for 4 hours. Upon completion of the reaction, the solvent was evaporated under reduced pressure to obtain ethyl 2-((R)-3-chloro-2-methoxypropyl)-3-methylenepyrrolidine-2-carboxylate (156 mg, crude product). ES-API: [M+H]⁺=262.1.

Step 6: Sodium bicarbonate (504 mg, 6.00 mmol) was added to an acetonitrile solution (5 mL) of ethyl 2-((R)-3-chloro-2-methoxypropyl)-3-methylenepyrrolidine-2-carboxylate (156 mg, 0.60 mmol), and the reaction was proceeded at 70°C for 4 hours. Upon completion of the reaction, the mixture was cooled to room temperature, filtered, and the filtrate was evaporated under reduced pressure to remove solvent. The crude product was purified by a flash silica gel column (methanol/dichloromethane: 0%-10%) to obtain ethyl (6R)-6-methoxy-1-methylenetetrahydro-1H-pyrrolizine-7a(5H)-carboxylate (96 mg, 0.43 mmol, total yield for the last two steps 55.4%). ES-API: [M+H]⁺=226.1.

Step 7: ethyl (6R)-6-methoxy-1-methylenetetrahydro-1H-pyrrolizine-7a(5H)-carboxylate (96 mg, 0.43 mmol) was dissolved in tetrahydrofuran (3 mL) and cooled to 0°C. Under a nitrogen atmosphere, lithium aluminium hydride solution (2 mL, 2.00 mmol, 1M in tetrahydrofuran) was slowly added to the solution and stirred at 0°C for 2 hours. Upon completion of the reaction, sodium sulfate decahydrate (1.3 g, 4.00 mmol) was added slowly and the mixture was stirred for 0.5 hours, followed by filtration After dried over anhydrous sodium sulfate. The filtrate was concentrated to obtain ((6R)-6-methoxy-1-methylenetetrahydro-1H-pyrrolizin-7a(5H)-yl)methanol (73 mg, 0.40 mmol, yield 92.8%), a colorless and transparent liquid. ES-API: [M+H]⁺=184.1.

Step 8: ((6R)-6-methoxy-1-methylenetetrahydro-1H-pyrrolizin-7a(5H)-yl)methanol (73 mg, 0.40 mmol) was dissolved in anhydrous tetrahydrofuran (4 mL). Under an ice water bath, sodium hydride (48 mg, 1.20 mmol) was added and the mixture reacted for 20 minutes in an ice water bath. Then, a tetrahydrofuran solution (2 mL) of tert-butyl (5S, 5aS, 6S, 9R)-1-fluoro-2-(7-fluoro-8-((tripropylsilyl)ethynyl)naphthalen-1 -yl)-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (157 mg, 0.20 mmol) was added dropwise to the above reaction mixture, which was then stirred at room temperature for 30 minutes. Upon completion of the reaction (monitored by LCMS), the reaction mixture was diluted with ethyl acetate (10 mL), washed with saturated ammonium chloride solution (5 mL × 2) and saturated brine (10 mL × 3), dried over anhydrous sodium sulfate, and filtered. The crude product was purified through silica gel by automatic flash chromatography (dichloromethane: methanol = 0%-6% as the mobile phase) to obtain tert-butyl (SS,SaS,6S,9R)-1-fluoro-2-(7-fluoro-8-((triisopropylsilyl)ethynyl)naphthalen-1-yl)-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (65 mg, 0.07 mmol, yield 35.9%). ES-API: [M+H]⁺= 907.1.

Step 9: Cesium fluoride (106 mg, 0.70 mmol) was added to a N,N-dimethylformamide (3 mL) solution of tert-butyl (5S,5aS,6S,9R)-1-fluoro-2-(7-fluoro-8-((triisopropylsilyl)ethynyl)naphthalen-1-yl)-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (65 mg, 0.07 mmol). The reaction mixture was stirred at room temperature for 2 hours. Upon completion of the reaction (monitored by LCMS), the reaction mixture was diluted with ethyl acetate (10 mL), washed with water (10 mL × 2) and saturated brine (10 mL × 3), dried over anhydrous sodium sulfate, filtered and concentrated to obtain tert-butyl (5S,5aS,6S,9R)-2-(8-ethynyl-7-fluoronaphthalen-1-yl)-1-fluoro-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (42 mg, 0.06 mmol, yield 80%). ES-API: [M+H]⁺= 751.1.

Step 10: Under an ice water bath, a 4 M hydrochloric acid-dioxane solution (3.0 mL) was slowly added to a acetonitrile (2.0 mL) solution of tert-butyl (5S,5aS,6S,9R)-2-(8-ethynyl-7-fluoronaphthalen-1-yl)-1-fluoro-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (42 mg, 0.06 mmol). The reaction mixture was stirred at 0°C for 5 hours. Upon completion of the reaction (monitored by LCMS), the reaction mixture was concentrated under reduced pressure, and the residue was purified by preparative HPLC (ammonium bicarbonate method 2) (chromatographic conditions: 15ml-35-85-13min) to obtain a pale yellow solid, (5S,5aS,6S,9R)-2-(8-ethynyl-7-fluoronaphthalen-1-yl)-1-fluoro-12-(((6R)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z688, 22 mg, 0.03 mmol, yield: 56.4%). ES-API: [M+H]⁺= 651.2.

### Example 80 Synthesis of (5S,5aS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[l,8-ab]heptalene (Z689) and (5S,5aS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hezahydro-5H-4-oza-3,10a,11,13,14-pentaaza-6,9-methanonaphtho[1,8-ab]heptalene (Z690)

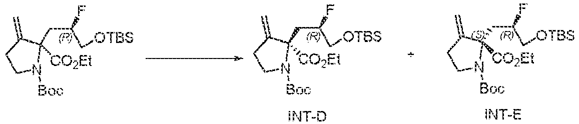

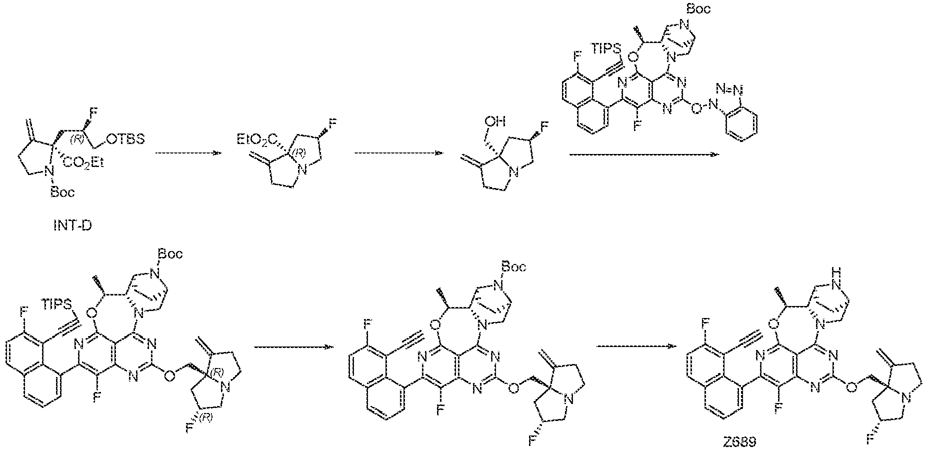

Step 1: A mixture of 1-(tert-butyl) 2-ethyl 2-((R)-3-((tert-butyldimethylsilyl)oxy)-2-fluoropropyl)-3-methylenepyrrolidine-1,2-dicarboxylate (1.5 g) was subjected to chiral separation (separation column: CHIRALPAK IG 250mm*4.6mm*5um; mobile phase: isopropanol: n-hexane = 5:95; flow rate: 1 mL/min; column temperature: 30°C) to obtain two enantiomers. One of the iomeric compounds was arbitrarily designated as 1-(tert-butyl) 2-ethyl (R)-2-((R)-3-((tert-butyidimethylsilyl)oxy)-2-fluoropropyl)-3-methylenepyrrolidine-1,2-dicarboxylate (INT-D, 720 mg, peak 1, retention time = 3.68 min), ES-API:[M+Na]⁺=468.3. The other iomeric compound was arbitrarily designated as 1-(tert-butyl) 2-ethyl (S)-2-((R)-3-((tert-butyldimethylsilyl)oxy)-2-fluoropropyl)-3-methylenepyrrolidine-1,2-dicarboxylate (INT-E, 330 mg, peak 2, retention time = 4.44 min). ES-API:[M+Na]⁺=468.3.

Step 2: Dimethyl sulfoxide (DMSO) (10 mL, 137.86 mmol) was added to 1-(tert-butyl) 2-ethyl (R)-2-((R)-3-((tert-butyldimethylsilyl)oxy)-2-fluoropropyl)-3-methylenepyrrolidine-1,2-dicarboxylate (INT-D, 700 mg, 1.57 mmol). The reaction mixture was stirred at 45°C for 24 hours. DMSO (10 mL, 137.86 mmol) was added, and the reaction mixture was stirred at 65°C for 2 days. The reaction mixture was then concentrated. Dichloromethane (5 mL) was added to the residue, which was then cooled to 0°C. Triethylamine (0.22 mL, 1.57 mmol) was added. The reaction mixture was stirred at room temperature for 30 minutes. The reaction was quenched with water (50 mL) and the mixture was extracted with dichloromethane (30 mL × 2). The organic phase was washed with saturated brine (30 mL), dried over anhydrous sodium sulfate, filtered, and concentrated. The residue was purified by a flash silica gel column (0-60% ethyl acetate/petroleum ether) to obtain ethyl (6R,7aR)-6-fluoro-1-methylenetetrahydro-1H-pyrrolizine-7a(5H)-carboxylate (200 mg, yield 58%). ES-API: [M+H]⁺=214.2.

Step 3: To a solution of ethyl (6R,7aR)-6-fluoro-1-methylenetetrahydro-1H-pyrrolizine-7a(5H)-carboxylate (200 mg, 0.94 mmol) in tetrahydrofuran (6 mL), a lithium aluminium hydride/tetrahydrofuran solution (1.8 mL, 1.8 mmol) was added dropwise. At 0°C, the reaction mixture was stirred for 1 hour. The reaction was then quenched with 3 drops of water followed by a 15% sodium hydroxide solution (0.1 mL). The mixture was stirred at room temperature for 30 minutes and then filtered through diatomaceous earth. The filtrate was concentrated to obtain a crude product of ((6R,7aR)-6-fluoro-1-methylenetetrahydro-1H-pyrrolizin-7a(5H)-yl)methanol (120 mg, yield 74%), a yellow oil. ES-API: [M+H]⁺=172.1.

Step 4: Sodium hydride (71 mg, 1.77 mmol) and tetrahydrofuran (10 mL) were added to a round-bottom flask. The mixture was cooled to 0°C, and a solution of ((6R,7aR)-6-fluoro-1-methylenetetrahydro-1H-pyrrolizin-7a(5H)-yl)methanol (91 mg, 0.53 mmol) in anhydrous tetrahydrofuran (1 mL) was added. The reaction was proceed at room temperature for 10 minutes. The reaction mixture was cooled to 0°C, and tert-butyl (SS,SaS,6S,9R)-12-((1H-benzo[d][1,2,3]triazol-1-yl)oxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (380 mg, 0.44 mmol) was added to the reaction mixture, which then reacted at room temperature for 1 hour. Water (30 mL) was added to the reaction mixture. The mixture was extracted with ethyl acetate (20 mL × 2). The organic phase was washed with saturated brine (30 mL), dried over anhydrous sodium sulfate, filtered, and concentrated to obtain tert-butyl (SS,SaS,6S,9R)-1-fluoro-12-(((6R,7aR)-b-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (375 mg, yield 94%). ES-API: [M+H]⁺=895.3.

Step 5: Cesium fluoride (1 g, 6.58 mmol) was added to N,N-dimethylformamide (5 mL) containing tert-butyl (5S,5aS,6S,9R)-1-fluoro-12-(((6R,7aR)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (490 mg, crude product). The reaction mixture was stirred at room temperature for 2 hours. Upon completion of the reaction (monitored by LCMS), ethyl acetate (100 mL) was added to dilute the reaction mixture, which was then washed with water (30 mL) and saturated brine (30 mL × 2). The organic phase was dried over anhydrous sodium sulfate, filtered, and concentrated. The residue was purified by a flash silica gel column (0-5% methanol/dichloromethane) to obtain tert-butyl (5S,5aS,6S,9R)-2-(8-ethynyl-7-fluoronaphthalen-1-yl)-1-fluoro-12-(((6R,7aR)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (300 mg, yield 74%). ES-API: [M+H]⁺= 739.2.

Step 6: Under an ice-water bath, a 4 M hydrochloric acid-dioxane solution (6 mL) was slowly added to a solution of tert-butyl (5S,5aS,6S,9R)-2-(8-ethynyl-7-fluoronaphthalen-1-yl)-1-fluoro-12-(((6R,7aR)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (300 mg, crude product) in acetonitrile (3 mL). The reaction mixture was stirred at room temperature for 1 hour. Upon completion of the reaction (monitored by LCMS), the reaction mixture was concentrated under reduced pressure to dryness, and the residue was dissolved in a saturated sodium bicarbonate aqueous solution and dichloromethane. The solution was extracted with dichloromethane/methanol (10:1, 50 mL). The organic phase was washed with saturated brine (30 mL), dried over anhydrous sodium sulfate, filtered, and concentrated to obtain (5S,5aS,6S,9R)-2-(8-ethynyl-7-fluoronaphthalen-1-yl)-1-fluoro-12-(((6R,7aR)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z689, 230mg, yield: 88%), yellow solid. ES-API: [M+H]⁺= 639.2. ¹H NMR(400MHz, CD₃OD): 8.10-8.05(m, 2H), 7.80-7.38 (m, 3H), 5.43-5.20 (m, 3H), 4.60-4.40(m, 3H), 4.20-3.80(m, 2H), 3.68-3.50 (m, 4H), 3.20-3.14(m, 1H), 2.83-2.71(m, 1H), 2.27-1.95(m, 3H), 1.90-1.77(m, 6H), 1.58-1.55 (m, 3H), 0.90-0.85(m, 1H).

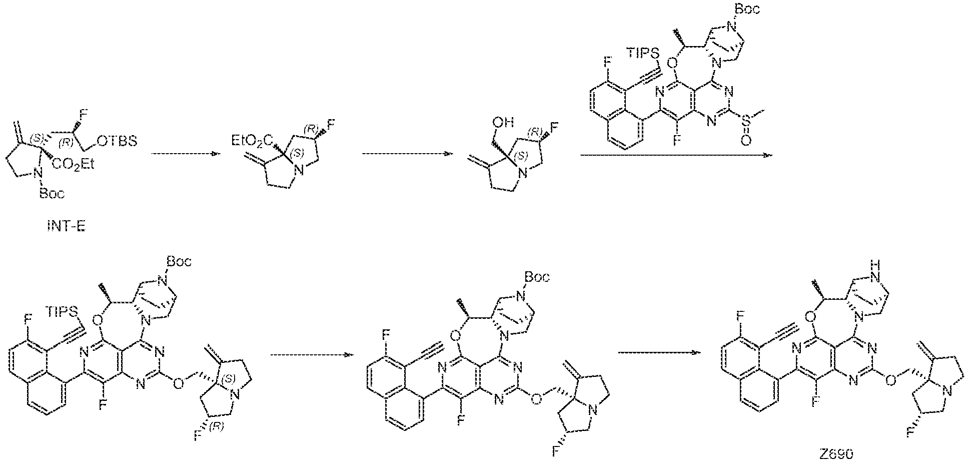

Step 1: Thionyl chloride (10 mL, 137.86 mmol) was added to the solution of 1-(tert-butyl) 2-ethyl (S)-2-((R)-3-((tert-butyldimethylsilyl)oxy)-2-fluoropropyl)-3-methylenepyrrolidine-1,2-dicarboxylate (75 mg, 0.74 mmol). The reaction mixture was stirred at 45 degrees for 24 hours. Thionyl chloride (10 mL, 137.86 mmol) was then added to the reaction mixture. The reaction was further stirred at 65 degrees for 2 days. The reaction mixture was concentrated, and the residue was added to dichloromethane (5 mL) and cooled to 0 degrees. The reaction was quenched with water (50 mL) and the mixture was extracted with dichloromethane (30 mL × 2). The organic phase was washed with saturated brine (30 mL), dried over anhydrous sodium sulfate, filtered, and concentrated. The residue was purified by flash silica gel column chromatography (0-60% ethyl acetate/petroleum ether) to obtain ethyl (6R,7aS)-6-fluoro-1-methylenetetrahydro-1H-pyrrolizine-7a(5H)-carboxylate (75 mg, yield 47%).ES-API:[M+H]⁺=214.2.

Step 2: Lithium aluminium hydride/tetrahydrofuran solution (0.75 mL, 0.75 mmol) was added dropwise to a tetrahydrofuran solution (5 mL) of ethyl (6R,7aS)-6-fluoro-1-methylenetetrahydro-1H-pyrrolizine-7a(5H)-carboxylate (75 mg, 0.37 mmol). At 0 °C, the mixture was stirred for 1 hour. The reaction was quenched successively with three drops of water and 15% sodium hydroxide solution (0.1 mL). The mixture was stirred at room temperature for 30 minutes and filtered through diatomaceous earth. The filtrate was concentrated to obtain a crude product, ((6R,7aS)-6-fluoro-1-methylenetetrahydro-1H-pyrrolizin-7a(5H)-yl)methanol (45 mg, yield 69%), as a yellow oil. ES-API: [M+H]⁺=172.1.

Step 3: Sodium hydride (60%, 16 mg, 0.41 mmol) and tetrahydrofuran (6 mL) were added to a round-bottom flask. The mixture was cooled to 0 degrees, and a solution of ((6R,7aS)-6-fluoro-1-methylenetetrahydro-1H-pyrrolizin-7a(5H)-yl)methanol (26 mg, 0.15 mmol) in anhydrous tetrahydrofuran (1 mL) was added. The reaction was proceed at room temperature for 10 minutes. The reaction mixture was then cooled to 0 degrees, and tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (80 mg, 0.1 mmol) was added to the reaction mixture, which then reacted at room temperature for 1 hour. Water (30 mL) was added to the reaction mixture. The mixture was extracted with ethyl acetate (20 mL × 2). The organic phase was washed with saturated brine (30 mL), dried over anhydrous sodium sulfate, filtered, and concentrated to obtain tert-butyl (5S,5aS,6S,9R)-1-fluoro-12-(((6R,7aS)-6-fluoro-1-methylenetetrahydro-1H-pynolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (90 mg, crude product). ES-API: [M+H]⁺=895.3.

Step 4: Cesium fluoride (300 mg, 1.97 mmol) was added to a solution of tert-butyl (5S,5aS,6S,9R)-1-fluoro-12-(((6R,7aS)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (90 mg, crude product) in N,N-dimethylformamide (3 mL). The reaction mixture was stirred at room temperature for 2 hours. Upon completion of the reaction (monitored by LCMS), the reaction mixture was diluted with ethyl acetate (100 mL) and washed with water (30 mL) and saturated brine (30 mL × 2). The organic phase was dried over anhydrous sodium sulfate, filtered, and concentrated. The residue was purified by flash silica gel column chromatography (0-5% methanol/dichloromethane) to obtain tert-butyl (5 S,5aS,6S,9R)-2-(8-ethynyl-7-fluoronaphthalen-1 -yl)-1 -fluoro- 12-(((6R,7aS)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (70 mg, crude product). ES-API: [M+H]⁺= 739.2.

Step 5: Under an ice water bath, a 4 M hydrochloric acid-dioxane solution (3 mL) was slowly added to a solution of tert-butyl (5S,5aS,6S,9R)-2-(8-ethynyl-7-fluoronaphthalen-1-yl)-1-fluoro-12-(((6R,7aS)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (70 mg, crude product) in acetonitrile (3 mL). The reaction mixture was stirred at room temperature for 1 hour. Upon completion of the reaction (monitored by LCMS), the reaction mixture was concentrated under reduced pressure to dryness. The residue was purified by preparative HPLC (ammonium bicarbonate method 1) to obtain (5S,5aS,6S,9R)-2-(8-ethynyl-7-fluoronaphthalen-1-yl)-1-fluoro-12-(((6R,7aS)-6-fluoro-1-mefihylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z690, 10mg, yield: 16%), white solid. ES-API: [M+H]⁺= 639.2. ¹HNMR(400MHz, CD₃OD): 8.10-8.05(m, 2H), 7.80-7.38 (m, 3H), 5.43-5.21 (m, 3H), 4.52-4.32 (m, 3H), 4.20-3.94 (m, 2H), 3.68-3.50 (m, 4H), 3.32-3.19(m, 3H), 2.27-2.05 (m, 3H), 1.90-1.80(m, 6H), 1.58-1.55 (m, 3H).

### Example 81 Synthesis of (5S,5aS,6S,9R)-2-(8-ethynyl-7-fluoronaphthalen-1-yl)-1-fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z691)

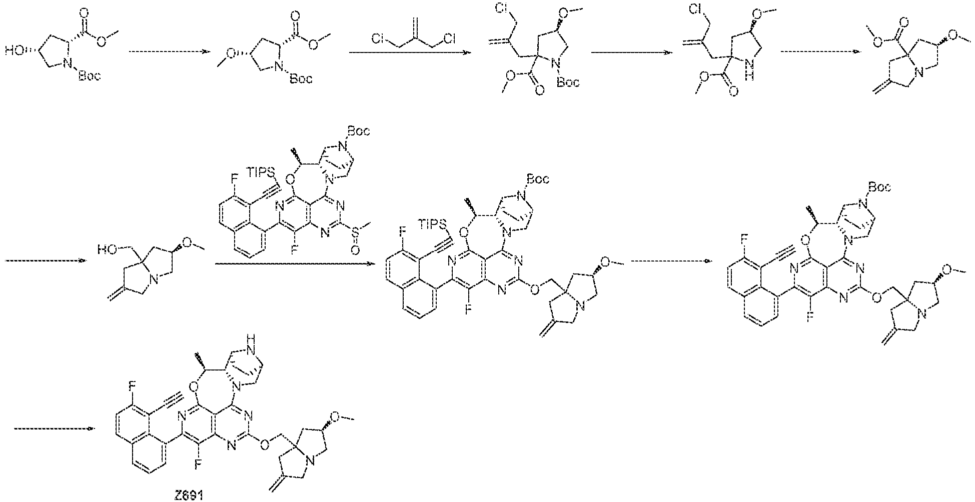

Step 1: Methyl (2R, 4R)-4-hydroxy-1-{[(2-methylprop-2-yl)oxy]carbonyl}tetrahydropyrrole-2-carboxylate (3 g, 12.231 mmol) was dissolved in tetrahydrofuran (40 mL). The solution was cooled to 0 °C in an ice-water bath, and then sodium hydride (465 mg, 11.619 mmol) was added batchwise. The mixture was stirred at 0 °C for 30 minutes. Methyl iodide (1.5 mL, 24.462 mmol) was added to the above solution, and the reaction mixture was slowly warmed to room temperature and stirred for 2 hours. Upon completion of the reaction (monitored by LCMS), the reaction was quenched with saturated ammonium chloride solution (20 mL) and the mixture was extracted with ethyl acetate (15 mL × 3). The organic phase was washed with saturated brine (15 mL × 2), dried over anhydrous sodium sulfate, filtered, and concentrated. The crude product was purified by automated flash chromatography through silica gel (ethyl acetate: petroleum ether = 0-20%) to obtain 1-(tert-butyl) 2-methyl (2R,4R)-4-methoxypyrrolidine-1,2-dicarboxylate (1807 mg, yield: 57%). ES-API: [M-55]⁺= 204.1.

Step 2: Under nitrogen protection, 1-(tert-butyl) 2-methyl (2R,4R)-4-methoxypyrrolidine-1,2-dicarboxylate (1 g, 3.857 mmol) was dissolved in 30 mL of anhydrous tetrahydrofuran. The mixture was placed in a dry ice-acetone bath. Diisopropylamine lithium (3.9 mL, 7.713 mmol) was slowly added dropwise to the above solution, and the mixture was stirred at this temperature for 1 hour. Then, a solution of 3-chloro-2-chloromethylpropene (2.410 g, 19.283 mmol) in anhydrous tetrahydrofuran (5 mL) was slowly added to the above solution via syringe. The reaction mixture was stirred at -78 °C for 1 hour, and then slowly warmed to room temperature and stirred overnight. Upon completion of the reaction (monitored by LCMS), the reaction was quenched with saturated ammonium chloride solution (30 mL) and the mixture was extracted with ethyl acetate (20 mL × 3). The organic phase was washed with saturated brine (15 mL × 3), dried over anhydrous sodium sulfate, filtered, and concentrated. The crude product was purified by automated flash chromatography through silica gel (ethyl acetate: petroleum ether = 0-9.5%) to obtain 1-(tert-butyl) 2-methyl (4R)-2-(2-(chloromethyl)allyl)-4-methoxypyrrolidine-1,2-dicarboxylate (943 mg, yield: 70%). ES-API: [M-55]⁺= 292.1.

Step 3: At room temperature, a 4 M hydrochloric acid-dioxane solution (10 mL) was slowly added to a solution of 1-(tert-butyl) 2-methyl (4R)-2-(2-(chloromethyl)allyl)-4-methoxypyrrolidine-1,2-dicarboxylate (943 mg, 2.711 mmol) in acetonitrile (5 mL). The reaction mixture was stirred at room temperature for 2 hours. Upon completion of the reaction (monitored by LCMS), the reaction mixture was concentrated under reduced pressure to dryness to obtain the crude product methyl (4R)-2-(2-(chloromethyl)allyl)-4-methoxypyrrolidine-2-carboxylate (671 mg). ES-API: [M+H]⁺= 248.2.

Step 4: At room temperature, sodium bicarbonate (1.138 g, 13.545 mmol) and potassium iodide (90 mg, 0.542 mmol) were added to a solution of methyl (4R)-2-(2-(chloromethyl)allyl)-4-methoxypyrrolidine-2-carboxylate (671 mg, 2.709 mmol) in acetonitrile (15 mL). The reaction mixture was stirred at room temperature for 1.5 hours. The reaction was monitored by LCMS. The mixture was then filtered, and the insoluble material was washed with acetonitrile. The filtrate was concentrated under reduced pressure. The residue was purified by automated flash chromatography through silica gel (ethyl acetate: petroleum ether = 0-45%) to obtain the crude product methyl (2R)-2-methoxy-6-methylenetetrahydro-1H-pyrrolizine-7a(5H)-carboxylate (613 mg). ES-API: [M+H]⁺= 212.2.

Step 5: Under nitrogen protection, methyl (2R)-2-methoxy-6-methylenetetrahydro-1H-pyrrolizine-7a(5H)-carboxylate (211 mg, 0.999 mmol) was dissolved in anhydrous tetrahydrofuran (5 mL), and the mixture was placed in an ice-water bath. Lithium aluminium hydride (2 mL, 1.998 mmol, 1 M in tetrahydrofuran solution) was slowly added dropwise to the above solution, and then the mixture was stirred at this temperature for 0.5 hours. Upon completion of the reaction (monitored by LCMS), the reaction mixture was quenched by adding sodium sulfate decahydrate (2.0 eq) and stirred at room temperature for 0.5 hours, then filtered to remove insoluble material. The filtrate was concentrated under reduced pressure to obtain the crude product ((2R)-2-methoxy-6-methylenetetrahydro-1H-pyrrolizin-7a(5H)-yl)methanol (162 mg, yield: 89%). ES-API: [M+H]⁺= 184.1.

Step 6: ((2R)-2-methoxy-6-methylenetetrahydro-1H-pyrrolizin-7a(5H)-yl)methanol (56 mg, 0.305 mmol) was dissolved in anhydrous tetrahydrofuran (4 mL). Under ice-water bath, sodium hydride (12 mg, 0.305 mmol) was added and the mixture reacted for 10 minutes under ice-water bath. Then, a tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (80 mg, 0.102 mmol) was added dropwise to the reaction mixture. The reaction was proceeded at 0 °C for 30 minutes. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure to obtain the crude product tert-butyl (SS,SaS,6S,9R)-1-fluoro-2-(7-fluoro-8-((triisopropylsilyl)ethynyl)naphthalen-1-yl)-12-(((2R)-2-methoxy-6-methylenetetrahydro-1H-pynolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (110 mg). ES-API: [M+H]⁺= 907.3.

Step 7: Cesium fluoride (184 mg, 1.213 mmol) was added to a solution of tert-butyl (SS,SaS,6S,9R)-1-fluoro-2-(7-fluoro-8-((triisopmpylsilyl)ethynyl)naphthalen-1-yl)-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-arboxylate (110 mg, 0.121 mmol) in N,N-dimethylformamide (5 mL). The reaction mixture was stirred at room temperature for 0.5 hours. Upon completion of the reaction (monitored by LCMS), the reaction mixture was diluted with ethyl acetate (15 mL), washed with water (15 mL × 2), and saturated brine (15 mL × 3), dried over anhydrous sodium sulfate, filtered, and concentrated to obtain the crude product tert-butyl (5 S,5aS,6S,9R)-2-(8-ethynyl-7-fluoronaphthalen-1 -yl)-1 -fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (91 mg). ES-API: [M+H]⁺= 751.3.

Step 8: Under ice-water bath condition, a 4 M hydrochloric acid-dioxane solution (5 mL) was slowly added to a solution of tert-butyl (SS,SaS,6S,9R)-2-(8-ethynyl-7-fluoronaphthalen-1-yl)-1-fluoro-12-(((2R)-2-methoxy-6-methylenetetahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (91 mg, 0.121 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic conditions: 15 mL-45-55-13 min) to obtain a off-white solid, (5S,5aS,6S,9R)-2-(8-ethynyl-7-fluoronaphthalen-1-yl)-1-fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z691, 18.51 mg, total yield for 3 steps: 28 %). ES-API: [M+H]⁺= 651.2. ¹H NMR (400 MHz, CD₃OD) δ 8.11-8.05 (m, 2 H), 7.66-7.54 (m, 2 H), 7.46-7.39 (m, 1 H), 5.39 (d, J= 16.0 Hz, 1 H), 4.99 (s, 2 H), 4.56-4.52 (m, 1 H), 4.39 (t, J= 12.5 Hz, 1 H), 4.29-4.25 (dd, J1= 3.5 Hz, J2= 12.5 Hz, 1 H), 4.09-4.07 (m, 2 H), 3.79-3.49 (m, 4 H), 3.29-3.15 (m, 7 H), 2.87-2.80 (m, 2 H), 2.49-2.46 (m,

1 H), 2.34-2.31 (m, 1 H), 2.07-1.99 (m, 2 H), 1.87-1.76 (m, 3 H), 1.57 (t, J= 9.0 Hz, 3 H).

### Example 82 Synthesis of (5S,5aS,6S,9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z692)

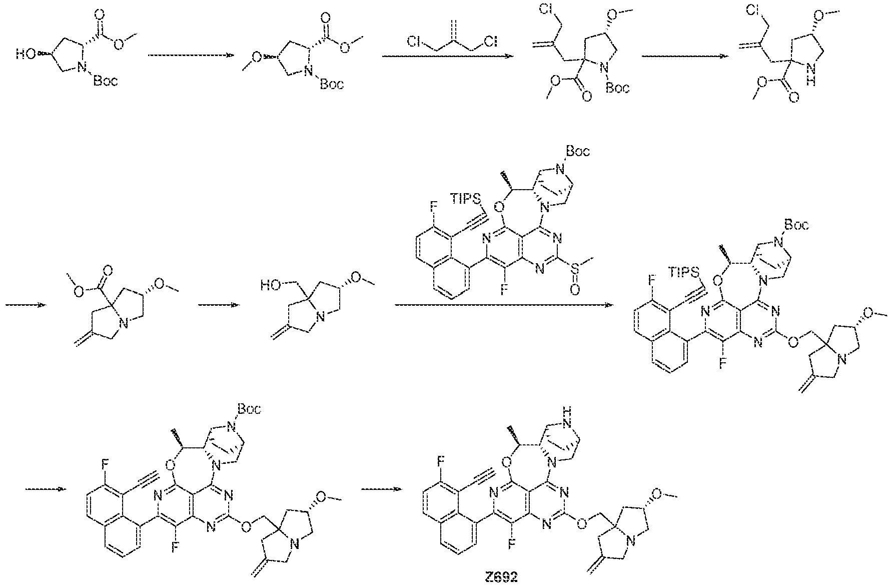

Step 1: 1-(tert-butyl)-2-methyl-(2R,4S)-4-hydroxypyrrolidine-1,2-dicarboxylate (3 g, 12.231 mmol) was dissolved in acetonitrile (60 mL). To this solution, methyl iodide (26.04 g, 183.464 mmol) and silver oxide (14.17 g, 61.155 mmol) were added. The reaction mixture was stirred at room temperature for 17 hours. Upon completion of the reaction (monitored by LCMS), the reaction mixture was filtered and concentrated to obtain the crude product. The crude product was purified by automated flash chromatography through silica gel (ethyl acetate: petroleum ether = 0-20%) to obtain 1-(tert-butyl)-2-methyl-(2R,4S)-4-methoxypyrrolidine-1,2-dicarboxylate (3 g, 11.570 mmol, yield 94.59%). ES-API: [M-Boc+H]⁺= 159.1.

Step 2: Under nitrogen protection, 1-(tert-butyl)-2-methyl-(2R,4S)-4-methoxypyrrolidine-1,2-dicarboxylate (1000 mg, 3.857 mmol) was dissolved in anhydrous tetrahydrofuran (30 mL). The mixture was placed in a dry ice-acetone bath. Diisopropylamine lithium (3.9 mL, 7.713 mmol) was slowly added dropwise to the above solution, and then the mixture was stirred at this temperature for 1 hour. Subsequently, a solution of 3-chloro-2-chloromethylpropene (2.410 g, 19.283 mmol) in anhydrous tetrahydrofuran (5 mL) was slowly added to the mixture via syringe. The reaction mixture was stirred at -78 °C for 1 hour, and then slowly warmed to room temperature and stirred overnight. Upon completion of the reaction (monitored by LCMS), the reaction was quenched with saturated ammonium chloride solution (30 mL) and the mixture was extracted with ethyl acetate (20 mL × 3). The organic phase was washed with saturated brine (15 mL × 3), dried over anhydrous sodium sulfate, filtered, and concentrated. The crude product was purified by automated flash chromatography through silica gel (ethyl acetate: petroleum ether = 0-9.5%) to obtain 1-(tert-butyl) 2-methyl (4S)-2-(2-(chloromethyl)allyl)-4-methoxypyrrolidine-1,2-dicarboxylate (500 mg, 1.437 mmol, yield 37.27%). ES-API: [M-55]⁺= 292.1.

Step 3: At room temperature, a 4 M hydrochloric acid-dioxane solution (2 mL) was slowly added to a solution of 1-(tert-butyl) 2-methyl (4S)-2-(2-(chloromethyl)allyl)-4-methoxypyrrolidine-1,2-dicarboxylate (500 mg, 1.437 mmol) in acetonitrile (2 mL). The reaction mixture was stirred at room temperature for 2 hours. Upon completion of the reaction (monitored by LCMS), the reaction mixture was concentrated under reduced pressure to dryness to obtain the crude product methyl (4S)-2-(2-(chloromethyl)allyl)-4-methoxypyrrolidine-2-carboxylate (408.49 mg, 1.437 mmol, yield 100.00%). ES-API: [M+H]⁺= 248.2.

Step 4: At room temperature, sodium bicarbonate (482.55 mg, 5.744 mmol) and potassium iodide (238.38 mg, 1.436 mmol) were added to a solution of methyl (4S)-2-(2-(chloromethyl)allyl)-4-methoxypyrrolidine-2-carboxylate (408 mg, 1.436 mmol) in acetonitrile (10 mL). The reaction mixture was stirred at room temperature for 1.5 hours. The reaction was monitored by LCMS, followed by filtration and acetonitrile washing of the insoluble material. The filtrate was concentrated under reduced pressure, and the residue was purified by flash silica gel column (methanol: dichloromethane = 0-3%) to obtain the crude product methyl (2S)-2-methoxy-6-methylenetetrahydro-1H-pyrrolizine-7a(5H)-carboxylate (200 mg, 0.947 mmol, yield 65.93%). ES-API: [M+H]⁺= 212.2.

Step 5: Under nitrogen protection, methyl (2S)-2-methoxy-6-methylenetetrahydro-1H-pyrrolizine-7a(5H)-carboxylate (150 mg, 0.710 mmol) was dissolved in anhydrous tetrahydrofuran (5 mL) and the mixture was placed in an ice-water bath. Lithium aluminium hydride (2 mL, 1.998 mmol, 1 M in tetrahydrofuran solution) was slowly added dropwise to the above solution, and then the mixture was stirred at this temperature for 0.5 hours. Upon completion of the reaction (monitored by LCMS), sodium sulfate decahydrate (2.0 eq) was added to quench the reaction, and the mixture was stirred at room temperature for 0.5 hours, followed by filtration to remove insoluble material. The filtrate was concentrated under reduced pressure to obtain the crude product ((2S)-2-methoxy-6-methylenetetrahydro-1H-pyrrolizin-7a(5H)-yl)methanol (100 mg, 0.546 mmol, yield 76.86%). ES-API: [M+H]⁺= 184.1.

Step 6: ((2S)-2-methoxy-6-methylenetetrahydro-1H-pyrrolizin-7a(5H)-y)methanol (34.19 mg, 0.187 mmol) was dissolved in anhydrous tetrahydrofuran (4 mL). Under an ice-water bath, sodium hydride (14.92 mg, 0.373 mmol) was added, and the mixture reacted for 10 minutes under an ice-water bath. A tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (75 mg, 0.093 mmol) was added dropwise to the reaction mixture, which then reacted at 0 °C for 30 minutes. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure to obtain the crude product tert-butyl (SS,SaS,6S,9R)-1-fluoro-2-(7-fluoro-8-((triisopropylsilyl)ethynyl)naphthalen-1 -yl)- 12-(((2S)-2-methoxy-6-methylenetetrahydro- 1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, 0.066 mmol, yield 70.90%). ES-API: [M+H]⁺= 907.3.

Step 7: Cesium fluoride (100.46 mg, 0.661 mmol) was added to a solution of tert-butyl (5S,5aS,6S,9R)-1-fluoro-2-(7-fluoro-8-((triisopropylsilyl)ethynyl)naphthalen-1-yl)-12-(((2S)-2-methoxy-6-methylenetetnihydro-11H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, 0.066 mmol) in N,N-dimethylformamide (5 mL). The reaction mixture was stirred at room temperature for 0.5 hours. Upon completion of the reaction (monitored by LCMS), the reaction mixture was diluted with ethyl acetate (15 mL), washed with water (15 mL × 2), and saturated brine (15 mL × 3), dried over anhydrous sodium sulfate, filtered, and concentrated to obtain the crude product tert-butyl (SS,SaS,6S,9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.053 mmol, yield 80.55%). ES-API: [M+H]⁺= 751.3.

Step 8: Under an ice-water bath, a hydrochloric acid-dioxane solution (4M, 5 mL) was slowly added to a solution of tert-butyl (5S,5aS,6S,9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.053 mmol) in acetonitrile (2 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 1) to obtain a white solid, (5S,5aS,6S,9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z692, 15 mg, 0.023 mmol, yield: 43.27%). ES-API: [M+H]⁺= 651.2.

### Example 83 Synthesis of (5S,5aS,6S,9R)-2-(8-ethynyl-7-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene (Z693)

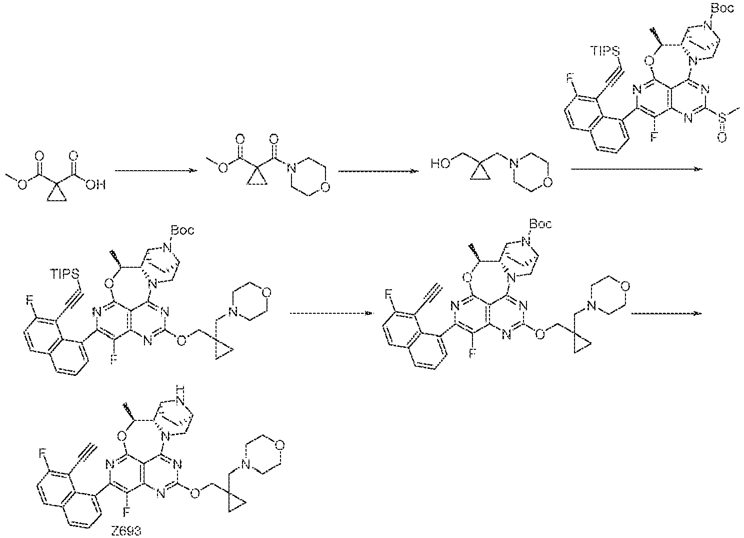

Step 1: At 0°C, oxalyl chloride (6.86 mL, 79.93 mmol) and N,N-dimethylformamide (0.05 mL) were added to a solution of 1-(methoxycarbonyl)cyclopropane-1-carboxylic acid (2.88 g, 19.99 mmol) in dichloromethane (50.0 mL). The reaction mixture was then stirred at 35°C for 3 hours. After 3 hours, the solvent was evaporated under reduced pressure. At 0°C, dichloromethane (80.0 mL), triethylamine (8.33 mL, 59.95 mmol), and morpholine (2.089 g, 23.98 mmol) were added, and the mixture was stirred at room temperature for 3 hours. Upon completion of the reaction, the mixture was filtered, and the filtrate was evaporated under reduced pressure to remove solvent and obtain a crude product, which was then purified by flash silica gel column chromatography (ethyl acetate/petroleum ether: 0%-8%) to obtain methyl 1-(morpholine-4-carbonyl)cyclopropane-1-carboxylate (2.0 g, yield 47%), as a pale yellow viscous liquid. ES-API: [M+H]⁺=214.1.

Step 2: To dry tetrahydrofuran (20.0 mL), methyl 1-(morpholine-4-carbonyl)cyclopropane-1-carboxylate (1.50 g, 7.01 mmol) was added, and the mixture was cooled to 0-5°C under an ice-water bath. Lithium aluminium hydride in tetrahydrofuran solution (14.0 mL, 1M, 14.0 mmol) was then added dropwise. The reaction was proceeded for 1 hour at this temperature, followed by successive addition of water (0.3 mL) and 15% sodium hydroxide solution (0.3 mL). The reaction mixture was filtered through diatomaceous earth, and the filtrate was evaporated under reduced pressure to obtain (1-(morpholinomethyl)cyclopropyl)methanol (2.0 g, yield 60%). ES-API: [M+H]⁺=172.1.

Step 3: (1-(Morpholinomethyl)cyclopropyl)methanol (200 mg, 1.162 mmol) was dissolved in anhydrous tetrahydrofuran (10 mL) under an ice-water bath. Sodium hydride (116.3 mg, 2.907 mmol) was added, and the reaction was proceeded for 30 minutes under an ice-water bath. A tetrahydrofuran (2 mL) solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (408 mg, 0.5188 mmol) was added dropwise to the reaction mixture, and the mixture reacted at 25°C for 30 minutes. Upon completion of the reaction (monitored by LCMS), the reaction mixture was diluted with ethyl acetate (50 mL), washed with saturated ammonium chloride solution (15 mL × 2) and saturated brine (50 mL × 3), dried over anhydrous sodium sulfate, filtered, and the crude product was purified by flash silica gel column chromatography (dichloromethane: methanol = 15:1) to obtain tert-butyl (5S,5aS,6S,9R)-1-fluoro-2-(7-fluoro-8-((triisopropylsilyl)ethynyl)naphthalen-1-yl)-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (200 mg, yield 43%). ES-API: [M+H]⁺= 895.3.

Step 4: Cesium fluoride (679.0 mg, 4.467 mmol) was added to a solution of tert-butyl (SS,SaS,6S,9R)-1-fluoro-2-(7-fluoro-8-((triisopropylsilyl)ethynyl)naphthalen-1-yl)-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (200 mg, 0.223 mmol) in N,N-dimethylformamide (10.0 mL). The reaction mixture was stirred at room temperature for 2 hours. Upon completion of the reaction (monitored by LCMS), the reaction mixture was diluted with ethyl acetate (60 mL), washed with water (30 mL × 2) and saturated brine (50 mL × 3), dried over anhydrous sodium sulfate, filtered, and concentrated to obtain tert-butyl (5S,5aS,6S,9R)-2-(8-ethynyl-7-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (160 mg, yield 96%). ES-API: [M+H]⁺= 739.2.

Step 5: Under an ice-water bath, a 4M hydrochloric acid-dioxane solution (5.0 mL, 4M, 20.0 mmol) was added slowly to a solution of tert-butyl (5S,5aS,6S,9R)-2-(8-ethynyl-7-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (160 mg, 0.217 mmol) in acetonitrile (10.0 mL). The reaction mixture was stirred at 0°C for 0.5 hours. Upon completion of the reaction (monitored by LCMS), the reaction mixture was concentrated under reduced pressure. The residue was purified by preparative HPLC (ammonium bicarbonate method 2) (chromatographic conditions: 15ml-35-85-13min) to obtain a pale yellow solid, (5S,5aS,6S,9R)-2-(8-ethynyl-7-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene (Z693, 27 mg, yield: 19.52 %). ES-API: [M+H]+= 639.2. ¹H NMR (400 MHz, CD₃OD) δ 8.10 -8.05 (m, 2H), 7.66 -7.54 (m, 2H), 7.42 (q, *J* = 9.1 Hz, 1H), 5.42 -5.33 (m, 1H), 4.56 -4.49 (m, 1H), 4.49 -4.43 (m, 1H), 4.34-4.38 (m, 1H), 4.094.07 (m, 1H), 3.77-3.47(m, 7H), 3.21 -3.15(m, 1H), 2.51 -2.34 (m, 6H), 2.07 (m, 1H), 1.88-1.78 (m, 3H), 1.59-1.55(m, 3H), 0.72 -0.69 (m, 2H), 0.52-0.46 (m, 2H).

### Example 84 Synthesis of (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z694)

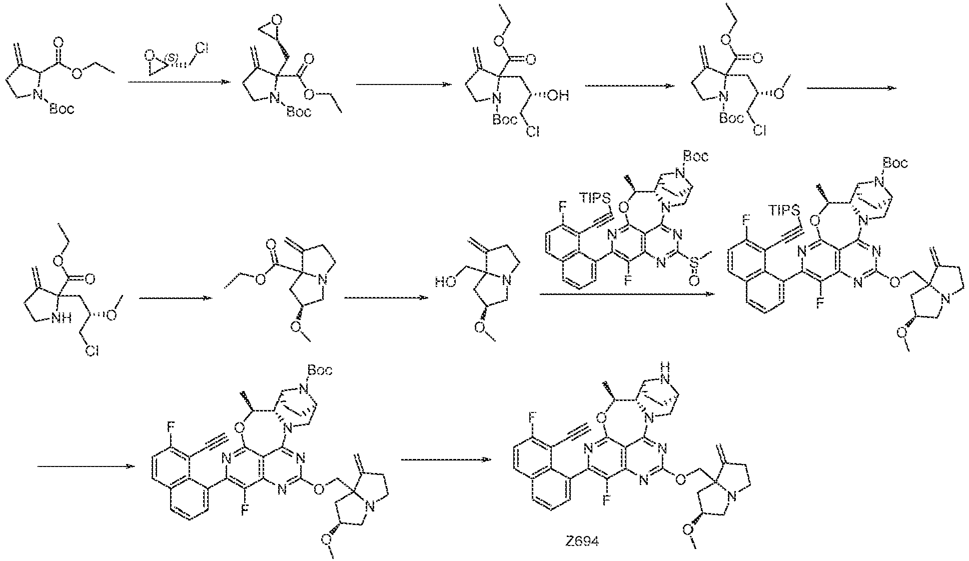

Step 1: At -70°C, bis(trimethylsilyl)aminolithium (4.218 mL, 5.484 mmol) was added to a solution of 1-(tert-butyl) 2-ethyl 3-methylenepyrrolidine-1,2-dicarboxylate (700 mg, 2.742 mmol) in dry tetrahydrofuran (20.0 mL). The mixture was stirred for 1 hour at this temperature. 1 hour later, (R)-epichlorohydrin (761.00 mg, 8.225 mmol) was added to the reaction mixture, which was then stirred overnight at room temperature. Upon completion of the reaction, saturated ammonium chloride solution (30 mL) was added, followed by extraction with ethyl acetate (20 mL × 4). The organic layers were washed with saturated brine (20 mL × 3), dried over anhydrous sodium sulfate, filtered, and the crude product was purified by flash silica gel column chromatography [dichloromethane:methanol=100:0~90:10, (v/v)] to obtain the target compound 1-(tert-butyl) 2-ethyl 3-methylene-2-(((S)-oxiran-2-yl)methyl)pyrrolidine-1,2-dicarboxylate (400 mg, 1.285 mmol, yield 46.85%). ES-API: [M-56+H]⁺=255.9.

Step 2: At room temperature, acetic acid (1 mL, 17.469 mmol) and lithium chloride (1000 mg, 23.590 mmol) were added to a solution of 1-(tert-butyl) 2-ethyl 3-methylene-2-(((S)-oxiran-2-yl)methyl)pyrrolidine-1,2-dicarboxylate (360 mg, 1.156 mmol) in dry tetrahydrofuran (10.0 mL). The mixture was stirred for 12 hours at this temperature. Upon completion of the reaction, saturated ammonium chloride (10 mL) was added and extracted with ethyl acetate (20 mL × 2). The combined organic phases were dried over anhydrous sodium sulfate, filtered, and the solvent of the filtrate was evaporated under reduced pressure to obtain the target compound 1-(tert-butyl) 2-ethyl 2-((S)-3-chloro-2-hydroxypropyl)-3-methylenepyrrolidine-1,2-dicarboxylate (150 mg, 0.431 mmol, yield 37.30%). ES-API: [M-Boc+H]⁺=248.2.

Step 3: 1-(tert-butyl) 2-ethyl 2-((S)-3-chloro-2-hydroxypropyl)-3-methylenepyrrolidine-1,2-dicarboxylate (280 mg, 1.058 mmol) was dissolved in dichloromethane (5 mL), and [8-(dimethylamino)-1-naphthyl]dimethylamine (792.44 mg, 3.703 mmol) and trimethyloxonium tetrafluoroborate (469.34 mg, 3.173 mmol) were added. The reaction was proceeded overnight at room temperature. Upon completion of the reaction, saturated ammonium chloride (10 mL) solution was added, and the mixture was extracted with dichloromethane (20 mL × 4). The organic layers were washed with saturated brine (10 mL × 3), dried over anhydrous sodium sulfate, filtered, concentrated, and the crude product was purified by flash silica gel column chromatography [dichloromethane:methanol=100:0~90:10,(v/v)] to obtain the target compound 1-(tert-butyl) 2-ethyl 2-((S)-3-chloro-2-methoxypropyl)-3-methylenepyrrolidine-1,2-dicarboxylate (180 mg, 0.646 mmol, yield 61.05%). ES-API: [M-Boc+H]⁺=262.1.

Step 4: 1-(tert-butyl) 2-ethyl 2-((S)-3-chloro-2-methoxypropyl)-3-methylenepyrrolidine-1,2-dicarboxylate (100 mg, 0.276 mmol) was dissolved in acetonitrile (1 mL) and hydrochloric acid dioxane solution (2 mL, 0.415 mmol) was added. The reaction was proceeded for 1 hour at room temperature, and the reaction mixture was concentrated under reduced pressure to obtain ethyl 2-((S)-3-chloro-2-methoxypropyl)-3-methylenepyrrolidine-2-carboxylate (50 mg, 0.191 mmol, yield 69.12%).

Step 5: Sodium bicarbonate (100 mg, 1.190 mmol) was added to a solution of ethyl 2-((S)-3-chloro-2-methoxypropyl)-3-methylenepyrrolidine-2-carboxylate (45 mg, 0.172 mmol) in acetonitrile (3.0 mL), and the reaction was proceeded at 70°C for 0.5 hours. Upon completion of the reaction, the mixture was cooled to room temperature, filtered, and the solvent of the filtrate was evaporated under reduced pressure. The crude product was purified by flash silica gel column chromatography (methanol/dichloromethane: 0%-10%) to obtain ethyl (6S)-6-methoxy-1-methylenetetrahydro-1H-pyrrolizine-7a(5H)-carboxylate (35 mg, 0.155 mmol, yield 90.37%). ES-API: [M+H]⁺=226.1. Step 6: Ethyl (6S)-6-methoxy-1-methylenetetrahydro-1H-pyrrolizine-7a(5H)-carboxylate (30 mg, 0.133 mmol) was dissolved in tetrahydrofuran (2 mL) and cooled to 0°C. Under nitrogen atmosphere, a solution of lithium aluminium hydride (0.399 mL, 0.399 mmol) was added. The reaction mixture was stirred at 0°C for 1 hour. Sodium sulfate decahydrate (500 mg) was added, and the mixture was dried over anhydrous sodium sulfate, filtered, and the filtrate was concentrated to obtain ((6S)-6-methoxy-1-methylenetetrahydro-1H-pyrrolizin-7a(5H)-yl)methanol (22 mg, 0.120 mmol, yield 90.16%), a colorless and transparent liquid. ES-API: [M +H]⁺=184.1.

Step 7: ((6S)-6-methoxy-1-methylenetetrahydro-1H-pyrrolizin-7a(5H)-yl)methanol (16.28 mg, 0.089 mmol) was dissolved in anhydrous tetrahydrofuran (2 mL), and under an ice water bath, sodium hydride (1.07 mg, 0.044 mmol) was added. The reaction was proceeded for 30 minutes under ice water bath, and a solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (35 mg, 0.044 mmol) in tetrahydrofuran (2 mL) was added dropwise to the reaction mixture, and the reaction was proceeded at room temperature for 30 minutes. Upon completion of the reaction (monitored by LCMS), the reaction mixture was diluted with ethyl acetate (10 mL), washed with saturated ammonium chloride solution (5 mL × 2), saturated brine (50 mL × 3), dried over anhydrous sodium sulfate, filtered, concentrated, and the crude product was purified by flash silica gel column chromatography (dichloromethane:methanol=15: 1) to obtain tert-butyl (5S,5aS,6S,9R)-1-fluoro-2-(7-fluoro-8-((triisopropylsilyl)ethynyl)naphthalen-1-yl)-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (32 mg, 0.035 mmol, yield 79.42%). ES-API: [M+H]⁺= 907.3.

Step 8: Cesium fluoride (33.49 mg, 0.220 mmol) was added to a solution of tert-butyl (5S,6aS,6S,9R)-1-fluoro-2-(7-fluoro-8-((triisopropylsilyl)ethynyl)naphthalen-1-yl)-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, 0.022 mmol) in N,N-dimethylformamide (1.0 mL). The reaction mixture was stirred at room temperature for 2 hours. Upon completion of the reaction (monitored by LCMS), the reaction mixture was diluted with ethyl acetate (10 mL), washed with water (10 mL × 2), saturated brine (10 mL x 3), dried over anhydrous sodium sulfate, filtered, and concentrated to obtain tert-butyl (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (15 mg, 0.020 mmol, yield 90.81%). ES-API: [M+H]⁺= 751.3.

Step 9: Under an ice water bath, 4 M hydrochloric acid-dioxane solution (1.0 mL) was slowly added to a solution of tert-butyl (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (13 mg, 0.017 mmol) in acetonitrile (1.0 mL). The reaction mixture was stirred at 0°C for 0.5 hours. Upon completion of the reaction (monitored by LCMS), the reaction mixture was concentrated under reduced pressure, and the residue was purified by preparative HPLC (ammonium bicarbonate method 2) (chromatographic conditions: 15ml-35-85-13min) to obtain a pale yellow solid, (5S,5aS,6S,9R)-2-(8-ethynyl-7-fluoronaphthalen-1-yl)-1-fluoro-12-(((6S)-6-methoxy-l-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z694, 5 mg, 0.008 mmol, yield: 44.38%). ES-API: [M+H]⁺= 651.3.

### Example 85 Synthesis of ((5R,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-5-yl)methanol (Z654)

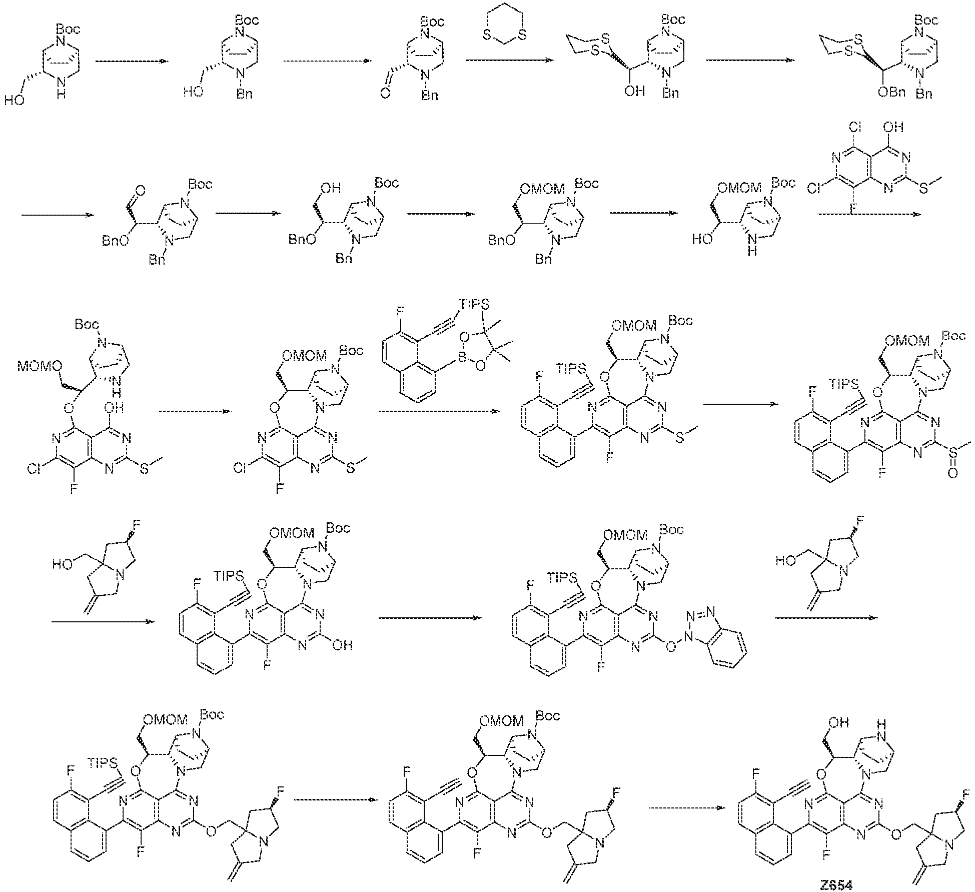

Step 1: Tert-butyl (1R, 2S, SS)-2-(hydroxymethyl)-3,8-diazabicyclo[3.2.1]octane-8-carboxylate (800 mg, 3.301 mmol) was dissolved in acetonitrile (20 mL). At room temperature, potassium carbonate (685 mg, 4.952 mmol) and benzyl bromide (0.589 mL, 4.952 mmol) were successively added to the reaction mixture, which was then stirred overnight at room temperature. Upon completion of the reaction (monitored by LCMS), water (30 mL) was added to quench the reaction, followed by extraction with ethyl acetate (30 mL × 3). The organic phase was combined, washed with saturated brine (20 mL × 2), dried over anhydrous sodium sulfate, filtered, concentrated, and the crude product was purified through silica gel using fast chromatography (tetrahydrofuran:petroleum ether = 0-30%) to obtain tert-butyl (1R, 2S, 5S)-3-benzyl-2-(hydroxymethyl)-3,8-diazabicyclo[3.2.1]octane-8-carboxylate (1 g, yield: 91%). ES-API: [M+H]⁺= 333.3.

Step 2: Under an ice water bath, tert-butyl (1R, 2S, 5S)-3-benzyl-2-(hydroxymethyl)-3,8-diazabicyclo[3.2.1]octane-8-carboxylate (1 g, 3.008 mmol) was dissolved in dichloromethane (50 mL). To this solution, Dess-Martin periodinane (1.66 g, 3.910 mmol) was added, and the reaction mixture was stirred at 0°C for 1 hour. Upon completion of the reaction (monitored by LCMS), the solution was diluted with dichloromethane (30 mL) and quenched with saturated sodium bisulfite solution (30 mL). The organic phase was washed with saturated sodium bicarbonate solution (20 mL × 2), dried over anhydrous sodium sulfate, filtered, and concentrated to obtain tert-butyl (1R, 2S, 5S)-3-benzyl-2-formyl-3,8-diazabicyclo[3.2.1]octane-8-carboxylate (1 g, yield: 100.6%). ES-API: [M+H]⁺= 331.1.

Step 3: Under nitrogen protection, at -20°C, 1,3-dithiane (728 mg, 6.053 mmol) was dissolved in anhydrous tetrahydrofuran (30 mL). Slowly, n-butyllithium (1.816 mL, 4.540 mmol) was added, and the reaction mixture was stirred at -20°C for 0.5 hours. The reaction mixture was then cooled to - 78°C in a dry ice-acetone bath, and slowly, sec-butyllithium (17.5 mL, 22.816 mmol, 1.3 M in hexane) was added. The reaction mixture was stirred at -78°C for 1 hour. Then, a solution of tert-butyl (1R, 2S, 5S)-3-benzyl-2-formyl-3,8-diazabicyclo[3.2.1]octane-8-carboxylate (1 g, 3.026 mmol) in tetrahydrofuran (10 mL) was added, and the reaction mixture was gradually warmed to room temperature and stirred for 1 hour. Upon completion of the reaction (monitored by LCMS), the reaction mixture was quenched by successively adding saturated ammonium chloride solution (50 mL), extracted with ethyl acetate (30 mL × 3), and the organic phases were combined, washed with saturated brine (15 mL × 2), dried over anhydrous sodium sulfate, filtered, and concentrated to obtain tert-butyl (1R, 2S, 5S)-2-((R)-(1,3-dithiane-2-yl)(hydroxy)methyl)-3-benzyl-3,8-diazabicyclo[3.2.1]octane-8-carboxylate (1352 mg). ES-API: [M+ H]⁺= 451.1.

Step 4: Under an ice water bath, tert-butyl (1R, 2S, 5S)-2-((R)-(1,3-dithiane-2-yl)(hydroxy)methyl)-3-benzyl-3,8-diazabicyclo[3.2.1]octane-8-carboxylate (1352 mg, 3.000 mmol) was dissolved in N,N-dimethylformamide (10 mL). At 0°C, sodium hydride (240 mg, 6.000 mmol) was added to the reaction mixture and stirred at this temperature for 10 minutes, followed by the addition of benzyl bromide (0.714 mL, 6.000 mmol). The reaction mixture was stirred at 0°C for 0.5 hours, then warmed to room temperature and stirred for another 0.5 hours. Upon completion of the reaction (monitored by LCMS), 30 mL of water was added to quench the reaction, followed by extraction with ethyl acetate (30 mL × 3). The organic phase was combined, washed with saturated brine (20 mL × 2), dried over anhydrous sodium sulfate, filtered, concentrated, and the crude product was purified through silica gel using fast chromatography (tetrahydrofuran:petroleum ether = 0~15%) to obtain tert-butyl (1R, 2S, 5S)-3-benzyl-2-((R)-(benzyloxy)(1,3-dithiane-2-yl)methyl)-3,8-diazabicyclo[3.2.1]octane-8-carboxylate (974 mg, yield: 60%). ES-API: [M+ H]⁺= 541.1.

Step 5: At -10°C, to a solution of N-bromosuccinimide (3.087 g, 17.345 mmol) in acetonitrile (16 mL) and water (4 mL), a solution of tert-butyl (1R, 2S, 5S)-3-benzyl-2-((R)-(benzyloxy)(1,3-dithiane-2-yl)methyl)-3,8-diazabicyclo[3.2.1]octane-8-carboxylate (938 mg, 1.735 mmol) in acetonitrile (10 mL) was slowly added dropwise. The reaction mixture was stirred at -10°C for 0.5 hours. Upon completion of the reaction (monitored by LCMS), saturated sodium bisulfite solution (30 mL) was added to quench the reaction, followed by extraction with dichloromethane (20 mL × 3). The organic phase was combined, washed with saturated brine (15 mL × 2), dried over anhydrous sodium sulfate, filtered, and concentrated to obtain tert-butyl (1S, 2S, 5R)-3-benzyl-2-((R)-1-(benzyloxy)-2-oxyethyl)-3,8-diazabicyclo[3.2.1]octane-8-carboxylate (781 mg). ES-API: [M+ H]⁺= 451.2.

Step 6: Under nitrogen protection and an ice water bath, to a solution of tert-butyl (1S, 2S, 5R)-3-benzyl-2-((R)-1-(benzyloxy)-2-oxyethyl)-3,8-diazabicyclo[3.2.1]octane-8-carboxylate (766 mg, 1.7 mmol) in tetrahydrofuran (30 mL), a solution of borane in tetrahydrofuran (8.5 mL, 8.5 mmol) was slowly added dropwise. The reaction mixture was stirred at 0°C for 0.5 hours, then gradually warmed to room temperature and stirred for another 0.5 hours. Upon completion of the reaction (monitored by LCMS), the reaction mixture was cooled to 0°C, and methanol was slowly added to quench the reaction. After stirred at room temperature for 0.5 hours, the reaction mixture was concentrated under reduced pressure to obtain tert-butyl (1S, 2S, 5R)-3-benzyl-2-((R)-1-(benzyloxy)-2-hydroxyethyl)-3,8-diazabicyclo[3.2.1]octane-8-carboxylate (768 mg). ES-API: [M+ H]⁺= 453.3.

Step 7: Under an ice water bath, N,N-diisopropylethylamine (0.162 mL, 0.928 mmol) was added to a solution of tert-butyl (1S, 2S, 5R)-3-benzyl-2-((R)-1-(benzyloxy)-2-hydroxyethyl)-3,8-diazabicyclo[3.2.1]octane-8-carboxylate (210 mg, 0.464 mmol) in dichloromethane (10 mL). The reaction mixture was stirred at 0°C for 0.5 hours. Bromomethyl methyl ether (0.057 mL, 0.696 mmol) was then added to the reaction mixture, which continued to be stirred at 0°C for 2.5 hours. Upon completion of the reaction (monitored by LCMS), the reaction mixture was concentrated and the crude product was purified through silica gel using fast chromatography (tetrahydrofuran:petroleum ether = 0-10%) to obtain tert-butyl (1S, 2S, 5R)-3-benzyl-2-((R)-1-(benzyloxy)-2-(methoxymethoxy)ethyl)-3,8-diazabicyclo[3.2.1]octane-8-carboxylate (185 mg, yield: 80%). ES-API: [M+ H]⁺= 497.4.

Step 8: Under hydrogen protection, to a solution of tert-butyl (1S, 2S, 5R)-3-benzyl-2-((R)-1-(benzyloxy)-2-(methoxymethoxy)ethyl)-3,8-diazabicyclo[3.2.1]octane-8-carboxylate (61 mg, 0.123 mmol) in 2,2,2-trifluoroethanol (2 mL), a catalytic amount of acetic acid and 10% palladium on carbon (53 mg) were successively added. The reaction mixture was stirred at room temperature overnight. Upon completion of the reaction (monitored by LCMS), the mixture was filtered through diatomaceous earth, concentrated under reduced pressure to obtain crude tert-butyl (1S, 2S, 5R)-2-((R)-1-hydroxy-2-(methoxymethoxy)ethyl)-3,8-diazabicyclo[3.2.1]octane-8-carboxylate (51 mg). ES-API: [M+ H]⁺= 317.3.

Step 9: Under an ice water bath, tert-butyl (1S, 2S, 5R)-2-((R)-1-hydroxy-2-(methoxymethoxy)ethyl)-3,8-diazabicyclo[3.2.1]octane-8-carboxylate (76 mg, 0.240 mmol) was dissolved in tetrahydrofuran (10 mL). Sodium hydride (48 mg, 1.200 mmol, 60% dispersed in oil) was added to the reaction mixture, which was then stirred at 0°C for 30 minutes. Then, at 0°C, 5,7-dichloro-8-fluoro-2-(methylthio)pyrido[4,3-d]pyrimidin-4-ol (202 mg, 0.720 mmol) was added to the reaction mixture, which was then gradually warmed to 60°C and stirred for 1.5 hours. Upon completion of the reaction (monitored by LCMS), saturated ammonium chloride solution (20 mL) was slowly added to the reaction mixture, which was then extracted with dichloromethane (20 mL × 3). The organic phase was dried over anhydrous sodium sulfate, filtered, concentrated to obtain tert-butyl (1S, 2S, 5R)-2-((R)-1-(7-chloro-8-fluoro-4-hydroxy-2-(methylthio)pyrido[4,3-d]pyrimidin-5-yl)oxy)-2-(methoxymethoxy)ethyl)-3,8-diazabicyclo[3.2.1]octane-8-carboxylate (129 mg, yield: 96%). ES-API: [M+ H]⁺= 560.2.

Step 10: Under an ice water bath, 2-(7-azabenzotriazole)-N,N,N',N'-tetramethyluronium hexafluorophosphate (162 mg, 0.425 mmol) was added to a dichloromethane (10 mL) solution of tert-butyl (1S,2S,5R)-2-((R)-1-((7-chloro-8-fluoro-4-hydroxy-2-(methylthio)pyrido[4,3-d]pyrimidin-5-yl)oxy)-2-(methoxymethoxy)ethyl)-3,8-diazabicyclo[3.2.1]octane-8-carboxylate (119 mg, 0.212 mmol) and N,N-diisopropylethylamine (0.15 mL, 0.850 mmol). The reaction mixture was stirred at 0°C for 0.5 hours and then warmed to room temperature and stirred for 1.5 hours. Upon completion of the reaction (monitored by LCMS), the reaction mixture was concentrated, and the crude product was purified through silica gel using fast chromatography (ethyl acetate:petroleum ether = 0-20%) to obtain tert-butyl (SR,SaS,6S,9R)-2-chloro-1-fluoro-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (79 mg, yield: 69%). ES-API: [M+ H]⁺= 542.2.

Step 11: Under nitrogen protection, tert-butyl (SR,SaS,6S,9R)-2-chloro-1-fluoro-5-((methoxymethoxy)methyl)-12-(methylthio)-5a,6,7,8,9,10-hexahydro-5H-4-oxa-3,10a,1,13,14-pentaaza-6,9-methanonaphtho[1,8-ab]heptalene-14-carboxylate (79 mg, 0.146 mmol), ((2-fluoro-8-(4,4,5,5-tetramethyl-1,3,2-dioxaborolane-2-yl)naphthalen-1-yl)ethynyl)triisopropylsilane (132 mg, 0.292 mmol), potassium phosphate (93 mg, 0.438 mmol), and butyldi(1-adamantyl)phosphine (2-amino-1,1'-biphenyl)palladium(II) methanesulfonate (16 mg, 0.022 mmol) were dissolved in tetrahydrofuran (5 mL) and water (1 mL). The reaction mixture was stirred at 80°C for 0.5 hours in a microwave reactor. Upon completion of the reaction (monitored by LCMS), the reaction mixture was concentrated, and the crude product was purified through silica gel (petroleum ether/ethyl acetate = 0-20%) to obtain tert-butyl (SR,SaS,6S,9R)-1-fluoro-2-(7-fluoro-8-((triisopropylsilyl)ethynyl)naphthalen-1-yl)-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (79 mg, yield: 65%). ES-API: [M+ H]⁺= 832.3.

Step 12: tert-butyl (5R,5aS,6S,9R)-1-fluoro-2-(7-fluoro-8-((triisopropylsilyl)ethynyl)naphthalen-1-yl)-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]heptalene-14-carboxylate (79 mg, 0.095 mmol) was dissolved in anhydrous dichloromethane (10 mL). M-chloroperbenzoic acid (16 mg, 0.143 mmol) was added to the solution, and the reaction mixture was stirred at room temperature for half an hour. Upon completion of the reaction (monitored by LCMS), the reaction mixture was diluted with dichloromethane (15 mL), quenched with saturated sodium bisulfite solution (15 mL), then washed with bicarbonate solution (15 mL) and saturated brine (15 mL). The organic phase was dried over anhydrous sodium sulfate, filtered, concentrated to obtain crude tert-butyl (5R,5aS,6S,9R)-1 -fluoro-2-(7-fluoro-8-((triisopropylsilyl)ethynyl)naphthalen-1 -yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (81 mg). ES-API: [M+ H]⁺= 848.3.

Step 13: ((2R)-2-fluoro-6-methylenetetrahydro-1H-pyrrolizin-7a(5H)-yl)methanol (33 mg, 0.190 mmol) was dissolved in anhydrous tetrahydrofuran (5 mL). Under an ice water bath, sodium hydride (11 mg, 0.285 mmol) was added, and the mixture reacted for 10 minutes. A tetrahydrofuran (5 mL) solution of tert-butyl (5R,5aS,6S,9R)-1-fluoro-2-(7-fluoro-8-((triisopropylsilyl)ethynyl)naphthalen-1-yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (81 mg, 0.095 mmol) was added dropwise to the reaction mixture, which was then reacted at 0°C for 30 minutes. The reaction was monitored by LCMS, the reactants disappeared, with the majority being hydrolysis products. The reaction mixture was diluted with ethyl acetate (10 mL), washed with saturated ammonium chloride solution (10 mL × 2) and saturated brine (10 mL × 3), dried over anhydrous sodium sulfate, filtered, and concentrated. The crude product was purified through silica gel using fast chromatography (methanol/dichloromethane = 0~10%) to obtain tert-butyl (5R,5aS,6S,9R)-1-fluoro-2-(7-fluoro-8-((triisopropylsilyl)ethynyl)naphthalen-1-yl)-12-hydroxy-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (70 mg, yield: 92%). ES-API: [M+H]⁺= 802.3.

Step 14: Carter cycloaddition catalyst (78 mg, 0.174 mmol) was added to tert-butyl (5R,5aS,6S,9R)-1 -fluoro-2-(7-fluoro-8-((triisopropylsilyl)ethynyl)naphthalen-1 -yl)-12-hydroxy-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (70 mg, 0.087 mmol), cesium carbonate (57 mg, 0.174 mmol), and tetrahydrofuran (10 mL). The reaction mixture was stirred at room temperature for 1 hour. The reaction mixture was quenched with water (20 mL) and extracted with ethyl acetate (20 mL × 2). The organic phase was washed with saturated brine (10 mL), dried over anhydrous sodium sulfate, filtered, and concentrated. The crude product was purified through silica gel using fast chromatography (ethyl acetate/petroleum ether = 0-50%) to obtain tert-butyl (5R,5aS,6S,9R)-12-((1H-benzo[d][1,2,3]triazol-1-yl)oxy)-1-fluoro-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (71 mg, yield: 89%). ES-API: [M+H]⁺= 919.4.

Step 15: ((2R)-2-fluoro-6-methylenetetrahydro-1H-pyrrolizin-7a(5H)-yl)methanol (13 mg, 0.116 mmol) was dissolved in anhydrous tetrahydrofuran (5 mL). Under an ice water bath, sodium hydride (6 mg, 0.155 mmol) was added, and the mixture reacted for 10 minutes. A tetrahydrofuran (5 mL) solution of tert-butyl (5R,5aS,6S,9R)-12-((1H-benzo[d][1,2,3]triazol-1-yl)oxy)-1-fluoro-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (71 mg, 0.077 mmol) was added dropwise to the reaction mixture, which was then reacted at 0°C for 30 minutes. The reaction was monitored by LCMS, the reactants disappeared, with the majority being hydrolysis products. The reaction mixture was diluted with ethyl acetate (10 mL), washed with saturated ammonium chloride solution (10 mL × 2) and saturated brine (10 mL × 3), dried over anhydrous sodium sulfate, filtered, and concentrated. The crude product was purified through silica gel using fast chromatography (methanol/dichloromethane = 0~3%) to obtain tert-butyl (5R,5aS,6S,9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (23 mg, yield: 31%). ES-API: [M+ H]⁺= 955.4.

Step 16: Cesium fluoride (37 mg, 0.241 mmol) was added to N,N-dimethylformamide (2 mL) containing tert-butyl (SR,SaS,bS,9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (23 mg, 0.024 mmol). The reaction mixture was stirred at room temperature for 30 minutes. Upon completion of the reaction (monitored by LCMS). The reaction mixture was diluted with ethyl acetate (15 mL), washed with water (15 mL × 2) and saturated brine (15 mL × 3), dried over anhydrous sodium sulfate, filtered, and concentrated to obtain crude product tert-butyl (SR,SaS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-((methoxymethoxy)methyl)-5a,6,7,8,9,10-hexahydro-5H-4-oxa-3,10a,11,13,14-pentaaza-6,9-methanonaphtho[1,8-ab]heptalene-14-carboxylate (19 mg). ES-API: [M+ H]⁺= 799.3.

Step 17: 4M Hydrochloric acid-dioxane solution (2 mL) was slowly added to an acetonitrile (2 mL) solution of tert-butyl (5R,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-((methoxymethoxy)methyl)-5,6,7,8,9,10-hexahydro-5H-4-oxa-3,10A,11,13,14-pentaaza-6,9-methanonaphtho[1,8-ab]heptalene-14-carboxylate (19 mg, 0.024 mmol), and the mixture was stirred at 0°C for 1 hour. Upon completion of the reaction (monitored by LCMS). The reaction mixture was concentrated under reduced pressure to dryness, and the residue was purified by preparative HPLC (bicarbonate method 2) (chromatographic conditions: 15mL-40-60-13 min) to obtain a white solid, ((5R,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-5-yl)methanol (Z654, 6.65 mg, yield: 42 %). ES-API: [M+H]⁺= 655.2. ¹H NMR (400 MHz, CD₃OD) δ 8.12-8.07 (m, 2 H), 7.68-7.56 (m, 2 H), 7.47-7.40 (m, 1 H), 5.44-5.24 (m, 2 H), 5.02-5.00 (m, 2 H), 4.39 (s, 2 H), 4.36 (d, J= 3.0 Hz, 1 H), 4.27 (d, J= 13.0 Hz, 1 H), 4.07-4.02 (m, 1 H), 3.95-3.89 (m, 1 H), 3.80-3.53 (m, 4 H), 3.48-3.37 (m, 1 H), 3.21-3.11 (m, 2 H), 2.98-2.83 (m, 2 H), 2.54-2.44 (m, 2 H), 2.21-2.02 (m, 2 H), 1.88-1.77 (m, 3 H), 1.37-1.24 (m, 2 H).

### Example 86 Synthesis of (5S,5aS,6S,9R)-2-(8-ethynyl-7-fluoronaphthalen-1-yl)-1-fluoro-12-(((1S,7aS)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z695) and (5S,5aS,6S,9R)-2-(8-ethynyl-7-fluoronaphthalen-1-yl)-1-fluoro-12-(((1S,7aR)-1-methoay-6-methylenetetrahydro-1H-pyrrolizin-7a(5H)-yl)methory)-5-methyl-5a,6,7,8,9,10-hexahydro-5H-4-oxa-3,10a,11,13,14-pentaaza-6,9-methanonaphtho[1,8-ab]heptalene (Z696)

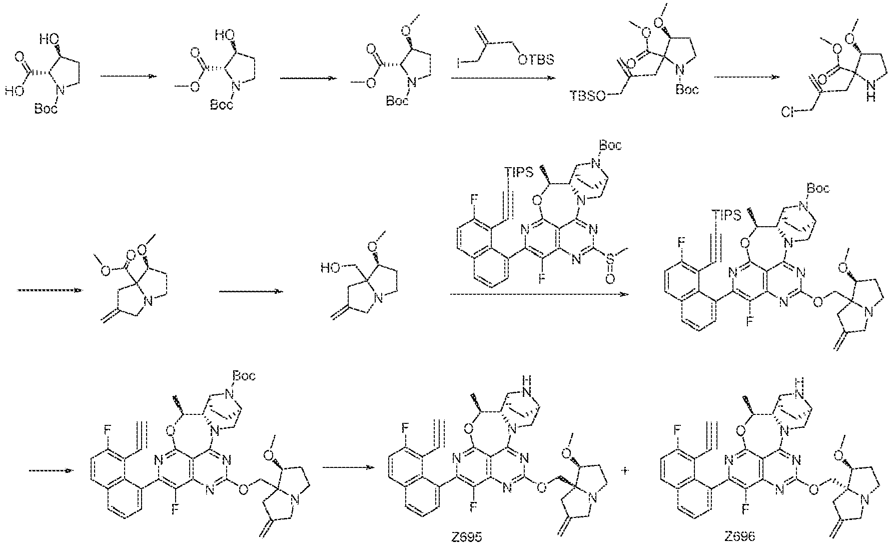

Step 1: Under an ice-water bath, potassium carbonate (1.79 g, 12.9 mmol) and iodomethane (1.84 g, 12.9 mmol) were added to a solution of (2S, 3S)-1-(tert-butoxycarbonyl)-3-hydroxypyrrolidine-2-carboxylic acid (1 g, 4.3 mmol) in N,N-dimethylfonnamide (10 mL). The mixture was stirred at room temperature for 2 hours. Upon completion of the reaction, as monitored by LCMS, the mixture was filtered and washed with ethyl acetate. The filtrate was washed with water (15 mL × 3) and aturated brine (15 mL × 3). After dried over anhydrous sodium sulfate, filtration, and concentration under reduced pressure, the crude product was purified by flash silica gel column chromatography (petroleum ether: ethyl acetate = 10:1) to obtain compound 1-(tert-butyl)-2-methyl-(2S,3S)-3-hydroxypyrrolidine-1,2-dicarboxylate (1.01 g, yield: 94%). ES-API: [M-100]'= 190.1.

Step 2: Under nitrogen protection, silver oxide (9.45 g, 40.8 mmol) and iodomethane (5.79 g, 40.8 mmol) were added to a solution of 1-(tert-butyl)-2-methyl-(2S,3S)-3-hydroxypyrrolidine-1,2-dicarboxylate (1.01 g, 4.08 mmol) in acetonitrile (10 mL). The mixture was stirred at room temperature for 48 hours. Upon completion of the reaction, as monitored by LCMS, the mixture was filtered and washed with ethyl acetate. The filtrate was concentrated under reduced pressure, and the crude product was purified by flash silica gel column chromatography (petroleum ether: ethyl acetate = 15:1) to obtain 1-(tert-butyl)-2-methyl-(2S, 3S)-3-methoxypyrrolidine-1,2-dicarboxylate (1.01 g, yield: 95%). ES-API: [M-100]⁺= 160.1.

Step 3: Under nitrogen protection, 1-(tert-butyl)-2-methyl-(2S, 3S)-3-methoxypyrrolidine-1,2-dicarboxylate (900 mg, 3.47 mmol) was dissolved in anhydrous tetrahydrofuran (10 mL). The solution was cooled to -78°C, and a solution of bis(trimethylsilyl)amine lithium (6.9 mL, 6.9 mmol, 1 M in tetrahydrofuran) was slowly added. The mixture was stirred at this temperature for 1 hour. A solution of tert-butyldimethylsilyl (2-(iodomethyl)vinyl) ether (2.17 g, 6.9 mmol) in anhydrous tetrahydrofuran (10 mL) was then slowly added via syringe. The reaction mixture was warm to room temperature and continued to be stirred for 2 hours. Upon completion of the reaction, as monitored by LCMS, the reaction was quenched with saturated ammonium chloride solution (50 mL), and the mixture was extracted with ethyl acetate (50 mL × 3). The organic phase was washed with saturated brine (50 mL × 3), dried over anhydrous sodium sulfate, filtered, and concentrated. The crude product was purified by flash silica gel column chromatography (petroleum ether: ethyl acetate = 20:1) to obtain 1-(tert-butyl) 2-methyl (3S)-2-(2-(((tert-butyldimethylsilyl)oxy)methyl)allyl)-3-methoxypyrrolidine-1,2-dicarboxylate (1.0 g, yield: 65 %). ES-API: [M-55]⁺= 388.2.

Step 4: At room temperature, thionyl chloride (15 mL) was slowly added to a solution of 1-(tert-butyl) 2-methyl (3S)-2-(2-(((tert-butyldimethylsilyl)oxy)methyl)allyl)-3-methoxypyrrolidine-1,2-dicarboxylate (1.0 g, 2.25 mmol) in dichloromethane (7 mL). The mixture was stirred overnight at room temperature. Upon completion of the reaction, as monitored by LCMS, the reaction mixture was concentrated, and the crude product was purified by flash silica gel column chromatography (petroleum ether: ethyl acetate = 10:1) to obtain methyl (3S)-2-(2-(chloromethyl)allyl)-3-methoxypyrrolidine-2-carboxylate (400 mg, yield: 71.6%). ES-API: [M+H]⁺= 248.1.

Step 5: At room temperature, sodium bicarbonate (678 mg, 8.0 mmol) and potassium iodide (26.8 mg, 0.164 mmol) were added to a solution of methyl (3S)-2-(2-(chloromethyl)allyl)-3-methoxypyrrolidine-2-carboxylate (400 mg, 1.6 mmol) in acetonitrile (10 mL). The mixture was stirred for 1.5 hours at room temperature. Upon completion of the reaction, as monitored by LCMS, the mixture was filtered and washed with acetonitrile. The filtrate was concentrated under reduced pressure, and the crude product was purified by flash silica gel column chromatography (dichloromethane: methanol = 15:1) to obtain methyl (1S)-1-methoxy-6-methylenetetrahydro-1H-pyrrolizine-7a(5H)-carboxylate (300 mg, yield: 88%). ES-API: [M+H]⁺=182.2.

Step 6: Under nitrogen protection, methyl (1S)-1-methoxy-6-methylenetetrahydro-1H-pyrrolizine-7a(5H)-carboxylate (300 mg, 1.42 mmol) was dissolved in anhydrous tetrahydrofuran (5 mL). The mixture was placed in an ice-water bath, and a solution of lithium aluminum hydride (2.84 mL, 2.84 mmol, 1 M in tetrahydrofuran) was slowly added. The mixture was stirred at this temperature for 0.5 hours. Upon completion of the reaction, as monitored by LCMS, sodium sulfate decahydrate (500 mg) was added to quench the reaction, and the mixture was stirred at room temperature for 0.5 hours and then filtered. The filtrate was concentrated under reduced pressure to obtain ((1S)-1-methoxy-6-methylenetetrahydro-1H-pyrrolizin-7a(5H)-yl)methanol (170 mg, yield: 65%). ES-API: [M+H]⁺= 184.1.

Step 7: ((1S)-1-methoxy-6-methylenetetrahydro-1H-pyrrolizin-7a(5H)-yl)methanol (48 mg, 0.26 mmol) was dissolved in anhydrous tetrahydrofuran (5 mL). Under an ice-water bath, sodium hydride (21 mg, 0.52 mmol) was added, and the reaction was proceeded under the ice-water bath for 30 minutes. A solution of tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (100 mg, 0.13 mmol) in tetrahydrofuran (2 mL) was slowly added dropwise to the reaction mixture, which the reacted at room temperature for 30 minutes. Upon completion of the reaction, monitored by LCMS, the reaction mixture was diluted with ethyl acetate (20 mL), washed with saturated ammonium chloride solution (15 mL × 2) and saturated brine (15 mL × 3), dried over anhydrous sodium sulfate, filtered, and the crude product was purified by automated flash chromatography (dichloromethane: methanol = 15:1 as the mobile phase) through silica gel to obtain tert-butyl (5S,5aS,6S,9R)-1-fluoro-2-(7-fluoro-8-((triisopropylsilyl)ethynyl)naphthalen-1-yl)-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, yield: 17%). ES-API: [M+H]⁺= 907.2.

Step 8: Cesium fluoride (34 mg, 0.22 mmol) was added to N,N-dimethylformamide (1.5 mL) containing tert-butyl (5S,5aS,6S,9R)-1-fluoro-2-(7-fluoro-8-((triisopropylsilyl)ethynyl)naphthalen-1-yl)-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (20 mg, 0.022 mmol). The reaction mixture was stirred at room temperature for 2 hours. Upon completion of the reaction, monitored by LCMS, the reaction mixture was diluted with ethyl acetate (20 mL), washed with water (15 mL × 2) and saturated brine (15 mL × 3), dried over anhydrous sodium sulfate, filtered, and concentrated to obtain crude tert-butyl (5S,5aS,6S,9R)-2-(8-ethynyl-7-fluoronaphthalen-1-yl)-1-fluoro-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (16 mg, yield: 97%). ES-API: [M+H]⁺= 751.2.

Step 9: Under an ice-water bath, a 4 M hydrochloric acid-dioxane solution (5 mL) was slowly added to a solution of tert-butyl (5S,5aS,6S,9R)-2-(8-ethynyl-7-fluoronaphthalen-1-yl)-1-fluoro-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (16 mg, 0.021 mmol) in acetonitrile (1 mL). The reaction mixture was stirred at 0°C for 0.5 hours. Upon completion of the reaction, monitored by LCMS, the reaction mixture was concentrated under reduced pressure to dryness. The residue was purified by preparative HPLC (bicarbonate method 2) (chromatographic conditions: 15ml-35-85-13min) to obtain two isomeric compounds, one of the isomeric compounds was arbitrarily designated as: (5S,5aS,6S,9R)-2-(8-ethynyl-7-fluoronaphthalen-1-yl)-1-fluoro-12-(((1S,7aS)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z695, 1.2 mg, yield: 8.8% , peak 1, retention time: 5.86min). ES-API: [M+H]⁺= 651.2. The other was arbitrarily designated as: (5S,5aS,6S,9R)-2-(8-ethynyl-7-fluoronaphthalen-1-yl)-1-fluoro-12-(((1S,7aR)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z696, 0.8 mg, yield: 5.9%, peak 2, retention time: 6.87min). ES-API: [M+H]⁺= 651.2.

### Example 87 Synthesis of 5-(2,2-dimethylcyclopropyl)-6-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)-4-methylpyridin-2-amine (Z697)

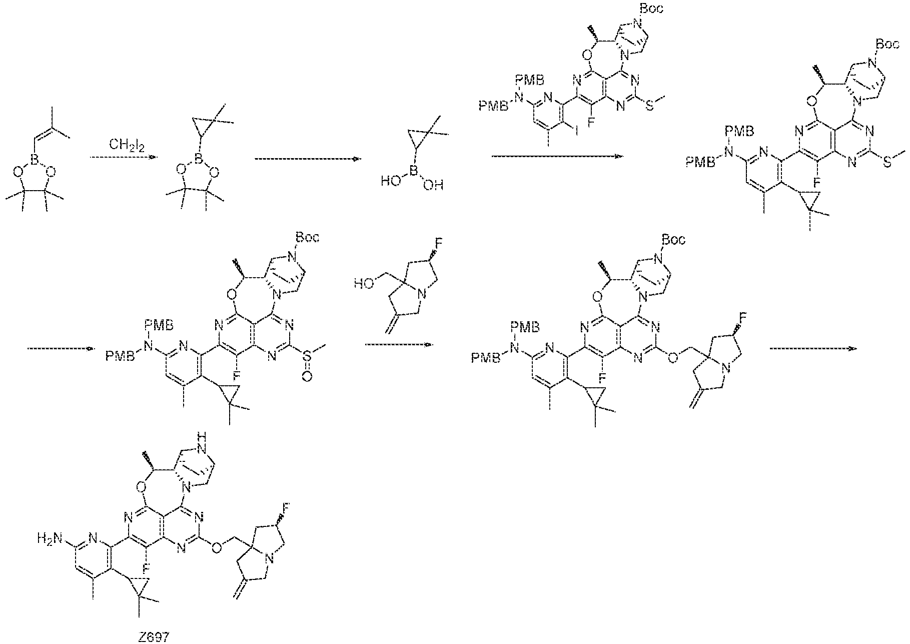

Step 1: A 1.0M solution of diethylzinc in n-hexane (22.0 mL, 22.0 mmol) was dissolved in dichloromethane (22 mL). At 0°C, a solution of trifluoroacetic acid (1.67 mL, 21.97 mmol) in dichloromethane (10 mL) was added dropwise, and the reaction mixture was stirred at 0°C for 30 minutes. Then, a solution of diiodomethane (1.84 mL, 21.97 mmol) in dichloromethane (10 mL) was added dropwise, and the reaction mixture was stirred at 0°C for another 30 minutes. This was followed by the dropwise addition of a solution of 2-methyl-1-propenyl boronic acid pinacol ester (2 g, 11.0 mmol) in dichloromethane (20 mL), and the reaction mixture was stirred at room temperature for 18 hours. The reaction mixture was quenched with 1M dilute hydrochloric acid solution (20 mL), and the separated organic phase was washed with saturated brine (30 mL × 2). After dried over anhydrous sodium sulfate and filtration, the filtrate was concentrated under reduced pressure. The crude product was purified by flash silica gel column chromatography (tetrahydrofuran/petroleum ether: 0-8%) to obtain the target product 2-(2,2-dimethylcyclopropyl)boronic acid pinacol ester (1.9 g, yield 88.2%), a colorless liquid. ES-API: [M+H]⁺= 197.1. ¹H NMR (400 MHz, CD₃OD) δ1.26 -1.18 (m, 12H), 1.15 (s, 3H), 1.12 (s, 3H), 0.63 -0.51 (m, 2H), -0.28 (dd, *J* = 9.2, 6.8 Hz, 1H).

Step 2: 2-(2,2-Dimethylcyclopropyl)boronic acid pinacol ester (700 mg, 3.57 mmol) was dissolved in tetrahydrofuran (24 mL) and water (6 mL), and sodium periodate (2.29 g, 10.71 mmol) was added. The reaction mixture was stirred at room temperature for 5 minutes, followed by the addition of 2M hydrochloric acid (1.18 mL, 2.36 mmol), and stirred at room temperature for 12 hours. The reaction mixture was added to water (10 mL) and extracted with ethyl acetate (60 mL). The organic phase was washed with water (10 mL) and saturated brine (30 mL). After dried over anhydrous sodium sulfate and filtration, the filtrate was concentrated under reduced pressure to obtain the target product 2-(2,2-dimethylcyclopropyl)boronic acid (340 mg, yield 83.6%), which was then used directly in the next reaction without purification.

Step 3: To tert-butyl (5S,5aS,6S,9R)-2-(6-(bis(4-methoxybenzyl)amino)-3-iodo-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, 0.44 mmol) and 2-(2,2-dimethylcyclopropyl)boronic acid (248 mg, 2.17 mmol) were in toluene (10 mL) and water (1 mL), potassium phosphate (277 mg, 1.31 mmol) and palladium(II) bis[(1-adamantyl)methyl] (2-aminobiphenyl) (63 mg, 0.087 mmol) were added. The mixture was purged with nitrogen three times and then reacted at 90°C for 18 hours. The reaction mixture was added to ethyl acetate (60 mL) and washed with saturated brine (30 mL). After dried over anhydrous sodium sulfate and filtration, the filtrate was concentrated under reduced pressure. The crude product was purified by flash silica gel column chromatography (ethyl acetate/petroleum ether: 0-40%) to obtain the target product tert-butyl (5S,5aS,6S,9R)-2-(6-(bis(4-methoxybenzyl)amino)-3-(2,2-dimethylcyclopropyl)-4-methylpyridin-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (265 mg, yield 70.7%), a pale yellow solid. ES-API: [M+H]⁺=862.3.

Step 4: Tert-butyl (5S,5aS,6S,9R)-2-(6-(bis(4-methoxybenzyl)amino)-3-(2,2-dimethylcyclopropyl)-4-methylpyridin-2-yl)-1 -fluoro-5-methyl- 12-(methylthio)-5a,6,7,8,9,10-hexahydro-5H-4-oxa-3,10a,11,13,14-pentaaza-6,9-methanonaphtho[1,8-ab]heptalene-14-carboxylate (240 mg, 0.28 mmol) was dissolved in dichloromethane (15 mL). At room temperature, m-chloroperbenzoic acid (74 mg, 0.36 mmol) was added, and the reaction mixture was stirred at room temperature for 1 hour. The reaction mixture was diluted with dichloromethane (50 mL), successively washed with saturated sodium thiosulfate (15 mL), saturated sodium bicarbonate (15 mL), and saturated brine (30 mL). The organic phase was dried over anhydrous sodium sulfate and filtered. The filtrate was concentrated under reduced pressure to obtain the target product tert-butyl (5S,5aS,6S,9R)-2-(6-(bis(4-methoxybenzyl)amino)-3-(2,2-dimethylcyclopropyl)-4-methylpyridin-2-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (240 mg, yield 98.1%), a white solid. ES-API:[M+H]⁺=878.2.

Step 5: ((2R)-2-fluoro-6-methylenetetrahydro-1H-pyrrolizin-7a(5H)-yl)methanol (140 mg, 0.82 mmol) was dissolved in tetrahydrofuran (8 mL). At 0°C, 60% sodium hydride (38 mg, 0.96 mmol) was added, and the reaction mixture was stirred at this temperature for 30 minutes. A solution of tert-butyl (5S,5aS,6S,9R)-2-(6-(bis(4-methoxybenzyl)amino)-3-(2,2-dimethylcyclopropyl)-4-methylpyridin-2-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (240 mg, 0.27mmol) in tetrahydrofuran (2 mL) was added dropwise, and the reaction mixture was stirred at the same temperature for 30 minutes. The reaction mixture was diluted with ethyl acetate (60 mL), successively washed with water (20 mL), and saturated brine (30 mL). The organic phase was dried over anhydrous sodium sulfate and filtered. The filtrate was concentrated under reduced pressure. The crude product was purified by preparative thin-layer chromatography (petroleum ether/tetrahydrofuran = 3:2) to obtain the target product tert-butyl (5S,5aS,6S,9R)-2-(6-(bis(4-methoxybenzyl)amino)-3-(2,2-dimethylcyclopropyl)-4-methylpyridin-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (95 mg, yield 35.3%), a pale yellow solid. ES-API: [M+H]⁺=985.3.

Step 6: Tert-butyl (5S,5aS,6S,9R)-2-(6-(bis(4-methoxybenzyl)amino)-3-(2,2-dimethylcyclopropyl)-4-methylpyridin-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (80 mg, 0.081 mmol) was dissolved in trifluoroacetic acid (4 mL). The reaction mixture was stirred at 50°C for 2 hours. The reaction mixture was concentrated under reduced pressure to dryness, and dichloromethane (5 mL) and 7.0M methanolic ammonia (5 mL) were added, followed by concentration under reduced pressure. The crude product was purified by preparative HPLC (bicarbonate method 1) to obtain the target product 5-(2,2-dimethylcyclopropyl)-6-((5S,5aS,6S,9R)-1-fluoro-12-(((2R)-2-fluoro-6-methylenetetrahydro-1H-pynolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)-4-methylpyridin-2-amine (Z697, 23 mg, yield: 43.9%), pale yellow solid. ES-API: [M+H]⁺= 645.1. ¹H NMR (400 MHz, CD₃OD) δ 6.57 (s, 1H), 5.44 -5.23 (m, 2H), 5.06 -4.97 (m, 2H), 4.59 -4.47 (m, 1H), 4.37 (d, *J* = 10.0 Hz, 1H), 4.28 (d, *J* = 10.0 Hz, 1H), 4.07 (d, *J* = 8.4 Hz, 1H), 3.74 (d, *J* = 14.0 Hz, 1H), 3.66 (d, *J* = 5.6 Hz, 1H), 3.62 -3.55 (m, 1H), 3.47 -3.37 (m, 1H), 3.30 -3.26 (m, 1H), 3.16 (d, *J* = 13.6 Hz, 1H), 3.02 -2.81 (m, 2H), 2.58 -2.43 (m, 2H), 2.41 -2.27 (m, 3H), 2.21 -1.99 (m, 2H), 1.92 -1.65 (m, 4H), 1.57 (d, *J* = 6.4 Hz, 3H), 1.34 -0.94 (m, 3H), 0.66 (s, 3H), 0.54 -0.24 (m, 1H),-0.08 --0.51 (m, 1H).

### Example 88 Synthesis of (5S,5aS,65,9R)-2-(8-ethynyl-7-fluoronaphthalen-1-yl)-1-fluoro-5-methyl-12-((1-((3-methylenepyrrolidin-1-yl)methyl)cyclopropyl)methoxy)-5a,6,7,8,9,10-hexahydro-SH-4-oxa-3,10a,11,13,14-pentaaza-6,9-methanonaphtho[1.,8-ab]heptalene (Z698)

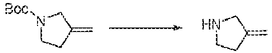

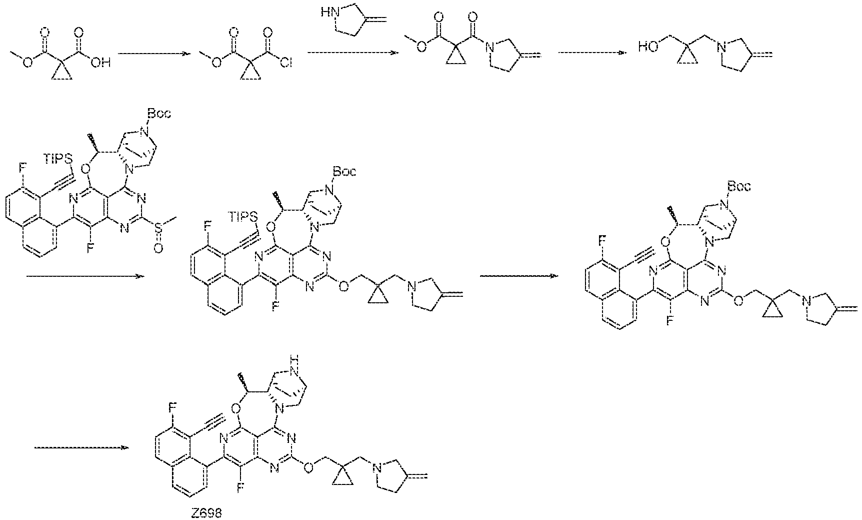

Step 1: Under an ice-water bath, 4 M hydrochloric acid-dioxane solution (13.64 mL, 54.57 mmol) was added to a solution of tert-butyl 3-methylenepyrrolidine-1-carboxylate (2 g, 10.9 mmol) in dichloromethane (5 mL). The reaction mixture was stirred at room temperature for 1 hour. The reaction mixture was concentrated to obtain 3-methylpyrrolidinium hydrochloride (907 mg, crude product), which was then used directly in the next step without purification.

Step 2: 1-(Methoxycarbonyl)cyclopropane-1-carboxylic acid (1.2 g, 8.33 mmol) was dissolved in a mixture of dichloromethane (10 mL) and N,N-dimethylformamide (0.032 mL). With the mixture cooled to zero degrees, oxalyl chloride (2.82 mL, 33.30 mmol) was added to the reaction mixture. The reaction mixture was stirred at 35 degrees for 3.5 hours. The reaction mixture was concentrated to obtain methyl 1-(chlorocarbonyl)cyclopropane-1-carboxylate (1.35g, crude product), which was then used directly in the next step without purification.

Step 3: 3-Methylpyrrolidinium hydrochloride (900 mg, crude product) and N,N-diisopropylethylamine (7.2 mL, 41.52 mmol) in dichloromethane (20 mL) were cooled to zero degrees. A solution of methyl 1-(chlorocarbonyl)cyclopropane-1-carboxylate (1.35g, crude product) in dichloromethane (10 mL) was added dropwise. After the addition was complete, the reaction mixture was stirred at room temperature for 1 hour. The reaction was quenched with 2M hydrochloric acid (30 mL). The mixture was extracted with dichloromethane (30 mLX2). The organic phase was washed with saturated sodium bicarbonate and brine, dried over anhydrous sodium sulfate, filtered, and concentrated to obtain methyl 1-(3-methylenepyrrolidine-1-carbonyl)cyclopropane-1-carboxylate (1.7g, yield 97%). ES-API: [M+H]⁺=210.1.

Step 4: At -20 degrees, a solution of lithium aluminium hydride in tetrahydrofuran (7.65 mL, 7.65 mmol) was added dropwise to a solution of methyl 1-(3-methylenepyrrolidine-1-carbonyl)cyclopropane-1-carboxylate (800 mg, 3.82 mmol) in tetrahydrofuran (15 mL). After the addition, the reaction mixture was stirred at -10 degrees for 30 minutes. The reaction was quenched successively with 15% sodium hydroxide solution (0.5 mL). The mixture was stirred at room temperature for 30 minutes and filtered through diatomaceous earth. The filtrate was concentrated to obtain (1-((3-methylpyrnolidin-1-yl)methyl)cyclopropyl)methanol (430 mg, yield 67%), a yellow oil. ES-API: [M+H]⁺=168.1.

Step 5: To a round-bottomed flask, 60% sodium hydride (9.75 mg, 0.406 mmol) and tetrahydrofuran (6 mL) were added. The mixture was cooled to 0 degrees, and a solution of (1-((3-methylpyrrolidin-1-yl)methyl)cyclopropyl)methanol (25.47 mg, 0.152 mmol) in anhydrous tetrahydrofuran (1 mL) was added. The reaction was proceeded at room temperature for 10 minutes. The reaction mixture was cooled to zero degrees, and tert-butyl (SS,SaS,6S,9R)-1-fluoro-2-(7-fluoro-8-((triisopropylsilyl)ethynyl)naphthalen-1-yl)-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (80 mg, 0.1 mmol) was added to the reaction mixture, and the reaction was proceeded at room temperature for 1 hour. Water (30 mL) was added to the reaction mixture. The mixture was extracted with ethyl acetate (20 mLX2). The organic phase was washed with saturated brine (30 mL) and dried over anhydrous sodium sulfate, filtered, and concentrated to obtain tert-butyl (5S,5aS,6S,9R)-1-fluoro-2-(7-fluoro-8-((triisopropylsilyl)ethynyl)naphthalen-1-yl)-5-methyl-12-((1-((3-methylenepyrrolidin-1-yl)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (80 mg, crude product). ES-API: [M+H]⁺=891.3.

Step 6: Cesium fluoride (109.09 mg, 0.718 mmol) was added to a solution of tert-butyl (5S,5aS,6S,9R)-1-fluoro-2-(7-fluoro-8-((triisopropylsilyl)ethynyl)naphthalen-1-yl)-5-methyl-12-((1-((3-methylenepyrrolidin-1-yl)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (80 mg, crude product) in N,N-dimethylformamide (3 mL). The reaction mixture was stirred at room temperature for 1 hour. Upon completion of the reaction (monitored by LCMS), the reaction mixture was diluted with ethyl acetate (50 mL) and washed with water (30 mL) and saturated brine (30 mLX2). The organic phase was dried over anhydrous sodium sulfate, filtered, and concentrated to obtain tert-butyl (5S,5aS,6S,9R)-2-(8-ethynyl-7-fluoronaphthalen-1-yl)-1-fluoro-5-methyl-12-((1-((3-methylenepyrrolidin-1-yl)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (60 mg, crude product). ES-API: [M+H]⁺= 735.3.

Step 7: Under an ice-water bath, 4 M hydrochloric acid-dioxane solution (3 mL) was slowly added to a solution of tert-butyl (5S,5aS,6S,9R)-2-(8-ethynyl-7-fluoronaphthalen-1-yl)-1-fluoro-5-methyl-12-((1-((3-methylenepyrrolidin-1-yl)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (60 mg, crude product) in acetonitrile (3 mL). The reaction mixture was stirred at room temperature for 1 hour. Upon completion of the reaction (monitored by LCMS), the reaction mixture was concentrated under reduced pressure. The residue was purified by preparative HPLC (ammonium bicarbonate method 1) to obtain (5S,5aS,6S,9R)-2-(8-ethynyl-7-fluoronaphthalen-1-yl)-1-fluoro-5-methyl-12-((1-((3-methylenepyrrolidin-1-yl)methyl)cyclopropyl)methoxy)-5a,6,7,8,9,10-hexahydro-5H-4-oxa-3,10a,1,13,14-pentaaza-6,9-methanonaphtho[1,8-ab]heptalene (Z698, 10mg, yield: 19%), ES-API: [M+H]⁺= 635.2. ¹HNMR(400MHz, CD₃OD): 8.10-8.05 (m, 2H), 7.64-7.38 (m, 3H), 5.39-5.36 (m, 1H), 4.92-4.87 (m, 2H), 4.54-4.34 (m, 3H), 4.08-4.06 (m, 1H), 3.76-3.46 (m, 3H), 3.25-3.13 (m, 3H), 2.90-2.40 (m, 6H), 2.10-2.02 (m, 1H), 1.82-1.76 (m, 3H), 1.58-1.55 (m, 3H), 0.71-0.55 (m, 4H).

### Example 89 Synthesis of 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,1.0a,11,13,14-pentaaza-6,9-methanonaphtho[1,8-ab]heptalen-2-yl)naphthalen-2-amine (Z699)

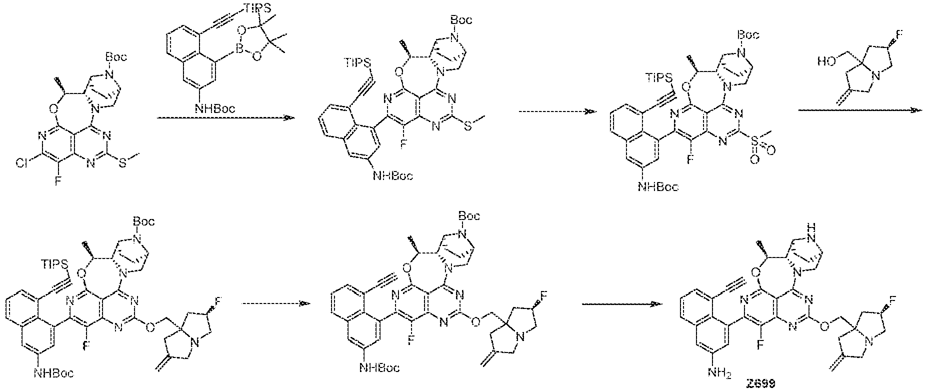

Step 1: Tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.166 mmol) and tert-butyl (4-(4,4,5,5-tetramethyl-1,3,2-dioxaborolan-2-yl)-5-((triisopropylsilyl)ethynyl)naphthalen-2-yl)carbamate (91.24 mg, 0.166 mmol) were dissolved in tetrahydrofuran (10 mL) and water (1 mL). Methylsulfonic acid [n-butylbis(1-adamantyl)phosphine](2-amino-1,1'-biphenyl-2-yl)palladium(II) (12.09 mg, 0.017 mmol) and potassium phosphate (105.70 mg, 0.498 mmol) were added to the solution. After the addition was complete, the reaction mixture was stirred at 65 degrees Celsius for 5 hours. Upon completion of the reaction, water (15 mL) was added to the reaction mixture, which was then extracted with ethyl acetate (15 mLX3). The organic phase was washed with saturated brine (30 mL), dried over anhydrous sodium sulfate, filtered, and concentrated. The crude product was purified by a flash silica gel column (ethyl acetate/petroleum ether: 0-30%) to obtain tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.092 mmol, yield 55.44%), a white solid. ES-API: [M +H]⁺= 869.3.

Step 2: Tert-butyl (5S,5aS,6S,9R)-2-(3-((tert-butoxycarbonyl)amino)-8-((triisopropylsilyl)ethynyl)naphthalen-1-yl)-1-fluoro-5-mεthyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (80 mg, 0.092 mmol) was dissolved in dichloromethane (5 mL). At room temperature, m-chloroperoxybenzoic acid (85% purity, 56.06 mg, 0.276 mmol) was added to the solution. After the addition was complete, the reaction mixture was stirred at room temperature for 1 hour. The reaction mixture was quenched with saturated sodium thiosulfate solution (5 mL) and extracted with dichloromethane (10 mLX3). The combined organic phases were washed with saturated sodium bicarbonate solution (20 mL) and saturated sodium chloride (20 mL). The organic phase was dried and concentrated to obtain tert-butyl (SS,SaS,6S,9R)-2-(3-((tert-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (80 mg, 0.089 mmol, yield 96.45%), a pale yellow solid. ES-API: [M +H]⁺= 901.0.

Step 3: ((2R)-2-fluoro-6-methylenetetrahydro-1H-pyrrolizin-7a(5H)-yl)methanol (30.40 mg, 0.178 mmol) was dissolved in tetrahydrofuran (3 mL) and cooled to 0 degrees Celsius. Under a nitrogen atmosphere, sodium hydride (60%, 14.20 mg, 0.355 mmol) was added to the solution, which was then stirred for 30 minutes. Subsequently, tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (80 mg, 0.089 mmol) was slowly added to the solution which was then stirred at 0 degrees Celsius for 2 hours. Water (5 mL) was added to quench the reaction and the mixture was extracted with ethyl acetate (20 mL X3). The organic phase was washed with saturated brine (30 mLX3), dried over anhydrous sodium sulfate, filtered, and concentrated to obtain a crude product. The crude product was purified by a flash silica gel column (ethyl acetate/petroleum ether: 0-70%) to obtain tert-butyl (5S,5aS,6S,9R)-2-(3-((tert-butoxycarbonyl)amino)-8-((triisopropylsilyl)ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, 0.050 mmol, yield 56.76%), a yellow solid. ES-API: [M+H]⁺=992.1.

Step 4: A mixed solution of tert-butyl (5S,5aS,6S,9R)-2-(3-((tert-butoxycarbonyl)amino)-8-((triisopropylsilyl)ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (50 mg, 0.050 mmol), cesium fluoride (76.54 mg, 0.504 mmol), and N, N-dimethylformamide (2 mL) was stirred at room temperature for 2 hours. Water (10 mL) was added to the reaction mixture, which was then extracted with ethyl acetate (15 mLX2). The organic phase was washed with saturated brine (15 mLX3), dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.036 mmol, yield 71.22%), a yellow oily liquid. ES-API: [M+H]⁺=835.1.

Step 5: Tert-butyl (5S,5aS,6S,9R)-2-(3-((tert-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.036 mmol) was dissolved in methanol (2 mL) and cooled to 0 degrees Celsius. Hydrochloric acid in dioxane solution (4 M, 1 mL) was added to the solution, and the reaction mixture was stirred at 0 degrees Celsius for 2 hours. The reaction mixture was concentrated, redissolved in dichloromethane (3 mL), and basified to a basic pH (pH 8) with ammonium hydroxide in methanol (7 M). After concentration of the solution, the crude product was purified by preparative HPLC (ammonium bicarbonate method 1) to obtain 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine (Z699, 9 mg, 0.014 mmol, yield: 39.45%), white solid. ES-API: [M+H]⁺=636.2.

### Example 90 Synthesis of 5-ethynyl-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-amine (Z700)

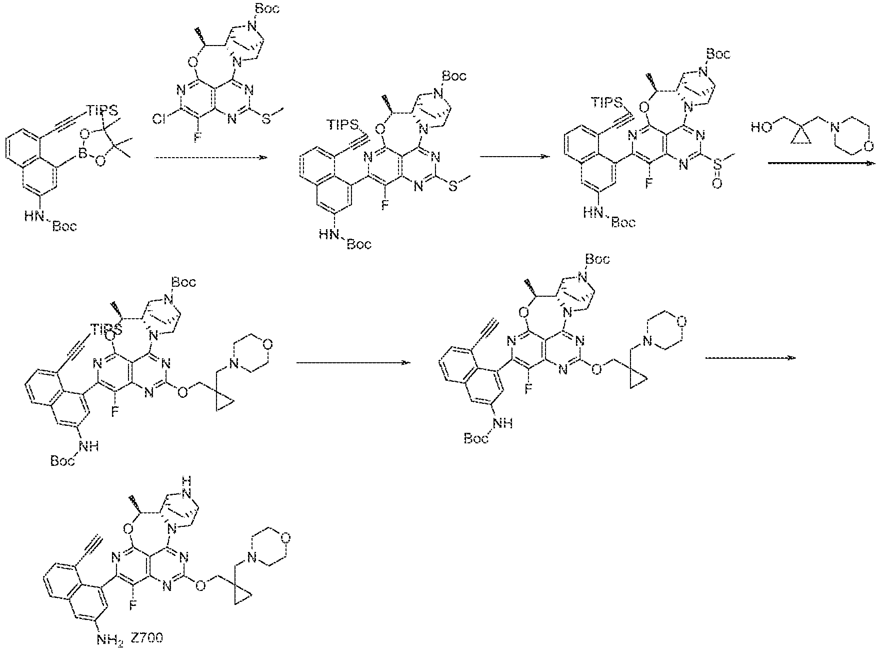

Step 1: To a solution of tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (700 mg, 1.452 mmol) and tert-butyl (4-(4,4,5,5-tetramethyl-1,3,2-dioxaborolan-2-yl)-5-((triisopropylsilyl)ethynyl)naphthalen-2-yl)carbamate (700 mg, 1.274 mmol) in tetrahydrofuran (30 mL) and water (5 mL), potassium phosphate (1.23 g, 5.80 mmol) and methanesulfonic acid [tert-butyl di(1-adamantyl)phosphine](2-amino-1,1'-biphenyl-2-yl)palladium(II) (158 mg, 0.218 mmol) were added. The reaction mixture was bubbled with nitrogen and heated in an oil bath at 80°C and reacted for 3 hours. Upon completion of the reaction, the mixture was extracted with ethyl acetate (30 mL X2) and saturated brine (30 mL). The combined ethyl acetate phases were dried over anhydrous sodium sulfate and filtered. The filtrate was concentrated under reduced pressure. The crude product was purified by silica gel column chromatography (0-4% methanol/dichloromethane) to obtain tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (900 mg, yield 71.29%), a yellow solid. ES-API: [M+H]⁺=869.2.

Step 2: At 0°C, to a solution of tert-butyl (5S,5aS,6S,9R)-2-(3-((tert-butoxycarbonyl)amino)-8-((triisopropylsilyl)ethynyl)naphthalen-1 -yl)-1 -fluoro-5-methyl- 12-(methylthio)-5a,6,7,8,9,10-hexahydro-5H-4-oxa-3,10a,11,13,14-pentaaza-6,9-methanonaphtho[1,8-ab]heptalene-14-carboxylate (800 mg, 0.920 mmol) in dichloromethane (15 mL), m-chloroperoxybenzoic acid (206.5 mg, 1.197 mmol) was added. The mixture was stirred at room temperature for 1 hour. The reaction mixture was quenched with saturated sodium sulfite and extracted with dichloromethane (100 mL X2). The organic layer was dried over anhydrous sodium sulfate and concentrated to obtain a crude product, which was then purified through silica gel column chromatography (0-10% methanol/dichloromethane) to obtain tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (700 mg, yield 85.92%), a white solid. ES-API:[M+H]⁺=885.3.

Step 3: At 0°C, sodium hydride (268.6 mg, 6.71 mmol) was added to a solution of (1-(morpholinomethyl)cyclopropyl)methanol (460 mg, 2.686 mmol) in tetrahydrofuran (10.0 mL). The mixture was stirred for 10 minutes at this temperature, followed by the addition of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (830 mg, 0.938 mmol) in tetrahydrofuran (5 mL). The reaction mixture was stirred for 10 minutes at 0°C, then stirred for 0.5 hours at room temperature. Upon completion of the reaction, the reaction was quenched with saturated sodium bicarbonate solution and the mixture was extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (900 mg, yield 96.73%), a yellow solid. ES-API: [M+H]⁺=993.2.

Step 4: To a solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (900 mg, 0.907 mmol) in N,N-dimethylformamide (10.0 mL), cesium fluoride (2.75 g, 18.14 mmol) was added, and the mixture was stirred at room temperature for 0.5 hours. Upon completion of the reaction, the mixture was quenched with water, extracted with ethyl acetate. The organic phase was washed with saturated brine, dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-5-methyl-12-« 1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (510 mg, yield 67.26%), a yellow oil. ES-API: [M+H]⁺=836.2.

Step 5: Under an ice-water bath, 4 M hydrochloric acid-dioxane solution (10.0 mL) was slowly added to a solution of tert-butyl (5S,5aS,6S,9R)-2-(3-((tert-butoxycarbonyl)amino)-8-ethynylnaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (510 mg, 0.610 mmol) in methanol (15.0 mL), and the mixture was stirred at room temperature for 0.5 hours. Upon completion of the reaction (monitored by LCMS), the mixture was concentrated under reduced pressure, and the residue was purified by preparative HPLC (ammonium bicarbonate method 2) (chromatography conditions: 15ml-35-85-13min) to obtain a pale yellow solid 5-ethynyl-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-amine (Z700, 73mg, yield: 18.82%). ES-API: [M+H]⁺= 636.2. ¹H NMR (400 MHz, CD₃OD) δ 7.65-7.69 (m, 1H), 7.35-7.25 (m, 2H), 7.13 -7.00 (m, 2H), 5.39-5.34 (m, 1H), 4.51-4.42 (m, 2H), 4.37-4.33 (m, 1H), 4.06 (d*, J* = 8.0 Hz, 1H), 3.69 -3.58 (m, 6H), 3.14-3.19 (m, 1H), 2.50 -2.37 (m, 6H), 2.07 -2.04 (m, 1H), 1.87 - 1.76 (m, 3H), 1.58 -1.54 (m, 3H), 0.75-0.65 (m, 2H), 0.53-0.45 (m, 2H).

### Example 91 Synthesis of (1-((((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oay)methyl)cyclopropyl)methanol (Z701)

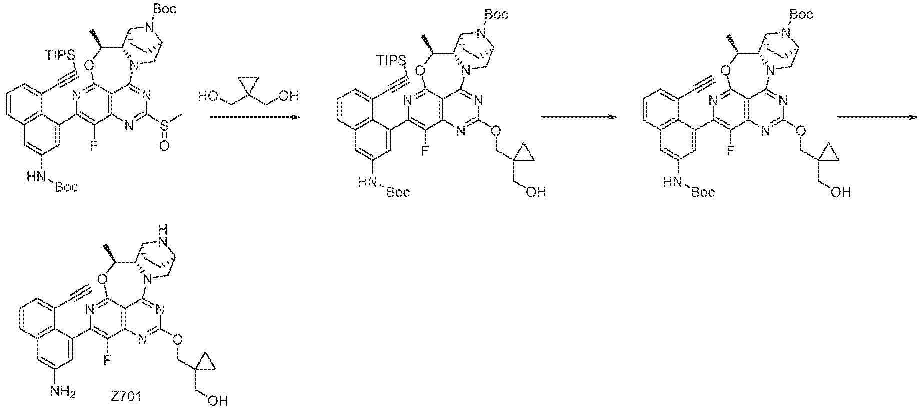

Step 1: Cyclopropane-1,1-dimethanol (8 mg, 0.07 mmol) was dissolved in anhydrous tetrahydrofuran (2 mL) and under an ice-water bath, sodium hydride (14 mg, 0.34 mmol) was added. The mixture reacted under an ice water bath for 10 minutes. Then, a tetrahydrofuran solution (1 mL) of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (60 mg, 0.07 mmol) was added dropwise to the reaction mixture. The mixture was stirred at room temperature for 30 minutes. Upon completion of the reaction (monitored by LCMS), ethyl acetate (5 mL) was added to dilute the reaction mixture, which was then washed with saturated ammonium chloride solution (5 mL × 2) and saturated brine (5 mL × 3). After dried over anhydrous sodium sulfate, filtration, and purification through silica gel using automated flash chromatography [mobile phase: dichloromethane:methanol = 0%-6%], tert-butyl (5S,5aS,6S,9R)-2-(3-((tert-butoxycarbonyl)amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (23 mg, 0.02 mmol, yield 35.6%) was obtained. ES-API: [M+H]⁺= 923.1.

Step 2: Cesium fluoride (34 mg, 0.20 mmol) was added to a solution of tert-butyl (5S,5aS,6S,9R)-2-(3-((tert-butoxycarbonyl)amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (23 mg, 0.02 mmol) in N,N-dimethylformamide (2 mL). The reaction mixture was stirred at room temperature for 2 hours. Upon completion of the reaction (monitored by LCMS), the reaction mixture was diluted with ethyl acetate (5 mL), washed with water (5 mL × 2), and saturated brine (5 mL × 3), dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-12-((1-(hydroxymethyl)cyclopropyl)methoxy)-5-methyl-5a,6,7,8,9,10-hexahydro-5H-4-oxa-3,10a, 11, 13,14-pentaaza-6,9-methanonaphtho[1,8-ab]heptalene-14-carboxylate (13 mg, 0.02 mmol, yield 84.9%). ES-API: [M+H]⁺= 767.1.

Step 3: Under an ice-water bath, a 4 M hydrochloric acid-dioxane solution (2.0 mL) was slowly added to a methanol solution (2.0 mL) of tert-butyl (5S,5aS,6S,9R)-2-(3-((tert-butoxycarbonyl)amino)-8-ethynylnaphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (13 mg, 0.02 mmol). The reaction mixture was stirred at 0°C for 4 hours. Upon completion of the reaction (monitored by LCMS), the reaction mixture was concentrated under reduced pressure to dryness, and the residue was purified by preparative HPLC (bicarbonate method 2) (chromatographic conditions: 15 ml-35-85-13 min) to obtain (1-((((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methanol (Z701, 6.14mg,0.01mmol,yield: 54.2%). ES-API: [M+H]⁺= 567.1.

### Example 92 Synthesis of 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z703)

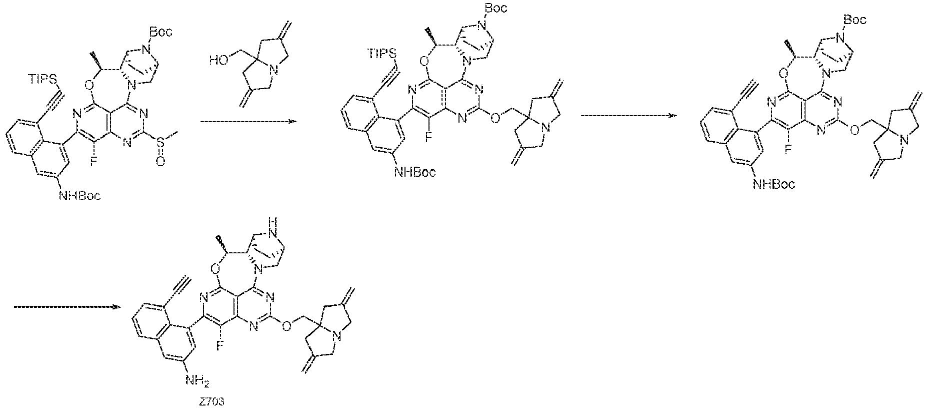

Step 1: (2,6-Dimethylenetetrahydro-1H-pyrrolizin-7a(5H)-yl)methanol (25 mg, 0.151 mmol) was dissolved in anhydrous tetrahydrofuran (2 mL), under an ice-water bath, sodium hydride (10.85 mg, 0.452 mmol) was then added. The mixture reacted under the ice-water bath for 30 minutes. To this reaction mixture, a tetrahydrofuran solution (2 mL) of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (50 mg, 0.056 mmol) was added dropwise and the mixture reacted at room temperature for 30 minutes. Upon completion of the reaction (monitored by LCMS), the reaction mixture was diluted with ethyl acetate (10 mL), washed with saturated ammonium chloride solution (5 mL × 2) and saturated brine (50 mL × 3), dried over anhydrous sodium sulfate, filtered, and purified by silica gel column chromatography (dichloromethane:methanol = 15:1) to obtain tert-butyl (5S,5aS,6S,9R)-2-(3-((tert-butoxycarbonyl)amino)-8-((triisopropylsilyl)ethynyl)naphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (44 mg, 0.045 mmol, yield 78.98%). ES-API: [M+H]⁺= 986.3.

Step 2: Cesium fluoride (33.49 mg, 0.220 mmol) was added to a solution of tert-butyl (5S,5aS,6S,9R)-2-(3-((tert-butoxycarbonyl)amino)-8-((triisopropylsilyl)ethynyl)naphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (35 mg, 0.035 mmol) in N,N-dimethylformamide (1.0 mL). The reaction mixture was stirred at room temperature for 2 hours. Upon completion of the reaction (monitored by LCMS), the reaction mixture was diluted with ethyl acetate (10 mL), washed with water (10 mL × 2) and saturated brine (10 mL × 3), dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (28 mg, 0.034 mmol, yield 95.08%). ES-API: [M+H]⁺=830.2.

Step 3: Under an ice-water bath, a 4 M hydrochloric acid-dioxane solution (1.0 mL) was slowly added to an ethyl acetate solution (1.0 mL) of tert-butyl (5S,5aS,6S,9R)-2-(3-((tert-butoxycarbonyl)arnino)-8-ethynylnaphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.030 mmol). The reaction mixture was stirred at 0°C for 0.5 hours. Upon completion of the reaction (monitored by LCMS), the reaction mixture was concentrated under reduced pressure to dryness, and the residue was purified by preparative HPLC (bicarbonate method 1) (chromatographic conditions: 15 ml-35-85-13 min) to obtain a white solid, 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z703, 8 mg, 0.013 mmol, yield: 42.35%). ES-API: [M+H]⁺= 630.2. ¹H NMR (400 MHz, CD₃OD) δ 7.67 (t*, J* = 7.2 Hz, 1H), 7.41 -7.35 (m, 1H) , 7.32 -7.26 (m, 1H) , 7.13 -7.02 (m, 2H) , 5.51 -5.27 (m, 1H), 5.02 (d, *J* = 6.8 Hz, 4H), 4.53 -4.47 (m, 1H) 4.30 (s, 2H), 4.06 (d, *J* = 8.4 Hz, 1H), 3.79 -3.75 (m, 2H), 3.67 (d, *J* = 5.2 Hz, 1H), 3.59 (s, 1H), 3.37 -3.33 (m, 2H), 3.31 -2.99 (m, 2H), 2.81 -2.78 (m, 2H), 2.61 -2.57 (m, 2H), 2.03 -2.02 (m, 1H), 1.94 -1.69 (m, 3H), 1.56 (t, *J* = 6.8 Hz, 3H).

### Example 93 Synthesis of (5S,5aS,6S,9R)-2-(8-ethynyl-7-fluoronaphthalen-1-yl)-1-fluoro-12-(((1R)-1-methory-6-methylenetetrahydro-1H-pyrrolizin-7a(5H)-yl)methory)-5-methyl-5a,6,7,8,9,10-hexahydro-5H-4-oxa-3,10a,11,13,14-pentaaza-6,9-methanonaphtho[1,8-ab]heptalene (Z704)

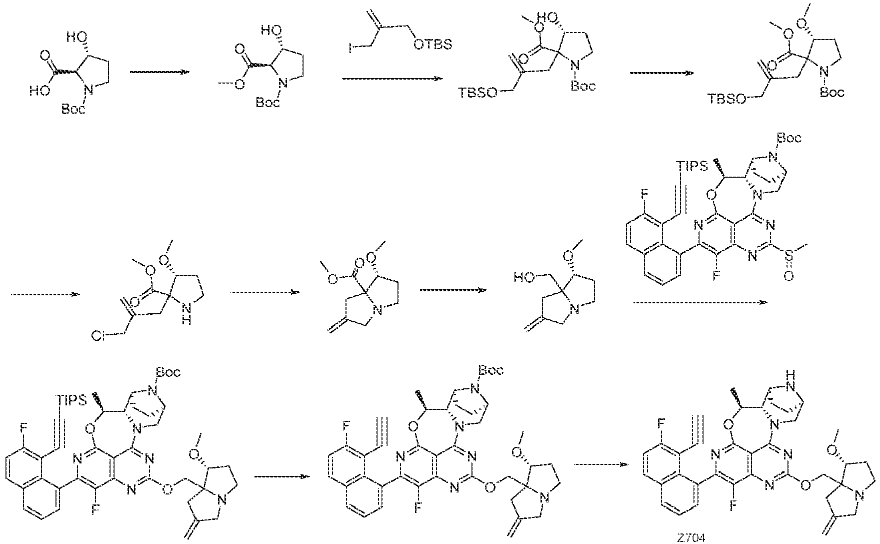

Step 1: Under an ice-water bath, potassium carbonate (1.79 g, 12.9 mmol) and iodomethane (1.84 g, 12.9 mmol) were added to a solution of (2R,3R)-1-(tert-butoxycarbonyl)-3-hydroxypyrolidine-2-carboxylic acid (1 g, 4.3 mmol) in N,N-dimethylformamide (10 mL). The mixture was stirred at room temperature for 2 hours. Upon completion of the reaction (monitored by LCMS), the reaction mixture was filtered and washed with ethyl acetate. The filtrate was then added to water (15 mL × 3) and washed with saturated brine (15 mL × 3). After dried over anhydrous sodium sulfate, filtered, and concentrated under reduced pressure, the crude product was purified by flash silica gel column chromatography (petroleum ether: ethyl acetate = 10:1) to obtain the compound 1-tert-butyl 2-methyl (2R,3R)-3-hydroxypyrolidine-1,2-dicarboxylate (1.0 g, yield: 94%). ES-API: [M-100]⁺= 190.1.

Step 2: Under nitrogen protection, 1-tert-butyl 2-methyl (2R,3R)-3-methoxypyrolidine-1,2-dicarboxylate (1.0 g, 4.1 mmol) was dissolved in anhydrous tetrahydrofuran (20 mL). The solution was cooled to -78°C, and a solution of diisopropylamine lithium (6.1 mL, 12.2 mmol, 2 M in tetrahydrofuran) was slowly added dropwise. The mixture was stirred at this temperature for 1 hour, then a solution of tert-butyl((2-(iodomethyl)allyl)oxy)dimethylsilane (2.55 g, 8.2 mmol) in anhydrous tetrahydrofuran (10 mL) was slowly added via syringe. The reaction mixture was gradually brought to room temperature and stirred for an additional 2 hours. Upon completion of the reaction (monitored by LCMS), the reaction was quenched with saturated ammonium chloride solution (50 mL). The mixture was extracted with ethyl acetate (50 mL × 3), and the organic phase was washed with saturated brine (50 mL × 3), dried over anhydrous sodium sulfate, filtered, and concentrated. The crude product was purified by flash silica gel column chromatography (petroleum ether: ethyl acetate = 20:1) to obtain 1-(tert-butyl) 2-methyl (3R)-2-(2-(((tertbutyldimethylsilyl)oxy)methyl)allyl)-3-hydroxypyrrolidine-1(450 mg, yield: 26%). ES-API: [M-55]⁺= 330.3.

Step 3: Under nitrogen protection, silver oxide (4.8 g, 20.9 mmol) and iodomethane (3.0 g, 20.9 mmol) were added to a solution of 1-(tert-butyl) 2-methyl (3R)-2-(2-(((tertbutyldimethylsilyl)oxy)methyl)allyl)-3-hydroxypyrrolidine-1,2-dicarboxylate (450 mg, 1.1 mmol) in acetonitrile (10 mL). The mixture was stirred at room temperature for 48 hours. Upon completion of the reaction (monitored by LCMS), the reaction mixture was filtered and washed with ethyl acetate. The filtrate was concentrated under reduced pressure, and the crude product was purified by flash silica gel column chromatography (petroleum ether: ethyl acetate = 15:1) to obtain 1-(tert-butyl) 2-methyl (3R)-2-(2-(((tert-butyldimethylsilyl)oxy)methyl)allyl)-3-methoxypyrrolidine-1,2-dicarboxylate (340 mg, yield: 73%). ES-API: [M-100]⁺= 344.2.

Step 4: At room temperature, thionyl chloride (15 mL) was slowly added to a solution of 1-(tert-butyl) 2-methyl (3R)-2-(2-(((tert-butyldimethylsilyl)oxy)methyl)allyl)-3-methoxypyrrolidine-1,2-dicarboxylate (340 mg, 0.76 mmol) in dichloromethane (7 mL). The mixture was stirred overnight at room temperature. Upon completion of the reaction (monitored by LCMS), the reaction mixture was concentrated under reduced pressure, and the crude product was purified by flash silica gel column chromatography (petroleum ether: ethyl acetate = 10:1) to obtain methyl (3R)-2-(2-(chloromethyl)allyl)-3-methoxypyrrolidine-2-carboxylate (189 mg, yield: 100%). ES-API: [M+H]⁺= 248.2.

Step 5: At room temperature, sodium bicarbonate (320 mg, 3.8 mmol) and potassium iodide (13 mg, 0.076 mmol) were added to a solution of methyl (3R)-2-(2-(chloromethyl)allyl)-3-methoxypyrrolidine-2-carboxylate (189 mg, 0.76 mmol) in acetonitrile (5 mL). The mixture was stirred at room temperature for 1.5 hours. Upon completion of the reaction (monitored by LCMS), the reaction mixture was filtered and washed with acetonitrile. The filtrate was concentrated under reduced pressure to obtain the crude product, methyl (1R)-1-methoxy-6-methylenetetrahydro-1H-pyrrolizine-7a(5H)-carboxylate (161 mg, yield: 100%). ES-API: [M+H]⁺=212.2.

Step 6: Under nitrogen protection, methyl (1R)-1-methoxy-6-methylenetetrahydro-1H-pyrrolizine-7a(5H)-carboxylate (161 mg, 0.76 mmol) was dissolved in anhydrous tetrahydrofuran (5 mL). The mixture was placed in an ice-water bath, and a solution of lithium aluminum hydride (1.5 mL, 1.5 mmol, 1 M in tetrahydrofuran) was slowly added dropwise. The mixture was stirred at this temperature for 0.5 hours. Upon completion of the reaction (monitored by LCMS), sodium sulfate decahydrate (500 mg) was added to quench the reaction. The mixture was stirred at room temperature for 0.5 hours and then filtered. The filtrate was concentrated under reduced pressure to obtain ((1R)-1-methoxy-6-methylenetetrahydro-1H-pyrrolizin-7a(5H)-yl)methanol (129 mg, yield: 92%). ES-API: [M+H]⁺= 184.1.

Step 7: ((1R)-1-methoxy-6-methylenetetrahydro-1H-pyrrolizin-7a(5H)-yl)methanol (24 mg, 0.13 mmol) was dissolved in anhydrous tetrahydrofuran (5 mL). Under an ice-water bath, sodium hydride (16 mg, 0.39 mmol) was added, and the reaction was proceeded for 30 minutes under the ice-water bath. A tetrahydrofuran solution (2 mL) of tert-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (102 mg, 0.13 mmol) was added dropwise to the reaction mixture, which then reacted at room temperature for 30 minutes. Upon completion of the reaction (monitored by LCMS), the reaction mixture was diluted with ethyl acetate (20 mL), washed with saturated ammonium chloride solution (15 mL × 2) and saturated brine (15 mL × 3), dried over anhydrous sodium sulfate, filtered, and purified by silica gel column chromatography (dichloromethane:methanol = 15:1) to obtain tert-butyl (SS,SaS,6S,9R)-1-fluoro-2-(7-fluoro-8-((triisopropylsilyl)ethynyl)naphthalen-1-yl)-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, yield: 26%). ES-API: [M+H]⁺= 907.2.

Step 8: Cesium fluoride (50 mg, 0.33 mmol) was added to a solution of tert-butyl (5S,5aS,6S,9R)-1-fluoro-2-(7-fluoro-8-((triisopropylsilyl)ethynyl)naphthalen-1-yl)-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.033 mmol) in N,N-dimethylformamide (1.5 mL). The reaction mixture was stirred at room temperature for 2 hours. Upon completion of the reaction (monitored by LCMS), the reaction mixture was diluted with ethyl acetate (20 mL), washed with water (15 mL × 2) and saturated brine (15 mL × 3), dried over anhydrous sodium sulfate, filtered, and concentrated to obtain the crude product, tert-butyl (5S,5aS,6S,9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, yield: 100%). ES-API: [M+H]⁺= 751.2.

Step 9: Under an ice-water bath, a 4 M hydrochloric acid-dioxane solution (5 mL) was slowly added to a solution of tert-butyl (5S,5aS,6S,9R)-2-(8-ethynyl-7-fluoronaphthalen-l-yl)-l-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.033 mmol) in acetonitrile (1 mL). The reaction mixture was stirred at 0°C for 0.5 hours. Upon completion of the reaction (monitored by LCMS), the reaction mixture was concentrated under reduced pressure to dryness, and the residue was purified by preparative HPLC (ammonium bicarbonate method 2) (chromatographic conditions: 15ml-35-85-13min) to obtain a white solid, (5S,5aS,6S,9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene (Z704, 3.3 mg, yield: 16%). ES-API: [M+H]⁺= 651.2.

### Example 94 Synthesis of 5-ethynyl-4-((5S,5aS,6S,9R)-1-fluoro-12-methoxy-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine (Z705)

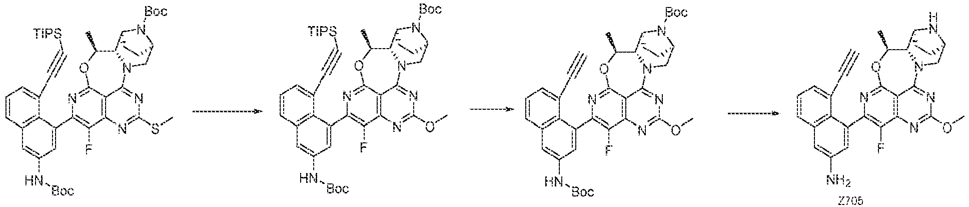

Step 1: Tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.092 mmol) was dissolved in methanol (1.5 mL) and dichloromethane (1.0 mL). Under an ice-water bath, m-chloroperbenzoic acid (48 mg, 0.27 mmol) was added, and the reaction was proceeded for 60 minutes under the ice-water bath. Sodium methoxide (2 mg, 0.02 mmol) was added to the reaction mixture, which then reacted at room temperature for 30 minutes. Upon completion of the reaction (monitored by LCMS), the reaction mixture was diluted with dichloromethane (20 mL), washed with saturated ammonium chloride solution (15 mL × 2) and saturated brine (15 mL × 3), dried over anhydrous sodium sulfate, filtered, and concentrated to obtain a crude product. The crude product was purified by silica gel column chromatography (mobile phase: petroleum ether: ethyl acetate = 20:1) to obtain tert-butyl (5S,5aS,6S,9R)-2-(3-((tert-butoxycarbonyl)amino)-8-((triisopropylsilyl)ethynyl)naphthalen-1-yl)-1-fluoro-12-methoxy-S-methyl-5a,6,7,8,9,10-hexahydro-5H-4-oxa-3,10a,11,13,14-pentaaza-6,9-methanonaphtho[1,8-ab]heptalene-14-carboxylate (30 mg, yield: 38%). ES-API: [M+H]⁺=853.3.

Step 2: Cesium fluoride (53 mg, 0.35 mmol) was added to a solution of tert-butyl (5S,5aS,6S,9R)-2-(3-((tert-butoxycarbonyl)amino)-8-((triisopropylsilyl)ethynyl)naphthalen-1-yl)-1-fluoro-12-methoxy-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.035 mmol) in N,N-dimethylformamide (3 mL). The reaction mixture was stirred at room temperature for 2 hours. Upon completion of the reaction (monitored by LCMS), the reaction mixture was diluted with ethyl acetate (20 mL), washed with water (15 mL × 2) and saturated brine (15 mL × 3), dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-12-methoxy-5-methyl-5a,6,7,8,9,10-hexahydro-5H-4-oxa-3,10a,11,13,14-pentaaza-6,9-methanonaphtho[1,8-ab]heptalene-14-carboxylate (24.5 mg, yield: 100%). ES-API: [M+H]⁺=697.1.

Step 3: Under an ice-water bath, a 4 M hydrochloric acid-dioxane solution (5 mL) was slowly added to a methanol (2 mL) solution of tert-butyl (5S,5aS,6S,9R)-2-(3-((tert-butoxycarbonyl)amino)-8-ethynylnaphthalen-1-yl)-1-fluoro-12-methoxy-5-methyl-5a,6,7,8,9,10-hexahydro-5H-4-oxa-3,10a,11,13,14-pentaaza-6,9-methanonaphtho[1,8-ab]heptalene-14-carboxylate (24.5 mg, 0.035 mmol). The reaction mixture was stirred at 0°C for 0.5 hours. Upon completion of the reaction (monitored by LCMS), the reaction mixture was concentrated under reduced pressure to dryness. The residue was purified by preparative HPLC (ammonium bicarbonate method 2) (chromatographic conditions: 15 mL-35-85-13 min) to obtain a pale yellow solid, 5-ethynyl-4-((5S,5aS,6S,9R)-1-fluoro-12-methoxy-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine (Z705, 3.4 mg, yield: 19 %). ES-API: [M+H]⁺= 497.2.

### Example 95 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ablheptalen-2-yl)-5-ethynylnaphthalen-2-amine (Z706)

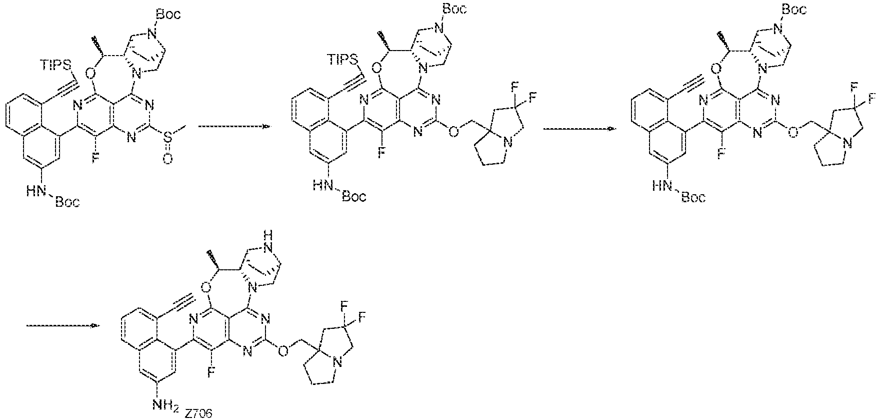

Step 1: (2,2-Difluoro-tetrahydro-1H-pyrrolizin-7a(5H)-yl)methanol (45 mg, 0.2542 mmol) was dissolved in anhydrous tetrahydrofuran (15 mL). Under an ice-water bath, sodium hydride (25.4 mg, 0.6356 mmol) was added and the mixture reacted for 30 minutes under the ice-water bath. Then, a tetrahydrofuran (5 mL) solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (112.0 mg, 0.127 mmol) was added dropwise to the above reaction mixture, and the reaction was continued for 30 minutes at room temperature. Upon completion of the reaction (monitored by LCMS), the reaction mixture was diluted with ethyl acetate (80 mL), washed with saturated ammonium chloride solution (15 mL × 2) and saturated brine (60 mL × 3), dried over anhydrous sodium sulfate, filtered, and concentrated. The crude product was purified by silica gel column chromatography (eluent: dichloromethane:methanol = 15:1) to obtain tert-butyl (SS,SaS,6S,9R)-2-(3-((tert-butoxycarbonyl)amino)-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (180 mg, crude product). ES-API: [M+H]⁺= 998.3.

Step 2: Cesium fluoride (900 mg, 5.921 mmol) was added to N,N-dimethylformamide (10 mL) containing tert-butyl (5S,5aS,6S,9R)-2-(3-((tert-butoxycarbonyl)amino)-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (180 mg, crude product). The reaction mixture was stirred at room temperature for 2 hours. Upon completion of the reaction (monitored by LCMS), the reaction mixture was diluted with ethyl acetate (60 mL), washed with water (15 mL × 2), and saturated brine (60 mL × 3), dried over anhydrous sodium sulfate, filtered, and concentrated. The crude product was obtained as tert-butyl (SS,SaS,6S,9R)-2-(3-((tert-butoxycarbonyl)amino)-8-ethynylnaphthalen-1 -yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (110 mg, crude product). ES-API: [M+H]⁺= 842.2.

Step 3: Under an ice-water bath, a 4 M hydrochloric acid/dioxane solution (5 mL) was slowly added to a acetonitrile (10.0 mL) solution of tert-butyl (5S,5aS,6S,9R)-2-(3-((tert-butoxycarbonyl)amino)-8-ethynylnaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (110 mg, crude product). The reaction mixture was stirred at 0°C for 0.5 hours. Upon completion of the reaction (monitored by LCMS), the reaction mixture was concentrated under reduced pressure to dryness. The residue was purified by preparative HPLC (ammonium bicarbonate method 2) to obtain a pale yellow solid, 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z706, 3.06mg, total yield for 5 steps18.2%). ES-API: [M+H]⁺= 642.2. ¹H NMR (400 MHz, CD₃OD) δ7.69-7.66(m, 1H), 7.41-7.25 (m, 2H), 7.14-7.00 (m, 2H), 5.43-5.40 (m, 1H), 4.60-4.10(m, 4H), 3.82-3.72(m, 2H), 3.41-3.39(m, 1H), 3.25-2.84(m, 4H), 2.87-2.85 (m, 1H), 2.60-2.56(m, 1H), 2.35-2.28 (m, 1H), 2.21-1.84 (m, 8H), 1.59-1.56 (m, 3H).

### Example 96 Synthesis of 5-ethynyl-3-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-2-yl)naphthalen-2-amine (Z707)

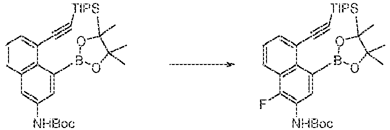

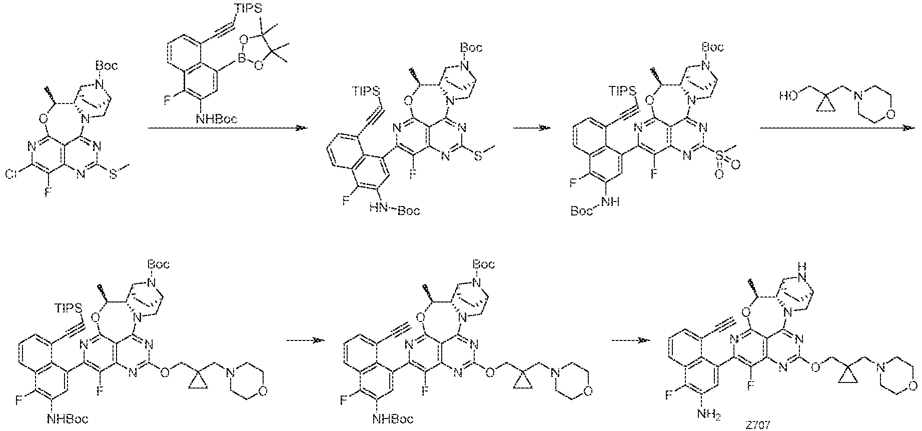

Step 1: 1-Chloromethyl-4-fluoro-1,4-diazabicyclo[2.2.2]octane bis(tetrafluoroborate) salt (354 mg, 1 mmol) was added to a solution of tert-butyl (4-(4,4,5,5-tetramethyl-1,3,2-dioxaborolan-2-yl)-5-((triisopropylsilyl)ethynyl)naphthalen-2-yl)carbamate (550 mg, 1 mmol) in acetonitrile (40 mL). The reaction mixture was stirred at room temperature for 8 hours. Upon completion of the reaction, as monitored by LCMS, the reaction mixture was concentrated under reduced pressure. The crude product was purified by flash silica gel column chromatography (petroleum ether/ethyl acetate = 0 - 1%) to obtain a pale yellow solid, tert-butyl (1-fluoro-4-(4,4,5,5-tetramethyl-1,3,2-dioxaborolan-2-yl)-5-((triisopropylsilyl)ethynyl)naphthalen-2-yl)carbamate (148 mg, yield: 26%). ES-API: [M+ H]⁺= 568.3.

Step 2: tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.166 mmol) and tert-butyl (1-fluoro-4-(4,4,5,5-tetramethyl-1,3,2-dioxaborolan-2-yl)-5-((triisopropylsilyl)ethynyl)naphthalen-2-yl)carbamate (90 mg, 0.159 mmol) were dissolved in tetrahydrofuran (5 mL) and water (1 mL). To this solution, methanesulfonic acid [butylbis(1-adamantyl)phosphine](2-amino-1,1'-biphenyl)palladium(II) (12.09 mg, 0.017 mmol) and potassium phosphate (105.70 mg, 0.498 mmol) were added. After the addition was complete, the reaction mixture was stirred at 65°C for 5 hours. Upon completion of the reaction, water (15 mL) was added to the reaction mixture, which was then extracted with ethyl acetate (15 mL × 3). The organic phase was washed with saturated brine (30 mL), dried over anhydrous sodium sulfate, filtered, and concentrated. The crude product was purified by flash silica gel column (ethyl acetate/petroleum ether: 0-30%) to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (35 mg, 0.039 mmol, yield 23.77%), a white solid. ES-API: [M +H]⁺= 887.3.

Step 3: Tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (35 mg, 0.039 mmol) was dissolved in dichloromethane (3 mL). At room temperature, m-chloroperoxybenzoic acid (85% purity, 24.03 mg, 0.118 mmol) was added to the solution. After the addition was complete, the reaction mixture was stirred at room temperature for 1 hour. Saturated sodium thiosulfate solution (2 mL) was added to the reaction mixture, followed by extraction with dichloromethane (3 mL × 3). The combined organic phases were washed successively with saturated bicarbonate solution (10 mL) and saturated sodium chloride solution (10 mL). The organic phase was dried over anhydrous sodium sulfate, filtered, and concentrated to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (35 mg, 0.038 mmol, yield: 96.53%) as a pale yellow solid. ES-API: [M +H]⁺= 919.3.

Step 4: (1-(Morpholinomethyl)cyclopropyl)methanol (3 mg, 0.018 mmol) was dissolved in tetrahydrofuran (1 mL) and cooled to 0°C. Under a nitrogen atmosphere, sodium hydride (60%, 3.05 mg, 0.076 mmol) was added to the solution, which was then stirred for 30 minutes. Then, tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (35 mg, 0.038 mmol) was slowly added to the solution which was then stirred at 0°C for 2 hours. Water (2 mL) was added to quench the reaction, followed by extraction with ethyl acetate (5 mL × 3). The organic phase was washed with saturated brine (5 mL × 3), dried over anhydrous sodium sulfate, and filtered and concentrated to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (30 mg, crude product), a yellow solid. ES-API: [M+H]⁺=1010.3.

Step 5: A mixted solution of tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (30 mg, crude product), cesium fluoride (45.10 mg, 0.297 mmol) and N, N-dimethylformamide (1 mL) was stirred at room temperature for 2 hours. Water (5 mL) was added to the reaction mixture, which was then extracted with ethyl acetate (5 mL × 2). The organic phase was washed with saturated brine (10 mL × 3), dried over anhydrous sodium sulfate, filtered, and concentrated to obtain tert-butyl (5S,5aS,6S,9R)-2-(3-((tert-butoxycarbonyl)amino)-8-ethynyl-4-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a, 11,13,14-pentaaza-6,9-methanonaphtho[1,8-ab]heptalene-14-carboxylate (25 mg, crude product), a yellow oily liquid. ES-API: [M+H]⁺=854.2.

Step 6: Tert-butyl (5S,5aS,6S,9R)-2-(3-((tert-butoxycarbonyl)amino)-8-ethynyl-4-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (25 mg, crude product) was dissolved in methanol (1 mL) and cooled to 0°C. Hydrochloric acid in dioxane solution (4 M, 1 mL) was added to the solution, and the reaction mixture was stirred at 0°C for 2 hours. The reaction mixture was concentrated, then redissolved in dichloromethane (2 mL) and basified to pH = 8 with a methanol solution of ammonia (7 M). The obtained solution was concentrated, and the crude product was purified by preparative HPLC (formic acid method 1) to obtain 5-ethynyl-3-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-meffimonaphtho[1,8-ab]heptalene-2-yl)naphthalen-2-ainine (Z707, 1.5 mg, 0.002 mmol, 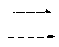 formate, total yield for 3 steps5%), white solid. ES-API: [M+H]⁺=654.2.

### Example 97 Synthesis of 5-ethynyl-6-fluoro-4-((5S,5aS,6S,9R)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ablheptalene-2-yl)naphthalen-2-ol (Z708)

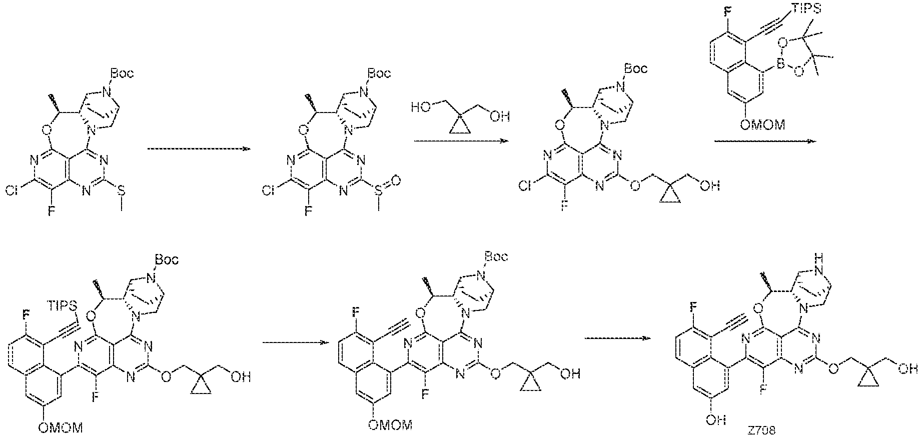

Step 1: Tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (100 mg, 0.21 mmol) and dichloromethane (3 mL) were added to a round-bottom flask. The mixture was cooled to 0°C, and m-chloroperoxybenzoic acid (54 mg, 0.31 mmol) was added. The reaction mixture was stirred at room temperature for 1 hour. Saturated sodium bicarbonate solution (30 mL) was then added to the reaction mixture. The mixture was extracted with ethyl acetate (20 mL × 2). The organic phase was washed with saturated brine (30 mL), dried over anhydrous sodium sulfate, filtered, and concentrated to obtain tert-butyl (5S,5aS,6S,9R)-2-chloro-1 -fluoro-5-methyl-12-(methylsulfmyl)-5a,6,7,8,9,10-hexahydro-5H-4-oxa-3,10a, 11,13,14-pentaaza-6,9-methanonaphtho[1,8-ab]heptalene-14-carboxylate (98 mg, crude product). ES-API: [M+H]⁺=498.2

Step 2: 60% sodium hydride (33 mg, 1.38 mmol) and tetrahydrofuran (6 mL) were added to a round-bottom flask. The mixture was cooled to 0°C, and a solution of cyclopropane-1,1-dicarboxaldehyde (121 mg, 1.18 mmol) in anhydrous tetrahydrofuran (1 mL) was added. The reaction was proceeded at room temperature for 10 minutes. The reaction mixture was then cooled to 0°C, and tert-butyl (SS,SaS,6S,9R)-2-chloro-1-fluoro-5-methyl-12-(methylsulfinyl)-Sa,6,7,8,9,10-hexahydro-SH-4-oxa-3,10a,11,13,14-pentaaza-6,9-methanonaphtho[1,8-ab]heptalene-14-carboxylate (98 mg, crude product) was added, and the reaction continued at room temperature for 0.5 hours. Water (30 mL) was then added to the reaction mixture. The mixture was extracted with ethyl acetate (20 mL × 2). The organic phase was washed with saturated brine (30 mL), dried over anhydrous sodium sulfate, filtered, and concentrated. The residue was purified by flash silica gel column chromatography (0-30% ethyl acetate/petroleum ether) to obtain tert-butyl (5S,5aS,6S,9R)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, yield 37%). ES-API: [M+H]⁺=536.2.

Step 3: Tert-butyl (5S,5aS,6S,9R)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.075 mmol), ((2-fluoro-6-(methoxymethoxy)-8-(4,4,5,5-tetramethyl-1,3,2-dioxaborolane-2-yl)naphthalen-1-yl)ethynyl)triisopropylsilane (76 mg, 0.15 mmol), methanesulfonic acid [butylbis(1-adamantyl)phosphine](2-amino-1,1'-biphenyl-2-yl)palladium(II) (5 mg, 0.007 mmol), potassium phosphate (47 mg, 0.22 mmol), tetrahydrofuran (3 mL) and water (0.6 mL) were added to a round-bottom flask. Under nitrogen protection, the system was heated in a microwave reactor at 80°C for 40 minutes. Water (30 mL) was added to the reaction mixture, and the mixture was extracted with ethyl acetate (30 mL × 3). The organic phase was dried over anhydrous sodium sulfate, filtered, and concentrated. The crude product was purified by a flash silica gel column (ethyl acetate/petroleum ether: 0-50%) to obtain tert-butyl (SS,SaS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (35 mg, yield 53%). ES-API: [M+H]⁺=886.2.

Step 4: Tert-butyl (5S,5aS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (35 mg, 0.039 mmol), cesium fluoride (60 mg, 0.39 mmol) and N, N-dimethylformamide (2 mL) were added to a round-bottom flask. The reaction mixture was stirred at room temperature for 1 hour. Water (30 mL) was added to the reaction mixture, followed by extraction with ethyl acetate (30 mL × 3). The organic phase was washed with saturated brine (30 mL × 2), dried over anhydrous sodium sulfate, filtered, and concentrated to obtain tert-butyl (5S,5aS,6S,9R)-2-(8-ethynyl-7-fluoro-3-(methoxymethoxy)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, yield 86%). ES-API: [M+H]⁺=730.2.

Step 5: Tert-butyl (5S,5aS,6S,9R)-2-(8-ethynyl-7-fluoro-3-(methoxymethoxy)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.034 mmol) was dissolved in acetonitrile (2 mL). The solution was cooled to 0°C, and 4M hydrochloric acid/dioxane solution (2 mL, 8 mmol) was added. The reaction mixture was stirred at 5°C for 1 hour. The reaction mixture was concentrated at 40°C, and the crude product was purified by preparative HPLC (bicarbonate method 1) to obtain the final product, S-ethynyl-6-fluoro-4-((SS,SaS,6S,9R)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-ol (Z708, 4mg, yield: 20%). ¹HNMR(400 MHz, CD₃OD): 7.85-7.80 (m, 1H), 7.33-7.27 (m, 2H), 7.22-7.11 (m, 1H), 5.42-5.38 (m, 1H), 4.57-4.52 (m, 1H), 4.42 (s, 2H), 4.11-4.08 (m, 1H), 3.76-3.52 (m, 3H), 3.22-3.12 (m, 1H), 2.10-2.03 (m, 1H), 1.95-1.81 (m, 3H), 1.59-1.56 (m, 3H), 1.40-1.25 (m, 2H), 0.67-0.60 (m, 4H).ES-API: [M+H]⁺=586.1.

### Example 98 Synthesis of 5-ethynyl-4-((5S,5aS,8R)-1-fluoro-5,8-dimethyl-11-((1-(morpholinomethyl)cyclopropyl)methoxy)-5,5a,6,7,8,9-hexahydro-4-oxa-3,7,9a,10,12-pentaazabenzo[4,5]cyclohepta[1,2,3-de]naphthalen-2-yl)naphthalen-2-amine (Z709)

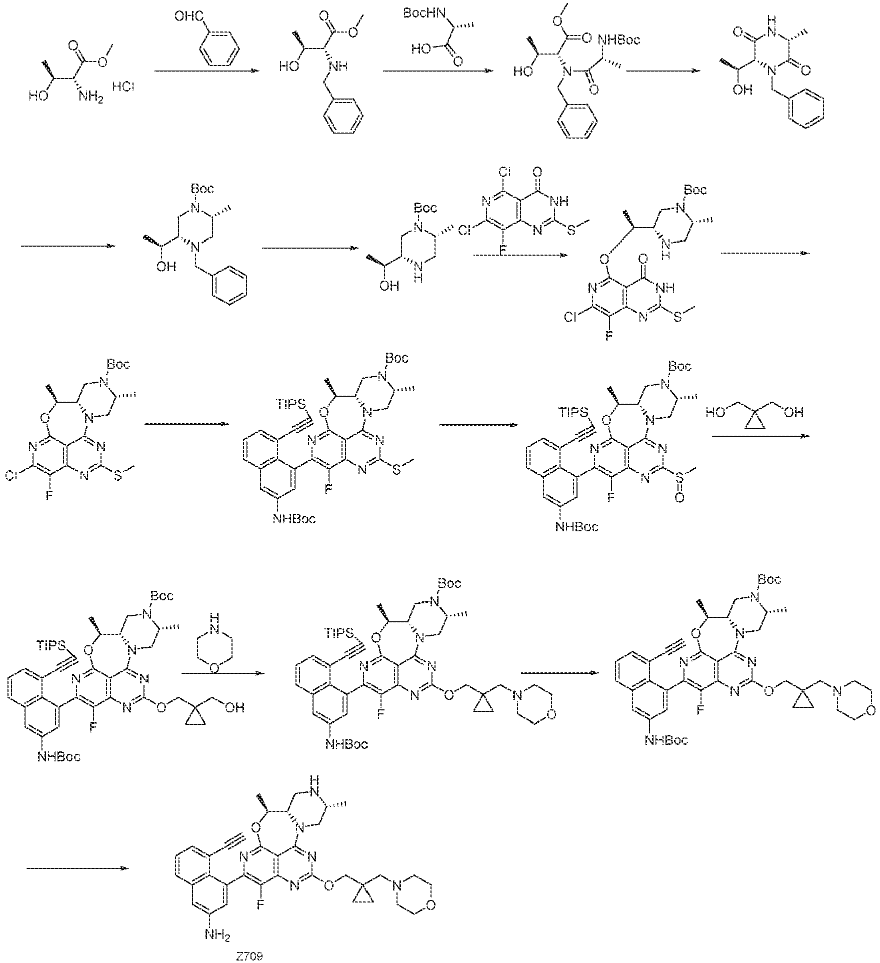

Step 1: Methyl D-threoninate hydrochloride (4.5 g, 26.53 mmol) was dissolved in methanol (50 mL) and benzaldehyde (2.82 g, 26.53 mmol) was added at 0 °C. The reaction mixture was stirred for 10 minutes at 0 °C, then at room temperature for 6 hours. The reaction mixture was cooled to 0 °C, and sodium borohydride (1.5 g, 39.80 mmol) was added in batches. The reaction mixture was stirred at room temperature for 18 hours. The reaction was quenched with saturated ammonium chloride solution (30 mL) and water (30 mL), and extracted with ethyl acetate (60 mL × 3). The combined organic phase was washed with saturated brine (50 mL). The organic phase was dried over anhydrous sodium sulfate, filtered, and concentrated. The crude product was purified by flash silica gel column chromatography (tetrahydrofuran/petroleum ether: 0-20%) to obtain methyl benzyl-D-threoninate (5.0 g, yield 84.4%), a colorless liquid. ES-API: [M+H]⁺= 224.0.

Step 2: N-(tert-butoxycarbonyl)-D-alanine (8.47 g, 44.79 mmol) and N-methylmorpholine (4.53 g, 44.79 mmol) were dissolved in tetrahydrofuran (80 mL). Isobutyl chloroformate (4.59 g, 33.59 mmol) was added dropwise at 0 °C, and the reaction was slowly brought to room temperature and stirred for 1 hour. The reaction mixture was cooled to 0 °C, and a solution of methyl benzyl-D-threoninate (5.0 g, 22.39 mmol) and N-methylmorpholine (3.40 g, 33.59 mmol) in tetrahydrofuran (20 mL) was added. The reaction mixture was stirred at room temperature for 48 hours. The reaction mixture was added to ethyl acetate (200 mL), and successively washed with 1N dilute hydrochloric acid (100 mL × 2), saturated sodium bicarbonate solution (60 mL), and saturated brine (60 mL). The organic phase was dried over anhydrous sodium sulfate and filtered. The filtrate was concentrated under reduced pressure. The crude product was purified by flash silica gel column chromatography (tetrahydrofuran/petroleum ether: 0-30%) to obtain methyl N-benzyl-N-((tert-butoxycarbonyl)-D-alanyl)-D-threoninate (2.3 g, yield 26.0%), a colorless liquid. ES-API: [M+H]⁺= 395.2.

Step 3: Methyl N-benzyl-N-((tert-butoxycarbonyl)-D-alanyl)-D-threoninate (2.3 g, 5.83 mmol) was dissolved in dichloromethane (25 mL), and trifluoroacetic acid (5 mL) was added. The reaction mixture was stirred at room temperature for 2 hours. The reaction mixture was concentrated under reduced pressure, with dichloromethane (20 mL) added, then basified with saturated sodium bicarbonate solution. The reaction mixture was stirred at room temperature for 1 hour. The reaction mixture was extracted with dichloromethane (50 mL). The organic phase was dried over anhydrous sodium sulfate and filtered. The filtrate was concentrated under reduced pressure. The crude product was purified by flash silica gel column chromatography (tetrahydrofuran/petroleum ether: 0-70%) to obtain (3R,6R)-1-benzyl-6-((S)-1-hydroxyethyl)-3-methylpiperazine-2,5-dione (1.2 g, yield 78.5%), a white solid. ES-API: [M+H]⁺= 263.1.

Step 4: (3R,6R)-1-Benzyl-6-((S)-1-hydroxyethyl)-3-methylpiperazine-2,5-dione (850 mg, 3.24 mmol) was dissolved in dimethoxyethane (50 mL). Under nitrogen protection at 0 °C, sodium borohydride (1.23 g, 32.40 mmol) was added, followed by a slow dropwise addition of boron trifluoride etherate (2.76 g, 19.44 mmol). The reaction mixture was heated to reflux for 4 hours. The reaction mixture was cooled to 0 °C, methanol (40 mL) was added slowly, followed by concentrated hydrochloric acid (17 mL). The reaction mixture was heated to reflux for 70 minutes. The reaction mixture was cooled to 0 °C, adjusted to pH 10 with 5M sodium hydroxide solution, and dichloromethane (40 mL) and di-tert-butyl dicarbonate (1.06 g, 4.86 mmol) were added. The mixture was stirred at room temperature for 30 minutes. The reaction mixture was extracted with dichloromethane (50 mL). The organic phase was dried over anhydrous sodium sulfate and filtered. The filtrate was concentrated under reduced pressure. The crude product was purified by flash silica gel column chromatography (tetrahydrofuran/petroleum ether: 0-20%) to obtain tert-butyl (2R,5S)-4-benzyl-5-((S)-1-hydroxyethyl)-2-methylpiperazine-1-carboxylate (920 mg, yield 84.9%), a white solid. ES-API: [M+H]⁺= 335.2.

Step 5: Tert-butyl (2R,5S)-4-benzyl-5-((S)-1-hydroxyethyl)-2-methylpiperazine-1-carboxylate (500 mg, 1.50 mmol) was dissolved in methanol (15 mL) and 7M ammonium methanol solution (1.5 mL). 10% palladium on carbon (200 mg) was added, and the reaction mixture was stirred under a hydrogen atmosphere at room temperature for 18 hours. The reaction mixture was filtered through diatomaceous earth, the filter cake was washed with methanol, and the filtrate was concentrated to obtain tert-butyl (2R,5S)-5-((S)-1-hydroxyethyl)-2-methylpiperazine-1-carboxylate (365 mg, yield 100%), a colorless liquid. ES-API: [M+H]⁺= 245.1.

Step 6: Tert-butyl (2R,SS)-5-((S)-1-hydroxyethyl)-2-methylpiperazine-1-carboxylate (365 mg, 1.49 mmol) was dissolved in tetrahydrofuran (25 mL). At 0 °C, 60% sodium hydride (299 mg, 7.47 mmol) was added. After the mixture stirred at 0 °C for 30 minutes, 5,7-dichloro-8-fluoro-2-(methylthio)pyrido[4,3-d]pyrimidin-4(3H)-one (544 mg, 1.94 mmol) was added. The reaction mixture was stirred at room temperature for 18 hours. The reaction mixture was quenched with saturated ammonium chloride solution (4 mL) and water (10 mL), then extracted with ethyl acetate (60 mLX2). The combined organic phases were washed with saturated brine (30 mL). The organic phase was dried over anhydrous sodium sulfate, filtered, and concentrated to obtain tert-butyl (2R,5S)-5-((S)-1-((7-chloro-8-fluoro-2-(methylthio)-4-oxo-3,4-dihydropyrido[4,3-d]pyrimidin-5-yl)oxy)ethyl)-2-methylpiperazine-1-carboxylate (728 mg, yield 100%), a pale yellow solid. ES-API: [M+H]⁺= 488.0.

Step 7: Tert-butyl (2R,5S)-5-((S)-1-((7-chloro-8-fluoro-2-(methylthio)-4-oxo-3,4-dihydropyrido[4,3-d]pyrimidin-5-yl)oxy)ethyl)-2-methylpiperazine-1-carboxylate (728 mg, 1.49 mmol) and diisopropylethylamine (964 mg, 7.46 mmol) were dissolved in dichloromethane (15 mL). At room temperature, 1-propylphosphonic anhydride 50% ethyl acetate solution (2.85 g, 4.48 mmol) was added, and the reaction mixture was stirred at room temperature for 3 hours. The reaction mixture was extracted with dichloromethane (30 mL), then successively washed with water (20 mLX2) and saturated brine (20 mL). The organic phase was dried over anhydrous sodium sulfate, filtered, and concentrated. The crude product was purified by flash silica gel column chromatography (tetrahydrofuran/petroleum ether: 0-20%) to obtain tert-butyl (5S,5aS,8R)-2-chloro-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalene-7(5H)-carboxylate (500 mg, yield 71.3%), a white solid. ES-API: [M+H]⁺= 470.1.

Step 8: To a mixture of tert-butyl (5S,5aS,8R)-2-chloro-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalene-7(5H)-carboxylate (100 mg, 0.21 mmol) and tert-butyl (4-(4,4,5,5-tetramethyl-1,3,2-dioxaborolan-2-yl)-5-((triisopropylsilyl)ethynyl)naphthalen-2-yl)carbamate (117 mg, 0.21 mmol) in tetrahydrofuran (5 mL) and water (1 mL), potassium phosphate (136 mg, 0.64 mmol), methanesulfonic acid butyl di(1-adamantyl)phosphine(2-amino-1,'-biphenyl-2-yl)palladium(II) (12 mg, 0.017 mmol) were added. After purged with nitrogen three times, the mixture was heated at 80°C and reacted for 3 hours. The reaction mixture was extracted with ethyl acetate (60 mL) and washed with saturated brine (30 mL). The organic phase was dried over anhydrous sodium sulfate, filtered, and concentrated under reduced pressure. The crude product was purified by flash silica gel column chromatography (tetrahydrofuran/petroleum ether: 0-25%) to obtain tert-butyl (5S,5aS,8R)-2-(3-((tert-butoxycarbonyl)amino)-8-((triisopropylsilyl)ethynyl)naphthalen-1-yl)-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalene-7(5H)-carboxylate (80 mg, yield 43.9%), a pale yellow solid. ES-API: [M+H]⁺=857.2.

Step 9: Tert-butyl (5S,5aS,8R)-2-(3-((tert-butoxycarbonyl)amino)-8-((triisopropylsilyl)ethynyl)naphthalen-1-yl)-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalene-7(5H)-carboxylate (80 mg, 0.093 mmol) was dissolved in dichloromethane (10 mL). At room temperature, m-chloroperoxybenzoic acid (28 mg, 0.14 mmol) was added, and the reaction mixture was stirred at room temperature for 1 hour. The reaction mixture was extracted with dichloromethane (20 mL), then successively washed with saturated sodium thiosulfate (2 mL), saturated bicarbonate (5 mL), and saturated brine (5 mL). The organic phase was dried over anhydrous sodium sulfate, filtered, and concentrated under reduced pressure to obtain tert-butyl (5S,5aS,8R)-2-(3-((tert-butoxycarbonyl)amino)-8-((triisopropylsilyl)ethynyl)naphthalen-1-yl)-1-fluoro-5,8-dimethyl-11-(methylsulfinyl)-5a,6,8,9-tetrahydro-4-oxa-3,7,9a,10,12-pentaazabenzo[4,5]cyclohepta[1,2,3-de]naphthalene-7(5H)-carboxylate (80 mg, yield 98.2%), a pale yellow solid. ES-API:[M+H]⁺=873.3.

Step 10: 1,1-Cyclopropanedimethanol (37 mg, 0.37 mmol) was dissolved in tetrahydrofuran (8 mL). At 0°C, 60% sodium hydride (11 mg, 0.28 mmol) was added, and the mixture was stirred for 30 minutes at this temperature. Then, a tetrahydrofuran solution (2 mL) of tert-butyl (5S,5aS,8R)-2-(3-((tert-butoxycarbonyl)amino)-8-((triisopropylsilyl)ethynyl)naphthalen-1-yl)-1-fluoro-5,8-dimethyl-11-(methylsulfinyl)-Sa,6,8,9-tetiahydro-4-oxa-3,7,9a,10,12-pentaazabenzo[4,5]cyclohepta[1,2,3-de]naphthalene-7(5H)-carboxylate (80 mg, 0.092 mmol) was added dropwise, and the reaction mixture was stirred for 30 minutes at the same temperature. The reaction mixture was extracted with ethyl acetate (40 mL), successively washed with water (10 mL) and saturated brine (15 mL). The organic phase was dried over anhydrous sodium sulfate, filtered, and concentrated under reduced pressure. The crude product was purified by flash silica gel column chromatography (tetrahydrofuran/petroleum ether: 0-50%) to obtain the target product tert-butyl (5S,5aS,8R)-2-(3-((tert-butoxycarbonyl)amino)-8-((triisopropylsilyl)ethynyl)naphthalen-1-yl)-1-fluoro-11-((1-(hydroxymethyl)cyclopropyl)methoxy)-5,8-dimethyl-5a,6,8,9-tetrahydro-4-oxa-3,7,9a,10,12-pentaazabenzo[4,5]cyclohepta[1,2,3-de]naphthalene-7(5H)-carboxylate (50 mg, yield 59.9%), a pale yellow solid. ES-API: [M+H]⁺=911.3.

Step 11: Tert-butyl (5S,5aS,8R)-2-(3-((tert-butoxycarbonyl)amino)-8-((triisopropylsilyl)ethynyl)naphthalen-1-yl)-1-fluoro-11-((1-(hydroxymethyl)cyclopropyl)methoxy)-5,8-dimethyl-5a,6,8,9-tetrahydro-4-oxa-3,7,9a,10,12-pentaazabenzo[4,5]cyclohepta[1,2,3-de]naphthalene-7(5H)-carboxylate (50 mg, 0.055 mmol) and diisopropylethylamine (35 mg, 0.275 mmol) were dissolved in N,N-dimethylformamide (3 mL). At 0°C, methanesulfonyl chloride (19 mg, 0.165 mmol) in tetrahydrofuran (0.5 mL) was added dropwise, and the mixture was stirred for 1 hour at the same temperature. Then, morpholine (96 mg, 1.10 mmol) and potassium carbonate (61 mg, 0.44 mmol) were added dropwise, and the reaction mixture was stirred at 50°C for 24 hours. The reaction mixture was extracted with ethyl acetate (40 mL), successively washed with water (10 mL) and saturated brine (15 mL × 3), dried over anhydrous sodium sulfate, filtered, and concentrated under reduced pressure. The crude product was purified by thin-layer chromatography (dichloromethane/methanol = 25:1) to obtain the target product tert-butyl (5S,5aS,8R)-2-(3-((tert-butoxycarbonyl)amino)-8-((triisopropylsilyl)ethynyl)naphthalen-1-yl)-1 -fluoro-5,8-dimethyl-11 -((1 -(morpholinomethyl)cyclopropyl)methoxy)-5a,6,8,9-tetrahydro-4-oxa-3,7,9a,10,12-pentaazabenzo[4,5]cyclohepta[1,2,3-de]naphthalene-7(5H)-carboxylate (20 mg, yield 37.2%), a white solid. ES-API: [M+H]⁺=980.3.

Step 12: Tert-butyl (5S,5aS,8R)-2-(3-((tert-butoxycarbonyl)amino)-8-((triisopropylsilyl)ethynyl)naphthalen-1-yl)-1-fluoro-5,8-dimethyl-11-((1-(morpholinomethyl)cyclopropyl)methoxy)-5a,6,8,9-tetrahydro-4-oxa-3,7,9a,10,12-pentaazabenzo[4,5]cyclohepta[1,2,3-de]naphthalene-7(5H)-carboxylate (20 mg, 0.020 mmol) was dissolved in N,N-dimethylformamide (1.5 mL). At room temperature, cesium fluoride (31 mg, 0.20 mmol) was added, and the reaction mixture was stirred for 30 minutes at room temperature. The reaction mixture was extracted with ethyl acetate (25 mL), successively washed with water (5 mL), dilute brine (10 mL × 3), and saturated brine (10 mL). The organic phase was dried over anhydrous sodium sulfate, filtered, and concentrated under reduced pressure to obtain the target product tert-butyl (5S,5aS,8R)-2-(3-((tert-butoxycarbonyl)amino)-8-ethynylnaphthalen-1-yl)-1-fluoro-5,8-dimethyl-11-((1 -(morpholinomethyl)cyclopropyl)methoxy)-5a,6,8,9-tetrahydro-4-oxa-3,7,9a,10,12-pentaazabenzo[4,5]cyclohepta[1,2,3-de]naphthalene-7(5H)-carboxylate (15 mg, yield 89.2%), a pale yellow solid. ES-API: [M+H]⁺=824.2.

Step 13: Tert-butyl (5S,5aS,8R)-2-(3-((tert-butoxycarbonyl)amino)-8-ethynylnaphthalen-1-yl)-l-fluoro-5,8-dimethyl-11-((1-(morpholinomethyl)cyclopropyl)methoxy)-5a,6,8,9-tetrahydro-4-oxa-3,7,9a,10,12-pentaazabenzo[4,5]cyclohepta[1,2,3-de]naphthalene-7(5H)-carboxylate (15 mg, 0.018 mmol) was dissolved in ethyl acetate (1 mL). At 0°C, 4M hydrochloric acid in 1,4-dioxane (2 mL) was added, and the reaction mixture was stirred at room temperature for 2 hours. The reaction mixture was concentrated under reduced pressure. The crude product was purified by preparative HPLC (ammonium bicarbonate method 1) to obtain the target product, 5-ethynyl-4-((5S,5aS,8R)-1-fluoro-5,8-dimethyl-11-((1-(morpholinomethyl)cyclopropyl)methoxy)-5,5a,6,7,8,9-hexahydro-4-oxa-3,7,9a,10,12-pentaazabenzo[4,5]cyclohepta[1,2,3-de]naphthalen-2-yl)naphthalen-2-amine (Z709, 4 mg, yield: 35.2%), white solid. ES-API: [M+H]⁺= 624.2.

### Example 99 Synthesis of 5-ethynyl-4-((5S,5aS,6S,9R)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine (Z710)

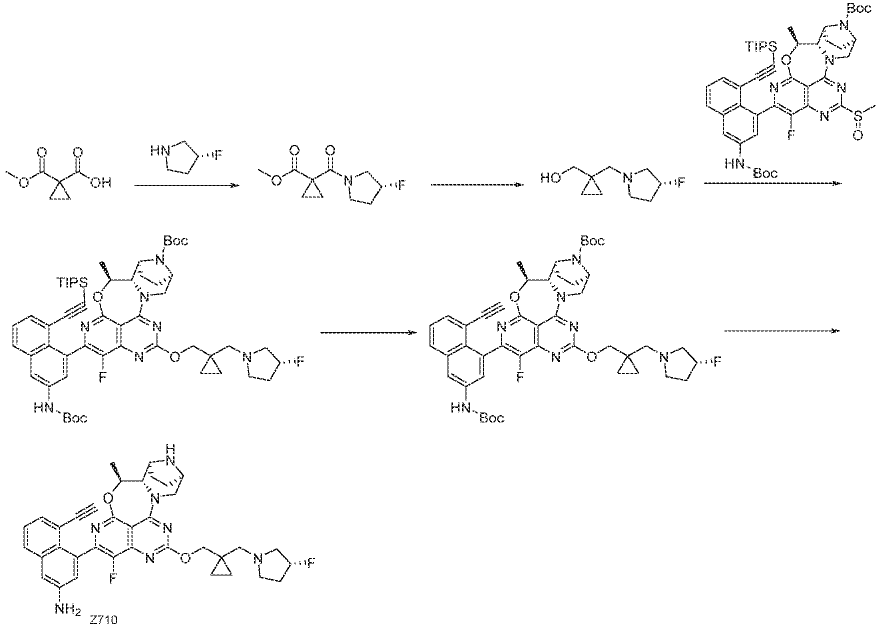

Step 1: To a solution of 1-(methoxycarbonyl)cyclopropane-1-carboxylic acid (1.4g, 9.713mmol) in dichloromethane (100 mL), oxalyl chloride (3.288 mL, 38.854 mmol) and N,N-dimethylformamide (0.05 mL) were added. The reaction mixture was stirred at 0°C, then at 35°C for 3 hours. The reaction mixture was concentrated to obtain a solution. The residue was dissolved in dichloromethane (50 mL), with triethylamine (6.750 mL, 48.565 mmol) and (3R)-3-fluorotetrahydropyrrole (0.95 g, 10.685 mmol) added at 0°C, and the mixture was stirred at room temperature for 3 hours. After filtration and concentration, the residue was purified by silica gel column chromatography (eluting with methanol in dichloromethane = 1:20) to obtain methyl (R)-1-(3-fluoropyrrolidine-1-carbonyl)cyclopropane-1-carboxylate (650 mg, 3.020 mmol, yield 31.09%). ES-API: [M+H]⁺=216.1.

Step 2: At -20°C, lithium aluminium hydride (1.859 mL, 1.859 mmol, 1M in tetrahydrofuran solution) was added to a tetrahydrofuran solution (15 mL) of methyl (R)-1-(3-fluoropyrrolidine-1-carbonyl)cyclopropane-1-carboxylate (650 mg, 3.020 mmol). The reaction mixture was stirred at - 10°C for 30 minutes. The reaction mixture was quenched with 15% sodium hydroxide solution (0.5 mL) and filtered through diatomaceous earth. The filtrate was dried over anhydrous sodium sulfate, filtered, and concentrated to obtain (R)-(1-((3-fluoropyrrolidin-1-yl)methyl)cyclopropyl)methanol (100 mg, 0.577 mmol, yield 62.12%), a yellow oil. ES-API: [M+H]⁺=174.1.

Step 3: Under ice-water bath condition, (R)-(1-((3-fluoropyrrolidin-1-yl)methyl)cyclopropyl)methanol (100 mg, 0.577 mmol) was dissolved in dry tetrahydrofuran (5 mL). Sodium hydride (60%, 12.88 mg, 0.322 mmol) was added under nitrogen protection, and the mixture reacted in an ice-water bath for 10 minutes. Then, tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (95 mg, 0.107 mmol) was added. Upon completion of the reaction, an aqueous solution of ammonium chloride (5 mL) was added, followed by extraction with ethyl acetate (30 mLx3). The combined organic phases were washed with saturated brine (30 mLx1), dried over anhydrous sodium sulfate, filtered, concentrated, and purified by silica gel column chromatography (petroleum ether/ethyl acetate=1/5) to obtain tert-butyl (5S,5aS,6S,9R)-2-(3-((tert-butoxycarbonyl)amino)-8-((triisopropylsilyl)ethynyl)naphthalen-1-yl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.030 mmol, yield 28.2%). ES-API: [M+H]⁺=994.5.

Step 4: Cesium fluoride (45.6 mg, 0.3 mmol) was added to a N,N-dimethylformamide solution (3 mL) of tert-butyl (5S,5aS,6S,9R)-2-(3-((tert-butoxycarbonyl)amino)-8-((triisopropylsilyl)ethynyl)naphthalen-1-yl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (30 mg, 0.030 mmol). The mixture was stirred at room temperature for 2 hours. Upon completion of the reaction (monitored by LCMS), the mixture was washed with ethyl acetate (20 mL), water (15 mLx2), and saturated brine (15 mLx3). The organic phase was dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-12-((1-(((R)-3-fluoropyrrolidin-1-yl)methyl)cyclopropyl)methoxy)-5-methyl-Sa,6,7,8,9,10-hexahydro-5H-4-oxa-3,10a,11,13,14-pentaaza-6,9-methanonaphtho[1,8-ab]heptalene-14-carboxylate (24.5 mg, yield: 98%). ES-API: [M+H]⁺= 838.3.

Step 5: Under an ice-water bath, a 4M hydrochloric acid/1,4-dioxane solution (2 mL) was slowly added to a solution of tert-butyl (5S,5aS,6S,9R)-2-(3-((tert-butoxycarbonyl)amino)-8-ethynylnaphthalen-1-yl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (24.5 mg, 0.029 mmol) in ethyl acetate (1 mL). The mixture was stirred at room temperature for 0.5 hours. Upon completion of the reaction (monitored by LCMS), the reaction mixture was concentrated to dryness under reduced pressure. The crude product was purified by preparative HPLC (ammonium bicarbonate method 2) to obtain a pale yellow solid, S-ethynyl-4-((SS,SaS,6S,9R)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine (Z710, 0.9 mg, yield: 4.9%). ES-API: [M+H]⁺=638.3.

### Example 100 Synthesis of 4-((5S,5aS,6S,9R)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z711)

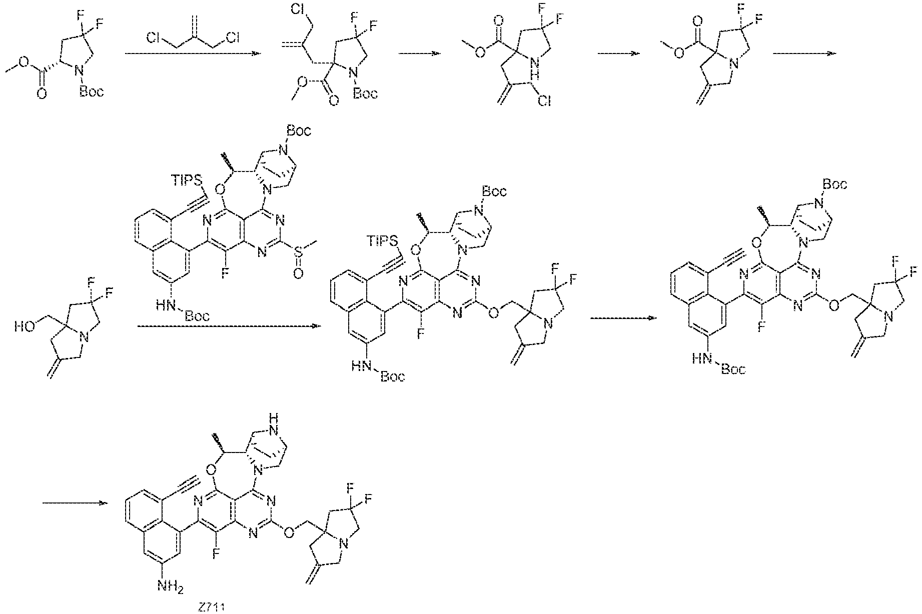

Step 1: Under nitrogen protection, (S)-1-tert-butyl 2-methyl 4,4-difluoropyrrolidine-1,2-dicarboxylate (1 g, 3.77 mmol) was dissolved in tetrahydrofuran (10 mL) and cooled to -60°C. Diisopropylamine lithium (7.54 mL, 7.54 mmol) was slowly added and the mixture was stirred for 1 hour. Then, 3-chloro-2-chloromethylpropene (0.71 g, 5.66 mmol) in tetrahydrofuran (5 mL) was added to the mixture, which was then slowly warmed to room temperature, and stirred for 2 hours. Upon completion of the reaction, the mixture was quenched with saturated ammonium chloride solution (10 mL) and extracted with ethyl acetate (10 mLx3). The organic phases were combined, dried over anhydrous sodium sulfate, filtered, concentrated, and purified by flash silica gel column chromatography (0-20% tetrahydrofuran/petroleum ether) to obtain a yellow oily substance, 1-(tert-butyl) 2-methyl 2-(2-(chloromethyl)allyl)-4,4-difluoropyrrolidine-1,2-dicarboxylate (450 mg, yield 34%). ES-API: [M+H⁺-56]= 298.0.

Step 2: Under ice water bath condition, compound 1-(tert-butyl) 2-methyl 2-(2-(chloromethyl)allyl)-4,4-difluoropyrrolidine-1,2-dicarboxylate (450 mg, 1.27 mmol) was dissolved in dichloromethane (6 mL). Trifluoroacetic acid (3 mL) was added and the mixture was stirred at room temperature for 1 hour. Upon completion of the reaction, the reaction mixture was concentrated to obtain methyl 2-(2-(chloromethyl)allyl)-4,4-difluoropyrrolidine-2-carboxylate (320 mg, crude product). ES-API: [M+H⁺]= 254.1.

Step 3: Compound methyl 2-(2-(chloromethyl)allyl)-4,4-difluoropyrrolidine-2-carboxylate (320 mg, 1.26 mmol) was dissolved in acetonitrile (10 mL). Sodium bicarbonate (546 mg, 6.51 mmol) and potassium iodide (22 mg, 0.13 mmol) were added and the mixture was stirred at room temperature for 1 hour. The reaction mixture was added to water (10 mL) and extracted with ethyl acetate (10 mLx3). The combined organic phases were dried over anhydrous sodium sulfate, filtered, concentrated, and the residue was purified by flash silica gel column chromatography (0-50% tetrahydrofuran/petroleum ether) to obtain a yellow oily substance, methyl 2,2-difluoro-6-methylenetetrahydro-1H-pyrrolizine-7a(5H)-carboxylate (220 mg, two-step yield 78%). ES-API[M+H⁺]= 218.1.

Step 4: Under ice water bath condition, a 1 M solution of lithium aluminum hydride in tetrahydrofuran (0.46 mL, 0.46 mmol) was added to a solution of methyl 2,2-difluoro-6-methylenetetrahydro-1H-pyrrolizine-7a(5H)-carboxylate (100 mg, 0.46 mmol) in tetrahydrofuran (2 mL). The mixture was stirred for 1 hour. Upon completion of the reaction, decahydrate sodium sulfate (2 g) was added to quench the reaction, amd the mixture was stirred for 10 minutes, filtered, and concentrated to obtain (2,2-difluoro-6-methylenetetrahydro-1H-pyrrolizin-7a(5H)-yl)methanol (85 mg, crude product). ES-API: [M+H⁺]= 190.1.

Step 5: Under ice water bath condition, (2,2difluoro-6-methylenetetrahydro-1H-pyrrolizin-7a(5H)-yl)methanol (72 mg, 0.38 mmol) in tetrahydrofuran (1 mL) was added to a solution of sodium hydride (40 g, 1.00 mmol) in tetrahydrofuran (1 mL), the mixture was stirred for 0.5 hours. Then a solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(metlxylsulfinyl)-Sa,6,7,8,9,10-hexahydro-5H-4-oxa-3,10a,11,13,14-pentaaza-6,9-methanonaphtho[1,8-ab]heptalene-14-carboxylate (112 mg, 0.13 mmol) in tetrahydrofuran (0.5 mL) was added and the mixture continued to be sitrred for 0.5 hours. Upon completion of the reaction, saturated ammonium chloride solution (10 mL) was added to quench the reaction, and the mixture was extracted with ethyl acetate (10 mLx3). The combined organic phases were dried over anhydrous sodium sulfate, filtered, concentrated, and purified by flash silica gel column chromatography (0-100% ethyl acetate/petroleum ether) to obtain a yellow oily substance, tert-butyl (5S,5aS,6S,9R)-2-(3-((tert-butoxycarbonyl)amino)-8-((triisopropylsilyl)ethynyl)naphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,1 1,1 3,14-pentaaza-6,9-methanonaphtho[1,8-ab]heptalene-14-carboxylate (60 mg, yield 47%). ES-API[M+H⁺]= 1010.2.

Step 6: Under nitrogen protection, the compound tert-butyl (5S,5aS,6S,9R)-2-(3-((tert-butoxycarbonyl)amino)-8-((triisopropylsilyl)ethynyl)naphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (70 mg, 0.07 mmol) was dissolved in N,N-dimethylformamide (1 mL). Cesium fluoride (105 mg, 0.69 mmol) was added and the mixture was stirred at room temperature for 1 hour. Ethyl acetate (10 mL) was added, and the mixture was washed successively with saturated saline (5 mL) and water (5 mL). The organic phase was dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (59 mg, crude product). ES-API[M+H⁺]= 854.2.

Step 7: The compound tert-butyl (5S,5aS,6S,9R)-2-(3-((tert-butoxycarbonyl)amino)-8-ethynylnaphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (59 mg, 0.07 mmol) was dissolved in ethyl acetate (1 mL). 4 M hydrochloric acid-dioxane (2 mL) was added and the mixture was stirred at room temperature for 2 hours. Upon completion of the reaction, the mixture was quenched with saturated bicarbonate solution and neutralized to pH = 8, extracted with dichloromethane (4 mLx3). The combined organic phases were dried over anhydrous sodium sulfate, filtered, concentrated, and purified by preparative HPLC (ammonium bicarbonate method 1) to obtain 4-((5S,5aS,6S,9R)-12-\((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z711, 11.95 mg, yield: 26%). ES-API[M+H⁺]= 654.3.

### Example 101 Synthesis of 4-((5S,5aS,6S,9R)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z712)

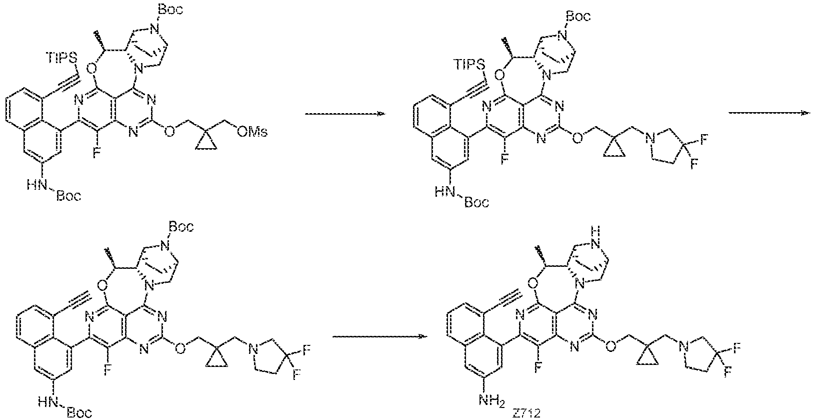

Step 1: Tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (45 mg, 0.05 mmol) was dissolved in acetone (2 mL). Then, 3,3-difluoropyrrolidine hydrochloride (19 mg, 0.14 mmol), potassium carbonate (37 mg, 0.27 mmol), and sodium iodide (3 mg, 0.02 mmol) were added. The mixture was microwaved at 80°C and reacted for 1 hour. Upon completion of the reaction, the mixture was diluted by adding ethyl acetate (5 mL), washed with water (5 mLX2) and sodium chloride (5 mLX2), dried over anhydrous sodium sulfate and filtered. The crude product was purified through silica gel using automatic flash chromatography (dichloromethane: methanol = 15:1) to obtain tert-butyl (5S,5aS,6S,9R)-2-(3-((tert-butoxycarbonyl)amino)-8-((triisopropylsilyl)ethynyl)naphthalen-1-yl)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (15 mg, 0.01 mmol, yield 32.9%). ES-API: [M/2+H]⁺= 507.1.

Step 2: Cesium fluoride (7 mg, 0.05mmol) was added to a solution of tert-butyl (5S,5aS,6S,9R)-2-(3-((tert-butoxycarbonyl)amino)-8-((triisopropylsilyl)ethynyl)naphthalen-1-yl)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (15 mg, 0.01 mmol) in N,N-dimethylformamide (2 mL). The reaction mixture was stirred at room temperature for 2 hours. Upon completion of the reaction (monitored by LC-MS), the mixture was diluted by adding ethyl acetate (5 mL), washed with water (4 mLX2) and sodium chloride (4 mLX3), dried over anhydrous sodium sulfate, filtered and concentrated to obtain tert-butyl (SS,SaS,6S,9R)-2-(3-((tert-butoxycarbonyl)amino)-8-ethynylnaphthalen-1-yl)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (12 mg, crude product). ES-API: [M+H]⁺= 857.2.

Step 3: Under an ice-water bath, 4 M hydrochloric acid-dioxane solution (2.0 mL, 4M, 8.0mmol) was slowly added to a solution of tert-butyl (5S,5aS,6S,9R)-2-(3-((tert-butoxycarbonyl)amino)-8-ethynylnaphthalen-1-yl)-12-((1-((3,3ilifluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (12 mg, 0.01 mmol) in ethyl acetate (1 mL). The reaction mixture was stirred at 0°C for 3 hours. Upon completion of the reaction (monitored by LC-MS), the reaction mixture was concentrated under reduced pressure to dryness. The residue was purified by preparative HPLC (formic acid method 2) to obtain 4-((SS,SaS,6S,9R)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z712, 0.18 mg, 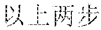 yield: 2.7%, formate). ES-API: [M+H]⁺= 656.1.

### Example 102 Synthesis of 4-((5S,5aS,6S,9R)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z713)

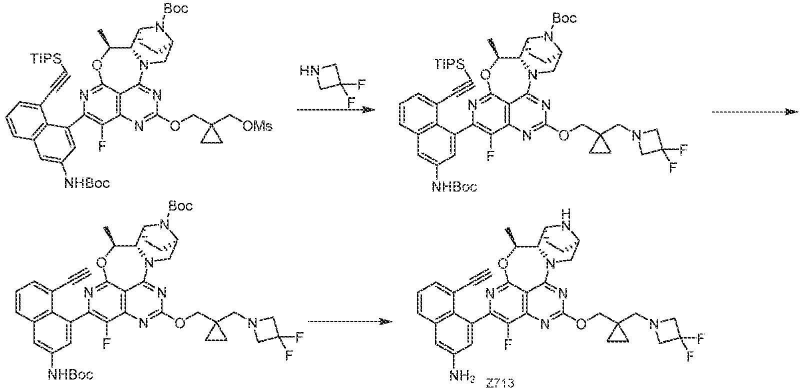

Step 1: Tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (80 mg, 0.080 mmol) was dissolved in acetone (1 mL). 3,3-Difluorotrimethylamine hydrochloride (74.37 mg, 0.719 mmol), potassium carbonate (44.10 mg, 0.320 mmol), and potassium iodide (53.05 mg, 0.320 mmol) were added, and the mixture was microwaved at 80 degrees Celsius and reacted for 1 hour. Upon completion of the reaction, the mixture was diluted by adding ethyl acetate (10 mL), washed with water (10 mL) and sodium chloride solution (10mL), dried over anhydrous sodium sulfate and filtered, the crude product was purified by silica gel column chromatography (dichloromethane: methanol = 15:1) to obtain tert-butyl (5S,5aS,6S,9R)-2-(3-((tert-butoxycarbonyl)amino)-8-((triisopropylsilyl)ethynyl)naphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (50 mg, 0.050 mmol, yield 62.69%). ES-API: [M+H]⁺= 998.4.

Step 2: Cesium fluoride (33.49 mg, 0.220 mmol) was added to a solution of tert-butyl (5S,5aS,6S,9R)-2-(3-((tert-butoxycarbonyl)amino)-8-((triisopropylsilyl)ethynyl)naphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (35 mg, 0.035 mmol) in N,N-dimethylformamide (1.0 mL). The reaction mixture was stirred at room temperature for 2 hours. Upon completion of the reaction (monitored by LC-MS), the mixture was diluted by adding ethyl acetate (10 mL), washed with water (10 mLX 2) and saturated saline (10 mLX 3). After dried over anhydrous sodium sulfate and filtration, the mixture was concentrated to obtain tert-butyl (5S,5aS,6S,9R)-2-(3-((tert-butoxycarbonyl)amino)-8-ethynylnaphthalen-1-yl)-12-((1-((3,3-difluoroazetidin-1 -yl)methyl)cyclopropyl)methoxy)-1 - fluoro-5-methyl-5a,6,7,8,9,10-hexahydro-5H-4-oxa-3,10a,11,13,14-pentaaza-6,9-methanonaphtho[1,8-ab]heptalene-14-carboxylate (28 mg, 0.033 mmol, yield 94.86%). ES-API: [M+H]⁺=842.3.

Step 3: Under an ice-water bath, trifluoroacetic acid (1.0 mL) was slowly added to a solution of tert-butyl (5S,5aS,6S,9R)-2-(3-((tert-butoxycarbonyl)amino)-8-ethynylnaphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (25 mg, 0.030 mmol) in dichloromethane (1.0 mL). The reaction mixture was stirred at 0°C for 0.5 hours. Upon completion of the reaction (monitored by LC-MS), the reaction mixture was concentrated under reduced pressure to dryness. The residue was purified by preparative HPLC (formic acid method 2) to obtain a white solid, 4-((5S,5aS,6S,9R)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z713, 1.7 mg, 0.002 mmol, yield: 20.81%, formate). ES-API: [M+H]⁺= 642.2.

### Example 103 Synthesis of 5-ethynyl-4-((5S,5aS,6S,9R)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ablheptalen-2-yl)naphthalen-2-amine (Z714)

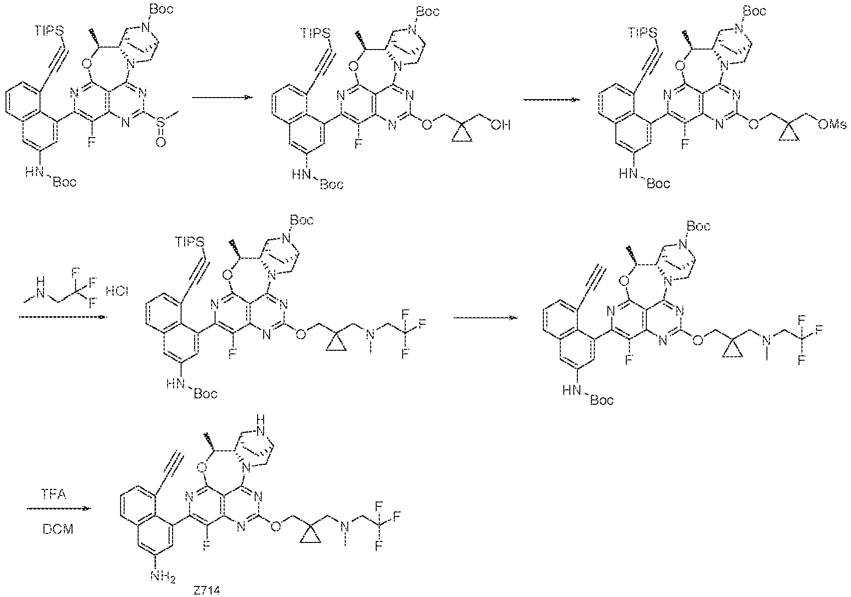

Step 1: 1,1-Cyclopropanedimethanol (220 mg, 0.25 mmol) was dissolved in anhydrous tetrahydrofuran (5 mL). Under an ice-water bath, sodium hydride (40 mg, 1.0 mmol) was added, and the mixture reacted for 30 minutes under the ice-water bath. A solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (127 mg, 1.2 mmol) in tetrahydrofuran (2 mL) was added dropwise to the above reaction mixture, and the reaction was proceeded for 30 minutes at room temperature. Upon completion of the reaction (monitored by LC-MS), the mixture was diluted by adding ethyl acetate (20 mL) and washed with saturated ammonium chloride solution (15 mLX 2) and saturated saline (15 mLX3). After dried over anhydrous sodium sulfate and filtration, the mixture was concentrated, and the crude product was purified by automatic flash chromatography (dichloromethane: methanol = 15:1 as mobile phase) through silica gel to obtain tert-butyl (5S,5aS,6S,9R)-2-(3-((tert-butoxycarbonyl)amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (180 mg, yield: 78%). ES-API: [M+H]⁺= 923.3.

Step 2: tert-butyl (5S,5aS,6S,9R)-2-(3-((tert-butoxycarbonyl)amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (180 mg, 0.19 mmol) was dissolved in anhydrous dichloromethane (5 mL). Under an ice-water bath, anhydrous N,N-diisopropylethylamine (76 mg, 0.6 mmol) and methanesulfonic anhydride (102 mg, 0.6 mmol) were added, and the reaction mixture was stirred at room temperature for 2 hours. Upon completion of the reaction (monitored by LC-MS), the mixture was diluted with dichloromethane (20 mL) and washed with saturated bicarbonate solution (15 mLX 2) and saturated saline (15 mLX 3). After dried over anhydrous sodium sulfate and filtration, the mixture was concentrated to obtain crude product tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (180 mg, yield: 92%). ES-API: [M+H]⁺= 1001.3.

Step 3: Tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (60 mg, 0.06 mmol) was dissolved in anhydrous N,N-dimethylformamide (3 mL). Potassium carbonate (83 mg, 0.6 mmol) and N-methyl-2,2,2-trifluoroethylamine hydrochloride (89 mg, 0.6 mmol) were added, and the reaction mixture was stirred at 50°C overnight. The reaction mixture was then diluted with ethyl acetate (20 mL) and washed with water (15 mLX2) and saturated saline (15 mLX3). After dried over anhydrous sodium sulfate and filtration, the crude product was purified by preparative thin layer chromatography (dichloromethane: methanol = 30:1) to obtain tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, yield: 24%). ES-API: [M+H]⁺= 1018.2.

Step 4: Cesium fluoride (46 mg, 0.30 mmol) was added to a solution of tert-butyl (SS,SaS,6S,9R)-2-(3-((tert-butoxycarbonyl)amino)-8-((triisopropylsilyl)ethynyl)naphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (15 mg, 0.015 mmol) in N,N-dimethylformamide (2 mL). The reaction mixture was stirred at room temperature for 1 hour. Upon completion of the reaction (monitored by LC-MS), the reaction mixture was diluted with ethyl acetate (20 mL) and washed with water (15 mLX 2) and saturated saline (15 mLX 3). After dried over anhydrous sodium sulfate and filtration, the mixture was concentrated to obtain tert-butyl (5S,5aS,6S,9R)-2-(3-((tert-butoxycarbonyl)amino)-8-ethynylnaphthalen-1-yl)-1-fluoro-5-methyl-12-((1-((methyl(2,2,2-tiifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (13 mg, yield: 100%). ES-API: [M+H]⁺=862.3.

Step 5: Under an ice-water bath, trifluoroacetic acid (2 mL) was slowly added to a solution of tert-butyl (5S,5aS,6S,9R)-2-(3-((tert-butoxycarbonyl)amino)-8-ethynylnaphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (13 mg, 0.015 mmol) in dichloromethane (1 mL). The reaction mixture was stirred at 0°C for 0.5 hours. Upon completion of the reaction (monitored by LC-MS), the reaction mixture was concentrated under reduced pressure to dryness. The residue was purified by preparative HPLC (ammonium bicarbonate method 2) to obtain 5-ethynyl-4-((5S,5aS,6S,9R)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-amine (Z714, 0.7 mg, yield: 7 % ). ES-API: [M+H]⁺= 662.2.

### Example 104 Synthesis of 5-ethynyl-1-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-amine (Z715)

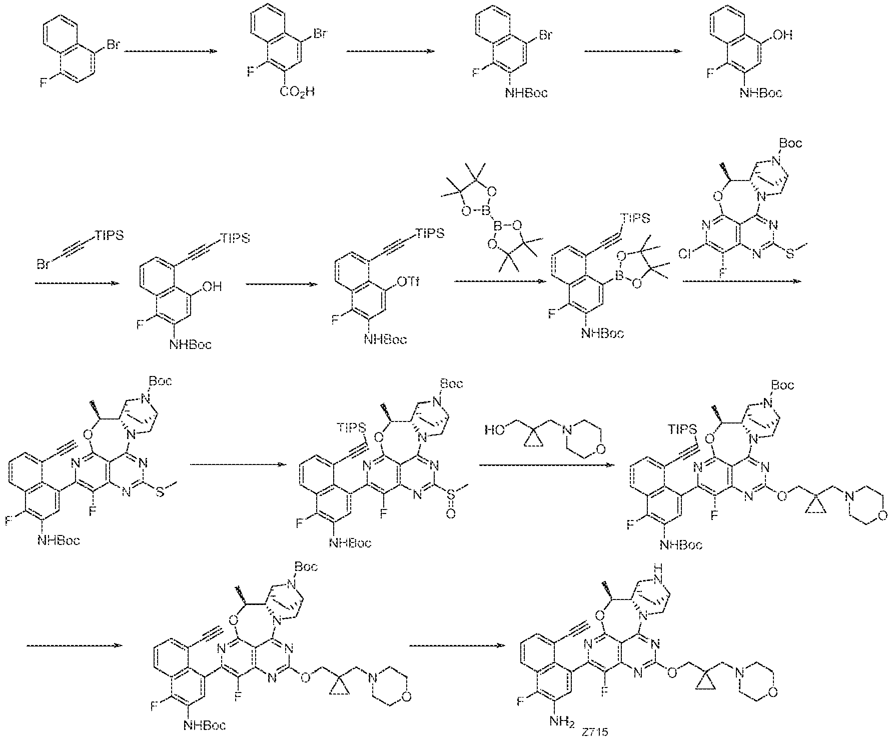

Step 1: 2,2,6,6-Tetramethylpiperidine (9.75 mL, 57.76 mmol) and tetrahydrofuran (70 mL) were added to a round-bottom flask. Under nitrogen protection, the reaction mixture was cooled to -78 degrees, and a solution of n-butyllithium in hexane (23.10 mL, 57.76 mmol) was added dropwise. After the addition was complete, the reaction mixture was stirred at -78 degrees for 30 minutes. A solution of 1-bromo-4-fluoronaphthalene (10 g, 44.433 mmol) in tetrahydrofuran (70 mL) was added dropwise to the reaction mixture, which was then stirred at -78 degrees for 1 hour. Dry ice (solid carbon dioxide, 19.55 g, 444.326 mmol) was added to the reaction mixture in batches. The reaction mixture continued to be stirred at -78 degrees for 1 hour. The reaction mixture was warmed to 0 degrees and quenched with water, adjusting the pH to 5 with 6M hydrochloric acid. A large amount of white solid precipitated out. The solid was filtered and washed with water. The filter cake was collected and dried under reduced pressure to obtain 4-bromo-1-fluoro-2-naphthoic acid (9 g, yield 75%).

Step 2: 4-Bromo-1-fluoro-2-naphthoic acid (7.5 g, 27.87 mmol), triethylamine (7.75 mL, 55.75 mmol), tert-butanol (60 mL), and toluene (10 mL) were added to a round-bottom flask. Diphenylphosphoryl azide (9 mL, 41.81 mmol) was then added to the above reaction mixture. Under nitrogen protection, the reaction mixture was stirred at 90 degrees Celsius for 4 hours. The reaction mixture was concentrated, and the residue was purified by flash silica gel chromatography (ethyl acetate/petroleum ether: 0-5%) to obtain tert-butyl (4-bromo-1-fluoronaphthalen-2-yl)carbamate (7.3 g, yield 77%). ES-API: [M+H-56]⁺=284.0.

Step 3: Tert-butyl (4-bromo-1-fluoronaphthalen-2-yl)carbamate (7.5 g, 22.05 mmol), potassium hydroxide (4.95 g, 88.19 mmol), tris(dibenzylideneacetone)dipalladium(0) (1 g, 1.10 mmol), 2-di-tert-butylphosphino-2',4',6'-triisopropylbiphenyl (1.4 g, 3.31 mmol), dioxane (60 mL) and water (50 mL) were added to a round-bottom flask. The reaction mixture was purged with nitrogen three times. Protected under a nitrogen atmosphere, the mixture was stirred in a 100-degree Celsius oil bath for 5 hours. The reaction mixture was quenched with water and extracted with ethyl acetate. The organic phase was washed with saturated saline, dried over anhydrous sodium sulfate, and filtered. The filtrate was concentrated, and the crude product was purified by flash silica gel chromatography (ethyl acetate/petroleum ether: 0-15%) to obtain tert-butyl (1-fluoro-4-hydroxynaphthalen-2-yl)carbamate (4.5 g, yield 73%). ES-API: [M+H-56]⁺=222.1.

Step 4: Tert-butyl (1-fluoro-4-hydroxynaphthalen-2-yl)carbamate (4.5 g, 16.22 mmol), (bromoethynyl)triisopropylsilane (5.51 g, 21.09 mmol), potassium acetate (3.18 g, 32.45 mmol), dichloro(4-methylisopropylphenyl)ruthenium(II) (795 mg, 1.30 mmol), and 1,4-dioxane (60 mL) were added to a round-bottom flask. Protected under a nitrogen atmosphere, the reaction mixture was stirred in a 100-degree Celsius oil bath for 5 hours. The reaction mixture was filtered. The filtrate was concentrated, and the crude product was purified by flash silica gel chromatography (ethyl acetate/petroleum ether: 0-2%) to obtain tert-butyl (1-fluoro-4-hydroxy-5-((triisopropylsilyl)ethynyl)naphthalen-2-yl)carbamate (5 g, yield 67%). ES-API: [M+H]⁺=458.2.

Step 5: Under nitrogen protection, tert-butyl (1-fluoro-4-hydroxy-5-((triisopropylsilyl)ethynyl)naphthalen-2-yl)carbamate (5 g, 10.92 mmol), N,N-diisopropylethylamine (4.76 mL, 27.31 mmol), and dichloromethane (60 mL) were added to a round-bottom flask. The reaction mixture was cooled to -40 degrees Celsius, and trifluoromethanesulfonic anhydride (2.76 mL, 16.39 mmol) was slowly added dropwise. The reaction mixture was stirred at -40 degrees Celsius for 1 hour. The reaction was quenched with saturated sodium bicarbonate, extracted with dichloromethane. The organic phase was washed with saturated saline, dried over anhydrous sodium sulfate, and filtered. The filtrate was concentrated, and the crude product was purified by flash silica gel chromatography (ethyl acetate/petroleum ether: 0-5%) to obtain 3-((tert-butoxycarbonyl)amino)-4-fluoro-8-((triisopropylsilyl)ethynyl)naphthalen-1-yl trifluoromethanesulfonate (5.5 g, yield 85%). ES-API: [M+Na]⁺=612.0.

Step 6: To a sealed tube, 3-((tert-butoxycarbonyl)amino)-4-fluoro-8-((triisopropylsilyl)ethynyl)naphthalen-1-yl trifluoromethanesulfonate (1 g, 1.70 mmol), pinacol boronic ester (861 mg, 3.39 mmol), 1,1'-bis(diphenylphosphino)ferrocene dichloropalladium(ll) (99 mg, 0.13 mmol), potassium acetate (499 mg, 5.09 mmol), and 1,4-dioxane (12 mL) were added. The reaction mixture was purged with nitrogen for 2 minutes, placed in a microwave reactor and stirred at 115 degrees Celsius for 4 hours. The reaction was quenched with water and extracted with ethyl acetate. The organic phase was washed with saturated saline, dried over anhydrous sodium sulfate, and filtered. The filtrate was concentrated, and the crude product was purified by flash silica gel chromatography (ethyl acetate/petroleum ether: 0-3%) to obtain tert-butyl (1-fluoro-4-(4,4,5,5-tetramethyl-1,3,2-dioxaborolan-2-yl)-5-((triisopropylsilyl)ethynyl)naphthalen-2-yl)carbamate (260 mg, yield 27%). ES-API: [M+H]⁺=568.3.

Step 7: Tert-butyl (1-fluoro-4-(4,4,5,5-tetramethyl-1,3,2-dioxaborolan-2-yl)-5-((triisopropylsilyl)ethynyl)naphthalen-2-yl)carbamate (212 mg, 0.37 mmol), tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (120 mg, 0.25 mmol), methanesulfonic acid [butylbis(1-adamantyl)phosphine](2-amino-1,1'-biphenyl-2-yl)palladium(II) (18 mg, 0.025 mmol), potassium phosphate (158 mg, 0.75 mmol), 1,4-dioxane (4 mL), and water (0.8 mL) were added to a round-bottom flask. Under nitrogen protection, the system was heated in a microwave reactor at 75°C for 2 hours. Water (30 mL) was added to the reaction mixture, which was then extracted with ethyl acetate (30 mL × 3). The organic phase was dired over anhydrous sodium sulfate, filtered, and concentrated, the crude product was purified by flash silica gel chromatography (ethyl acetate/petroleum ether: 0-30%) to obtain tert-butyl (5S,5aS,6S,9R)-2-(3-((tert-butoxycarbonyl)amino)-8-ethynyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (210 mg, yield 95%). ES-API: [M+H]⁺=887.2.

Step 8: Tert-butyl (5S,5aS,6S,9R)-2-(3-((tert-butoxycarbonyl)amino)-8-ethynyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (210 mg, 0.23 mmol) and dichloromethane (6 mL) were added to a round-bottom flask. The mixture was cooled to 0 degrees Celsius, and m-chloroperoxybenzoic acid (53 mg, 0.31 mmol) was added. The reaction mixture was stirred at room temperature for 0.5 hours. Saturated sodium thiosulfate solution (30 mL) and saturated sodium bicarbonate solution were added to the reaction mixture, which was then extracted with ethyl acetate (30 mL × 3). The organic phase was dried and concentrated to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (180 mg, yield 84%). ES-API: [M+H]⁺=903.2.

Step 9: Sodium hydride (32 mg, 0.80 mmol) and tetrahydrofuran (8 mL) were added to a round-bottom flask. The mixture was cooled to 0 degrees Celsius, and (1-(morpholinomethyl)cyclopropyl)methanol (68 mg, 0.40 mmol) dissolved in anhydrous tetrahydrofuran (1 mL) was added. The reaction was proceeded at room temperature for 5 minutes. Tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (180 mg, 0.20 mmol) was added, and the reaction was proceeded at 0°C for 30 minutes. Upon completion of the reaction (monitored by LCMS), saturated ammonium chloride solution (30 mL) was added. The reaction mixture was extracted with ethyl acetate (20 mL × 2). The organic phase was washed with saturated brine (30 mL), dried over anhydrous sodium sulfate, filtered, and concentrated. The crude product was purified by flash silica gel chromatography (dichloromethane/methanol: 0-10%) to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ablheptalene-14-carboxylate (60 mg, yield 30%). ES-API: [M+H]⁺=1010.2.

Step 10: Tert-butyl (SS,SaS,bS,9R)-2-(3-((tert-butoxycarbonyl)amino)-4-fluoro-8-((triisopropylsilyl)ethynyl)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (60 mg, 0.06 mmol), cesium fluoride (89 mg, 0.59 mmol), and N,N-dimethylformamide (3 mL) were added to a round-bottom flask. The reaction mixture was stirred at room temperature for 1 hour. Water (30 mL) was added, and the reaction mixture was extracted with ethyl acetate (30 mL × 3). The organic phase was washed with saturated brine (30 mL × 2), dried over anhydrous sodium sulfate, filtered, and concentrated to obtain tert-butyl (5S,5aS,6S,9R)-2-(3-((tert-butoxycarbonyl)amino)-8-ethynyl-4-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (50 mg, crude product). ES-API: [M+H]⁺=854.2.

Step 11: Tert-butyl (5S,5aS,6S,9R)-2-(3-((tert-butoxycarbonyl)amino)-8-ethynyl-4-fluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (50 mg, crude product) was dissolved in ethyl acetate (1.5 mL). The solution was cooled to zero degrees Celsius, and 4M hydrochloric acid in dioxane solution (1.5 mL, 6 mmol) was added. The reaction mixture was stirred at room temperature for 1 hour. The reaction mixture was concentrated at 40°C to dryness, and the crude product was purified by preparative HPLC (ammonium bicarbonate method 2) to obtain 5-ethynyl-1-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-amine (Z715, 12mg, yield: 31%). ES-API: [M+H]⁺=654.2.

### Example 105 Synthesis of 2-cyclopropyl-5-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)-3,6-dimethylaniline (Z716)

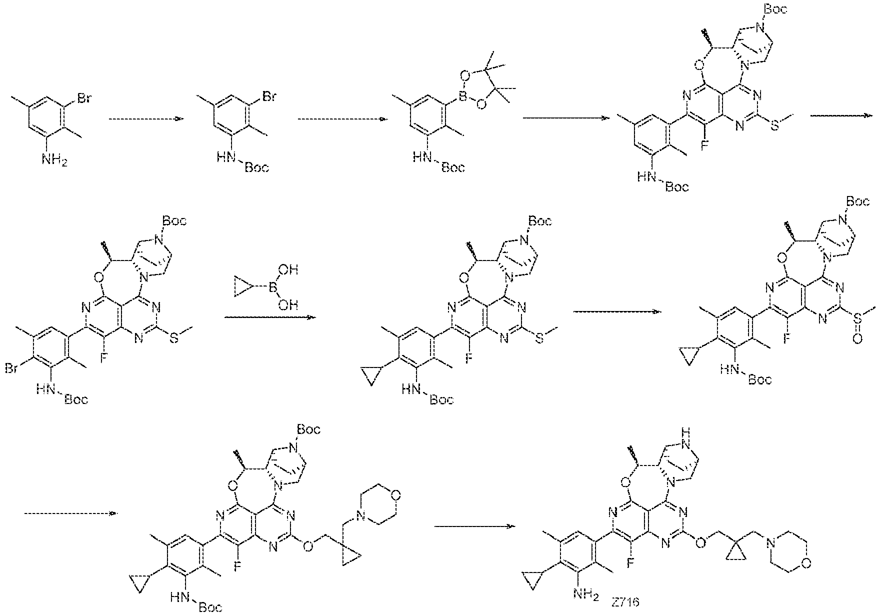

Step 1: At room temperature, 3-bromo-2,5-dimethylaniline (1.0 g, 4.998 mmol) and di-tert-butyl dicarbonate (1.42 g, 6.497 mmol) were successively added to a sodium hydroxide solution (2M, 20 mL) and the mixture reacted for 1 hour at 100°C. Upon completion of the reaction, the reaction mixture was extracted with ethyl acetate (200 mL × 2) and saturated brine (80 mL). The combined ethyl acetate phases were dried over anhydrous sodium sulfate, filtered, and the filtrate was concentrated under reduced pressure. The crude product was purified by silica gel column chromatography (0-50% ethyl acetate/petroleum ether) to obtain tert-butyl (3-bromo-2,5-dimethylphenyl)carbamate (1.40 g, yield 93.31%). ES-API: [M-55+H]⁺=245.9.

Step 2: Tert-butyl (3-bromo-2,5-dimethylphenyl)carbamate (0.9 g, 2.998 mmol), pinacol boronic ester (1.52 g, 5.996 mmol), potassium acetate (1.173 g, 11.99 mmol), and [1,1'-bis(diphenylphosphino)ferrocene]dichloropalladium(II) (0.323 g, 0.45 mmol) were added to N,N-dimethylformamide (30 mL). After purged with nitrogen 4 times, the mixture reacted at 80°C for 2 hours. Upon completion of the reaction, the reaction mixture was extracted with ethyl acetate (200 mL × 2) and saturated brine (80 mL). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain tert-butyl (2,5-dimethyl-3-(4,4,5,5-tetramethyl-1,3,2-dioxaborolan-2-yl)phenyl)carbamate (490 mg, yield 47%). ES-API: [M-55+H]⁺=292.2.

Step 3: Potassium phosphate (0.616 g, 2.90 mmol), methylsulfonic acid [butyl(di(1-adamantyl)phosphino](2-amino-1,1'-biphenyl)palladium(II) (79.3 mg, 0.109 mmol) were added to a tetrahydrofuran (30 mL) and water (3 mL) solution of tert-butyl (5S,5aS,6S,9R)-2-chloro-1-fluoro-5-methyl-12-(methylthio)-5a,6,7,8,9,10-hexahydro-5H-4-oxygen-3,10a,11,13,14-pentaaza-6,9-methanonaphtho[1,8-ab]heptalene-14-carboxylate (350 mg, 0.726 mmol) and tert-butyl (2,5-dimethyl-3-(4,4,5,5-tetramethyl-1,3,2-dioxaborolan-2-yl)phenyl)carbamate (0.490 g, 1.411 mmol). The system was bubbled with nitrogen and heated in an oil bath at 80°C for 3 hours. Upon completion of the reaction, the reaction mixture was extracted with ethyl acetate (30 mL × 2) and saturated brine (30 mL). The combined ethyl acetate phases were dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-2-(3-((tert-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (570 mg, crude product), a yellow solid. ES-API: [M+H]⁺=667.2.

Step 4: At 0°C, tert-butyl (5S,5aS,6S,9R)-2-(3-((tert-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (483.0 mg, 0.726 mmol) was added to dichloromethane (20 mL), followed by N-bromosuccinimide (168.0 mg, 0.944 mmol). The reaction was proceeded at this temperature for 0.5 hours. Upon completion of the reaction, the mixture was extracted with ethyl acetate (100 mLX 2) and saturated brine (100 mL). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain tert-butyl (5S,5aS,6S,9R)-2-(4-bromo-3-((tert-butoxycarbonyl)amino)-2,5-dimethylphenyl)-1-fluoro-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (400 mg, yield 74.0%). ES-API: [M+H]⁺=745.1.

Step 5: To a mixture of tert-butyl (SS,SaS,6S,9R)-2-(4-bromo-3-((tert-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, 0.536 mmol) and cyclopropylboronic acid (230.0 mg, 2.68 mmol) in toluene (50 mL) and water (5 mL), potassium phosphate (455.4 mg, 2.146 mmol), 2-dicyclohexylphosphino-2',6'-dimethoxybiphenyl (66.06 mg, 0.161 mmol), and palladium acetate (24.04 mg, 0.107 mmol) were added. The mixture was bubbled with nitrogen and heated in an oil bath at 105°C for 2 hours. Upon completion of the reaction, the mixture was extracted with ethyl acetate (200 mL × 2) and saturated brine (100 mL × 4). The combined ethyl acetate phases were dried over anhydrous sodium sulfate, filtered, and the filtrate was concentrated under reduced pressure. The crude product was purified by silica gel column chromatography (0-70% ethyl acetate/petroleum ether) to obtain tert-butyl (SS,SaS,6S,9R)-2-(3-((tert-butoxycarbonyl)amino)-4-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (520 mg, crude product), a yellow solid. ES-API: [M+H]⁺=707.2.

Step 6: At 0°C, m-chloroperoxybenzoic acid (188.3 mg, 1.09 mmol) was added to a dichloromethane (30.0 mL) solution of tert-butyl (5S,5aS,6S,9R)-2-(3-((tert-butoxycarbonyl)amino)-4-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (379.0 mg, 0.537 mmol). The reaction mixture was stirred in an ice-water bath for 1 hour, quenched with saturated sodium bisulfite, and extracted with dichloromethane (100 mL × 2). The organic layer was dried over anhydrous sodium sulfate, filtered, concentrated to obtain a crude product, which was then purified by silica gel column chromatography (0-10% methanol/dichloromethane) to obtain tert-butyl (5S,5aS,6S,9R)-2-(3-((tert-butoxycarbonyl)amino)-4-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (390 mg, crude product), a white solid. ES-API:[M+H]⁺=723.3.

Step 7: At 0°C, sodium hydride (70.0 mg, 1.754 mmol) was added to a tetrahydrofuran (10.0 mL) solution of (1-(morpholinomethyl)cyclopropyl)methanol (100 mg, 0.5850 mmol), the mixture was stirred for 10 minutes at this temperature, then tert-butyl (5S,5aS,6S,9R)-2-(3-((tert-butoxycarbonyl)amino)-4-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (390 mg, crude product) and tetrahydrofuran (5 mL) were added. The reaction mixture was stirred at 0°C for 10 minutes and then at room temperature for 0.5 hours. Upon completion of the reaction, the reaction mixture was quenched with saturated ammonium chloride solution (20 mL) and extracted with ethyl acetate. The organic phase was washed with saturated brine, dried over anhydrous sodium sulfate, filtered, concentrated. The crude product was purified by silica gel column chromatography (0-5% methanol/dichloromethane) to obtain tert-butyl (5S,5aS,6S,9R)-2-(3-((tert-butoxycarbonyl)amino)-4-cyclopropyl-2,5-dimethylphenyl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (150 mg, crude product), a yellow solid. ES-API: [M+H]⁺=830.3.

Step 8: Under an ice-water bath, tert-butyl (SS,SaS,6S,9R)-2-(3-((tert-butoxycarbonyl)amino)-4-cyclopropyl-2,5-dimethylphenyl)-1-fluoro-5-methyl-12-((1-(morpholinomethyl)cyclopropyl)methoxy)-5a,6,7,8,9, 10-hexahydro-5H-4-oxa-3, 10a, 11, 13,14-pentaaza-6,9-methanonaphtho[1,8-ab]heptalene-14-carboxylate (150 mg, crude product) was slowly added to dichloromethane (10.0 mL), then a dioxane solution of hydrochloric acid (5.0 mL, 4M, 20.0 mmol) was added and the reaction mixture was stirred at room temperature for 1.0 hour. Upon completion of the reaction (monitored by LCMS), the reaction mixture was concentrated under reduced pressure to dryness, and the residue was purified by preparative HPLC (ammonium bicarbonate method 2) to obtain a white solid, 2-cyclopropyl-5-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)-3,6-dimethylaniline (Z716, 3.0mg, yield: 0.8%). ES-API: [M+H]⁺= 630.4.

### Example 106 Synthesis of 5,b-difluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-2-yl)naphthalen-2-amine (Z71.9)

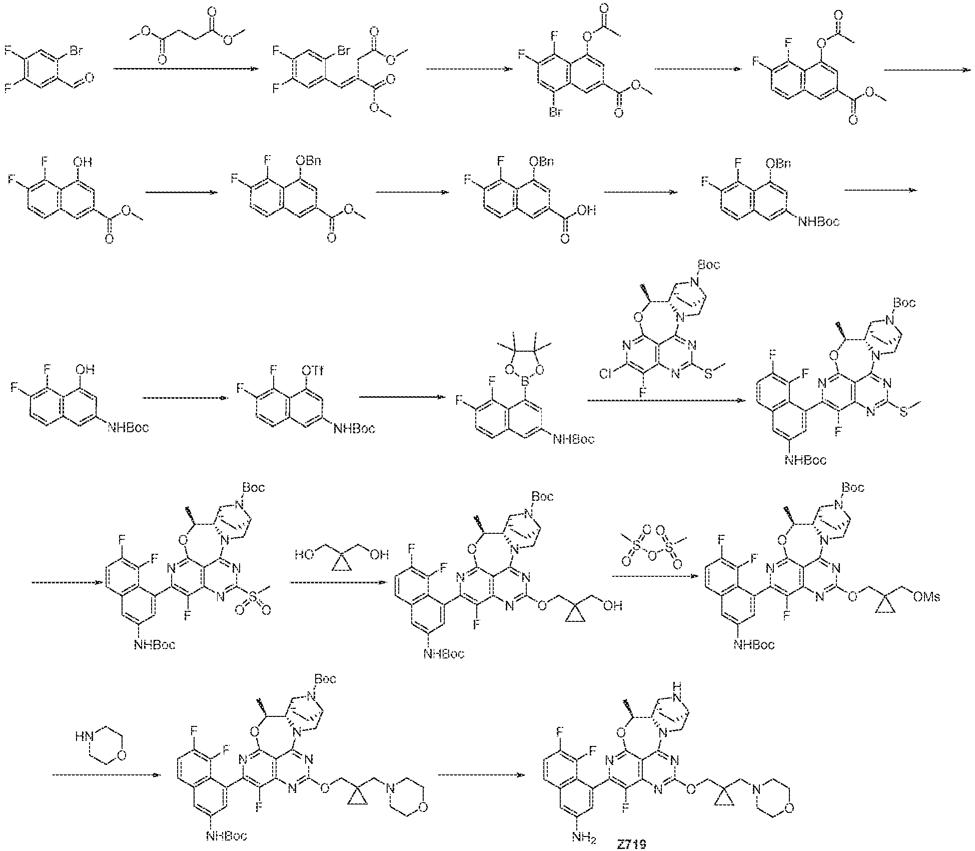

Step 1: Under nitrogen protection, dimethyl succinate (7.04 g, 48.19 mmol) and a 30% methanol solution of sodium methoxide (4.5 mL, 22.62 mmol) were dissolved in toluene (5 mL). The reaction mixture was heated to 75 °C. A toluene solution (10 mL) of 2-bromo-4,5-difluorobenzaldehyde (5.0 g, 22.62 mmol) was added dropwise to the reaction mixture. After the addition was complete, the reaction mixture was heated to 85 °C and stirred for 5 hours. Upon completion of the reaction, monitored by LCMS, the mixture was cooled to room temperature. Methyl tert-butyl ether (100 mL) was added to the reaction mixture and stirred for 5 minutes. The reaction mixture was then poured into water (100 mL), the aqueous phase was separated, and the organic phase was washed with water (30 mL × 2). The combined aqueous phases were acidified with 2 M dilute hydrochloric acid to an acidic pH, then extracted with methyl tert-butyl ether (30 mL × 3). The combined organic phases were washed with water (30 mL) and saturated brine (30 mL), dried over anhydrous sodium sulfate, filtered, and concentrated under reduced pressure to obtain a crude product of dimethyl (E)-2-(2-bromo-4,5-difluorobenzylidene)succinate (6.591 g, yield: 83%). ES-API: [M- 2H]⁺= 346.0.

Step 2: dimethyl (E)-2-(2-bromo-4,5-difluorobenzylidene)succinate (6.591 g, 18.878 mmol) was dissolved in acetic anhydride (15 mL). Anhydrous sodium acetate (5.661 g, 69.011 mmol) was added to the reaction mixture, which was then heated to 140 °C and stirred overnight. Upon completion of the reaction, monitored by LCMS, the reaction mixture was cooled to room temperature and toluene (30 mL) was added. The reaction mixture was concentrated under reduced pressure to remove the solvent. Dilute hydrochloric acid (30 mL) was added to the residue, which was then extracted with ethyl acetate (30 mL × 3), and the combined organic phases were washed with water (30 mL) and saturated brine (30 mL), dried over anhydrous sodium sulfate, filtered, and concentrated. The residue was purified by silica gel column chromatography (ethyl acetate: petroleum ether = 0 - 4%) to obtain methyl 4-acetoxy-8-bromo-5,6-difluoro-2-naphthoate (1.2 g, yield: 18%), a white solid. ES-API: [M+H]⁺= 359.0.

Step 3: Methyl 4-acetoxy-8-bromo-5,6-difluoro-2-naphthoate (1.2 g, 3.342 mmol) and triethylamine (406 mg, 4.010 mmol) were dissolved in ethyl acetate (36 mL). 10% palladium on carbon (356 mg) was added to the reaction mixture, which was then stirred at room temperature under a hydrogen atmosphere for 1 hour. Upon completion of the reaction, monitored by LCMS, the mixture was filtered through diatomaceous earth. The filtrate was concentrated under reduced pressure to obtain a crude product of methyl 4-acetoxy-5,6-difluoro-2-naphthoate (936 mg, yield: 99%). ES-API: [M+ H]⁺= 281.1.

Step 4: Methyl 4-acetoxy-5,6-difluoro-2-naphthoate (936 mg, 3.340 mmol) was dissolved in methanol (20 mL), and sodium methoxide (271 mg, 5.010 mmol) was added to the reaction mixture. The reaction mixture was stirred at room temperature for 1 hour. Upon completion of the reaction, monitored by LCMS, the reaction mixture was acidified to about pH 2.0 with 1M dilute hydrochloric acid, resulting in the precipitation of a large amount of solid. After filtration, the filter cake was dissolved in ethyl acetate (20 mL), dried over anhydrous sodium sulfate, filtered, and concentrated under reduced pressure to obtain a crude product of methyl 5,6-difluoro-4-hydroxy-2-naphthoate (748 mg, yield: 94%). ES-API: [M+ H]⁺= 239.0.

Step 5: Methyl 5,6-difluoro-4-hydroxy-2-naphthoate (748 mg, 3.140 mmol), potassium carbonate (868 mg, 6.281 mmol), and benzyl bromide (806 mg, 4.711 mmol) were dissolved in an acetone solution (20 mL). The reaction mixture was heated to reflux for 2 hours. Upon completion of the reaction, monitored by LCMS, the mixture was cooled to room temperature, filtered and dried under reduced pressure to obtain methyl 4-(benzyloxy)-5,6-difluoro-2-naphthoate (1.031 g) as an off-white solid. ES-API: [M+ H]⁺= 329.1.

Step 6: Methyl 4-(benzyloxy)-5,6-difluoro-2-naphthoate (1031 mg, 3.140 mmol) was dissolved in toluene (12 mL). An aqueous sodium hydroxide solution (26 mL, 12 M) was added, and the mixture was heated to 105 °C and stirred for 16 hours. Upon completion of the reaction, monitored by LCMS, most of the solvent was removed under reduced pressure. The residue was dissolved in ethyl acetate (100 mL), acidified to about pH 2.0 with 6M dilute hydrochloric acid. The organic phase was separated, dried over anhydrous sodium sulfate, filtered, and concentrated under reduced pressure to obtain a crude product of 4-(benzyloxy)-5,6-difluoro-2-naphthoic acid (1081 mg) as an off-white solid. ES-API: [M+ H]⁺= 315.3.

Step 7: 4-(benzyloxy)-5,6-difluoro-2-naphthoic acid (981 mg, 3.121 mmol), diphenylphosphoryl azide (1.0 mL, 4.682 mmol), and triethylamine (2.0 mL, 14.046 mmol) were dissolved in a mixture of toluene (15 mL) and tert-butanol (15 mL). The reaction mixture was heated to 90 °C and stirred overnight, cooled to room temperature and concentrated under reduced pressure. The crude product was purified by silica gel column chromatography (petroleum ether/ethyl acetate = 0 - 3%) to obtain tert-butyl (4-(benzyloxy)-5,6-difluoronaphthalen-2-yl)carbamate (350 mg, yield: 26%) as an off-white solid. ES-API: [M- 55]⁺= 330.1.

Step 8: Tert-butyl (4-(benzyloxy)-5,6-difluoronaphthalen-2-yl)carbamate (350 mg, 0.908 mmol) was dissolved in a mixture of methanol (10 mL) and tetrahydrofuran (1 mL). 10% palladium on carbon (97 mg) was added, and the mixture was stirred at room temperature under a hydrogen atmosphere for 3 hours. Upon completion of the reaction, monitored by LCMS, the mixture was filtered through diatomaceous earth. The filtrate was concentrated under reduced pressure to obtain a crude product of tert-butyl (5,6-difluoro-4-hydroxynaphthalen-2-yl)carbamate (268 mg). ES-API: [M-55]⁺= 240.1.

Step 9: At -40 °C, tert-butyl (5,6-difluoro-4-hydroxynaphthalen-2-yl)carbamate (250 mg, 0.847 mmol) was dissolved in dichloromethane (15 mL). To the reaction mixture, N,N-diisopropylethylamine (0.7 mL, 5.080 mmol) and trifluoromethanesulfonic anhydride (358 mg, 1.270 mmol) were added successively. The mixture was stirred at -40 °C for 0.5 hours. Upon completion of the reaction, monitored by LCMS, saturated sodium bicarbonate solution (30 mL) was added to quench the reaction. The mixture was extracted with dichloromethane (20 mL × 3), and the organic phases were combined, concentrated under reduced pressure, and the crude product was purified by silica gel column chromatography (petroleum ether/ethyl acetate = 0 - 6%) to obtain 3-((tert-butoxycarbonyl)amino)-7,8-difluoronaphthalen-1-yl trifluoromethanesulfonate (269 mg, yield: 74%). ES-API: [M-55]⁺= 372.0.

Step 10: Under nitrogen protection, 3-((tert-butoxycarbonyl)amino)-7,8-difluoronaphthalen-1-yl trifluoromethanesulfonate (269 mg, 0.629 mmol), pinacol boronate ester (400 mg, 1.574 mmol), potassium acetate (185 mg, 1.888 mmol), and [1,1'-bis(diphenylphosphino)ferrocene]dichloropalladium(II) (92 mg, 0.126 mmol) were dissolved in 1,4-dioxane (15 mL). The reaction mixture was heated to 105 °C and stirred overnight. Upon completion of the reaction, monitored by LCMS, the mixture was cooled to room temperature, concentrated under reduced pressure, and purified by silica gel column chromatography (petroleum ether/ethyl acetate = 0 - 2%) to obtain tert-butyl (5,6-difluoro-4-(4,4,5,5-tetramethyl-1,3,2-dioxaborolan-2-yl)naphthalen-2-yl)carbamate (307 mg), an off-white solid. ES-API: [M-55]⁺= 350.1.

Step 11: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (90 mg, 0.187 mmol), tert-butyl (5,6-difluoro-4-(4,4,5,5-tetramethyl-1,3,2-dioxaborolan-2-yl)naphthalen-2-yl)carbamate (83 mg, 0.205 mmol), potassium phosphate (119 mg, 0.560 mmol), and palladium(II) bis(dibenzylideneacetone) diphenylphosphine (20 mg, 0.028 mmol) were dissolved in tetrahydrofuran (4 mL) and water (0.8 mL). The reaction mixture was stirred at 70 °C under microwave for 40 minutes. Upon completion of the reaction, monitored by LCMS, the mixture was dried over anhydrous sodium sulfate, filtered, and concentrated under reduced pressure, and purified by silica gel column chromatography (petroleum ether/ethyl acetate = 0 ~ 30%) to obtain tert-butyl (5S,5aS,6S,9R)-2-(3-((tert-butoxycarbonyl)amino)-7,8-difluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (150 mg). ES-API: [M+H]⁺= 725.2.

Step 12: Under an ice-water bath, tert-butyl (5S,5aS,6S,9R)-2-(3-((tert-butoxycarbonyl)amino)-7,8-difluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (150 mg, 0.207 mmol) was dissolved in anhydrous dichloromethane (10 mL). m-Chloroperoxybenzoic acid (55 mg, 0.269 mmol) was added to the solution, and the mixture was stirred at 0 °C for 1 hour. Upon completion of the reaction, monitored by LCMS, dichloromethane (15 mL) was added to dilute the reaction mixture, which was then quenched with saturated sodium bisulfite solution (15 mL). The mixture was washed with sodium bicarbonate solution (15 mL) and saturated brine (15 mL). The organic phase was dried over anhydrous sodium sulfate, filtered, and concentrated to obtain a crude product of tert-butyl (SS,SaS,6S,9R)-2-(3-((tert-butoxycarbonyl)amino)-7,8-difluoronaphthalen-1-yl)-1-fluoro-5-methyl-12-(methylsulfonyl)-Sa,6,7,8,9,10-hexahydro-SH-4-oxa-3,10a,11,13,14-pentaaza-6,9-methanonaphtho[1,8-ab]heptalene-14-carboxylate (153 mg). ES-API: [M+ H]⁺= 741.1.

Step 13: Cyclopropyl-1,1-diethyl diol (105 mg, 1.026 mmol) was dissolved in anhydrous tetrahydrofuran (6 mL). Under an ice-water bath, sodium hydride (21 mg, 0.515 mmol) was added and reacted for 10 minutes. A tetrahydrofuran solution (4 mL) of tert-butyl (5S,5aS,6S,9R)-2-(3-((tert-butoxycarbonyl)amino)-7,8-difluoronaphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (76 mg, 0.103 mmol) was added dropwise to the reaction mixture at 0 °C and reacted for 30 minutes. Upon completion of the reaction, monitored by LCMS, the reaction mixture was diluted with ethyl acetate (10 mL) and washed with saturated ammonium chloride solution (10 mL × 2) and saturated brine (10 mL × 3), dried over anhydrous sodium sulfate, filtered, and concentrating under reduced pressure. The crude product was purified on a silica gel thin-layer chromatography (dichloromethane/methanol = 30:1) to obtain tert-butyl (5S,5aS,6S,9R)-2-(3-((tert-butoxycarbonyl)amino)-7,8-difluoronaphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (46 mg, yield: 58%). ES-API: [M+ H]⁺= 779.2.

Step 14: Under an ice-water bath, tert-butyl (SS,SaS,6S,9R)-2-(3-((tert-butoxycarbonyl)amino)-7,8-difluoronaphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (46 mg, 0.059 mmol) was dissolved in anhydrous dichloromethane (5 mL). N,N-Diisopropylethylamine (46 mg, 0.354 mmol) and methanesulfonic anhydride (174 mg, 0.354 mmol) were added successively to the solution, and the mixture was stirred at 0 °C for 1 hour. Upon completion of the reaction, monitored by LCMS, dichloromethane (15 mL) was added to dilute the reaction mixture, which was then quenched with saturated sodium bicarbonate solution (30 mL). The mixture was extracted with dichloromethane (15 mL × 3), and the organic phases were dried over anhydrous sodium sulfate, filtered, and concentrated to obtain a crude product of tert-butyl (5S,5aS,6S,9R)-2-(3-((tert-butoxycarbonyl)amino)-7,8-difluoronaphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (55 mg). ES-API: [M+ H]⁺= 857.0.

Step 15: Tert-butyl (5S,5aS,6S,9R)-2-(3-((tert-butoxycarbonyl)amino)-7,8-difluoronaphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (45 mg, 0.053 mmol), morpholine (23 mg, 0.263 mmol), potassium carbonate (36 mg, 0.263 mmol), and potassium iodide (8.7 mg, 0.053 mmol) were dissolved in acetone (3 mL). The reaction mixture was heated to 80 °C in a sealed tube and stirred for 1 hour. Upon completion of the reaction, monitored by LCMS, water (10 mL) and ethyl acetate (10 mL) were added. The organic phase was separated and washed with saturated brine (10 mL × 2), dried over anhydrous sodium sulfate, filtered, and concentrated to obtain a crude product of tert-butyl (5S,5aS,6S,9R)-2-(3-((tert-butoxycarbonyl)amino)-7,8-difluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (44 mg). ES-API: [M+H]⁺= 848.2.

Step 16: Under an ice-water bath, 4 M hydrochloric acid-dioxane solution (5 mL) was slowly added to a methanol (2 mL) solution of tert-butyl (SS,SaS,6S,9R)-2-(3-((tert-butoxycarbonyl)amino)-7,8-difluoronaphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (44 mg, 0.052 mmol), and the mixture was warm to room temperature and stirred for 1 hour. Upon completion of the reaction, monitored by LCMS, the reaction mixture was concentrated under reduced pressure until dry. The residue was purified by preparative HPLC (bicarbonate method 2) to obtain 5,6-difluoro-4-((SS,SaS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-2-yl)naphthalen-2-amine (Z719, 4.13 mg, yield:

10 %), off-white solid. ES-API: [M+H]⁺= 648.2.

### Example 107 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5,6-difluoronaphthalen-2-amine (Z720)

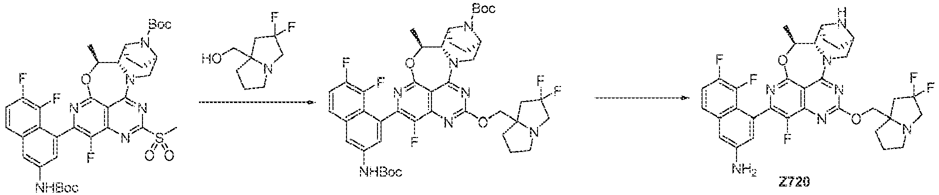

Step 1: (2,2-difluorotetrahydro-1H-pyrrolizin-7a(5H)-yl)methanol (36 mg, 0.205 mmol) was dissolved in anhydrous tetrahydrofuran (5 mL). Under an ice-water bath, sodium hydride (12 mg, 0.308 mmol) was added, and the reaction mixture was stirred for 10 minutes under the ice-water bath. Then, a tetrahydrofuran (5 mL) solution of tert-butyl (5S,5aS,6S,9R)-2-(3-((tert-butoxycarbonyl)amino)-7,8-difluoronaphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (70 mg, 0.103 mmol) was added dropwise to the reaction mixture. The reaction mixture was stirred at 0 °C for 30 minutes. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure to obtain a crude product of tert-butyl (5S,5aS,6S,9R)-2-(3-((tert-butoxycarbonyl)amino)-7,8-difluoronaphthalen-1-yl)-12-((2,2-difluorotetrahydro-1H-pyrrolizin-7a(5H-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (87 mg). ES-API: [M+ H]⁺= 854.2.

Step 2: Under an ice-water bath, 4 M hydrochloric acid-dioxane solution (5 mL) was slowly added to a methanol (1 mL) solution of tert-butyl (SS,SaS,6S,9R)-2-(3-((tert-butoxycarbonyl)amino)-7,8-difluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (87 mg, 0.102 mmol). The reaction mixture was warm to room temperature and stirred for 1 hour. Upon completion of the reaction, monitored by LCMS, the reaction mixture was concentrated under reduced pressure to dryness. The residue was purified by preparative HPLC (bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5,6-difluoronaphthalen-2-amine (Z720, 16.83 mg, yield: 25 %), off-white solid. ES-API: [M+H]⁺= 654.2.

### Example 108 Synthesis of 1-((1-((((5S,5aS,6S,9R)-2-(8-ethynyl-7-fluoronaphthalen-1-yl)-1-fluoro-5-methyl-5a,6,7,8,9,10-heacahydro-SH-4-oxa-3,10a,11,13,14-pentaaza-6,9-methanonaphtho[1,8-ab]pyrrolidi-12-yl)oxy)methyl)cyclopropyl)methyl)pyrrolidine-3-ol (Z721)

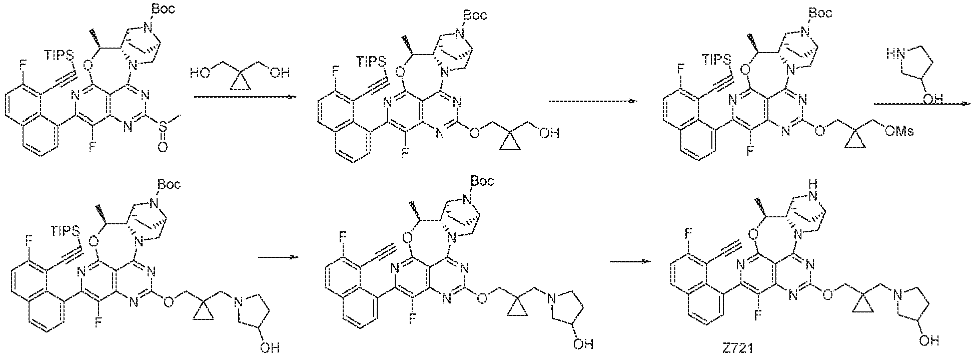

Step 1: 1,1-Cyclopropanedimethanol (58 mg, 0.57 mmol) was dissolved in anhydrous tetrahydrofuran (2 mL). Under an ice-water bath, 60% sodium hydride (38 mg, 0.95 mmol) was added, and the reaction mixture was stirred for 30 minutes under the ice-water bath. A tetrahydrofuran (2 mL) solution of tert-butyl (SS,Sa5,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, 0.19 mmol) was added dropwise to the reaction mixture, which was then stirred at room temperature for 30 minutes. Upon completion of the reaction, monitored by LCMS, the reaction mixture was diluted with ethyl acetate (20 mL) and washed with saturated ammonium chloride solution (15 mL × 2) and saturated brine (15 mL × 3), dried over anhydrous sodium sulfate, filtered and concentrated. The crude product was purified by silica gel column chromatography (ethyl acetate:petroleum ether=0-80%) to obtain tert-butyl (SS,SaS,6S,9R)-1-fluoro-2-(7-fluoro-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (90 mg, yield: 57.3%), a white solid. ES-API: [M+H|⁺= 826.3.

Step 2: Tert-butyl (5S,5aS,6S,9R)-1-fluoro-2-(7-fluoro-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (59 mg, 0.071 mmol) was dissolved in anhydrous dichloromethane (5 mL). Under an ice-water bath, triethylamine (36 mg, 0.36 mmol) and methanesulfonic anhydride (37 mg, 0.21 mmol) were added, and the reaction mixture was stirred at room temperature for 2 hours. Upon completion of the reaction, monitored by LCMS, the reaction mixture was diluted with dichloromethane (20 mL) and washed with saturated sodium bicarbonate (15 mL × 2) and saturated brine (15 mL × 3), dried over anhydrous sodium sulfate, filtered and concentrated. The crude product was purified by silica gel column chromatography (ethyl acetate:petroleum ether=0-60%) to obtain tert-butyl (SS,SaS,6S,9R)-1-fluoro-2-(7-fluoro-8-((triisopropylsilyl)ethynyl)naphthalen-1-yl)-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (57 mg, yield: 88.3%), a colorless liquid. ES-API: [M+H]⁺= 904.1.

Step 3: Tert-butyl (5S,5aS,6S,9R)-1-fluoro-2-(7-fluoro-8-((triisopropylsilyl)ethynyl)naphthalen-1-yl)-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate (57 mg, 0.063 mmol) was dissolved in acetone (2 mL). Potassium carbonate (26 mg, 0.19 mmol), sodium iodide (10 mg, 0.063 mmol), and pyrrolidin-3-ol (17 mg, 0.19 mmol) were added. The reaction mixture was stirred at 80°C in a microwave reactor for 40 minutes. The reaction mixture was concentrated to obtain a crude product, which was then purified by silica gel column chromatography (ethyl acetate:petroleum ether=0-100%) to obtain tert-butyl (5S,5aS,6S,9R)-1-fluoro-2-(7-fluoro-8-((triisopropylsilyl)ethynyl)naphthalen-1-yl)-12-((1-((3-hydroxypyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (55 mg, yield: 97.5%), a yellow solid. ES-API: [M+H]⁺= 895.4.

Step 4: Cesium fluoride (93 mg, 0.61 mmol) was added to N,N-dimethylformamide (1 mL) containing tert-butyl (5S,5aS,6S,9R)-1-fluoro-2-(7-fluoro-8-((triisopropylsilyl)ethynyl)naphthalen-1-yl)-12-((1-((3-hydroxypyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (55 mg, 0.061 mmol). The reaction mixture was stirred at room temperature for 1 hour. Upon completion of the reaction, monitored by LCMS, the reaction mixture was diluted with ethyl acetate (20 mL) and washed with water (15 mL × 2) and saturated brine (15 mL × 3), dried over anhydrous sodium sulfate, filtered, and concentrated to obtain tert-butyl (SS,SaS,6S,9R)-2-(8-ethynyl-7-fluoronaphthalen-1-yl)-1-fluoro-12-((1-((3-hydroxypyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (45 mg, yield: 99.1%). ES-API: [M+H]⁺=739.2.

Step 5: tert-butyl (5S,5aS,6S,9R)-2-(8-ethynyl-7-fluoronaphthalen-1-yl)-1-fluoro-12-((1-((3-hydroxypyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (45 mg, 0.061 mmol) was dissolved in ethyl acetate (1 mL) and 4M hydrochloric acid in 1,4-dioxane (2 mL) was added under 0°C. The reaction mixture was stirred at room temperature for 1 hour. The reaction mixture was concentrated under reduced pressure. The crude product was purified by preparative HPLC (formic acid method 1) to obtain 1-((1-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]pyrrolidi-12-yl)oxy)methyl)cyclopropyl)methyl)pyrrolidine-3-ol (Z721, 5 mg, yield: 12.8%, formate), white solid. ES-API: [M+H]⁺= 639.2.

### Example 109 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-6-fluoronaphthalen-2-amine (Z722)

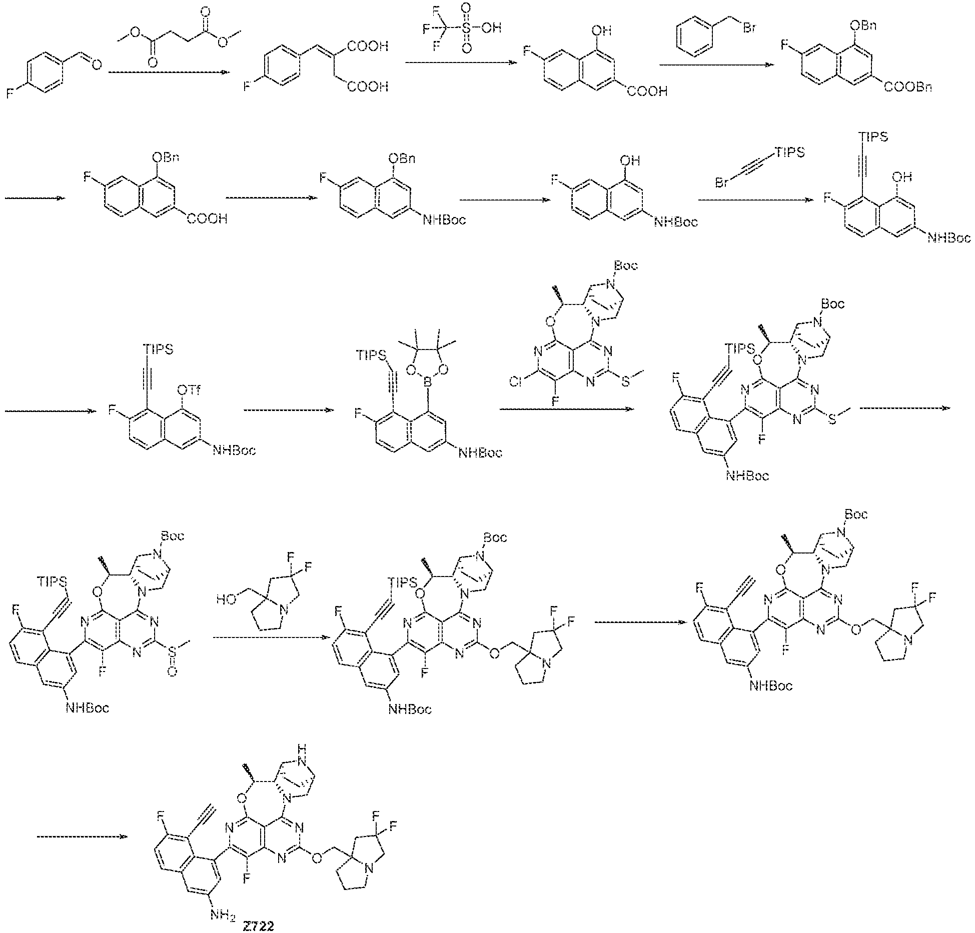

Step 1: Potassium tert-butoxide (14.92 g, 0.133 mol) was dissolved in tert-butanol (120 mL), and the reaction mixture was heated to reflux. Succinic acid dimethyl ester (21.15 g, 0.145 mol) and 4-fluorobenzaldehyde (15 g, 0.121 mol) in tert-butanol (30 mL) were added dropwise to the reaction mixture, which was then stirred overnight under reflux. Upon completion of the reaction, monitored by LCMS, the mixture was cooled to room temperature and concentrated under reduced pressure to remove the solvent. The residue was added to 150 mL of dilute hydrochloric acid (1 M) and extracted with ethyl acetate (50 mL × 3). The combined organic phases were dried, filtered, and concentrated to obtain an oily liquid. The oily liquid was dissolved in methanol (150 mL) and added to sodium hydroxide solution (150 mL, 2 M), and the mixture was heated to reflux overnight. The mixture was concentrated under reduced pressure, the crude product was dissolved in water (200 mL), and extracted with ethyl acetate (100 mL × 2). The aqueous phase was adjusted to pH 2.0 with concentrated hydrochloric acid and extracted with ethyl acetate (100 mL × 3), dried over anhydrous sodium sulfate, filtered and concentrated. The crude product was slurred in petroleum ether to obtain 2-(4-fluorobenzylidene)succinic acid (16.517 g, yield: 61%), a white solid. ES-API: [M+H]⁺= 225.1.

Step 2: 2-(4-Fluorobenzylidene)succinic acid (10 g, 44.605 mmol) was dissolved in trifluoromethanesulfonic acid (60 mL) and the mixture was stirred at room temperature overnight. Upon completion of the reaction, monitored by LCMS, the reaction mixture was slowly added to ice water (200 mL), resulting in the precipitation of a large amount of yellow solid. The mixture was extracted with 2-methyltetrahydrofuran (30 mL × 3), dried over anhydrous sodium sulfate, filtered, and concentrated. The crude product was purified by silica gel column chromatography (ethyl acetate:petroleum ether = 0 - 20 %) to obtain 6-fluoro-4-hydroxy-2-naphthoic acid (5.3 g, yield: 58%). ES-API: [M+18]⁺= 224.1.

Step 3: 6-Fluoro-4-hydroxy-2-naphthoic acid (5.3 g, 25.707 mmol), potassium carbonate (12.434 g, 89.974 mmol), and benzyl bromide (9.673 g, 56.555 mmol) were dissolved in N,N-dimethylformamide (60 mL). The reaction mixture was stirred at 70°C overnight. Upon completion of the reaction, monitored by LCMS, the mixture was cooled to room temperature and added to ice water (100 mL). The mixture was extracted with ethyl acetate (30 mL × 3), and the combined organic phases were washed with saturated brine (50 mL × 3), dried over anhydrous sodium sulfate, filtered, and concentrated. The crude product was purified by silica gel column chromatography (ethyl acetate/petroleum ether = 0 - 2 %) to obtain benzyl 4-(benzyloxy)-6-fluoro-2-naphthoate (7.029 g, yield: 71%), an off-white solid. ES-API: [M+ 18]⁺= 404.1.

Step 4: Under ice water bath condition, benzyl 4-(benzyloxy)-6-fluoro-2-naphthoate (7.0 g, 18.115 mmol) was dissolved in a 20 wt% potassium hydroxide solution (100 mL), the reaction mixture was slowly brought to room temperature, and then heated to 90°C and stirred overnight. Upon completion of the reaction (monitored by LCMS), the mixture was cooled to room temperature and the pH was adjusted to about 1.0 with concentrated hydrochloric acid, causing a large amount of white solid to precipitate. After filtration and concentration under reduced pressure, the crude product was stirred with ethyl acetate/petroleum ether (V:V= 1:10) to obtain 4-(benzyloxy)-6-fluoro-2-naphthoic acid (4.917 g, yield: 92%), an off-white solid. ES-API: [M+18]⁺= 314.0.

Step 5: 4-(Benzyloxy)-6-fluoro-2-naphthoic acid (4.4 g, 14.850 mmol), diphenyl phosphoryl azide (6.13 g, 22.275 mmol), and triethylamine (3.01 g, 29.700 mmol) were dissolved in toluene (50 mL) and tert-butanol (50 mL), and the reaction mixture was heated to 90°C and stirred for 4 hours. The reaction mixture was cooled to room temperature and concentrated under reduced pressure, and the crude product was purified by silica gel column chromatography (ethyl acetate/petroleum ether= 0 - 3 %) to obtain tert-butyl (4-(benzyloxy)-6-fluoronaphthalen-2-yl)carbamate (5.316 g, yield: 97%), an off-white solid. ES-API: [M- 55]⁺= 312.1.

Step 6: 10% palladium on carbon (1 g) was added to a methanol solution (60 mL) of tert-butyl (4-(benzyloxy)-6-fluoronaphthalen-2-yl)carbamate (5.32 g, 14.468 mmol). The reaction mixture was stirred overnight at room temperature under hydrogen protection. Upon completion of the reaction (monitored by LCMS), the mixture was concentrated under reduced pressure to obtain the crude product, tert-butyl (6-fluoro-4-hydroxynaphthalen-2-yl)carbamate (4.01 g). ES-API: [M-55]⁺= 222.1.

Step 7: Under nitrogen protection, tert-butyl (6-fluoro-4-hydroxynaphthalen-2-yl)carbamate (4.01 g, 14.468 mmol), (bromoethynyl)triisopropylsilane (4.536 g, 17.362 mmol), potassium acetate (2.84 g, 28.936 mmol), and dichloro(p-cymene)ruthenium(II) dimer (886 mg, 1.447 mmol) were dissolved in 1,4-dioxane (50 mL). The reaction mixture was heated to 100°C and stirred for 5 hours. Upon completion of the reaction (monitored by LCMS), the mixture was cooled to room temperature, concentrated under reduced pressure, and the crude product was purified by silica gel column chromatography (ethyl acetate/petroleum ether= 0 - 2 %) to obtain tert-butyl (6-fluoro-4-hydroxy-5-((triisopropylsilyl)ethynyl)naphthalen-2-yl)carbamate (4.01 g, yield: 61%), a yellow solid. ES-API: [M- 55]⁺= 402.2.

Step 8: At -40°C, tert-butyl (6-fluoro-4-hydroxy-5-((triisopropylsilyl)ethynyl)naphthalen-2-yl)carbamate (1 g, 2.185 mmol) was dissolved in dichloromethane (20 mL). N,N-Diisopropylethylamine (0.8 mL, 13.110 mmol) and trifluoromethanesulfonic anhydride (925 mg, 3.278 mmol) were added successively. The reaction mixture was stirred at -40°C for 1 hour. Upon completion of the reaction (monitored by LCMS), saturated sodium bicarbonate solution (30 mL) was added to quench the reaction. The mixture was extracted with dichloromethane (20 mL × 3), the organic phases were combined, concentrated under reduced pressure, and the crude product was purified by silica gel column chromatography (ethyl acetate/petroleum ether= 0 - 2 %) to obtain 3-((tert-butoxycarbonyl)amino)-7-fluoro-8-((triisopropylsilyl)ethynyl)naphthalen-1-yl trifluoromethanesulfonate (1107 mg, yield: 86%), a pale yellow solid. ES-API: [M-55]⁺= 534.1.

Step 9: Under nitrogen protection, 3-((tert-butoxycarbonyl)amino)-7-fluoro-8-((triisopropylsilyl)ethynyl)naphthalen-1-yl trifluoromethanesulfonate (1.1 g, 1.865 mmol), pinacolborane (710 mg, 2.797 mmol), potassium acetate (458 mg, 4.662 mmol), and [1,1'bis(diphenylphosphino)ferrocene]dichloropalladium(II) (136 mg, 0.187 mmol) were dissolved in 1,4-dioxane (40 mL). The reaction mixture was heated to 100°C and stirred overnight. Upon completion of the reaction (monitored by LCMS), the mixture was cooled to room temperature and concentrated under reduced pressure. The crude product was purified by silica gel column chromatography (ethyl acetate/petroleum ether- 0 - 5 %) to obtain tert-butyl (6-fluoro-4-(4,4,5,5-tetramethyl-1,3,2-dioxaborolan-2-yl)-5-((triisopropylsilyl)ethynyl)naphthalen-2-yl)carbamate (185 mg, yield: 17%), an off-white solid. ES-API: [M+ H]⁺= 568.3.

Step 10: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (70 mg, 0.145 mmol) and tert-butyl (6-fluoro-4-(4,4,5,5-tetramethyl-1,3,2-dioxaborolan-2-yl)-5-((triisopropylsilyl)ethynyl)naphthalen-2-yl)carbamate (87 mg, 0.152 mmol), potassium phosphate (92 mg, 0.436 mmol), and methanesulfonic acid [(n-butyl)bis(1-adamantyl)phosphine](2-amino-1,1'-biphenyl)palladium(II) (106 mg, 0.145 mmol) were dissolved in tetrahydrofuran (3 mL) and water (0.6 mL). The reaction mixture was stirred at 80°C under microwave for 1 hour. Upon completion of the reaction (monitored by LCMS), the mixture was dried over anhydrous sodium sulfate, concentrated under reduced pressure, and the crude product was purified by silica gel column chromatography (ethyl acetate/petroleum ether-- 0 - 20 %) to obtain tert-butyl (5S,5aS,6S,9R)-2-(3-((tert-butoxycarbonyl)amino)-7-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (135 mg). ES-API: [M+H]⁺= 887.3.

Step 11: Under an ice water bath, tert-butyl (SS,SaS,6S,9R)-2-(3-((tert-butoxycarbonyl)amino)-7-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (135 mg, 0.152 mmol) was dissolved in anhydrous dichloromethane (6 mL). Then, m-chloroperbenzoic acid (40 mg, 0.198 mmol) was added to the solution, and the reaction mixture was stirred at 0°C for 30 minutes. Upon completion of the reaction (monitored by LCMS), dichloromethane (15 mL) was added to dilute the reaction mixture, which was then quenched with saturated sodium bisulfite solution (15 mL), followed by washed with sodium bicarbonate solution (15 mL) and saturated brine (15 mL). The organic phase was dried over anhydrous sodium sulfate, filtered, and concentrated to obtain the crude product of tert-butyl (5S,5aS,6S,9R)-2-(3-((tert-butoxycarbonyl)amino)-7-fluoro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (92 mg, yield: 67%). ES-API: [M+ H]⁺= 903.3.

Step 12: (2,2-difluorotetrahydro-1H-pyrrolizin-7a(5H)-yl)methanol (36 mg, 0.204 mmol) was dissolved in anhydrous tetrahydrofuran (5 mL) under an ice water bath. Sodium hydride (12 mg, 0.306 mmol) was added, and the reaction mixture was stirred for 10 minutes under an ice water bath. Then, a solution of tert-butyl (5S,5aS,6S,9R)-2-(3-((tert-butoxycarbonyl)amino)-7-fluoro-8-((triisopropylsilyl)ethynyl)naphthalen-1-yl)-1-fluoro-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (92 mg, 0.102 mmol) in tetrahydrofuran (5 mL) was added dropwise. The reaction was proceeded at 0°C for 30 minutes. Upon completion of the reaction (monitored by LCMS), the mixture was diluted with ethyl acetate (10 mL), washed with saturated ammonium chloride solution (10 mL × 2) and saturated brine (10 mL × 3), then dried over anhydrous sodium sulfate, filtered, and concentrated under reduced pressure to obtain the crude product of tert-butyl (SS,SaS,6S,9R)-2-(3-((tert-butoxycarbonyl)amino)-7-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (103 mg). ES-API: [M+ H]⁺= 1016.5.

Step 13: Cesium fluoride (307 mg, 2.020 mmol) was added to a solution of tert-butyl (5S,5aS,6S,9R)-2-(3-((tert-butoxycarbonyl)amino)-7-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (103 mg, 0.101 mmol) in N,N-dimethylformamide (5 mL). The reaction mixture was stirred at room temperature for 1 hour. Upon completion of the reaction (monitored by LCMS), the reaction mixture was diluted with ethyl acetate (20 mL), washed with water (15 mL × 2) and saturated brine (15 mL × 3), and then dried over anhydrous sodium sulfate, filtered, and concentrated to obtain tert-butyl (5S,5aS,6S,9R)-2-(3-((tert-butoxycarbonyl)amino)-8-ethynyl-7-fluoronaphthalen-1-yl)-12-((2,2-difluorotetrahydro-1H-pyrrolizin-7a(5H)-yl)methoxy)-1 -fluoro-5-methyl-5a,6,7,8,9,10-hexahydro-5H-4-oxa-3,10a,11,13,14-pentaaza-6,9-methanonaphtho[1,8-ab]heptalene-14-carboxylate (87 mg). ES-API: [M+H]⁺= 859.3.

Step 14: Under an ice water bath, 4 M hydrochloric acid-dioxane solution (5 mL) was slowly added to a methanol (1 mL) solution of tert-butyl (5S,5aS,6S,9R)-2-(3-((tert-butoxycarbonyl)amino)-8-ethynyl-7-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (87 mg, 0.101 mmol). The reaction mixture was warm to room temperature and stirred for 1 hour. Upon completion of the reaction (monitored by LCMS), the reaction mixture was concentrated under reduced pressure to dryness, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-6-fluoronaphthalen-2-amine (Z722, 13.86 mg, yield: 21 %), pale yellow solid. ES-API: [M+H]⁺= 660.3.

### Example 110 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-6-fluoronaphthalen-2-o1 (Z723)

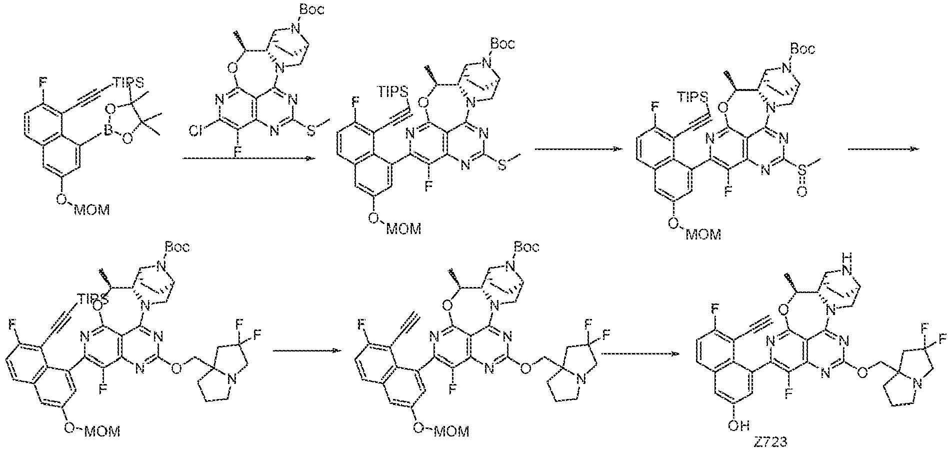

Step 1: To a solution of tert-butyl (SS,SaS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (200 mg, 0.415 mmol) and ((2-fluoro-6-(methoxymethoxy)-8-(4,4,5,5-tetramethyl-1,3,2-dioxaborolane-2-yl)naphthalen-1-yl)ethynyl)triisopropylsilane (425 mg, 0.829 mmol) in tetrahydrofuran (30 mL) and water (5 mL), potassium phosphate (352.0 mg, 1.660 mmol), methanesulfonic acid [n-butyl di(1-adamantyl)phosphine](2-amino-1,1'-biphenyl-2-yl)palladium(II) (45.31 mg, 0.062 mmol) were added, and the mixture was bubbled with N₂ and heated in an oil bath at 80°C for 3 hours. Upon completion of the reaction, the mixture was extracted with ethyl acetate (30 mL × 2) and saturated brine (30 mL). The combined ethyl acetate layers were dried over anhydrous sodium sulfate, filtered, and the filtrate was concentrated under reduced pressure. The crude product was purified by silica gel column chromatography (0-4% methanol/dichloromethane) to obtain tert-butyl (5S,5aS,6S,9R)-1-fluoro-2-(7-fluoro-3-(methoxymethoxy)-8-((triisopropylsilyl)ethynyl)naphthalen-1-yl)-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (310 mg, yield: 89.78%), a yellow solid. ES-API: [M+H]⁺=832.2.

Step 2: At 0°C, m-chloroperbenzoic acid (83.58 mg, 0.484 mmol) was added to a dichloromethane (15 mL) solution of tert-butyl (5S,5aS,6S,9R)-1-fluoro-2-(7-fluoro-3-(methoxymethoxy)-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (310 mg, 0.373 mmol). The mixture was stirred at room temperature for 1 hour, quenched with saturated sodium sulfite, and extracted with dichloromethane (100 mL × 2). The organic layer was dried over anhydrous sodium sulfate and filtered, and the filtrate was concentrated. The crude product was purified by silica gel column chromatography (methanol/dichloromethane 0-10%) to obtain tert-butyl (SS,SaS,6S,9R)-1-fluoro-2-(7-fluoro-3-(methoxymethoxy)-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (350 mg, crude), a white solid. ES-API:[M+H]⁺=848.2.

Step 3: At 0°C, sodium hydride (75.0 mg, 1.865 mmol) was added to a solution of (2,2-difluorotetrahydro-1H-pyrazin-7a(5H)-yl)methanol (132.18 mg, 0.746 mmol) in tetrahydrofuran (10.0 mL). The mixture was stirred for 10 minutes at the same temperature, and then tert-butyl (5S,5aS,6S,9R)-1-fluoro-2-(7-fluoro-3-(methoxymethoxy)-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (314.86 mg, 0.373 mmol) in tetrahydrofuran (5 mL) was added. The mixture was stirred for 10 minutes at 0°C and then at room temperature for 0.5 hours. Upon completion of the reaction, the reaction mixture was quenched with saturated sodium bicarbonate solution and extracted with ethyl acetate. The organic phase was washed with saturated brine, dried over anhydrous sodium sulfate, filtered, and concentrated. The crude product was purified by silica gel column chromatography (methanol/dichloromethane 0-5%) to obtain tert-butyl (5S,5aS,6S,9R)-12-((2,2-difluorotetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (280 mg, yield: 78.48%), a yellow solid. ES-API: [M+H]⁺=961.3.

Step 4: To a solution of tert-butyl (5S,5aS,6S,9R)-12-((2,2-difluorotetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptalene-14-carboxylate (280.0 mg, 0.299 mmol) in N,N-dimethylformamide (15.0 mL), cesium fluoride (2.75 g, 18.14 mmol) was added. The reaction mixture was stirred at room temperature for 0.5 hours. Upon completion of the reaction, the reaction mixture was quenched with water and extracted with ethyl acetate. The organic phase was washed with saturated brine, dried over anhydrous sodium sulfate, filtered, and concentrated to obtain tert-butyl (5S,5aS,6S,9R)-12-((2,2-difluorotetrahydro-1H-pyrrolizin-7a(5H)-yl)methoxy)-2-(8-ethynyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (300 mg, crude), a yellow oil. ES-API: [M+H]⁺=805.2.

Step 5: Under an ice-water bath, 4 M hydrochloric acid-dioxane solution (10.0 mL) was slowly added to a methanol (15.0 mL) solution of tert-butyl (5S,5aS,6S,9R)-12-((2,2-difluorotetrahydro-1H-pyrrolizin-7a(5H)-yl)methoxy)-2-(8-ethynyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (240.0 mg, 0.2990 mmol). The reaction mixture was stirred at room temperature for 0.5 hours. Upon completion of the reaction (monitored by LCMS), the reaction mixture was concentrated under reduced pressure to dryness, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-6-fluoronaphthalen-2-ol (Z723, 77.90 mg, yield: 18.82%), pale yellow solid. ES-API: [M+H]⁺= 661.2.

### Example 111 Synthesis of 4-((5S,5aS,6S,9R)-12-(((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z724)

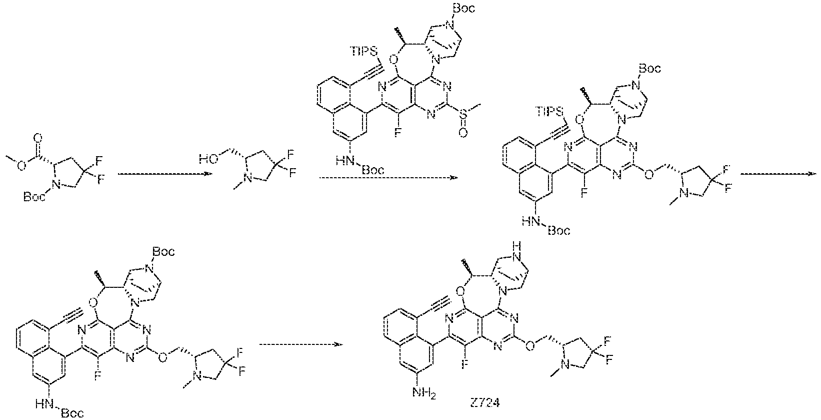

Step 1: Under nitrogen protection, 1-(tert-butyl) 2-methyl (S)-4,4-difluoropyrrolidine-1,2-dicarboxylate (1.5 g, 5.65 mmol) was dissolved in anhydrous tetrahydrofuran (5 mL). The mixture was placed in an ice-water bath, and a lithium aluminium hydride solution (17.0 mL, 17.0 mmol, 1 M in tetrahydrofuran) was slowly added dropwise to the above solution. After the addition was completed, the reaction mixture was warmed to room temperature and stirred for 1 hour. Upon completion of the reaction (monitored by LCMS), sodium sulfate decahydrate was added to quench the reaction. The mixture was stirred at room temperature for 0.5 hours, then filtered. The filtrate was concentrated under reduced pressure to obtain the crude product, (S)-(4,4-difluoro-1-methylpyrrolidin-2-yl)methanol (850 mg, yield: 99.4%). ES-API: [M+H]⁺= 152.1.

Step 2: (S)-(4,4-difluoro-1-methylpyrrolidin-2-yl)methanol (67 mg, 0.44 mmol) was dissolved in anhydrous tetrahydrofuran (5 mL). Under an ice-water bath, sodium hydride (29 mg, 0.73 mmol) was added, and the reaction was proceeded for 30 minutes in the ice-water bath. A tetrahydrofuran (5 mL) solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (130 mg, 0.15 mmol) was added dropwise to the reaction mixture. The mixture reacted at 0 °C for 30 minutes. Upon completion of the reaction (monitored by LCMS), the reaction mixture was diluted with ethyl acetate (30 mL) and washed with saturated ammonium chloride solution (10 mL × 2) and saturated brine (10 mL × 3). The organic phase was dried over anhydrous sodium sulfate and filtered. The filtrate was concentrated under reduced pressure. The crude product was purified by flash silica gel column chromatography (ethyl acetate/petroleum ether: 0-80%) to obtain the target compound, tert-butyl (5S,5aS,6S,9R)-2-(3-((tert-butoxycarbonyl)amino)-8-((triisopropylsilyl)ethynyl)naphthalen-1-yl)-12-(((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (100 mg, yield: 70.0%), a pale yellow solid. ES-API: [M+H]⁺=972.3.

Step 3: Cesium fluoride (156 mg, 1.03 mmol) was added to a solution of tert-butyl (SS,SaS,6S,9R)-2-(3-((tert-butoxycarbonyl)amino)-8-((triisopropylsilyl)ethynyl)naphthalen-1-yl)-12-(((S)-4,4-difluoro-1-methylpyrrolidin-2-yl)methoxy)-1-fluoro-5-methyl-Sa,6,7,8,9,10-hexahydro-SH-4-oxa.-3,10a,11,13,14-pentaaza-6,9-methanonaphtho[1,8-ab]heptalene-14-carboxylate (100 mg, 0.10 mmol) in N,N-dimethylformamide (2 mL). The reaction mixture was stirred at room temperature for 1 hour. Upon completion of the reaction (monitored by LCMS), the reaction mixture was diluted with ethyl acetate (20 mL) and washed with water (15 mL × 2) and saturated brine (15 mL × 3), dried over anhydrous sodium sulfate, filtered and concentrated. The product tert-butyl (5S,5aS,6S,9R)-2-(3-((tert-butoxycarbonyl)amino)-8-ethynylnaphthalen-1-yl)-12-(((S)-4,4-difluoro-1 -methylpyrrolidin-2-yl)methoxy)-1 -fhroro-5-methyl-5a,6,7,8,9,10-hexahydro-5H-4-oxa-3,10a,11,13,14-pentaaza-6,9-methanonaphtho[1,8-ab]heptalene-14-carboxylate (84 mg, yield: 100%) was obtained, a pale yellow solid. ES-API: [M+ H]⁺= 816.3.

Step 4: Tert-butyl (5S,5aS,6S,9R)-2-(3-((tert-butoxycarbonyl)amino)-8-ethynylnaphthalen-1-yl)-12-(((S)-4,4-difluoro-1-methylpyrrolidin-2-yl)-methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (60 mg, 0.074 mmol) was dissolved in ethyl acetate (1.5 mL). At 0°C, 4M hydrochloric acid in 1,4-dioxane (1.5 mL) was added, and the reaction mixture was stirred at room temperature for 1 hour. The reaction mixture was concentrated under reduced pressure, alkalinized with saturated sodium bicarbonate solution, and extracted with ethyl acetate (20 mL × 2), dried over anhydrous sodium sulfate, filtered and concentrated to obtain a crude product. The crude product was purified by preparative HPLC (ammonium bicarbonate method 1) to obtain the target product, 4-((SS,SaS,6S,9R)-12-(((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z724, 12 mg, yield: 26.5%), white solid. ¹H NMR (400 MHz, DMSO-d6) δ 7.72 - 7.62 (m, 1H), 7.33 - 7.22 (m, 2H), 7.03 - 6.85 (m, 2H), 5.63 (d, *J* = 12.1 Hz, 2H), 5.22 - 5.07 (m, 1H), 4.56 - 4.40 (m, 2H), 4.39 - 4.27 (m, 1H), 3.97 (d, *J* = 8.8 Hz, 1H), 3.85 - 3.50 (m, 2H), 3.46 (t, *J* = 5.2 Hz, 1H), 3.42 - 3.33 (m, 2H), 3.10 - 3.01 (m, 1H), 2.96 (s, 1H), 2.74 - 2.60 (m, 1H), 2.38 (s, 3H), 2.29 - 2.14 (m, 1H), 1.90 - 1.80 (m, 1H), 1.78 - 1.53 (m, 3H), 1.49 - 1.39 (m, 3H).ES-API: [M+H)⁺= 616.2.

### Example 112 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-1-fluoronaphthalen-2-amine (Z725)

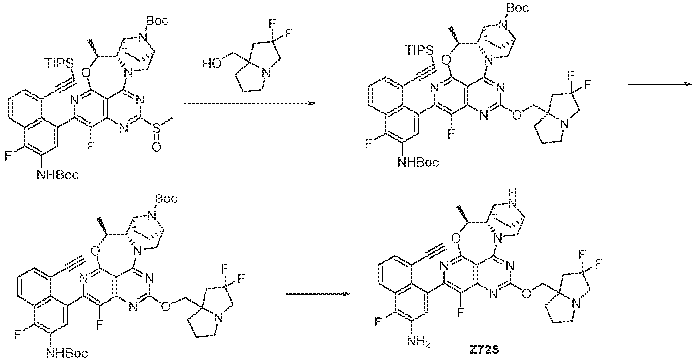

Step 1: Sodium hydride (39 mg, 0.97 mmol) and tetrahydrofuran (8 mL) were added to a round-bottom flask. The mixture was cooled to 0°C, and (2,2-difluorotetrahydro-1H-pyrrolizin-7a(5H)-yl)methanol (74 mg, 0.41 mmol) was added to the reaction mixture. The reaction was proceeded at room temperature for 5 minutes. Tert-butyl (SS,SaS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (250 mg, 0.28 mmol) was added to the above reaction mixture, which then reacted at room temperature for 30 minutes. Upon completion of the reaction (monitored by LCMS), the reaction mixture was quenched with saturated ammonium chloride solution (30 mL) and extracted with ethyl acetate (20 mL × 2). The organic phase was washed with saturated brine (30 mL), dried over anhydrous sodium sulfate, filtered, and concentrated. The crude product was purified by flash silica gel column chromatography (ethyl acetate/petroleum ether: 0-30%) to obtain tert-butyl (5S,5aS,6S,9R)-2-(3-((tert-butoxycarbonyl)amino)-4-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (130 mg, yield: 46%). ES-API: [M+H]⁺=1016.2.

Step 2: Tert-butyl (5S,5aS,6S,9R)-2-(3-((tert-butoxycarbonyl)amino)-4-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (130 mg, 0.13 mmol), cesium fluoride (194 mg, 1.28 mmol), and N,N-dimethylformamide (3 mL) were added to a round-bottom flask. The reaction mixture was stirred at room temperature for 1 hour. Water (30 mL) was added to the reaction mixture, followed by extraction with ethyl acetate (30 mL × 3). The organic phase was washed with saturated brine (30 mL × 2), dried over anhydrous sodium sulfate, filtered, and concentrated to obtain the crude product, tert-butyl (5S,5aS,6S,9R)-2-(3-((tert-butoxycarbonyl)amino)-8-ethynyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-axa-3,10a,11,13,14-pentaaza-6,9-methanonaphtho[1,8-ab]heptalene-14-carboxylate (110 mg). ES-API: [M+H]⁺=860.1.

Step 3: Tert-butyl (5S,5aS,6S,9R)-2-(3-((tert-butoxycarbonyl)amino)-8-ethynyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (110 mg, crude product) was dissolved in ethyl acetate (3 mL). The solution was cooled to 0°C, and 4M hydrochloric acid/1,4-dioxane solution (3 mL, 12 mmol) was added. The reaction mixture was stirred at room temperature for 1 hour. The reaction mixture was concentrated to dryness at 40°C, and the crude product was purified by preparative HPLC (ammonium bicarbonate method 1) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-1-fluoronaphthalen-2-amine (Z725, 35mg, yield: 41%). ES-API: [M+H]⁺=660.2. ¹HNMR(400 MHz, CD₃OD): 8.00-7.98(m, 1H), 7.50-7.39(m, 2H), 7.24-7.13 (m, 1H), 5.39-5.35(m, 1H), 4.50-4.47(m, 1H), 4.27-4.22(m, 2H), 4.06-4.03(m, 1H), 3.73-3.60(m, 2H), 3.48-3.15(m, 5H), 2.88-2.85(m, 1H), 2.60-2.55(m, 1H), 2.36-2.25(m, 1H), 2.16-1.77(m, 8H), 1.55-1.53(m, 3H).

### Example 113 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-S-ethynylquinolin-2-amine (Z726)

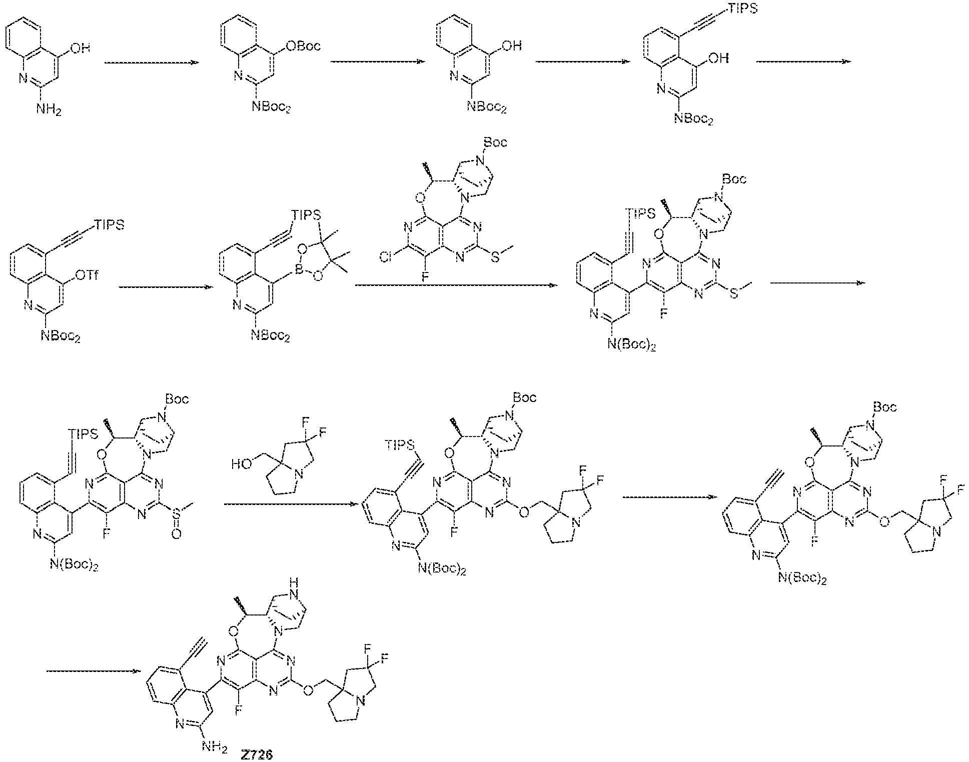

Step 1: Di-tert-butyl carbonate (10.8 mL, 46.82 mmol) and 4-dimethylaminopyridine (343 mg, 2.809 mmol) were added to a solution of 2-aminoquinolin-4-ol (1.5 g, 9.364 mmol) in tetrahydrofuran (50 mL). The reaction mixture was stirred at room temperature for 3 hours. Upon completion of the reaction (monitored by LCMS), most of the solvent was removed under reduced pressure. The residue was dissolved in ethyl acetate (20 mL) and washed with water (20 mL). The aqueous phase was extracted with ethyl acetate (15 mL × 3). The organic phases were combined, dried over anhydrous sodium sulfate, filtered, and concentrated to obtain the crude product, tert-butyl (tertbutoxycarbonyl)(4-((tert-butoxycarbonyl)oxy)quinolin-2-yl)carbamate (4.312 g). ES-API: [M+ H]⁺= 461.2.

Step 2: Sodium carbonate (992 mg, 9.363 mmol) was added to a solution of tert-butyl (tertbutoxycarbonyl)(4-((tert-butoxycarbonyl)oxy)quinolin-2-yl)carbamate (4.312 g, 9.363 mmol) in methanol (50 mL) and water (10 mL). The reaction mixture was stirred at room temperature overnight. Upon completion of the reaction (monitored by LCMS), most of the solvent was removed under reduced pressure. The residue was dissolved in water (15 mL), the pH was adjusted to weakly acidic with oxalic acid, and a large amount of insoluble material was precipitated. The solution was filtered, and dried under reduced pressure to obtain the crude product, 2-(N,N-di-tert-butoxycarbonyl)aminoquinoline-4-ol (2.908 g, yield: 86 %). ES-API: [M+ H]⁺= 361.1.

Step 3: Under nitrogen protection, 2-(N,N-di-tert-butoxycarbonyl)aminoquinoline-4-ol (1.5 g, 4.162 mmol), (bromoethynyl)triisopropylsilane (1.305 g, 4.9945 mmol), potassium acetate (817 mg, 8.324 mmol), and dichloro(p-cymene)ruthenium(II) dimer (255 mg, 0.416 mmol) were dissolved in 1,4-dioxane (30 mL). The reaction mixture was heated to 100°C and stirred overnight. Upon completion of the reaction (monitored by LCMS), the reaction mixture was cooled to room temperature, concentrated under reduced pressure, and the crude product was purified by silica gel column chromatography (ethyl acetate/petroleum ether = 0 - 10 %) to obtain a yellow solid, 2-(N,N-di-tert-butoxycarbonyl)amino-5-((triisopropylsilyl)ethynyl)quinoline-4-ol (1.442 g, yield: 64 %). ES-API: [M+ H]⁺= 541.3.

Step 4: At -40°C, 2-(N,Nili-tert-butoxycarbonyl)amino-5-((triisopropylsilyl)ethynyl)quinoline-4-ol (1.442 mg, 2.667 mmol) was dissolved in dichloromethane (30 mL). N,N-diisopropylethylamine (2.2 mL, 15.999 mmol) and trifluoromethanesulfonic anhydride (0.7 mL, 4.000 mmol) were successively added to the reaction mixture. The mixture was stirred at -40°C for 30 minutes. Upon completion of the reaction (monitored by LCMS), saturated sodium bicarbonate solution (30 mL) was added to quench the reaction. The mixture was extracted with dichloromethane (20 mL × 3) and the organic phases were combined and concentrated under reduced pressure. The crude product was purified by silica gel column chromatography (ethyl acetate/petroleum ether = 0 - 1.9%) to obtain a yellow oily substance, 2-(N,N-di-tert-butoxycarbonyl)amino-5-((triisopropylsilyl)ethynyl)quinoline-4-yl trifluoromethanesulfonate (1.443 g, yield: 80%). ES-API: [M+ H]⁺= 673.2.

Step 5: Under nitrogen protection, 2-(N,N-di-tert-butoxycarbonyl)amino-5-((triisopropylsilyl)ethynyl)quinoline-4-yl trifluoromethanesulfonate (1.443 g, 2.145 mmol), pinacolborane (1.362 g, 5.362 mmol), potassium acetate (526 mg, 5.362 mmol), and [1,1'-bis(diphenylphosphino)ferrocene]dichloropalladium(II) (300 mg, 0.429 mmol) were dissolved in 1,4-dioxane (30 mL). The reaction mixture was heated to 105°C and stirred overnight. Upon completion of the reaction (monitored by LCMS), the reaction mixture was cooled to room temperature, concentrated under reduced pressure, and the crude product was purified by silica gel column chromatography (ethyl acetate/petroleum ether = 0 - 2%) to obtain 4-(4,4,5,5-tetramethyl-1,3,2-dioxaborolane-2-yl)-5-((triisopropylsilyl)ethynyl)quinoline-2-(N,N-di-tert-butoxycarbonyl)amine (998 mg, yield: 72%), a pale yellow solid. ES-API: [M+ H]⁺= 651.3.

Step 6: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (105 mg, 0.218 mmol), 4-(4,4,5,5-tetramethyl-1,3,2-dioxaborolane-2-yl)-5-((triisopropylsilyl)ethynyl)quinoline-2-(N,N-di-tert-butoxycarbonyl)amine (149 mg, 0.229 mmol), potassium phosphate (139 mg, 0.654 mmol), and methanesulfonic acid [n-butyl bis(1-adamantyl)phosphine](2-amino-1,1'-biphenyl-2-yl)palladium(II) (24 mg, 0.033 mmol) were dissolved in tetrahydrofuran (3 mL) and water (0.6 mL). The reaction mixture was stirred at 80°C under microwave for 40 minutes. Upon completion of the reaction (monitored by LCMS), the reaction mixture was dried over anhydrous sodium sulfate and concentrated under reduced pressure. The crude product was purified by silica gel column chromatography (ethyl acetate/petroleum ether = 0 - 15%) to obtain tert-butyl (5S,5aS,6S,9R)-2-(2-(bis(tert-butoxycarbonyl)amino)-5-((triisopropylsilyl)ethynyl)quinolin-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (184 mg, yield: 87%). ES-API: [M+H]⁺= 970.3.

Step 7: Under an ice-water bath, tert-butyl (SS,SaS,bS,9R)-2-(2-(bis(tert-butoxycarbonyl)amino)-5-((triisopropylsilyl)ethynyl)quinolin-4-yl)-1-fluoro-5-methyl-12-(methylthio)-Sa,6,7,8,9,10-hexahydro-5H-4-oxa-3,10a,11,13,14-pentaaza-6,9-methanonaphtho[1,8-ab]heptalene-14-carboxylate (184 mg, 0.190 mmol) was dissolved in anhydrous dichloromethane (8 mL). Then, m-chloroperoxybenzoic acid (50 mg, 0.247 mmol) was added to the solution, and the reaction mixture was stirred at 0°C for 30 minutes. Upon completion of the reaction (monitored by LCMS), the reaction mixture was diluted with dichloromethane (15 mL) and quenched with saturated sodium bisulfite solution (15 mL). The mixture was then washed with sodium bicarbonate solution (15 mL) and saturated brine (15 mL). The organic phase was dried over anhydrous sodium sulfate, filtered, and concentrated to obtain the crude product, tert-butyl (5S,5aS,6S,9R)-2-(2-(bis(tert-butoxycarbonyl)amino)-5-((triisopropylsilyl)ethynyl)quinolin-4-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (187 mg, yield: 67%). ES-API: [M+ H]⁺= 986.3.

Step 8: Under an ice-water bath, (2,2-difluorotetrahydro-1H-pyrrolizin-7a(5H)-yl)methanol (33 mg, 0.189 mmol) was dissolved in anhydrous tetrahydrofuran (3 mL), and sodium hydride (11.3 mg, 0.283 mmol) was added. The mixture reacted under ice-water bath for 10 minutes. Then, a tetrahydrofuran (3 mL) solution of tert-butyl (5S,5aS,6S,9R)-2-(2-(bis(tert-butoxycarbonyl)amino)-5-((triisopropylsilyl)ethynyl)quinolin-4-yl)-1-fluoro-5-methyl-12-(methylsulfinyl)-5a,6,7,8,9,10-hexahydro-5H-4-oxa-3,10a,1 1,1 3,14-pentaaza-6,9-methanonaphtho[1,8-ab]heptalene-14-carboxylate (93 mg, 0.094 mmol) was added dropwise, and the reaction was proceeded at 0°C for 30 minutes. Upon completion of the reaction (monitored by LCMS), the reaction mixture was diluted with ethyl acetate (10 mL) and washed with saturated ammonium chloride solution (10 mL × 2) and saturated brine (10 mL × 3). The organic phase was dried over anhydrous sodium sulfate, filtered, and concentrated under reduced pressure to obtain the crude product, tert-butyl (5S,5aS,6S,9R)-2-(2-(bis(tert-butoxycarbonyl)amino)-5-((triisopropylsilyl)ethynyl)quinolin-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (103 mg). ES-API: [M- 100]⁺= 998.5.

Step 9: Cesium fluoride (143 mg, 0.940 mmol) was added to a solution of tert-butyl (5S,5aS,6S,9R)-2-(2-(bis(tert-butoxycarbonyl)amino)-5-((triisopropylsilyl)ethynyl)quinolin-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate(103 mg, 0.094 mmol) in N,N-dimethylformamide (5 mL). The reaction mixture was stirred at room temperature for 1 hour. Upon completion of the reaction (monitored by LCMS), the reaction mixture was diluted with ethyl acetate (20 mL) and washed with water (15 mL × 2) and saturated brine (15 mL × 3). The mixture was then dried over anhydrous sodium sulfate, filtered, and concentrated to obtain tert-butyl (SS,SaS,6S,9R)-2-(2-(bis(tert-butoxycarbonyl)amino)-5-ethynylquinolin-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (88 mg). ES-API: [M-100]⁺= 843.2.

Step 10: Under an ice-water bath, a 4 M solution of hydrochloric acid-dioxane (10 mL) was slowly added to a methanol (1 mL) solution of tert-butyl (5S,5aS,6S,9R)-2-(2-(bis(tert-butoxycarbonyl)amino)-5-ethynylquinolin-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (88 mg, 0.093 mmol). The reaction mixture was then warm to room temperature and stirred for 1 hour. Upon completion of the reaction (monitored by LCMS), the mixture was concentrated under reduced pressure to dryness. The residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylquinolin-2-amine (Z726, 12.99 mg, yield: 22 %), white solid. ES-API: [M+H]⁺= 643.0. ¹H NMR (400 MHz, CD₃OD) δ 7.67- 7.64 (m, 1 H), 7.54- 7.47 (m, 1 H), 7.44- 7.37 (m, 1 H), 6.91-6.82 (m, 1 H), 5.40- 5.34 (m, 1 H), 4.57- 4.51 (m, 1 H), 4.31- 4.23 (m, 2 H), 4.09 (d, J= 8.4 Hz, 1 H), 3.72- 3.70 (m, 1 H), 3.63- 3.61 (m, 1 H), 3.48- 3.40 (m, 1 H), 3.22- 3.09 (m, 4 H), 2.89- 2.83 (m, 1 H), 2.61- 2.52 (m, 1 H), 2.39- 2.28 (m, 1 H), 2.21- 1.75 (m, 8 H), 1.57 (t, J1= 6.4 Hz, J2= 12.8 Hz, 3 H).

### Example 114 Synthesis of 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-1-fluoronaphthalen-2-amine (Z727)

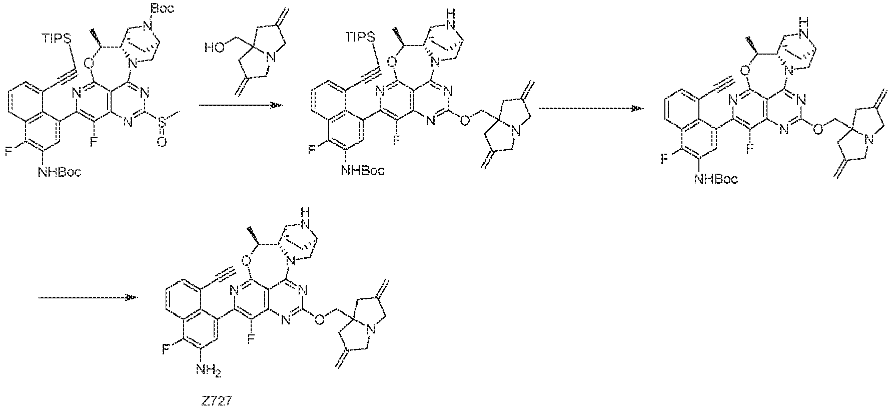

Step 1: At 0°C, sodium hydride (12 mg, 0.5 mmol) was added to a tetrahydrofuran (10.0 mL) solution of (2,6-dimethylenetetrahydro-1H-pyrrolizin-7a(5H)-yl)methanol (40.25 mg, 0.244 mmol). After the mixture stirred for 10 minutes at this temperature, a tetrahydrofuran solution (5 mL) of tert-butyl (SS,SaS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (110 mg, 0.122 mmol) was added. The reaction mixture was stirred for 10 minutes at 0°C, then at room temperature for 0.5 hours. Upon completion of the reaction, the mixture was quenched with saturated sodium bicarbonate solution and extracted with ethyl acetate. The organic layer was washed with saturated brine, dried over anhydrous sodium sulfate, filtered, and concentrated. The crude product was purified by silica gel column chromatography (methanol/dichloromethane = 0-5%) to obtain tert-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-1-fluoro-5-((triisopropylsilyl)ethynyl)naphthalen-2-yl)carbamate (70 mg, 0.070 mmol, yield: 57.23%), a yellow solid. ES-API: [M+H]⁺=1004.2.

Step 2: To a solution of tert-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-1-fluoro-5-((triisopropylsilyl)ethynyl)naphthalen-2-yl)carbamate (70 mg, 0.070 mmol) in N,N-dimethylformamide (1.0 mL), cesium fluoride (105.87 mg, 0.697 mmol) was added. The reaction mixture was stirred at room temperature for 0.5 hours. Upon completion of the reaction, the mixture was quenched with water and extracted with ethyl acetate. The organic layer was washed with saturated brine, dried over anhydrous sodium sulfate, filtered, and concentrated to obtain tert-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-1-fluoronaphthalen-2-yl)carbamate (35 mg, 0.044 mmol, yield: 83.78%), a yellow oil. ES-API: [M+H]⁺=848.3.

Step 3: Under an ice-water bath, a 4 M solution of hydrochloric acid-dioxane (1.0 mL) was slowly added to an ethyl acetate (1.0 mL) solution of tert-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynyl-1-fluoronaphthalen-2-yl)carbamate (35 mg, 0.044 mmol). The reaction mixture was stirred at room temperature for 0.5 hours. Upon completion of the reaction (monitored by LCMS), the mixture was concentrated under reduced pressure to dryness. The residue was purified by preparative HPLC (ammonium bicarbonate method 2) to obtain 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynyl-1-fluoronaphthalen-2-amine (Z727, 7.1mg, yield: 23.24%), pale yellow solid. ES-API: [M+H]⁺= 648.0.

### Example 115 Synthesis of 5-ethynyl-1-fluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z728)

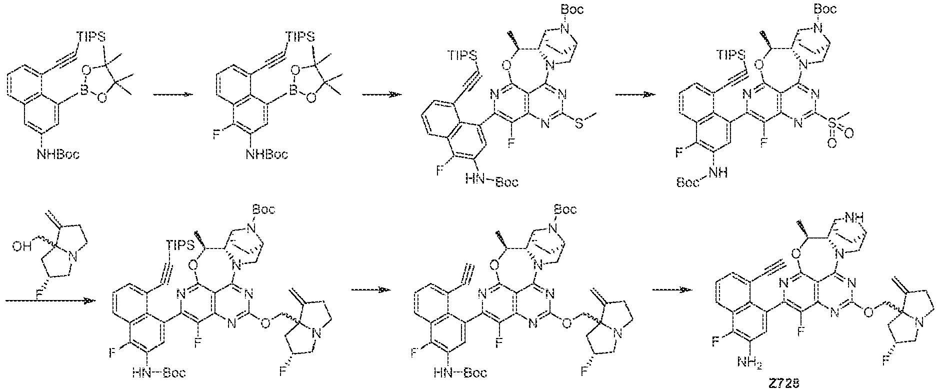

Step 1: Tert-butyl (4-(4,4,5,5-tetramethyl-1,3,2-dioxaborolane-2-yl)-5-((triisopropylsilyl)ethynyl)naphthalen-2-yl)carbamate (1000 mg, 1.819 mmol) was dissolved in acetonitrile (50 mL). At room temperature, 1-chloromethyl-4-fluoro-1,4-diazabicyclo[2.2.2]octane bis(tetrafluoroborate) salt (837.91 mg, 2.365 mmol) was added to the solution. After the addition was complete, the reaction mixture was stirred at room temperature for 17 hours. Upon completion of the reaction, the reaction mixture was concentrated to remove acetonitrile. The crude product was purified by a flash silica gel column (ethyl acetate/petroleum ether: 0-2%) to obtain (1-fluoro-4-(4,4,5,5-tetramethyl-1,3,2-dioxaborolane-2-yl)-5-((triisopropylsilyl)ethynyl)naphthalen-2-yl)carbamate (550 mg, 0.969 mmol, yield: 53.26%), a reddish-brown solid. ES-API: [M +H]⁺= 568.1.

Step 2: Tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (400 mg, 0.830 mmol) and (1-fluoro-4-(4,4,5,5 tetramethyl-1,3,2-dioxaborolane-2-yl)-5-((triisopropylsilyl)ethynyl)naphthalen-2-yl)carbamate (942.17 mg, 1.660 mmol) were dissolved in dioxane (15 mL) and water (2 mL). To this solution, methanesulfonic acid [butyl(di-1-adamantyl)phosphine](2-amino-1,1'-biphenyl-2-yl)palladium(II) (60.42 mg, 0.083 mmol) and potassium phosphate (528.51 mg, 2.490 mmol) were added. After the addition was complete, the reaction mixture was stirred under microwave conditions at 90 degrees for 1 hour. Upon completion of the reaction, water (20 mL) was added to the reaction mixture, which was then extracted with ethyl acetate (20 mL × 3). The organic layer was washed with saturated brine (30 mL), dried over anhydrous sodium sulfate, filtered, and concentrated. The crude product was purified by a flash silica gel column (ethyl acetate/petroleum ether: 0-18%) to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (600 mg, 0.676 mmol, yield: 81.49%), a white solid. ES-API: [M +H]⁺= 887.3.

Step 3: Tert-butyl (5S,5aS,6S,9R)-2-(3-((tert-butoxycarbonyl)amino)-4-fluoro-8-((triisopropylsilyl)ethynyl)naphthalen-1 -yl)-1 -fluoro-5-methyl- 12-(methylthio)-5a,6,7,8,9,10-hexahydro-5H-4-oxa-3,10a,11,13,14-pentaaza-6,9-methanonaphtho[1,8-ab]heptalene-14-carboxylate (720 mg, 0.812 mmol) was dissolved in dichloromethane (15 mL). At room temperature, m-chloroperbenzoic acid (purity 85%, 494.30 mg, 2.435 mmol) was added to the solution. After the addition was complete, the reaction mixture was stirred at room temperature for 1 hour. The reaction mixture was then quenched with saturated sodium thiosulfate solution (10 mL) and extracted with dichloromethane (15 mL × 3). The combined organic phases were washed successively with saturated sodium bicarbonate solution (15 mL) and saturated sodium chloride solution (30 mL). The organic phase was dried and concentrated to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a, 11,13,14-pentaaza-6,9-methanonaphtho[1,8-ab]heptalene-14-carboxylate (720 mg, 0.783 mmol, yield: 96.52%). Pale yellow solid. ES-API: [M +H]⁺= 919.3.

Step 4: ((6R)-6-fluoro-1-methylenetetrahydro-1H-pyrrolizin-7a(5H)-yl)methanol (150 mg, 0.876 mmol) was dissolved in tetrahydrofuran (5 mL) and cooled to 0 degrees. Under a nitrogen atmosphere, sodium hydride (60%, 94.00 mg, 2.350 mmol) was added to the solution, which was then stirred for 30 minutes. Subsequently, tert-butyl (5S,5aS,6S,9R)-2-(3-((tert-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]heptalene-14-carboxylate (720 mg, 0.783 mmol) was slowly added and stirred at 0 degrees for 2 hours. The reaction was quenched with water (20 mL) and extracted with ethyl acetate (20 mL × 3). The organic phase was washed with saturated brine (10 mL × 3), dried over anhydrous sodium sulfate, filtered, and concentrated to obtain a crude product. The crude product was purified by a flash silica gel column (methanol/dichloromethane: 0-3%) to obtain tert-butyl (5S,5aS,6S,9R)-2-(3-((tert-butoxycarbonyl)amino)-4-fluoro-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (550 mg, 0.544 mmol, yield: 69.50%). Pale yellow solid. ES-API: [M+H]⁺=1010.3.

Step 5: A mixed solution of tert-butyl (5S,5aS,6S,9R)-2-(3-((tert-butoxycarbonyl)amino)-4-fluoro-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (550 mg, 0.544 mmol), cesium fluoride (826.94 mg, 5.444 mmol) and N,N-dimethylformamide (10 mL) was stirred at room temperature for 2 hours. Water (30 mL) was added to the reaction mixture, which was then extracted with ethyl acetate (20 mL × 2). The organic phase was washed with saturated brine (30 mL × 3), dried over anhydrous sodium sulfate, filtered, and concentrated to obtain tert-butyl (5S,5aS,6S,9R)-2-(3-((tert-butoxycarbonyl)amino)-8-ethynyl-4-fluoronaphthalen-1 -yl)-1 -fluoro- 12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (400 mg, 0.468 mmol, yield: 86.04%), a pale yellow solid. ES-API: [M+H]⁺=854.2. Step 6: Tert-butyl (5S,5aS,6S,9R)-2-(3-((tert-butoxycarbonyl)amino)-8-ethynyl-4-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (400 mg, 0.468 mmol) was dissolved in ethyl acetate (5 mL) and the mixture was cooled to 0°C. A 4 M hydrochloric acid in dioxane solution (15 mL) was added, and the mixture was stirred at 0°C for 2 hours. The reaction mixture was concentrated and then redissolved in dichloromethane (2 mL), basified to pH 8 with 7 M ammonia methanol solution. The obtained solution was concentrated and the crude product was purified by preparative HPLC (formic acid method 1) to obtain 5-ethynyl-1-fluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z728, 130mg,0.199 mmol, yield: 42.45%, formate), white solid. ES-API: [M+H]⁺=654.2.

### Example 116 Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethyl-1-fluoronaphthalen-2-amine (Z729)

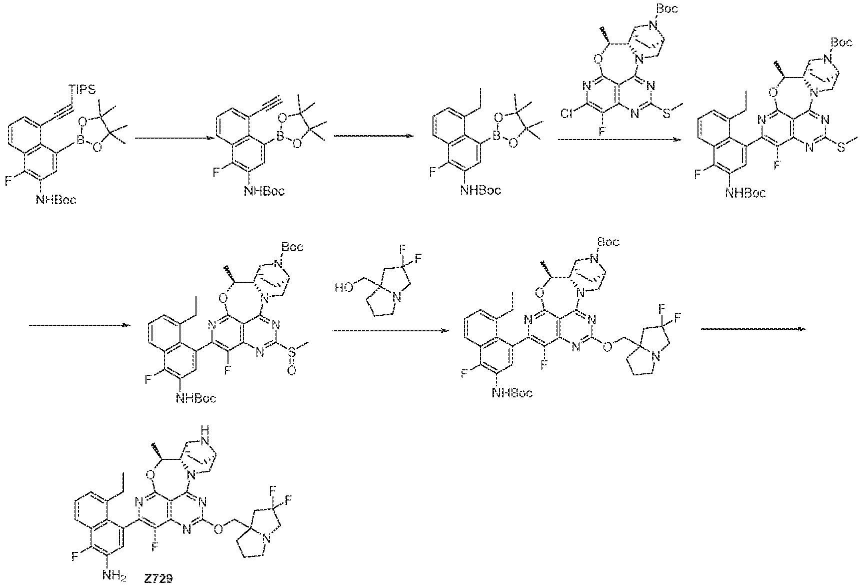

Step 1: Cesium fluoride (803 mg, 5.285 mmol) was added to N,N-dimethylformamide (5 mL) containing tert-butyl (1-fluoro-4-(4,4,5,5-tetramethyl-1,3,2-dioxaborolane-2-yl)-5-((triisopropylsilyl)ethynyl)naphthalen-2-yl)carbamate (300 mg, 0.529 mmol). The reaction mixture was stirred at room temperature for 1 hour. Upon completion of the reaction (monitored by LCMS), the reaction mixture was diluted with ethyl acetate (20 mL) and washed with water (15 mL × 2) and saturated brine (15 mL × 3), dried over anhydrous sodium sulfate, filtered, and concentrated to obtain tert-butyl (5-ethynyl-1-fluoro-4-(4,4,5,5-tetramethyl-1,3,2-dioxaborolan-2-yl)naphthalen-2-yl)carbamate (217 mg). ES-API: [M- 55]⁺= 356.1.

Step 2: Tert-butyl (5-ethynyl-1-fluoro-4-(4,4,5,5-tetramethyl-1,3,2-dioxaborolan-2-yl)naphthalen-2-yl)carbamate (217 mg, 0.528 mmol) was dissolved in methanol (10 mL), and 10% palladium on carbon (45 mg) was added to the reaction mixture. The reaction mixture was stirred at room temperature under a hydrogen atmosphere for 2 hours. Upon completion of the reaction (monitored by LCMS), the mixture was filtered through celite. The filtrate was concentrated under reduced pressure to obtain crude tert-butyl (5-ethyl-1-fluoro-4-(4,4,5,5-tetramethyl-1,3,2-dioxaborolan-2-yl)naphthalen-2-yl)carbamate (219 mg). ES-API: [M- 55]⁺= 360.2.

Step 3: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (80 mg, 0.166 mmol), tert-butyl ((5-ethyl-1-fluoro-4-(4,4,5,5-tetramethyl-1,3,2-dioxaborolane-2-yl)naphthalen-2-yl)methyl)carbamate (103 mg, 0.249 mmol), potassium phosphate (106 mg, 0.498 mmol), and palladium (II) bis[(2-amino-1,1'-biphenyl)-4-yl]butyl(di-1-adamantyl)phosphine methanesulfonate (18 mg, 0.025 mmol) were dissolved in tetrahydrofuran (5 mL) and water (1 mL). The reaction mixture was stirred at 80°C under microwave for 45 minutes. Upon completion of the reaction (monitored by LCMS), the reaction mixture was dried over anhydrous sodium sulfate and concentrated under reduced pressure. The crude product was purified by silica gel column chromatography (ethyl acetate/petroleum ether = 0 - 20%) to obtain tert-butyl (SS,SaS,6S,9R)-2-(3-((tert-butoxycarbonyl)amino)-8-ethyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (115 mg, yield: 94%). ES-API: [M+H]⁺= 735.3.

Step 4: Under an ice-water bath, tert-butyl (5S,5aS,6S,9R)-2-(3-((tert-butoxycarbonyl)amino)-8-ethyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (143 mg, 0.195 mmol) was dissolved in anhydrous dichloromethane (6 mL). M-chloroperbenzoic acid (51 mg, 0.254 mmol) was added to the solution, and the reaction mixture was stirred at 0°C for 30 minutes. Upon completion of the reaction (monitored by LCMS), the reaction mixture was diluted with dichloromethane (15 mL) and quenched with saturated sodium bisulfite solution (15 mL). The mixture was then washed with sodium bicarbonate solution (15 mL) and saturated brine (15 mL), dried over anhydrous sodium sulfate, filtered, and concentrated under reduced pressure to obtain crude tert-butyl (5S,5aS,6S,9R)-2-(3-((tert-butoxycarbonyl)amino)-8-ethyl-4-fluoronaphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (146 mg). ES-API: [M+H]⁺= 751.3. Step 5: (2,2-Difluorotetrahydro-1H-pyrrolizin-7a(5H)-yl)methanol (69 mg, 0.389 mmol) was dissolved in anhydrous tetrahydrofuran (5 mL). Under an ice-water bath, sodium hydride (23 mg, 0.583 mmol) was added, and the reaction mixture was stirred for 10 minutes. The solution of tert-butyl (5 S,5aS,6S,9R)-2-(3-((tert-butoxycarbonyl)amino)-8-ethyl-4-fluoronaphthalen-1 -yl)-1 - fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (146 mg, 0.389 mmol) in tetrahydrofuran (5 mL) was added dropwise, and the reaction mixture was stirred at 0°C for 30 minutes. Upon completion of the reaction (monitored by LCMS), the reaction mixture was diluted with ethyl acetate (10 mL), washed with saturated ammonium chloride solution (10 mL × 2) and saturated brine (10 mL × 3), dried over anhydrous sodium sulfate, filtered, and concentrated under reduced pressure to obtain crude tert-butyl (5S,5aS,6S,9R)-2-(3-((tert-butoxycarbonyl)amino)-8-ethyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (167 mg). ES-API: [M+ H]⁺= 864.3.

Step 6: Under an ice-water bath, a 4 M solution of hydrochloric acid-dioxane (10 mL) was slowly added to a methanol (1 mL) solution of tert-butyl (5S,5aS,6S,9R)-2-(3-((tert-butoxycarbonyl)amino)-8-ethyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (167 mg, 0.194 mmol). The reaction mixture was warm to room temperature and stirred for 1 hour. Upon completion of the reaction (monitored by LCMS), the reaction mixture was concentrated under reduced pressure to dryness. The residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethyl-1-fluoronaphthalen-2-amine (Z729, 13.05 mg, yield: 10 %), white solid. ES-API: [M+H]⁺= 664.0. ¹H NMR (400 MHz, CD₃OD) δ 7.85- 7.83 (m, 1 H), 7.43- 7.37 (m, 1 H), 7.19- 6.98 (m, 2 H), 5.39- 5.34 (m, 1 H), 4.58- 4.52 (m, 1 H), 4.31- 4.25 (m, 2 H), 4.10- 4.05 (m, 1 H), 3.70- 3.69 (m, 1 H), 3.63-3.59 (m, 1 H), 3.49- 3.39 (m, 1 H), 3.21- 3.09 (m, 3 H), 2.88- 2.82 (m, 1 H), 2.61- 2.47 (m, 2 H), 2.39- 2.17 (m, 3 H), 2.07- 1.77 (m, 7 H), 1.57- 1.54 (m, 3 H), 1.01 (t, J1= 7.6 Hz, J2= 15.2 Hz, 1 H), 0.88 (t, J1= 7.6 Hz, J2= 15.2 Hz, 2 H).

### Example 117 Synthesis of 5-ethynyl-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oza-3,10a,11,13,14-pentaaza-6,9-methanonaphtho[1,8-ab]heptalene-2-yl)naphthalen-2-amine (Z730)

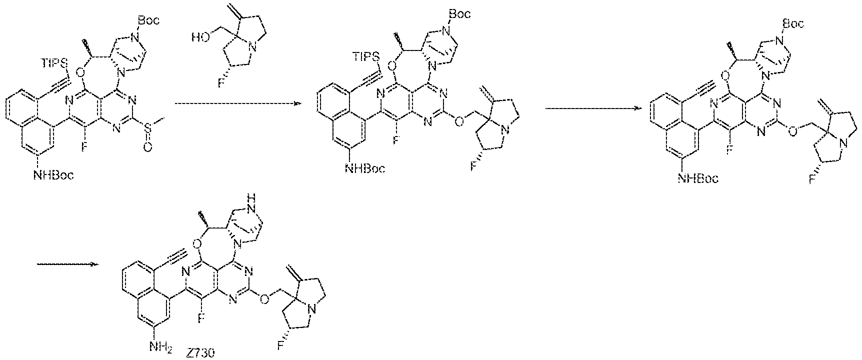

Step 1: 60% sodium hydride (25 mg, 0.62 mmol) and tetrahydrofuran (5 mL) were added to a round-bottom flask. The mixture was cooled to 0 degrees, and ((6R)-6-fluoro-1-methylenetetrahydro-1H-pyrrolizin-7a(5H)-yl)methanol (25 mg, 0.15 mmol) was added to the reaction mixture. The reaction was proceeded at room temperature for 5 minutes. Tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (110 mg, 0.12 mmol) was added to the reaction mixture and stirred at room temperature for 30 minutes. Upon completion of the reaction (monitored by LCMS), the reaction mixture was quenched with saturated ammonium chloride solution (30 mL). The mixture was extracted with ethyl acetate (20 mL × 2). The organic phase was washed with saturated brine (30 mL), dried over anhydrous sodium sulfate, filtered, and concentrated. The crude product was purified by flash silica gel column chromatography (methanol/dichloromethane: 0-3%) to obtain tert-butyl (SS,SaS,6S,9R)-2-(3-((tert-butoxycarbonyl)amino)-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (75 mg, yield: 60%). ES-API: [M+H]⁺=992.3.

Step 2: Tert-butyl (5S,5aS,6S,9R)-2-(3-((tert-butoxycarbonyl)amino)-8-((triisopropylsilyl)ethynyl)naphthalen-1 -yl)-1 -fluoro- 12-(((6R)-6-fluoro-1 -methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (75 mg, 0.076 mmol), cesium fluoride (115 mg, 0.76 mmol), and N,N-dimethylformamide (3 mL) were added to a round-bottom flask. The reaction mixture was stirred at room temperature for 1 hour. Water (30 mL) was added to the reaction mixture, followed by extraction with ethyl acetate (30 mL × 3). The organic phase was washed with saturated brine (30 mL × 2), dried over anhydrous sodium sulfate, filtered, and concentrated to obtain crude tert-butyl (5S,5aS,6S,9R)-2-(3-((tert-butoxycarbonyl)amino)-8-ethynylnaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, crude). ES-API: [M+H]⁺=836.0.

Step 3: Crude tert-butyl (SS,SaS,6S,9R)-2-(3-((tert-butoxycarbonyl)amino)-8-ethynylnaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (60 mg, crude) was dissolved in ethyl acetate (1 mL). The solution was cooled to 0 degrees, and 4M hydrochloric acid/dioxane solution (1 mL, 4 mmol) was added. The reaction mixture was stirred at room temperature for 1 hour. The reaction mixture was concentrated to dryness at 40 degrees, and the crude product was purified by preparative HPLC (formic acid method 1) to obtain 5-ethynyl-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z730, 15mg, yield: 28%, formate). ES-API: [M+H]⁺=636.3. ¹HNMR(400 MHz, CD₃OD): 8.41(s, 2H, Formic acid proton), 7.71-7.67(m, 1H), 7.42-7.27(m, 2H), 7.15-7.01(m, 2H), 5.51-5.20(m, 3H), 4.90-4.86(m, 1H), 4.61-4.45(m, 2H), 4.26-4.18(m, 2H), 3.93-3.79(m, 3H), 3.63-3.48(m, 1H), 3.31-2.95(m, 3H), 2.49-2.26(m, 2H), 2.20-2.10(m, 1H), 1.98-1.85(m, 6H), 1.60-1.57(m, 3H).

### Example 118 Synthesis of 1,5,6-trifluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z731)

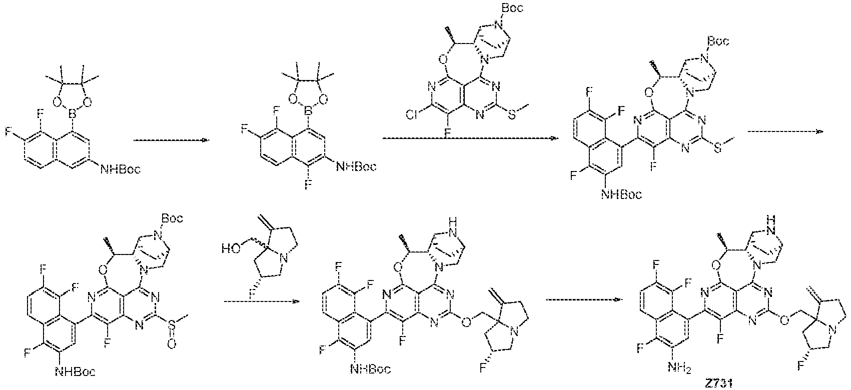

Step 1: 1-Chloromethyl-4-fluoro-1,4-diazabicyclo[2.2.2]octane bis(tetrafluoroborate) salt (136 mg, 0.385 mmol) was added to a solution of tert-butyl (5,6-difluoro-4-(4,4,5,5-tetramethyl-1,3,2-dioxaborolan-2-yl)naphthalen-2-yl)carbamate (120 mg, 0.296 mmol) in acetonitrile (50 mL). The reaction mixture was stirred at room temperature overnight. Upon completion of the reaction (monitored by LCMS), the solvent was removed under reduced pressure. The residue was purified by automated flash chromatography through silica gel (ethyl acetate/petroleum ether = 0 - 10%) to obtain tert-butyl (1,5,6-trifluoro-4-(4,4,5,5-tetramethyl-1,3,2-dioxaborolan-2-yl)naphthalen-2-yl)carbamate (43 mg, yield: 34%). ES-API: [M+ H]⁺= 424.0.

Step 2: Under nitrogen protection, tert-butyl (5S,5aS,6S,9R)-2-chloro-1-fluoro-5-methyl-12-(methylthio)-5a,6,7,8,9,10-hexahydro-5H-4-oxa-3,10a, 11,13,14-pentaaza-6,9-methanonaphtho[1,8-ab]heptalene-14-carboxylate (48 mg, 0.100 mmol), tert-butyl (1,5,6-trifluoro-4-(4,4,5,5-tetramethyl-1,3,2-dioxaborolan-2-yl)naphthalen-2-yl)carbamate (43 mg, 0.102 mmol), potassium phosphate (64 mg, 0.300 mmol), and palladium (II) bis[(2-amino-1,1'-biphenyl-2-yl)butyl(di-1-adamantyl)phosphine] methane sulfonate (11 mg, 0.015 mmol) were dissolved in tetrahydrofuran (2 mL) and water (0.4 mL). The reaction mixture was stirred at 80°C under microwave for 45 minutes. Upon completion of the reaction (monitored by LCMS), the reaction mixture was dried over anhydrous sodium sulfate and concentrated under reduced pressure. The crude product was purified by thin-layer chromatography through silica gel (ethyl acetate/petroleum ether = 1:3) to obtain tert-butyl (5S,5aS,6S,9R)-2-(3-((tert-butoxycarbonyl)amino)-4,7,8-trifluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (66 mg, yield: 89%). ES-API: [M+H]⁺= 743.2.

Step 3: Under an ice water bath, tert-butyl (5S,5aS,6S,9R)-2-(3-((tert-butoxycarbonyl)amino)-4,7,8-trifluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]heptalene-14-carboxylate (66 mg, 0.089 mmol) was dissolved in anhydrous dichloromethane (6 mL). M-Chloroperbenzoic acid (24 mg, 0.116 mmol) was added to the solution, and the reaction mixture was stirred at 0°C for 30 minutes. Upon completion of the reaction (monitored by LCMS), dichloromethane (15 mL) was added to dilute the reaction mixture, which was then quenched with saturated sodium bisulfite solution (15 mL). The mixture was subsequently washed with saturated sodium bicarbonate solution (15 mL) and saturated brine (15 mL). The organic phase was dried over anhydrous sodium sulfate, filtered, and concentrated to obtain crude tert-butyl (SS,SaS,6S,9R)-2-(3-((tert-butoxycarbonyl)amino)-4,7,8 trifluoronaphthalen-1-yl)-1-fluoro-5-methyl-12-(methylsulfinyl)-Sa,6,7,8,9,10-hexahydro-5H-4-oxa-3,10a,11,13,14-pentaaza-6,9-methanonaphtho[1,8-ab]heptalene-14-carboxylate (76 mg). ES-API: [M+ H]⁺= 759.2.

Step 4: ((6R)-6-fluoro-1-methylenetetrahydro-1H-pyrrolizin-7a(5H)-yl)methanol (34.3 mg, 0.200 mmol) was dissolved in anhydrous tetrahydrofuran (5 mL). Under an ice water bath, sodium hydride (23 mg, 0.583 mmol) was added, and the mixture reacted for 10 minutes under the ice water bath. A solution of tert-butyl (SS,SaS,6S,9R)-2-(3-((tert-butoxycarbonyl)amino)-4,7,8-trifluoronaphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (76 mg, 0.100 mmol) in tetrahydrofuran (3 mL) was added dropwise to the reaction mixture at 0°C, and the reaction mixture was stirred for 30 minutes. Upon completion of the reaction (monitored by LCMS), the mixture was diluted with ethyl acetate (10 mL) and washed with saturated ammonium chloride solution (10 mL × 2) and saturated brine (10 mL × 3). The organic phase was dried over anhydrous sodium sulfate, filtered, and concentrated under reduced pressure to obtain crude tert-butyl (1,5,6-trifluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-yl)carbamate (86 mg). ES-API: [M+ H]⁺= 866.3.

Step 5: Under an ice water bath, 4M hydrochloric acid-dioxane solution (10 mL) was slowly added to a methanol (1 mL) solution of tert-butyl (1,5,6-trifluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-yl)carbamate (86 mg, 0.099 mmol). The reaction mixture was stirred at room temperature for 1 hour. Upon completion of the reaction (monitored by LCMS), the reaction mixture was concentrated under reduced pressure until dry. The residue was purified by preparative HPLC (ammonium bicarbonate method 2) to obtain 1,5,6-trifluoro-4-((SS,SaS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z731, 5.32 mg, yield: 8 %), white solid. ES-API: [M+H]⁺= 666.0.

### Example 119 Synthesis of 5-ethynyl-4-((5S,5aS,6S,9R)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z732)

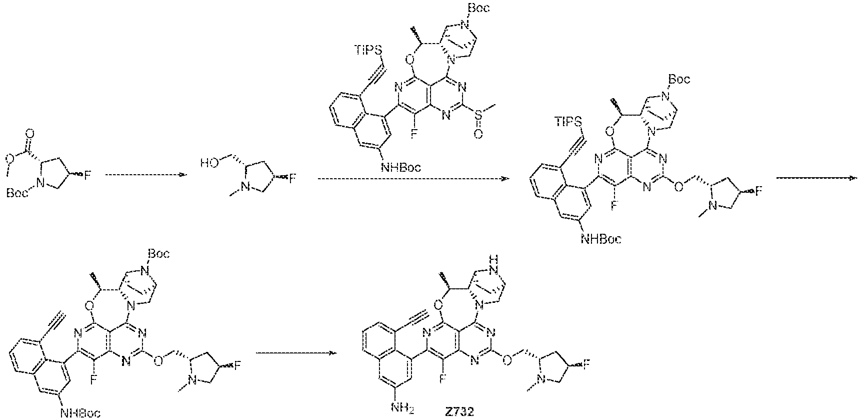

Step 1: Under nitrogen protection, 1-(tert-butyl) 2-methyl (2S,4R)-4-fluoropyrrolidine-1,2-dicarboxylate (1046 mg, 4.23 mmol) was dissolved in anhydrous tetrahydrofuran (25 mL). The mixture was placed in an ice-water bath, and a solution of lithium aluminum hydride (12.7 mL, 12.69 mmol, 1 M in tetrahydrofuran) was slowly added dropwise to the above solution. After the addition was complete, the mixture was stir overnight at room temperature. Upon completion of the reaction (monitored by LCMS), sodium sulfate decahydrate was added to quench the reaction. The mixture was stirred at room temperature for 0.5 hours, filtered, and the filtrate was concentrated under reduced pressure to obtain crude ((2S, 4R)-4-fluoro-1-methylpyrrolidin-2-yl)methanol (563 mg). ES-API: [M+H]⁺= 134.2.

Step 2: ((2S, 4R)-4-fluoro-1-methylpyrrolidin-2-yl)methanol (28 mg, 0.208 mmol) was dissolved in anhydrous tetrahydrofuran (5 mL). Under an ice water bath, sodium hydride (12.5 mg, 0.312 mmol) was added and the reaction was proceeded for 10 minutes under the ice water bath. A solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (92 mg, 0.104 mmol) in tetrahydrofuran (5 mL) was added dropwise to the reaction mixture, and the reaction was proceeded at 0°C for 30 minutes. Upon completion of the reaction (monitored by LCMS), the reaction mixture was diluted with ethyl acetate (10 mL) and washed with saturated ammonium chloride solution (10 mL × 2) and saturated brine (10 mL × 3). The organic phase was dried over anhydrous sodium sulfate, filtered, and concentrated under reduced pressure to obtain crude tert-butyl (SS,SaS,6S,9R)-2-(3-((tert-butoxycarbonyl)amino)-8-((triisopropylsilyl)ethynyl)naphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (99 mg). ES-API: [M+ H]⁺= 954.4.

Step 3: Cesium fluoride (158 mg, 1.037 mmol) was added to a solution of tert-butyl (SS,SaS,6S,9R)-2-(3-((tert-butoxycarbonyl)amino)-8-((triisopropylsilyl)ethynyl)naphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (99 mg, 0.104 mmol) in N,N-dimethylformamide (5 mL). The reaction mixture was stirred at room temperature for 1 hour. Upon completion of the reaction (monitored by LCMS), the reaction mixture was diluted with ethyl acetate (20 mL) and washed with water (15 mL × 2) and saturated brine (15 mL × 3). The organic phase was dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-1-fluoro-12-(((2S,4R)-4-fluoro-1-methylpyrrolidin-2-yl)methoxy)-5-methyl-Sa,6,7,8,9,10-hexahydro-SH-4-oxa-3,10a,11,13,14-pentaaza-6,9-methanonaphtho[1,8-ab]heptalene-14-carboxylate (82 mg). ES-API: [M+ H]⁺= 798.1.

Step 4: Under an ice water bath, a 4 M hydrochloric acid-dioxane solution (10 mL) was slowly added to a methanol (1 mL) solution of tert-butyl (5S,5aS,6S,9R)-2-(3-((tert-butoxycarbonyl)amino)-8-ethynylnaphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate (82 mg, 0.103 mmol). The reaction mixture was warm to room temperature and stirred for 1 hour. Upon completion of the reaction (monitored by LCMS), the reaction mixture was concentrated under reduced pressure to dryness, and the residue was purified by preparative HPLC (ammonium bicarbonate method 2) to obtain 5-ethynyl-4-((5S,5aS,6S,9R)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]heptalene-2-yl)naphthalen-2-amine (Z732, 7.59 mg, yield: 12 %), white solid. ES-API: [M+H]⁺= 598.0. H NMR (400 MHz, CD₃OD) δ 7.69- 7.66 (m, 1 H), 7.41- 7.35 (m, 1 H), 7.32- 7.26 (m, 1 H), 7.14- 7.00 (m, 2 H), 5.41- 5.35 (m, 1 H), 5.27- 5.10 (m, 1 H), 4.54- 4.45 (m, 3 H), 4.10- 4.07 (m, 1 H), 3.70- 3.69 (m, 1 H), 3.62- 3.60 (m, 1 H), 3.57- 3.46 (m, 1 H), 3.21- 3.13 (m, 2 H), 2.72- 2.61 (m, 1 H), 2.56 (m, 3 H), 2.35- 2.25 (m, 1 H), 2.09- 1.77 (m, 6 H), 1.57 (t, J1= 6.8 Hz, J2= 13.2 Hz, 3 H).

### Example 120 Synthesis of 4-((5S,5aS,6S,9R)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z733)

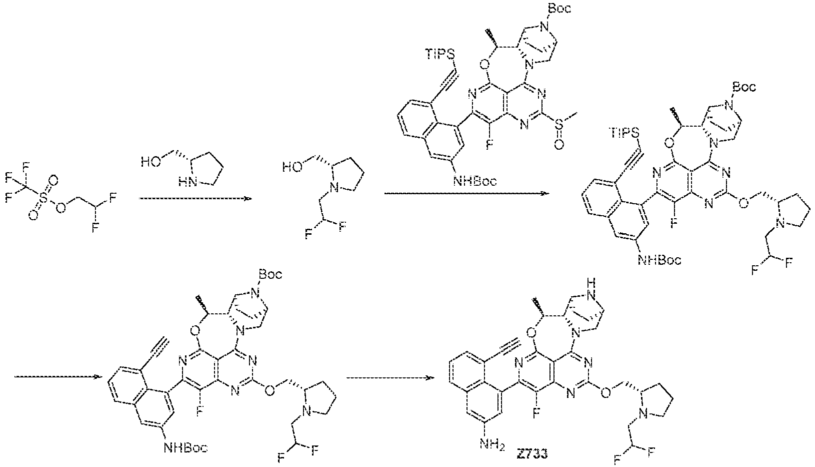

Step 1: (S)-pyrrolidin-2-ylmethanol (505 mg, 4.993 mmol) was dissolved in acetonitrile (10 mL) and cooled in an ice-water bath. Then, 2,2-difluoroethyl trifluoromethanesulfonate (1.1 g, 5.138 mmol) and potassium carbonate (760 mg, 5.499 mmol) were added to the reaction mixture. After the addition, the reaction mixture was stirred at 0 °C for 1 hour, then warmed to room temperature and stirred for 3 hours. Upon completion of the reaction (monitored by LCMS), 100 mL of water was added to quench the reaction. The mixture was extracted with ethyl acetate (30 mL × 3), the combined organic phases were dried over anhydrous sodium sulfate, filtered, and concentrated under reduced pressure to obtain the crude product (S)-(1-(2,2-difluoroethyl)pyrrolidin-2-yl)methanol (600 mg, yield: 73%). ES-API: [M+H]⁺= 166.1.

Step 2: (S)-(1-(2,2-difluoroethyl)pyrrolidin-2-yl)methanol (34 mg, 0.208 mmol) was dissolved in anhydrous tetrahydrofuran (5 mL) and cooled in an ice-water bath. Sodium hydride (12.5 mg, 0.312 mmol) was added, and the mixture reacted for 10 minutes under the ice-water bath. A tetrahydrofuran (5 mL) solution of ((2S, 4R)-4-fluoro-1-methylpyrrolidin-2-yl)methanol and tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (92 mg, 0.104 mmol) was added dropwise to the reaction mixture and reacted for 30 minutes at 0 °C. Upon completion of the reaction (monitored by LCMS), the reaction mixture was diluted with ethyl acetate (10 mL) and washed with saturated ammonium chloride solution (10 mL × 2) and saturated brine (10 mL × 3). The organic phase was dried over anhydrous sodium sulfate, filtered, and concentrated under reduced pressure to obtain the crude product tert-butyl (5S,5aS,6S,9R)-2-(3-((tert-butoxycarbonyl)amino)-8-((triisopropylsilyl)ethynyl)naphthalen-1-yl)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (102 mg). ES-API: [M+ H]⁺= 986.4.

Step 3: Cesium fluoride (157 mg, 1.034 mmol) was added to a solution of tert-butyl (SS,SaS,6S,9R)-2-(3-((tert-butoxycarbonyl)amino)-8-((triisopropylsilyl)ethynyl)naphthalen-1-yl)-12-(((S)-1-(2,2-difluoroethyl)pyrrolidin-2-yl)methoxy)-1-fluoro-5-methyl-Sa,6,7,8,9,10-hexahydro-SH-4-oxa-3,10a,11,13,14-pentaaza-6,9-methanonaphtho[1,8-ab]heptalene-14-carboxylate (102 mg, 0.103 mmol) in N,N-dimethylformamide (5 mL). The reaction mixture was stirred at room temperature for 1 hour. Upon completion of the reaction (monitored by LCMS), the mixture was diluted with ethyl acetate (20 mL), washed with water (15 mL × 2), and saturated brine (15 mL × 3), then dried over anhydrous sodium sulfate, filtered, and concentrated to obtain tert-butyl (5S,5aS,6S,9R)-2-(3-((tert-butaxycarbonyl)amino)-8-ethynylnaphthalen-1-yl)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (85 mg). ES-API: [M+ H]⁺= 830.0.

Step 4: Under an ice-water bath, 4 M hydrochloric acid-dioxane solution (10 mL) was slowly added to a methanol (1 mL) solution of tert-butyl (SS,SaS,6S,9R)-2-(3-((tert-butoxycarbonyl)amino)-8-ediynylnaphthalen-1-yl)-12-(((S)-1-(2,2-difluoroethyl)pyrrolidin-2-yl)medioxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (85 mg, 0.102 mmol). The reaction mixture was warmed to room temperature and stirred for 1 hour. Upon completion of the reaction (monitored by LCMS), the mixture was concentrated under reduced pressure to dryness, and the residue was purified by preparative HPLC (ammonium bicarbonate method 2) to obtain 4-((SS,SaS,6S,9R)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethynylnaphthalen-2-amine (Z733, 12.01 mg, yield: 19 %), white solid. ES-API: [M+H]⁺= 630.1. ¹H NMR(400 MHz, CD₃OD) δ 7.70- 7.66 (m, 1 H), 7.42- 7.27 (m, 2 H), 7.14 (d, J= 2.4 Hz, 1 H), 7.11- 7.00 (m, 1 H), 6.08- 5.78 (m, 1 H), 5.52- 5.48 (m, 1 H), 4.64- 4.57 (m, 1 H), 4.48- 4.43 (m, 1 H), 4.37- 4.32 (m, 1 H), 4.22-4.19 (m, 1 H), 4.06- 4.05 (m, 1 H), 3.96- 3.94 (m, 1 H), 3.48- 3.36 (m, 2 H), 3.26- 3.21 (m, 1 H), 3.13- 3.07 (m, 1 H), 2.91- 2.83 (m, 1 H), 2.54- 2.47 (m, 1 H), 2.35- 2.19 (m, 1 H), 2.07- 1.70 (m, 8 H), 1.58 (t, J1= 6.8 Hz, J2= 13.2 Hz, 3 H).

### Example 121 Synthesis of 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z734) and 4-((5S,5aS,6S,9R)-12-(((S)-2,2-difluorotetrahydro-1H-pyrrolizin-7a(5H)-yl)methoxy)-1-fluoro-S-methyl-Sa,6,7,8,9,10-hexahydro-5H-4-oxa-3,10a, 11,13,14-pentaaza-6,9-methanonaphtho[1,8-ab]heptalene-2-yl)-5-ethynylnaphthalen-2-amine (Z735)

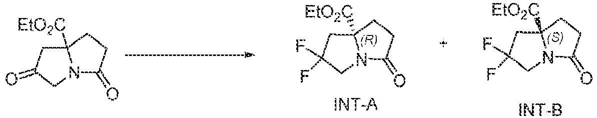

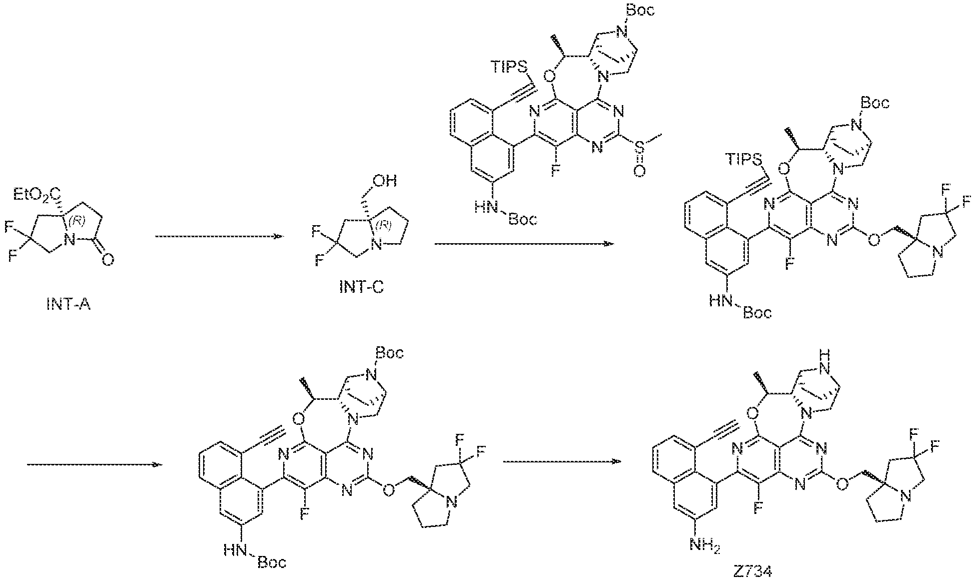

Step 1: Under an ice-water bath, bis(ethylamino)sulfur trifluoride (1.9 mL, 14.2 mmol) was slowly added to a solution of ethyl 2,5-dioxotetrahydro-1H-pyrrolizine-7a(5H)-carboxylate (1 g, 4.73 mmol) in dichloromethane (15 mL). After the ice water bath was removed, the reaction mixture was stirred at room temperature for 16 hours. Under an ice-water bath, the reaction mixture was quenched with saturated sodium bicarbonate solution. The mixture was then extracted with ethyl acetate, and the organic layer was dried over anhydrous sodium sulfate. After filtration, the filtrate was concentrated, and the residue was purified by a flash silica gel column (ethyl acetate/petroleum ether: 0-40%) to obtain the racemate (800 mg). The racemate was subjected to chiral separation (chromatographic column: Daicel CHIRALPAK^{®} IG 250*4.6 mm, 5µm; mobile phase: ethanol: n-hexane = 30:70; flow rate: 1 mL/min; column temperature: 30°C) to obtain two isomers. One of the isomers, designated as ethyl (R)-2,2-difluoro-5-oxotetrahydro-1H-pyrrolizine-7a(5H)-carboxylate (INT-A, 270 mg, peak 1, retention time = 7.419 min), a white solid.ES-API: [M+H]⁺=234.1. The other isomer, designated as ethyl (S)-2,2-difluoro-5-oxotetrahydro-1H-pyrrolizine-7a(5H)-carboxylate (INT-B, 220 mg, peak 2, retention time = 12.34 min), a white solid. ES-API:[M+H]⁺=234.1.

Step 2: Under an ice-water bath, a tetrahydrofuran solution of 1M lithium aluminium hydride (4.63 mL, 4.63 mmol) was slowly added to a tetrahydrofuran solution of ethyl (R)-2,2-difluoro-5-oxotetrahydro-1H-pyrrolizine-7a(5H)-carboxylate (INT-A, 270 mg, 1.16 mmol). After removing the ice water bath, the reaction mixture was stirred at 65 degrees Celsius for 5 hours. The reaction mixture was cooled to 0 degrees Celsius and 15% sodium hydroxide solution (0.1 mL) was added dropwise. The mixture was filtered through diatomaceous earth and the filtrate was concentrated to obtain (R)-(2,2-difluorotetrahydro-1H-pyrrolizin-7a(5H)-yl)methanol (130 mg, yield 63%). ES-API:[M+Na]⁺=178.0.

Step 3: (R)-(2,2-difluorotetrahydro-1H-pyrrolizin-7a(5H)-yl)methanol (50 mg, 0.28 mmol) was dissolved in anhydrous tetrahydrofuran (6 mL). Under an ice-water bath, sodium hydride (24 mg, 0.59 mmol) was added, and the reaction was proceeded at room temperature for 5 minutes. A tetrahydrofuran (2 mL) solution of tert-butyl (5S,5aS,6S,9R)-2-(3-((tert-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, 0.17 mmol) was added dropwise to the reaction mixture, which then reacted at room temperature for 30 minutes. The reaction mixture was quenched with water (30 mL), extracted with ethyl acetate (30 mL × 2), washed with saturated brine (60 mL), dried over anhydrous sodium sulfate, filtered, and concentrated. The crude product was purified by a flash silica gel column (ethyl acetate/petroleum ether: 0-30%) to obtain tert-butyl (5S,5aS,6S,9R)-2-(3-((tert-butoxycarbonyl)amino)-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (100 mg, yield: 59%). ES-API: [M+H]⁺= 998.2.

Step 4: Cesium fluoride (152 mg, 1 mmol) was added to a solution of N,N-dimethylformamide (3 mL) containing tert-butyl (SS,SaS,6S,9R)-2-(3-((tert-butoxycarbonyl)amino)-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (100 mg, 0.1 mmol). The reaction mixture was stirred at room temperature for 1 hour. Upon completion of the reaction (monitored by LCMS), the reaction mixture was diluted with ethyl acetate (30 mL), washed with saturated brine (30 mL × 3), dried over anhydrous sodium sulfate, filtered, and concentrated to obtain tert-butyl (SS,SaS,6S,9R)-2-(3-((tert-butoxycarbonyl)amino)-8-ethynyinaphthalen-1-yl)-12-(((R)-2,2-difluorotetrahydro-1H-pyrrolizin-7a(5H)-yl)methoxy)-1 -fluoro-5-methyl-5a,6,7,8,9,10-hexahydro-5H-4-oxa-3,10a,11,13,14-pentaaza-6,9-methanonaphtho[1,8-ab]heptalene-14-carboxylate (80 mg, crude product). ES-API: [M+H]⁺= 842.0.

Step 5: Under an ice-water bath, 4 M hydrochloric acid-dioxane solution (3 mL) was slowly added to a solution of tert-butyl (SS,SaS,6S,9R)-2-(3-((tert-butoxycarbonyl)amino)-8-ethynylnaphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (80 mg, crude product) in acetonitrile (2 mL). The reaction mixture was stirred at room temperature for 1 hour. Upon completion of the reaction (monitored by LCMS), the reaction mixture was concentrated under reduced pressure to dryness, and the residue was purified by preparative HPLC (ammonium bicarbonate method 1) to obtain 4-((5S,5aS,6S,9R)-12-(((R)-2,2-difluorotetrahydro-1H-pyrrolizin-7a(SH)-yl)methoxy)-1-fluoro-5-methyl-Sa,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z734, 15 mg, yield: 24%). ES-API: [M+H]⁺= 642.2.

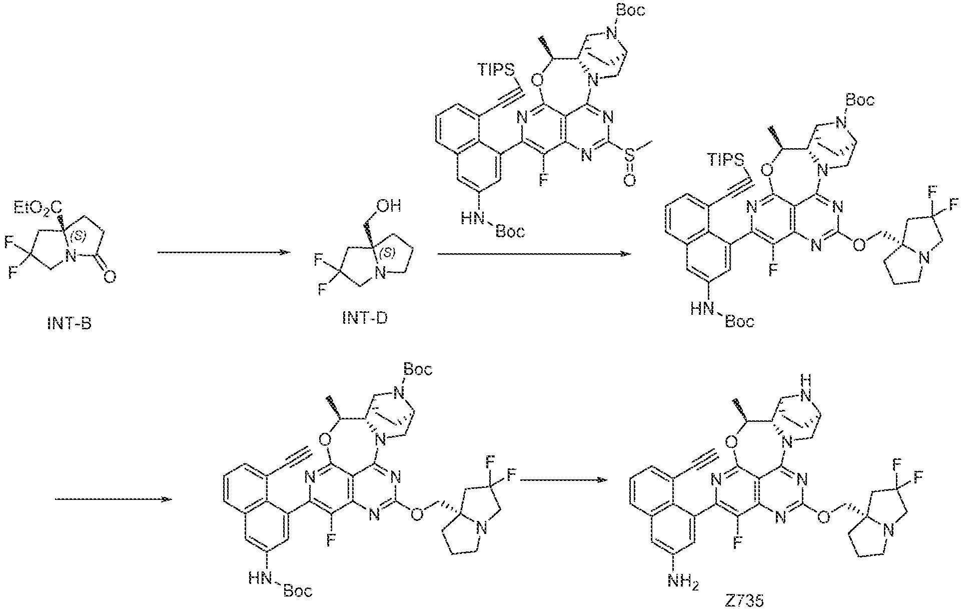

Step 1: Under an ice-water bath, a 1M solution of lithium aluminum hydride in tetrahydrofuran (3.77 mL, 3.77 mmol) was slowly added to a solution of ethyl (S)-2,2-difluoro-5-oxotetrahydro-1H-pyrrolizine-7a(5H)-carboxylate (INT-B, 220 mg, 0.94 mmol) in tetrahydrofuran (5 mL). The ice water bath was removed, and the reaction mixture was stirred at 65 degrees Celsius for 5 hours. The reaction mixture was cooled to 0 degrees Celsius and a 15% sodium hydroxide solution (0.1 mL) was added dropwise. The mixture was filtered through diatomaceous earth and the filtrate was concentrated to obtain (S)-(2,2-difluorotetrahydro-1H-pyrrolizin-7a(5H)-yl)methanol (100 mg, yield: 59%). ES-API:[M+Na]⁺=178.0.

Step 2: (S)-(2,2-difluorotetrahydro-1H-pyrrolizin-7a(5H)-yl)methanol (50 mg, 0.28 mmol) was dissolved in anhydrous tetrahydrofuran (6 mL). Under an ice-water bath, sodium hydride (24 mg, 0.59 mmol) was added and the reaction was proceeded at room temperature for 5 minutes. A tetrahydrofuran (2 mL) solution of tert-butyl (5S,5aS,6S,9R)-2-(3-((tert-butoxycarbonyl)amino)-8-((triisopropylsilyl)ethynyl)naphthalen-1 -yl)-1 -fluoro-5-methyl- 12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, 0.17 mmol) was added dropwise to the reaction mixture, and the mixture was stirred at room temperature for 30 minutes. The reaction was quenched with water (30 mL), extracted with ethyl acetate (30 mL × 2), washed with saturated brine (60 mL), dried over anhydrous sodium sulfate, filtered, and concentrated. The crude product was purified by a flash silica gel column (ethyl acetate/petroleum ether: 0-30%) to obtain tert-butyl (5S,5aS,6S,9R)-2-(3-((tert-butoxycarbonyl)amino)-8-((triisopropylsilyl)ethynyl)naphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (40 mg, yield: 23%). ES-API: [M+H]⁺= 998.2.

Step 3: Cesium fluoride (61 mg, 0.4 mmol) was added to a solution of N,N-dimethylformamide (2 mL) containing tert-butyl (SS,SaS,6S,9R)-2-(3-((tert-butoxycarbonyl)amino)-8-((triisopropylsilyl)ethynyl)naphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (40 mg, 0.04 mmol). The reaction mixture was stirred at room temperature for 1 hour. Upon completion of the reaction (monitored by LCMS), the reaction mixture was diluted with ethyl acetate (30 mL), washed with saturated brine (30 mL × 3), dried over anhydrous sodium sulfate, filtered, and concentrated to obtain tert-butyl (5S,5aS,6S,9R)-2-(3-((tert-butoxycarbonyl)amino)-8-ethynylnaphthalen-1-yl)-12-(((S)-2,2-difluorotetrahydro-1H-pyrrolizin-7a(5H)-yl)methoxy)-1 -fluoro-5-methyl-5a,6,7,8,9,10-hexahydro-5H-4-oxa-3,10a,11,13,14-pentaaza-6,9-methanonaphtho[1,8-ab]heptalene-14-carboxylate (30 mg, crude product). ES-API: [M+H]⁺= 842.0.

Step 4: Under an ice-water bath, 4 M hydrochloric acid-dioxane solution (2 mL) was slowly added to a solution of tert-butyl (5S,5aS,6S,9R)-2-(3-((tert-butoxycarbonyl)amino)-8-ethynylnaphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate (30 mg, crude product) in acetonitrile (2 mL). The reaction mixture was stirred at room temperature for 1 hour. Upon completion of the reaction (monitored by LCMS), the reaction mixture was concentrated under reduced pressure to dryness, and the residue was purified by preparative HPLC (ammonium bicarbonate method 1) to obtain 4-((5S,5aS,6S,9R)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-2-yl)-5-ethynylnaphthalen-2-amine (Z735, 5 mg, yield: 21%). ES-API: [M+H]⁺= 642.2. ¹HNMR(400MHz,CD₃OD):7.69-7.65(m,1H),7.41-7.26(m,2H),7.13-6.99(m,2H),5.40-5.36(m,1H),4.57-4.48(m,1H),4.30-4.22(m,2H),4.09-4.06(m,1H),3.71-3.61(m,2H),3.48-3.38(m,1H),3.21-2.99(m,4H),2.90-2.75(m,1H),2.65-2.50(m,1H),2.38-2.28 (m,1H),2.20-1.75(m,8H), 1.58-1.55(m,3H).

### Example 122 Synthesis of (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z648)

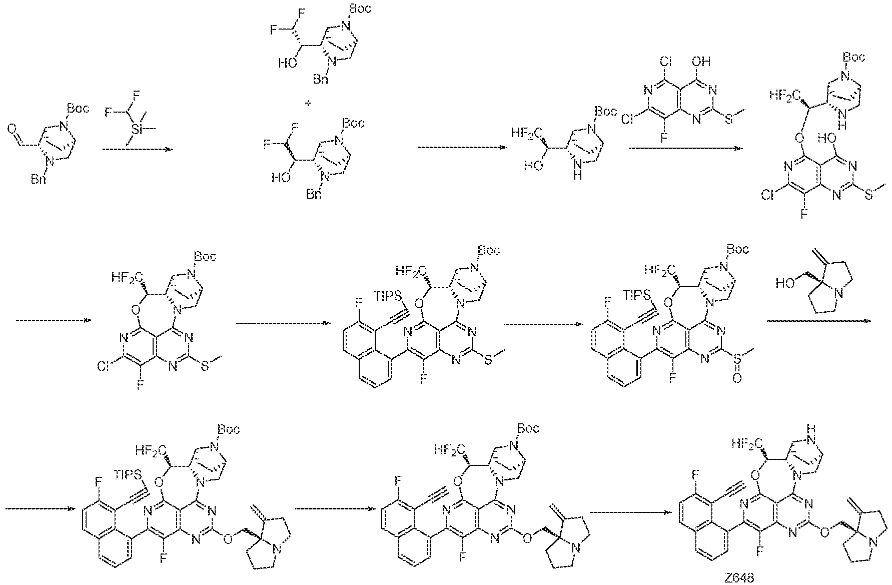

Step 1: Tert-butyl (1S,2S,5R)-3-benzyl-2-formyl-3,8-diazabicyclo[3.2.1]octane-8-carboxylate (1.15 g, 3.48 mmol) and (difluoromethyl)trimethylsilane (1.08 g, 8.70 mmol) were dissolved in N,N-dimethylformamide (10 mL). At 0°C, cesium fluoride (159 mg, 1.04 mmol) was added, and the reaction mixture was stirred at room temperature for 18 hours. A 1.0 M solution of tetrabutylammonium fluoride in tetrahydrofuran (3.48 mL, 3.48 mmol) was added, and the reaction mixture continued to stir at room temperature for 1 hour. The reaction mixture was quenched with water (10 mL) and extracted with ethyl acetate (40 mL). The organic phase was washed with saturated brine (15 mL × 3), dried over anhydrous sodium sulfate, and concentrated. The crude product was purified by a flash silica gel column (tetrahydrofuran/petroleum ether: 0-10%) to obtain tert-butyl (1S,2S,5R)-3-benzyl-2-((S)-2,2-difluoro-1-hydroxyethyl)-3,8-diazabicyclo[3.2.1]octane-8-carboxylate (isomer 1, 470 mg, yield: 35.3%), a colorless liquid. ES-API: [M+H]⁺= 383.0. (Column type: X-Bridge C18, 4.6*50mm,3.5um; Mobile phase: A: 10mmol ammonium bicarbonate aqueous solution; B: preparative grade acetonitrile; Flow rate: 1.7ml/min; B%=10%-95%; Column temperature: 40°C; Retention time: 1.74min), and tert-butyl (1S,2S,5R)-3-benzyl-2-((R)-2,2-difluoro-1-hydroxyethyl)-3,8-diazabicyclo[3.2.1]octane-8-carboxylate (isomer 2, 700 mg, yield: 52.6%), a colorless liquid. ES-API: [M+H]⁺= 383.0. (Column type: X-Bridge C18, 4.6*50mm,3.5um; Mobile phase: A: 10mmol ammonium bicarbonate aqueous solution; B: preparative grade acetonitrile; Flow rate: 1.7ml/min; B%=10%-95%; Column temperature: 40°C; Retention time: 1.71min).

Step 2: Tert-butyl (1S,2S,SR)-3-benzyl-2-((R)-2,2-difluoro-1-hydroxyethyl)-3,8-diazabicyclo[3.2.1]octane-8-carboxylate (760 mg, 1.99 mmol) was dissolved in methanol (15 mL) and 7M ammonium hydroxide solution (1 mL), 10% palladium on carbon (150 mg) was added, and the reaction mixture was stirred under a hydrogen atmosphere at room temperature for 18 hours. The reaction mixture was filtered through diatomaceous earth, the filter cake was washed with methanol, and the filtrate was concentrated to obtain the target product tert-butyl (1S,2S,5R)-2-((R)-2,2-difluoro-1-hydroxyethyl)-3,8-diazabicyclo[3.2.1]octane-8-carboxylate (580 mg, yield: 99.8%), a colorless liquid. ES-API: [M+H]⁺= 293.0.

Step 3: Tert-butyl (1S,2S,5R)-2-((R)-2,2-difluoro-1-hydroxyethyl)-3,8-diazabicyclo[3.2.1]octane-8-carboxylate (550 mg, 1.88 mmol) was dissolved in tetrahydrofuran (30 mL). At 0 °C, 60% sodium hydride (376 mg, 9.41 mmol) was added and the reaction mixture was stirred at 0 °C for 30 minutes. Then, 5,7-dichloro-8-fluoro-2-(methylthio)pyrido[4,3-d]pyrimidin-4-ol (632 mg, 2.26 mmol) was added and the reaction mixture was stirred at room temperature for 18 hours. The reaction mixture was quenched with a saturated ammonium chloride solution (2 mL) and water (20 mL), and extracted with ethyl acetate (100 mL). The combined organic phases were washed with saturated brine (30 mL). The organic phase was dried over anhydrous sodium sulfate and concentrated to obtain the target product tert-butyl (1R,2S,5S)-2-((R)-1-((7-chloro-8-fluoro-4-hydroxy-2-(methylthio)pyrido[4,3-d]pyrimidin-5-yl)oxy)-2,2-difluoroethyl)-3,8-diazabicyclo[3.2.1]octane-8-carboxylate (1.0 g, yield: 99.2%), a pale yellow solid. ES-API: [M+H]⁺= 535.9.

Step 4: Tert-butyl (1R,2S,5S)-2-((R)-1-((7-chloro-8-fluoro-4-hydroxy-2-(methylthio)pyrido[4,3-d]pyrimidin-5-yl)oxy)-2,2-difluoroethyl)-3,8-diazabicyclo[3.2.1]octane-8-carboxylate (1.0 g, 1.87 mmol) and diisopropylethylamine (1.21 g, 9.33 mmol) were dissolved in dichloromethane (25 mL). A 50% solution of propyl phosphoric anhydride in ethyl acetate (3.56 g, 5.60 mmol) was added at room temperature, and the reaction mixture was stirred at room temperature for 18 hours. The reaction mixture was diluted with dichloromethane (50 mL), washed successively with water (25 2) and saturated brine (25 mL), dried over anhydrous sodium sulfate, filtered, and concentrated. The crude product was purified by a flash silica gel column (tetrahydrofuran/petroleum ether: 0-20%) to obtain the target product tert-butyl (5R,5aS,6S,9R)-2-chloro-5-(difluoromethyl)-1-fluoro-12-(methylthio)-5a,6,7,8,9,10-hexahydro-5H-4-oxa-3,10a,11,13,14-pentaaza-6,9-methanonaphtho[1,8-ab]heptalene-14-carboxylate (435 mg, yield: 45.0%), a pale orange-red solid. ES-API: [M+H]⁺= 517.9.

Step 5: To a 5 mLmicrowave tube, tert-butyl (5R,5aS,6S,9R)-2-chloro-5-(difluoromethyl)-1-fluoro-12-(methylthio)-5a,6,7,8,9,10-hexahydro-5H-4-oxa-3,10a, 11,13,14-pentaaza-6,9-methanonaphtho[1,8-ab]heptalene-14-carboxylate (130 mg, 0.25 mmol), ((2-fluoro-8-(4,4,5,5-tetramethyl-1,3,2-dioxaborolan-2-yl)naphthalen-1-yl)ethynyl)triisopropylsilane (182 mg, 0.40 mmol), potassium phosphate (160 mg, 0.75 mmol), (S)-2-amino-1,1'-binaphthalene-2-yl palladium(II) methanesulfonate [Butyl(di-1-adamantyl)phosphine] (18 mg, 0.025 mmol), tetrahydrofuran (2.5 mL), and water (0.5 mL) were added. Purged with nitrogen for 30 seconds, the reaction mixture was stirred at 80 °C in a microwave reactor for 30 minutes. Ethyl acetate (40 mL) and water (10 mL) was added to the reaction mixture. The separated organic phase was washed with saturated brine (10 mL), dried over anhydrous sodium sulfate, and filtered. The filtrate was concentrated under reduced pressure. The crude product was purified by a flash silica gel column (tetrahydrofuran/petroleum ether: 0-30%) to obtain the target product tert-butyl (5R,5aS,6S,9R)-5-(difluoromethyl)-1-fluoro-2-(7-fluoro-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (200 mg, yield: 98.6%), a pale yellow solid. ES-API: [M+H]⁺=808.2.

Step 6: Tert-butyl (5R,5aS,6S,9R)-5-(difluoromethyl)-1-fluoro-2-(7-fluoro-8-((triisopropylsilyl)ethynyl)naphthalen-1 -yl)- 12-(methylthio)-5a,6,7,8,9,10-hexahydro-5H-4-oxa-3,10a,11,13,14-pentaaza-6,9-methanonaphtho[1,8-ab]heptalene-14-carboxylate (200 mg, 0.25 mmol) was dissolved in dichloromethane (15 mL). At room temperature, m-chloroperoxybenzoic acid (65 mg, 0.32 mmol) was added, and the reaction mixture was stirred in an ice water bath for 1 hour. The reaction mixture was added to dichloromethane (30 mL), washed successively with saturated sodium thiosulfate (2 mL), saturated sodium bicarbonate (15 mL), and saturated brine (15 mL). The organic phase was dried over anhydrous sodium sulfate, filtered, and concentrated under reduced pressure to obtain the target product tert-butyl (SR,SaS,6S,9R)-5-(difluoromethyl)-1-fluoro-2-(7-fluoro-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (195 mg, yield: 95.6%), a pale yellow solid. ES-API:[M+H]⁺=824.2.

Step 7: (R)-(1-methylenetetrahydro-1H-pyrrolizin-7a(5H)-yl)methanol (73 mg, 0.47 mmol) was dissolved in tetrahydrofuran (10 mL). At 0°C, 60% sodium hydride (57 mg, 1.42 mmol) was added, and the reaction mixture was stirred at this temperature for 30 minutes. Then, a tetrahydrofuran solution (3 mL) of tert-butyl (5R,5aS,6S,9R)-5-(difluoromethyl)-1-fluoro-2-(7-fluoro-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (195 mg, 0.24 mmol) was added dropwise. The reaction mixture was stirred in the ice water bath for 30 minutes. With saturated ammonium chloride solution (2 mL) and water (5 mL) added, the reaction mixture was extracted with ethyl acetate (40 mL). The organic phase was washed with saturated brine (15 mL), dried over anhydrous sodium sulfate, filtered, and concentrated under reduced pressure. The crude product was purified by a flash silica gel column (methanol/dichloromethane: 0-5%) to obtain the target product tert-butyl (5R,5aS,6S,9R)-5-(difluoromethyl)-1-fluoro-2-(7-fluoro-8-((triisopropylsilyl)ethynyl)naphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (100 mg, yield: 46.3%), a light brown solid. ES-API: [M+H]⁺=913.3.

Step 8: Tert-butyl (5R,5aS,6S,9R)-5-(difluoromethyl)-1-fluoro-2-(7-fluoro-8-((triisopropylsilyl)ethynyl)naphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (100 mg, 0.11 mmol) was dissolved in N,N-dimethylformamide (5 mL). At room temperature, cesium fluoride (166 mg, 1.10 mmol) was added, and the reaction mixture was stirred at room temperature for 1 hour. The reaction mixture was then diluted with ethyl acetate (50 mL) and successively washed with water (15 mL), dilute brine (15 mL×3), and saturated brine (15 mL). The organic phase was dried over anhydrous sodium sulfate, filtered, and concentrated under reduced pressure. The crude product was purified to obtain the target compound tert-butyl (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (82 mg, yield: 98.9%), a light brown solid. ES-API: [M+H]⁺=757.2.

Step 9: Tert-butyl (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (77 mg, 0.10 mmol) was dissolved in acetonitrile (2 mL). At 0°C, 4M hydrochloric acid in 1,4-dioxane (2 mL) was added, and the reaction mixture was stirred at room temperature for 2 hours. The reaction mixture was then concentrated under reduced pressure. The crude product was purified by preparative HPLC (formic acid method 1) to obtain the target compound, (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z648, 28 mg, yield: 36.7%, formate), pale yellow solid. ES-API: [M+H]⁺= 657.2. ¹H NMR (400 MHz, CD₃OD) δ 8.39 (s, 2H), 8.18 - 8.05 (m, 2H), 7.71 - 7.53 (m, 2H), 7.50 - 7.38 (m, 1H), 6.59 - 6.29 (m, 1H), 5.66 - 5.14 (m, 3H), 5.04 - 4.92 (m, 1H), 4.85 - 4.42 (m, 4H), 4.02 - 3.48 (m, 5H), 3.42 - 3.21 (m, 2H), 3.01 - 2.90 (m, 1H), 2.54 - 1.76 (m, 9H).

### Example 123 Synthesis of (5S,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z736)

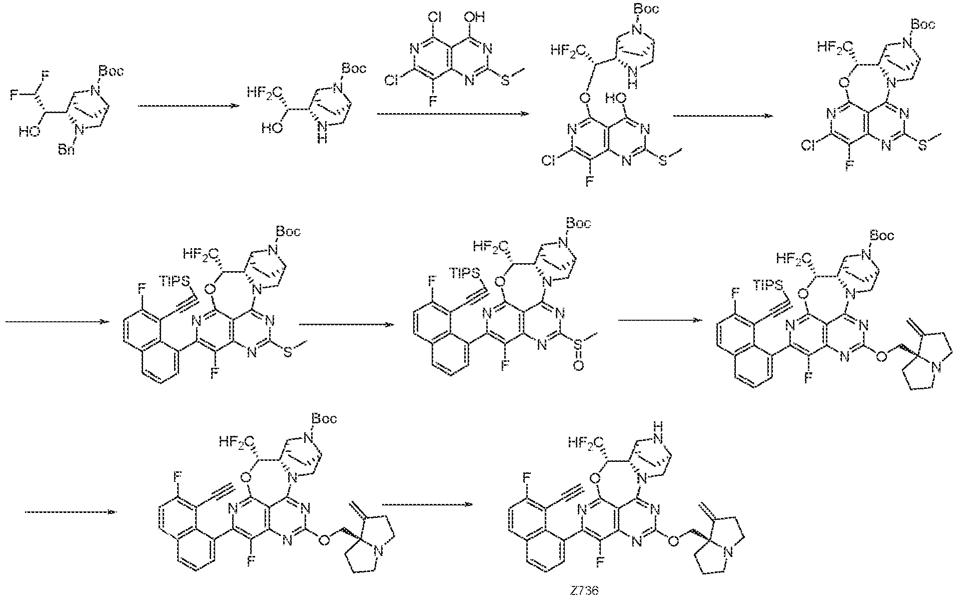

Step 1: Tert-butyl (1S,2S,5R)-3-benzyl-2-((S)-2,2-difluoro-1-hydroxyethyl)-3,8-diazabicyclo[3.2.1]octane-8-carboxylate (470 mg, 1.23 mmol) was dissolved in methanol (10 mL) and 7M ammonia methanol solution (0.7 mL), 10% palladium on carbon (100 mg) was added, and the reaction mixture was stirred at room temperature under a hydrogen atmosphere for 18 hours. The reaction mixture was filtered through diatomaceous earth, the filter cake was washed with methanol, and the filtrate was concentrated to obtain the target compound tert-butyl (1S,2S,5R)-2-((S)-2,2-difluoro-1-hydroxyethyl)-3,8-diazabicyclo[3.2.1]octane-8-carboxylate (350 mg, yield: 97.4%), a colorless liquid. ES-API: [M+H]⁺= 293.0.

Step 2: tert-butyl (1S,2S,5R)-2-((S)-2,2-difluoro-1-hydroxyethyl)-3,8-diazabicyclo[3.2.1]octane-8-carboxylate (350 mg, 1.20 mmol) was dissolved in tetrahydrofuran (25 mL). At 0 °C, 60% sodium hydride (240 mg, 5.99 mmol) was added, and the reaction mixture was stirred at 0 °C for 30 minutes. Then, 5,7-dichloro-8-fluoro-2-(methylthio)pyrido[4,3-d]pyrimidin-4(3H)-one (402 mg, 1.44 mmol) was added, and the reaction mixture was stirred at room temperature for 18 hours. The reaction mixture was quenched with saturated ammonium chloride solution (2 mL) and water (20 mL), and extracted with ethyl acetate (100 mL). The combined organic phases were washed with saturated brine (30 mL). The organic phase was dried over anhydrous sodium sulfate, filtered, and concentrated. The crude product was purified by flash silica gel column chromatography (tetrahydrofuran/petroleum ether: 0-50%) to obtain the target compound tert-butyl (1R,2S,5S)-2-((S)-1-((7-chloro-8-fluoro-4-hydroxy-2-(methylthio)pyrido[4,3-d]pyrimidin-5-yl)oxy)-2,2-difluoroethyl)-3,8-diazabicyclo[3.2.1]octane-8-carboxylate (500 mg, yield: 77.9%), a pale yellow solid. ES-API: [M+H]⁺= 535.9.

Step 3: Tert-butyl (1R,2S,5S)-2-((S)-1-((7-chloro-8-fluoro-4-hydroxy-2-(methylthio)pyrido[4,3-d]pyrimidin-5-yl)oxy)-2,2ilifluoroethyl)-3,8-diazabicyclo[3.2.1]octane-8-carboxylate (500 mg, 0.93 mmol) and diisopropylethylamine (603 mg, 4.66 mmol) were dissolved in dichloromethane (15 mL). At room temperature, 1-propylphosphonic anhydride 50% ethyl acetate solution (1.78 g, 2.80 mmol) was added, and the reaction mixture was stirred at room temperature for 18 hours. The reaction mixture was diluted with dichloromethane (50 mL) and successively washed with water (25 mL × 2) and saturated brine (25 mL). The organic phase was dried over anhydrous sodium sulfate, filtered, and concentrated. The crude product was purified by flash silica gel column chromatography (tetrahydrofuran/petroleum ether: 0-20%) to obtain the target compound tert-butyl (SS,SaS,6S,9R)-2-chloro-5-(difluoromethyl)-1-fluoro-12-(methylthio)-Sa,6,7,8,9,10-hexahydro-5H-4-oxa-3,10a,11,13,14-pentaaza-6,9-methanonaphtho[1,8-ab]heptalene-14-carboxylate (350 mg, yield: 72.4%), a white solid. ES-API: [M+H]⁺= 517.9.

Step 4: To a 5 mL microwave tube, tert-butyl (SS,SaS,6S,9R)-2-chloro-5-(difluoromethyl)-1-fluoro-12-(methylthio)-5a,6,7,8,9,10-hexahydro-5H-4-oxa-3,10a, 11,13,14-pentaaza-6,9-methanonaphtho[1,8-ab]heptalene-14-carboxylate (100 mg, 0.19 mmol), ((2-fluoro-8-(4,4,5,5-tetramethyl-1,3,2-dioxaborolane-2-yl)naphth-1-yl)ethynyl)triisopropylsilane (140 mg, 0.31 mmol), potassium phosphate (123 mg, 0.58 mmol), methanesulfonic acid [butyl bis(1-adamantyl)phosphine](2-amino-1,1'-biphenyl-2-yl)palladium(II) (14 mg, 0.019 mmol), tetrahydrofuran (2.5 mL), and water (0.5 mL) were added. The reaction mixture was purged with nitrogen for 30 seconds, and then stirred in a microwave reactor at 80 °C for 30 minutes. The reaction mixture was diluted with ethyl acetate (40 mL) and water (10 mL). The separated organic phase was washed with saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-30%) to obtain the target compound tert-butyl (5S,5aS,6S,9R)-5-(difluoromethyl)-1-fluoro-2-(7-fluoro-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (150 mg, yield: 96.1%), a pale yellow solid. ES-API: [M+H]⁺=808.2.

Step 5: Tert-butyl (5S,5aS,6S,9R)-5-(difluoromethyl)-1-fluoro-2-(7-fluoro-8-((triisopropylsilyl)ethynyl)naphthalen-1 -yl)-12-(methylthio)-5a,6,7,8,9,10-hexahydro-5H-4-oxa-3,10a,11,13,14-pentaaza-6,9-methanonaphtho[1,8-ab]heptalene-14-carboxylate (150 mg, 0.19 mmol) was dissolved in dichloromethane (15 mL). M-chloroperoxybenzoic acid (49 mg, 0.24 mmol) was added at room temperature. The reaction mixture was stirred in an ice water bath for 1 hour. The reaction mixture was then diluted with dichloromethane (30 mL) and successively washed with saturated sodium thiosulfate solution (2 mL), saturated sodium bicarbonate solution (15 mL), and saturated brine (15 mL). The organic phase was dried over anhydrous sodium sulfate and filtered. The filtrate was concentrated under reduced pressure to obtain the target product tert-butyl (5S,5aS,6S,9R)-5-(difluoromethyl)-1-fluoro-2-(7-fluoro-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, yield: 98.1%), a pale yellow solid. ES-API:[M+H]⁺=824.0.

Step 6: (R)-(1-methylenetetrahydro-1H-pyrrolazin-7a(5H)-yl)methanol (56 mg, 0.36 mmol) was dissolved in tetrahydrofuran (10 mL) and 60% sodium hydride (44 mg, 1.09 mmol) was added at 0°C. The reaction mixture was stirred at this temperature for 30 minutes, then a tetrahydrofuran solution (3 mL) of tert-butyl (5S,5aS,6S,9R)-5-(difluoromethyl)-1-fluoro-2-(7-fluoro-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]heptalene-14-carboxylate (150 mg, 0.18 mmol) was added dropwise. The reaction mixture was stirred in an ice water bath for 30 minutes. With saturated ammonium chloride solution (2 mL) and water (5 mL) added, the reaction mixture was extracted with ethyl acetate (40 mL). The organic phase was washed with saturated brine (15 mL), dried over anhydrous sodium sulfate, and filtered. The filtrate was concentrated under reduced pressure. The crude product was purified by flash silica gel column chromatography (methanol/dichloromethane: 0-5%) to obtain the target product tert-butyl (5S,5aS,6S,9R)-5-(difluoromethyl)-1-fluoro-2-(7-fluoro-8-((triisopropylsilyl)ethynyl)naphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (78 mg, yield: 46.9%), a light brown solid. ES-API: [M+H]⁺=913.3.

Step 7: Tert-butyl (5S,5aS,6S,9R)-5-(difluoromethyl)-1-fluoro-2-(7-fluoro-8-((triisopropylsilyl)ethynyl)naphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (78 mg, 0.085 mmol) was dissolved in N,N-dimethylformamide (4mL). Cesium fluoride (130 mg, 0.85 mmol) was added at room temperature, and the reaction mixture was stirred at room temperature for 1 hour. The reaction mixture was added to ethyl acetate (50 mL), washed successive with water (15 mL), dilute brine (15 mL × 3), and saturated brine (15 mL). The organic phase was dried over anhydrous sodium sulfate, filtered, and the filtrate was concentrated under reduced pressure to obtain the target product tert-butyl (5 S,5aS,6S,9R)-5-(difluoromethyl)-2-(8-ethynyl-7-fluoronaphthalen-1 -yl)-1 -fluoro- 12-(((R)-1 - methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, yield: 99.0%), a light brown solid. ES-API: [M+H]⁺=757.2.

Step 8: Tert-butyl (5S,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene-14-carboxylate (64 mg, 0.085 mmol) was dissolved in acetonitrile (1.5 mL). 4M hydrochloric acid in 1,4-dioxane (1.5 mL) was added at 0 °C, and the reaction mixture was stirred at room temperature for 2 hours. The reaction mixture was concentrated under reduced pressure. The crude product was purified by preparative HPLC (ammonium bicarbonate method 1) to obtain the target compound, (5 S,5aS,6S,9R)-5-(difluoromethyl)-2-(8-ethynyl-7-fluoronaphthalen-1 -yl)-1 -fluoro- 12-(((R)-1 - methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalene (Z736, 18 mg, yield: 32.4%), white solid. ES-API: [M+H]⁺= 657.2. ¹H NMR (400 MHz, CD₃OD) δ 8.15 - 8.05 (m, 2H), 7.67 - 7.54 (m, 2H), 7.47 - 7.39 (m, 1H), 6.42 (td, *J* = 53.2, 7.2 Hz, 1H), 5.45 - 5.06 (m, 3H), 4.79 - 4.55 (m, 1H), 4.46 - 4.25 (m, 2H), 4.06 - 3.89 (m, 2H), 3.83 - 3.71 (m, 2H), 3.56 - 3.42 (m, 1H), 3.28 - 3.10 (m, 2H), 2.88 - 2.63 (m, 3H), 2.25 - 1.68 (m, 9H).

The compounds in Table B or Table C prepared by referring to the methods outlined in the provided examples, with modifications to some of the starting materials.

**Table B**

| Structure and Number | MS [M+H] ⁺ | Structure and Number | MS [M+H] ⁺ | Structure and Number | **MS** [M+H]⁺ |
|---|---|---|---|---|---|
| 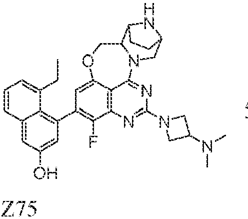 | 555.3 | 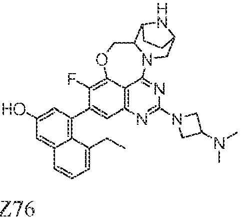 | 555.3 | 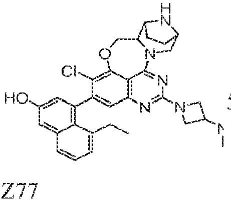 | 571.3 |
| 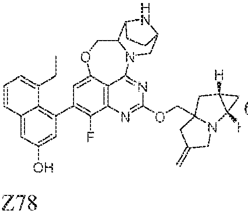 | 520.3 | 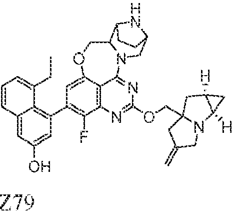 | 520.3 | 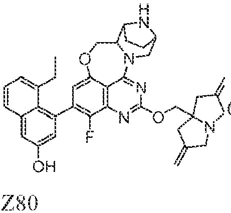 | 620.3 |
| 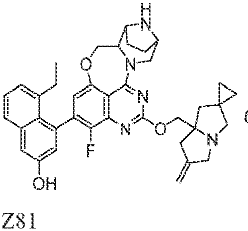 | 634.3 | 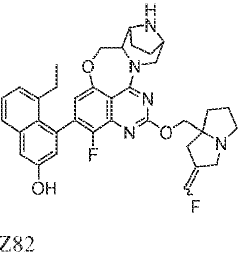 | 526.3 | 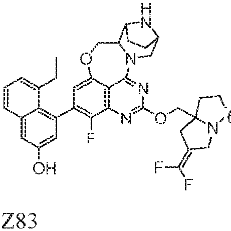 | 644.3 |
| 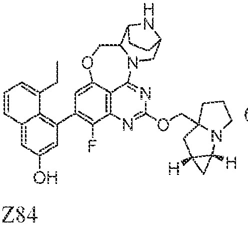 | 608.3 | 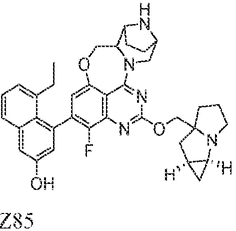 | 508.3 | 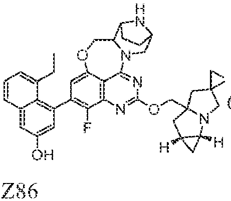 | 634.3 |
| 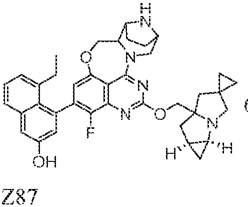 | 634.3 | 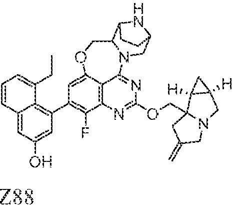 | 520.3 | 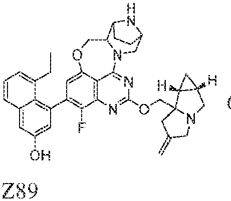 | 620.3 |
| 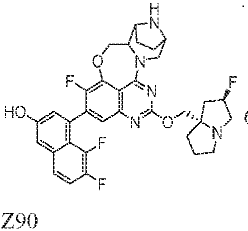 | 622.2 | 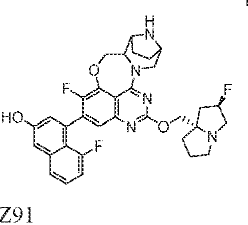 | 504.3 | 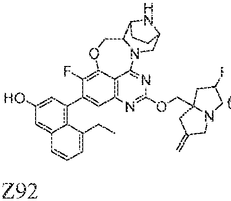 | 626.3 |
| 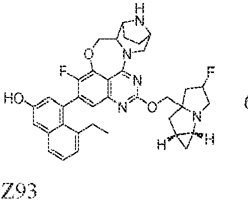 | 626.3 | 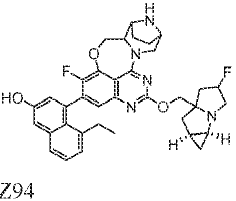 | 526.3 | 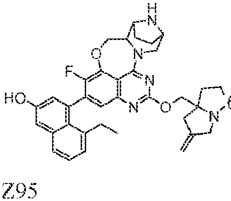 | 608.3 |
| 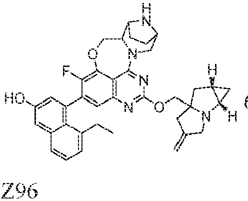 | 620.3 | 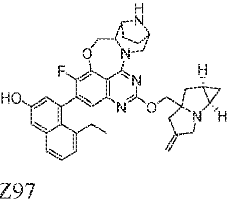 | 520.3 | 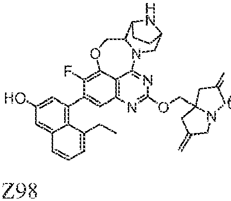 | 620.3 |
| 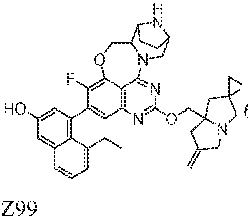 | 634.3 | 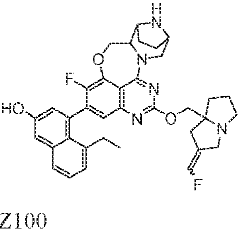 | 526.3 | 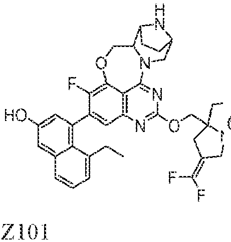 | 644.3 |
| 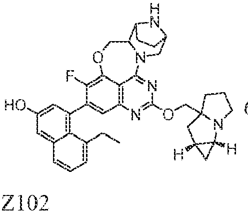 | 608.3 | 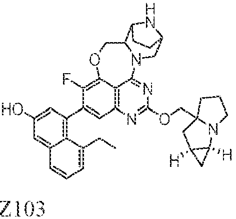 | 508.3 | 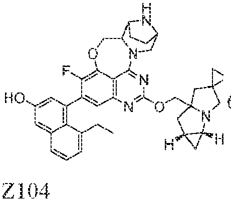 | 634.3 |
| 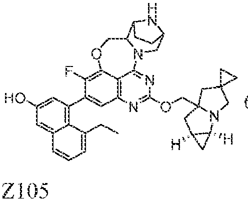 | 634.3 | 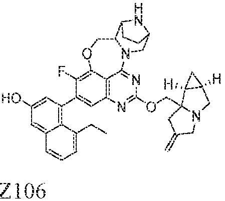 | 520.3 | 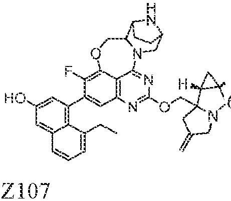 | 620.3 |
| 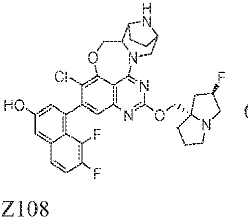 | 638.2 | 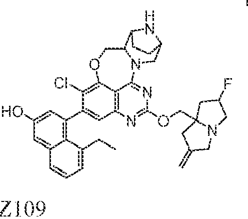 | 542.3 | 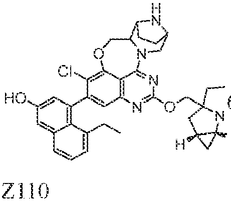 | 642.3 |
| 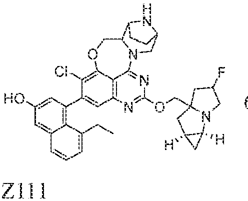 | 642.3 | 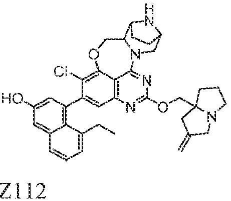 | 524.3 | 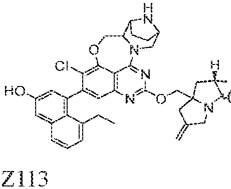 | 636.3 |
| 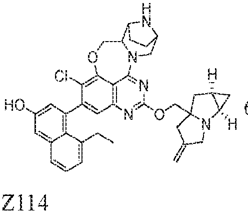 | 636.3 | 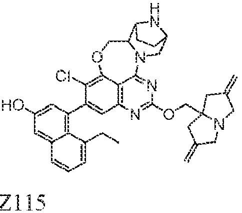 | 536.3 | 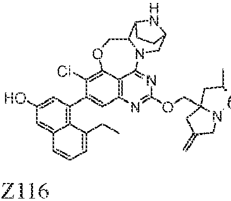 | 650.3 |
| 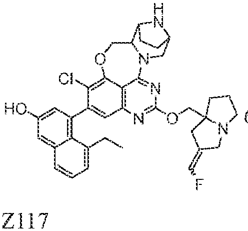 | 642.3 | 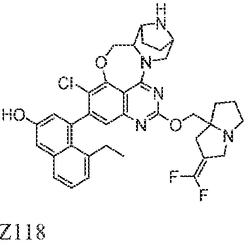 | 660.3 | 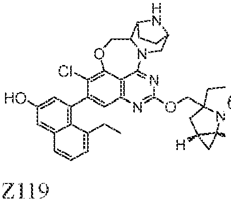 | 624.4 |
| 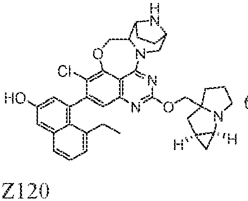 | 624.4 | 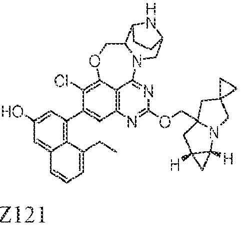 | 550.2 | 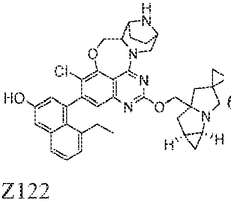 | 650.3 |
| 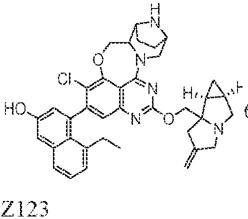 | 636.3 | 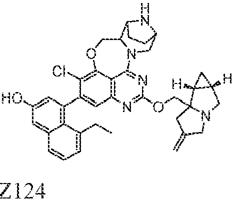 | 636.3 | 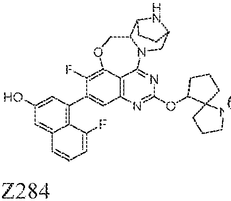 | 600.3 |
| 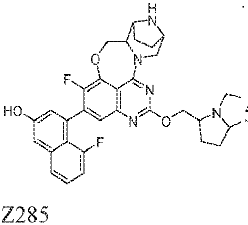 | 586.3 | 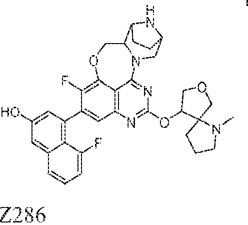 | 502.3 | 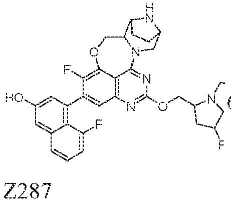 | 604.3 |
| 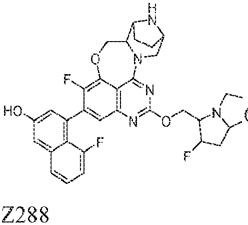 | 604.3 | 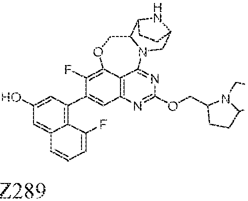 | 504.3 | 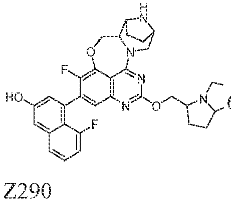 | 604.3 |
| 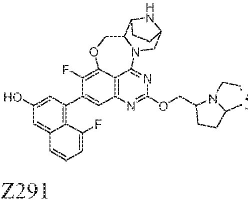 | i98.3 | 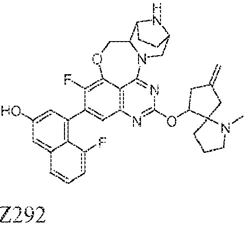 | 512.3 | 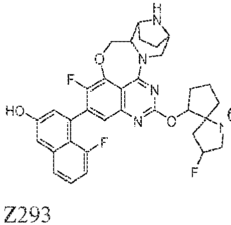 | 618.3 |
| 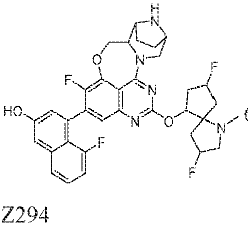 | 636.3 | 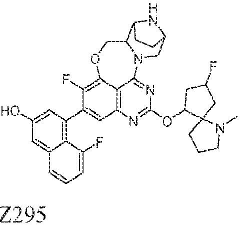 | 518.3 | 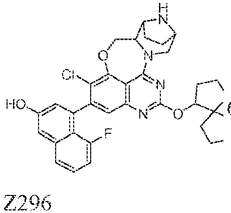 | 616.2 |
| 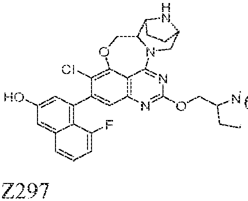 | 602.2 | 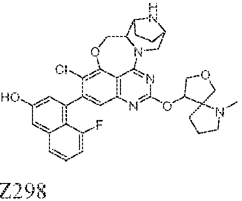 | 518.2 | 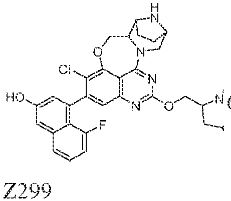 | 620.2 |
| 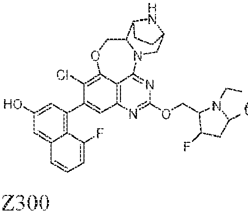 | 620.2 | 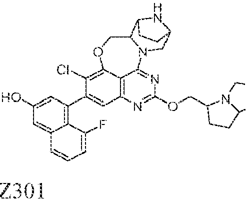 | 520.2 | 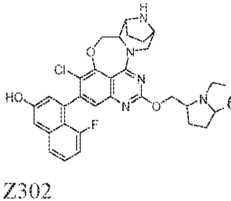 | 620.2 |
| 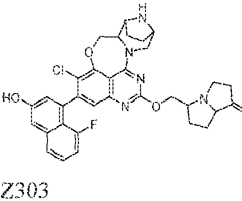 | 614.2 | 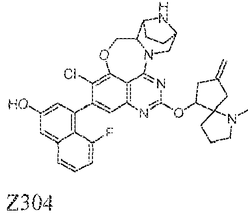 | 628.2 | 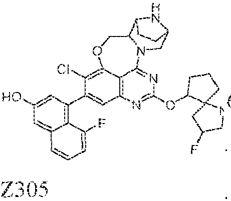 | 634.2 |
| 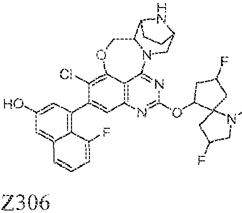 | 652.2 | 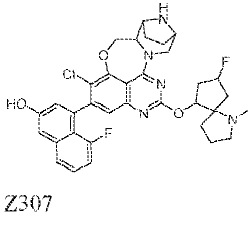 | 634.2 | 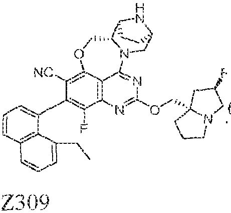 | 623.3 |
| 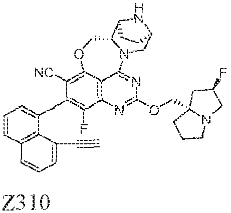 | 619.3 | 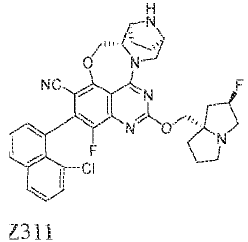 | 529.2 | 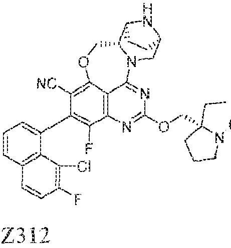 | 547.2 |
| 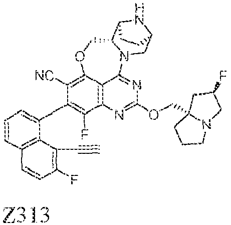 | 637.3 | 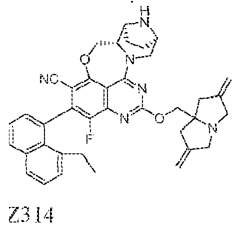 | 629.3 | 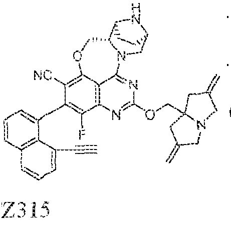 | 525.3 |
| 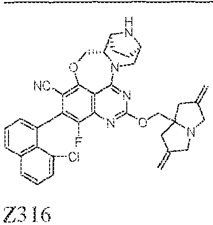 | 635.2 | 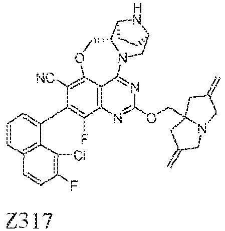 | 653.2 | 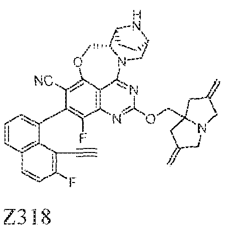 | 543.3 |
| 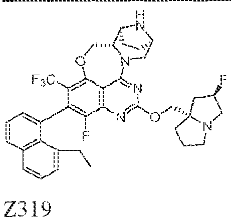 | 666.3 | 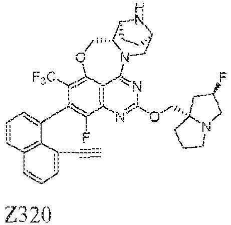 | 562.3 | 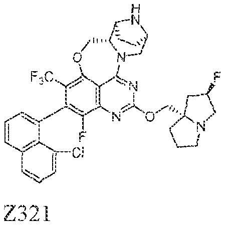 | 672.2 |
| 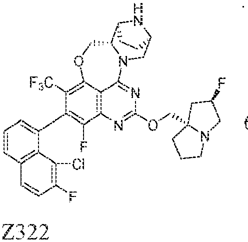 | 690.2 | 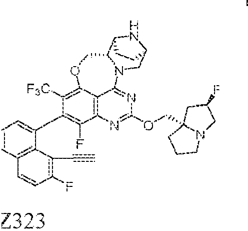 | 680.2 | 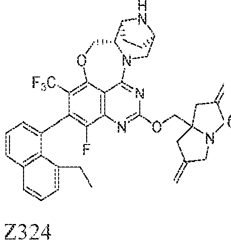 | 672.3 |
| 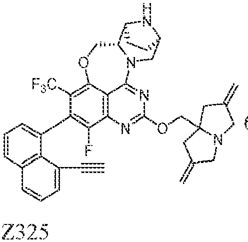 | 668.3 | 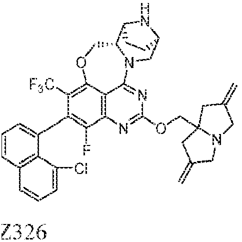 | 678.2 | 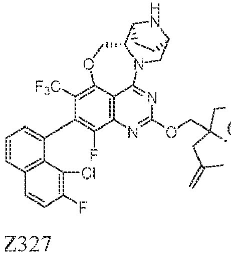 | 696.2 |
| 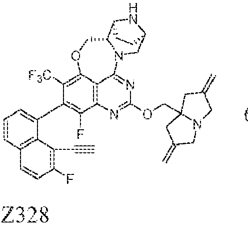 | 686.3 | 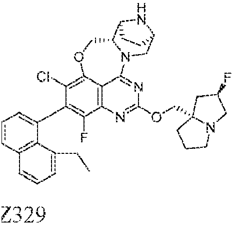 | 532.3 | 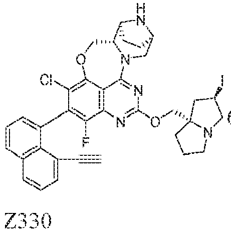 | 628.2 |
| 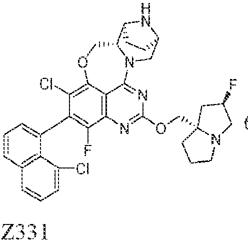 | 638.2 | 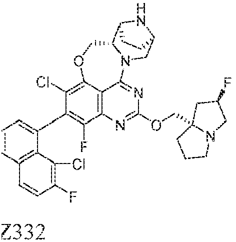 | 556.2 | 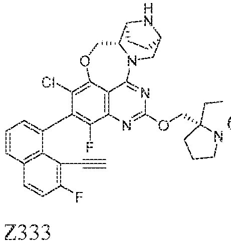 | 646.2 |
| 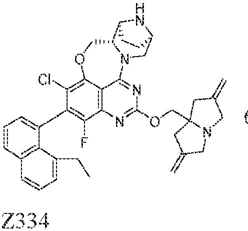 | 638.3 | 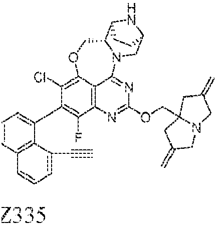 | 534.2 | 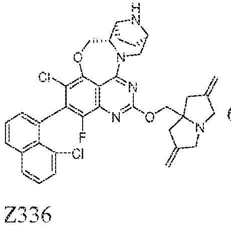 | 644.2 |
| 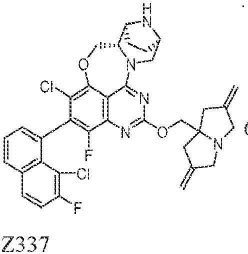 | 662.2 | 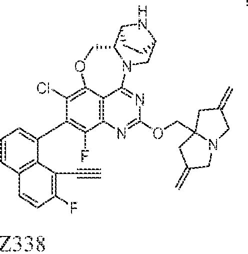 | 552.2 | 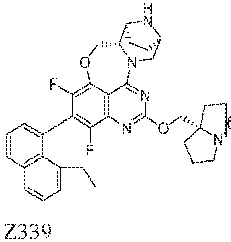 | 616.3 |
| 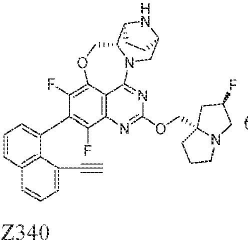 | 612.3 | 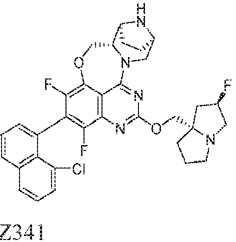 | 522.2 | 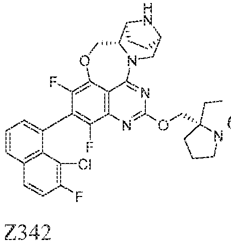 | 640.2 |
| 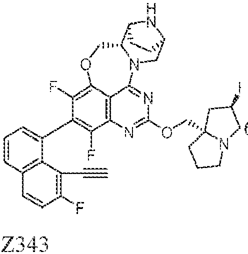 | 630.2 | 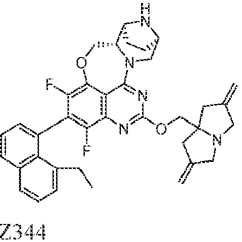 | 522.3 | 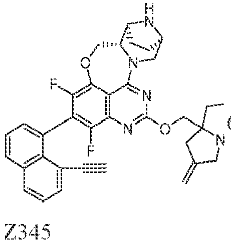 | 618.3 |
| 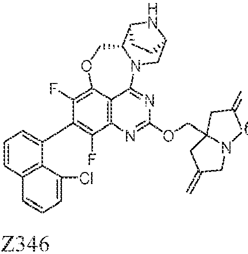 | 628.2 | 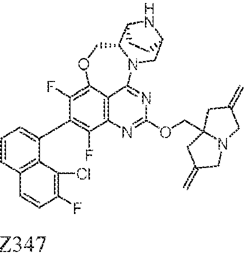 | 546.2 | 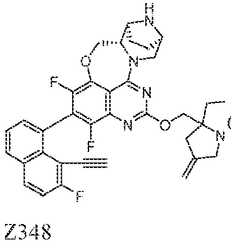 | 636.3 |

**Table C**

| Structure and Number | MS [M+H] ⁺ | Structure and Number | MS [M+H] ⁺ | Structure and Number | MS [M+H] ⁺ |
|---|---|---|---|---|---|
| 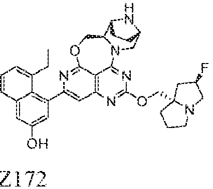 | 597.3 | 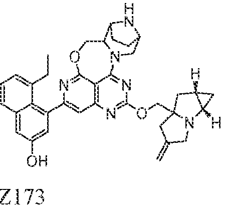 | 603.3 | 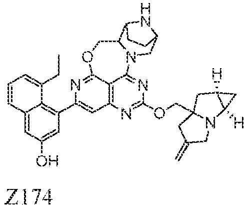 | 503.3 |
| 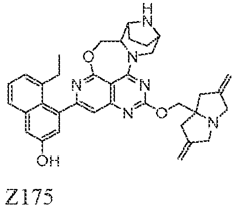 | 603.3 | 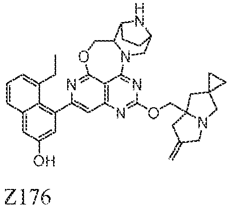 | 617.3 | 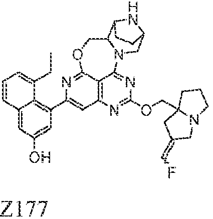 | 609.3 |
| 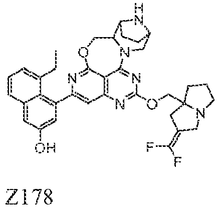 | 627.3 | 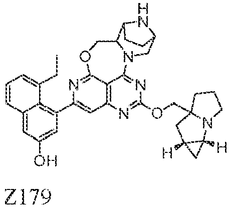 | 591.3 | 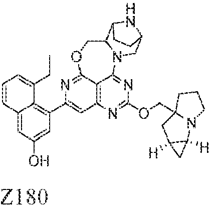 | 591.3 |
| 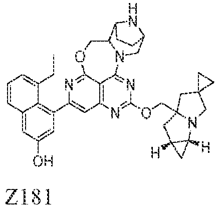 | 617.3 | 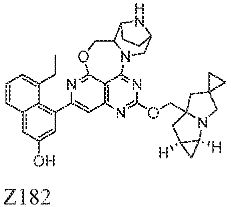 | 617.3 | 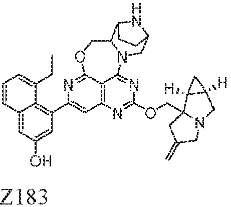 | 603.3 |
| 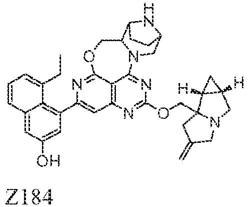 | 603.3 | 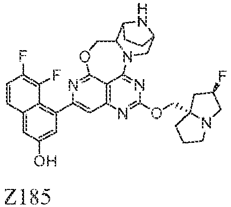 | 605.2 | 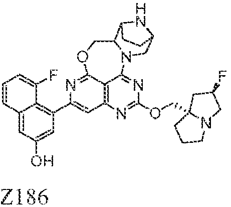 | 587.3 |
| 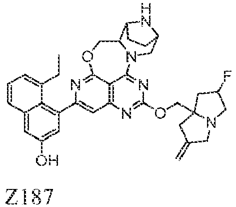 | 609.3 | 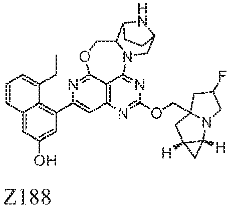 | 609.3 | 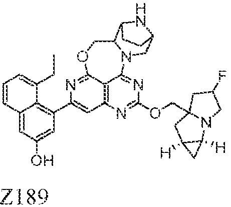 | 609.3 |
| 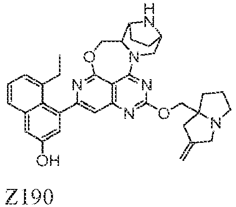 | 591.3 | 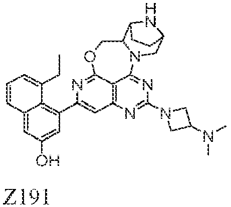 | 538.3 | 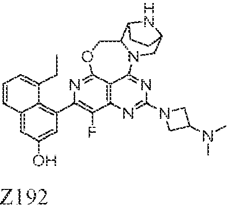 | 556.3 |
| 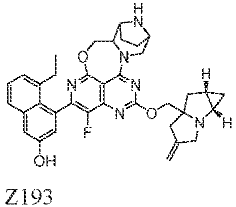 | 621.3 | 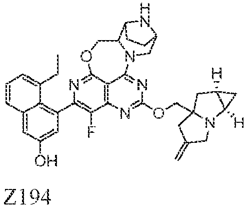 | 621.3 | 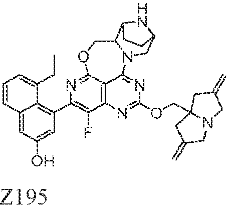 | 521.3 |
| 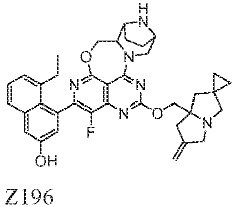 | 635.3 | 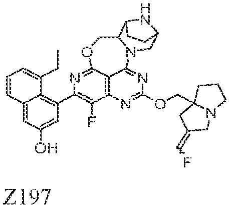 | 637.3 | 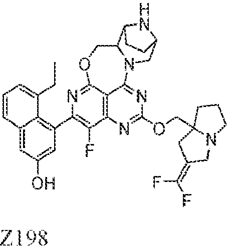 | 545.3 |
| 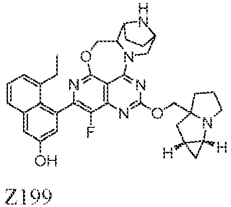 | 609.3 | 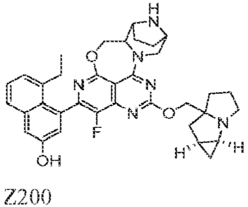 | 609.3 | 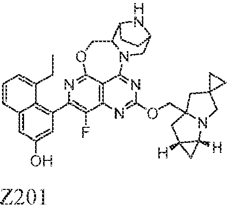 | 535.3 |
| 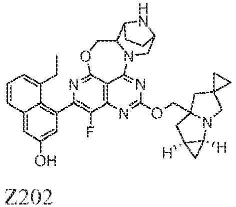 | 635.3 | 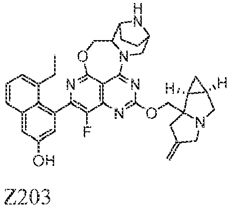 | 621.3 | 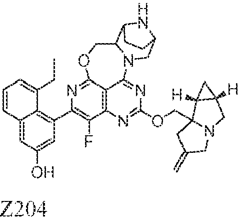 | 621.3 |
| 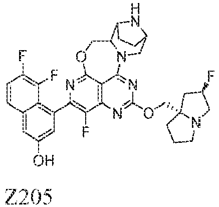 | 623.2 | 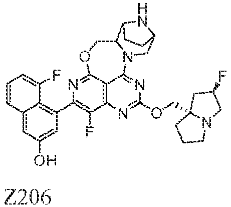 | 605.2 | 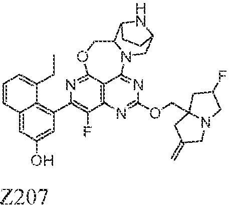 | 527.3 |
| 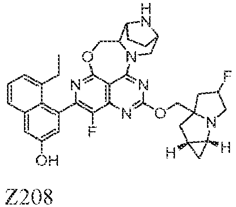 | 627.3 | 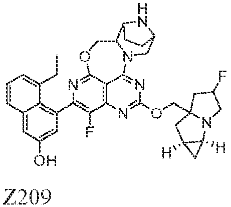 | 627.3 | 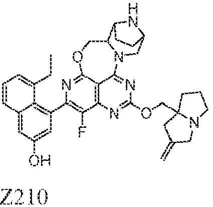 | 609.3 |
| 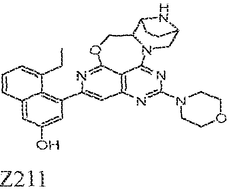 | 525.3 | 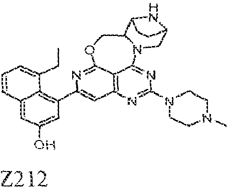 | 538.3 | 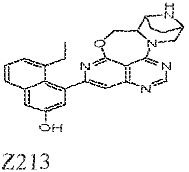 | 440.2 |
| 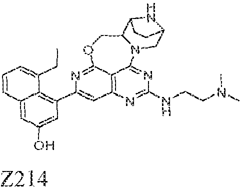 | 526.3 | 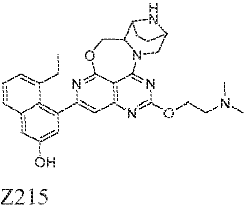 | 527.3 | 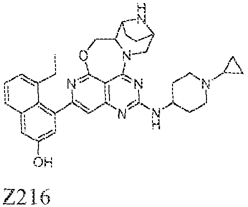 | 578.3 |
| 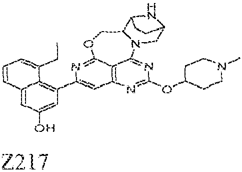 | 553.3 | 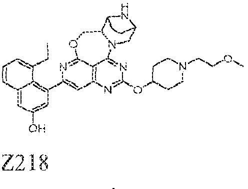 | 597.3 | 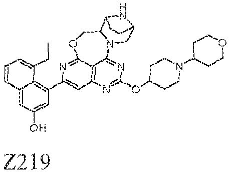 | 623.3 |
| 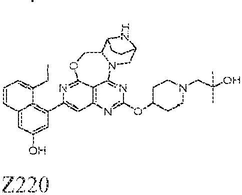 | 611.3 | 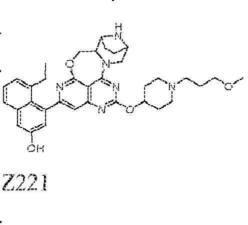 | 611.3 | 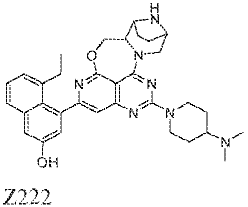 | 566.3 |
| 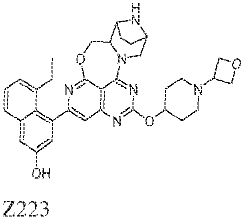 | 595.3 | 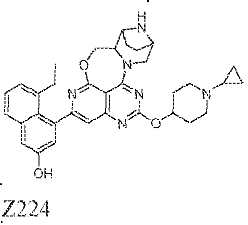 | 579.3 | 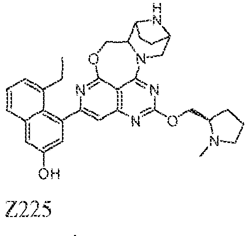 | 553.3 |
| 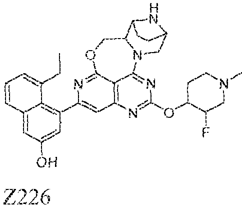 | 571.3 | 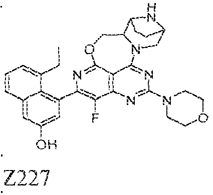 | 543.2 | 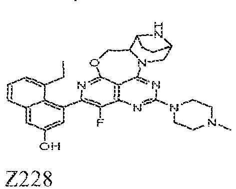 | 556.3 |
| 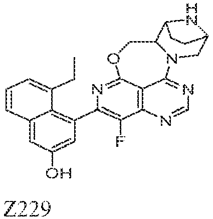 | 458.2 | 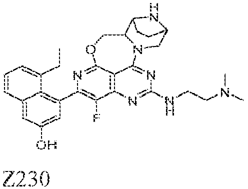 | 544.3 | 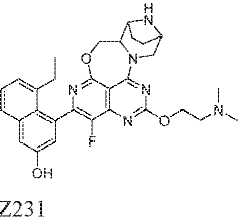 | 545.3 |
| 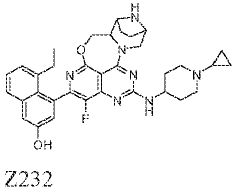 | 596.3 | 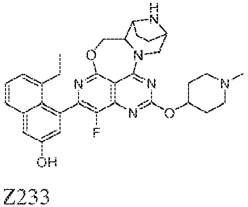 | 571.3 | 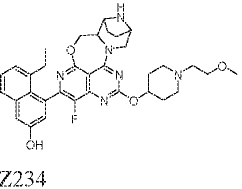 | 615.3 |
| 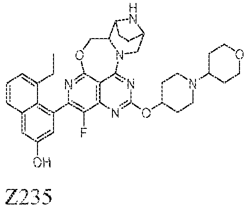 | 641.3 | 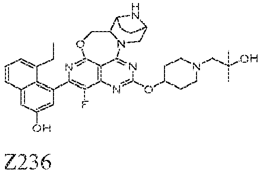 | 629.3 | 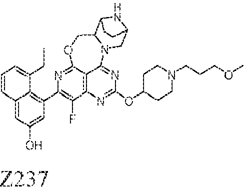 | 529.3 |
| 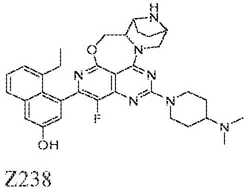 | 584.3 | 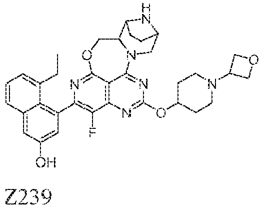 | 613.3 | 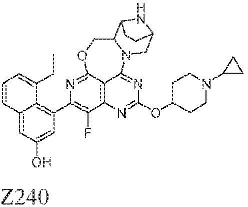 | 597.3 |
| 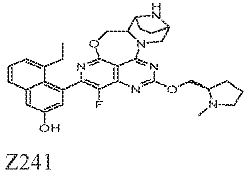 | 571.3 | 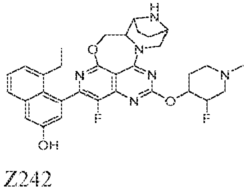 | 589.3 | 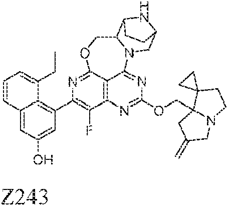 | 535.1 |
| 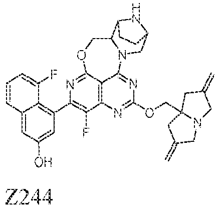 | 611.3 | 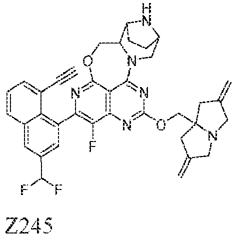 | 651.3 | 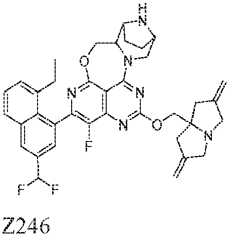 | 655.3 |
| 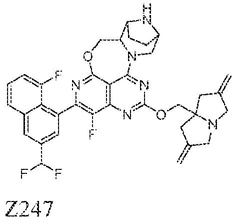 | 545.3 | 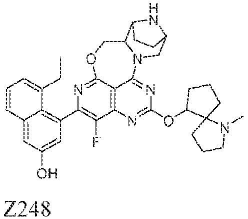 | 611.3 | 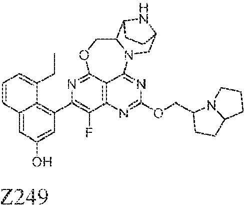 | 597.3 |
| 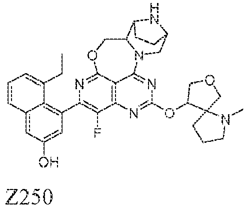 | 513.3 | 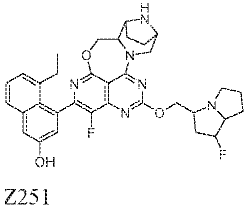 | 615.3 | 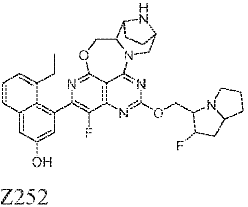 | 515.3 |
| 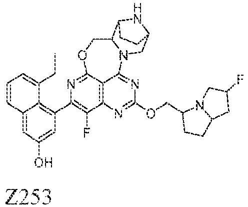 | 515.3 | 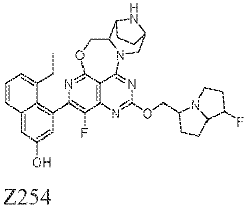 | 615.3 | 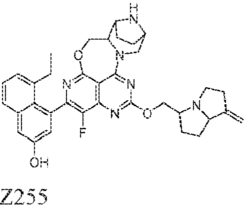 | 609.3 |
| 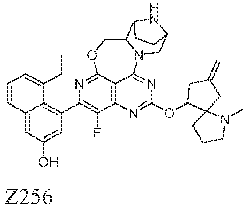 | 523.3 | 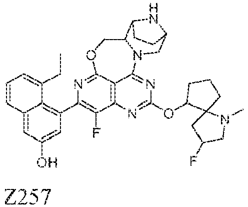 | 629.3 | 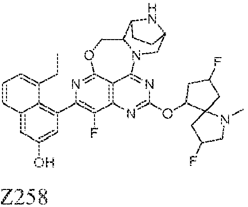 | 547.3 |
| 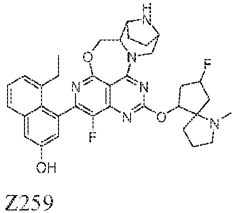 | 529.3 | 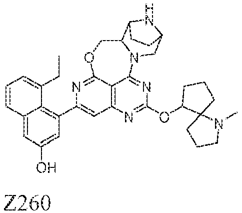 | 593.3 | 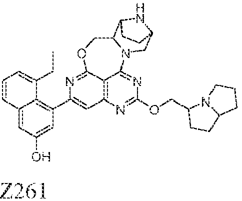 | 579.3 |
| 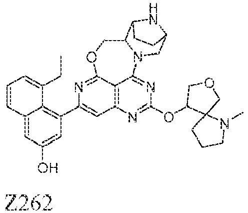 | 595.3 | 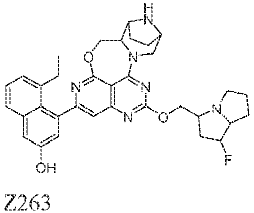 | 597.3 | 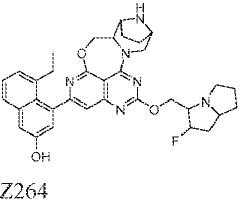 | 597.3 |
| 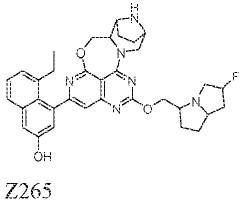 | 597.3 | 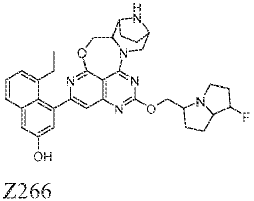 | 597.3 | 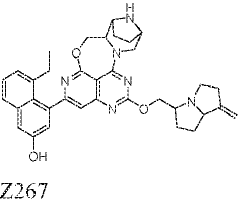 | 591.3 |
| 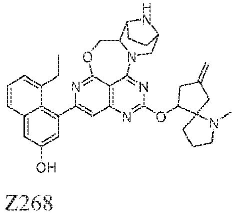 | 605.3 | 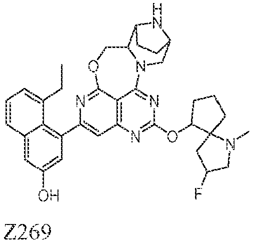 | 511.3 | 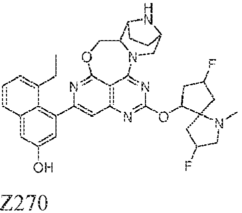 | 629.3 |
| 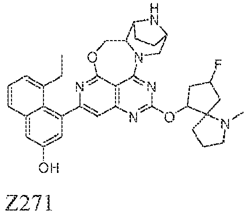 | 611.3 | 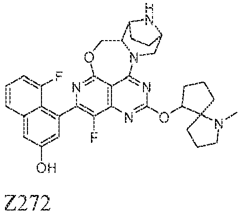 | 601.3 | 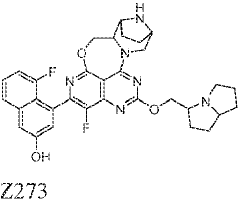 | 587.3 |
| 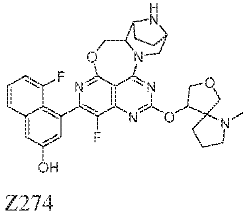 | 603.3 | 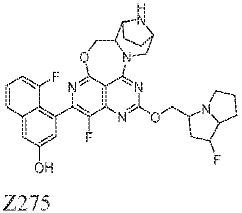 | 605.2 | 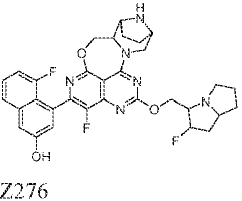 | 505.2 |
| 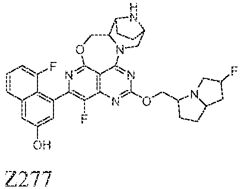 | 605.2 | 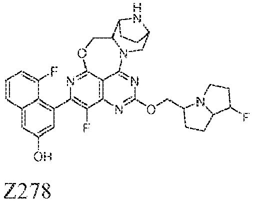 | 505.2 | 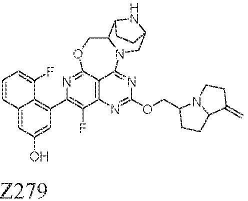 | 599.3 |
| 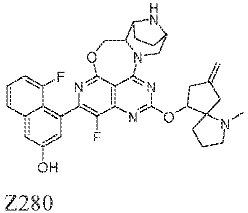 | 613.3 | 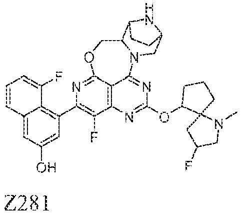 | 519.3 | 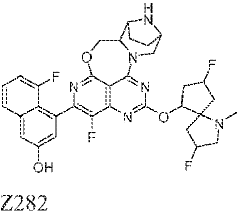 | .537.3 |
| 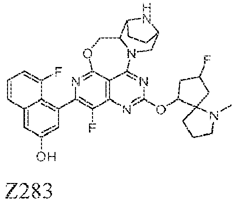 | 619.3 | 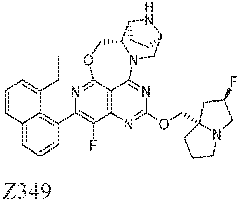 | 599.3 | 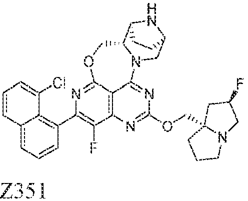 | 505.2 |
| 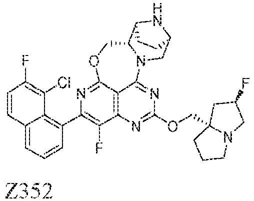 | 623.2 | 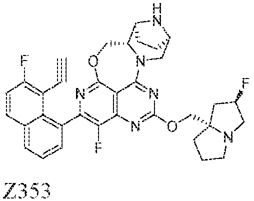 | 613.3 | 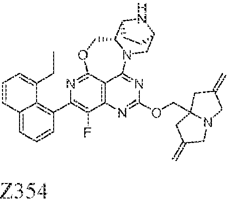 | 505.3 |
| 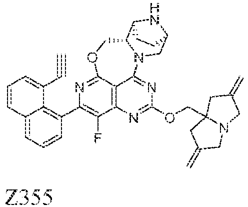 | 601.3 | 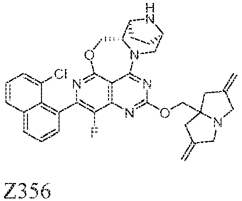 | 611.2 | 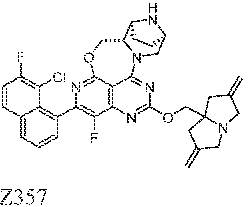 | 529.2 |
| 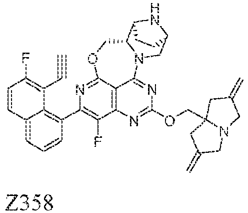 | 619.3 | 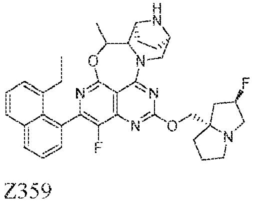 | 613.3 | 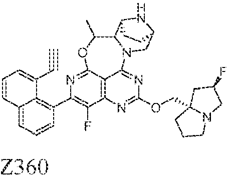 | 509.3 |
| 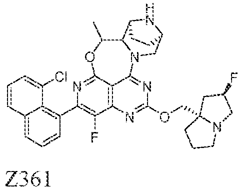 | 619.2 | 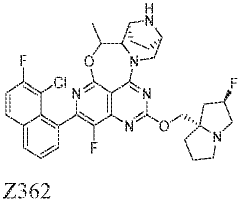 | 637.2 | 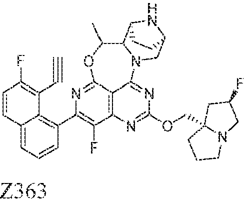 | 527.3 |
| 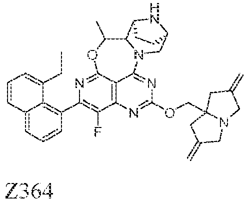 | 619.3 | 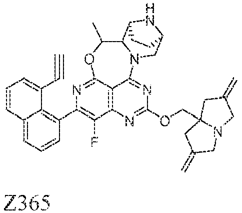 | 615.3 | 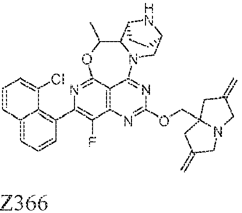 | 525.2 |
| 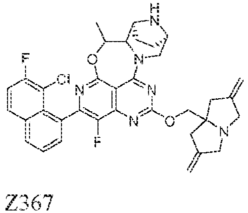 | 643.2 | 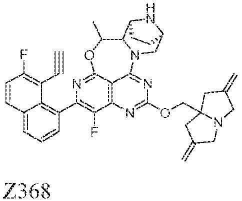 | 633.3 | 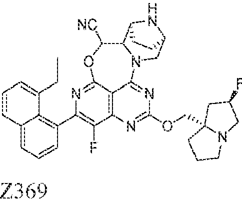 | 524.3 |
| 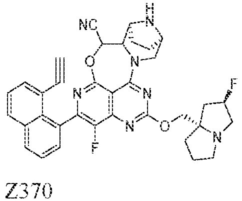 | 620.3 | 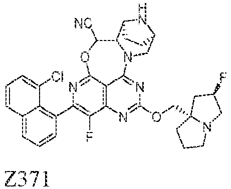 | 630.2 | 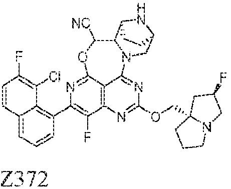 | 548.2 |
| 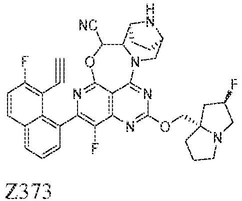 | 638.2 | 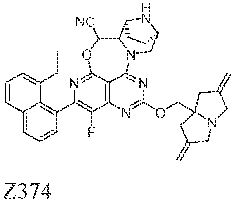 | 630.3 | 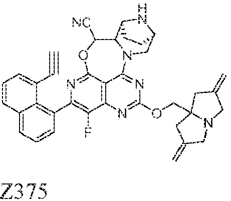 | 626.3 |
| 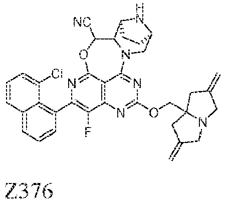 | 636.2 | 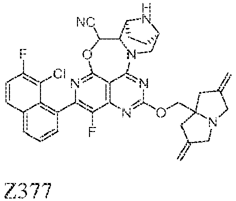 | 654.2 | 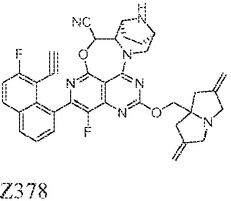 | 544.3 |
| 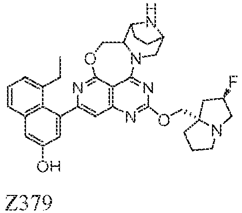 | 597.3 | 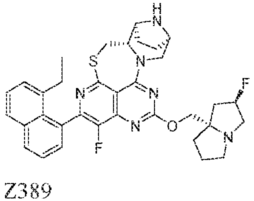 | 615.3 | 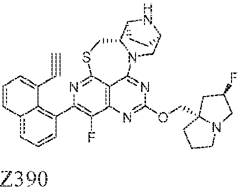 | 511.2 |
| 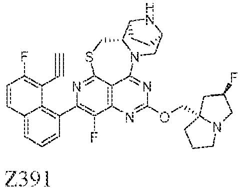 | 629.2 | 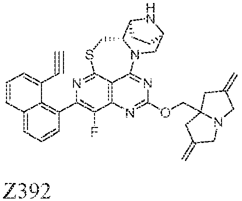 | 617.2 | 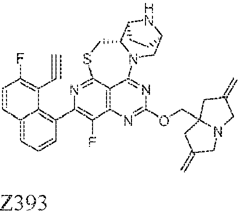 | 535.2 |
| 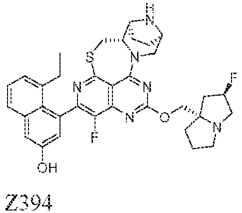 | 631.3 | 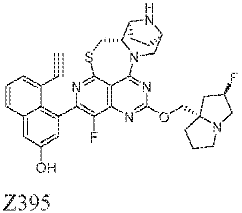 | 627.2 | 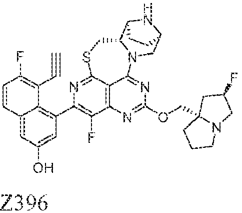 | 545.2 |
| 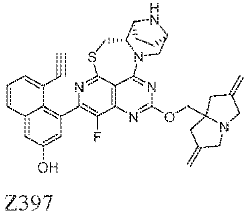 | 633.2 | 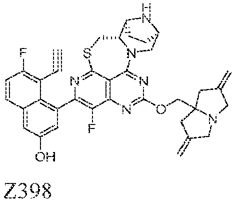 | 651.2 | 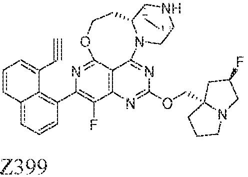 | 509.3 |
| 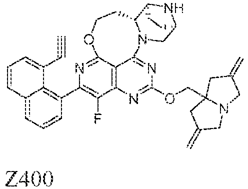 | 615.3 | 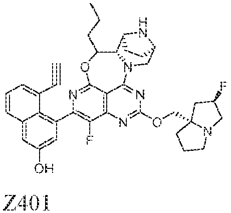 | 653.3 | 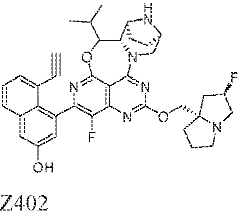 | 553.3 |
| 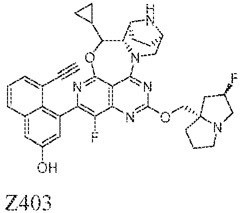 | 651.3 | 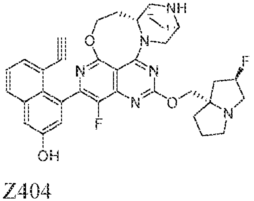 | 525.3 | 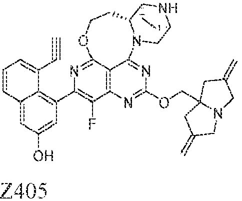 | 531.3 |
| 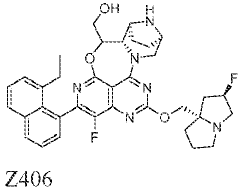 | 629.3 | 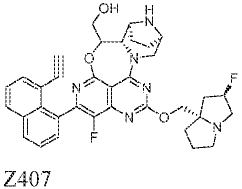 | 625.3 | 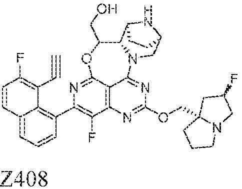 | 543.3 |
| 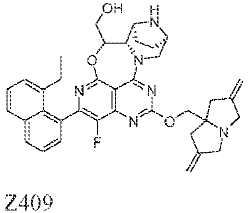 | 635.3 | 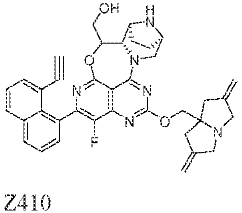 | 631.3 | 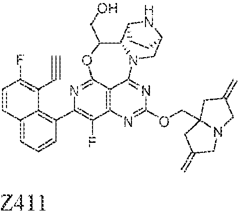 | 649.3 |
| 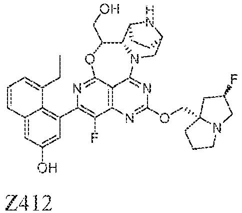 | 645.3 | 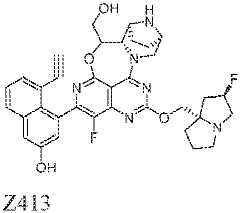 | 641.3 | 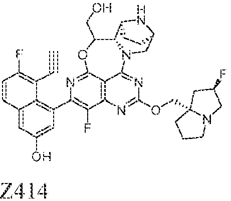 | 659.3 |
| 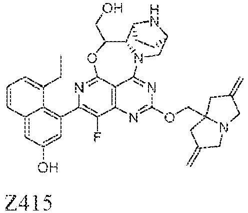 | 651.3 | 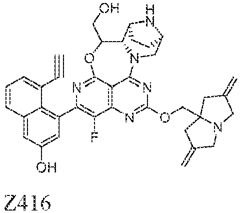 | 647.3 | 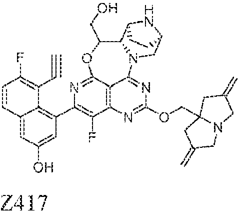 | 664.3 |
| 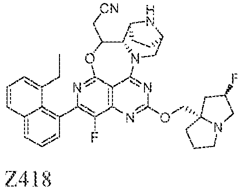 | 638.3 | 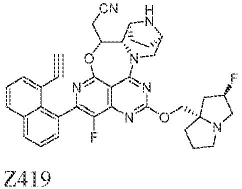 | 634.3 | 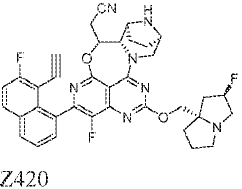 | 552.3 |
| 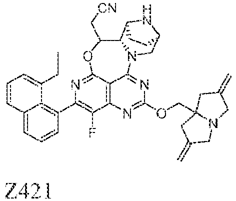 | 644.3 | 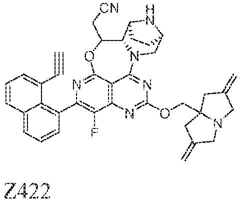 | 640.3 | 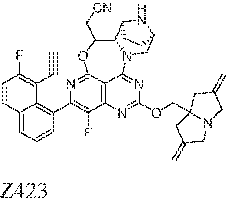 | 558.3 |
| 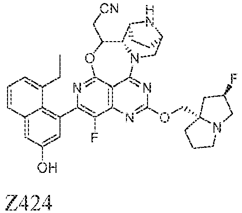 | 654.3 | 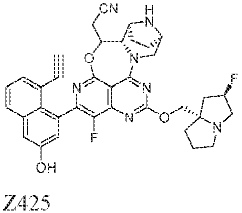 | 650.3 | 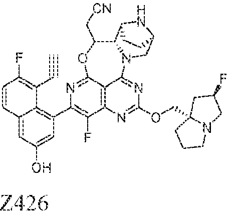 | 568.3 |
| 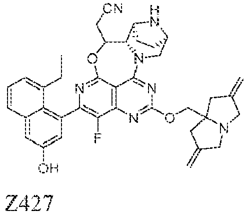 | 660.3 | 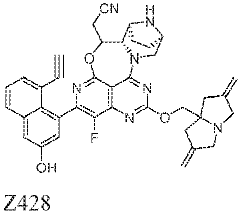 | 656.3 | 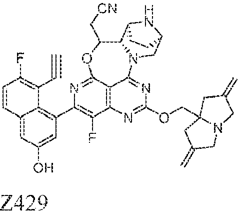 | 674.3 |
| 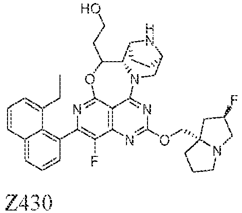 | 643.3 | 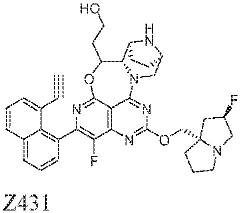 | 639.3 | 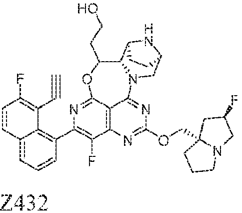 | 657.3 |
| 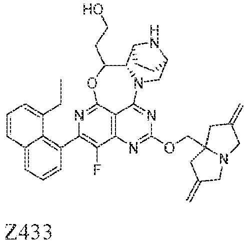 | 649.3 | 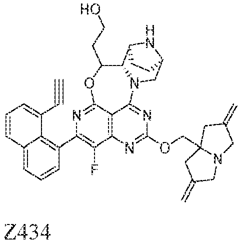 | 645.3 | 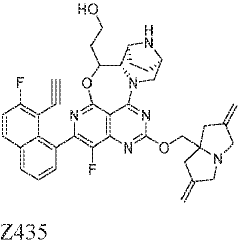 | 663.3 |
| 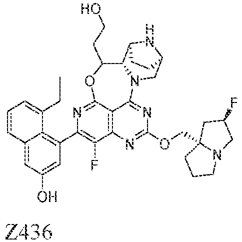 | 659.3 | 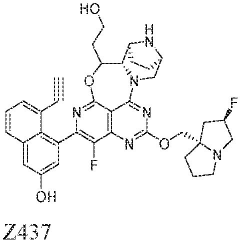 | 655.3 | 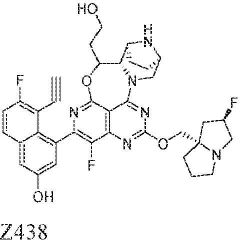 | 573.3 |
| 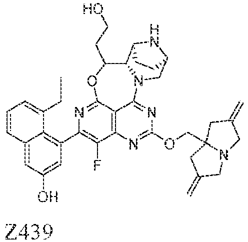 | 665.3 | 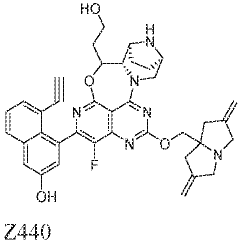 | 661.3 | 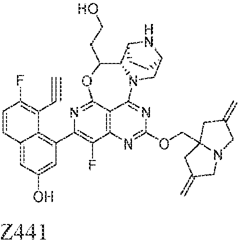 | 579.3 |
| 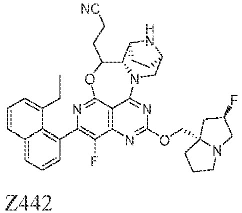 | 652.3 | 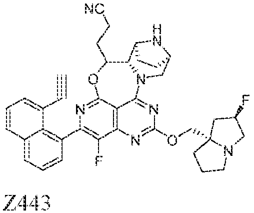 | 648.3 | 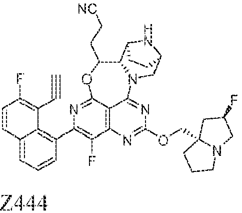 | 666.3 |
| 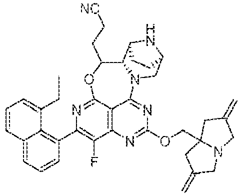 | 658.3 | 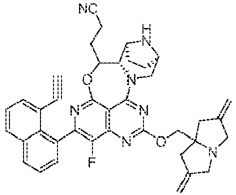 | 654.3 | 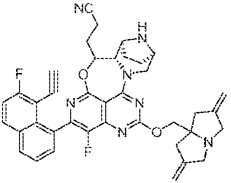 | 572.3 |
| 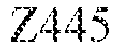 | | 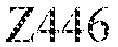 | | 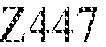 | |
| 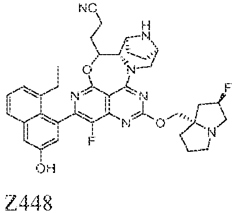 | 668.3 | 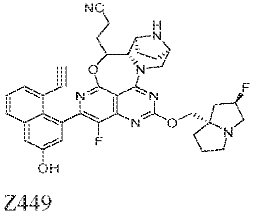 | 664.3 | 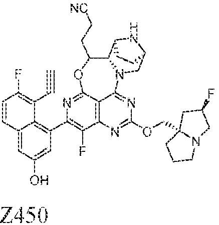 | 682.3 |
| 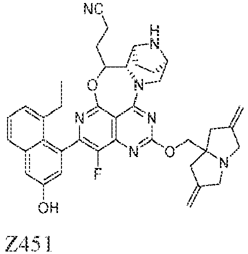 | 674.3 | 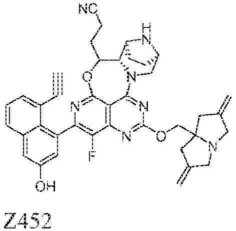 | 670.3 | 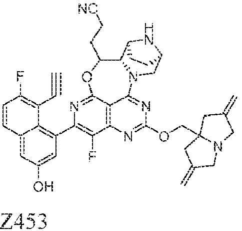 | 668.3 |
| 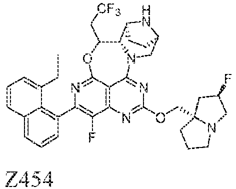 | 681.3 | 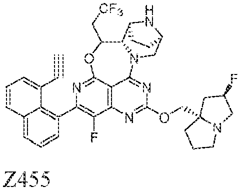 | 677.3 | 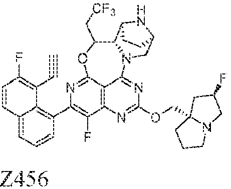 | 695.2 |
| 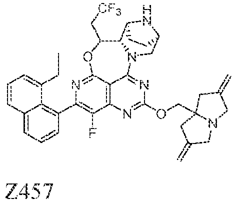 | 687.3 | 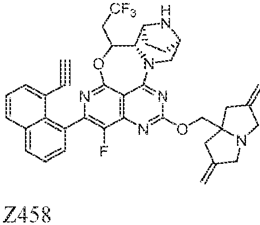 | 683.3 | 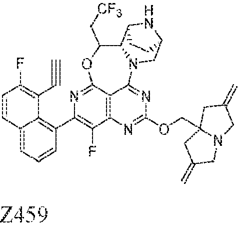 | 701.3 |
| 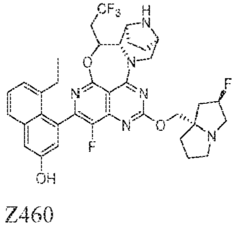 | 597.3 | 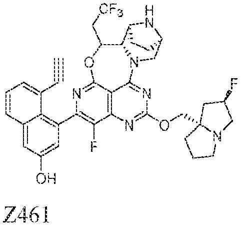 | 693.3 | 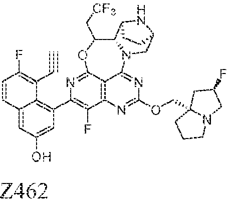 | 711.2 |
| 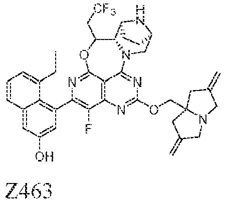 | 703.3 | 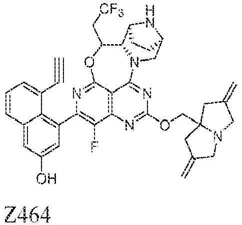 | 699.3 | 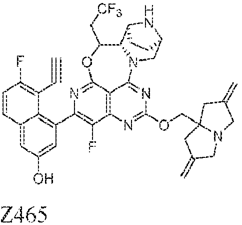 | 717.3 |
| 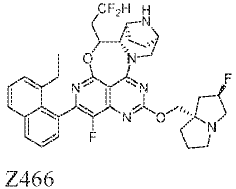 | 663.3 | 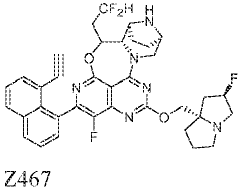 | 659.3 | 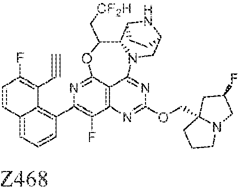 | 577.3 |
| 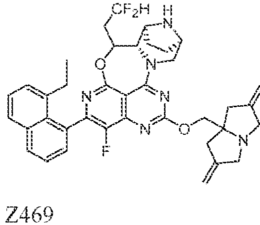 | 669.3 | 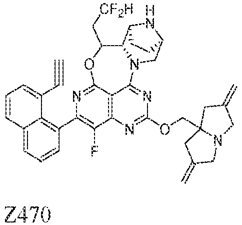 | 665.3 | 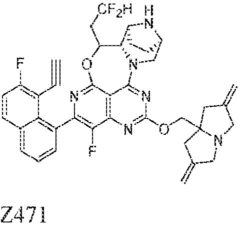 | 583.3 |
| 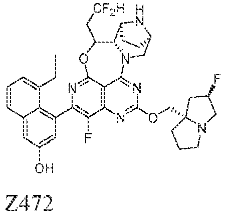 | 679.3 | 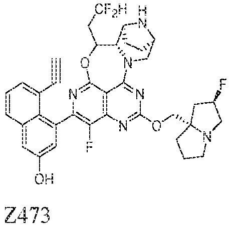 | 675.3 | 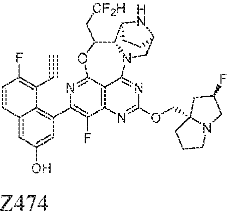 | 693.3 |
| 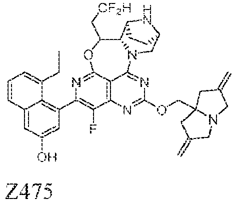 | 685.3 | 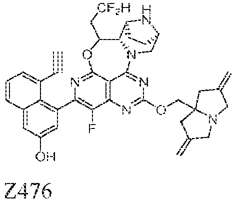 | 681.3 | 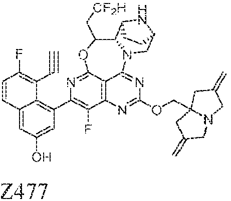 | 699.3 |
| 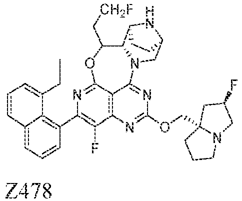 | 645.3 | 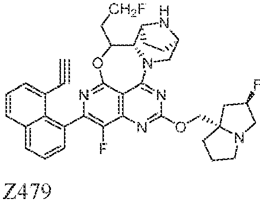 | 641.3 | 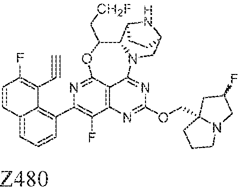 | 559.3 |
| 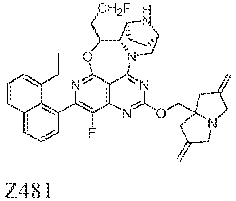 | 651.3 | 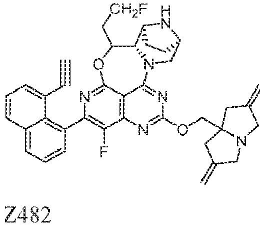 | 647.3 | 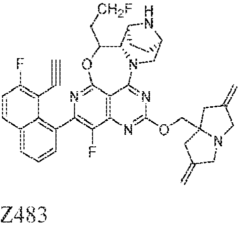 | 665.3 |
| 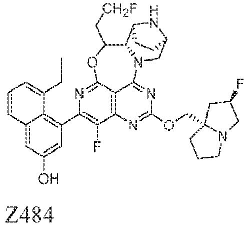 | 561.3 | 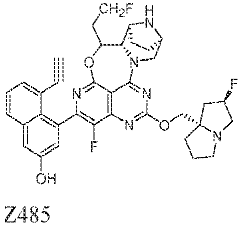 | 557.3 | 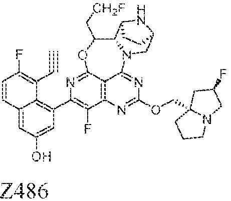 | 575.3 |
| 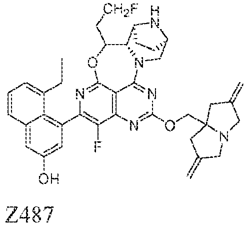 | 667.3 | 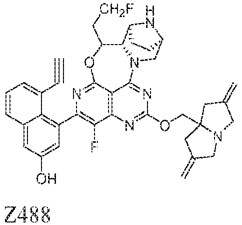 | 563.3 | 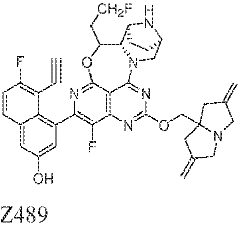 | 681.3 |
| 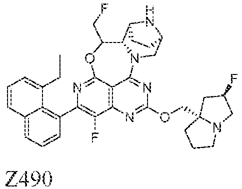 | 631.3 | 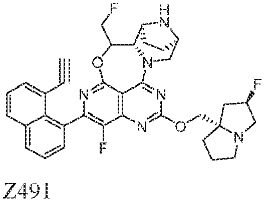 | 627.3 | 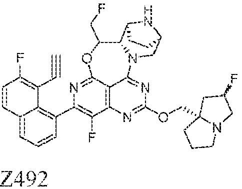 | 545.3 |
| 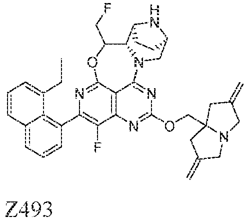 | 537.3 | 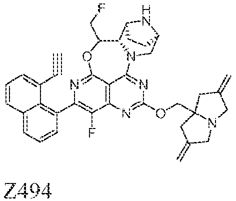 | 533.3 | 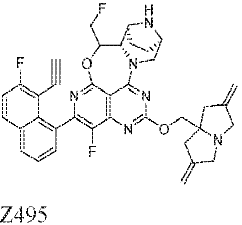 | 651.3 |
| 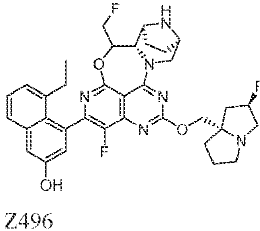 | 547.3 | 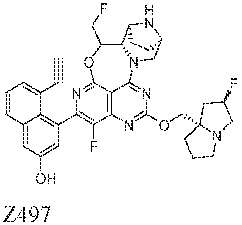 | 543.3 | 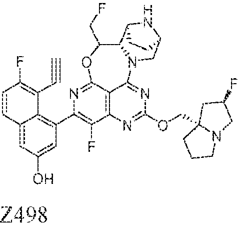 | 561.3 |
| 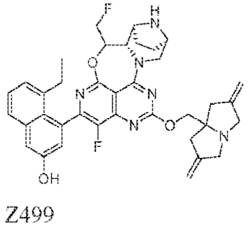 | 553.3 | 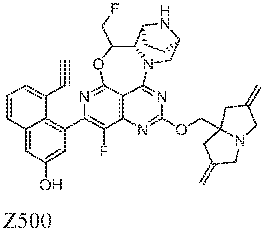 | 549.3 | 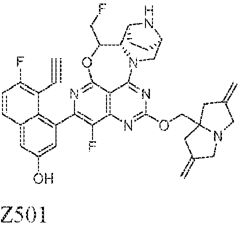 | 567.3 |
| 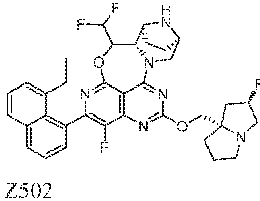 | 649.3 | 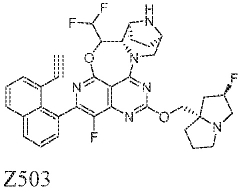 | 645.3 | 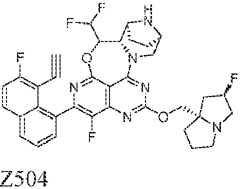 | 563.2 |
| 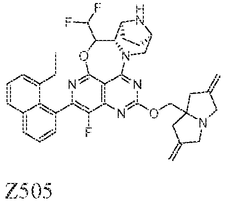 | 655.3 | 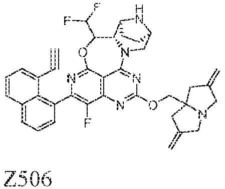 | 651.3 | 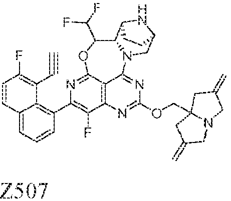 | 569.3 |
| 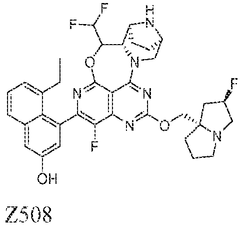 | 665.3 | 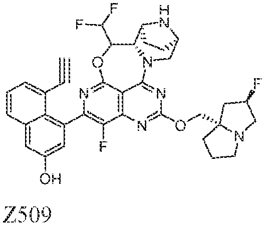 | 561.2 | 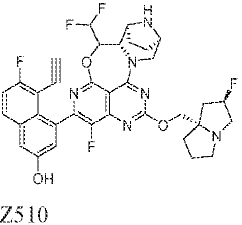 | 579.2 |
| 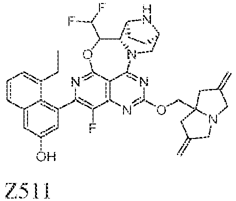 | 671.3 | 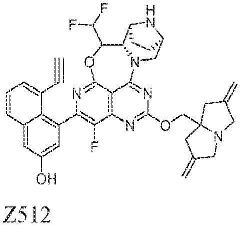 | 567.3 | 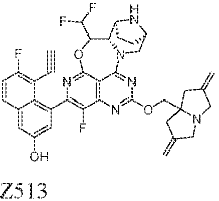 | 685.2 |
| 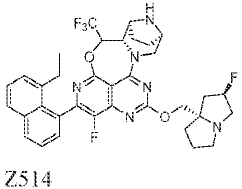 | 667.3 | 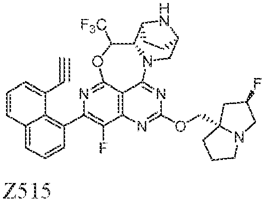 | 663.2 | 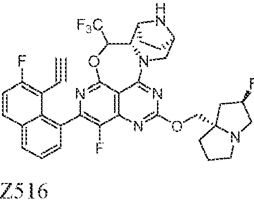 | 581.2 |
| 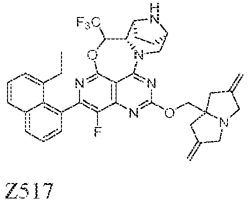 | 673.3 | 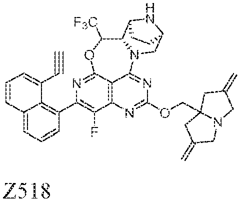 | 569.3 | 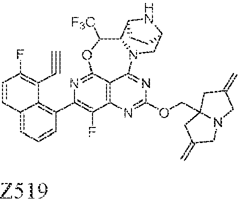 | 687.2 |
| 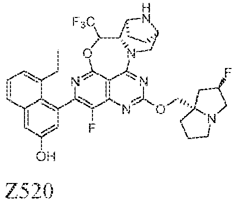 | 683.3 | 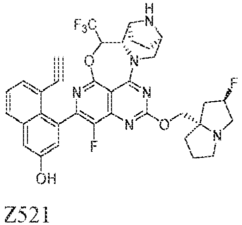 | 579.2 | 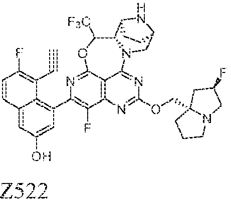 | 697.2 |
| 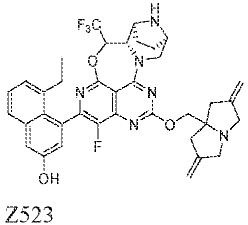 | 689.3 | 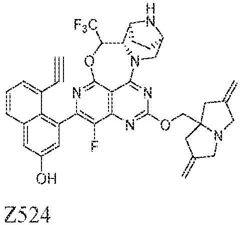 | 585.2 | 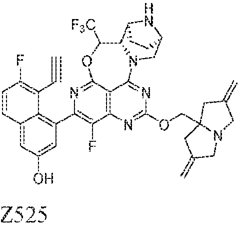 | 703.2 |
| 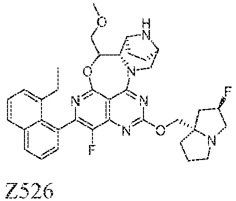 | 643.3 | 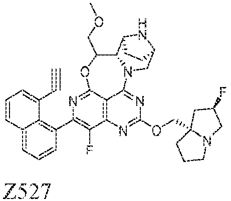 | 639.3 | 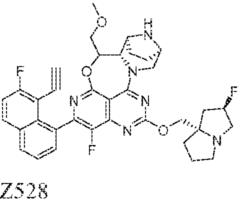 | 557.3 |
| 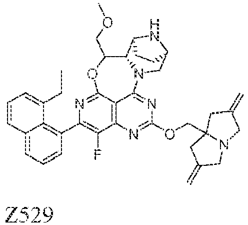 | 649.3 | 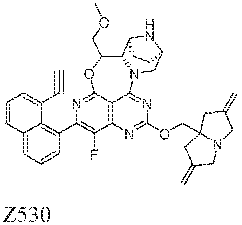 | 545.3 | 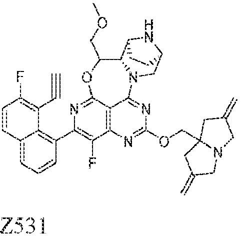 | 663.3 |
| 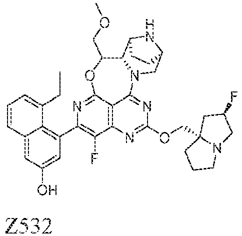 | 659.3 | 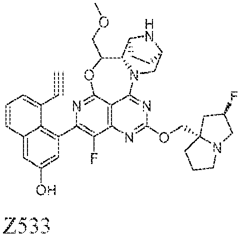 | 655.3 | 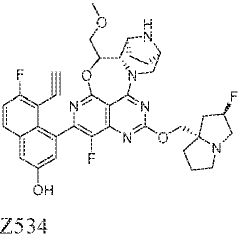 | 573.3 |
| 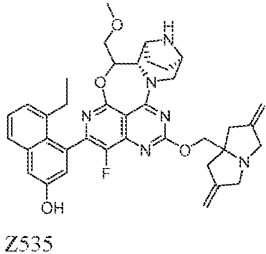 | 665.3 | 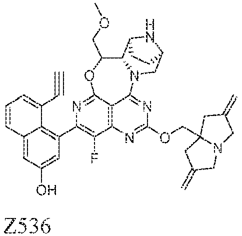 | 661.3 | 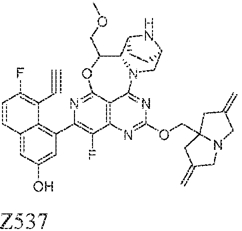 | 579.3 |
| 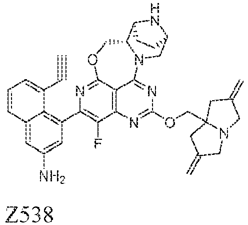 | 516.3 | 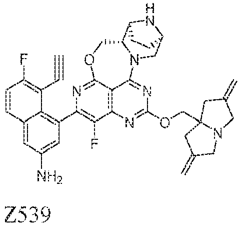 | 534.3 | 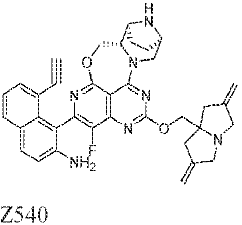 | 516.3 |
| 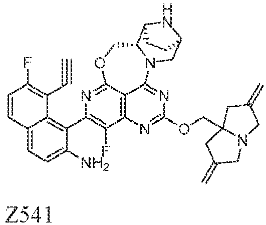 | 543.3 | 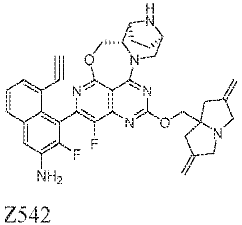 | 534.3 | 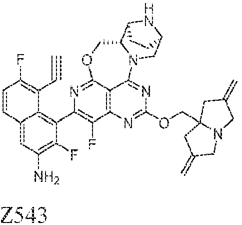 | 652.3 |
| 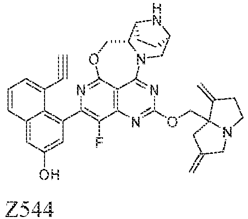 | 517.3 | 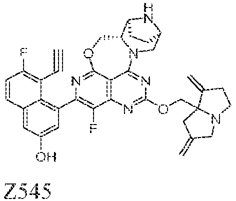 | 534.3 | 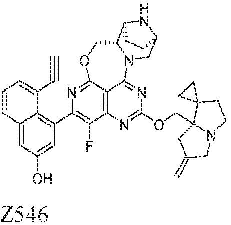 | 631.3 |
| 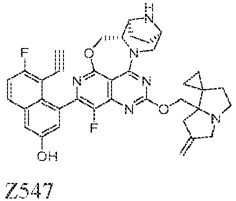 | 549.3 | 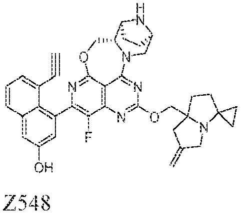 | 631.3 | 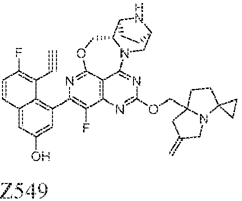 | 549.3 |
| 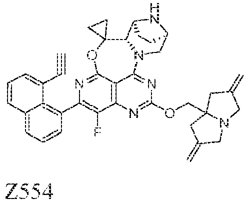 | 527.3 | 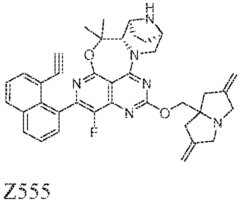 | 629.3 | 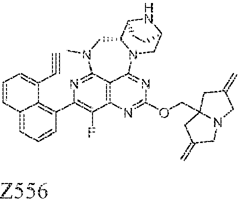 | 514.3 |
| 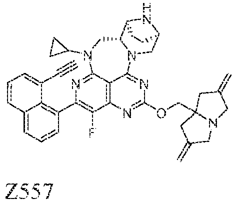 | 540.3 | 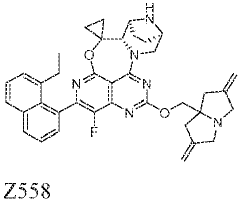 | 531.3 | 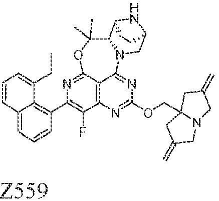 | 533.3 |
| 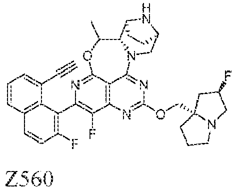 | 527.3 | 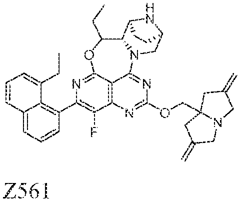 | 633.3 | 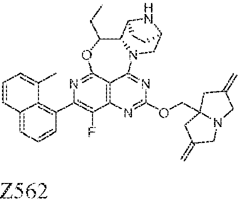 | 519.3 |
| 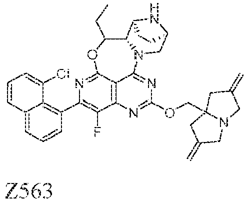 | 639.3 | 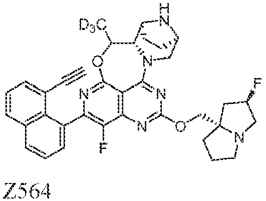 | 612.3 | 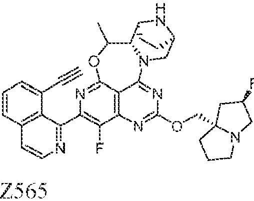 | 610.3 |
| 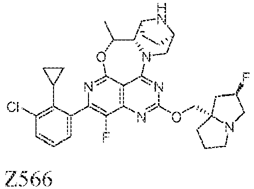 | 509.2 | 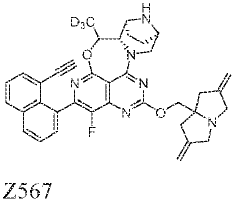 | 618.3 | 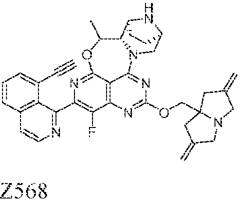 | 616.3 |
| 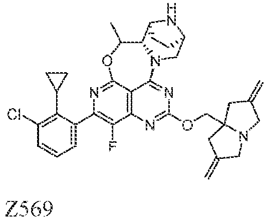 | 515.3 | 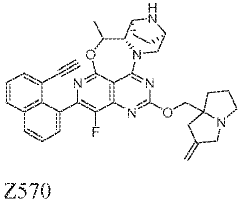 | 603.3 | 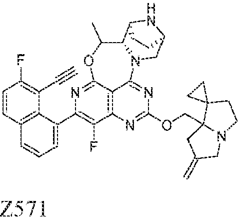 | 647.3 |
| 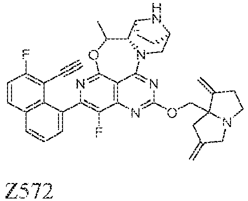 | 533.3 | 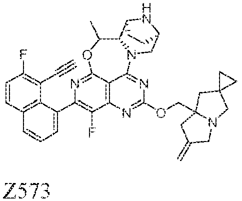 | 647.3 | 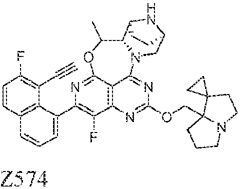 | 635.3 |
| 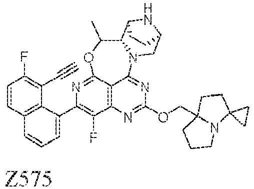 | 535.3 | 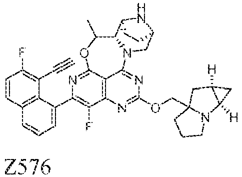 | 621.3 | 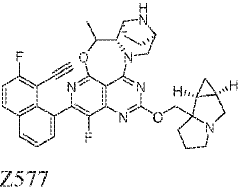 | 621.3 |
| 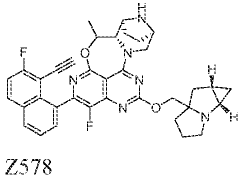 | 521.3 | 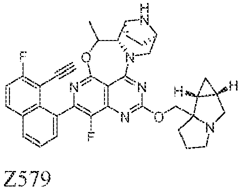 | 521.3 | 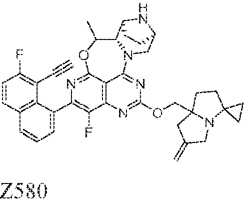 | 647.3 |
| 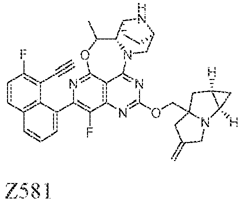 | 533.3 | 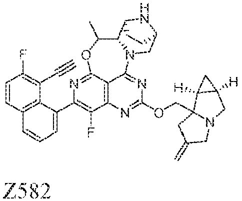 | 633.3 | 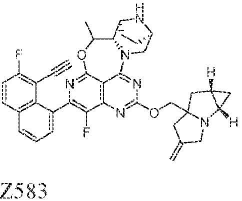 | 633.3 |
| 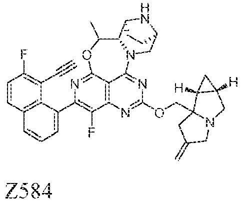 | 533.3 | 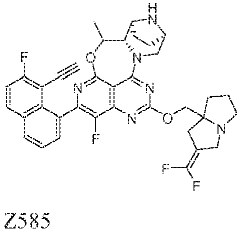 | 657.3 | 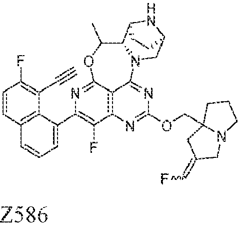 | 539.3 |
| 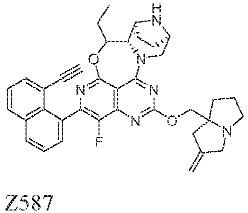 | 617.3 | 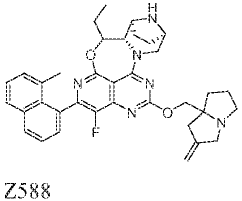 | 607.3 | 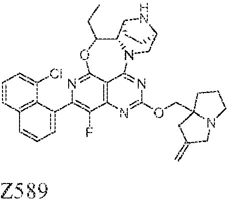 | 527.3 |
| 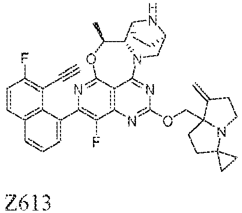 | 547.3 | 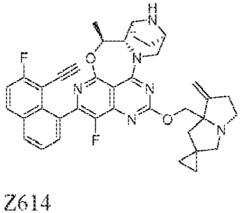 | 647.3 | 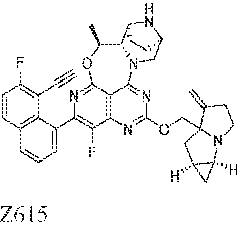 | 633.3 |
| 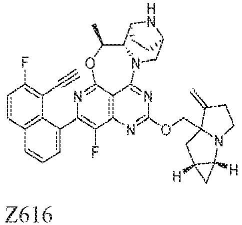 | 533.3 | 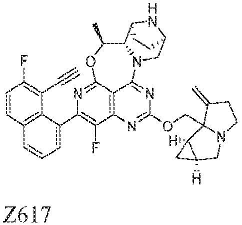 | 533.3 | 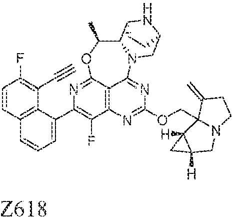 | 633.3 |
| 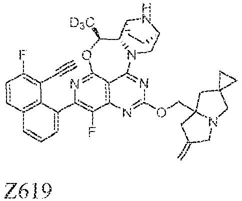 | 650.3 | 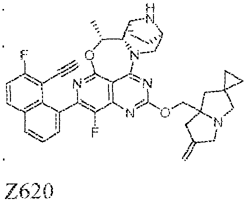 | 647.3 | 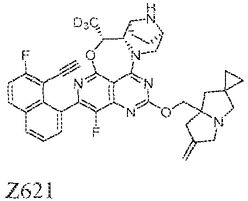 | 550.3 |
| 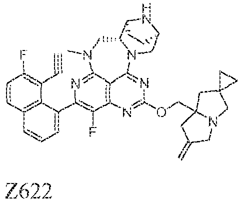 | 646.3 | 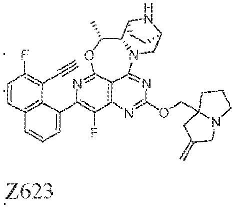 | 621.3 | 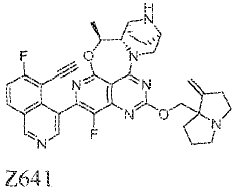 | 622.3 |
| 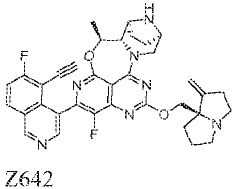 | 622.3 | 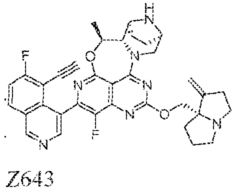 | 522.3 | 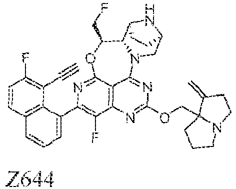 | 639.3 |
| 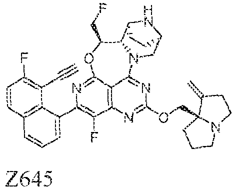 | 639.3 | 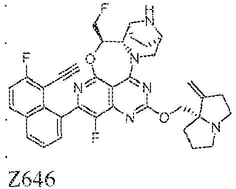 | 639.3 | 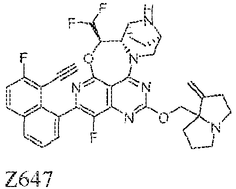 | 657.3 |
| 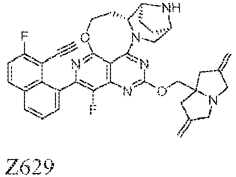 | 533.3 | 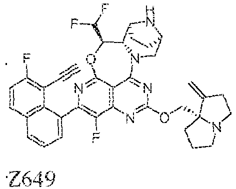 | 657.3 | 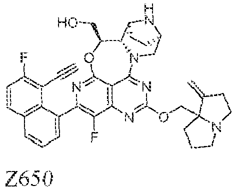 | 637.3 |
| 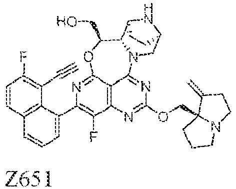 | 637.3 | 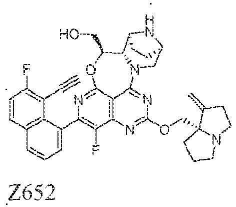 | 637.3 | 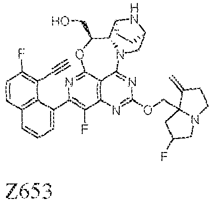 | 655.3 |
| 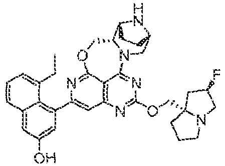 | 597.3 | 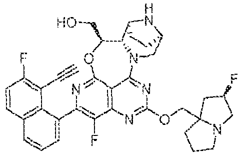 | 543.3 | 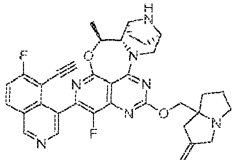 | 622.3 |
| 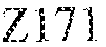 | | 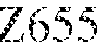 | | 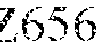 | |
| 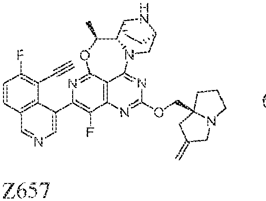 | 622.3 | 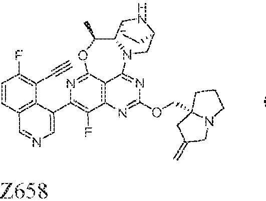 | 522.3 | 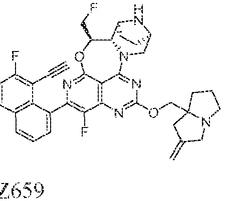 | 639.3 |
| 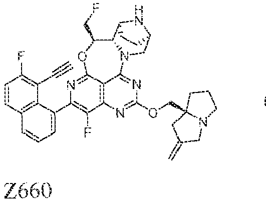 | 539.3 | 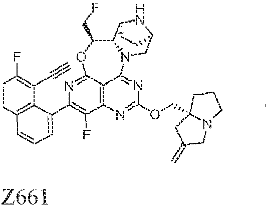 | 539.3 | 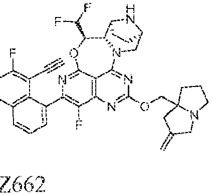 | 657.3 |
| 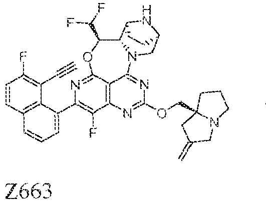 | 557.3 | 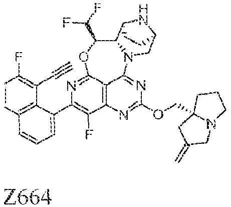 | 557.3 | 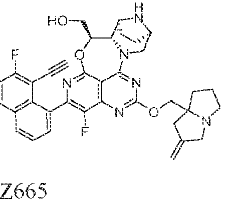 | 637.3 |
| 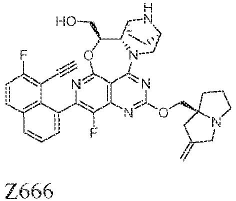 | 537.3 | 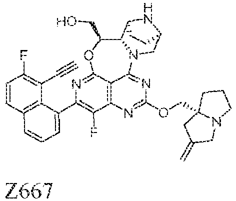 | 637.3 | 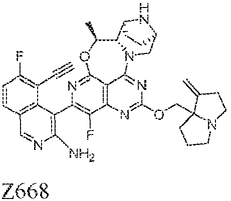 | 637.3 |
| 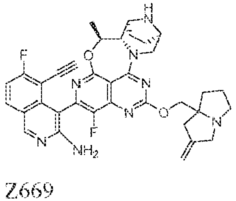 | 637.3 | 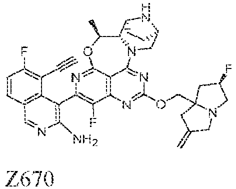 | 655.3 | 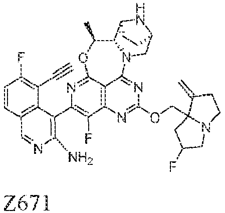 | 555.3 |
| 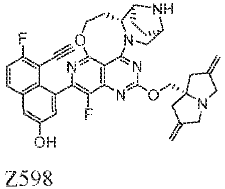 | 549.3 | | | | |

### Test Example 1: Cell p-ERK Assay

AGS was purchased from ATCC; catalog No: CRL-1739; F12K was purchased from Gibco; catalog No: 21127-022; FBS was purchased from Gibco; catalog No: 10099-141C; trypsin (containing EDTA) was purchased from Gibco; catalog No: 25200-072; DMSO was purchased from VWRAMRESCO; catalog No: 0231-500ML; 96-well cell culture plates were purchased from Coming; catalog No: 3599; white opaque 96-well plates were purchased from Cisbio; catalog No: 66PL96025; Advance ERK phospho-T202/Y204 kit was purchased from Cisbio; catalog No: 64AERPEH; cell counters were purchased from CHEMOMETEC; model No: NC-200; microplate reader was purchased from PerkinElmer; model No: Envison.

AGS is an endogenous human gastric adenocarcinoma cell line containing the KRAS^{G12D} mutation, cultured in F12K+10% FBS medium. On the first day of the experiment, cells in the logarithmic growth phase were taken, digested with trypsin (containing EDTA), collected, and counted. Approximately 20000 cells/well were seeded in a 96-well cell culture plate and cultured overnight in an incubator with 5% COz. 1000X stock solutions of compounds at a 3.16-fold concentration gradient were prepared using DMSO, and diluted 200-fold into a 5X compound stock solution using the medium described above. The 5X compound stock solution was added to each cell culturing well the day after cell seeding at a final concentration of 1X and a DMSO content of 0.1%. DMSO was used as a control in the experiment. After the compound was added and the incubation was kept for 3 hours, the medium in the culture wells was removed. 50 µl of Cell lysis solution (from Advance ERK phospho-T202/Y204 kit, the same below) was added to each well, mixed well, and incubated for 30 minutes and then 16 µl of the mixed solution was transferred to a white opaque 96-well plate, another 16 µl of cell lysis solution was added to a well as a blank well. After transferring, 4 µl of p-ERK HTRF antibody mixture (from Advance ERK phospho-T202/Y204 kit) was added to each well. After incubation for 4 hours, HTRF fluorescence value (RLU) was read by a microplate reader, and p-ERK level inhibition rate (IR(%)=(RLU control-RLU compound)/(RLU control-RLU blank)×100%) of the compounds was calculated. The concentration of the compounds and corresponding cell p-ERK level inhibition rate were fitted using Prism 8 four-parameters method, and the IC₅₀ value was calculated. The results showed that the compounds of the present disclosure have high inhibitory activity on p-ERK. The inhibitory activity IC₅₀ of some compounds is less than 1 µM, or some compounds have an IC₅₀ of less than 500 nM, even less than 100 nM or 50 nM. The results of some exemplary compounds are shown in Table 1.

**Table 1**

| Number | AGS p-ERK IC₅₀ (nM) | Number | AGS p-ERK IC₅₀ (nM) |
|---|---|---|---|
| Z2 | 10.65 | Z131-1 | 1.58 |
| Z384 | 0.65 | Z360-1 | 29.95 |
| Z140-1 | 2.85 | Z386 | 0.76 |
| Z350 | 46.98 | Z387 | 50.21 |
| Z380 | 0.78 | Z388 | 17.87 |
| Z381 | 435.88 | Z550 | 91.16 |
| Z552 | 0.67 | Z551 | 61.04 |
| Z553 | 0.48 | Z590 | 7.11 |
| Z593 | 28.97 | Z595 | 118.62 |
| Z597 | 48.52 | Z599 | 27.58 |
| Z600 | 18.29 | Z624 | 47.29 |
| Z625 | 81.86 | Z626-2 | 20.64 |
| Z601 | 10.49 | Z627-2 | 59.26 |
| Z628 | 45.48 | Z626-1 | 411.02 |
| Z605 | 553.75 | Z608 | 28.40 |
| Z604 | 95.01 | Z603 | 64.10 |
| Z602 | 34.12 | Z607 | 24.44 |
| Z630 | 728.71 | Z606 | 874.36 |
| Z631 | 91.90 | Z612 | 99.29 |
| Z611 | 19.57 | Z632 | 30.42 |
| Z610 | 17.24 | Z633 | 14.48 |
| Z635 | 49.97 | Z636 | 10.49 |
| Z637 | 48.75 | Z638 | 7.79 |
| Z639 | 33.44 | Z640 | 65.54 |
| Z609 | 49.23 | Z673 | 146.56 |
| Z674 | 10.70 | Z675 | 11.12 |
| Z676 | 69.99 | Z677 | 185.01 |
| Z678 | 12.44 | Z679 | 117.06 |
| Z680 | 142.56 | Z681 | 5.73 |
| Z682 | 82.05 | Z683 | 5.84 |
| Z684 | 57.52 | Z685 | 30.28 |
| Z686 | 446.58 | Z687 | 192.57 |
| Z688 | 8.77 | Z689 | 6.61 |
| Z691 | 14.23 | Z692 | 187.53 |
| Z693 | 9.23 | Z698 | 21.69 |
| Z654 | 135.88 | Z690 | 16.70 |
| Z695 | 4.71 | Z696 | 72.63 |
| Z699 | 0.44 | Z700 | 0.39 |
| Z701 | 39.48 | Z702 | 11.06 |
| Z703 | 1.05 | Z704 | 542.28 |
| Z705 | 194.79 | Z706 | 6.64 |
| Z707 | 0.95 | Z708 | 38.55 |
| Z709 | 12.24 | Z710 | 0.34 |
| Z711 | 13.30 | Z718 | 157.35 |
| Z712 | 28.18 | Z713 | 22.03 |
| Z714 | 621.69 | Z715 | 1.94 |
| Z716 | 81.84 | Z717 | 14.80 |
| Z719 | 1.16 | Z720 | 35.09 |
| Z721 | 37.51 | Z722 | 7.22 |
| Z723 | 6.96 | Z724 | 45.34 |
| Z725 | 42.33 | Z726 | 78.84 |
| Z727 | 5.01 | Z728 | 0.95 |
| Z729 | 212.17 | Z730 | 0.54 |
| Z731 | 1.60 | Z732 | 2.88 |
| Z733 | 76.04 | Z598 | 0.68 |
| Z629 | 50.02 | Z648 | 14.62 |
| Z734 | 3.42 | Z735 | 5.62 |

### Test Example 2: Cell Proliferation Inhibition Assay

AsPC-1 was purchased from ATCC; catalog No: CRL-1682; AGS was purchased from ATCC; catalog No: CRL-1739; RPMI1640 was purchased from Gibco; catalog No: 11875-093 2235123; F12K was purchased from Gibco; catalog No: 21127-022; FBS was purchased from Gibco; catalog No: 10099-141C; trypsin (containing EDTA) was purchased from Gibco; catalog No: 25200-072; CellTiter Glo-3D was purchased from Progema; catalog No: G9683; 384-well sphere plates were purchased from Coming; catalog No: 3830; white opaque 384-well plates were purchased from Coming; catalog No: 3570; cell counters were purchased from CHEMOMETEC; model: NC-200; microplate reader was purchased from PerkinElmer; model: Envison;
AsPC-1 is an endogenous human pancreatic cancer cell containing KRAS*^{G12D}* mutation, cultured in RPMI-1640 medium containing 10% FBS; AGS is a human gastric cancer cell line containing KRAS*^{G12D}*, cultured in F-12K medium containing 10% FBS. Cells in the logarithmic growth phase were taken, digested with trypsin (containing EDTA), collected, and counted. 800 AsPC-1 cells/well or 400 AGS cells/well, respectively, were seeded in a 384-well sphere plate and cultured overnight at 5% COz to establish a 3D cell model. 1000X stock solutions of compounds at a 3.16-fold concentration gradient of were prepared using DMSO, diluted 100-fold into a 10X compound stock solution using the medium described above. The 10X compound stock solution was added to each cell on 2nd day after cell seeding at a final concentration of 1X and a DMSO content of 0.1%. DMSO was used as a control in the experiment, and the medium was used as blank. After the addition of the compounds, the cells were incubated for 5 days. 30 µl of Cellliter-Glo work solution was added to each well, mixed well, and incubated for 30 minutes. After standing at room temperature for 30 minutes, 40 µl of the mixed solution was transferred to an opaque 384-well plate with a white clear bottom. Luminescence chemiluminescence value was read by a microplate reader, and the cell proliferation inhibition rate (IR(%)=(RLU control-RLU compound)/(RLU control-RLU blank)×100%) was calculated. The gradiently diluted concentration of the compounds and corresponding cell proliferation inhibition rate were fitted using XLFit four-parameters method, and the IC₅₀ value was calculated. The results showed that the compounds of the present disclosure have high inhibitory activity on cells having KRAS*^{G12D}* mutantion. The inhibitory activity IC₅₀ of some compounds is less than 1000 nM; or an IC₅₀ of less than 500 nM; and even less than 100 nM or 50 nM. The results of some exemplary compounds are shown in Table 2 ("-" represents untested).

**Table 2**

| Number | AGS IC₅₀ (nM) | AsPC-1 IC₅₀ (nM) | Number | AGS IC₅₀ (nM) | AsPC-1 IC₅₀ (nM) |
|---|---|---|---|---|---|
| | | | | | |
| Z2 | 10.35 | 127.31 | Z384 | 1.50 | 7.34 |
| Z388 | 53.1 | 191.2 | Z386 | 2.40 | 14.70 |
| Z380 | 2.68 | 26.16 | Z550 | 108.68 | 393.62 |
| Z360-1 | 27.8 | 36.4 | Z551 | 45.38 | 169.72 |
| Z552 | 0.53 | 5.77 | Z553 | 0.53 | 9.13 |
| Z590 | 17.31 | 113.54 | Z592 | 354.25 | - |
| Z387 | 73.28 | 201.91 | Z593 | 35.35 | 78.84 |
| Z595 | 99.01 | 157.29 | Z596 | 268.28 | 411.24 |
| Z599 | 10.76 | 22.31 | Z597 | 47.86 | 98.73 |
| Z624 | 88.23 | 194.07 | Z600 | 15.32 | 64.84 |
| Z626-2 | 28.19 | 56.58 | Z625 | 107.06 | 404.55 |
| Z627-2 | 83.42 | 286.81 | Z601 | 13.52 | 39.00 |
| Z626-1 | 502.15 | - | Z628 | 39.97 | 144.17 |
| Z602 | 26.54 | 90.36 | Z608 | 82.26 | 145.57 |
| Z606 | 907.6 | 802.34 | Z604 | 573.15 | 468.39 |
| Z612 | 65.41 | 124.21 | Z607 | 81.18 | 101.07 |
| Z632 | 26.16 | 53.83 | Z631 | 201.17 | 254.15 |
| Z633 | 59.01 | 56.73 | Z611 | 10.38 | 49.12 |
| Z635 | 223.59 | 218.24 | Z610 | 18.58 | 42.34 |
| Z637 | 67.59 | 111.81 | Z634 | - | 882.73 |
| Z639 | 112.61 | 93.66 | Z636 | 10.87 | 30.59 |
| Z638 | 19.71 | 32.01 | Z603 | 110.21 | 216.73 |
| Z674 | 108.26 | 475.07 | Z675 | 31.20 | 31.20 |
| Z676 | 266.67 | - | Z677 | 794.94 | 981.41 |
| Z678 | 100.5 | 73.48 | Z679 | 393.33 | 352.08 |
| Z680 | 385.92 | - | Z681 | 13.00 | 37.90 |
| Z682 | 145.71 | 236.94 | Z683 | 22.70 | 79.00 |
| Z684 | 340.62 | 326.14 | Z685 | 145.20 | 257.82 |
| Z698 | 18.76 | 49.29 | Z687 | 392.19 | 513.32 |
| Z688 | 19.96 | 39.19 | Z689 | 19.29 | 17.42 |
| Z691 | 54.60 | 87.94 | Z692 | 378.21 | 289.51 |
| Z693 | 27.29 | 31.12 | Z694 | 396.7 | 405.39 |
| Z654 | 725.87 | 388.84 | Z690 | 31.51 | 45.92 |
| Z695 | 36.14 | 77.67 | Z696 | 598.15 | 694.49 |
| Z699 | 1.98 | 2.37 | Z702 | 26.8 | 51.9 |
| Z700 | 1.91 | 2.14 | Z701 | 230.46 | 245.38 |
| Z703 | 2.64 | 1.78 | Z704 | - | 687.84 |
| Z706 | 25.99 | 37.87 | Z707 | 7.44 | 5.24 |
| Z708 | 175.87 | 940.69 | Z709 | 86.75 | 117.46 |
| Z710 | 4.26 | 3.72 | Z140-1 | 4.00 | 21.11 |
| Z131-1 | 2.53 | 32.8 | Z712 | 60.68 | 30.39 |
| Z713 | 73.54 | 55.59 | Z714 | 672.92 | 814.88 |
| Z715 | 3.44 | 3.83 | Z716 | 48.59 | 89.15 |
| Z717 | 29.11 | 50.18 | Z718 | 455.23 | 429.35 |
| Z719 | 1.03 | 3.00 | Z720 | 15.71 | 33.37 |
| Z721 | 43.60 | 105.26 | Z722 | 12.77 | 9.29 |
| Z723 | 15.10 | 55.40 | Z724 | 92.00 | 195.78 |
| Z725 | 62.70 | 105.80 | Z726 | 133.36 | 637.76 |
| Z727 | 9.30 | 12.35 | Z728 | 3.75 | 5.41 |
| Z729 | 584.08 | 757.02 | Z730 | 1.66 | 3.61 |
| Z731 | 5.70 | 15.76 | Z732 | 10.77 | 11.83 |
| Z733 | 296.69 | 267.29 | Z734 | 17.17 | 12.14 |
| Z735 | 35.23 | 18.14 | Z598 | 1.23 | 5.55 |
| Z629 | 51.69 | 166.87 | Z711 | 54.16 | 85.58 |
| Z648 | 39.13 | 53.51 | | | |

While particular embodiments of the present disclosure have been described in detail, it will be apparent to those skilled in the art that various modifications and alternatives to those details could be made in light of the teachings of the present disclosure, and shall fall in the protection scope of the present disclosure. The full scope of the present disclosure is given by the appended claims and any equivalents thereof.

## Claims

1. A compound of formula (A), or a pharmaceutically acceptable salt, a stereoisomer or a solvate thereof: 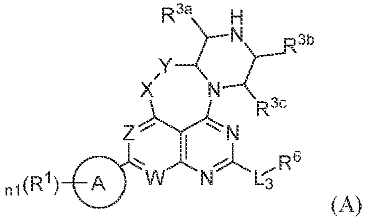 in the formula,
X is O, S or NR¹¹; wherein R¹¹ is selected from H, C1-C6 alkyl, C1-C6 deuterated alkyl and cycloalkyl;
Y is CR¹²R¹³, CR¹⁴R¹⁵CR¹⁶R¹⁷, C(O) or C(O)CR¹⁸R¹⁹; wherein
R¹², R¹³ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; or R¹², R¹³ are taken together with the carbon atoms to which R¹², R¹³ are attached to form a cycloalkyl;
R¹⁴ and R¹⁵ are each independently selected from H, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²; or R¹⁴, R¹⁵ are taken together with the carbon atoms to which R¹⁴, R¹⁵ are attached to form a cycloalkyl;
R¹⁶, R¹⁷ are each independently selected from H, halogen, hydroxyl, nitro, cyano, C1-C6 alkyl, C1-C6 deuterated alkyl, C1-C6 halogenated alkyl, C1-C6 alkoxy, C1-C6 halogenated alkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 halogenated alkoxy, -C1-C4 alkyl-hydroxyl, -C1-C4 alkyl-cyano, -C1-C4 alkyl-NR¹²¹R¹²²;
R¹⁸, R¹⁹ are each independently selected from H, C1-C3 alkyl and halogen;
R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl;
wherein the cycloalkyl, the heterocyclyl, the aryl, the heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy;
W is N or CR²⁰; wherein R²⁰ is H, cyano, C1-C6 alkyl, halogen, C1-C6 halogenated alkyl, C1-C6 alkoxy, cycloalkyl, cycloalkyl-O-, heterocyclyl or heterocyclyl-O-; wherein the cycloalkyl, the heterocyclyl are independently and optionally substituted by halogen;
ring A is selected from the group: aryl and heteroaryl;
R¹ is a substituent at any position on ring A; n1 is 0, 1, 2, 3, 4 or 5;
each R¹ is respectively and independently selected from: C1-C6 alkyl, halogen, hydroxyl, cyano, C1-C6 alkoxy, C1-C6 halogenated alkyl, C1-C6 halogenated alkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxy) C1-C6 halogenated alkyl- or cycloalkyl; wherein the cycloalkyl is optionally substituted by halogen or C1-C6 alkyl; wherein,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C6 alkyl or C1-C6 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; wherein R²⁵¹, R²⁵², R²⁵³, R²⁵⁴ are each independently H or C1-C6 alkyl; or R²⁵¹, R²⁵² are taken together with the nitrogen atom to which R²¹¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C6 alkyl, C1-C6 halogenated alkyl or NR²⁶¹R²⁶², wherein R²⁶¹, R²⁶² are each independently H or C1-C6 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
Z is N or CR²; wherein R² is H, cyano, halogen, C1-C6 alkyl, C1-C6 alkoxy, C1-C6 halogenated alkyl, C1-C6 halogenated alkoxy, C1-C6 deuterated alkoxy, NR³¹R³², C2-C4 alkynyl or CH₂OR³³; wherein R³¹, R³² are each independently hydrogen or C1-C6 alkyl; or R³¹, R³² are taken together with the nitrogen atom to which R³¹, R³² are attached to form a 3- to 6-membered nitrogen-containing heterocyclyl; R³³ is hydrogen or C1-C6 alkyl;
R^{3a}, R^{3b}, R^{3c} are each independently hydrogen, halogen, hydroxyl, cyano, C1-C3 alkyl, C1-C3 deuterated alkyl or C1-C3 halogenated alkyl; or
R^{3a} and R^{3b} are connected to form -CHR^{3a1}- or -CHR^{3a2}CHR^{3a3}-; R^{3c} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl; or
R^{3a} and R^{3c} are connected to form -CHR^{3a1}- or -CHR^{3a2}CHR^{3a3}-; R^{3b} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl;
L₁ is a bond, O or NR³⁴;
R⁶ is -L-6- to 10-membered fused heterocyclyl, -L-7- to 11-membered spiro heterocyclyl, -L-spirocyclic ring substituted 6- to 10-membered fused heterocyclyl, -L-spirocyclic ring substituted 7-to 11-membered spiro heterocyclyl, -L-fused ring substituted 6- to 10-membered fused heterocyclyl, -L-fused ring substituted 7- to 11-membered spiro heterocyclyl or -L-spirocyclic and fused ring substituted 6- to 10-membered fused heterocyclyl;
the spirocyclic ring substituted 6- to 10-membered fused heterocyclyl refers to that two hydrogen atoms on the same carbon atom of any one or two carbon atoms on the 6- to 10-membered fused heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 6- to 10-membered fused heterocyclyl; the spirocyclic ring substituted 7- to 11-membered spiro heterocyclyl refers to that two hydrogen atoms on the same carbon atom of any one or two carbon atoms on the 7- to 11-membered spiro heterocyclyl are simultaneously substituted by -(CH₂)ₘ₄-, -(CH₂)ₘ₅O(CH₂)ₘ₆- or - (CH₂)ₘ₇NRⁿ(CH₂)ₘ₈ to form spirocyclic ring substituted 7- to 11-membered spiro heterocyclyl;
the fused ring substituted 6- to 10-membered fused heterocyclyl refers to that one hydrogen atom on each carbon atom on the same carbon atom pair of any one or two pairs of adjacent carbon atoms on the 6- to 10-membered fused heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 6- to 10-membered fused heterocyclyl; or the fused ring substituted 6- to 10-membered fused heterocyclyl refers to that hydrogen atoms on any two non-adjacent carbon atoms on the 6- to 10-membered fused heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 6- to 10-membered fused heterocyclyl;
the fused ring substituted 7- to 11-membered spiro heterocyclyl refers to that one hydrogen atom on each carbon atom on the same carbon atom pair of any one or two pairs of adjacent carbon atoms on the 7- to 11-membered spiro heterocyclyl is simultaneously substituted by -(CH₂)ₘ₄-, - (CH₂)ₘ₅O(CH₂)ₘ₆- or -(CH₂)ₘ₇NRⁿ(CH₂)ₘ₈ to form fused ring substituted 7- to 11-membered spiro heterocyclyl; or the fused ring substituted 7- to 11-membered spiro heterocyclyl refers to that hydrogen atoms on any two non-adjacent carbon atoms on the 7- to 11-membered spiro heterocyclyl are substituted by -(CH₂)ₘ₄-, -(CH₂)ₘ₅O(CH₂)ₘ₆- or -(CH₂)ₘ₇NRⁿ(CH₂)ₘ₈ to form fused ring substituted 7- to 11-membered spiro heterocyclyl;
the spirocyclic and fused ring substituted 6- to 10-membered fused heterocyclyl refers to that one hydrogen atom on each carbon atom on any pair of adjacent carbon atoms on the 6- to 10-membered fused heterocyclyl is simultaneously substituted by -(CH₂)ₘ₄-, -(CH₂)ₘ₅O(CH₂)ₘ₆- or - (CH₂)ₘ₇NRⁿ(CH₂)ₘ₈ to form fused ring substituent, and two hydrogen atoms on the other carbon atom on the 6- to 10-membered fused heterocyclyl are simultaneously substituted by -(CH₂)ₘ₄-, - (CH₂)ₘ₅O(CH₂)ₘ₆- or -(CH₂)ₘ₇NRⁿ(CH₂)ₘ₈ to form spirocyclic ring substituent, thereby to form spirocyclic and fused ring substituted 6- to 10-membered fused heterocyclyl;
the 6- to 10-membered fused heterocycly or the 7- to 11-membered spiro heterocyclyl containing 1, 2, 3 or 4 heteroatoms selected from N(R^{m}), S, S(=O), S(=O)₂, O as ring atoms;
the spirocyclic ring substituted 6- to 10-membered fused heterocyclyl, the spirocyclic ring substituted 7- to 11-membered spiro heterocyclyl, the fused ring substituted 6- to 10-membered fused heterocyclyl, the fused ring substituted 7- to 11 -membered spiro heterocyclyl or the spirocyclic and fused ring substituted 6- to 10-membered fused heterocyclyl each independently containing 1, 2, 3, 4 or 5 heteroatoms selected from N(R^{m}), S, S(=O), S(=O)z, O as ring atoms;
Rⁿ is hydrogen, C1-C4 alkyl, deuterated C1-C4 alkyl, C1-C4 halogenated alkyl, C3-C20 cycloalkyl or 3- to 20-membered heterocyclyl;
R^{m} is absent, hydrogen, C1-C4 alkyl, deuterated C1-C4 alkyl, C1-C4 halogenated alkyl, C3-C20 cycloalkyl or 3- to 20-membered heterocyclyl;
wherein the 6- to 10-membered fused heterocyclyl, the 7- to 11-membered spiro heterocyclyl are each independently saturated or partially unsaturated; when the 6- to 10-membered fused heterocyclyl, the 7- to 11-membered spiro heterocyclyl is partially unsaturated, the ring containing 1 or 2 double bonds;
two hydrogen atoms on the same carbon atom of any one or two carbon atoms on the 6- to 10-membered fused heterocyclyl are optionally and simultaneously substituted by =CR^{2a}R^{2b}; the 6- to 10-membered fused heterocyclyl is also optionally substituted by one or more R^{6a};
two hydrogen atoms on the same carbon atom of any one or two carbon atoms on the 7- to 11-membered spiro heterocyclyl are optionally and simultaneously substituted by =CR^{2a}R^{2b}; the 7- to 11-membered spiro heterocyclyl is also optionally substituted by one or more R^{6a};
wherein R^{2a}, R^{2b} are each independently hydrogen, halogen, cyano, C1-C4 alkyl, C1-C4 halogenated alkyl, C6-C10 aryl or 5- or 6-membered heteroaryl; the C6-C10 aryl or 5- or 6-membered heteroaryl is optionally substituted by 1, 2, 3 groups selected from halogen, cyano, C1-C4 alkyl, C1-C4 alkoxy, C1-C4 halogenated alkyl, C1-C4 halogenated alkoxy; or each pair of R^{2a}, R^{2b} independently are taken together with the carbon atom to which R^{2a}, R^{2b} are attached to form a C3-C6 monocyclic cycloalkyl or a 3- to 6-membered monocyclic heterocyclyl; the C3-C6 monocyclic cycloalkyl or 3-to 6-membered monocyclic heterocyclyl is optionally substituted by 1, 2, or 3 groups selected from halogen, cyano, C1-C4 alkyl, C1-C4 alkoxy, C1-C4 halogenated alkyl, C1-C4 halogenated alkoxy, C6-C10 aryl, 5- or 6-membered monocyclic heteroaryl;
each R^{6a} is respectively and independently halogen, hydroxyl, C1-C6 hydroxyalkyl, C1-C6 alkyl, C2-C6 alkenyl, C2-C6 halogenated alkenyl, C2-C6 alkynyl, C2-C6 halogenated alkynyl, C1-C6 halogenated alkyl, C1-C6 alkoxy, C1-C6 halogenated alkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyloxy CH₂-, -N(R^{6b})2, (C1-C3 alkoxy)C1-C3 alkyl, (C1-C3 alkyl)C(O)-, oxo, (C1-C3 halogenated alkyl)C(O)-, -SO₂F, (C1-C3 alkoxy)Cl-C3 alkoxy, - CH₂OC(O)N(R^{6b})₂, -CH₂NHC(O)OC1-C6 alkyl, -CH₂NHC(O)N(R^{6b})₂, -CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, - OC(O)NH(C1-C3 alkyl)O(C1-C3 alkyl), -OC(O)NH(C1-C3 allcyl)O(C1-C3 alkyl)phenyl(Cl-C3 alkyl)N(CH₃)₂, -OC(O)NH(C1-C3 alkyl)O(Cl-C3 alkyl)phenyl, -OC(O)heterocyclyl, -OC(O)C1-C6 alkyl, -OC(O)C1-C6 halogenated alkyl, -C1-C3 alkyl-OC(O)Cl-C6 alkyl, -C1-C3 alkyl-OC(O)C1-C6 halogenated alkyl, -OC(O)phenyl, -C1-C3 alkyl-OC(O)phenyl or -CH₂ heterocyclyl;
wherein the phenyl in the groups above is optionally substituted by -C(O)H or OH; the heterocyclyl in the -C1-C3 alkyl-heterocyclyl is optionally substituted by oxo; Q is a bond or O; each R^{6b} above is independently hydrogen, C1-C6 alkyl, C1-C6 deuterated alkyl or C1-C6 halogenated alkyl; or two R^{6b} each independently are taken together with the nitrogen atom to which R^{6b} are attached form a 3- to 6-membered nitrogen-containing heterocyclyl, the 3- to 6-membered nitrogen-containing heterocyclyl is optionally substituted by 1 or 2 groups selected from halogen, methyl, trifluoromethyl, methoxy, and trifluoromethoxy; the C3-C6 cycloalkyl is optionally substituted by 1 or 2 C1-C6 alkyl;
each m4 is 2, 3 or 4;
each m5, m6, m7, m8 are each independently 0, 1, 2 or 3; m5 and m6 are not simultaneously 0; m7 and m8 are not simultaneously 0;
or
R⁶ is hydrogen, -N(R³⁴)₂, heterocyclyl, cycloalkyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH or -L-C(O)OC1-C6 alkyl; wherein the heterocyclyl, the cycloalkyl, the aryl in the -L-NR³⁴C(O)-aryl, the heterocyclyl in the - L-heterocyclyl, the cycloalkyl in the -L-cycloalkyl each optionally substituted by one or more R³⁵ or two hydrogen atoms on the same carbon atom are optionally and simultaneously substituted by - CH₂CH₂- or -CH₂CH₂CH₂- to form cyclopropyl or cyclobutyl; the aryl in the -L-aryl, the heteroaryl in the -L-heteroaryl each optionally substituted by one or more R³⁶;
wherein each L is independently a bond, C1-C4 alkylene or heteroaryl; wherein one or two hydrogen atoms on any carbon atom or on any same carbon atom of the C1-C4 alkylene are independently and optionally substituted by deuterium, C1-C6 alkyl, C1-C6 halogenated alkyl, C2-C4 alkenylene or C2-C4 halogenated alkenylene; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, - (CH₂)ₘ₁-NR⁹-(CH₂)ₘ₂- to form a cyclic substituent; m3 is 1 or 2; each ml is respectively and independently 0, 1, 2, or 3; each m2 is respectively and independently 0, 1, 2, or 3; and m1, m2 are not simultaneously 0;
R⁹ is hydrogen, C1-C6 alkyl, C1-C6 deuterated alkyl, C1-C6 halogenated alkyl, C1-C6 alkyl-hydroxyl, C1-C6 alkyl-cyano, C1-C6 alkoxy, C1-C6 alkyl-Cl-C6 alkoxy, C3-C6 monocyclic cycloalkyl, C3-C6 deuterated cycloalkyl or C3-C6 halogenated cycloalkyl;
each R³⁵ is respectively and independently halogen, hydroxyl, C1-C3 alkyl, C2-C4 alkenylene, C2-C4 halogenated alkenylene, C1-C3 halogenated alkyl, C1-C3 alkoxy, C3-C6 cycloalkyl, -C1-C3 alkyl-C1-C3 alkoxy, -C1-C3 alkyl-hydroxyl, heterocyclyl(e.g., 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy)C1-C3 alkyl, (C1-C3 alkyl)C(O), oxo, (C1-C3 halogenated alkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂, - CH₂NHC(O)C1-C6 alkyl, -CH₂(pyrazolyl), -CH₂NHSO₂C1-C6 alkyl, -CH₂OC(O)heterocyclyl, - OC(O)N(R³⁴)₂, -OC(O)NH(C1-C3 alkyl)O(Cl-C3 alkyl), -OC(O)NH(C1-C3 alkyl)O(Cl-C3 alkyl)phenyl(Cl-C3 alkyl)N(CH₃)₂, -OC(O)NH(C1-C3 alkyl)O(Cl-C3 alkyl)phenyl, - OC(O)heterocyclyl(e.g., -OC(O)-3- to 6-membered heterocyclyl) or -CH₂ heterocyclyl(e.g., -CH₂-3- to 6-membered heterocyclyl); wherein, the phenyl in -NHC(O)phenyl or -OC(O)NH(C1-C3alkyl)O(Cl-C3alkyl)phenyl is optionally substituted by -C(O)H or OH; the heterocyclyl in the - CH₂ heterocyclyl is optionally substituted by oxo; the hydrogen atom on -C1-C3 alkyl- is substituted by 1 or 2 groups selected from halogen, deuterium, C1-C3 alkyl, C1-C3 halogenated alkyl;
each R³⁶ is respectively and independently halogen, hydroxyl, HC(O)-, C1-C4 alkyl, C1-C4 alkoxy, C1-C4 halogenated alkyl, C1-C4 hydroxyalkyl or -N(R³⁴)₂;
each R³⁴ is respectively and independently hydrogen, C1-C3 alkyl or C1-C3 halogenated alkyl; or
two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

2. The compound of claim 1, or a pharmaceutically acceptable salt, a stereoisomer or a solvate thereof, wherein the compound is shown in formula (I): 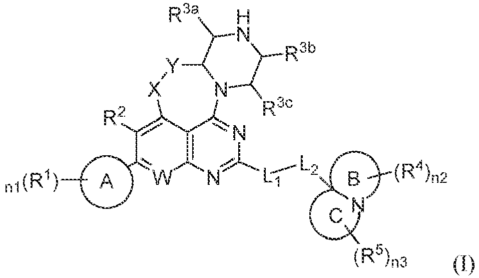 in the formula,
X, Y, W, ring A, R¹, n1, R² are each defined as above;
R^{3a}, R^{3b}, R^{3c} are each independently hydrogen, halogen, hydroxyl, cyano, C1-C3 alkyl, C1-C3 deuterated alkyl or C1-C3 halogenated alkyl;
L₁ is a bond, O or NR³⁴;
L₂ is C1-C4 alkylene or heteroaryl, wherein one or two hydrogen atoms on any carbon atom or on any same carbon atom of the C1-C4 alkylene are independently and optionally substituted by deuterium, C1-C6 alkyl, or C1-C6 halogenated alkyl; or two hydrogen atoms on any same carbon atom of the C1-C4 alkylene are optionally and simultaneously substituted by -(CH₂)ₘ₃-, -(CH₂)ₘ₁-0-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1, m2 are not simultaneously 0;
ring B is a 3- to 6-membered nitrogen-containing heterocyclyl;
ring C is a 3- to 6-membered nitrogen-containing heterocyclyl;
R⁴ is a substituent at any position on ring B; n2 is 0, 1, 2 or 3;
R⁵ is a substituent at any position on ring C; n3 is 0, 1, 2 or 3;
R⁴, R⁵ are defined as follow:
(a) each R⁴, each R⁵ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SOzF, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), - CH₂NHSO₂C1-C6 alkyl, -CH₂OC(O) heterocyclyl, -OC(O)N(R³⁴)₂, -OC(O)NH(C1-C3 alkyl)O(Cl-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(Cl-C3 alkyl)phenyl is optionally substituted by - C(O)H or OH; the heterocyclyl in -CH₂ heterocyclyl is optionally substituted by oxo; or
(b) one R⁴ is -CH₂CH₂- or -CH₂CH₂CH₂-, and the R⁴ are taken together with the carbon atom to which R⁴ is attached to form a cyclopropyl or cyclobutyl; the remaining R⁴, each R⁵ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(Cl-C3 alkyl), - OC(O)NH(C1-C3 alkyl)O(C1-C3 alkyl)phenyl(Cl-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in - NHC(O)phenyl or -OC(O)NH(C1-C3 alkyl)O(Cl-C3 alkyl)phenyl is optionally substituted by - C(O)H or OH; the heterocyclyl in -CHz heterocyclyl is optionally substituted by oxo; or
(c) one R⁵ is -CH₂CH₂- or -CH₂CH₂CH₂-, and the R⁵ are taken together with the carbon atom to which R⁵ is attached to form a cyclopropyl or cyclobutyl; the remaining R⁵, each R⁴ are respectively and independently halogen, hydroxyl, C1-C3 hydroxyalkyl, C1-C3 alkyl, C1-C3 halogenated alkyl, C1-C3 alkoxy, C2-C6 alkenyl, C2-C6 halogenated alkenyl, C2-C4 alkenylene, C2-C4 halogenated alkenylene, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SOzF, (C1-C3 alkoxy) C1-C3 alkoxy, -CH₂OC(O)N(R34)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), - OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂ heterocyclyl; wherein, the phenyl in - NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo; or
(d) one R⁴ is -CH₂CH₂- or -CH₂CH₂CH₂-, and the R⁴ is taken together with the carbon atom to which R⁴ is attached to form a cyclopropyl or cyclobutyl; one R⁵ is -CH₂CH₂- or -CH₂CH₂CH₂-, and the R⁵ is taken together with the carbon atom to which R⁵ is attached to form a cyclopropyl or cyclobutyl; the remaining R⁴, the remaining R⁵ are each independently halogen, hydroxyl, C1-C3 hydroxyalkyl, C1-C3 alkyl, C1-C3 halogenated alkyl, C2-C4 alkenylene, C2-C4 halogenated alkenylene, C1-C3 alkoxy, C2-C6 alkenyl, C2-C6 halogenated alkenyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxy) C1-C3 alkyl, (C1-C3 alkyl)C(O), oxo, (C1-C3 halogenated alkyl)C(O)-, -SO₂F, (C1-C3 alkoxy) C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), - CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(Cl-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O) heterocyclyl or -CH₂ heterocyclyl; wherein, the phenyl in -NHC(O)phenyl or -OC(O)NH(C1-C3 alkyl)O(C1-C3 alkyl)phenyl is optionally substituted by - C(O)H or OH; the heterocyclyl in -CH₂ heterocyclyl is optionally substituted by oxo;
each R³⁴ is respectively and independently hydrogen, C1-C3 alkyl or C1-C3 halogenated alkyl; or two R³⁴ are taken together with the nitrogen atom to which R³⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

3. The compound of claim 1, or a pharmaceutically acceptable salt, a stereoisomer or a solvate thereof, wherein the compound is shown in formula (II): 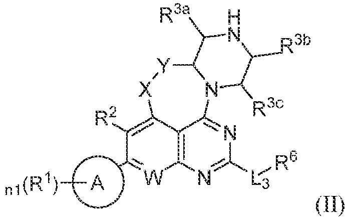 in the formula,
X, Y, W, ring A, R¹, n1, R², L₃, R⁶ are each defined as above;
R^{3a} and R^{3b} are connected to form -CHR^{3a1}- or -CHR^{3a2}CHR^{3a3}-; R^{3c} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl; or
R^{3a} and R^{3c} are connected to form -CHR^{3a1}- or -CHR^{3a2}CHR^{3a3}-; R^{3b} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, C1-C3 alkyl, C1-C3 deuterated alkyl or C1-C3 halogenated alkyl.

4. The compound of claim 1, or a pharmaceutically acceptable salt, a stereoisomer or a solvate thereof, wherein the compound is shown in formula (II-1) or formula (II-2): 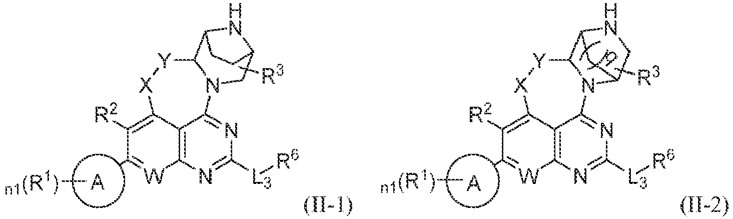 in each formula,
q is 1 or 2;
R³ is a substituent at any position on a bridge ring, and is each independently hydrogen, halogen, hydroxyl, cyano, C1-C3 alkyl, C1-C3 deuterated alkyl or C1-C3 halogenated alkyl;
R¹, n1, ring A, R², X, Y, W, L₃, R⁶ are each defined as above.

5. The compound of claim 1, or a pharmaceutically acceptable salt, a stereoisomer or a solvate thereof, wherein the compound is shown in formula (II-1A), formula (II-1B), formula (II-2A) or formula (II-2B): 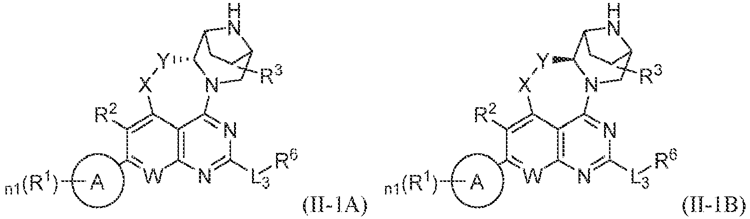 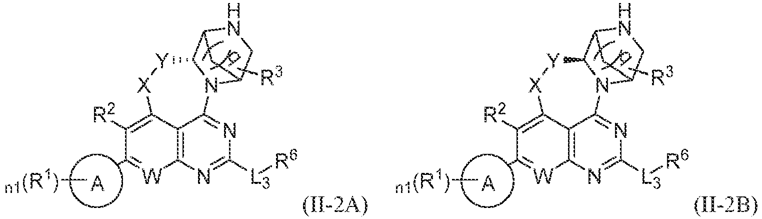 in each formula, X, Y, W, R¹, R², R³, R⁶, n1, L₃, q, ring A are each defined as above.

6. The compound of claim 1, or a pharmaceutically acceptable salt, a stereoisomer or a solvate thereof, wherein the compound is shown in formula (II-IAl), formula (II-1A2), formula (II-1B1), formula (II-1B2), formula (II-1C1) or formula (II-1C2): 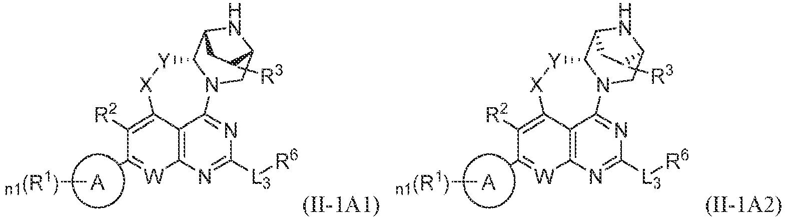 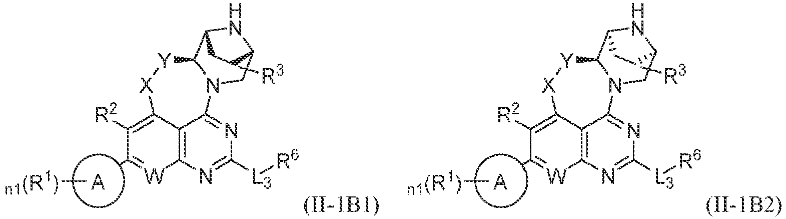 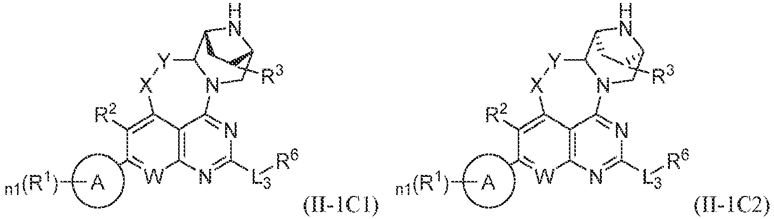 in each formula, X, Y, W, R¹, R², R³, R⁶, n1, L₃, ring A are each defined as above.

7. The compound of claim 1, or a pharmaceutically acceptable salt, a stereoisomer or a solvate thereof, wherein the compound is shown in formula (III): 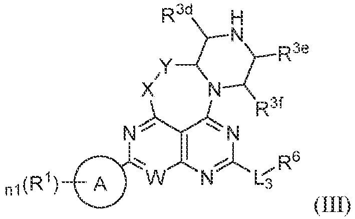 in the formula,
X, Y, W, ring A, R¹, n1, L₃, R⁶ are each defined as above;
R ^{3d}, R^{3e}, R^{3f} are each independently hydrogen, halogen, hydroxyl, cyano, C1-C3 alkyl, C1-C3 deuterated alkyl or C1-C3 halogenated alkyl; or
R^{3d} and R^{3e} are connected to form -CHR^{3d1}- or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3d1}, R^{3d2}, R^{3d3} are each independently hydrogen, halogen, hydroxyl, cyano, C1-C3 alkyl, C1-C3 deuterated alkyl or C1-C3 halogenated alkyl; or
R^{3d} and R^{3f} are connected to form -CHR^{3f1}- or -CHR^{3f2}CHR^{3f3}-; R^{3e} is hydrogen, halogen, hydroxyl, cyano, C1-C3 alkyl, C1-C3 deuterated alkyl or C1-C3 halogenated alkyl; R^{3f1}, R^{3f2}, R^{3f3} are each independently hydrogen, halogen, hydroxyl, cyano, C1-C3 alkyl, C1-C3 deuterated alkyl or C1-C3 halogenated alkyl.

8. The compound of claim 1, or a pharmaceutically acceptable salt, a stereoisomer or a solvate thereof, wherein the compound is shown in formula (III-1a), formula (III-1b), formula (III-2a) or formula (III-2b): 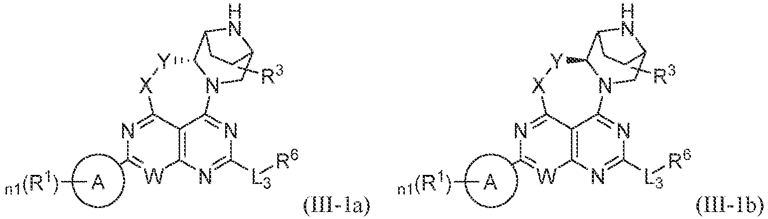 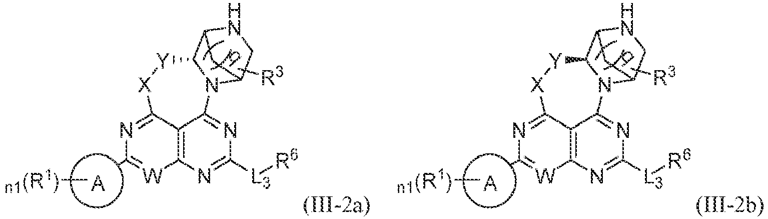 in each formula,
q is 1 or 2;
R³ is a substituent at any position on a bridge ring, and is each independently hydrogen, halogen, hydroxyl, cyano, C1-C3 alkyl, C1-C3 deuterated alkyl or C1-C3 halogenated alkyl;
X, Y, W, ring A, R¹, n1, L₃, R⁶ are each defined as above.

9. The compound of claim 7, or a pharmaceutically acceptable salt, a stereoisomer or a solvate thereof, wherein the compound is shown in formula (III-1a1), formula (III-1a2), formula (III-1b1), formula (III-1b2), formula (III-1c1) or formula (III-1c2): 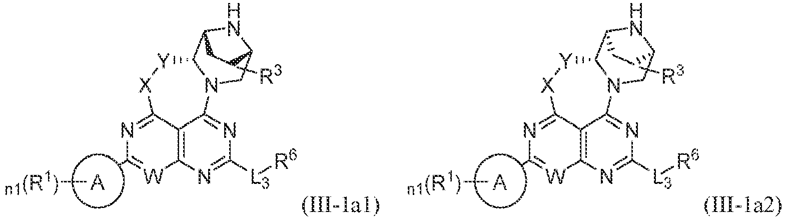 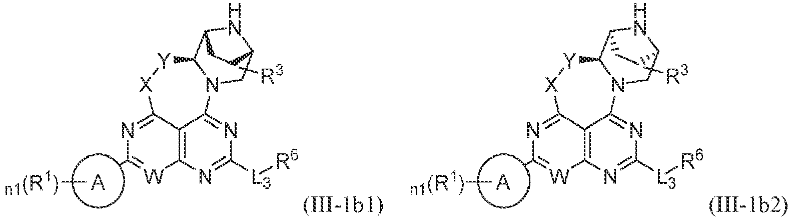 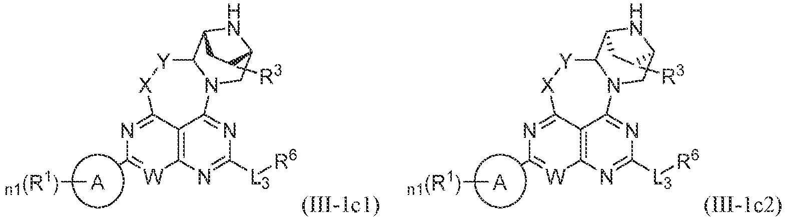 in each formula,
R³ is a substituent at any position on a bridge ring, and is each independently hydrogen, halogen, hydroxyl, cyano, C1-C3 alkyl, C1-C3 deuterated alkyl or C1-C3 halogenated alkyl;
X, Y, W, ring A, R¹, n1, L₃, R⁶ are each independently defined as above.

10. The compound of any of claims 1-9, or a pharmaceutically acceptable salt, a stereoisomer or a solvate thereof, wherein W is N or CR²⁰; wherein R²⁰ is H, cyano, C1-C3 alkyl, halogen, C1-C3 halogenated alkyl, C1-C3 alkoxy, C3-C6 monocyclic cycloalkyl, C3-C6 monocyclic cycloalkyl-O-, 3- to 6-membered heterocyclyl or 3- to 6-membered heterocyclyl-O-; wherein the C3-C6 monocyclic cycloalkyl, the 3- to 6-membered heterocyclyl are independently and optionally substituted by halogen.

11. The compound of any of claims 1-9, or a pharmaceutically acceptable salt, a stereoisomer or a solvate thereof, wherein Y is CR¹²R¹³ or CR¹⁴R¹⁵CR¹⁶R¹⁷; wherein R¹², R¹³, R¹⁴ and R¹⁵ are each independently selected from H, cyano, C1-C6 alkyl, C1-C6 halogenated alkyl, C1-C6 alkoxy, C1-C6 halogenated alkoxy, C3-C6 monocyclic cycloalkyl, C3-C6 monocyclic cycloalkyl-O-, 3- to 6-membered heterocyclyl, 3- to 6-membered heterocyclyl-O-, phenyl, 5- or 6-membered heteroaryl, phenyl-O-, 5- or 6-membered heteroaryl-O-, -C1-C2 alkyl-C1-C3 alkoxy, -C1-C2 alkyl-C1-C3 halogenated alkoxy, -C1-C2 alkyl-hydroxyl, -C1-C2 alkyl-cyano, -C1-C2 alkyl-NR¹²¹R¹²²; R¹⁶, R¹⁷ are each independently selected from H; R¹²¹, R¹²² are each independently selected from H, C1-C3 alkyl; or R¹²¹, R¹²² are taken together with the nitrogen atom to which R¹²¹, R¹²² are attached to form a 3- to 6-membered nitrogen-containing heterocyclyl; wherein the C3-C6 monocyclic cycloalkyl, the 3- to 6-membered heterocyclyl, the phenyl, the 5- or 6-membered heteroaryl, the 3- to 6-membered nitrogen-containing heterocyclyl are independently and optionally substituted by groups selected from halogen, hydroxyl, nitro, cyano, C1-C3 alkyl, C1-C3 deuterated alkyl, C1-C3 halogenated alkyl, C1-C3 alkoxy, C1-C3 halogenated alkoxy.

12. The compound of claim 1, or a pharmaceutically acceptable salt, a stereoisomer or a solvate thereof, wherein Z is N or CR²; wherein R² is H, cyano, halogen, C1-C3 alkyl, C1-C3 alkoxy, C1-C3 halogenated alkyl, C1-C3 halogenated alkoxy, C1-C3 deuterated alkoxy, NR³¹R³², C2-C4 alkynyl or CH₂OR³³; wherein R³¹, R³² are each independently hydrogen or C1-C6 alkyl; or R³¹, R³² are taken together with the nitrogen atom to which R³¹, R³² are attached to form a 3- to 6-membered nitrogen-containing heterocyclyl; R³³ is hydrogen or C1-C6 alkyl.

13. The compound of any of claims 1-9, or a pharmaceutically acceptable salt, a stereoisomer or a solvate thereof, wherein ring A is phenyl, naphthyl, pyridyl, pyrimidinyl, pyrazinyl, pyridazinyl, pyrrolyl, pyrazolyl, imidazolyl, 1,2,3,4-tetrahydronaphthyl, 2,3-dihydro-1H-indenyl, quinolyl, isoquinolinyl, quinazolinyl, indolyl, indazolyl or benzo[d][l,3]dioxolane.

14. The compound of any of claims 1-9, or a pharmaceutically acceptable salt, a stereoisomer or a solvate thereof, wherein R¹ is a substituent at any position on ring A; nl is 0, 1, 2 or 3; each R¹ is respectively and independently selected from: C1-C3 alkyl, halogen, hydroxyl, C1-C3 alkoxy, C1-C3 halogenated alkyl, C1-C3 halogenated alkoxy, cyano, NR²¹R²², C(O)NR²³R²⁴, CH₂R²⁵, N=S(O) (C1-C3 alkyl)₂, S(O)C1-C3 alkyl, S(O)₂R²⁶, -S-C1-C3 alkyl, C2-C4 alkenyl, C2-C4 alkynyl, C2-C4 hydroxyalkynyl, C1-C3 cyanoalkyl, triazolyl, -S-C1-C3 halogenated alkyl, C1-C3 hydroxyalkyl, - CH₂C(O)NR²⁷R²⁸, -C2-C4 alkynyl NR²⁹R³⁰, C2-C4 deuterated alkynyl, (C1-C3 alkoxy) C1-C3 halogenated alkyl- or cycloalkyl; wherein the cycloalkyl is optionally substituted by halogen or C1-C3 alkyl; wherein,
R²¹ is H, C1-C3 alkyl, C1-C3 halogenated alkyl, C(O)C1-C3 alkyl or C(O)₂C1-C3 alkyl; R²² is H, C1-C3 alkyl or C1-C3 halogenated alkyl; or R²¹, R²² are taken together with the nitrogen atom to which R²¹, R²² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²³, R²⁴ are each independently H, C1-C3 alkyl or C1-C3 halogenated alkyl; or R²³, R²⁴ are taken together with the nitrogen atom to which R²³, R²⁴ are attached to form a 3-to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁵ is hydroxyl, cyano, heterocyclyl, NR²⁵¹R²⁵², C(U)NR²⁵³R²⁵⁴ or SO₂C1-C3 alkyl; wherein R²⁵¹, R²⁵², R²⁵³, R²⁵⁴ are each independently H or C1-C3 alkyl; or R²⁵¹, R²⁵² are taken together with the nitrogen atom to which R²⁵¹, R²⁵² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy; or R²⁵³, R²⁵⁴ are taken together with the nitrogen atom to which R²⁵³, R²⁵⁴ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁶ is C1-C3 alkyl, C1-C3 halogenated alkyl or NR²⁶¹R²⁶², wherein R²⁶¹, R²⁶² are each independently H or C1-C3 alkyl; or R²⁶¹, R²⁶² are taken together with the nitrogen atom to which R²⁶¹, R²⁶² are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁷, R²⁸ are each independently H, C1-C3 alkyl or C1-C3 halogenated alkyl; or R²⁷, R²⁸ are taken together with the nitrogen atom to which R²⁷, R²⁸ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy;
R²⁹, R³⁰ are each independently H, C1-C3 alkyl or C1-C3 halogenated alkyl; or R²⁹, R³⁰ are taken together with the nitrogen atom to which R²⁹, R³⁰ are attached to form a 3- to 6-membered nitrogen-containing heterocyclyl, the 3- to 6-membered nitrogen-containing heterocyclyl is optionally substituted by one or two groups selected from halogen, methyl, trifluoromethyl, methoxy, trifluoromethoxy.

15. The compound of any of claims 1, 3, 7, or a pharmaceutically acceptable salt, a stereoisomer or a solvate thereof, wherein
R^{3a} and R^{3b} are connected to form -CHR^{3a1}- or -CHR^{3a2}CHR^{3a3}-; R^{3c} is hydrogen, halogen, hydroxyl, cyano, C1-C3 alkyl, C1-C3 deuterated alkyl or C1-C3 halogenated alkyl; R^{3a1}, R^{3a2}, R^{3a3} are each independently hydrogen, halogen, hydroxyl, cyano, methyl, deuterated methyl or halogenated methyl; and/or
R^{3d} and R^{3e} are connected to form -CHR^{3d1}- or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3d1}, R^{3d2}, R^{3d3} are each independently hydrogen, halogen, hydroxyl, cyano, methyl, deuterated methyl or halogenated methyl.

16. The compound of any of claims 1-9, or a pharmaceutically acceptable salt, a stereoisomer or a solvate thereof, wherein L is methylene or ethylene or propylene; wherein one or two hydrogen atoms on any carbon atom or on any same carbon atom of the methylene or ethylene or propylene are independently and optionally substituted by deuterium or C1-C3 alkyl; or two hydrogen atoms on any same carbon atom of the methylene or ethylene or propylene are optionally and simultaneously substituted by -CH₂CH₂- or -CH₂CH₂CH₂- to form cyclopropyl or cyclobutyl.

17. The compound of any of claims 1-9, or a pharmaceutically acceptable salt, a stereoisomer or a solvate thereof, wherein R⁶ is -L-6- to 10-membered fused heterocyclyl; two hydrogen atoms on the same carbon atom of any one or two carbon atoms on the 6- to 10-membered fused heterocyclyl are optionally and simultaneously substituted by =CR^{2a}R^{2b}; and/or the 6- to 10-membered fused heterocyclyl are optionally substituted by one or more R^{6a}; wherein R^{2a}, R^{2b} are each independently hydrogen, halogen, cyano, C1-C4 alkyl or C1-C4 halogenated alkyl; each R^{6a} is respectively and independently halogen or C1-C6 alkoxy; or
R⁶ is -L-heterocyclyl; the heterocyclyl in the -L-heterocyclyl optionally substituted by one or more R³⁵ or two hydrogen atoms on the same carbon atom are optionally and simultaneously substituted by -CH₂CH₂- or -CH₂CH₂CH₂- to form cyclopropyl or cyclobutyl; R³⁵ is defined as above;
the L is a bond or C1-C4 alkylene; wherein one or two hydrogen atoms on any carbon atom or on any same carbon atom of the C1-C4 alkylene are independently and optionally substituted by deuterium, C1-C6 alkyl, C1-C6 halogenated alkyl, C2-C4 alkenylene or C2-C4 halogenated alkenylene; or two hydrogen atoms on any same carbon atom of the C1-C4 alkylene are optionally and simultaneously substituted by -(CH₂)ₘ₃-, -(CH₂)ₘ₁-O-(CH₂)ₘ₂-, -(CH₂)ₘ₁-NR⁹-(CH₂)ₘ₂- to form a cyclic substituent; m3 is 1 or 2; each m1 is respectively and independently 0, 1, 2, or 3; each m2 is respectively and independently 0, 1, 2, or 3; and ml, m2 are not simultaneously 0; R⁹ is defined as above.

18. The compound of any of claims 1-9, or a pharmaceutically acceptable salt, a stereoisomer or a solvate thereof, wherein R⁶ is selected from the group: methyl,

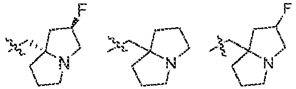

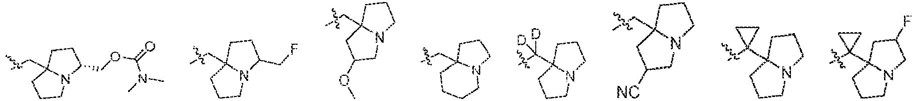

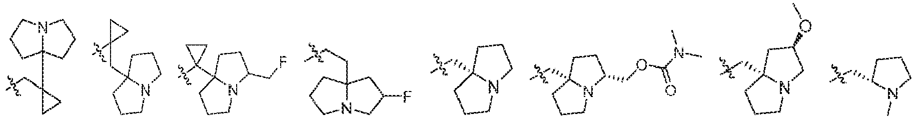

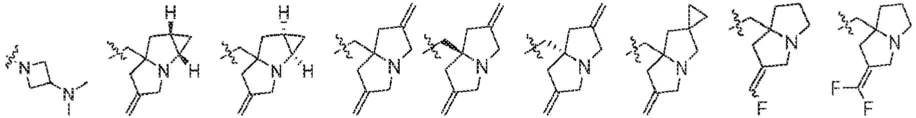

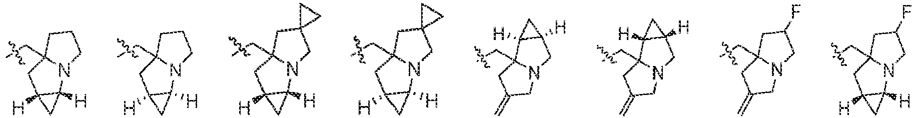

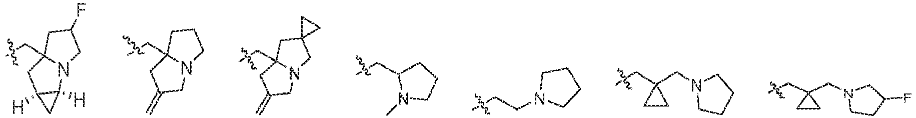

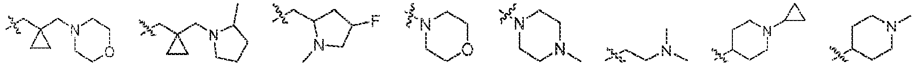

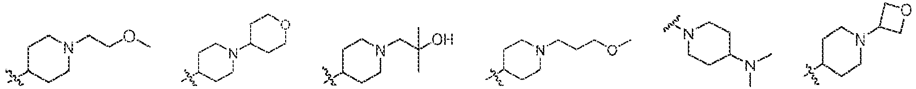

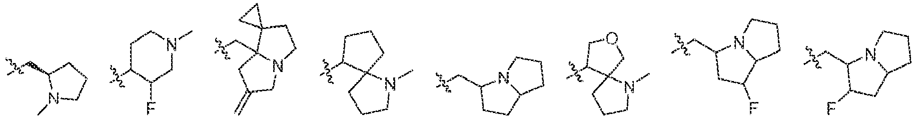

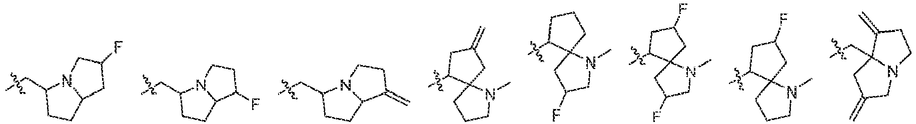

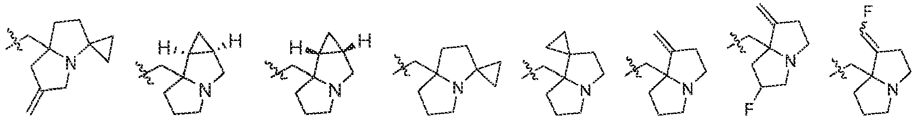

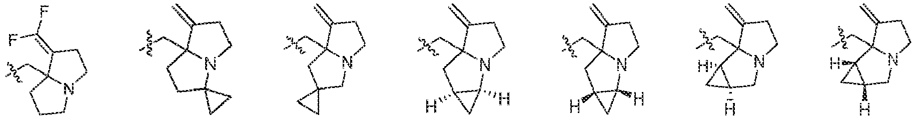

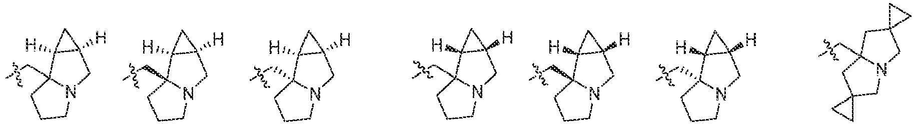

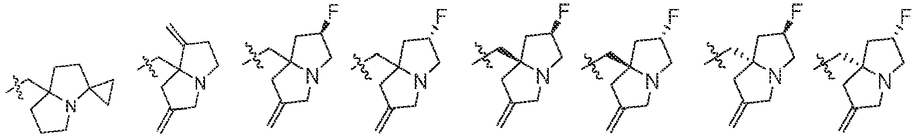

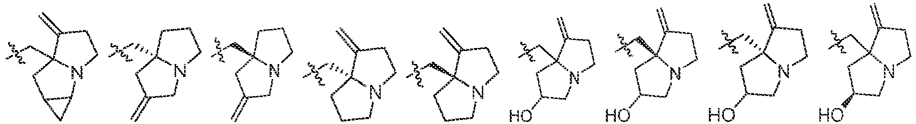

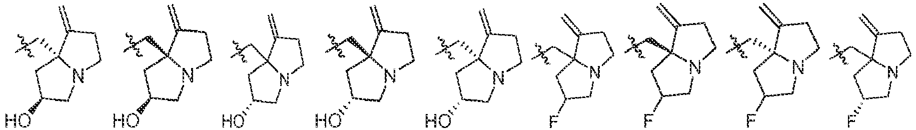

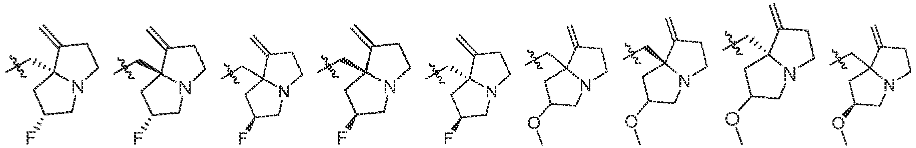

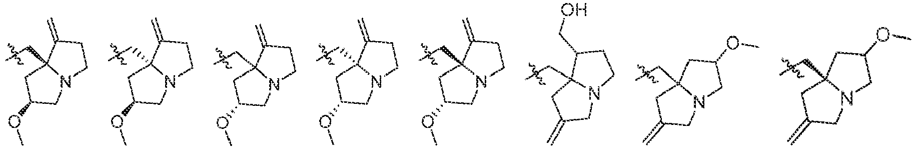

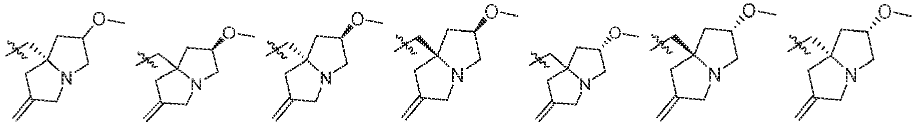

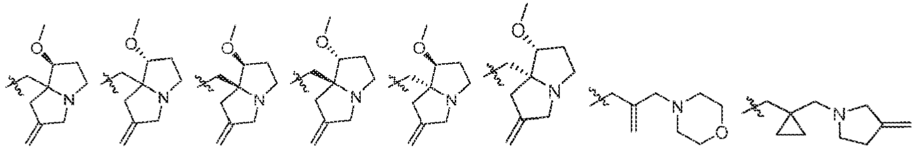

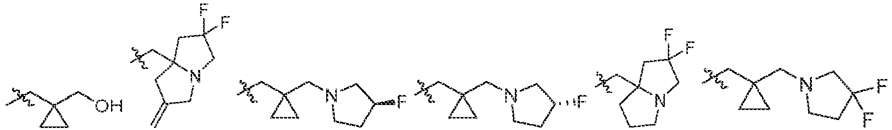

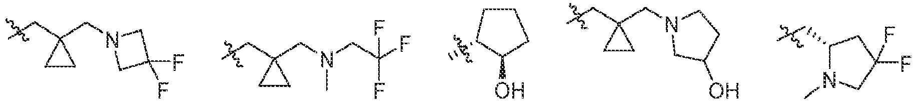

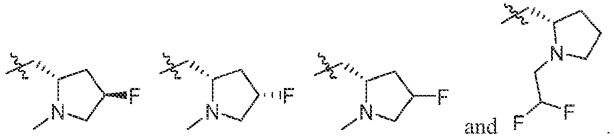

19. The compound of claim 1, or a pharmaceutically acceptable salt, a stereoisomer or a solvate thereof, the compound is selected from table (I).

20. A pharmaceutical composition comprising a therapeutically effective amount of the compound of any of claims 1-19, or a pharmaceutically acceptable salt, a stereoisomer or a solvate thereof; and a pharmaceutically acceptable excipient thereof.

21. Use of the compound of any of claims 1-19, or a pharmaceutically acceptable salt, a stereoisomer or a solvate thereof or the pharmaceutical composition of claim 20 in preparation of a medicament for preventing and/or treating of a disease or condition, the disease or condition is a KRAS G12D associated disease or disorder.

22. The use of claim 21, wherein the KRAS G12D associated disease or disorder is cancer.
